# EUROPEAN PATENT APPLICATION

(11) **EP 2 628 803 A2**
(43) Date of publication of application: **21.08.2013**
(21) Application number: 13159760.1
(22) Date of filing: 25.02.2010
(51) Int. Cl.: C12Q 1/68

(54) **Methods of detecting lung cancer**

(30) Priority: 25.02.2009 US 155364 P
(62) Divisional of application: 10746849.8
(71) Applicant: Cepheid, Sunnyvale, CA 94089 (US)
(72) Inventor: Michot, Bernard, F-81470 Maurens-Scopont (FR); Delfour, Olivier, F-81470 Maurens-Scopont (FR); Persing, David H., Sunnyvale, CA 94089 (US)
(74) Representative: Carpmaels & Ransford LLP

(57) **Abstract**

Methods of lung cancer in a sample from a patient are provided. Methods of detecting changes in expression of one or more target RNAs associated with lung cancer are also provided. Compositions and kits are also provided.

## Description

This application claims priority to U.S. Provisional Application No. 61/155,364, filed February 25, 2009, which is incorporated by reference herein in its entirety for any purpose.

### 1. BACKGROUND

Lung cancer is the leading cause of death due to cancer in the world. Lung cancer is categorized into two types, small cell lung cancer ("SCLC") and non-small cell lung cancer ("NSCLC"). SCLC is an extremely aggressive form of lung cancer with poor prognosis and a median length of survival after diagnosis of about 1 to 3 months. About 80% of lung cancer cases are categorized as NSCLC, which is categorized into three subtypes: adenocarcinoma, squamous cell carcinoma and large cell carcinoma. Greater than 85% of all NSCLCs are either adenocarcinoma or squamous-cell carcinoma.

Lung cancer is difficult to diagnose in the early stages because it may manifest no outward symptoms. When symptoms do occur, they can vary depending on the type, location and spreading pattern of the cancer, and therefore, are not readily associated with cancer. Often, lung cancer is only correctly diagnosed when it has already metastasized.

Current techniques for diagnosing lung cancer include chest x-ray and/or computed tomography ("CT") scan. Diagnosis by one of these techniques is usually confirmed by a more invasive procedure, such as transthoracic needle biopsy or transbronchial biopsy, which may still result in misdiagnosis of lung cancer. (Butnor (2008) Arch. Pathol. Lab. Med. 132:1118-1132.)

Despite advances in treatment (*e.g*., by surgery, chemotherapy, radiation or a combination), the prognosis for lung cancer remains poor, with only 15% of patients surviving more than 5 years from the time of diagnosis. Of the most common NSCLCs, adenocarcinoma progresses more rapidly and therefore has a poorer prognosis than squamous-cell carcinoma, which takes several years to develop and is therefore more likely to be diagnosed in an early stage.

One proposal for reducing the mortality and morbidity of lung cancer is to institute regular screening of high-risk individuals, e.g., those who smoke or have smoked heavily for a certain period of time, in order to detect and treat lung cancer in asymptomatic individuals. In this way, early stage lung cancer can be eradicated by surgical resection, which is thought to be the only realistic option for a cure. (Field et al. (2008) Br. J. Cancer 99:557-562*).*

There remains a need for molecular markers in lung cancer.

### 2. SUMMARY

In some embodiments, methods of detecting the presence of lung cancer in a subject are provided. In some embodiments, a method comprises detecting a level of at least one target RNA in a sample from the subject. In some embodiments, the at least one target RNA: (i) is capable of specifically hybridizing to a nucleic acid having a sequence selected from SEQ ID NOs: 1 to 397, 1063 to 1210, 2064 to 2183, 2673 to 2680, and 2689; or (ii) comprises a sequence that is complementary to at least 15 contiguous nucleotides of a sequence selected from SEQ ID NOs: 1 to 397, 1063 to 1210, 2064 to 2183, 2673 to 2680, and 2689; or (iii) comprises at least 15 contiguous nucleotides of a sequence selected from SEQ ID NOs: 794 to 1043, 2576 to 2672, and 2692. In some embodiments, a level of at least one target RNA in the sample that is greater than a normal level of the at least one target RNA indicates the presence of lung cancer in the subject. In some embodiments, the method further comprises comparing the level of the at least one target RNA in the sample to a normal level of the at least one target RNA.

In some embodiments, methods for facilitating the detection of lung cancer in a subject are provided. In some embodiments, a method comprises detecting a level of at least one target RNA in a sample from the subject. In some embodiments, the target RNA (i) is capable of specifically hybridizing to a nucleic acid having a sequence selected from SEQ ID NOs: 1 to 397, 1063 to 1210, 2064 to 2183, 2673 to 2680, and 2689; or (ii) comprises a sequence that is complementary to at least 15 contiguous nucleotides of a sequence selected from SEQ ID NOs: 1 to 397, 1063 to 1210, 2064 to 2183, 2673 to 2680, and 2689; or (iii) comprises at least 15 contiguous nucleotides of a sequence selected from SEQ ID NOs: 794 to 1043, 2576 to 2672, and 2692. In some embodiments, a method comprises communicating the results of the detection to a medical practitioner for the purpose of determining whether the subject has lung cancer.

In some embodiments, detecting a level of at least one target RNA in a sample comprises hybridizing nucleic acids of the sample with at least one polynucleotide that is complementary to a target RNA in the sample or to a complement thereof; and detecting at least one complex comprising a polynucleotide hybridized to at least one nucleic acid selected from the target RNA, a DNA amplicon of the target RNA, and a complement of the target RNA.

In some embodiments, a method for detecting the presence of lung cancer in a subject comprises (a) obtaining a sample from the subject; (b) providing the sample to a laboratory for detection of the level of at least one target RNA in the sample; and (c) receiving from the laboratory a communication indicating the level of at least one target RNA in the sample. In some embodiments, the at least one target RNA (i) is capable of specifically hybridizing to a nucleic acid having a sequence selected from SEQ ID NOs: 1 to 397, 1063 to 1210, 2064 to 2183, 2673 to 2680, and 2689; or (ii) comprises a sequence that is complementary to at least 15 contiguous nucleotides of a sequence selected from SEQ ID NOs: 1 to 397, 1063 to 1210, 2064 to 2183, 2673 to 2680, and 2689; or (iii) comprises at least 15 contiguous nucleotides of a sequence selected from SEQ ID NOs: 794 to 1043, 2576 to 2672, and 2692. In some embodiments, a level of at least one target RNA that is greater than a normal level of the at least one target RNA indicates the presence of lung cancer.

In some embodiments, levels of at least two, at least three, or at least five target RNAs are detected. In some embodiments, detection of levels of at least one, at least two, at least three, or at least five target RNAs that are greater than a normal level of the at least one target RNA indicates the presence of lung cancer.

In some embodiments, a method further comprises detecting a level of at least one target RNA that: (i) does not specifically hybridize to a nucleic acid having a sequence selected from SEQ ID NOs: 1 to 397, 1063 to 1210, 2064 to 2183, 2673 to 2680, and 2689; or (ii) does not comprise a sequence that is complementary to at least 15 contiguous nucleotides of a sequence selected from SEQ ID NOs: 1 to 397, 1063 to 1210, 2064 to 2183, 2673 to 2680, and 2689; and (iii) does not comprise at least 15 contiguous nucleotides of a sequence selected from SEQ ID NOs: 794 to 1043, 2576 to 2672, and 2692.

In some embodiments, a method comprises detection of at least one target RNA (a) is capable of specifically hybridizing to a polyncleotide sequence in Table 6; or (b) comprises a sequence that is complementary to at least 15 contiguous nucleotides of a polynucleotide sequence in Table 6. In some such embodiments, detection of a level of the at least one target RNA that is greater than a normal level of the at least one target RNA indicates the presence of non-small cell lung cancer.

In some embodiments, a method comprises detection of at least one target RNA (a) is capable of specifically hybridizing to a polyncleotide sequence in Table 7; or (b) comprises a sequence that is complementary to at least 15 contiguous nucleotides of a polynucleotide sequence in Table 7. In some such embodiments, detection of a level of the at least one target RNA that is greater than a normal level of the at least one target RNA indicates the presence of squamous cell carcinoma.

In some embodiments, a method comprises detection of at least one target RNA (a) is capable of specifically hybridizing to a polyncleotide sequence in Table 8; or (b) comprises a sequence that is complementary to at least 15 contiguous nucleotides of a polynucleotide sequence in Table 8. In some such embodiments, detection of a level of the at least one target RNA that is greater than a normal level of the at least one target RNA indicates the presence of adenocarcinoma.

In some embodiments, a method comprises detection of at least one target RNA (a) is capable of specifically hybridizing to a polyncleotide sequence in Table 9; or (b) comprises a sequence that is complementary to at least 15 contiguous nucleotides of a polynucleotide sequence in Table 9. In some such embodiments, detection of a level of the at least one target RNA that is greater than a normal level of the at least one target RNA indicates the presence of aggressive lung cancer.

In some embodiments, a method comprises detection of at least one target RNA (a) is capable of specifically hybridizing to a polyncleotide sequence in Tables 32 or 33; or (b) comprises a sequence that is complementary to at least 15 contiguous nucleotides of a polynucleotide sequence in Table 32 or Table 33.

In some embodiments, a method of detecting the presence of lung cancer is provided that comprises detecting a level of at least one target RNA in a sample from the subject, wherein the at least one target RNA (i) is capable of specifically hybridizing to a nucleic acid having a sequence selected from SEQ ID NOs: 1 to 397, 1363 to 1707, and 2312 to 2452; or (ii) comprises a sequence that is complementary to at least 15 contiguous nucleotides of a sequence selected from SEQ ID NOs: 1 to 397, 1363 to 1707, and 2312 to 2452. In some embodiments, a level of at least one target RNA in the sample that is reduced relative to a normal level of the at least one target RNA indicates the presence of lung cancer in the subject. In some embodiments, a method comprises comprises comparing the level of the at least one target RNA in the sample to a normal level of the at least one target RNA.

In some embodiments, a method of facilitating the detection of lung cancer in a subject is provided, comprising (a) detecting a level of at least one target RNA in a sample from the subject, wherein the at least one target RNA (i) is capable of specifically hybridizing to a nucleic acid having a sequence selected from SEQ ID NOs: 1 to 397, 1363 to 1707, and 2312 to 2452; or (ii) comprises a sequence that is complementary to at least 15 contiguous nucleotides of a sequence selected from 1 to 397, 1363 to 1707, and 2312 to 2452; and (b) communicating the results of the detection to a medical practitioner for the purpose of determining whether the subject has lung cancer.

In some embodiments, a method of detecting the presence of lung cancer in a subject is provided, wherein the method comprises (a) obtaining a sample from the subject, (b) providing the sample to a laboratory for detection of the level of at least one target RNA in the sample, wherein the at least one target RNA (i) is capable of specifically hybridizing to a nucleic acid having a sequence selected from SEQ ID NOs: 1 to 397, 1363 to 1707, and 2312 to 2452; or (ii) comprises a sequence that is complementary to at least 15 contiguous nucleotides of a sequence selected from SEQ ID NOs: 1 to 397, 1363 to 1707, and 2312 to 2452; and (c) receiving from the laboratory a communication indicating the level of at least one target RNA in the sample. In some embodiments, a level of at least one target RNA that is reduced relative to a normal level of the at least one target RNA indicates the presence of lung cancer.

In some embodiments, a method further comprises detecting a level of at least one second target RNA in a sample from the subject, wherein the at least one second target RNA: (i) is capable of specifically hybridizing to a nucleic acid having a sequence selected from SEQ ID NOs: 1 to 397, 1063 to 1210, 2064 to 2183, 2673 to 2680, and 2689; or (ii) comprises a sequence that is complementary to at least 15 contiguous nucleotides of a sequence selected from SEQ ID NOs: 1 to 397, 1063 to 1210, 2064 to 2183, 2673 to 2680, and 2689; or (iii) comprises at least 15 contiguous nucleotides of a sequence selected from SEQ ID NOs: 794 to 1043, 2576 to 2672, and 2692. In some embodiments, a level of at least one second target RNA in the sample that is greater than a normal level of the at least one second target RNA indicates the presence of lung cancer in the subject.

In some embodiments, a method comprises isolating nucleic acids from a sample. In some embodiments, the nucleic acids comprise RNA that has been separated from DNA. In some embodiments, a target RNA in its mature form comprises fewer than 30 nucleotides. In some embodiments, a target RNA is a microRNA.

In some embodiments, synthetic polynucleotides are provided. In some embodiments, compositions comprising a plurality of synthetic polynucleotides are provided. In some embodiments, a synthetic polynucleotide comprises a first region, wherein the first region comprises a sequence of at least 8, at least 9, at least 10, at least 11, at least 12, at least 13, at least 14, or at least 15 contiguous nucleotides that is identical or complementary to a sequence of at least 8, at least 9, at least 10, at least 11, at least 12, at least 13, at least 14, or at least 15 contiguous nucleotides of one of SEQ ID NOs: 1 to 165, 254 to 360, 1063 to 1081, 1090 to 1190, 2064 to 2091, 2107 to 2168, 1363 to 1659, 2312 to 2440, and 2576 to 2680. In some embodiments, a synthetic polynucleotide comprises a detectable label. In some embodiments, the detectable label is a FRET label. In some embodiments, the first region is identical or complementary to a region of a target RNA. In some embodiments, the polynucleotide further comprises a second region that is not identical or complementary to a region of the target RNA.

In some embodiments, kits are provided. In some embodiments, a kit comprises a synthetic polynucleotide. In some embodiments, a kit comprises a composition comprising a plurality of synthetic polynucleotides. In some embodiments, a kit comprises at least one polymerase. In some embodiments, a kit comprises dNTPs.

Further embodiments and details of the inventions are described below.

### 3. BRIEF DESCRIPTION OF THE FIGURES

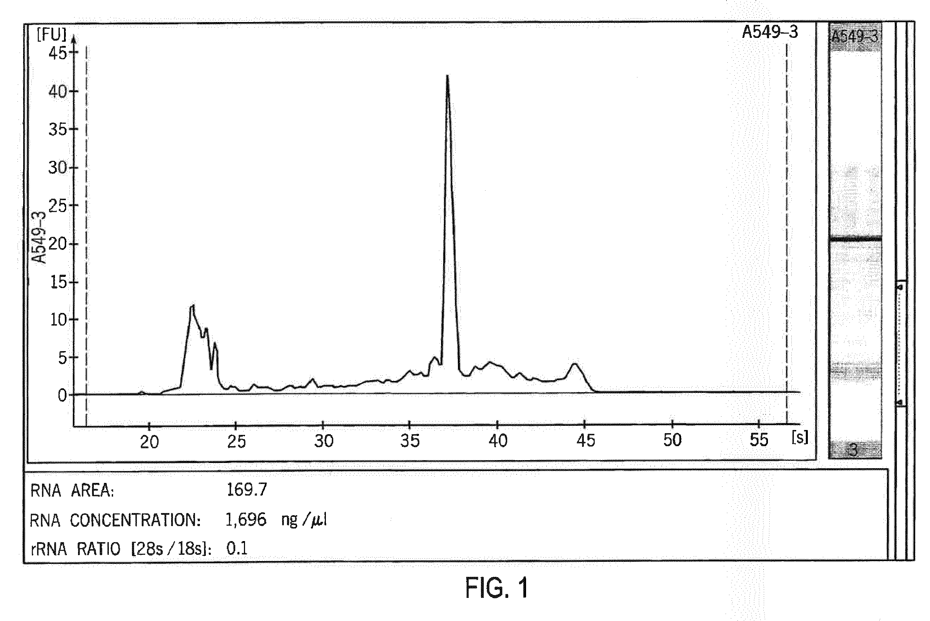

FIG. 1 shows an electropherogram obtained on an Agilent Bioanalyser 2100 to assess the quality of total RNA purified as described in Example 1 from A549 human adenocarcinoma cell line.

### 4. DETAILED DESCRIPTION

### 4.1. Detecting lung cancer

### 4.1.1. General methods

Methods of detecting lung cancer by measuring levels of target RNAs are provided. In some embodiments, methods are presented for detecting non-small cell lung cancer in a human. In some embodiments, a method comprises detecting altered levels of at least one target RNA relative to normal levels of the at least one target RNA. In some embodiments, elevated levels of one or more target RNAs are indicative of lung cancer. In some embodiments, reduced levels of one or more target RNAs are indicative of lung cancer. In some embodiments, the method comprises detecting an altered level of at least one target RNA that is capable of specifically hybridizing to a sequence selected from the sequences in Tables 1, 2, 6 to 9, 18, 20, 23, 27, 28, 30, and 32 to 34. In some embodiments, the method comprises detecting an altered level of at least one target RNA that is capable of specifically hybridizing to a sequence selected from SEQ ID NOs: 1 to 397, 1063 to 1210, 1363 to 1707, 2064 to 2183, 2312 to 2452, 2673 to 2680, and 2689. In some embodiments, the method comprises detecting an altered level of at least one target RNA selected from the microRNAs in Tables 4, 5, and 38. In some embodiments, the method comprises detecting an altered level of at least one target RNA that comprises at least 15, at least 16, at least 17, at least 18, at least 19, at least 20, at least 21, at least 22, at least 23, or at least 24 contiguous nucleotides of a sequence selected from SEQ ID NOs.: 794 to 1043, 2576 to 2672, and 2692. In some embodiments, the method comprises detecting an altered level of at least one target RNA that comprises a sequence that is complementary to at least 15, at least 16, at least 17, at least 18, at least 19, at least 20, at least 21, at least 22, at least 23, or at least 24 contiguous nucleotides of a sequence selected from SEQ ID NO.: 1 to 397, 1063 to 1210, 1363 to 1707, 2064 to 2183, 2312 to 2452, 2673 to 2680, and 2689. In some embodiments, an altered level is an elevated level. In some embodiments, an altered level is a reduced level. In some embodiments, a method comprises detecting an elevated level of at least one target RNA and a reduced level of at least one other target RNA. In some embodiments, the target RNA, in its mature form, comprises fewer than 30 nucleotides. The target RNA, in some embodiments, is a microRNA.

In the present disclosure, "a sequence selected from" encompasses both "one sequence selected from" and "one or more sequences selected from." Thus, when "a sequence selected from" is used, it is to be understood that one, or more than one, of the listed sequences may be chosen.

Detection of a level of target RNA that is greater than a normal level of target RNA indicates the presence of lung cancer in the patient from whom the sample is taken. In some embodiments, the detecting is done quantitatively. In other embodiments, the detecting is done qualitatively. In some embodiments, detecting a target RNA comprises forming a complex comprising a polynucleotide and a nucleic acid selected from a target RNA, a DNA amplicon of a target RNA, and a complement of a target RNA. In some embodiments, the level of the complex is then detected and compared to a normal level of the same complex. The level of the complex, in some embodiments, correlates with the level of the target RNA in the sample.

"Non-small cell lung cancer" or "NSCLC" is one of two categories of lung cancer found in humans. About 80% of patients diagnosed with lung cancer have non-small cell lung cancer. NSCLC is further broken down into three sub-categories, depending on the cells in which they originate: (i) adcnocarcinoma, which originates in the cells that line the alveoli and make substances such as mucus; (ii) squamous cell or epidermoid carcinoma, which originates in the squamous cells; and (iii) large cell carcinoma, which may originate in several different types of large cells. More than 50% of patients with NSCLC have either adenocarcinoma or squamous cell carcinoma. The histology class nonsquamous cell carcinoma includes both adenocarcinoma and large cell carcinoma.

Cancer can be divided into clinical and pathological stages. The clinical stage is based on all available information about a tumor, such as information gathered through physical examination, radiological examination, endoscopy, etc. The pathological stage is based on the microscopic pathology of a tumor.

The TNM (tumor, node, metastasis) system classifies a cancer by three parameters - the size of the tumor and whether it has invaded nearby tissues, involvement of lymph nodes, and metastases. T (tumor) is assigned a number from 1 to 4, according to the size and extent of the primary tumor. N (node) is assigned a number from 0 to 3, in which 0 means no spreading to the lymph nodes, 1 is spreading to the closest lymph nodes, and 3 is spreading to the most distant and greatest number of lymph nodes, and 2 is intermediate between 1 and 3. M (metastasis) is assigned 0 for no distant metastases, or 1 for distant metastases beyond regional lymph nodes.

For lung cancer, Overall Stage Grouping assigns a cancer a roman numeral of 0, 1, II, III, and IV, and a letter, A or B, depending on the stage. Stage 0 is carcinoma in situ, which usually does not form a tumor. Stages IA (T1N0M0) and IB (T2N0M0) is cancer that is localized to one part of the body. Stage IIA (T1N1M0) and IIB (T2N1M0 and T3N0M0) is cancer that is localized, but more advanced. Stage IIIA (T1-3N2M0 or T3N1M0) and IIIB (any T4 or any N3M0) cancer is also locally advanced. Stage IV (any M1) is cancer that has metastasized. As used herein, the term "early stage cancer" refers to Stages IA and IB and Stages IIA and IIB cancers.

As used herein, an "aggressive" form of lung cancer is a lung cancer that advances quickly from one stage to the next and/or metastasizes at an early stage, resulting in a poor prognosis for the patient.

Tables 1 and 2, below, list 397 hybridization probes that have been found to be complementary to, and hybridize with, target RNAs in lung cancer cells. These target RNAs were detected at elevated levels or at reduced levels in certain primary tumors and/or human lung cancer cell lines (respectively Examples 1 and 2). Two hundred seventy-five of the probes are complementary to, and hybridize with, novel target RNA species that are expressed in human cells. The other one hundred and twenty-two probes are complementary to, and hybridize with, publicly known microRNAs that have been accessioned by others into miRBase (http://microma.sanger.ac.uk/; *see* Griffiths-Jones S. et al. (2007) Nucl. Acids Res. 36:154-158). These latter probes are designated by either "mir" or "let" in Tables 1 and 2.

Tables 18 to 21, below, list hybridization probes that have been found to be complementary to, and hybridize with, target RNAs that were detected at elevated levels in certain primary tumors (see Example 4). Certain probes listed in Tables 18 and 20 are complementary to, and hybridize with, novel target RNA species that are expressed in human cells. Other probes in Tables 18 and 20 are complementary to, and hybridize with, publicly known microRNAs that have been accessioned by others into miRBase (http://microma.sanger.ac.uk/; *see* Griffiths-Jones S. et al. (2007) Nucl. Acids Res. 36:154-158). These latter probes are designated by either "mir" or "let."

Tables 23 and 24, below, list hybridization probes that have been found to be complementary to, and hybridize with, target RNAs that were detected at reduced levels in certain primary tumors (see Example 4). Certain probes listed in Tables 23 and 24 are complementary to, and hybridize with, novel target RNA species that are expressed in human cells. Other probes in Tables 23 and 24 are complementary to, and hybridize with, publicly known microRNAs that have been accessioned by others into miRBase (http://microma.sanger.ac.uk/; *see* Griffiths-Jones S. et al. (2007) Nucl. Acids Res. 36:154-158). These latter probes are designated by either "mir" or "let."

Tables 27 and 28, below, list hybridization probes that have been found to be complementary to, and hybridize with, target RNAs that were detected at elevated levels in certain lung cancer cell lines (see Example 5). Certain probes listed in Tables 27 and 28 are complementary to, and hybridize with, novel target RNA species that are expressed in human cells. Other probes in Tables 27 and 28 are complementary to, and hybridize with, publicly known microRNAs that have been accessioned by others into miRBase (http://microma.sanger.ac.uk/; see Griffiths-Jones S. et al. (2007) Nucl. Acids Res. 36:154-158). These latter probes are designated by either "mir" or "let."

Table 30, below, lists hybridization probes that have been found to be complementary to, and hybridize with, target RNAs that were detected at reduced levels in certain lung cancer cell lines (see Example 5). Certain probes listed in Table 30 are complementary to, and hybridize with, novel target RNA species that are expressed in human cells. Other probes in Table 30 are complementary to, and hybridize with, publicly known microRNAs that have been accessioned by others into miRBase (http://microma.sanger.ac.uk/; *see* Griffiths-Jones S. et al. (2007) Nulc. Acids Res. 36:154-158). These latter probes are designated by either "mir" or "let."

In Tables 1 and 2, respectively, the expression levels of target RNAs measured for each of the identified primary tumors, and for each of the identified cell lines, are expressed as fold-changes in expression relative to expression levels measured in normal human lung total RNA (see Examples 1 and 2). Similarly, in Tables 18 to 21, 23, 24, 27, 28, and 30, the expression levels of target RNAs measured for each of the identified primary tumors, and for each of the identified cell lines, are expressed as fold-changes in expression relative to expression levels measured in normal human lung total RNA (see Examples 4 and 5).

Table 6 lists target RNAs from Table 1 that are present at increased levels in NSCLCs. Table 7 lists target RNAs that are more frequently present at elevated levels in squamous cell carcinoma. In some embodiments, a method comprises detecting an elevated level of at least one target RNA from Table 7. In some such embodiments, detection of an elevated level of at least one target RNA from Table 7 is indicative of squamous cell carcinoma. Table 8 lists target RNAs that arc more frequently present at elevated levels in adenocarcinoma. In some embodiments, a method comprises detecting an elevated level of at least one target RNA from Table 8. In some such embodiments, detection of an elevated level of at least one target RNA from Table 8 is indicative of adenocarcinoma. Table 9 lists target RNAs that are present at increased levels in aggressive forms of lung cancer. In some embodiments, a method comprises detecting an elevated level of at least one target RNA from Table 9. In some such embodiments, detection of an elevated level of at least one target RNA from Table 9 is indicative of an aggressive form of lung cancer.

In some embodiments, a method comprises detecting multiple isomirs with a single probe. Detection of an elevated level of one or multiple isomirs is considered to be indicative of lung cancer. When multiple microRNAs having the same sequence but arc expressed from different genes, one or more of the genes may be uprcgulatcd in a lung cancer patient. Detection of a microRNA expressed from any one of the genes is considered to be indicative of lung cancer.

For convenience of reference herein, and not by way of limitation, some "target RNA" species are denominated "microRNAs" in the tables set forth herein and Example 1. In some embodiments, the target RNA is a single mature microRNA capable of specifically hybridizing to a hybridization probe set forth in any of Tables 1, 2, 6 to 9, 18, 20, 23, 27, 28, 30, and 32 to 34. In some embodiments, a target RNA is a single mature microRNA that comprises a sequence that is complementary to at least 15 contiguous nucleotides of a sequence selected from SEQ ID NO.: 1 to 397, 1063 to 1210, 1363 to 1707, 2064 to 2183, 2312 to 2452, 2673 to 2680, and 2689. In some embodiments, a target RNA is a single mature microRNA that comprises at least 15 contiguous nucleotides of a sequence selected from SEQ ID NOs: 794 to 1043, 2576 to 2672, and 2692. In some embodiments, target RNA may include a plurality of target RNAs, all of which are capable of specifically hybridizing to a single complementary probe sequence (for example, when two or more target microRNAs are isomirs). In some embodiments, the so-denominated "microRNA" is one or more RNA species capable of specifically hybridizing to the respective hybridization probe, such that one or more target RNAs do not meet canonical definitions for mature microRNAs. In some embodiments, a target RNA is an mRNA. In some embodiments, the "target RNA" is a piwi-interacting RNA (piRNA), *i.e.,* a small RNA expressed in animal cells that is distinct in size (26-31 nt) from microRNAs and that forms distinct complexes with Piwi proteins that are involved in transcriptional gene silencing.

Mature human microRNAs are typically composed of 17 to 27 contiguous ribonucleotides, and often are from 19 to 25 nucleotides in length, or 21 or 22 nucleotides in length. The sequences of some target microRNAs that can be detected in accordance with the present disclosure can be found within the pre-microRNA sequences shown in Tables 3, 22, 25, 29, 31, and 37 (SEQ ID NOs: 398 to 793, 1211 to 1362, 1708 to 2063, 2184 to 2311, 2453 to 2575, and 2681 to 2688, 2690, and 2691). In some embodiments, more than one mature target RNA is derived from a single pre-microRNA shown in Tables 3, 22, 25, 29, 31, and 37. The sequences of some publicly known mature microRNAs are shown below in Tables 4 and 5 (SEQ ID NOs: 794 to 1043, and 2692). Further, in some embodiments, a microRNA comprises at least 10, at least 11, at least 12, at least 13, at least 14, at least 15, at least 16, at least 17, at least 18, at least 19, at least 20, at least 21, at least 22, at least 23, at least 24, at least 25, or at least 26 contiguous nucleotides of a sequence in Table 38 (SEQ ID NOs: 2576 to 2672).

While not intending to be bound by theory, mammalian microRNAs mature as described herein. A gene coding for a mieroRNA is transcribed, leading to production of a microRNA precursor known as the "pri-microRNA" or "pri-miRNA." The pri-miRNA can be part of a polycistronic RNA comprising multiple pri-miRNAs. In some circumstances, the pri-miRNA forms a hairpin with a stem and loop, which may comprise mismatched bases. The hairpin structure of the pri-miRNA is recognized by Drosha, which is an RNase III endonuclease protein. Drosha can recognize terminal loops in the pri-miRNA and cleave approximately two helical turns into the stem to produce a 60-70 nucleotide precursor known as the "pre-microRNA" or "pre-miRNA." Drosha can cleave the pri-miRNA with a staggered cut typical of RNase III endonucleases yielding a pre-miRNA stem loop with a 5' phosphate and an approximately 2-nucleotide 3' overhang. Approximately one helical turn of the stem (about 10 nucleotides) extending beyond the Drosha cleavage site can be essential for efficient processing. The pre-miRNA is subsequently actively transported from the nucleus to the cytoplasm by Ran-GTP and the export receptor Exportin-5.

The pre-miRNA can be recognized by Dicer, another RNase III endonuclease. In some circumstances, Dicer recognizes the double-stranded stem of the pre-miRNA. Dicer may also recognize the 5' phosphate and 3' overhang at the base of the stem loop. Dicer may cleave off the terminal loop two helical turns away from the base of the stem loop leaving an additional 5' phosphate and an approximately 2-nucleotide 3' overhang. The resulting siRNA-like duplex, which may comprise mismatches, comprises the mature microRNA and a similar-sized fragment known as the microRNA*. The microRNA and microRNAs may be derived from opposing arms of the pri-miRNA and pre-miRNA. The mature microRNA is then loaded into the RNA-induced silencing complex ("RISC"), a ribonucleoprotein complex. In some cases, the microRNA* also has gene silencing or other activity.

It is understood that where a sequence includes thymine (T) bases, a target RNA may contain uracil (U) bases instead.

**Table 1**

| | | | **fold-changes in primary tumors vs. normal Lung** | | | | | | | | | |
|---|---|---|---|---|---|---|---|---|---|---|---|---|
| **Array probe** | **Array probe sequence (5' to 3', without linker)** | **probe SEQ ID NO:** | **Epi4** | **Epi7** | **Epi5** | **Adk1** | **Adk3** | **Adk11** | **Adk8** | **Adk9** | **Adk2** | **Adk10** |
| 266-R4-1 | GTCGCCCCCTCCCCCAAGTTGAGACTTGCAGCTAC | 1 | 7.33 | 1.43 | 1.97 | 3.81 | -3.74 | -2.28 | -3.72 | -1.19 | -11.61 | -2.38 |
| 673-L4-1 | GCTCCCCTCACTGTGAACTTTTCACCCAGCTAACCTGC TCCTCAC | 2 | 3.55 | 1.45 | -1.26 | 1.44 | (nd) < -6.23 | -2.70 | -5.73 | -1.21 | -6.23 | -4.03 |
| 836-R4-1 | AAATAATCATTCCAAATGGTTCTCCCTGCTATGATTCAC | 3 | 6.78 | 1.06 | (nd) < -6.03 | 2.23 | -1.23 | -2.34 | -3.54 | -1.02 | -6.03 | -1.43 |
| 3249-L4-1 | GCGGAGCCGCCGCCATCCCCGGAGCCGCCGCCGCCG CCGCC | 4 | 1.46 | 1.91 | 2.34 | -1.38 | -2.37 | -3.34 | -3.73 | -2.89 | -6.52 | -2.00 |
| 3371-L4-1 | TTTCCTTTCCTCCCCTCCACACCCCATGACTCCCCACA CTTGAG | 5 | 7.78 | 1.44 | (nd) < -3.63 | 3.28 | (nd) < -3.63 | -1.54 | -2.45 | -1.89 | (nd) < -3.63 | -1.75 |
| 3717-L2-1 | CCGCCCTCCCCATAGCCTCACCCCAAACCCACTCACA | 6 | 5.45 | -1.46 | 2.40 | 2.66 | -3.24 | -3.42 | -6.97 | -22.96 | -22.96 | -2.75 |
| 3799-R3-1 | CCAGAGGCCCCCCGCCGGCC | 7 | 4.81 | 1.62 | 1.19 | 3.07 | -4.42 | -2.84 | -5.33 | -1.46 | -18.87 | -2.91 |
| 3872-L1-1 | TCATTTTCTTGTCTTCTTCCCTTATGCAC | 8 | > 6.17 | nd | nd | > 2.09 | nd | nd | nd | nd | nd | nd |
| 3875-R3-1 | CTCTCTCCCACTTTAATAA | 9 | > 20.25 | nd | nd | > 2.90 | nd | nd | nd | nd | nd | nd |
| 3897-R3-1 | CAGCCGCCTCCCCCTCAGCGTTAA | 10 | 5.07 | 1.62 | 1.80 | 1.97 | -3.20 | -3.17 | -4.86 | -1.07 | -2.38 | -1.89 |
| 3923-R3-1 | GCCTCTCACAAAGGATCTCCTTCATCCCTCTCC | 11 | 17.53 | 1.49 | (nd) < -1.39 | 4.86 | (nd) < -1.39 | 2.05 | -1.32 | (nd) < -1.39 | (nd) < -1.39 | -1.16 |
| 3953-R3-2 | ACTCCAGCCTCCGCCGCCTCAGCTTCCCGAGC | 12 | 1.50 | 1.86 | 2.21 | 1.28 | -2.29 | -3.82 | -5.17 | nd G3 | nd G3 | -2.73 |
| 3995-L2-2 | CTATAAAACTTCGAAAAGTCCCTCCTCCTCACGT | 13 | 6.16 | 1.93 | 1.05 | 3.01 | -3.51 | -2.18 | -4.19 | 1.21 | -5.70 | -1.85 |
| 4026-R3-1 | GGCGAGAGAGAAAGCCCCCCT | 14 | 6.37 | -1.17 | 2.06 | 3.81 | -3.69 | -3.06 | -5.40 | -1.70 | -18.38 | -4.41 |
| 4037-R3-2 | GCCTGTTCCCTGGCATGTACTGTAATTTATCT | 15 | 6.57 | 1.44 | (nd) < -1.28 | 2.17 | (nd) < -1.28 | 2.17 | 2.34 | 2.64 | 6.81 | -1.06 |
| 4143-R3-1 | TCAGCGTCTTGCTCTCCTCCTGGTA | 16 | > 7.19 | nd | nd | > 2.08 | nd | nd | nd | nd | nd | nd |
| 4203-R3-2 | GCACATTCCCACTTCCCCAGAGGCAGGCTCCATAT | 17 | > 4.30 | nd | nd | nd | nd | nd | nd | nd | nd | nd |
| 4205-R3-2 | GCCCTACAACTTCATCCTCACCACTCACACCAC | 18 | > 3.92 | nd | nd | > 2.47 | nd | nd | nd | nd | nd | nd |
| 4303-R1-1 | AGTGCCCGCTCCTCCGACCTCCCTGCGCACC | 19 | 4.55 | 2.06 | 1.53 | 2.49 | -3.30 | -1.83 | -3.64 | -2.09 | -10.57 | -2.26 |
| 4315-R3-2 | TCCCCGGCCCTCTCCATTCTCGGCTCCGGAGCA | 20 | 5.42 | 1.69 | 1.44 | 2.31 | -2.65 | -2.17 | -2.74 | -1.64 | -4.81 | -1.87 |
| 4361-R3-1 | CGTCTCCCTCCCTCATGTGC | 21 | 13.35 | 3.46 | (nd) < -1.73 | 4.34 | (nd) < -1.73 | -1.41 | -1.74 | 2.20 | (nd) < -1.73 | 1.27 |
| 4440-L3-2 | TTTGACATTCAGAGCACTGGGCAGAAATCACA | 22 | 6.02 | -1.51 | 1.14 | 1.60 | (nd) < -3.39 | -1.05 | -1.83 | -1.14 | 1.57 | -2.75 |
| 4440-R3-2 | GTCATAGTTACTCCCGCCGTTTACCCGCATTTC | 23 | 9.42 | 1.72 | 1.53 | 3.52 | -3.11 | -1.63 | -3.21 | -1.13 | -1.70 | -2.72 |
| 4448-R3-1 | CCTACCCCCAGCATCTCCTCACGCCATTGCC | 24 | > 2.43 | nd | nd | > 1.62 | nd | nd | nd | nd | nd | nd |
| 4479-R3-1 | AGCCCCCTGCCCGGAAATTCAAAACAACTGC | 25 | 3.07 | 3.03 | -1.01 | 2.30 | -7.11 | -3.04 | -3.90 | -2.34 | -5.33 | -2.81 |
| 4593-R3-1 | AGCAGATGACATAACTCCCCCGGCATCAG | 26 | 8.31 | 2.80 | 1.23 | 1.93 | -4.73 | -2.65 | -5.00 | 1.79 | -9.77 | 1.20 |
| 4666-R4-1 | GCCGACTCCCCCCAACACCTGCGGGTGGCAC | 27 | 24.77 | 2.27 | 3.49 | 7.40 | -2.36 | -1.70 | -2.05 | 2.37 | -3.62 | -1.01 |
| 4790-L4-1 | AACCTGTCTCCCTCATTACTAGAATTCTGGG | 28 | > 14.59 | > 1.58 | nd | > 4.07 | nd | nd | nd | nd | nd | nd |
| 4829-R2-1 | TCCCTTTGTGCTGCCCGAGTGCCTTCCCCCTG | 29 | 12.09 | 1.52 | 1.69 | 5.73 | -6.10 | -1.29 | -4.20 | -1.12 | -9.92 | -2.88 |
| 4855-R3-1 | GGGCTGCCGGGTCTCCCGCTTCC | 30 | 8.26 | 1.91 | 1.44 | 1.78 | -3.56 | -1.50 | -1.81 | 1.87 | -2.80 | 1.20 |
| 4875-R2-2 | CACAGCCCCTTCCTGTGACTTCACAC | 31 | 4.28 | -1.44 | 1.02 | 2.82 | (nd) < -5.97 | -2.46 | -5.03 | -1.17 | (nd) < -5.97 | -4.78 |
| 4988-R4-1 | CTCCTCCTCCCCGTCTTTGGATACCAAACACTGGAC | 32 | 9.10 | 1.72 | 1.15 | 2.70 | (nd) < -3.75 | -2.04 | -2.34 | -1.34 | (nd) < -3.75 | -1.55 |
| 5006-L3-1 | ACAGCTCCCTCTGCTGGCTCC | 33 | 6.58 | 1.11 | 2.00 | 5.46 | (nd) < -1.43 | (nd) < -1.43 | (nd) < -1.43 | (nd) < -1.43 | (nd) < -1.43 | (nd) < -1.43 |
| 5080-R3-1 | CTTGCAAAGGGTCTCCTTCATCCCTCTCCA | 34 | 5.91 | -1.01 | -1.35 | 2.60 | (nd) < -4.53 | 1.28 | -1.87 | 1.18 | (nd) < -4.53 | -3.70 |
| 5192-L3-2 | CATTTTTCCCCTTCCTTCCTCTATATCAGCAA | 35 | 1.54 | 1.24 | (nd) < -1.61 | 2.92 | (nd) < -1.61 | (nd) < -1.61 | -1.79 | 1.39 | (nd) < -1.61 | (nd) < -1.61 |
| 5342-L3-1 | CACCACCAAACCAAATGCCGCTGCTCTCCTTCCA | 36 | 1.95 | 1.46 | 2.07 | 1.19 | (nd) < -4.91 | -2.70 | -4.03 | -1.81 | (nd) < -4.91 | -1.99 |
| 5521-L2-1 | GTCTTGGGTGGGCCCTCCCCAGAGCACACCCTCT | 37 | 4.19 | (nd) < -1.44 | 2.44 | 2.24 | (nd) < -1.44 | (nd) < -1.44 | (nd) < -1.44 | (nd) < -1.44 | (nd) < -1.44 | (nd) < -1.44 |
| 5554-R2-1 | CCCCACCCCCTCATCAGCTGCTCCCAGAT | 38 | 3.45 | 1.79 | 1.00 | 1.73 | (nd) < -6.36 | -2.78 | -2.03 | -1.57 | (nd) < -6.36 | -1.91 |
| 5638-R2-1 | GGCCCTCCCCCTGCCTGTGATAGGCTGCTTG | 39 | 5.54 | -1.87 | 2.47 | 3.18 | -2.79 | -3.13 | -9.55 | 1.18 | -15.66 | -3.29 |
| 5640-L3-1 | GCCATGGAACACCGTGCCTGCCCCTCTCGAGA | 40 | 3.56 | 2.33 | 1.22 | 2.16 | -4.15 | -1.75 | -2.54 | -1.31 | -9.19 | -2.51 |
| 5726-L3-1 | TAATAAAATATCTTCTCACTGTGCCCTTG | 41 | > 8.13 | nd | nd | > 1.83 | nd | nd | nd | nd | nd | nd |
| 5782-L3-1 | GATTCCAGCCCCTTCCCCC | 42 | 6.87 | 1.80 | 1.13 | 3.16 | (nd) <-3.21 | -1.78 | -3.13 | (nd) <-3.21 | (nd) <-3.21 | -2.66 |
| 5795-R1-1 | CTGCCCTCCAAGAAATAAATTACCCGCAATTACT | 43 | 3.41 | 1.05 | 1.52 | 2.16 | (nd) < -2.02 | (nd) < -2.02 | (nd) < -2.02 | (nd) < -2.02 | (nd) < -2.02 | (nd) < -2.02 |
| 5836-R3-2 | CATTAACCCCCATTATCACAGCACGCCCCATTC | 44 | 13.99 | 1.99 | (nd) < -1.09 | 2.26 | (nd) < -1.09 | (nd) < -1.09 | (nd) < -1.09 | (nd) < -1.09 | (nd) < -1.09 | (nd) < -1.09 |
| 5854-R3-1 | CCCTCCCTCTCCGAAAGAATGTGTCAC | 45 | 22.51 | -1.01 | 1.54 | 4.54 | (nd) <-4.11 | -1.03 | -2.56 | 5.86 | -1.93 | -1.08 |
| 5971-R3-1 | CTGCTCAGCCTCCCACATCTGT | 46 | 5.67 | -1.01 | (nd) < -1.78 | 2.33 | (nd) < -1.78 | -1.08 | -1.64 | 2.13 | (nd) < -1.78 | -1.38 |
| 6008-R1-1 | ACAATACCCCCACCTTTTTCCTGTACCTTAC | 47 | > 3.56 | nd | nd | > 2.06 | nd | nd | nd | nd | nd | nd |
| 6016-R2-1 | AAACTCCAGCAGCCCCGTCAGCCTCCTGCT | 48 | 4.31 | 1.91 | 1.65 | 2.09 | (nd) < -2.53 | (nd) < -2.53 | -2.58 | -1.41 | (nd) < -2.53 | -1.80 |
| 6037-R3-2 | GCAATTCCCTTTCCTCCATCTCCAATTTTCCTC | 49 | 4.15 | -1.55 | 1.15 | 1.38 | (nd) < -2.24 | (nd) < -2.24 | (nd) < -2.24 | (nd) < -2.24 | (nd) < -2.24 | (nd) < -2.24 |
| 6096-R3-1 | TGTTTTAATCCTGCCCCGT | 50 | 8.12 | 2.89 | 2.83 | 1.81 | (nd) < -1.12 | (nd) <-1.12 | (nd) < -1.12 | (nd) <-1.12 | (nd) < -1.12 | (nd) < -1.12 |
| 6183-R3-1 | GATTCCACTTTTCTTAATGACTTTCCCCTCCT | 51 | > 2.90 | nd | nd | > 3.58 | nd | nd | nd | nd | nd | > 1.17 |
| 6192-L3-1 | AGATAAAAAACCACCCACCCAGCAC | 52 | 6.12 | 1.20 | (nd) < -3.75 | 1.36 | (nd) < -3.75 | -1.17 | -3.42 | (nd) < -3.75 | (nd) < -3.75 | (nd) < -3.75 |
| 6233-L3-1 | AAAATTAGATTTCCACTTTATCCTTCTCCC | 53 | > 15.87 | nd | nd | > 4.27 | nd | nd | nd | > 2.53 | nd | nd |
| 6235-R3-1 | GCTCCAAAAATCCATTTAATATATTGTCCTT | 54 | 7.85 | 1.15 | -2.40 | 2.29 | -6.59 | -2.77 | -3.73 | -1.08 | -12.11 | -2.01 |
| 6287-L3-2 | GCCCCGCCCCACCTTTCGGGGCTCACCTGGC | 55 | 5.41 | 2.01 | 1.64 | 3.04 | (nd) < -3.75 | -2.07 | -2.75 | (nd) < -3.75 | (nd) < -3.75 | -2.87 |
| 6409-L3-1 | CGTTCCCAACCGCACGCGCCGCCTTCTGGAAC | 56 | 2.22 | 1.37 | 2.40 | 1.28 | -2.50 | -3.09 | -5.39 | -2.53 | -6.45 | -2.76 |
| 6434-R3-1 | AGCCCTCCCACCAGCCAGCTGCAGTGC | 57 | 2.54 | -1.65 | 1.71 | 2.49 | -3.08 | -2.94 | -6.08 | -3.25 | -44.90 | -7.25 |
| 6484-R3-2 | CGCTTCGGGATCCTCTCCAACTGCAACCACA | 58 | > 6.42 | nd | > 2.40 | > 4.37 | nd | nd | nd | nd | nd | nd |
| 6490-R4-1 | CCCATCCCCCATATGACGCTTCCCCCTCCTAACCTCAC CACCCCCAGCA | 59 | 3.28 | 1.04 | 1.30 | 2.00 | -4.20 | -3.75 | -5.00 | -2.07 | -7.58 | -2.49 |
| 6496-R3-1 | CCCCTCCCCCACCCACCACTTCCCCTAGAGTCC | 60 | 14.40 | 1.47 | 1.69 | 4.18 | -3.34 | -2.65 | -2.54 | -2.65 | -5.20 | -1.59 |
| 6584-L1-1 | TCGGCCCTGCCTCCTCCTCCT | 61 | 8.46 | 1.93 | 1.09 | 2.11 | (nd) < -2.02 | (nd) < -2.02 | (nd) < -2.02 | (nd) < -2.02 | (nd) < -2.02 | -1.50 |
| 6602-R3-2 | AGAGCCCCAGTGGAAATCTCTCCTCCAAATCCAT | 62 | > 4.95 | nd | nd | > 1.86 | nd | nd | nd | nd | nd | nd |
| 6642-R3-1 | CACGTCCTCCCCTCCCCTCGAGGTGTCACACA | 63 | 5.93 | 1.20 | (nd) < -5.26 | 2.84 | (nd) < -5.26 | -2.73 | -3.47 | -1.15 | (nd) < -5.26 | -2.02 |
| 6681-R2-1 | CCTGTTTTCTCCCCTCTCTCTCTGCCCCTCC | 64 | 6.76 | 2.18 | 1.13 | 3.85 | (nd) < -6.34 | -1.90 | -2.74 | -1.12 | (nd) <-6.34 | -1.77 |
| 6683-R3-1 | AAAATAAACTCTTCCTGCTCAAG | 65 | > 10.55 | > 1.57 | nd | > 4.34 | nd | nd | nd | nd | nd | nd |
| 6752-R1-1 | CCCTCCTTTCCCCACCTCAGT | 66 | 14.74 | 1.34 | (nd) < -1.39 | 4.34 | (nd) < -1.39 | (nd) < -1.39 | (nd) < -1.39 | (nd) < -1.39 | (nd) < -1.39 | (nd) < -1.39 |
| 6795-R4-1 | CTTCCCGAGGCCACATGCTTCTTTATATCCCCATA | 67 | > 11.94 | nd | nd | > 1.62 | nd | > 1.60 | nd | nd | nd | nd |
| 6803-R3-1 | GCTCCCTCTCTGGTTGGACCTCACCCAAAGAT | 68 | 19.16 | 1.39 | 1.71 | 4.21 | (nd) < -4.36 | -1.08 | -3.03 | 1.87 | (nd) < -4.36 | -1.90 |
| 6839-L3-1 | GCCCGCTGGGCCCTGCCACCCCCACCCCT | 69 | 1.71 | 1.71 | 1.97 | 1.77 | (nd) < -6.04 | -3.04 | -3.58 | -2.14 | (nd) < -6.04 | -2.36 |
| 6880-L3-2 | ACCTCCCCCGCGAAGACATCCACATTCTGCA | 70 | 7.16 | 1.64 | 1.21 | 3.97 | -4.57 | -2.33 | -3.69 | 1.35 | -12.07 | -1.50 |
| 6906-L3-1 | GTGTCTTCTCCCCAACCAGCCAGCTCTCCTGG | 71 | > 7.07 | nd | nd | > 2.10 | nd | nd | nd | nd | nd | nd |
| 6930-R3-1 | ATTAATCCTTCTCTCCCCTCTG | 72 | 34.08 | 2.60 | 2.04 | 9.78 | (nd) < -1.68 | (nd) < -1.68 | (nd) < -1.68 | 2.54 | (nd) < -1.68 | 1.50 |
| 6984-R4-1 | CCCCCTGCCCAAGCATTTGCTTGGGCACCAAAGTCCCT GCAA | 73 | 17.80 | 2.11 | 2.35 | 4.74 | (nd) < -1.93 | (nd) < -1.93 | (nd) < -1.93 | 1.69 | (nd) < -1.93 | (nd) < -1.93 |
| 7026-L3-2 | CCTGATCGAAAACCTCACCCACCAGATCCGGG | 74 | 4.22 | 2.58 | (nd) < -1.59 | 1.21 | (nd) < -1.59 | (nd) < -1.59 | -1.35 | (nd) < -1.59 | (nd) < -1.59 | (nd) < -1.59 |
| 7061-R3-1 | ATGGAAACCCCACCCTTCCC | 75 | 3.76 | 2.16 | -1.23 | -1.09 | -4.11 | -2.69 | -1.66 | -1.31 | -9.30 | -1.73 |
| 7066-R4-1 | CAAGGCCCGTAGCCTGAAAAAAGATGCCCCCACCAGC CCTGCC | 76 | 2.80 | 1.32 | 1.45 | 1.90 | (nd) < -7.42 | -4.34 | -4.43 | -3.00 | (nd) < -7.42 | -3.99 |
| 7126-L3-1 | GCACACCCGCTCTCCGGCCCGCGCCCCTG | 77 | 4.39 | 2.59 | 1.54 | 1.19 | -3.23 | -2.33 | -3.21 | -1.99 | -3.87 | -2.12 |
| 7182-L4-1 | TCTGGGTAACTAGCCGTTTCCGTCACCTTCCCCTGCCC CC | 78 | 8.66 | 1.60 | (nd) < -7.09 | 3.08 | (nd) < -7.09 | -2.00 | -2.46 | -1.11 | -1.39 | -1.82 |
| 7192-R4-1 | GCAAAGCACTTCCCCCTCTAAGTCTGCCTGGGCTCTTG GCAC | 79 | 5.65 | 1.06 | 1.60 | 3.31 | (nd) < -2.01 | (nd) < -2.01 | -2.07 | 1.24 | (nd) < -2.01 | -1.76 |
| 7292-L3-2 | TAAATAGCTTCTGAACCTCCCTGCATTCTAATTGC | 80 | 6.77 | 1.34 | 1.45 | 3.96 | (nd) < -1.55 | (nd) < -1.55 | (nd) < -1.55 | 1.41 | (nd) < -1.55 | (nd) < -1.55 |
| 7352-R3-2 | GCCCCTGCCAGAATCCTCTAACAGCTCTAATTGG | 81 | 2.28 | 2.91 | (nd) < -5.74 | 1.79 | (nd) < -5.74 | -2.74 | -3.58 | -1.97 | (nd) < -5.74 | -2.31 |
| 7356-L2-1 | ACCGCGACATAGCCTCGCCCCC | 82 | 5.16 | 2.76 | 1.18 | 2.46 | (nd) < -3.81 | -1.64 | -3.10 | -1.59 | (nd) < -3.81 | -1.82 |
| 7356-R2-1 | GAAGCTCCGCGGCGACGTCCCGTTACTCC | 83 | > 15.13 | nd | nd | > 1.62 | nd | nd | nd | nd | nd | nd |
| 7367-L1-1 | AGGGTTAGAGCTGCCCCCTCTGGGGACCG | 84 | 2.18 | -1.94 | -1.30 | 1.62 | (nd) < -3.55 | (nd) < -3.55 | -3.23 | (nd) < -3.55 | (nd) < -3.55 | -1.80 |
| 7384-R3-1 | CTCGCAAAGGATCTCCTTCATCCCTCCCCA | 85 | 6.05 | 1.05 | 2.67 | 3.02 | (nd) < -2.34 | -1.86 | (nd) < -2.34 | 1.20 | (nd) < -2.34 | -1.32 |
| 7411-R3-2 | AGTCCCCTGCCTCATCTGCCACCCCTAATGAC | 86 | 2.46 | 2.81 | -1.22 | 1.74 | (nd) < -5.03 | -2.99 | -3.82 | -1.84 | (nd) < -5.03 | -2.12 |
| 7421-R2-1 | TAAAGAGACTTCCTCCACTGCCAGAGATCT | 87 | 3.81 | (nd) < -1.11 | (nd) <-1.11 | 3.90 | (nd) < -1.11 | (nd) <-1.11 | (nd) < -1.11 | (nd) <-1.11 | (nd) < -1.11 | (nd) <-1.11 |
| 7426-L3-1 | TGGGAGACGAACACCTCCTGCTGTGCTTG | 88 | > 6.19 | nd | nd | > 3.67 | nd | nd | nd | > 2.02 | nd | nd |
| 7569-L3-1 | TCAGGCCACAAAGCTACCCCCAAGACAGGCC | 89 | 1.43 | 1.15 | -1.03 | -1.03 | (nd) <-3.88 | (nd) <-3.88 | (nd) <-3.88 | (nd) <-3.88 | (nd) <-3.88 | (nd) <-3.88 |
| 7571-L1-1 | AGGGCTCCCCCACCCCTAAG | 90 | 2.27 | -1.15 | -1.09 | 1.24 | -8.82 | -5.67 | -5.94 | -2.92 | -13.36 | -3.96 |
| 7572-R2-1 | ATCACCCTTCCCCCTCCCAAATAAAG | 91 | 11.01 | 1.19 | 1.42 | 4.86 | (nd) < -4.50 | -2.48 | -3.89 | -1.79 | (nd) < -4.50 | -1.63 |
| 7578-L3-1 | CGCAGTGCACACCCTGAGCTACAGCCCCTC | 92 | -1.03 | -1.06 | -2.38 | -1.25 | -14.04 | -6.24 | -17.14 | -2.93 | -6.73 | -2.44 |
| 7660-L2-1 | CCCGGCCTCCGCCTGGCCCGAGCGATAA | 93 | 3.94 | 2.11 | 2.13 | 1.41 | (nd) < -3.09 | (nd) < -3.09 | -3.26 | -1.11 | (nd) < -3.09 | -1.77 |
| 7702-L2-1 | CCCAGAGAACCGGAATTCCTCCCCGCCCC | 94 | 6.99 | 2.64 | 3.18 | 3.26 | (nd) < -3.36 | -1.95 | -2.34 | -1.20 | (nd) < -3.36 | -1.44 |
| 7726-R3-2 | CATCCCTCTCCAGAAGAGGAGAAGAGGAAACA | 95 | 4.39 | -1.31 | (nd) < -3.13 | 2.45 | (nd) < -3.13 | 1.32 | -1.96 | -1.06 | (nd) < -3.13 | (nd) < -3.13 |
| 7764-R3-2 | CCCTCTCTGCCTCTCTCATCACCAATAACAGAC | 96 | 9.88 | 1.35 | 1.56 | 3.58 | (nd) < -1.65 | -1.06 | (nd) < -1.65 | 1.15 | (nd) < -1.65 | -1.46 |
| 8004-R3-2 | GGAACTGCTTCTCCTTGCTCCAGTCATTGAAG | 97 | 6.92 | 1.21 | -2.65 | 3.26 | (nd) < -9.22 | -3.19 | -4.22 | (nd) < -9.22 | (nd) < -9.22 | -2.06 |
| 8016-L3-1 | TCAGCGCAACAAGCCCCGCAGTCACCCCTCT | 98 | 4.04 | 2.25 | 1.06 | 1.40 | (nd) < -4.39 | -2.38 | -3.04 | -1.84 | (nd) < -4.39 | -2.39 |
| 8077-R3-1 | CCATTCCCCACCCTCAGGTAGTAAAAATA | 99 | 4.61 | 2.89 | (nd) < -1.93 | 1.35 | (nd) < -1.93 | (nd) < -1.93 | -1.28 | 1.17 | (nd) < -1.93 | -1.04 |
| 8169-L3-1 | AACAGAAATGATTATTTACCTCCCCACATG | 100 | 8.55 | 1.42 | 1.85 | 5.76 | (nd) < -1.59 | (nd) < -1.59 | (nd) < -1.59 | 1.97 | (nd) < -1.59 | -1.39 |
| 8250-R3-1 | CAGCCGCCTCTCCCTCAGCGTTAA | 101 | 7.73 | 1.48 | 1.79 | 2.25 | -2.24 | -3.20 | -5.50 | 1.35 | -1.23 | -1.26 |
| 8263-R3-1 | GATTAAAAACAAGAATCTATCTTCCCCCAGT | 102 | > 4.54 | nd | nd | > 1.78 | nd | nd | nd | nd | nd | nd |
| 8281-L3-1 | AGCCCCTCCCCAGCTGCAGCTGAGGGCTGG | 103 | 2.72 | -1.03 | 1.97 | 3.10 | -3.22 | -2.88 | -5.10 | -2.09 | -19.58 | -4.66 |
| 8316-R3-1 | ATCAGGGTATCCTCTCCCCA | 104 | > 39.96 | nd | nd | > 7.56 | nd | nd | nd | > 2.43 | nd | nd |
| 8394-L3-1 | CCCCCGCCCTGCCCATCTCCGACT | 105 | 3.70 | 2.46 | 1.62 | 2.36 | -4.53 | -3.13 | -3.91 | -2.65 | -14.49 | -2.52 |
| 8433-L3-1 | AAATGGCTCCTTTCCCCTTTCCCTCCACCG | 106 | 11.25 | 1.11 | 1.90 | 4.23 | (nd) < -2.16 | -1.64 | -2.20 | 1.18 | (nd) < -2.16 | -1.36 |
| 8564-L3-1 | CCCTTCACCCCAGTTGCCAAACA | 107 | > 12.45 | nd | nd | > 3.14 | nd | nd | nd | nd | nd | nd |
| 8587-R2-2 | CCCGGCGCCCCTCGCCGGCTCCAAACTTTCCCCAA | 108 | 3.02 | 1.99 | 1.87 | 2.01 | (nd) < -3.01 | (nd) < -3.01 | -3.33 | (nd) < -3.01 | (nd) < -3.01 | -1.53 |
| 8724-R3-1 | GCCAAGCTTGGAACCTCTCCCTGCCAGCATCAC | 109 | 16.37 | 1.40 | 1.47 | 5.34 | (nd) < -1.51 | (nd) < -1.51 | (nd) < -1.51 | 1.87 | (nd) < -1.51 | -1.00 |
| 8731-R3-1 | TCATTCATGCCCCATCCTGCCAG | 110 | 3.79 | 3.51 | (nd) < -1.34 | 3.09 | (nd) < -1.34 | (nd) < -1.34 | (nd) < -1.34 | (nd) < -1.34 | (nd) < -1.34 | (nd) < -1.34 |
| 8808-R3-1 | CCAAAGACCCCTTTCTCCCAGCCTGTTTCTGCAA | 111 | 15.38 | (nd) < -1.38 | 1.74 | 3.85 | (nd) < -1.38 | (nd) < -1.38 | (nd) < -1.38 | 1.65 | (nd) < -1.38 | -1.15 |
| 8898-R3-1 | CGGACGCCCGCTCCCGCCA | 112 | 4.61 | 2.99 | 2.21 | 2.39 | -3.44 | -2.32 | -3.50 | -1.73 | -8.22 | -2.57 |
| 9021-L4-1 | AAACAAACACCCAAGCTCCCCACACCATC | 113 | 4.79 | 1.54 | -1.06 | 1.48 | (nd) < -3.02 | (nd) < -3.02 | -3.04 | -1.15 | (nd) < -3.02 | (nd) < -3.02 |
| 9053-R3-1 | TTCTTGCCCTCCAATCCCCGGGCTCCACCAGCC | 114 | 2.98 | 1.64 | -1.13 | 1.16 | -3.72 | -2.19 | -3.05 | -1.88 | -8.27 | -2.12 |
| 9068-R2-1 | CTGCCCTCCCTCTTGATCAAGACTGCTCTCCTAA | 115 | 3.42 | -1.69 | 2.79 | 2.64 | -2.57 | -3.10 | -11.82 | -1.57 | -40.43 | -4.16 |
| 9087-L4-1 | GGAAAAGAAACCCTCCCAGTCCATTCCCTTCCT | 116 | 2.03 | -2.72 | 1.81 | 1.31 | -2.99 | -3.69 | -4.84 | -1.39 | -9.51 | -2.38 |
| 9217-L3-1 | ACGATCCCCGCCGTGACTAAAGCCAACAGTGGA | 117 | 3.28 | 2.48 | 1.88 | 1.93 | (nd) < -4.55 | -2.32 | -3.01 | -1.46 | (nd) < -4.55 | -1.91 |
| 9245-R2-1 | AACCTCTCATTAGCCAGCCACTCGCTCCCAAG | 118 | 5.25 | 4.01 | 1.82 | 1.83 | (nd) < -1.69 | (nd) < -1.69 | (nd) < -1.69 | (nd) < -1.69 | (nd) < -1.69 | (nd) < -1.69 |
| 9287-L4-1 | GATATTCAGAGCCCTCCCCAGCCCACACATGC | 119 | 4.16 | -1.56 | 2.12 | 2.52 | -2.91 | -2.86 | -6.49 | -1.43 | -32.89 | -4.05 |
| 9347-L2-1 | GCCCAATATGCATTTTACATTTTAACAAAGA | 120 | > 3.17 | nd | nd | > 1.54 | nd | nd | nd | nd | nd | nd |
| 9349-R3-1 | GTGATGCAGAGGACTTCCTGCTCCAGGTCTC | 121 | 22.69 | 1.06 | (nd) <-1.13 | 7.70 | (nd) < -1.13 | (nd) <-1.13 | (nd) < -1.13 | (nd) <-1.13 | (nd) < -1.13 | (nd) < -1.13 |
| 9387-R2-2 | TCCATCCTTGCCGTCGCCTTCATCTCAAAGCCATC | 122 | 2.28 | 1.28 | 2.25 | -1.06 | -2.17 | -3.48 | -4.42 | -2.26 | -4.76 | -1.91 |
| 9391-R3-1 | CAGCTGCCAGGGAGACATAGAAATTAAAAACAA | 123 | -1.34 | 1.31 | 1.27 | -1.69 | -1.57 | -1.49 | -2.45 | nd | nd | -1.82 |
| 9507-L3-2 | GTCTCCCTCATCCATCATCC | 124 | > 8.07 | nd | nd | > 3.35 | nd | nd | nd | nd | nd | nd |
| 9564-R1-1 | GCCGCCCGCCGGGCACCGGGCC | 125 | 1.64 | 1.84 | 2.11 | 1.01 | -2.74 | -3.42 | -4.08 | -2.21 | -7.78 | -2.50 |
| 9594-R2-1 | CTTAGACTTCCTTCCCACTCCCTGCAT | 126 | 11.20 | 1.01 | (nd) < -2.11 | 1.95 | (nd) < -2.11 | (nd) <-2.11 | (nd) < -2.11 | 2.21 | (nd) < -2.11 | -1.03 |
| 9656-R3-1 | GCCCTTAAAGTACATACTGTGGAGATTAATGCT | 127 | > 5.47 | nd | nd | > 2.33 | nd | nd | nd | nd | nd | nd |
| 9691-L4-1 | AATCATCCATTTCATCCGCATCTCCCTCTTGGCCCCTTG C | 128 | 5.99 | 1.18 | 1.32 | 1.99 | (nd) < -4.24 | -1.97 | -2.87 | -1.57 | (nd) < -4.24 | -3.39 |
| 9733-L3-1 | AAGGCTGTCCCTCACCAGACTTCCCCACCCCT | 129 | 7.61 | 3.56 | (nd) < -1.48 | 2.44 | (nd) < -1.48 | (nd) < -1.48 | 1.07 | 1.69 | (nd) < -1.48 | 1.33 |
| 9774-R2-2 | CCGCCCCCTCACCGCCTCCTGCTCCCATCAGGC | 130 | 3.72 | 1.83 | 2.45 | 2.54 | -2.98 | -2.83 | -4.46 | -1.84 | -12.28 | -2.00 |
| 9816-R2-1 | CTGGCCCTTTAAGAGCCTCTCCGCGCGCTGCCG | 131 | 2.42 | 2.46 | 2.03 | 2.04 | -2.71 | -2.17 | -2.86 | -1.67 | -4.18 | -2.08 |
| 9840-L3-2 | TTCAGGTTTTTATAAATCAGGATGTCAACAAAT | 132 | -1.39 | 1.32 | 1.13 | -2.15 | -1.71 | -1.66 | -2.89 | nd | nd | -1.70 |
| 10010-R2-2 | CCCGGCGCCCCTCGCCGGCTCCAAACTTTCCCCAA | 133 | 4.99 | 2.90 | 2.53 | 3.23 | (nd) < -1.75 | (nd) < -1.75 | -1.85 | (nd) < -1.75 | (nd) < -1.75 | -1.31 |
| 10030-R3-1 | AACTCAGCTGCCTTCCGCC | 134 | > 6.94 | > 1.63 | > 3.55 | > 2.08 | nd | nd | nd | nd | nd | nd |
| 10138-L2-1 | AGCCTGCTCCGCTCTCCCCTCCCACCAGAAAAAGT | 135 | 10.06 | 2.02 | 1.51 | 3.69 | -3.13 | -2.11 | -3.65 | 1.01 | -7.30 | -1.76 |
| 10145-L2-1 | CTTTGCTCTCTCTTCTGTTATCTGGACC | 136 | > 5.20 | nd | nd | > 1.95 | nd | nd | nd | nd | nd | nd |
| 10175-L1-1 | CCTGCTCCTAGCAACCAGGAGCCACAA | 137 | > 7.31 | nd | nd | > 4.44 | nd | nd | nd | nd | nd | nd |
| 10209-L3-1 | AGAAAATAAGTTAGCTATGTAACAAATTGA | 138 | -1.56 | 1.29 | -1.09 | -2.17 | -2.06 | -1.65 | -3.13 | nd | nd | -1.86 |
| 10231-L3-1 | GTGCAGCAGCCCGCGCCAGCCTCCGCAGCCGCC | 139 | 1.76 | 1.50 | 3.56 | -1.02 | -1.97 | -3.47 | -5.87 | -6.71 | -3.38 | -2.65 |
| 10231-R3-1 | TGAACTTTAGCTGGGCCGCCGCCTGTCAGC | 140 | 1.15 | 1.48 | 2.17 | -1.45 | -2.34 | -4.21 | -5.32 | -3.26 | -4.14 | -2.20 |
| 10242-R3-1 | GGAAGAAGCCCTTCCGCTTCCACCCCGAACAC | 141 | 5.35 | 2.89 | 1.21 | 3.14 | -2.60 | -1.56 | -3.46 | -1.09 | -6.84 | -2.88 |
| 10333-L3-1 | TGTGCCCTGCCCACCCCCTCCCCTGCCCCG | 142 | 5.80 | 1.70 | 1.74 | 2.93 | -3.43 | -2.75 | -4.34 | -2.25 | -9.80 | -2.32 |
| 10335-L3-1 | CACTCCCCTCCTTTTTAATTAGAAAGCACTAAGA | 143 | 10.97 | 1.56 | 2.36 | 6.06 | (nd) < -2.08 | (nd) < -2.08 | (nd) < -2.08 | (nd) < -2.08 | (nd) < -2.08 | -1.22 |
| 10342-R2-2 | CCCGCCGCCGGAGCATCTCGAAGTTAATTAAA | 144 | 1.75 | 2.08 | 2.13 | -1.05 | -2.55 | -2.86 | -2.31 | -2.36 | -10.37 | -1.85 |
| 10366-R3-2 | AAACACCACCCACGCTCTTGCTACAAGACCCACAT | 145 | 2.46 | 1.21 | -1.12 | -1.11 | (nd) < -3.19 | -2.45 | -2.94 | (nd) < -3.19 | (nd) < -3.19 | (nd) < -3.19 |
| 10374-R3-2 | GACACCGCCCGCTACTTTGTTAATGAAAAGCCCCC | 146 | 2.59 | 2.62 | 2.48 | 1.28 | -2.47 | -2.10 | -4.29 | nd | nd | -1.97 |
| 10533-R3-1 | GCCCCTTCTTTATATTGCCAAGA | 147 | 4.36 | (nd) | (nd) | 2.33 | (nd) | (nd) | (nd) | (nd) | (nd) | (nd) |
| | | | | < -1.58 | < -1.58 | | < -1.58 | < -1.58 | < -1.58 | < -1.58 | < -1.58 | < -1.58 |
| 11370-L4-1 | CCTCCGCCCCCACACTGCATCCTTGCCCAGTTTGGCTG CCATCAGTATTGTCCCCTGAGAACTGGAC | 148 | 7.22 | 1.56 | (nd) < -6.13 | 2.49 | -2.48 | -2.20 | -6.13 | -1.28 | -3.12 | -1.10 |
| 12184-L4-1 | GACCTCAGCGTGCCCCCTTTCAACCACAGACGAATATT GTGTACAA | 149 | 2.50 | 1.10 | -1.39 | 1.69 | (nd) < -4.07 | -2.20 | -3.80 | -1.92 | (nd) < -4.07 | -1.97 |
| 12223-L4-1 | CCCAGAAGACATCAGACAGAGTTGTTTCTTCTCCCTCTA | 150 | 1.99 | 1.06 | (nd) < -4.89 | 1.27 | (nd) < -4.89 | 1.25 | -1.49 | 1.03 | -2.26 | -3.14 |
| 4315_C-L4-1 | GCAGCCCCTCCTCCGAGAGGTTGGGGGTCGCGGCCG CCCGGCCCTCCCGGTCCCCTCCCC | 151 | -2.79 | 1.91 | 1.97 | 2.72 | -3.38 | -2.74 | -3.87 | -1.53 | -9.33 | -7.93 |
| 4315_D-R4-1 | GGAAAGTCAGCCCCCAGCGCCCCCCGGAGTTCTTGG | 152 | 4.01 | 1.41 | 1.66 | 2.02 | -4.14 | -2.64 | -7.06 | -2.32 | -9.62 | -2.60 |
| 4315_E-R4-1 | CCCCCACCAAACCTATTCCCGCATCCTCCCCGGCTCTG G | 153 | 7.07 | 1.72 | 1.30 | 2.47 | -4.50 | -2.77 | -5.04 | -2.24 | -10.19 | -2.01 |
| 4315_F-R4-1 | AACCCGGGCTCCCCCACCCGCTCCCTGAGC | 154 | 3.58 | 1.70 | 1.23 | 1.44 | -5.04 | -3.19 | -3.15 | -2.24 | -20.64 | -2.42 |
| 4315_I-L4-1 | ACACCTCTGCGCCCCTCAGGCGCCCTGGCCTCGGCG CCCCGCCCGTCCCAG | 155 | 5.01 | 2.26 | 2.05 | 2.48 | -3.25 | -2.26 | -2.87 | -1.61 | -6.36 | -2.28 |
| 4315_K-L4-1 | TCCCAGGGGGCCCTGAACTTGTCAAATCCTCGCCATCC TCCACCCCCAGCCCCGG | 156 | 4.24 | 1.87 | 2.15 | 1.72 | -3.72 | -2.41 | -4.85 | -1.91 | -6.99 | -1.83 |
| 10010_B-L4-1 | GCCGAGCCCCCGCCCCCGCCGGGATGCTGCCCTCCG GAAGGAGGGGCGCTGCCC | 157 | 2.68 | 2.95 | 1.31 | 1.80 | -4.25 | -3.10 | -3.43 | -2.54 | -5.76 | -2.39 |
| 10010_D-L4-1 | TGGCGCCCTCCCCGCCCGGGGCTCAGCCTCTCACCT G | 158 | 4.06 | 1.62 | 1.93 | 2.59 | -3.56 | -2.99 | -4.26 | -1.54 | -15.24 | -2.37 |
| 7356_AR4-1 | CAGAGCCCGCTCTCGCGACCGACCTGCCGCCGACCGC CACAG | 159 | 1.71 | 1.65 | 3.03 | -1.13 | -1.99 | -3.31 | -4.25 | -3.28 | -3.88 | -2.82 |
| 12722-L4-1 | AATACGGACAAGCCCCACTCCCTCATTAGCATAAAAAA CAAAGTACTTCCGACCTCCCCGCCCGCCCGC | 160 | 3.15 | 2.05 | 2.31 | 2.04 | -3.42 | -2.39 | -3.53 | -2.00 | -5.91 | -2.63 |
| 999999-R4-1 | CCCCTTGTCACCCCCAGCCCCTTCCTGGCCAGGACCC CAGCGAGGCCCAGAGAA | 161 | 10.71 | 2.08 | 1.97 | 3.33 | (nd) < -2.88 | -1.64 | -2.59 | 2.58 | (nd) < -2.88 | -1.53 |
| 999997-R4-1 | TCCTCACTGGGCCCCACCAAAACTGTGCCACCCCCTCA AGCCCCCAGGAGCTTCCTTAAC | 162 | 6.84 | -1.42 | 1.59 | 2.77 | -3.46 | -2.91 | -4.06 | -1.00 | -5.37 | -1.68 |
| 8433_B-L4-1 | CATATTTTTGTGTTGCTGAGTATTTGGGGTTTGCTCGCC CGT | 163 | > 5.03 | > 3.56 | > 2.45 | > 2.44 | nd | nd | nd | nd | nd | nd |
| 8433_C-R4-1 | AAACCAAAAAAAAAAATTAAAAGCGACGAAAATGCAA TTGTGTGCCTTCTCCCTCC | 164 | 24.29 | 1.73 | 1.87 | 5.70 | (nd) < -1.45 | (nd) < -1.45 | (nd) < -1.45 | 1.95 | (nd) < -1.45 | 1.13 |
| 8433_D-R4-1 | CCCGAGCCCGGCGCCCTGTGTTGTGCTCCGCTCTCCG GGAAATGCCATCACTAAT | 165 | 8.52 | 2.31 | 1.63 | 2.00 | (nd) < -4.10 | -1.76 | -3.76 | -1.33 | (nd) < -4.10 | -2.68 |
| let-7a | AACTATACAACCTACTACCTCA | 166 | -5.65 | -1.33 | 1.33 | -2.81 | 1.20 | -1.63 | -1.75 | -1.18 | 1.20 | -3.59 |
| let-7b | AACCACACAACCTACTACCTCA | 167 | -2.30 | -1.30 | 1.41 | -2.86 | 1.19 | -1.79 | -2.26 | 1.05 | 1.51 | -4.67 |
| let-7c | AACCATACAACCTACTACCTCA | 168 | -2.52 | -1.34 | 1.39 | -2.93 | 1.22 | -1.77 | -1.97 | 1.17 | 1.38 | -3.51 |
| let-7d | AACTATGCAACCTACTACCTCT | 169 | -2.41 | -1.35 | 1.23 | -2.86 | -1.03 | -1.69 | -2.05 | 1.31 | 1.64 | -3.27 |
| let-7e | AACTATACAACCTCCTACCTCA | 170 | -2.38 | -1.32 | -1.20 | -2.76 | -1.49 | -1.83 | -5.03 | 1.11 | 1.34 | -8.91 |
| let-7f | AACTATACAATCTACTACCTCA | 171 | -3.05 | -1.28 | 1.19 | -3.04 | 1.01 | -1.73 | -4.92 | -1.20 | 1.06 | -3.85 |
| let-7g | AACTGTACAAACTACTACCTCA | 172 | -2.34 | -1.12 | 1.13 | -2.73 | -3.04 | -1.16 | -1.34 | 1.28 | 2.07 | -3.06 |
| let-7i | AACAGCACAAACTACTACCTCA | 173 | -1.56 | 1.86 | (nd) < -3.56 | -1.68 | 1.05 | 2.20 | -3.56 | 1.63 | 3.60 | -1.90 |
| miR-100 | CACAAGTTCGGATCTACGGGTT | 174 | -1.91 | -1.56 | 1.24 | -3.21 | -1.72 | -2.50 | -2.63 | 1.39 | 2.17 | -5.22 |
| miR-1224-5p | CCACCTCCCGAGTCCTCAC | 175 | 5.56 | 1.51 | 1.89 | 1.57 | (nd) < -1.14 | (nd) < -1.14 | -1.11 | (nd) < -1.14 | (nd) < -1.14 | (nd) < -1.14 |
| miR-1225-5p | CCCCCCACTGGGCCGTACCCAC | 176 | 8.21 | -1.18 | 2.95 | 4.25 | (nd) < -2.30 | (nd) < -2.30 | -2.35 | (nd) < -2.30 | (nd) < -2.30 | (nd) < -2.30 |
| miR-1228* | CACACACCTGCCCCCGCCCAC | 177 | 3.27 | 3.43 | 1.06 | 1.96 | -6.69 | -3.06 | -3.39 | -1.87 | -12.52 | -2.10 |
| miR-125a-5p | TCACAGGTTAAAGGGTCTCAGGGA | 178 | (nd) < -10.19 | -1.52 | 1.28 | -3.29 | -2.87 | -1.81 | -6.80 | -1.99 | -1.10 | -6.52 |
| miR-125b | TCACAAGTTAGGGTCTCAGGGA | 179 | -3.83 | -1.52 | 1.33 | -3.15 | -1.08 | -1.77 | -2.46 | -1.78 | 1.01 | -5.55 |
| miR-126 | CGCATTATTACTCACGGTACGA | 180 | -2.39 | -1.40 | 5.58 | -2.14 | 1.33 | -1.45 | -1.81 | 1.07 | 1.71 | -1.42 |
| miR-135a* | CGCCACGGCTCCAATCCCTATA | 181 | > 3.61 | nd | nd | 2.49 | nd | nd | nd | nd | nd | nd |
| miR-142-3p | TCCATAAAGTAGGAAACACTACA | 182 | -2.17 | 1.15 | 1.09 | -2.54 | -4.28 | 1.14 | 1.45 | -1.22 | 1.18 | -3.10 |
| miR-145 | AGGGATTCCTGGGAAAACTGGAC | 183 | -4.94 | -1.08 | 2.41 | -2.67 | 1.13 | 1.01 | -1.07 | -2.03 | 1.31 | -3.13 |
| miR-146b-5p | AGCCTATGGAATTCAGTTCTCA | 184 | (nd) < -1.89 | 1.12 | 2.44 | < -1.89 | < -1.89 | 2.71 | 1.07 | 1.30 | 2.48 | -1.21 |
| miR-149* | GCACAGCCCCCGTCCCTCCCT | 185 | 3.46 | 3.38 | -1.02 | 2.16 | -8.57 | -3.25 | -6.43 | -2.34 | -21.89 | -2.17 |
| miR-150* | CTGTCCCCCAGGCCTGTACCAG | 186 | > 4.16 | nd | nd | > 1.76 | nd | nd | nd | nd | nd | nd |
| miR-155 | ACCCCTATCACGATTAGCATTAA | 187 | nd | nd | nd | nd | nd | > 1.83 | > 1.07 | nd | nd | nd |
| miR-16 | CGCCAATATTTACGTGCTGCTA | 188 | -4.17 | -1.04 | (nd) < - 10.19 | -3.62 | -1.53 | -1.05 | -1.04 | 1.73 | 2.03 | -3.14 |
| miR-181c | ACTCACCGACAGGTTGAATGTT | 189 | (nd) < -3.23 | -1.73 | 1.58 | -2.37 | -1.79 | 1.13 | -2.25 | -1.16 | 1.07 | -3.23 |
| miR-198 | GAACCTATCTCCCCTCTGGACC | 190 | > 33.49 | > 2.21 | nd | > 8,74 | nd | nd | nd | nd | nd | nd |
| miR-199a-3p | TAACCAATGTGCAGACTACTGT | 191 | -3.52 | 1.40 | 1.43 | -1.94 | 1.72 | 1.56 | 1.33 | 1.59 | 2.97 | -4.20 |
| miR-19b | TCAGTTTTGCATGGATTTGCACA | 192 | -1.07 | -1.01 | 1.41 | -1.94 | (nd) < -2.18 | -1.10 | 1.48 | 1.28 | 4.82 | (nd) < -2.18 |
| miR-200b | TCATCATTACCAGGCAGTATTA | 193 | 1.18 | 1.01 | (nd) < -5.45 | 1.43 | -1.58 | -1.56 | -1.38 | 1.72 | 2.60 | -1.45 |
| miR-200c | TCCATCATTACCCGGCAGTATTA | 194 | 1.45 | 1.11 | -1.19 | 1.72 | -1.88 | -1.84 | -1.47 | 1.92 | 2.85 | -1.10 |
| miR-205 | CAGACTCCGGTGGAATGAAGGA | 195 | > 11.24 | > 24.11 | nd | nd | nd | nd | nd | > 3.48 | > 9.48 | nd |
| miR-21 | TCAACATCAGTCTGATAAGCTA | 196 | -2.52 | 1.77 | -1.51 | 1.45 | 1.41 | 3.51 | 2.27 | 3.43 | 5.38 | 1.25 |
| miR-23a | GGAAATCCCTGGCAATGTGAT | 197 | 1.38 | 2.90 | (nd) < -4.40 | 1.37 | (nd) < -4.4 | 2.28 | (nd) < -4.4 | 2.38 | 7.76 | -2.09 |
| miR-23a* | AAATCCCATCCCCAGGAACCCC | 198 | > 4.54 | nd | nd | > 1.79 | nd | nd | nd | nd | nd | nd |
| miR-23b | GGTAATCCCTGGCAATGTGAT | 199 | -1.43 | 1.45 | (nd) < -9.07 | -1.34 | 1.26 | 1.24 | -2.02 | 1.27 | 4.35 | -1.98 |
| miR-24 | CTGTTCCTGCTGAACTGAGCCA | 200 | -2.08 | 1.26 | 2.49 | -1.46 | 1.08 | 1.22 | -1.17 | 1.09 | 2.86 | -2.76 |
| miR-25* | CAATTGCCCAAGTCTCCGCCT | 201 | > 4.17 | nd | nd | > 1.38 | nd | nd | nd | nd | nd | nd |
| miR-26a | AGCCTATCCTGGATTACTTGAA | 202 | -2.98 | -1.73 | 2.04 | -3.66 | 1.04 | 1.00 | -1.33 | 1.07 | 1.67 | -2.29 |
| miR-26b | ACCTATCCTGAATTACTTGAA | 203 | -2.84 | -1.67 | 1.20 | -3.56 | -1.49 | -1.16 | -1.18 | 1.13 | 1.74 | -2.23 |
| miR-27a | GCGGAACTTAGCCACTGTGAA | 204 | -1.27 | 1.78 | 2.19 | -1.78 | -1.02 | 1.44 | 1.07 | 1.73 | 4.25 | -2.26 |
| miR-27b | GCAGAACTTAGCCACTGTGAA | 205 | -1.11 | 2.20 | 2.52 | -1.91 | 1.18 | 1.64 | 1.24 | 2.05 | 6.50 | -1.85 |
| miR-298 | TGGGAGAACCTCCCTGCTTCTGCT | 206 | 24.71 | 2.84 | (nd) < -1.29 | 10.70 | (nd) < -1.29 | (nd) < -1.29 | -1.15 | 2.09 | (nd) < -1.29 | 1.13 |
| miR-29a | TAACCGATTTCAGATGGTGCTA | 207 | -2.68 | 1.00 | 3.18 | 1.50 | -1.90 | 2.13 | 1.41 | 1.23 | 2.18 | -2.96 |
| miR-29b | AACACTGATTTCAAATGGTGCTA | 208 | -5.79 | -1.32 | 1.33 | -1.17 | -3.06 | 1.46 | 1.26 | 1.01 | 1.30 | -5.30 |
| miR-29c* | TAACCGATTTCAAATGGTGCTA | 209 | -4.12 | -1.00 | 3.14 | 1.30 | -3.52 | 2.01 | 1.49 | 1.66 | 2.61 | -4.26 |
| miR-30a | CTTCCAGTCGAGGATGTTTACA | 210 | -4.44 | -3.14 | 3.50 | -3.84 | -1.50 | -2.18 | -3.97 | -7.98 | 2.09 | 1.23 |
| miR-30b | AGCTGAGTGTAGGATGTTTACA | 211 | (nd) < -6.56 | -1.98 | 2.09 | -4.38 | -2.50 | -1.73 | -2.27 | 2.12 | 2.77 | 1.03 |
| miR-30b* | GAAGTAAACATCCACCTCCCAG | 212 | 3.38 | -1.21 | 1.05 | -1.36 | -2.31 | -2.53 | -2.76 | -1.56 | -3.51 | -3.51 |
| miR-30c | GCTGAGAGTGTAGGATGTTTACA | 213 | -4.45 | -2.14 | (nd) <-6.05 | (nd) <-6.05 | (nd) <-6.05 | -1.95 | (nd) <-6.05 | 1.86 | 2.21 | -2.40 |
| miR-30c-1* | GGAGTAAACAACCCTCTCCCAG | 214 | 39.98 | (nd) < -1.58 | 1.57 | 7.84 | (nd) < -1.58 | (nd) < -1.58 | -1.42 | 2.67 | (nd) < -1.58 | (nd) < -1.58 |
| miR-30c-2 | GCTGAGAGTGTAGGATGTTTACA | 215 | -4.45 | -2.14 | (nd) | (nd) | (nd) | -1.95 | (nd) | 1.86 | 2.21 | -2.40 |
| | | | | | <-6.05 | <-6.05 | <-6.05 | | <-6.05 | | | |
| miR-30d | CTTCCAGTCGGGGATGTTTACA | 216 | -6.68 | -2.87 | 3.73 | -3.55 | -1.26 | -2.03 | -4.02 | 1.32 | 3.66 | 1.39 |
| miR-30e | CTTCCAGTCAAGGATGTTTACA | 217 | (nd) < -4.99 | -2.42 | 2.08 | (nd) < -4.99 | (nd) < -4.99 | -2.00 | (nd) < -4.99 | 1.45 | 1.71 | -1.30 |
| miR-320a | TCGCCCTCTCAACCCAGCTTTT | 218 | 4.68 | 1.05 | 2.16 | 2.43 | 1.02 | 1.33 | -1.39 | nd | nd | -1.20 |
| miR-331-3p | TTCTAGGATAGGCCCAGGGGC | 219 | (nd) < -1.21 | (nd) < -1.21 | 2.17 | 1.42 | 1.24 | 1.47 | 1.27 | -1.21 | -1.21 | 1.36 |
| miR-371-5p | AGTGCCCCCACAGTTTGAGT | 220 | > 2.65 | nd | nd | > 1.30 | nd | nd | nd | nd | nd | nd |
| miR-373* | GGAAAGCGCCCCCATTTTGAGT | 221 | 10.21 | 1.72 | 2.16 | 3.92 | (nd) < -3.77 | -1.66 | -3.39 | 1.66 | (nd) < -3.77 | -1.73 |
| miR-375 | TCACGCGAGCCGAACGAACAAA | 222 | nd | nd | nd | nd | nd | nd | nd | nd | nd | > 2.88 |
| miR-423-5p | AAAGTCTCGCTCTCTGCCCCTCA | 223 | 6.96 | 1.49 | 1.63 | 1.87 | (nd) < -6.09 | -1.38 | -1.95 | 1.01 | -1.06 | -1.83 |
| miR-424 | TTCAAAACATGAATTGCTGCTG | 224 | nd | nd | nd | nd | nd | > 2.14 | nd | > 2.99 | > 11.35 | nd |
| miR-483-5p | CTCCCTTCTTTCCTCCCGTCTT | 225 | 24.38 | 1.51 | 1.68 | 5.17 | (nd) < -1.54 | 1.11 | -1.64 | 3.19 | (nd) < -1.54 | -1.03 |
| miR-486-3p | ATCCTGTACTGAGCTGCCCCG | 226 | 3.44 | 2.92 | 2.61 | 1.43 | (nd) < -1.91 | (nd) < -1.91 | -1.85 | 1.05 | (nd) < -1.91 | (nd) < -1.91 |
| miR-491-3p | GTAGAAGGGAATCTTGCATAAG | 227 | -2.17 | 1.13 | 4.21 | -2.65 | (nd) < -3.87 | -1.40 | -3.03 | nd | nd | -3.87 |
| miR-491-5p | CCTCATGGAAGGGTTCCCCACT | 228 | > 2.09 | nd | nd | nd | nd | nd | nd | nd | nd | nd |
| miR-513a-5p | ATGACACCTCCCTGTGAA | 229 | > 6.38 | nd | nd | > 3.28 | nd | nd | nd | nd | nd | nd |
| miR-513b | ATAAATGACACCTCCTTGTGAA | 230 | > 1.98 | nd | nd | > 1.29 | nd | nd | nd | nd | nd | nd |
| miR-516a-5p | GAAAGTGCTTCTTTCCTCGAGAA | 231 | > 23.45 | nd | nd | > 5.80 | nd | > 1.65 | nd | > 2.27 | nd | nd |
| miR-550 | GGGCTCTTACTCCCTCAGGCACT | 232 | > 2.04 | nd | nd | nd | nd | nd | nd | nd | nd | nd |
| miR-557 | AGACAAGGCCCACCCGTGCAAAC | 233 | 4.19 | 1.38 | 2.01 | 1.78 | (nd) < -1.39 | (nd) < -1.39 | -1.15 | 1.57 | (nd) < -1.39 | -1.21 |
| miR-575 | GCTCCTGTCCAACTGGCTC | 234 | > 4.55 | nd | nd | nd | nd | nd | nd | nd | nd | nd |
| miR-612 | AAGGAGCTCAGAAGCCCTGCCCAGC | 235 | 5.20 | 2.50 | -1.18 | 1.81 | (nd) < -5.94 | -2.60 | -2.93 | -1.98 | (nd) < -5.94 | -2.85 |
| miR-614 | CCACCTGGCAAGAACAGGCGTTC | 236 | > 2.61 | nd | nd | nd | nd | nd | nd | nd | nd | nd |
| miR-630 | ACCTTCCCTGGTACAGAATACT | 237 | > 4.81 | nd | nd | > 1.88 | nd | nd | nd | nd | nd | nd |
| miR-637 | ACGCAGAGCCCGAAAGCCCCCAGT | 238 | 1.35 | 5.51 | 2.82 | 7.31 | (nd) < -1.81 | (nd) < -1.81 | (nd) < -1.81 | 1.46 | (and) < - 1.81 | -1.52 |
| miR-638 | AGGCCGCCACCCGCCCGCGATCCCT | 239 | 2.50 | 2.06 | 3.13 | 1.32 | -1.55 | -3.45 | -4.86 | -2.17 | -2.60 | -2.40 |
| miR-658 | ACCAACGGACCTACTTCCCTCCGCC | 240 | 3.65 | 3.18 | 1.67 | 3.41 | (nd) < -3.56 | -1.70 | -1.93 | 1.04 | (nd) < -3.56 | -1.76 |
| miR-663 | GCGGTCCCGCGGCGCCCCGCCT | 241 | 3.51 | 2.57 | 2.02 | 1.44 | (nd) < -3.06 | (nd) < -3.06 | (nd) < -3.06 | (nd) < -3.06 | (nd) < -3.06 | (nd) < -3.06 |
| miR-671-5p | CTCCAGCCCCTCCAGGGCTTCCT | 242 | > 7.67 | nd | nd | > 5.82 | nd | nd | nd | > 1.94 | nd | nd |
| miR-675 | CACTGTGGGCCCTCTCCGCACCA | 243 | 7.25 | 4.60 | (nd) < -1.19 | 4.46 | (nd) < -1.19 | (nd) < -1.19 | -1.07 | 1.88 | (nd) < -1.19 | (nd) < -1.19 |
| miR-708 | CCCAGCTAGATTGTAAGCTCCTT | 244 | nd | nd | nd | nd | nd | nd | < -1.01 | nd | nd | > 2.16 |
| miR-744 | TGCTGTTAGCCCTAGCCCCGCA | 245 | 4.02 | 1.97 | 1.40 | 1.40 | (nd) < -3.47 | -2.20 | -3.66 | -1.68 | (nd) < -3.47 | (nd) < -3.47 |
| miR-765 | CATCACCTTCCTTCTCCTCCA | 246 | 25.46 | 3.15 | 3.08 | 6.06 | (nd) | 3.70 | 1.82 | 3.00 | (nd) | 1.02 |
| | | | | | | | < -1.58 | | | | < -1.58 | |
| miR-920 | TACTGCTTCCACAGCTCCCC | 247 | > 3.57 | nd | nd | nd | nd | nd | nd | nd | nd | nd |
| miR-923 | AGTTTCTTTTCCTCCGCTGAC | 248 | 6.70 | 1.19 | 1.24 | 1.35 | -2.18 | 1.11 | -1.43 | 1.19 | 2.00 | -2.79 |
| miR-92a-2* | GTAATGCAACAAATCCCCACCC | 249 | 3.79 | 2.45 | 1.23 | 1.20 | (nd) < -1.93 | (nd) < -1.93 | (nd) < -1.93 | 1.18 | (nd) < -1.93 | -1.59 |
| miR-92b^{*} | CACTGCACCGCGTCCCGTCCCT | 250 | 5.09 | 1.52 | 1.50 | 1.81 | (nd) < -3.D6 | (nd) < -3.06 | -3.14 | (nd) < -3.06 | (nd) < -3.06 | (nd) < -3.06 |
| miR-93 | CTACCTGCACGAACAGCACTTTG | 251 | (nd) < -2.50 | 1.27 | (nd) < -2.50 | (nd) < -2.50 | (nd) < -2.50 | 1.17 | -1.22 | 1.14 | 2.52 | 1.03 |
| miR-98 | AACAATACAACTTACTACCTCA | 252 | -4.47 | -1.37 | 1.17 | -2.84 | -4.10 | -1.83 | -2.38 | -1.22 | 1.21 | -4.82 |
| miR-99b | CGCAAGGTCGGTTCTACGGGTG | 253 | (nd) < -3.33 | -1.36 | 1.59 | -2.74 | (nd) < -3.33 | -2.23 | (nd) < -3.33 | 1.30 | -1.51 | (nd) < -3,33 |

**Table 2**

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adenocarcinoma)** | **BEA2B (Immortalized bronchial epithelial cells** - **normal phenotype)** | **A549 (Adenocarcinoma)** |
| 3717-L2-1 | CCGCCCTCCCCATAGCCTCACCCCAAACCCACTCACA | 6 | 1.02 | 1.86 | 1.77 | 2.27 |
| 3758-R2-2 | TGCAGGCTCCACTGACATTTTCACAATTTAAATCAT | 254 | -1.85 | 2.07 | 1.89 | 2.26 |
| 3799-R3-1 | CCAGAGGCCCCCCGCCGGCC | 7 | -1.63 | 1.14 | 1.33 | (nd) <6,30 |
| 3820-R3-1 | CCCCCACCCCTCTGTGGGGCCATCCCTG | 255 | 1.60 | nd | nd | nd |
| 3851-R3-4 | GAGCTCCCAACCCTCCTTTATGTTTTGTCTAAAGC | 256 | nd | nd | 6.82 | nd |
| 3874-L3-1 | TGAATATTATCCCTAATACCTGCCACCCCA | 257 | 2.75 | 10.71 | 10.14 | 10.68 |
| 3897-R3-1 | CAGCCGCCTCCCCCTCAGCGTTAA | 10 | -1.97 | 1.50 | 1.79 | 1.75 |
| 3906-L3-1 | AACATATGTAAACCCCTTTATTCCTCATTCTG | 258 | (nd) <4,59 | 1.25 | 1.70 | 2.14 |
| 3923-R3-1 | GCCTCTCACAAAGGATCTCCTTCATCCCTCTCC | 11 | -1.74 | 2.11 | 1.72 | 2.03 |
| 3952-L3-2 | GCGCACAGAGCACTCAATCTGACACCCCTCGC | 259 | -1.66 | 1.90 | 1.73 | 2.13 |
| 3953-R3-2 | ACTCCAGCCTCCGCCGCCTCAGCTTCCCGAGC | 12 | -1.22 | 1.78 | 1.59 | 1.81 |
| 3976-L2-2 | TCATACTCCTGCTTGCTGATCCACATCTGCTGGAA | 260 | 1.58 | 5.83 | 7.55 | 8.31 |
| 3995-L2-2 | CTATAAAACTTCGAAAAGTCCCTCCTCCTCACGT | 13 | -1.17 | 1.41 | 1.31 | 1.36 |
| 4037-R3-2 | GCCTGTTCCCTGGCATGTACTGTAATTTATCT | 15 | -2.05 | 2.26 | 1.66 | 1.88 |
| 4064-R3-1 | CATAGCTGAATTCCATCCCAGCCCCAG | 261 | 2.32 | nd | nd | nd |
| 4118-L2-2 | TCATACTCCTGCTTGCTGATCCACATCTGCTGGAA | 262 | 1.80 | 6.34 | 8.91 | 9.64 |
| 4130-L3-1 | GCCAGCACGCCGTCCATGTCCACCAGCACCC | 263 | -1.51 | 1.55 | 1.91 | (nd) <4.64 |
| 4143-R3-1 | TCAGCGTCTTGCTCTCCTCCTGGTA | 16 | 2.11 | 3.64 | nd | nd |
| 4155-R1-1 | AGACCGGACTCGCCTCTTCCAACTCGAGTTCA | 264 | nd | 3.56 | nd | nd |
| 4182-R2-2 | AGTTCAGCAGCCCAGTGGACATGCTGGGGGTGGT | 265 | 9.76 | 14.90 | nd | nd |
| 4203-R3-2 | GCACATTCCCACTTCCCCAGAGGCAGGCTCCATAT | 17 | nd | 5.60 | 7.52 | nd |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells** - **normal phenotype)** | **A549 (Adeno-carcinoma)** |
| 4205-R3-1 | ACTTCATCCTCACCACTCACACCACCCTAG | 266 | -1.94 | 1.83 | 2.35 | (nd) <3,98 |
| 4216-R3-1 | TCCCTCCCTTATACACAGATCAATTCCCCC | 267 | -1.53 | 1.78 | 1.86 | 2.51 |
| 4303-R1-1 | AGTGCCCGCTCCTCCGACCTCCCTGCGCACC | 19 | -1.09 | 1.56 | 1.42 | 1.57 |
| 4315-R3-2 | TCCCCGGCCCTCTCCATTCTCGGCTCCGGAGCA | 20 | -1.54 | 1.63 | 1.53 | 1.61 |
| 4340-R3-1 | ATTTTCCAGCCCCTTGTCCCCAGGCCAAAC | 268 | -1.75 | 1.98 | 1.79 | 2.09 |
| 4361-R3-1 | CGTCTCCCTCCCTCATGTGC | 21 | -1.60 | 1.46 | 1.56 | (nd) <4,18 |
| 4391-R2-1 | GCCAAATTCTCAACCAATATAACTCTGTGA | 269 | 2.65 | 7.30 | 10.84 | nd |
| 4413-L3-1 | GGCACCTCCAGCTACAGTAAACAAAT | 270 | -1.65 | 1.74 | 1.74 | 1.99 |
| 4417-R1-1 | GCTCATCAAAAAGTTCCCTGT | 271 | -1.75 | 1.40 | 1.49 | 1.66 |
| 4440-R3-1 | TACTCCCGCCGTTTACCCGCATTTCACTGAA | 272 | -1.71 | 1.75 | 1.76 | 2.23 |
| 4440-L3-1 | TTCAGAGCACTGGGCAGAAATCACATCAC | 273 | -1.70 | 1.70 | 1.81 | 2.20 |
| 4448-R3-1 | CCTACCCCCAGCATCTCCTCACGCCATTGCC | 24 | -1.76 | 2.12 | 1.76 | 2.36 |
| 4479-R3-1 | AGCCCCCTGCCCGGAAATTCAAAACAACTGC | 25 | -1.47 | 1.10 | 1.38 | 1.60 |
| 4498-L3-2 | GAGATCCAGACGGCCGTGCGCCTGCTGCTGCCT | 274 | -1.95 | 2.00 | 1.73 | 2.11 |
| 4567-L1-1 | ATCTGCCCAGTTCCCAGCACACTCCC | 275 | 2.50 | 6.95 | 8.90 | nd |
| 4579-L3-2 | GGCTTCACTTGCCTCCTGCAAAACACCAATAGC | 276 | -1.75 | 1.60 | 1.64 | 1.82 |
| 4593-R3-1 | AGCAGATGACATAACTCCCCCGGCATCAG | 26 | -1.83 | -1.24 | 1.68 | 1.74 |
| 4610-R3-1 | GCCCTCTGGCCCCTGCCTAATTGGCTGC | 277 | 1.04 | 1.55 | 1.43 | 1.66 |
| 4724-L3-2 | CTTGGCATCTCTAGCACCTTCAGCTTTCTGTGCCT | 278 | -1.60 | 1.82 | 1.71 | 1.91 |
| 4754-R3-2 | CCAAAGCCTTAGACAAGTGCCAAGCCCATCTTT | 279 | -1.58 | 1.60 | 2.35 | 2.49 |
| 4801-L3-1 | AACTCTGCCTCCTGTTTGCTACAAAAACATTAAT | 280 | -1.47 | 1.76 | 1.15 | 1.36 |
| 4829-R2-1 | TCCCTTTGTGCTGCCCGAGTGCCTTCCCCCTG | 29 | -1.59 | 1.81 | 1.62 | 1.92 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| 4855-R3-1 | GGGCTGCCGGGTCTCCCGCTTCC | 30 | 2.95 | 3.52 | 3.97 | nd |
| 4964-L3-2 | AGGGCTAACTTCTGAAAACCCACCAAATTCCCCAA | 281 | -2.00 | 1.44 | 1.52 | 1.79 |
| 5006-L3-1 | ACAGCTCCCTCTGCTGGCTCC | 33 | -2.37 | 1.57 | 1.68 | 1.78 |
| 5071-R2-1 | CCCCAGTCCCAGCCCAATTAATAAATGGG | 282 | -1.48 | 1.42 | 2.11 | 2.49 |
| 5080-R3-1 | CTTGCAAAGGGTCTCCTTCATCCCTCTCCA | 34 | -1.69 | 2.01 | 1.67 | 1.96 |
| 5192-L3-1 | CCCCTTCCTTCCTCTATATCAGCAAAGAC | 283 | -1.45 | 1.75 | 1.73 | 2.15 |
| 5306-L3-2 | CCTCTGACCCCAGCTCTGGCCCTTTCTAGGG | 284 | -1.72 | 1.66 | 1.75 | 2.05 |
| 5327-L3-1 | CTCAACCTCTGGGACTATGTCCTGTCTCC | 285 | nd | 6.36 | 8.44 | nd |
| 5342-L3-1 | CACCACCAAACCAAATGCCGCTGCTCTCCTTCCA | 36 | 2.47 | nd | nd | nd |
| 5372-R3-2 | CCCTCTGTTTTCAACTTACACAAGATTCTTTTT | 286 | -2.07 | 2.01 | 1.61 | 2.10 |
| 5380-R2-2 | GGCTCCCAGATGTGTCCCACATTGAAGAATTATC | 287 | -2.19 | 1.91 | 1.82 | 1.88 |
| 5441-L3-2 | GCCAAGCTCCAAGTCAGTATAGCTAACAGAGCAG | 288 | nd | 3.14 | nd | nd |
| 5474-L3-2 | CTCCTCTCACCTGGCCAGACTCTGACCCACCTAC | 289 | 2.77 | 4.11 | 7.78 | 8.58 |
| 5513-L3-1 | CAACTGTTCTCCATGATGCCTCAGAGCCACTT | 290 | -2.43 | 2.15 | 1.58 | 1.91 |
| 5554-R2-1 | CCCCACCCCCTCATCAGCTGCTCCCAGAT | 38 | -1.67 | 1.88 | 1.71 | 2.23 |
| 5598-R2-2 | CTCCCACCTCCGTGAAGCTATTTTTAACTGTGCA | 291 | -1.27 | 1.56 | 1.62 | 1.72 |
| 5618-R3-1 | TCCCAGCCCACCAGTGCCACATTACAGCCCA | 292 | -1.39 | 1.41 | 1.93 | 1.95 |
| 5619-L3-1 | AATGCCAGTTCTGCTCAATCTTCCCTCAATGAG | 293 | -2.45 | 1.94 | 1.69 | 2.04 |
| 5638-R2-1 | GGCCCTCCCCCTGCCTGTGATAGGCTGCTTG | 39 | -1.06 | 1.87 | 1.63 | 1.59 |
| 5640-L3-1 | GCCATGGAACACCGTGCCTGCCCCTCTCGAGA | 40 | -1.37 | 1.75 | 1.80 | 1.84 |
| 5733-R3-2 | CTCACCCAGCTCATCCTGCTTCTCAGTCCCAC | 294 | -1.58 | 1.47 | 1.34 | 1.76 |
| 5735-L3-1 | AGAAAGTTGCTGTTTCCTCTGGCCTCAAGCCT | 295 | -1.64 | 1.94 | 1.78 | 2.10 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| 5782-L3-1 | GATTCCAGCCCCTTCCCCC | 42 | 3.41 | 5.68 | 7.06 | 9.69 |
| 5795-R1-1 | CTGCCCTCCAAGAAATAAATTACCCGCAATTACT | 43 | -1.04 | 2.08 | 1.83 | 2.32 |
| 5836-R3-2 | CATTAACCCCCATTATCACAGCACGCCCCATTC | 44 | -1.60 | 1.58 | 2.26 | 2.56 |
| 5854-R3-1 | CCCTCCCTCTCCGAAAGAATGTGTCAC | 45 | -1.42 | 2.05 | 1.96 | 2.24 |
| 5863-L3-1 | GCCGTTGCTGCTGGCAATTCCTGTCG | 296 | -2.05 | 2.24 | 1.56 | 2.05 |
| 5919-L3-1 | AAGCAACACTGTCACTTTATCTCCCTAGA | 297 | -1.92 | 1.46 | 1.50 | 2.28 |
| 5971-R3-1 | CTGCTCAGCCTCCCACATCTGT | 46 | -1.53 | 1.49 | 2.17 | 2.31 |
| 6008-R1-1 | ACAATACCCCCACCTTTTTCCTGTACCTTAC | 47 | 2.16 | 4.91 | 7.51 | 8.14 |
| 6016-R2-1 | AAACTCCAGCAGCCCCGTCAGCCTCCTGCT | 48 | -1.45 | 1.75 | 1.66 | 1.86 |
| 6026-R3-1 | CAGCCACCTTGGTTTTGTGGTTTGGCAAA | 298 | nd | 5.34 | 7.28 | 7.92 |
| 6192-L3-1 | AGATAAAAAACCACCCACCCAGCAC | 52 | nd | 4.46 | nd | nd |
| 6218-R3-1 | AATCACATTACTGCCTCTCATGTCACA | 299 | 4.96 | nd | nd | nd |
| 6235-R3-1 | GCTCCAAAAATCCATTTAATATATTGTCCTT | 54 | -1.96 | 2.79 | -1.84 | (nd) <12,83 |
| 6253-L3-1 | CCTGACAATATCCTGGCTGCCATAATGCCAGC | 300 | -2.63 | 1.74 | 1.98 | (nd) <6,02 |
| 6287-L3-2 | GCCCCGCCCCACCTTTCGGGGCTCACCTGGC | 55 | -2.16 | 1.72 | 1.47 | 1.68 |
| 6355-R3-1 | TCCAGATCATCTGTTCCCTGAGGATTTACAGT | 301 | 1.94 | 5.81 | nd | nd |
| 6409-L3-1 | CGTTCCCAACCGCACGCGCCGCCTTCTGGAAC | 56 | -1.32 | 2.14 | 1.60 | 2.05 |
| 6421-R3-2 | CCCTCCTGTGAGAGTCTGAAGGACACTATTG | 302 | nd | 10.40 | nd | 10.50 |
| 6434-R3-1 | AGCCCTCCCACCAGCCAGCTGCAGTGC | 57 | 1.33 | 1.69 | 1.63 | 1.70 |
| 6450-R3-2 | CTCCAATGGTGCTCTCCTGGTACTCATGGAAC | 303 | 1.41 | nd | nd | nd |
| 6478-R2-2 | GCCAAATTCTGCCCCTGGATATGCATGCACAATT | 304 | -1.72 | 1.93 | 1.69 | 2.04 |
| 6496-R3-1 | CCCCTCCCCCACCCACCACTTCCCCTAGAGTCC | 60 | -1.36 | 1.95 | 2.28 | 2.20 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells** - **normal phenotype)** | **A549 (Adeno-carcinoma)** |
| 6554-L3-2 | TCCTTGTCATCTAGAACTACTTTGGTGCCTCCATA | 305 | nd | 3.79 | nd | nd |
| 6584-L1-1 | TCGGCCCTGCCTCCTCCTCCT | 61 | -1.36 | 1.82 | 1.33 | 1.52 |
| 6602-R3-2 | AGAGCCCCAGTGGAAATCTCTCCTCCAAATCCAT | 62 | 2.63 | 13.21 | 10.59 | 18.66 |
| 6642-R3-1 | CACGTCCTCCCCTCCCCTCGAGGTGTCACACA | 63 | 1.20 | 1.79 | 1.25 | 1.90 |
| 6647-R2-1 | CTCAGCCCCAGCTGGAGAATTTTTCCCCTCATTA | 306 | 1.56 | nd | nd | nd |
| 6664-R2-1 | GCCACCACCTCTCTTTTTCACAGGACATTACCA | 307 | nd | nd | 5.29 | nd |
| 6681-R2-1 | CCTGTTTTCTCCCCTCTCTCTCTGCCCCTCC | 64 | 4.99 | 7.90 | 9.03 | 11.45 |
| 6712-L2-1 | GCTGTGGTCTTGTGATATCAGTTGTCAGCCTG | 308 | 1.83 | 4.86 | 4.41 | nd |
| 6718-L3-2 | GCCTCCACCACCATAGGGGCCAGAGCTTCTGCCT | 309 | -1.53 | 2.03 | 1.89 | 1.92 |
| 6718-R3-1 | ATAGCCACCTTGGTTTTGTGGTTTGGCAAAG | 310 | 1.66 | 7.03 | nd | nd |
| 6752-R1-1 | CCCTCCTTTCCCCACCTCAGT | 66 | -2.15 | 1.89 | 1.60 | 2.23 |
| 6803-R3-1 | GCTCCCTCTCTGGTTGGACCTCACCCAAAGAT | 68 | -1.53 | 2.01 | 1.94 | 2.16 |
| 6839-L3-1 | GCCCGCTGGGCCCTGCCACCCCCACCCCT | 69 | -1.88 | 1.57 | 1.43 | 1.81 |
| 6880-L3-2 | ACCTCCCCCGCGAAGACATCCACATTCTGCA | 70 | -2.04 | 1.49 | 1.48 | 2.07 |
| 6906-L3-1 | GTGTCTTCTCCCCAACCAGCCAGCTCTCCTGG | 71 | nd | 3.61 | nd | nd |
| 6912-L3-1 | GCCTTCAGCCTCTGGGTCCAGCAGTTAATTCT | 311 | -1.45 | 1.82 | 1.06 | 1.68 |
| 6930-R3-1 | ATTAATCCTTCTCTCCCCTCTG | 72 | 2.11 | nd | nd | nd |
| 7019-R3-1 | AGCATCAAACCTCCGTGCTAAATTTAAA | 312 | 2.73 | nd | nd | nd |
| 7061-R3-1 | ATGGAAACCCCACCCTTCCC | 75 | nd | 5.97 | nd | nd |
| 7070-R3-1 | AGTCAACCTATACTGTCAGCACCAGGACCCAC | 313 | (nd) <3,72 | 1.57 | 2.14 | 2.63 |
| 7089-R1-1 | CCTGAGCCAGCTCACATCACCCCTGACC | 314 | -2.12 | 1.57 | 1.68 | 2.43 |
| 7126-L3-1 | GCACACCCGCTCTCCGGCCCGCGCCCCTG | 77 | -1.46 | 1.95 | 1.68 | 2.00 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| 7158-R3-1 | TACATTTATAGATTCCCTCTTCAGCCATA | 315 | nd | nd | 8.25 | nd |
| 7292-L3-4 | GTCACCCAGTTAAATAGCTTCTGAACCTCCCTGCA | 316 | (nd) <4,32 | 1.40 | 1.39 | (nd) <4,32 |
| 7304-L3-1 | TTGATCCAAGCTCCCACATTTG | 317 | 1.98 | 5.85 | 8.70 | 8.84 |
| 7340-R3-1 | CTGCCACCAACTCTAATTGATTC | 318 | (nd) <4,01 | 1.56 | 2.37 | 2.64 |
| 7352-R3-2 | GCCCCTGCCAGAATCCTCTAACAGCTCTAATTGG | 81 | -1.43 | 1.57 | 1.61 | 2.40 |
| 7356-R2-1 | GAAGCTCCGCGGCGACGTCCCGTTACTCC | 83 | -1.80 | 2.04 | 2.41 | 3.11 |
| 7375-L3-1 | AGCGCTGCTGTCTCCACAGTTACATACCTG | 319 | nd | 7.15 | 9.97 | 12.05 |
| 7384-R3-1 | CTCGCAAAGGATCTCCTTCATCCCTCCCCA | 85 | -1.49 | 2.00 | 1.91 | 2.17 |
| 7411-R3-2 | AGTCCCCTGCCTCATCTGCCACCCCTAATGAC | 86 | -1.61 | 1.70 | 1.62 | 1.87 |
| 7421-R2-1 | TAAAGAGACTTCCTCCACTGCCAGAGATCT | 87 | 2.12 | 4.83 | 8.07 | nd |
| 7426-L3-1 | TGGGAGACGAACACCTCCTGCTGTGCTTG | 88 | 4.29 | nd | nd | nd |
| 7435-L3-2 | CTCCCCATCTGTTGCTAAGCCCCATTAGCTGTGT | 320 | -1.79 | 2.09 | 1.86 | 2.33 |
| 7543-L3-2 | TGCCGATGTCGTCCTAATTCACCAGGCCCCGA | 321 | nd | 3.71 | 6.97 | nd |
| 7571-L1-1 | AGGGCTCCCCCACCCCTAAG | 90 | -1.73 | 1.43 | 1.50 | 1.43 |
| 7572-R2-1 | ATCACCCTTCCCCCTCCCAAATAAAG | 91 | -1.39 | 1.80 | 1.94 | 2.21 |
| 7578-L3-1 | CGCAGTGCACACCCTGAGCTACAGCCCCTC | 92 | 1.13 | 6.99 | 5.80 | 1.72 |
| 7597-L3-1 | TTAATGGAACCTGGGCTCTGTGTC | 322 | 1.75 | 4.17 | 8.05 | 8.93 |
| 7660-L2-1 | CCCGGCCTCCGCCTGGCCCGAGCGATAA | 93 | -1.11 | 1.74 | 1.69 | 1.78 |
| 7702-L2-1 | CCCAGAGAACCGGAATTCCTCCCCGCCCC | 94 | -2.10 | 1.41 | 1.47 | 1.48 |
| 7726-R3-2 | CATCCCTCTCCAGAAGAGGAGAAGAGGAAACA | 95 | -1.60 | 1.79 | 2.31 | 2.56 |
| 7763-R3-1 | GTGCTCCCAATCCAGACGATCCATTA | 323 | -1.67 | 1.67 | 2.28 | 2.52 |
| 7764-R3-2 | CCCTCTCTGCCTCTCTCATCACCAATAACAGAC | 96 | -1.09 | 1.93 | 1.74 | 2.01 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells** - **normal phenotype)** | **A549 (Adeno-carcinoma)** |
| 7824-R3-1 | TGGTGCCAGCTTCATCGCCG | 324 | nd | 3.25 | nd | nd |
| 8004-R3-2 | GGAACTGCTTCTCCTTGCTCCAGTCATTGAAG | 97 | nd | 3.51 | nd | nd |
| 8016-L3-1 | TCAGCGCAACAAGCCCCGCAGTCACCCCTCT | 98 | -1.99 | 1.68 | 1.62 | 2.30 |
| 8075-L3-1 | CCCAGCTACACCTCCACGCA | 325 | -1.64 | 1.92 | 1.68 | 1.83 |
| 8077-R3-1 | CCATTCCCCACCCTCAGGTAGTAAAAATA | 99 | -1.69 | 1.48 | 2.13 | 2.56 |
| 8169-L3-1 | AACAGAAATGATTATTTACCTCCCCACATG | 100 | -2.46 | 1.52 | 1.79 | (nd) <5,79 |
| 8250-R3-1 | CAGCCGCCTCTCCCTCAGCGTTAA | 101 | -1.41 | 1.55 | 1.60 | 1.84 |
| 8263-R3-1 | GATTAAAAACAAGAATCTATCTTCCCCCAGT | 102 | nd | nd | 5.24 | nd |
| 8281-L3-1 | AGCCCCTCCCCAGCTGCAGCTGAGGGCTGG | 103 | -1.34 | 1.78 | 2.04 | 2.78 |
| 8336-R3-1 | CTCAGTCCCCACACCCCCAGCCAGAGTC | 326 | -1.64 | 1.95 | 1.94 | 2.16 |
| 8394-L3-1 | CCCCCGCCCTGCCCATCTCCGACT | 105 | -1.43 | 1.63 | 1.71 | 1.75 |
| 8433-L3-1 | AAATGGCTCCTTTCCCCTTTCCCTCCACCG | 106 | -1.35 | 1.78 | 1.76 | 2.47 |
| 8434-R3-1 | CCTGAGGCTCCACTCCTAGAAGAATTGC | 327 | -1.61 | 2.16 | 1.98 | 2.42 |
| 8552-R3-1 | CTCCCAAGGGTCACCATAAAGAGGACACTATAAA | 328 | nd | 3.14 | nd | nd |
| 8564-L3-1 | CCCTTCACCCCAGTTGCCAAACA | 107 | nd | nd | 5.92 | nd |
| 8587-R2-1 | CTCGCCGGCTCCAAACTTTCCCCAACTCCAGG | 329 | -2.38 | 1.99 | 1.58 | 2.15 |
| 8685-L3-1 | CTGCTCTTTGCCTCCTATAAGTGGAATGTCTCCC | 330 | -1.07 | 2.02 | 1.43 | 1.80 |
| 8719-L3-2 | CTCACTTGCCTCCTGCAAAGCACCAGTAGCTGC | 331 | nd | 3.12 | nd | nd |
| 8724-R3-1 | GCCAAGCTTGGAACCTCTCCCTGCCAGCATCAC | 109 | nd | 3.62 | 7.40 | nd |
| 8731-R3-1 | TCATTCATGCCCCATCCTGCCAG | 110 | -1.66 | 1.77 | 1.84 | 2.45 |
| 8760-L3-1 | CTGGAGCCCCGAGGCAAAACTCACCCCAGGCA | 332 | -1.85 | 1.57 | 2.29 | 2.59 |
| 8808-R3-1 | CCAAAGACCCCTTTCTCCCAGCCTGTTTCTGCAA | 111 | -1.69 | 2.22 | 1.94 | 2.25 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells** - **normal phenotype)** | **A549 (Adeno-carcinoma)** |
| 8898-R3-1 | CGGACGCCCGCTCCCGCCA | 112 | -1.39 | 1.61 | 1.58 | 1.74 |
| 8898-L3-1 | GAGTTGCCGGCGGCCGCCCCGGCCGACAGCGCC | 333 | -1.51 | 1.99 | 1.88 | 2.15 |
| 9053-L3-1 | GGCCCTGGGAATCAGAGAGACAGTGCCCTTCC | 334 | -1.75 | 2.11 | 1.90 | 2.20 |
| 9053-R3-1 | TTCTTGCCCTCCAATCCCCGGGCTCCACCAGCC | 114 | -1.57 | 1.95 | 1.65 | 2.13 |
| 9068-R2-1 | CTGCCCTCCCTCTTGATCAAGACTGCTCTCCTAA | 115 | nd | nd | nd | 71.77 |
| 9092-R3-2 | TCCTAGAAGCCATCAGTATCCCACAGAGCCAG | 335 | -1.79 | 1.53 | 2.01 | 2.23 |
| 9217-L3-1 | ACGATCCCCGCCGTGACTAAAGCCAACAGTGGA | 117 | -1.38 | 2.00 | 1.87 | 2.15 |
| 9245-R2-1 | AACCTCTCATTAGCCAGCCACTCGCTCCCAAG | 118 | 1.91 | 4.41 | 6.66 | nd |
| 9387-R2-2 | TCCATCCTTGCCGTCGCCTTCATCTCAAAGCCATC | 122 | 1.93 | nd | nd | nd |
| 9507-L3-2 | GTCTCCCTCATCCATCATCC | 124 | -1.86 | 2.14 | 1.82 | 2.24 |
| 9557-R3-1 | ACTGGCCCAGTCCATTCTGCACCTCTTGCCCTA | 336 | -1.26 | 1.52 | 1.53 | 2.58 |
| 9582-R3-2 | TACAAATCCTCAGATGTTTCCACAAAGGCTCCCTT | 337 | -1.80 | 1.69 | 2.19 | 2.19 |
| 9688-L2-1 | GCTAAATGGCCCCAGACTGTTCTGCTGCA | 338 | nd | 3.61 | 6.62 | nd |
| 9694-R3-1 | GCCATCTGCCACCGACACTCATACTCTGT | 339 | -1.27 | 1.56 | 1.76 | 2.24 |
| 9733-L3-1 | AAGGCTGTCCCTCACCAGACTTCCCCACCCCT | 129 | -1.55 | 2.36 | 2.12 | 2.58 |
| 9747-L3-1 | GCACACCGCCTCCGGCAAACTGC | 340 | -1.27 | 1.88 | 1.37 | 1.56 |
| 9772-L3-1 | ATTCTACAGCATTTTTCCCATGACCTTTCCTGA | 341 | -2.02 | 2.12 | 1.72 | 2.14 |
| 9774-R2-2 | CCGCCCCCTCACCGCCTCCTGCTCCCATCAGGC | 130 | -1.09 | 1.68 | 1.54 | 1.66 |
| 9798-R3-2 | GTGCTTTCATTCCCCCAACAGAAGGGCATTA | 342 | -2.10 | 1.69 | 2.24 | 2.40 |
| 9812-L3-1 | AAGCTCTATTTATCTGGGCTCCCCAGCTTGCT | 343 | -1.54 | 1.49 | 2.00 | (nd) <4,17 |
| 9813-R3-2 | GAAAGTAGAATTTGGCCCTCCAACTGTACAGGATGA | 344 | -1.08 | 1.68 | 1.65 | 1.87 |
| 9816-R2-1 | CTGGCCCTTTAAGAGCCTCTCCGCGCGCTGCCG | 131 | -1.25 | 1.26 | 1.67 | 1.80 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| 9987-R2-2 | CAGGCTTCACCCCTCAGCCCACTTTGTTAAC | 345 | -1.73 | 2.09 | 1.92 | 2.17 |
| 10010-R2-1 | CTCGCCGGCTCCAAACTTTCCCCAACTCCAGG | 346 | -1.90 | 2.06 | 1.75 | 2.07 |
| 10030-R3-1 | AACTCAGCTGCCTTCCGCC | 134 | -1.41 | 1.98 | 1.48 | 1.79 |
| 10093-R2-2 | ACCAGCCAGACCCCCTGTAGGTCTAACCCAAGGT | 347 | -2.07 | 2.17 | 1.72 | 2.24 |
| 10120-R3-1 | TTCCCCTTGTTAAAATTACAGCTGCACCA | 348 | -1.97 | 1.92 | 1.73 | 1.98 |
| 10133-R3-1 | CTCCTCCCATTTCCTAATTTGATTTCAC | 349 | -1.45 | 1.90 | 1.88 | 2.28 |
| 10138-L2-1 | AGCCTGCTCCGCTCTCCCCTCCCACCAGAAAAAGT | 135 | 1.10 | 1.72 | 1.74 | 1.93 |
| 10154-R1-1 | GATCTGTGCCCTTTGCCCTT | 350 | 3.51 | 7.66 | 5.06 | (nd) <8,10 |
| 10198-R3-1 | AATTCTCTTTACCTGGCACCTTTAGGGCAAAGCA | 351 | 1.64 | 3.88 | 7.36 | 8.75 |
| 10231-L3-1 | GTGCAGCAGCCCGCGCCAGCCTCCGCAGCCGCC | 139 | -1.49 | 1.78 | 1.67 | 1.98 |
| 10231-R3-1 | TGAACTTTAGCTGGGCCGCCGCCTGTCAGC | 140 | -2.21 | -1.70 | -1.39 | (nd) <8,10 |
| 10242-R3-1 | GGAAGAAGCCCTTCCGCTTCCACCCCGAACAC | 141 | -1.11 | 1.49 | 1.33 | 1.40 |
| 10260-L3-1 | AGGGCCCCCACCCGATGTCTCCCAC | 352 | -1.85 | 2.24 | 1.79 | 2.27 |
| 10333-L3-1 | TGTGCCCTGCCCACCCCCTCCCCTGCCCCG | 142 | -1.21 | 1.48 | 1.74 | 1.85 |
| 10335-L3-1 | CACTCCCCTCCTTTTTAATTAGAAAGCACTAAGA | 143 | -1.31 | 1.94 | 1.97 | 2.22 |
| 10342-L2-1 | CCACTTTCCTGGCGACCCTCCGTGCGTGGG | 353 | -1.41 | 1.54 | 1.44 | 1.68 |
| 10342-R2-2 | CCCGCCGCCGGAGCATCTCGAAGTTAATTAAA | 144 | -2.74 | -2.11 | -1.51 | (nd) <13,60 |
| 10346-R3-2 | AGCCTGTCTGTGCCCTCTGCAGCAGCTCACC | 354 | 2.40 | 4.47 | 4.41 | nd |
| 10366-R3-2 | AAACACCACCCACGCTCTTGCTACAAGACCCACAT | 145 | -1.64 | 1.88 | 1.91 | 2.29 |
| 10539-R3-1 | TATTTGAGAAAATTTACTATCCCCCAGCCT | 355 | -1.64 | 1.82 | 1.96 | 2.08 |
| 10543-R3-1 | GCCTTCACCCTTCCCATCC | 356 | -1.44 | 1.70 | 1.76 | 2.27 |
| 10553-R1-1 | GCTGGCTCCATGCTCCAGTGGG | 357 | -2.27 | 2.01 | 1.64 | 2.01 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| 10562-L1-2 | GGGTCCTGACTCCCACAGCCTGTCATATCAAGCGC | 358 | 2.42 | 6.15 | 8.86 | 8.95 |
| 10594-L3-1 | ATTGTTACCCACACCAACACCCACTCAACAG | 359 | -1.91 | 1.96 | 1.74 | 2.18 |
| 10639-R2-1 | AGACCGGACTCGCCTCTTCCAACTCGAGTTCA | 360 | 1.44 | nd | 6.00 | nd |
| let-7a | AACTATACAACCTACTACCTCA | 166 | -1.87 | -1.06 | 1.39 | -1.34 |
| let-7b | AACCACACAACCTACTACCTCA | 167 | -1.85 | -1.12 | 1.72 | -1.38 |
| let-7c | AACCATACAACCTACTACCTCA | 168 | -2.23 | -1.18 | 1.47 | -1.30 |
| let-7d | AACTATGCAACCTACTACCTCT | 169 | -2.07 | -1.13 | 1.38 | -1.40 |
| let-7e | AACTATACAACCTCCTACCTCA | 170 | -2.12 | -1.05 | 1.46 | -1.10 |
| let-7f | AACTATACAATCTACTACCTCA | 171 | -1.94 | 1.00 | 1.29 | -1.33 |
| let-7g | AACTGTACAAACTACTACCTCA | 172 | 2.40 | 1.81 | 1.31 | 1.75 |
| let-7i | AACAGCACAAACTACTACCTCA | 173 | 3.29 | 1.99 | 1.31 | 2.02 |
| miR-100 | CACAAGTTCGGATCTACGGGTT | 174 | -3.44 | 1.01 | 1.30 | (nd) <7,51 |
| miR-103 | TCATAGCCCTGTACAATGCTGCT | 361 | 3.15 | 4.00 | 3.73 | nd |
| miR-106a | CTACCTGCACTGTAAGCACTTTT | 362 | -1.18 | 1.46 | -1.32 | (nd) <8,74 |
| miR-106b | ATCTGCACTGTCAGCACTTTA | 363 | 3.49 | 7.29 | 4.80 | nd |
| miR-107 | TGATAGCCCTGTACAATGCTGCT | 364 | 3.09 | 4.03 | 3.57 | nd |
| miR-125a-5p | AGGGACTCTGGGAAATTGGACACT | 365 | nd | 4.38 | 4.89 | nd |
| miR-125b | TCACAAGTTAGGGTCTCAGGGA | 179 | nd | 9.62 | 7.23 | nd |
| miR-130a | ATGCCCTTTTAACATTGCACTG | 366 | 2.00 | 4.87 | 5.06 | nd |
| miR-130b | ATGCCCTTTCATCATTGCACTG | 367 | 1.85 | 4.20 | 4.06 | nd |
| miR-134 | CCCCTCTGGTCAACCAGTCACA | 368 | -1.92 | 1.87 | 1.64 | 1.92 |
| miR-138 | CGGCCTGATTCACAACACCAGCT | 369 | 1.56 | nd | nd | nd |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| miR-155 | ACCCCTATCACGATTAGCATTAA | 187 | nd | nd | 3.73 | nd |
| miR-15a | CACAAACCATTATGTGCTGCTA | 370 | nd | nd | 5.75 | nd |
| miR-15b | TGTAAACCATGATGTGCTGCTA | 371 | nd | nd | 6.48 | nd |
| miR-16 | CGCCAATATTTACGTGCTGCTA | 188 | -1.03 | 2.33 | 3.10 | (nd) <5,85 |
| miR-17 | CAAAGTGCTTACAGTGCAGGTAG | 372 | -1.18 | 1.52 | -1.35 | (nd) <8,91 |
| miR-181a | ACTCACCGACAGCGTTGAATGTT | 373 | -1.45 | (nd) <3,46 | -1.05 | (nd) <3,46 |
| miR-181b | ACCCACCGACAGCAATGAATGTT | 374 | 2.17 | nd | nd | nd |
| miR-191 | CAGCTGCTTTTGGGATTCCGTTG | 375 | 1.89 | 6.53 | 3.50 | nd |
| miR-195 | GCCAATATTTCTGTGCTGCTA | 376 | nd | 4.38 | 5.32 | nd |
| miR-196b | CCCAACAACAGGAAACTACCTA | 377 | 1.49 | nd | nd | nd |
| miR-198 | GAACCTATCTCCCCTCTGGACC | 190 | nd | 5.34 | 7.25 | nd |
| miR-19a | TCAGTTTTGCATAGATTTGCACA | 378 | -1.38 | 1.29 | -1.40 | (nd) <7,33 |
| miR-19b | TCAGTTTTGCATGGATTTGCACA | 192 | -1.42 | 1.30 | -1.56 | (nd) <10,00 |
| miR-200b | TCATCATTACCAGGCAGTATTA | 193 | (nd) <8,612 | (nd) <8,61 | (nd) <8,61 | (nd) <8,61 |
| miR-200c | TCCATCATTACCCGGCAGTATTA | 194 | (nd) <18,47 | (nd) <18,47 | (nd) <18,47 | (nd) <18,47 |
| miR-205 | CAGACTCCGGTGGAATGAAGGA | 195 | (nd) <28,34 | (nd) <28,34 | (nd) <28,34 | (nd) <28,34 |
| miR-20a | CTACCTGCACTATAAGCACTTTA | 379 | -1.33 | 1.22 | -1.87 | (nd) <8,17 |
| miR-20b | CTACCTGCACTATGAGCACTTTG | 380 | -1.53 | 1.33 | -1.76 | (nd) <6,30 |
| miR-21 | TCAACATCAGTCTGATAAGCTA | 196 | -7.45 | 1.45 | 1.38 | 1.98 |
| miR-22 | ACAGTTCTTCAACTGGCAGCTT | 381 | -1.31 | -1.07 | 1.33 | -1.71 |
| miR-221 | GAAACCCAGCAGACAATGTAGCT | 382 | -4.50 | -1.92 | -1.27 | -2.19 |
| miR-222 | ACCCAGTAGCCAGATGTAGCT | 383 | -3.60 | -2.07 | -1.17 | (nd) <11,32 |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| miR-23a | GGAAATCCCTGGCAATGTGAT | 197 | -2.44 | -1.24 | 1.05 | (nd) <14,57 |
| miR-23b | GGTAATCCCTGGCAATGTGAT | 199 | -2.14 | -1.20 | 1.22 | -1.04 |
| miR-24 | CTGTTCCTGCTGAACTGAGCCA | 200 | -2.89 | 1.30 | 1.02 | 1.18 |
| miR-25 | TCAGACCGAGACAAGTGCAATG | 384 | 3.98 | 8.33 | 5.07 | nd |
| miR-26a | AGCCTATCCTGGATTACTTGAA | 202 | -3.08 | 1.23 | 1.24 | (nd) <7,67 |
| miR-26b | ACCTATCCTGAATTACTTGAA | 203 | nd | 3.58 | nd | nd |
| miR-27a | GCGGAACTTAGCCACTGTGAA | 204 | -2.28 | -1.28 | -1.04 | 1.02 |
| miR-27b | GCAGAACTTAGCCACTGTGAA | 205 | -1.92 | 1.27 | 1.14 | 1.38 |
| miR-29a | TAACCGATTTCAGATGGTGCTA | 207 | -1.77 | 1.26 | -1.01 | -1.07 |
| miR-29b | AACACTGATTTCAAATGGTGCTA | 208 | 1.16 | 1.16 | -1.30 | (nd) <5,77 |
| miR-29c | GAACACCAGGAGAAATCGGTCA | 385 | -1.29 | 1.29 | -1.29 | (nd) <7,48 |
| miR-30a | ACATTTGTAGGAGCTGACCTTC | 386 | -1.62 | 3.78 | 2.06 | (nd) <4,49 |
| miR-30d | CTTCCAGTCGGGGATGTTTACA | 216 | 2.12 | 16.27 | 7.18 | nd |
| miR-31 | AGCTATGCCAGCATCTTGCCT | 387 | -10.63 | -2.12 | -2.25 | -1.74 |
| miR-320a | TTTTCGACCCAACTCTCCCGCT | 388 | -1.22 | -1.05 | 1.49 | 1.15 |
| miR-335 | ACATTTTTCGTTATTGCTCTTGA | 389 | nd | nd | 3.55 | nd |
| miR-342-3p | AGAGTGTGTCTTTAGCGTGGGCA | 390 | -3.37 | 1.81 | 1.25 | 1.76 |
| miR-370 | ACCAGGTTCCACCCCAGCAGGC | 391 | 2.02 | 5.04 | 8.94 | nd |
| miR-424 | TTCAAAACATGAATTGCTGCTG | 222 | nd | nd | 5.25 | nd |
| miR-452 | TCAGTTTCCTCTGCAAACAGTT | 392 | nd | nd | 4.54 | nd |
| miR-494 | GAGGTTTCCCGTGTATGTTTCA | 393 | 1.30 | 2.20 | 1.01 | 1.55 |
| miR-513a-5p | AAGTGTCCCTCCACAGTA | 394 | 3.47 | 5.11 | 7.21 | nd |

| | | | **fold-changes in cell lines v. normal Lung** | | | |
|---|---|---|---|---|---|---|
| **Array probe** | **Array probe sequence (5' to 3', without linker)** | **probe SEQ ID NO:** | **H460 (Large cell carcinoma)** | **H1703 (Adeno-carcinoma)** | **BEA2B (Immortalized bronchial epithelial cells-normal phenotype)** | **A549 (Adeno-carcinoma)** |
| miR-614 | CCACCTGGCAAGAACAGGCGTTC | 236 | -1.85 | 1.83 | 1.69 | 1.99 |
| miR-638 | AGGCCGCCACCCGCCCGCGATCCCT | 239 | -1.95 | -1.02 | 1.09 | -1.05 |
| miR-658 | ACCAACGGACCTACTTCCCTCCGCC | 240 | -1.35 | 1.50 | 1.30 | 1.60 |
| miR-663 | GCGGTCCCGCGGCGCCCCGCCT | 241 | -1.86 | 1.08 | 1.37 | (nd) <6,22 |
| miR-671-5p | TCCTTCGGGACCTCCCCGACCTC | 242 | 1.85 | nd | nd | nd |
| miR-7 | ACAACAAAATCACTAGTCTTCCA | 395 | 3.27 | nd | nd | nd |
| miR-765 | CATCACCTTCCTTCTCCTCCA | 246 | nd | 4.51 | nd | nd |
| miR-92-a | ATAACGTGAACAGGGCCGGACA | 396 | 4.00 | 6.61 | 3.83 | nd |
| miR-93 | CTACCTGCACGAACAGCACTTTG | 251 | -1.07 | 1.75 | -1.04 | (nd) <5,78 |
| miR-98 | AACAATACAACTTACTACCTCA | 252 | -1.90 | -1.05 | 1.43 | (nd) <5,20 |
| miR-99a | CACAAGATCGGATCTACGGGTT | 397 | -2.49 | 1.13 | 1.31 | (nd) <5,34 |
| miR-99b | CGCAAGGTCGGTTCTACGGGTG | 253 | 2.05 | 4.50 | 3.79 | nd |

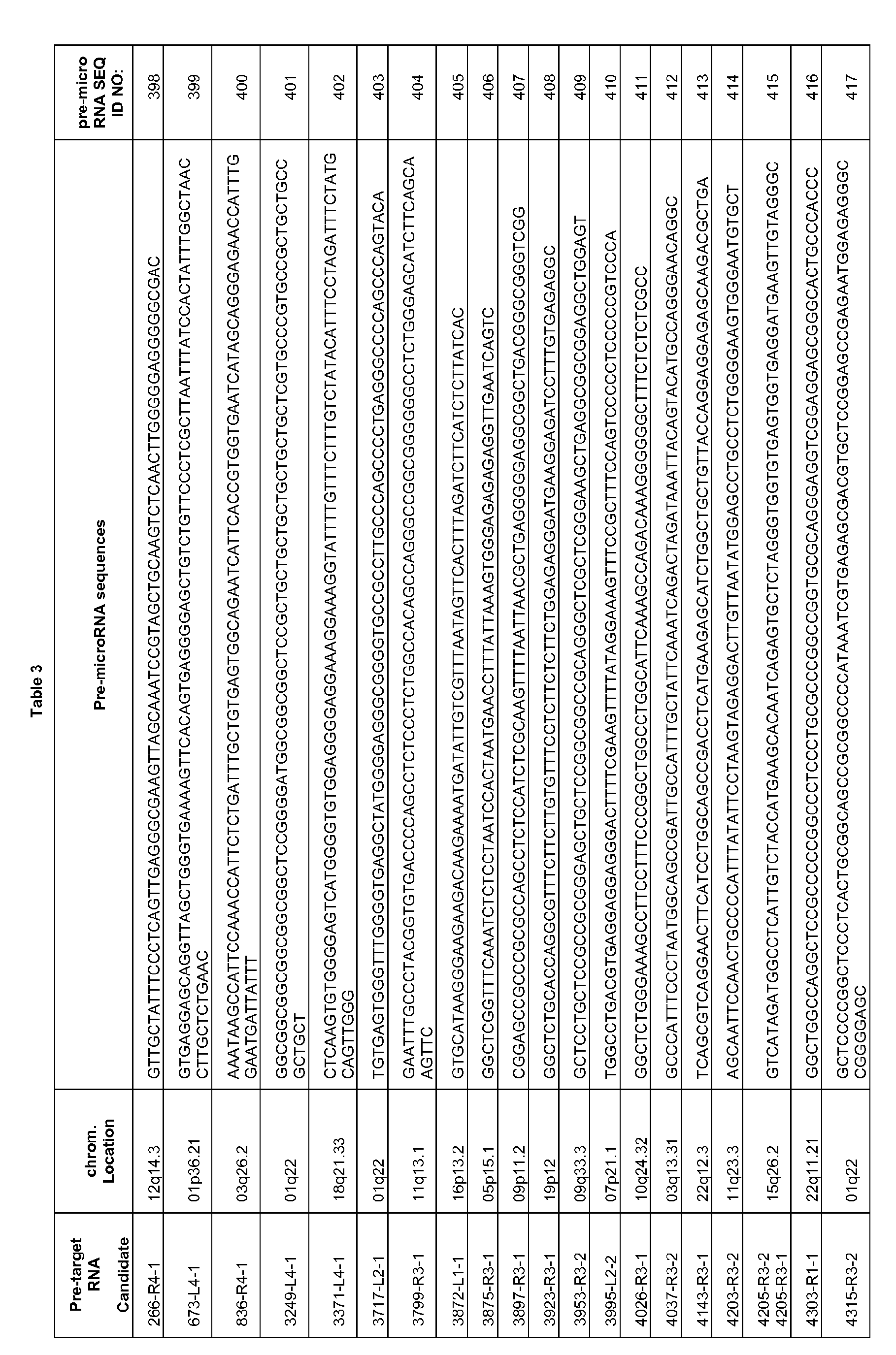

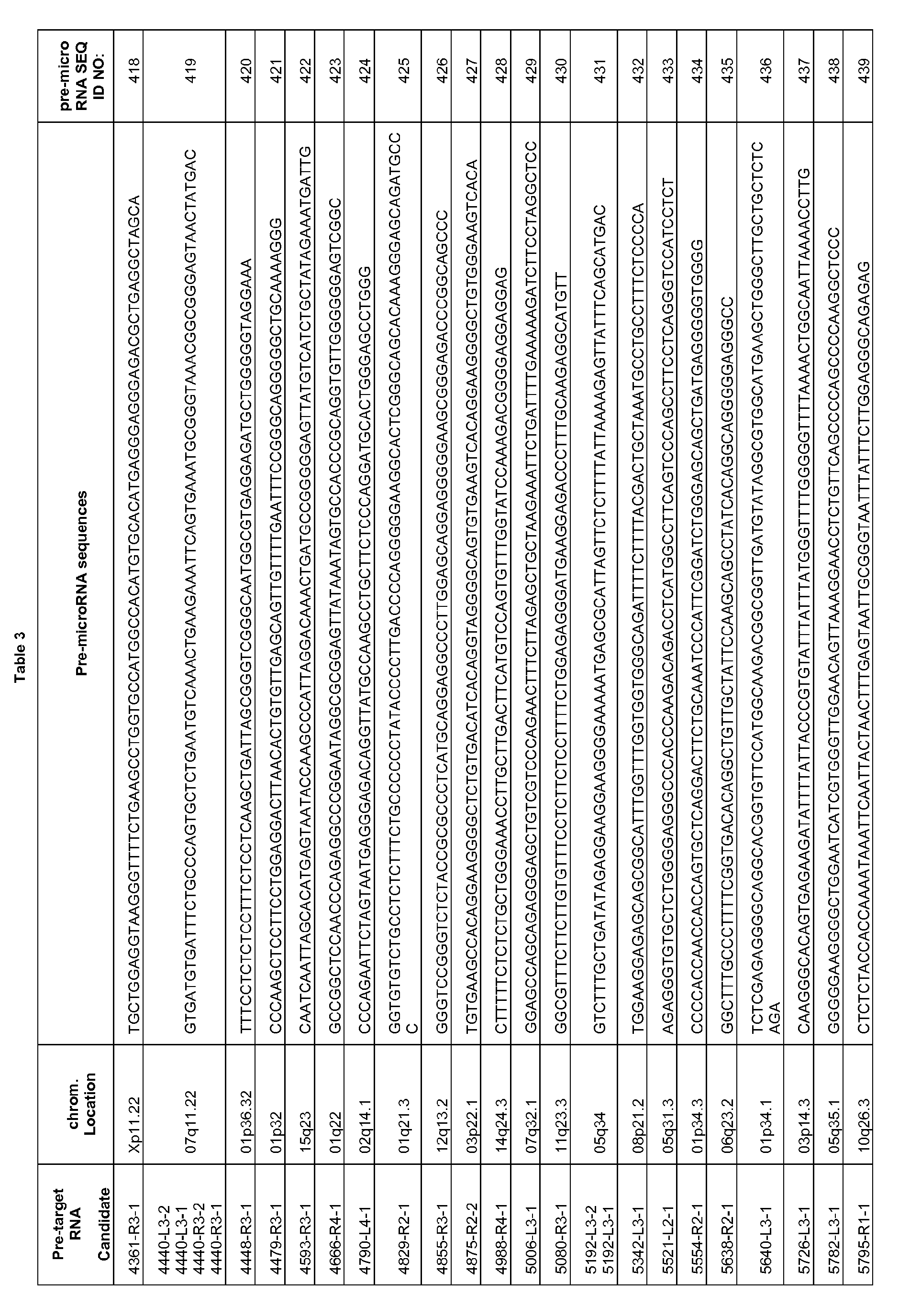

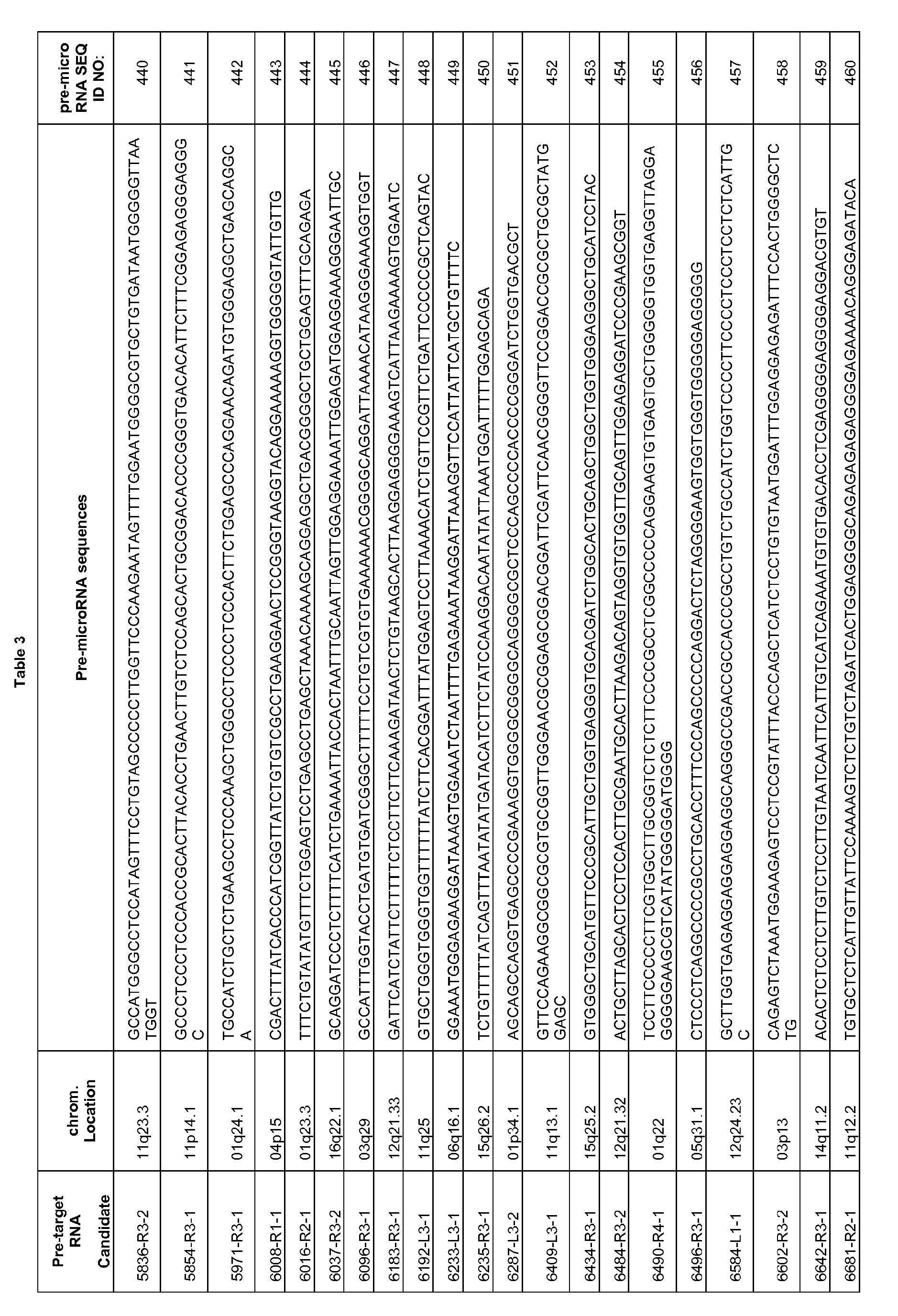

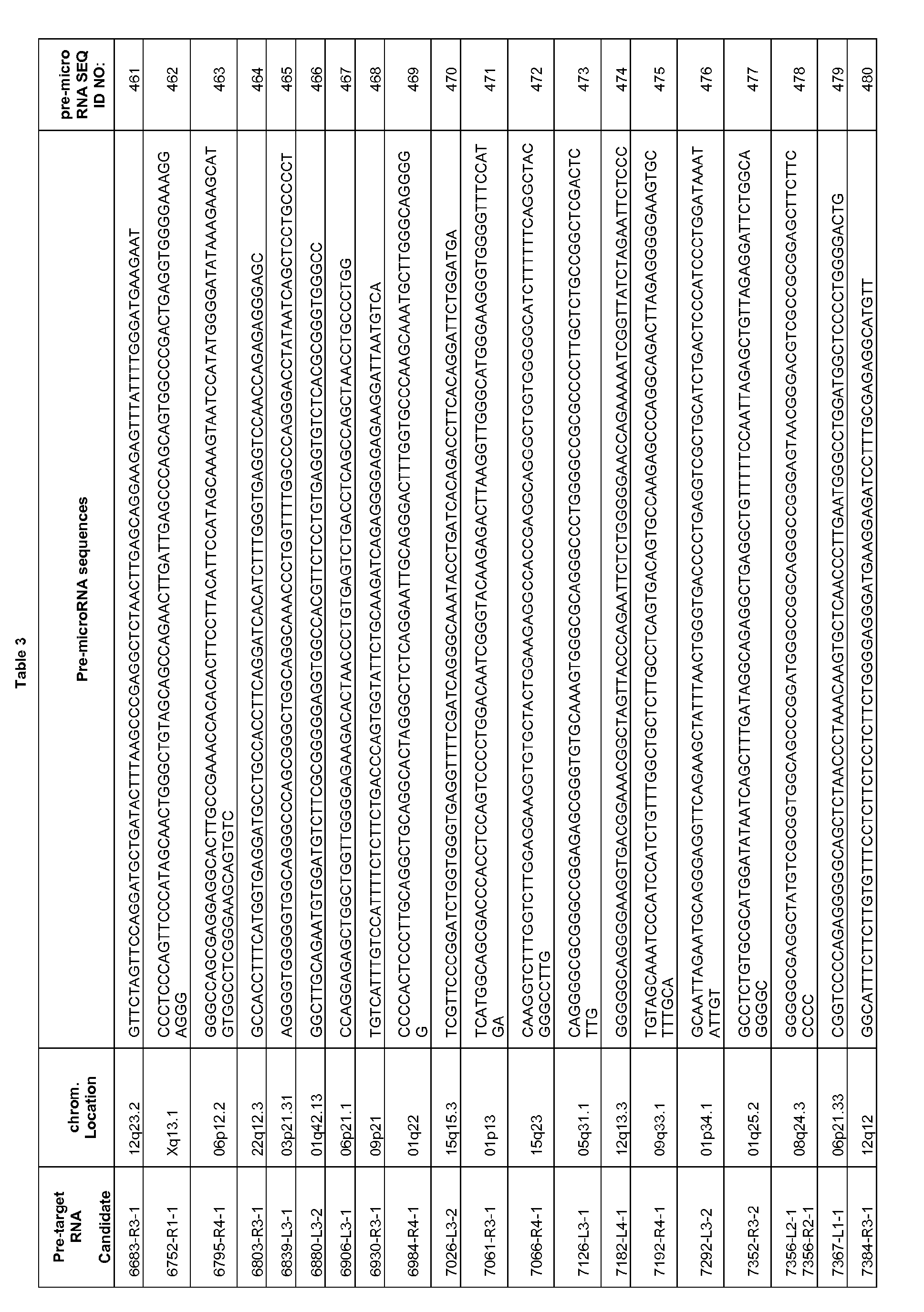

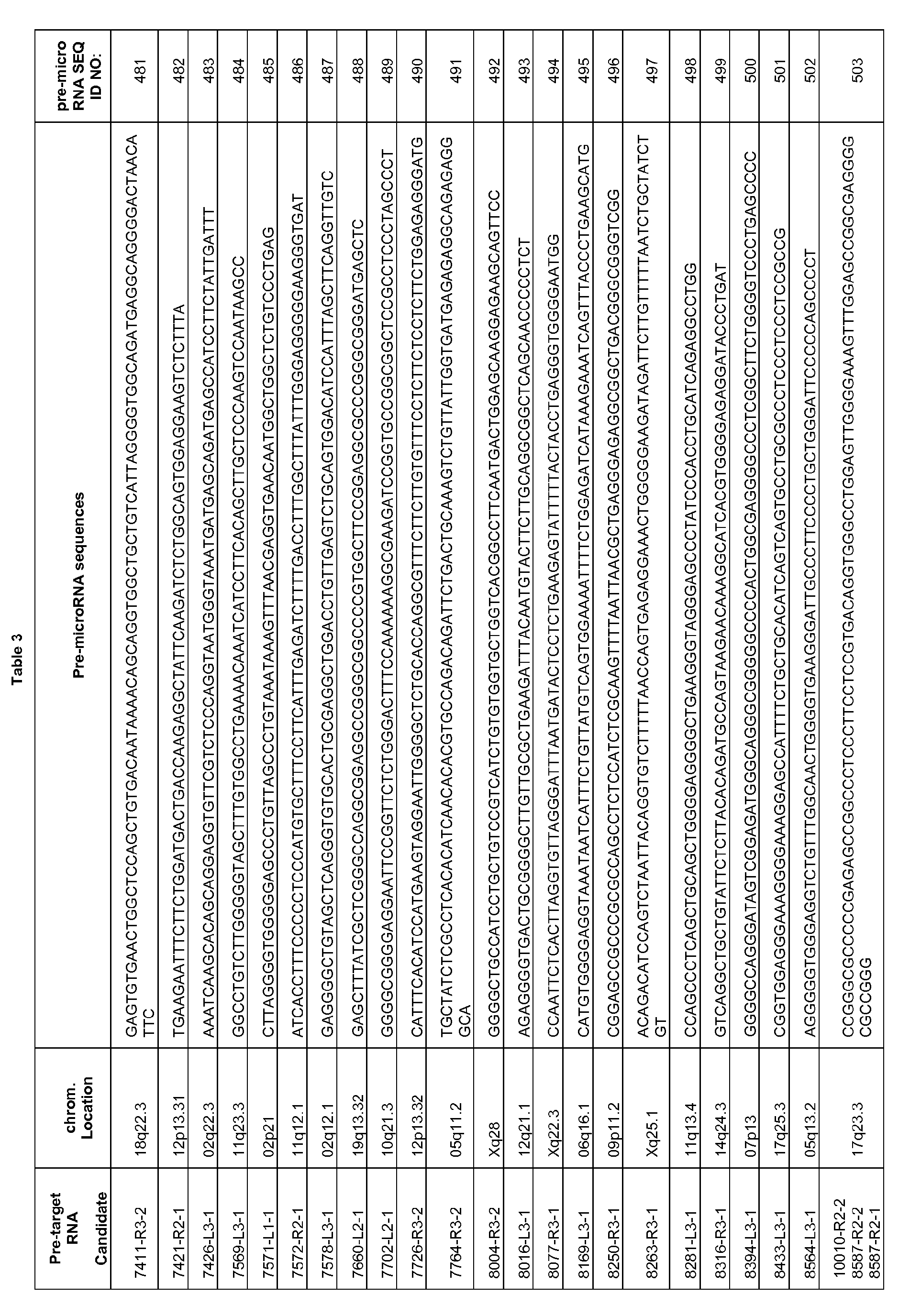

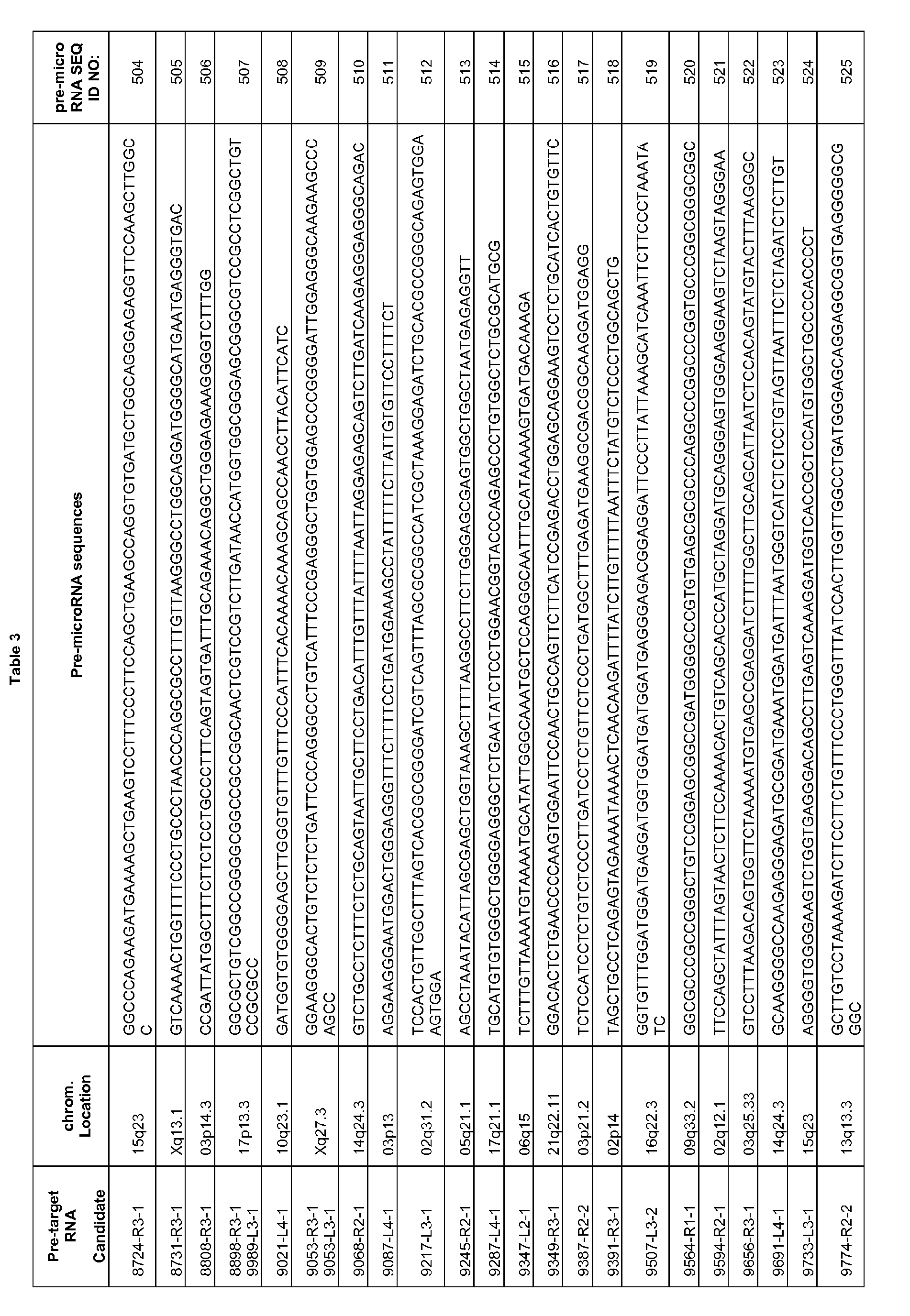

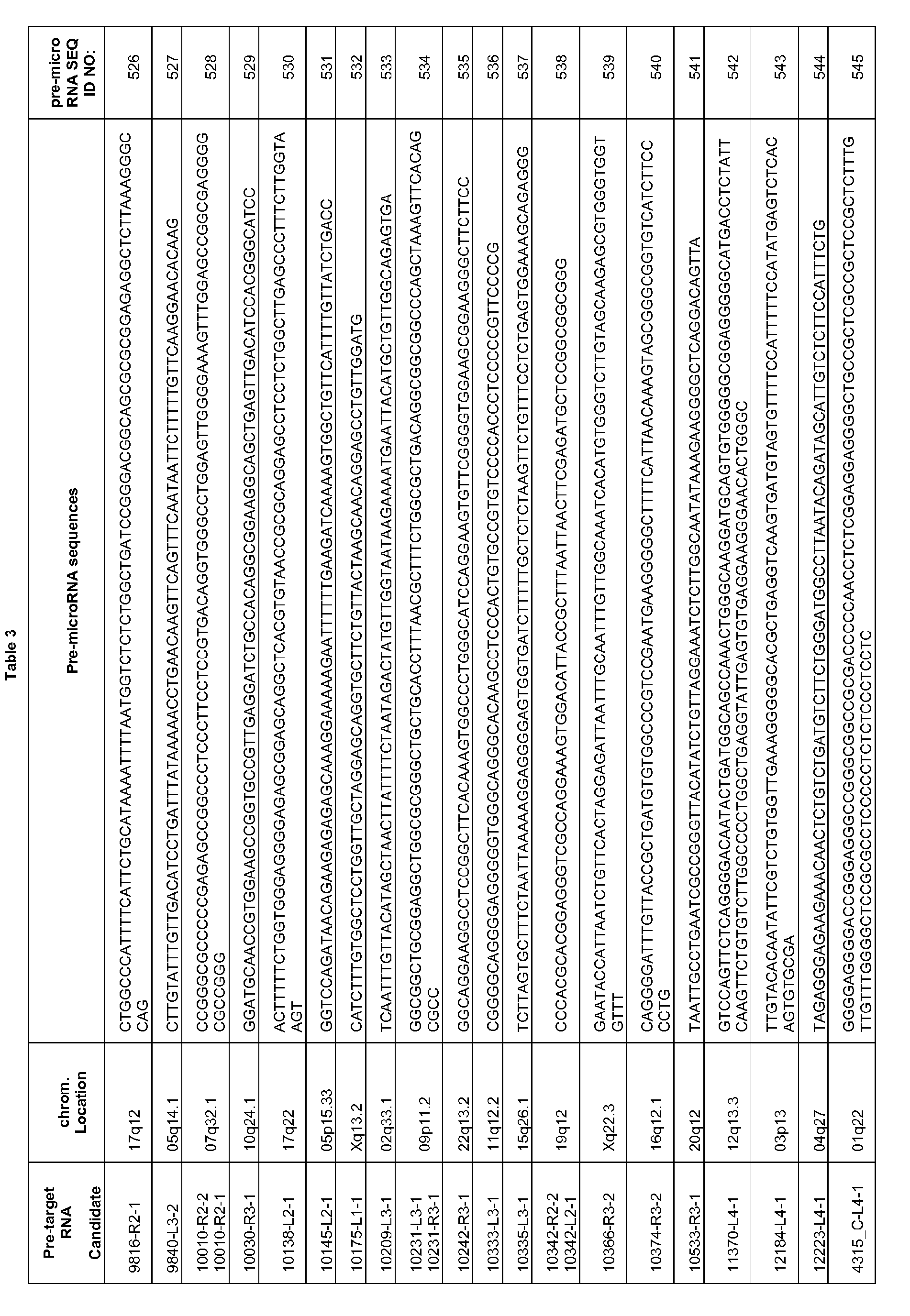

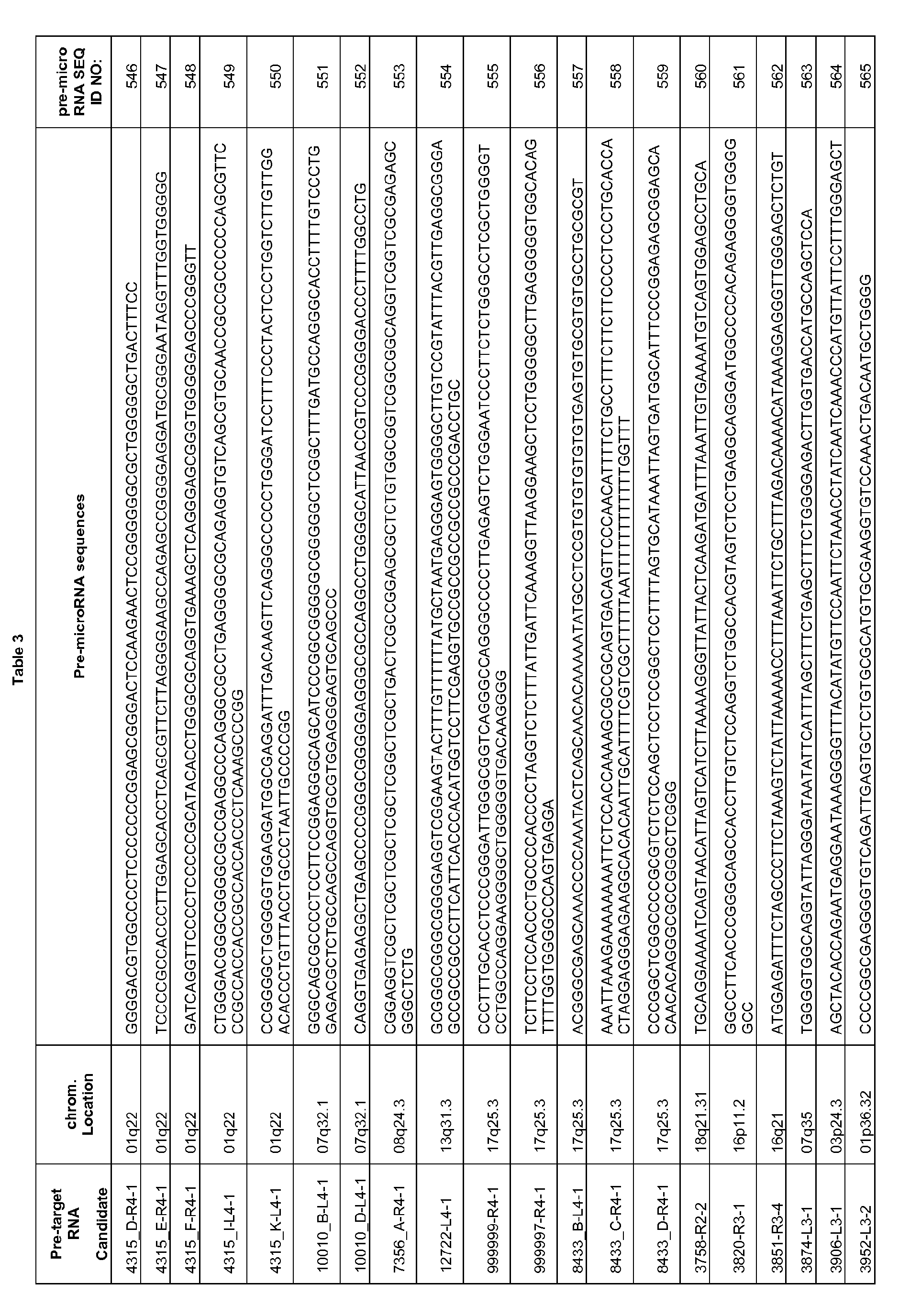

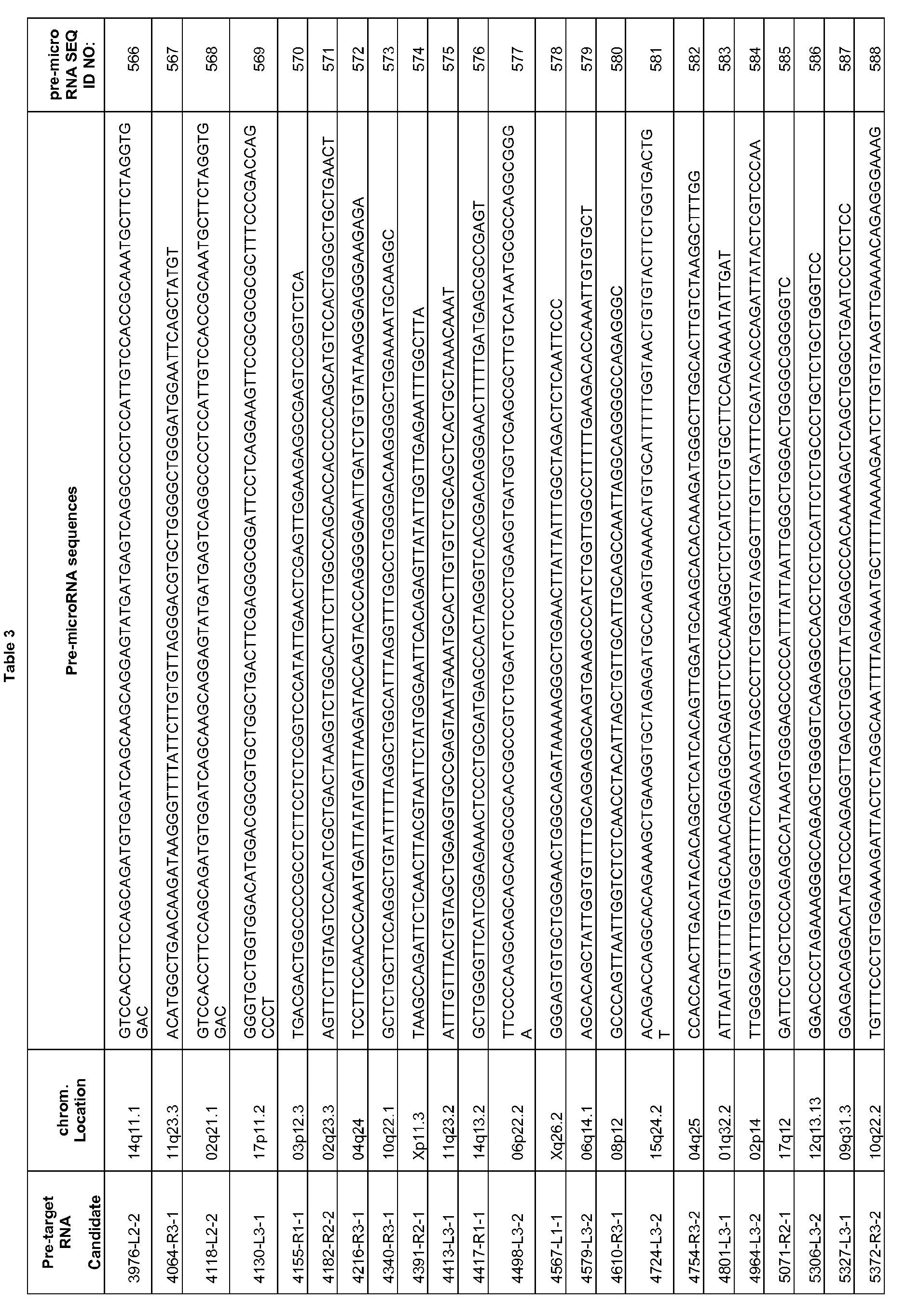

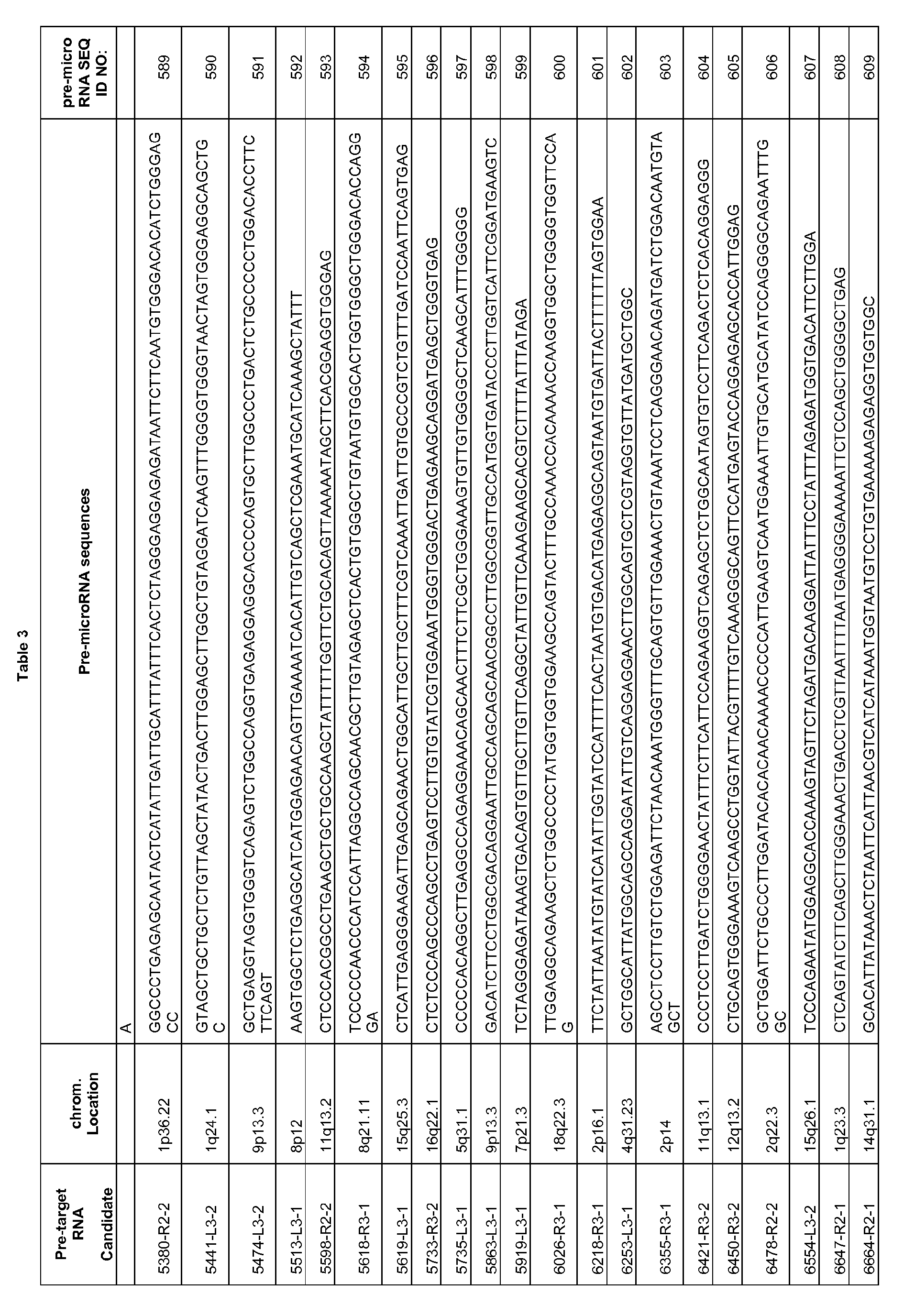

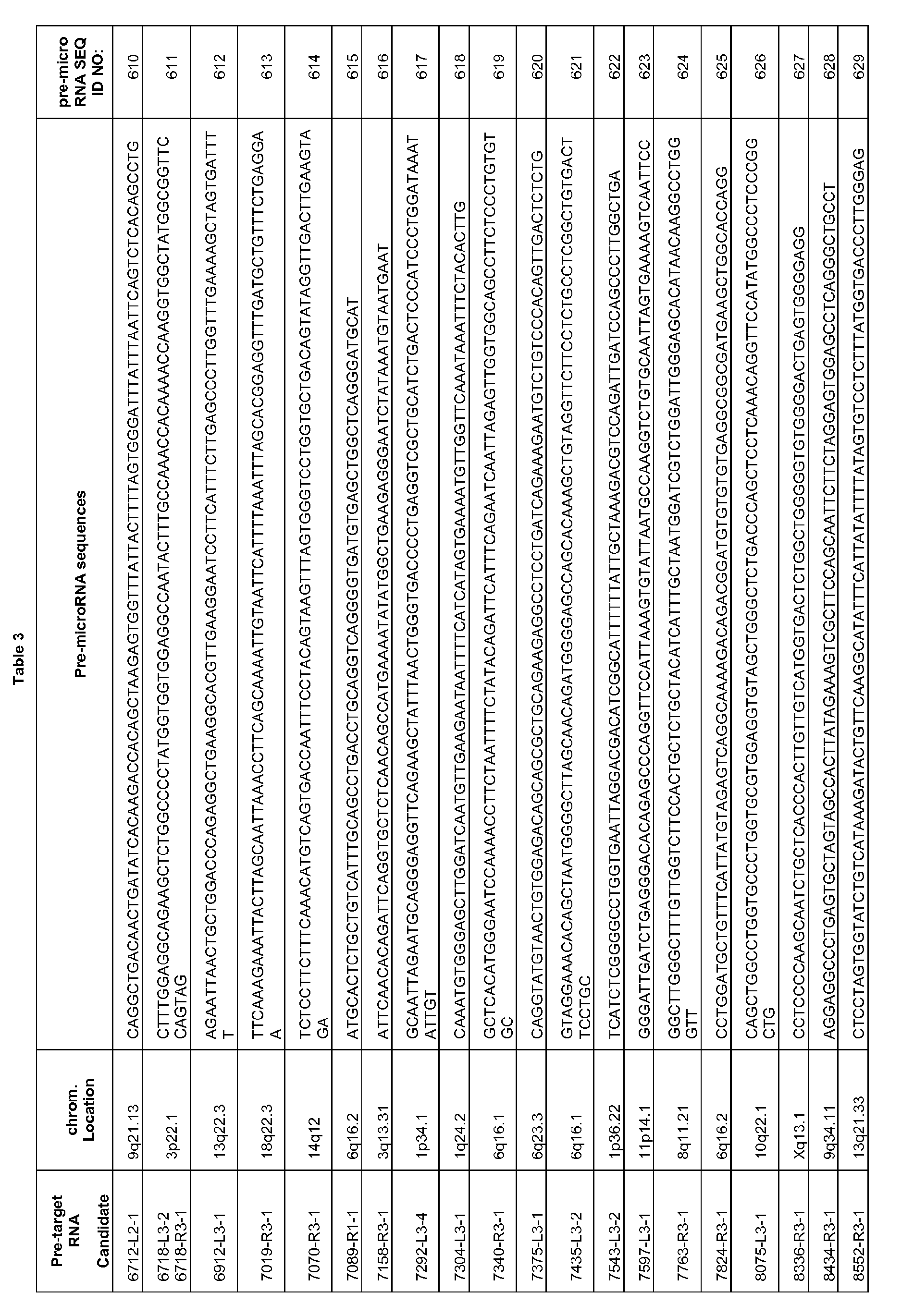

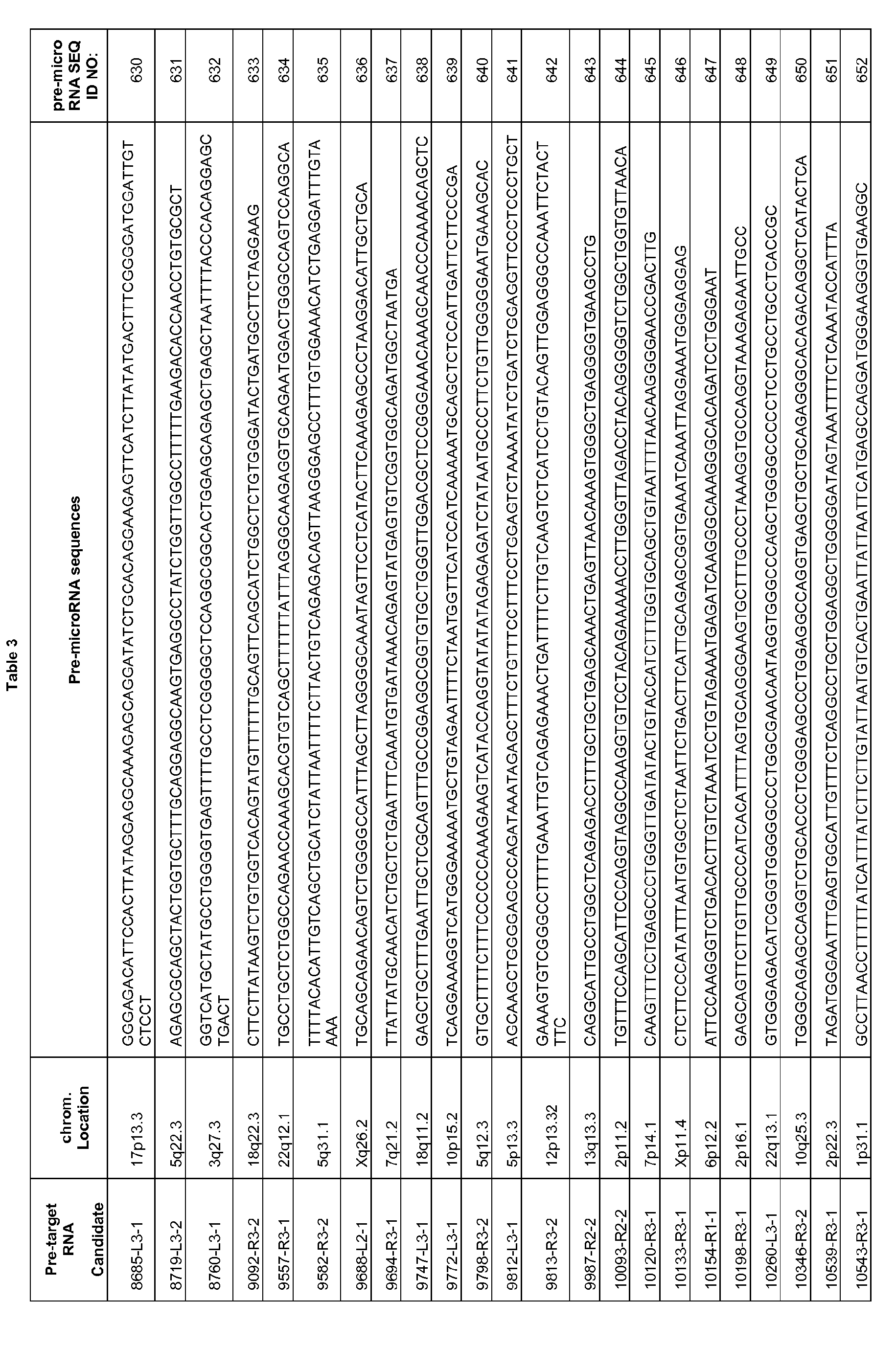

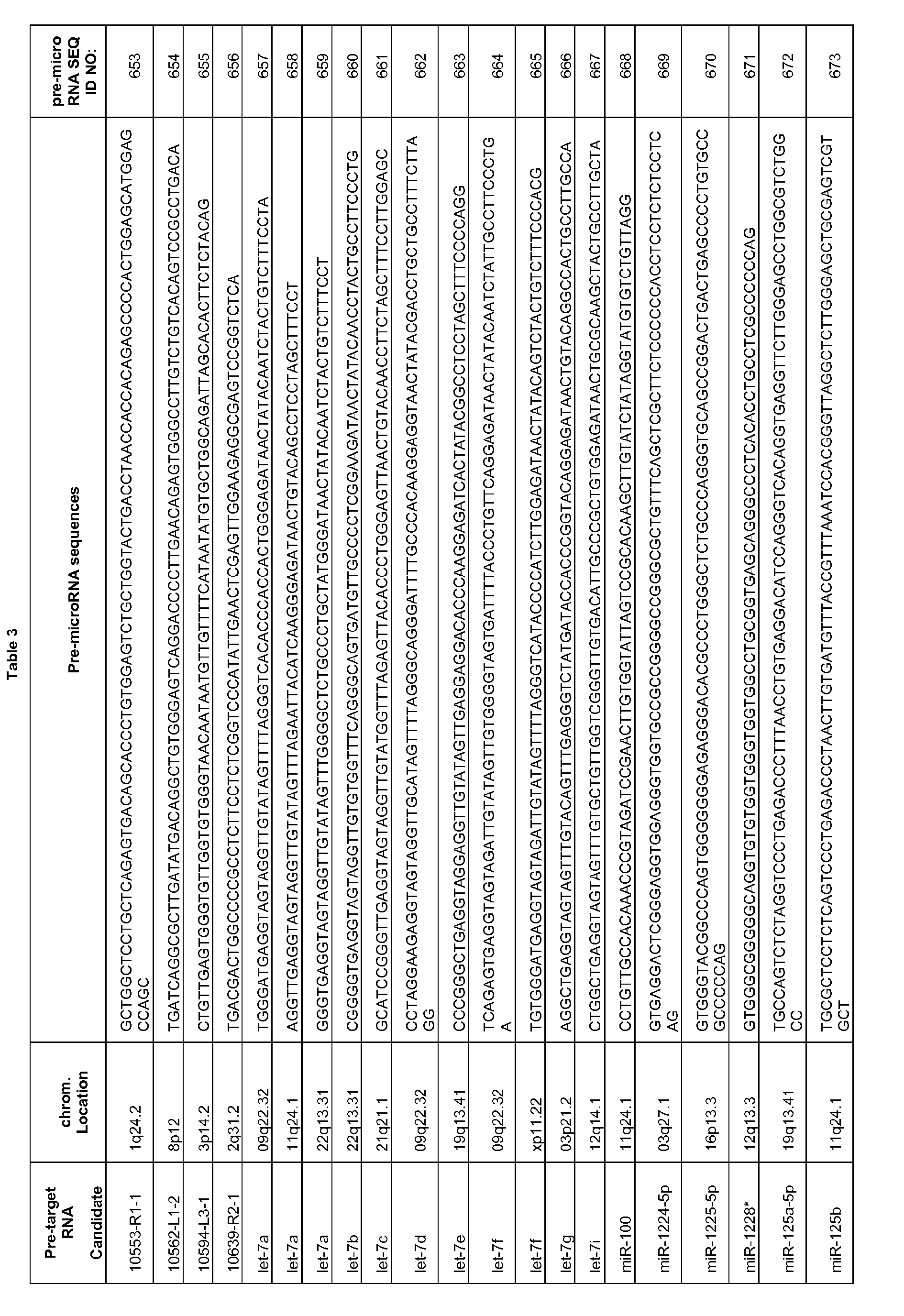

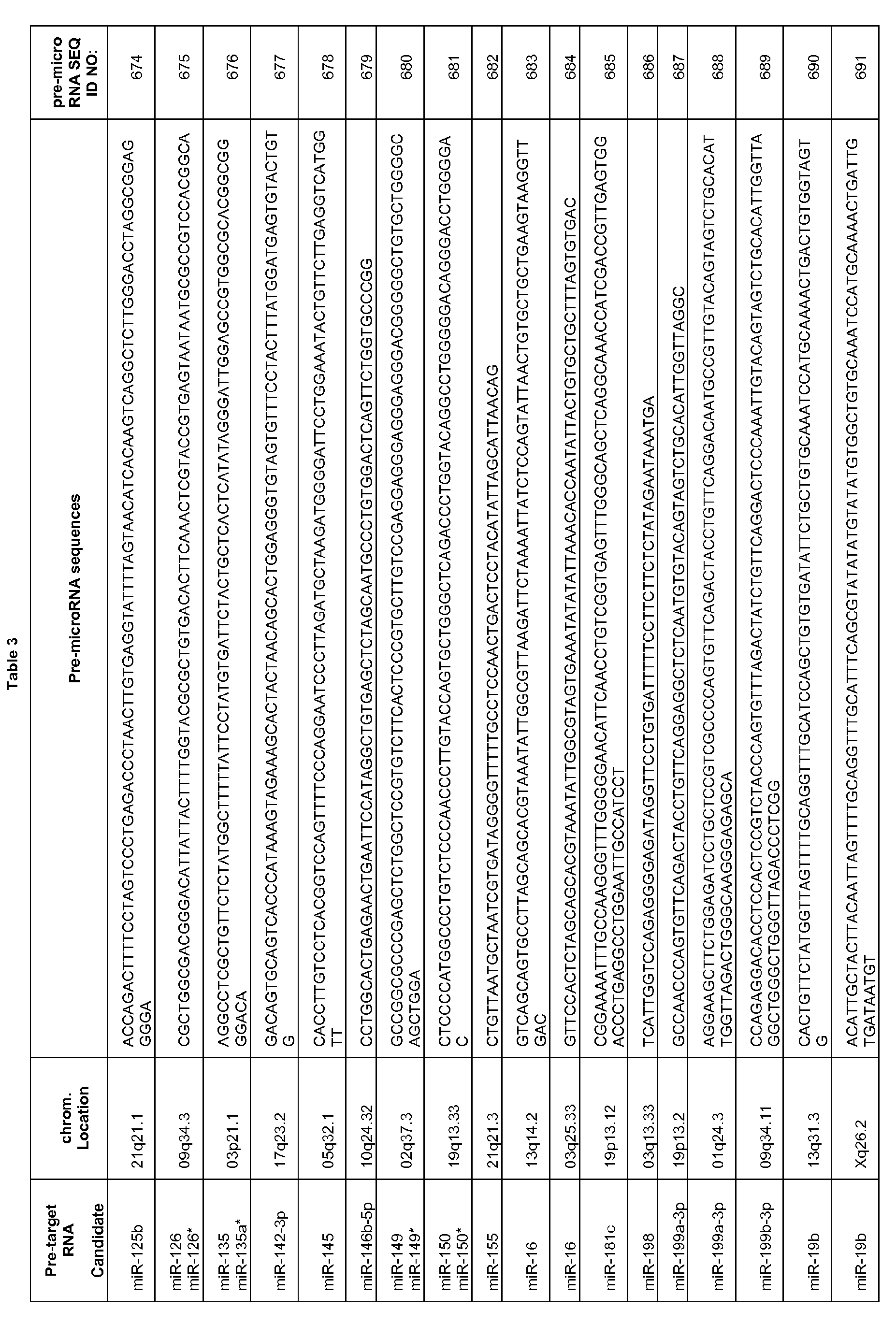

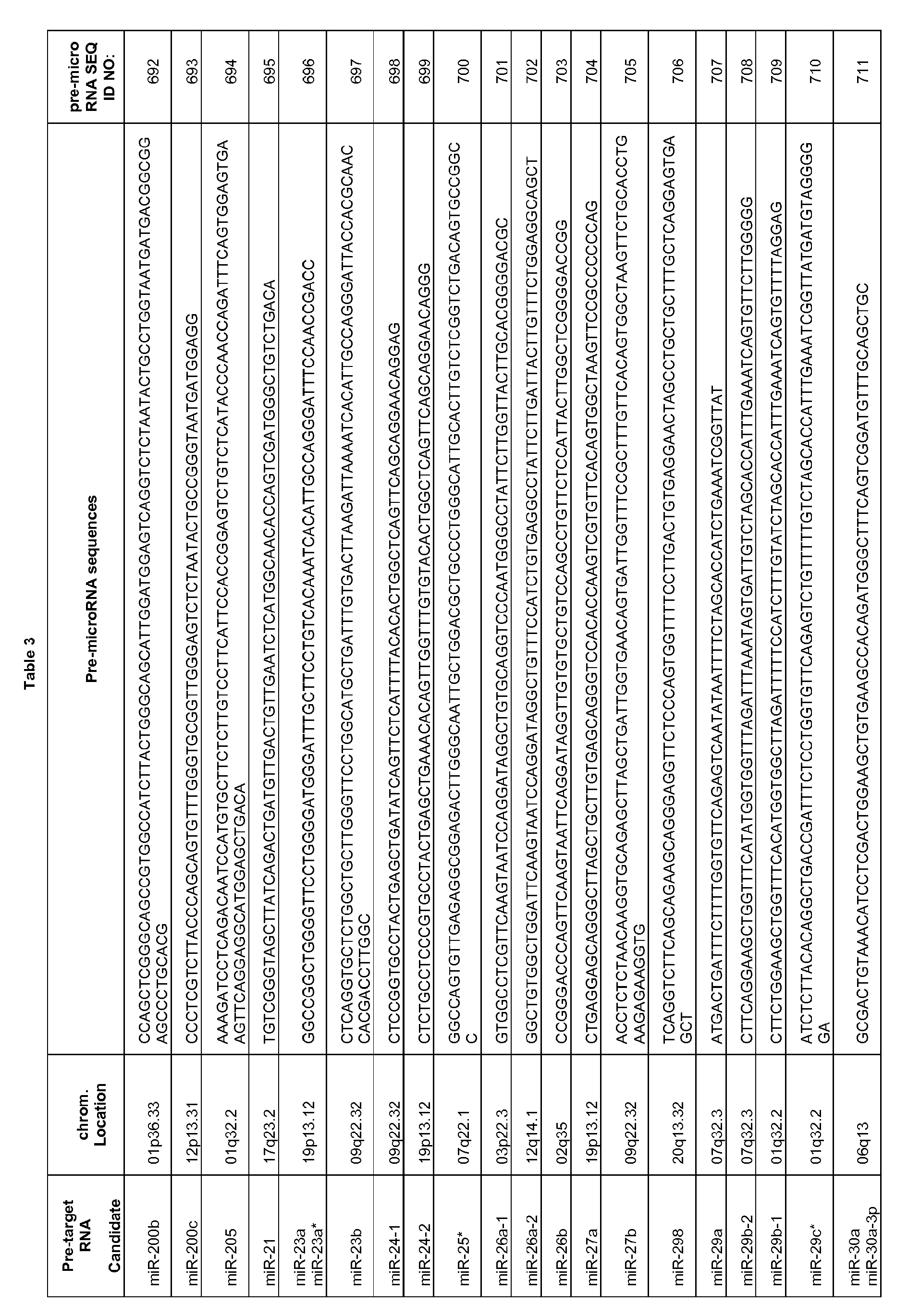

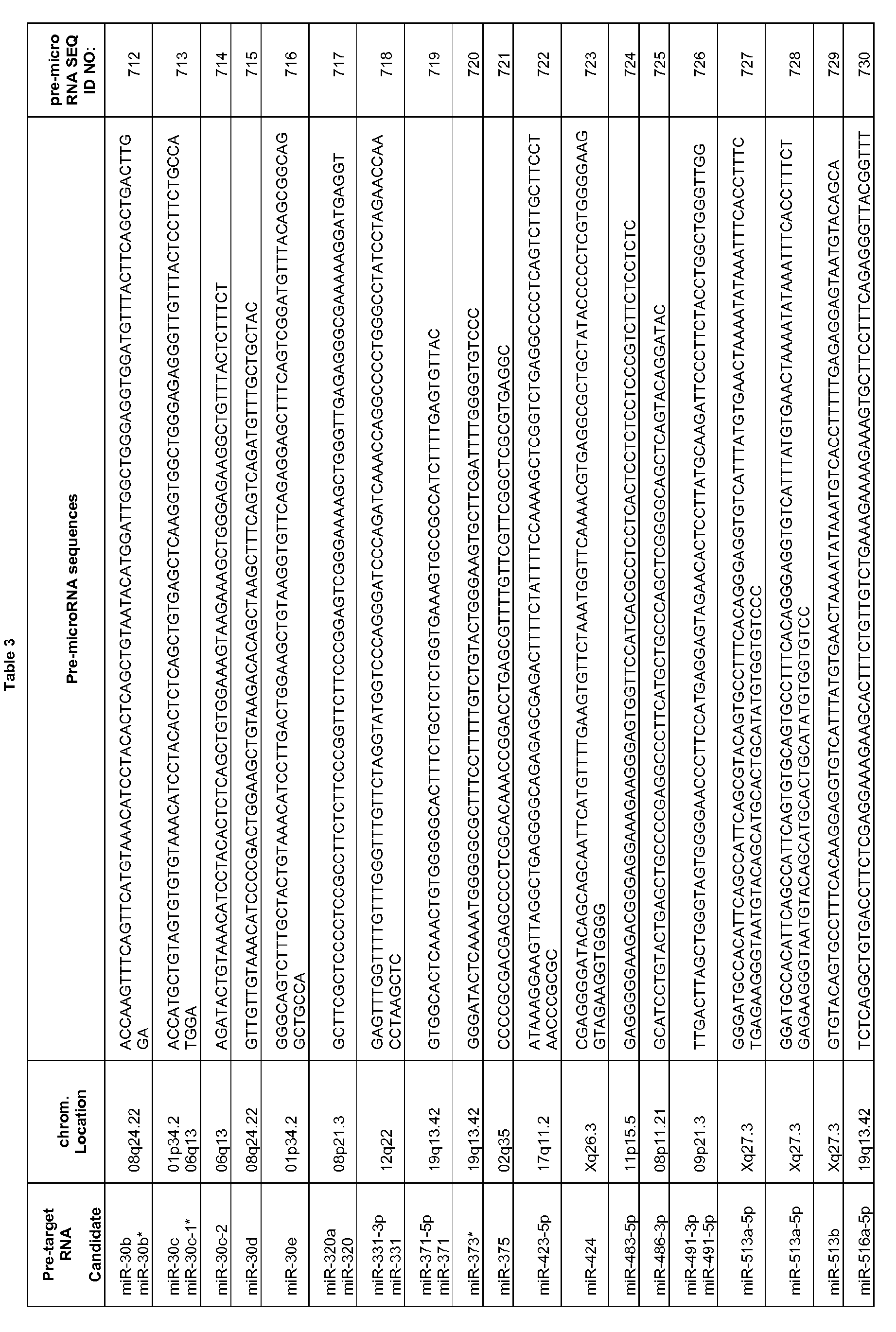

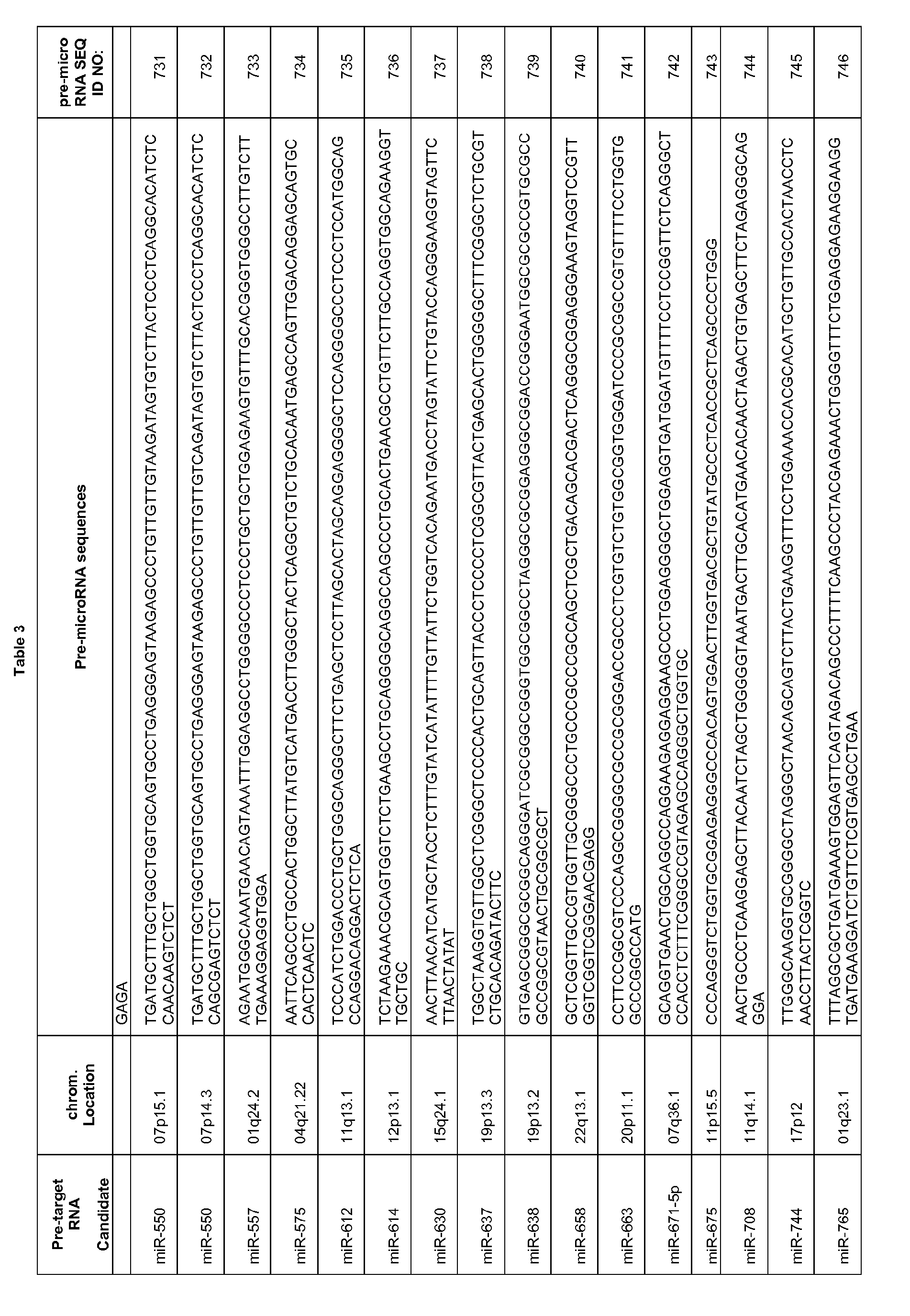

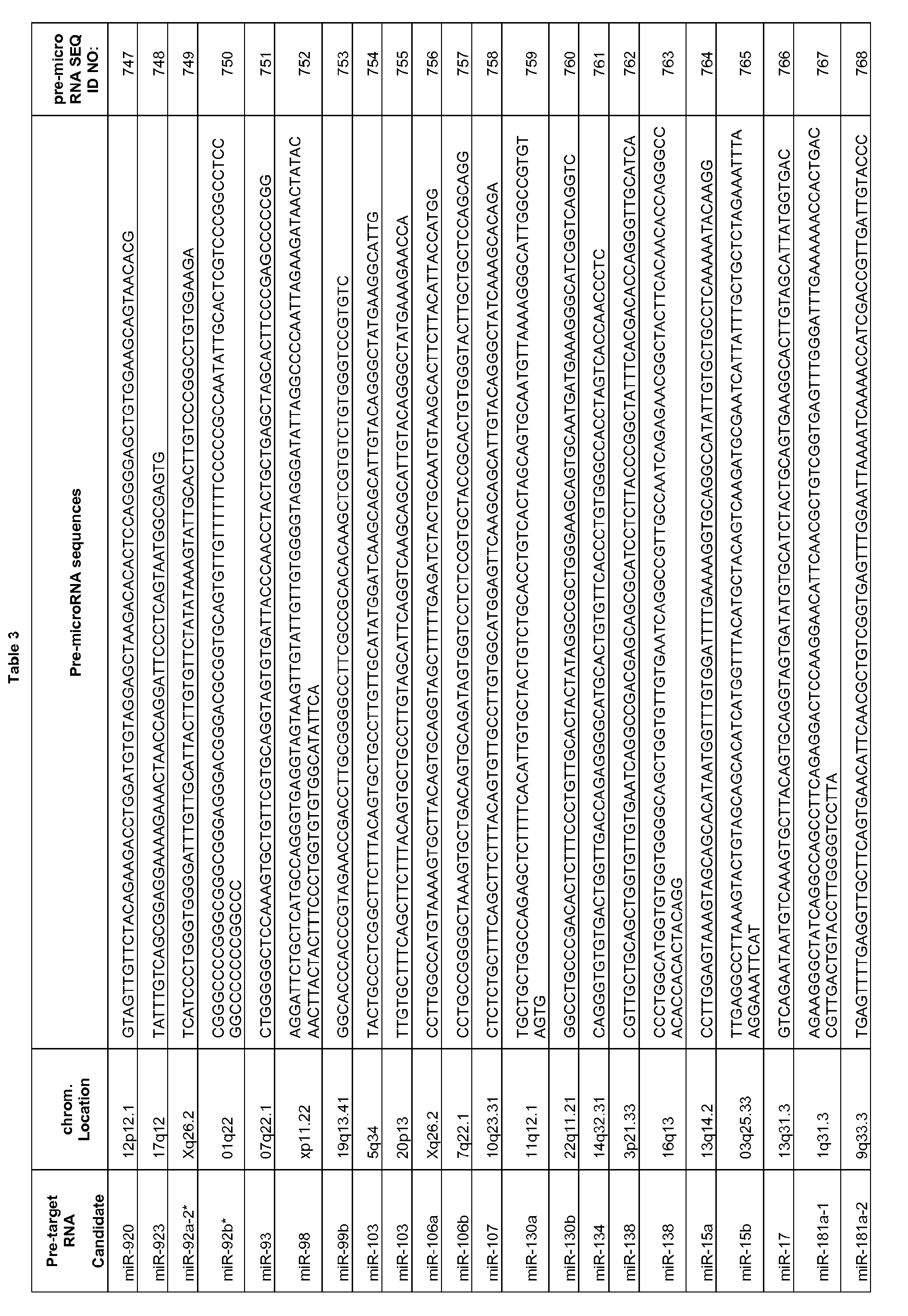

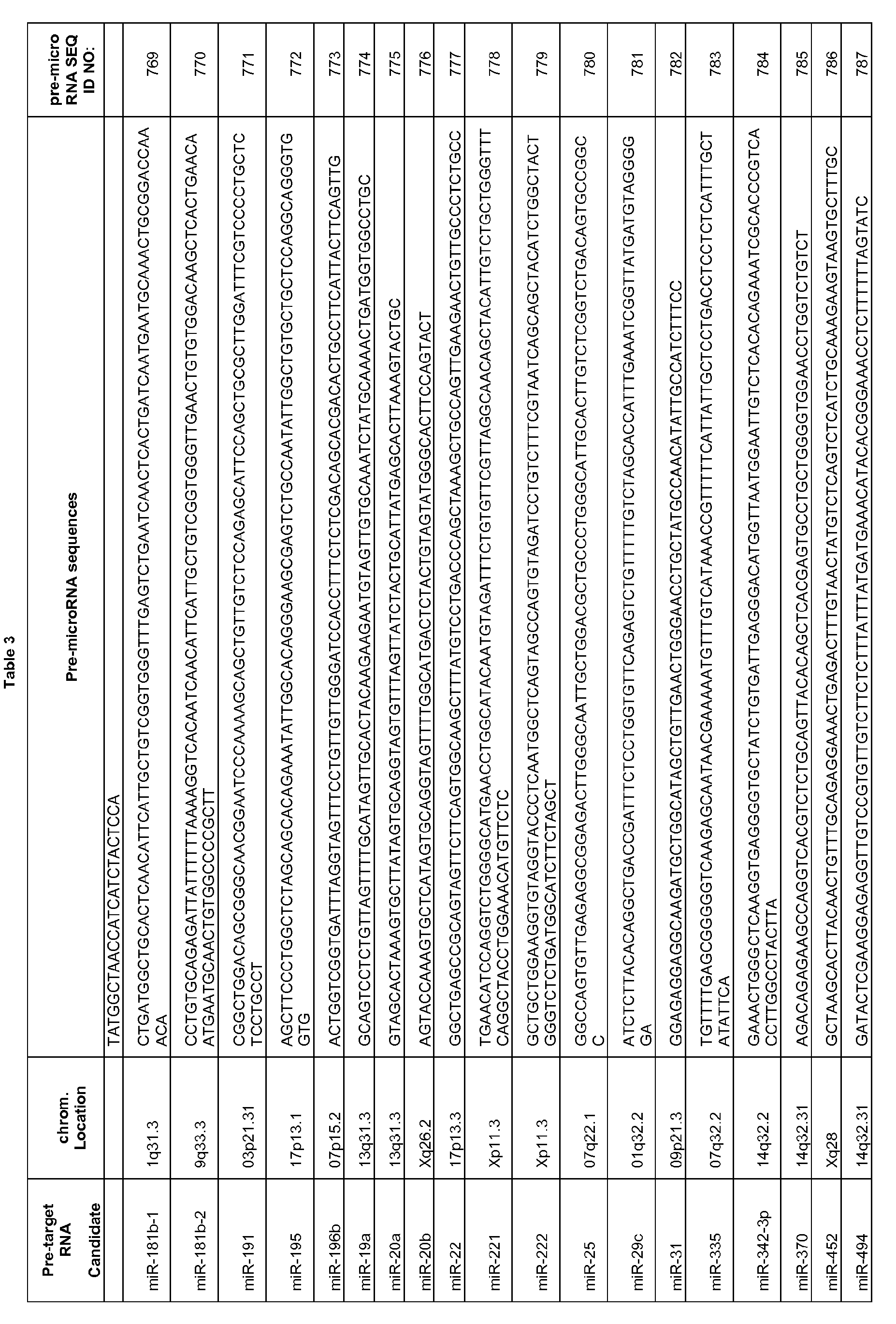

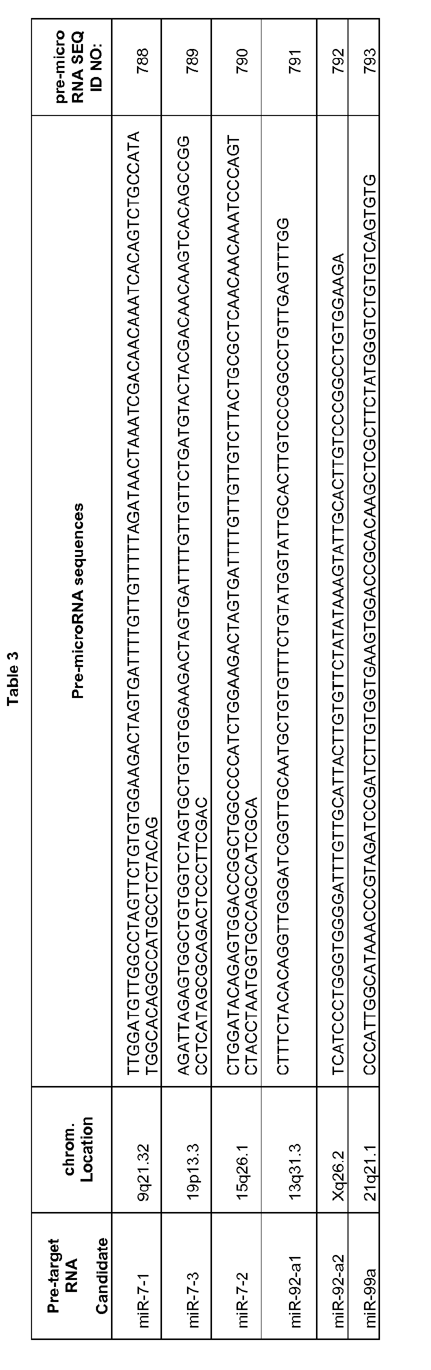

| **Table 4** | | |
|---|---|---|
| **Mature microRNA Sequences (5' to 3')** | | |
| **microRNA** | **sequence** | **SEQ ID NO** |
| let-7a | UGAGGUAGUAGGUUGUAUAGUU | 794 |
| let-7b | UGAGGUAGUAGGUUGUGUGGUU | 795 |
| let-7c | UGAGGUAGUAGGUUGUAUGGUU | 796 |
| let-7d | AGAGGUAGUAGGUUGCAUAGUU | 797 |
| let-7e | UGAGGUAGGAGGUUGUAUAGUU | 798 |
| let-7f | UGAGGUAGUAGAUUGUAUAGUU | 799 |
| let-7a | UGAGGUAGUAGUUUGUACAGUU | 800 |
| let-7i | UGAGGUAGUAGUUUGUGCUGUU | 801 |
| miR-100 | AACCCGUAGAUCCGAACUUGUG | 802 |
| miR-103 | AGCAGCAUUGUACAGGGCUAUGA | 803 |
| miR-106a | AAAAGUGCUUACAGUGCAGGUAG | 804 |
| miR-106b | UAAAGUGCUGACAGUGCAGAU | 805 |
| miR-107 | AGCAGCAUUGUACAGGGCUAUCA | 806 |
| miR-1224-5p | GUGAGGACUCGGGAGGUGG | 807 |
| miR-1225-5p | GUGGGUACGGCCCAGUGGGGGG | 808 |
| miR-1228* | GUGGGCGGGGGCAGGUGUGUG | 809 |
| miR-125a-5p | UCCCUGAGACCCUUUAACCUGUGA | 810 |
| miR-125b | UCCCUGAGACCCUAACUUGUGA | 811 |
| miR-126 | UCGUACCGUGAGUAAUAAUGCG | 812 |
| miR-130a | CAGUGCAAUGUUAAAAGGGCAU | 813 |
| miR-130b | CAGUGCAAUGAUGAAAGGGCAU | 814 |
| miR-134 | UGUGACUGGUUGACCAGAGGGG | 815 |
| miR-138 | AGCUGGUGUUGUGAAUCAGGCCG | 816 |
| miR-135a* | UAUAGGGAUUGGAGCCGUGGCG | 817 |
| miR-142-3p | UGUAGUGUUUCCUACUUUAUGGA | 818 |
| miR-145 | GUCCAGUUUUCCCAGGAAUCCCU | 819 |
| miR-146b-5p | UGAGAACUGAAUUCCAUAGGCU | 820 |
| miR-149* | AGGGAGGGACGGGGGCUGUGC | 821 |
| miR-150* | CUGGUACAGGCCUGGGGGACAG | 822 |
| miR-155 | UUAAUGCUAAUCGUGAUAGGGGU | 823 |
| miR-15a | UAGCAGCACAUAAUGGUUUGUG | 824 |
| miR-15b | UAGCAGCACAUCAUGGUUUACA | 825 |
| miR-16 | UAGCAGCACGUAAAUAUUGGCG | 826 |
| miR-17 | CAAAGUGCUUACAGUGCAGGUAG | 827 |
| miR-181a | AACAUUCAACGCUGUCGGUGAGU | 828 |
| miR-181b | AACAUUCAUUGCUGUCGGUGGGU | 829 |
| miR-181c | AACAUUCAACCUGUCGGUGAGU | 830 |
| miR-191 | CAACGGAAUCCCAAAAGCAGCUG | 831 |
| miR-195 | UAGCAGCACAGAAAUAUUGGC | 832 |
| miR-196b | UAGGUAGUUUCCUGUUGUUGGG | 833 |
| miR-198 | GGUCCAGAGGGGAGAUAGGUUC | 834 |
| miR-199a-3p | ACAGUAGUCUGCACAUUGGUUA | 835 |
| miR-199b-3p | ACAGUAGUCUGCACAUUGGUUA | 836 |
| miR-19a | UGUGCAAAUCUAUGCAAAACUGA | 837 |
| miR-19b | UGUGCAAAUCCAUGCAAAACUGA | 838 |
| miR-200b | UAAUACUGCCUGGUAAUGAUGA | 839 |
| miR-200c | UAAUACUGCCGGGUAAUGAUGGA | 840 |
| miR-205 | UCCUUCAUUCCACCGGAGUCUG | 841 |
| miR-20a | UAAAGUGCUUAUAGUGCAGGUAG | 842 |
| miR-20b | CAAAGUGCUCAUAGUGCAGGUAG | 843 |
| miR-21 | UAGCUUAUCAGACUGAUGUUGA | 844 |
| miR-22 | AAGCUGCCAGUUGAAGAACUGU | 845 |
| miR-221 | AGCUACAUUGUCUGCUGGGUUUC | 846 |
| miR-222 | AGCUACAUCUGGCUACUGGGU | 847 |
| miR-23a | AUCACAUUGCCAGGGAUUUCC | 848 |
| miR-23a* | GGGGUUCCUGGGGAUGGGAUUU | 849 |
| miR-23b | AUCACAUUGCCAGGGAUUACC | 850 |
| miR-24 | UGGCUCAGUUCAGCAGGAACAG | 851 |
| miR-25 | CAUUGCACUUGUCUCGGUCUGA | 852 |
| miR-25* | AGGCGGAGACUUGGGCAAUUG | 853 |
| miR-26a | UUCAAGUAAUCCAGGAUAGGCU | 854 |
| miR-26b | UUCAAGUAAUUCAGGAUAGGU | 855 |
| miR-27a | UUCACAGUGGCUAAGUUCCGC | 856 |
| miR-27b | UUCACAGUGGCUAAGUUCUGC | 857 |
| miR-298 | AGCAGAAGCAGGGAGGUUCUCCCA | 858 |
| miR-29a | UAGCACCAUCUGAAAUCGGUUA | 859 |
| miR-29b | UAGCACCAUUUGAAAUCAGUGUU | 860 |
| miR-29c | UAGCACCAUUUGAAAUCGGUUA | 861 |
| miR-29c* | UGACCGAUUUCUCCUGGUGUUC | 862 |
| miR-30a | UGUAAACAUCCUCGACUGGAAG | 863 |
| miR-30b | UGUAAACAUCCUACACUCAGCU | 864 |
| miR-30b* | CUGGGAGGUGGAUGUUUACUUC | 865 |
| miR-30c | UGUAAACAUCCUACACUCUCAGC | 866 |
| miR-30c-1* | CUGGGAGAGGGUUGUUUACUCC | 867 |
| miR-30c-2 | UGUAAACAUCCUACACUCUCAGC | 868 |
| miR-30d | UGUAAACAUCCCCGACUGGAAG | 869 |
| miR-30e | UGUAAACAUCCUUGACUGGAAG | 870 |
| miR-31 | AGGCAAGAUGCUGGCAUAGCU | 871 |
| miR-320a | AAAAGCUGGGUUGAGAGGGCGA | 872 |
| miR-331-3p | GCCCCUGGGCCUAUCCUAGAA | 873 |
| miR-335 | UCAAGAGCAAUAACGAAAAAUGU | 874 |
| miR-342-3p | UCUCACACAGAAAUCGCACCCGU | 875 |
| miR-370 | GCCUGCUGGGGUGGAACCUGGU | 876 |
| miR-371-5p | ACUCAAACUGUGGGGGCACU | 877 |
| miR-373* | ACUCAAAAUGGGGGCGCUUUCC | 878 |
| miR-375 | UUUGUUCGUUCGGCUCGCGUGA | 879 |
| miR-423-5p | UGAGGGGCAGAGAGCGAGACUUU | 880 |
| miR-424 | CAGCAGCAAUUCAUGUUUUGAA | 881 |
| miR-452 | AACUGUUUGCAGAGGAAACUGA | 882 |
| miR-483-5p | AAGACGGGAGGAAAGAAGGGAG | 883 |
| miR-486-3p | CGGGGCAGCUCAGUACAGGAU | 884 |
| miR-491-3p | CUUAUGCAAGAUUCCCUUCUAC | 885 |
| miR-491-5p | AGUGGGGAACCCUUCCAUGAGG | 886 |
| miR-494 | UGAAACAUACACGGGAAACCUC | 887 |
| miR-513a-5p | UUCACAGGGAGGUGUCAU | 888 |
| miR-513b | UUCACAAGGAGGUGUCAUUUAU | 889 |
| miR-516a-5p | UUCUCGAGGAAAGAAGCACUUUC | 890 |
| miR-550 | AGUGCCUGAGGGAGUAAGAGCCC | 891 |
| miR-557 | GUUUGCACGGGUGGGCCUUGUCU | 892 |
| miR-575 | GAGCCAGUUGGACAGGAGC | 893 |
| miR-612 | GCUGGGCAGGGCUUCUGAGCUCCUU | 894 |
| miR-614 | GAACGCCUGUUCUUGCCAGGUGG | 895 |
| miR-630 | AGUAUUCUGUACCAGGGAAGGU | 896 |
| miR-637 | ACUGGGGGCUUUCGGGCUCUGCGU | 897 |
| miR-638 | AGGGAUCGCGGGCGGGUGGCGGCCU | 898 |
| miR-658 | GGCGGAGGGAAGUAGGUCCGUUGGU | 899 |
| miR-663 | AGGCGGGGCGCCGCGGGACCGC | 900 |
| miR-671-5p | AGGAAGCCCUGGAGGGGCUGGAG | 901 |
| miR-675 | UGGUGCGGAGAGGGCCCACAGUG | 902 |
| miR-7 | UGGAAGACUAGUGAUUUUGUUGU | 903 |
| miR-708 | AAGGAGCUUACAAUCUAGCUGGG | 904 |
| miR-744 | UGCGGGGCUAGGGCUAACAGCA | 905 |
| miR-765 | UGGAGGAGAAGGAAGGUGAUG | 906 |
| miR-920 | GGGGAGCUGUGGAAGCAGUA | 907 |
| miR-923 | GUCAGCGGAGGAAAAGAAACU | 908 |
| miR-92a | UAUUGCACUUGUCCCGGCCUGU | 909 |
| miR-92a-2* | GGGUGGGGAUUUGUUGCAUUAC | 910 |
| miR-92b* | AGGGACGGGACGCGGUGCAGUG | 911 |
| miR-93 | CAAAGUGCUGUUCGUGCAGGUAG | 912 |
| miR-98 | UGAGGUAGUAAGUUGUAUUGUU | 913 |
| miR-99a | AACCCGUAGAUCCGAUCUUGUG | 914 |
| miR-99b | CACCCGUAGAACCGACCUUGCG | 915 |
| miR-200a | ACAUCGUUACCAGACAGUGUUA | 916 |
| miR-720 | UGGAGGCCCCAGCGAGA | 917 |
| miR-1202 | CUCCCCCACUGCAGCUGGCAC | 918 |
| miR-1249 | UGAAGAAGGGGGGGAAGGGCGU | 919 |
| miR-1275 | GACAGCCUCUCCCCCAC | 920 |
| miR-129-3p | AUGCUUUUUGGGGUAAGGGCUU | 921 |
| miR-1321 | AUCACAUUCACCUCCCUG | 922 |
| miR-1323 | AGAAAAUGCCCCUCAGUUUUGA | 923 |
| miR-376c | ACGUGGAAUUUCCUCUAUGUU | 924 |
| miR-429 | ACGGUUUUACCAGACAGUAUUA | 925 |

**Table 5**

| **Mature microRNA Sequences (5'-3')** | | |
|---|---|---|
| **microRNA** | **sequence** | **SEQ ID NO** |
| miR-1 | UGGAAUGUAAAGAAGUAUGUA | 926 |
| miR-9 | UCUUUGGUUAUCUAGCUGUAUGA | 927 |
| miR-9* | UAAAGCUAGAUAACCGAAAGU | 928 |
| miR-10a | UACCCUGUAGAUCCGAAUUUGUG | 929 |
| miR-10b | UACCCUGUAGAACCGAAUUUGU | 930 |
| miR-17-3p | ACUGCAGUGAAGGCACUUGU | 931 |
| miR-18 | UAAGGUGCAUCUAGUGCAGAUA | 932 |
| miR-20 | UAAAGUGCUUAUAGUGCAGGUA | 933 |
| miR-28 | AAGGAGCUCACAGUCUAUUGAG | 934 |
| miR-30a-3p | CUUUCAGUCGGAUGUUUGCAGC | 935 |
| miR-32 | UAUUGCACAUUACUAAGUUGC | 936 |
| miR-33 | GUGCAUUGUAGUUGCAUUG | 937 |
| miR-34a | UGGCAGUGUCUUAGCUGGUUGU | 938 |
| miR-34b | AGGCAGUGUCAUUAGCUGAUUG | 939 |
| miR-34c | AGGCAGUGUAGUUAGCUGAUUG | 940 |
| miR-92 | UAUUGCACUUGUCCCGGCCUGU | 941 |
| miR-95 | UUCAACGGGUAUUUAUUGAGCA | 942 |
| miR-96 | UUUGGCACUAGCACAUUUUUGC | 943 |
| miR-101 | UACAGUACUGUGAUAACUGAAG | 944 |
| miR-105 | UCAAAUGCUCAGACUCCUGU | 945 |
| miR-122a | UGGAGUGUGACAAUGGUGUUUGU | 946 |
| miR-124a | UUAAGGCACGCGGUGAAUGCCA | 947 |
| miR-126* | CAUUAUUACUUUUGGUACGCG | 948 |
| miR-127 | UCGGAUCCGUCUGAGCUUGGCU | 949 |
| miR-128a | UCACAGUGAACCGGUCUCUUUU | 950 |
| miR-128b | UCACAGUGAACCGGUCUCUUUC | 951 |
| miR-129 | CUUUUUGCGGUCUGGGCUUGC | 952 |
| miR-132 | UAACAGUCUACAGCCAUGGUCG | 953 |
| miR-133a | UUGGUCCCCUUCAACCAGCUGU | 954 |
| miR-133b | UUGGUCCCCUUCAACCAGCUA | 955 |
| miR-135a | UAUGGCUUUUUAUUCCUAUGUGA | 956 |
| miR-135b | UAUGGCUUUUCAUUCCUAUGUG | 957 |
| miR-136 | ACUCCAUUUGUUUUGAUGAUGGA | 958 |
| miR-137 | UAUUGCUUAAGAAUACGCGUAG | 959 |
| miR-139 | UCUACAGUGCACGUGUCU | 960 |
| miR-140 | AGUGGUUUUACCCUAUGGUAG | 961 |
| miR-141 | AACACUGUCUGGUAAAGAUGG | 962 |
| miR-142-5p | CAUAAAGUAGAAAGCACUAC | 963 |
| miR-143 | UGAGAUGAAGCACUGUAGCUCA | 964 |
| miR-144 | UACAGUAUAGAUGAUGUACUAG | 965 |
| miR-146 | UGAGAACUGAAUUCCAUGGGUU | 966 |
| miR-147 | GUGUGUGGAAAUGCUUCUGC | 967 |
| miR-148a | UCAGUGCACUACAGAACUUUGU | 968 |
| miR-148b | UCAGUGCAUCACAGAACUUUGU | 969 |
| miR-149 | UCUGGCUCCGUGUCUUCACUCC | 970 |
| miR-150 | UCUCCCAACCCUUGUACCAGUG | 971 |
| miR-151 | ACUAGACUGAAGCUCCUUGAGG | 972 |
| miR-152 | UCAGUGCAUGACAGAACUUGG | 973 |
| miR-153 | UUGCAUAGUCACAAAAGUGA | 974 |
| miR-154 | UAGGUUAUCCGUGUUGCCUUCG | 975 |
| miR-154* | AAUCAUACACGGUUGACCUAUU | 976 |
| miR-182 | UUUGGCAAUGGUAGAACUCACA | 977 |
| miR-182^{*} | UGGUUCUAGACUUGCCAACUA | 978 |
| miR-183 | UAUGGCACUGGUAGAAUUCACUG | 979 |
| miR-184 | UGGACGGAGAACUGAUAAGGGU | 980 |
| miR-185 | UGGAGAGAAAGGCAGUUC | 981 |
| miR-186 | CAAAGAAUUCUCCUUUUGGGCUU | 982 |
| miR-187 | UCGUGUCUUGUGUUGCAGCCG | 983 |
| miR-188 | CAUCCCUUGCAUGGUGGAGGGU | 984 |
| miR-189 | GUGCCUACUGAGCUGAUAUCAGU | 985 |
| miR-190 | UGAUAUGUUUGAUAUAUUAGGU | 986 |
| miR-192 | CUGACCUAUGAAUUGACAGCC | 987 |
| miR-193 | AACUGGCCUACAAAGUCCCAG | 988 |
| miR-194 | UGUAACAGCAACUCCAUGUGGA | 989 |
| miR-196a | UAGGUAGUUUCAUGUUGUUGG | 990 |
| miR-197 | UUCACCACCUUCUCCACCCAGC | 991 |
| miR-200a | UAACACUGUCUGGUAACGAUGU | 992 |
| miR-202 | AGAGGUAUAGGGCAUGGGAAGA | 993 |
| miR-203 | GUGAAAUGUUUAGGACCACUAG | 994 |
| miR-204 | UUCCCUUUGUCAUCCUAUGCCU | 995 |
| miR-206 | UGGAAUGUAAGGAAGUGUGUGG | 996 |
| miR-208 | AUAAGACGAGCAAAAAGCUUGU | 997 |
| miR-210 | CUGUGCGUGUGACAGCGGCUG | 998 |
| miR-211 | UUCCCUUUGUCAUCCUUCGCCU | 999 |
| miR-212 | UAACAGUCUCCAGUCACGGCC | 1000 |
| miR-213 | ACCAUCGACCGUUGAUUGUACC | 1001 |
| miR-214 | ACAGCAGGCACAGACAGGCAG | 1002 |
| miR-215 | AUGACCUAUGAAUUGACAGAC | 1003 |
| miR-216 | UAAUCUCAGCUGGCAACUGUG | 1004 |
| miR-217 | UACUGCAUCAGGAACUGAUUGGAU | 1005 |
| miR-218 | UUGUGCUUGAUCUAACCAUGU | 1006 |
| miR-219 | UGAUUGUCCAAACGCAAUUCU | 1007 |
| miR-220 | CCACACCGUAUCUGACACUUU | 1008 |
| miR-223 | UGUCAGUUUGUCAAAUACCCC | 1009 |
| miR-224 | CAAGUCACUAGUGGUUCCGUUUA | 1010 |
| miR-296 | AGGGCCCCCCCUCAAUCCUGU | 1011 |
| miR-299 | UGGUUUACCGUCCCACAUACAU | 1012 |
| miR-301 | CAGUGCAAUAGUAUUGUCAAAGC | 1013 |
| miR-302a | UAAGUGCUUCCAUGUUUUGGUGA | 1014 |
| miR-302b* | ACUUUAACAUGGAAGUGCUUUCU | 1015 |
| miR-302b | UAAGUGCUUCCAUGUUUUAGUAG | 1016 |
| miR-302c* | UUUAACAUGGGGGUACCUGCUG | 1017 |
| miR-302c | UAAGUGCUUCCAUGUUUCAGUGG | 1018 |
| miR-302d | UAAGUGCUUCCAUGUUUGAGUGU | 1019 |
| miR-320 | AAAAGCUGGGUUGAGAGGGCGAA | 1020 |
| miR-321 | UAAGCCAGGGAUUGUGGGUUC | 1021 |
| miR-323 | GCACAUUACACGGUCGACCUCU | 1022 |
| miR-324-5p | CGCAUCCCCUAGGGCAUUGGUGU | 1023 |
| miR-324-3p | CCACUGCCCCAGGUGCUGCUGG | 1024 |
| miR-325 | CCUAGUAGGUGUCCAGUAAGU | 1025 |
| miR-326 | CCUCUGGGCCCUUCCUCCAG | 1026 |
| miR-328 | CUGGCCCUCUCUGCCCUUCCGU | 1027 |
| miR-330 | GCAAAGCACACGGCCUGCAGAGA | 1028 |
| miR-331 | GCCCCUGGGCCUAUCCUAGAA | 1029 |
| miR-337 | UCCAGCUCCUAUAUGAUGCCUUU | 1030 |
| miR-338 | UCCAGCAUCAGUGAUUUUGUUGA | 1031 |
| miR-339 | UCCCUGUCCUCCAGGAGCUCA | 1032 |
| miR-340 | UCCGUCUCAGUUACUUUAUAGCC | 1033 |
| miR-342 | UCUCACACAGAAAUCGCACCCGUC | 1034 |
| miR-345 | UGCUGACUCCUAGUCCAGGGC | 1035 |
| miR-346 | UGUCUGCCCGCAUGCCUGCCUCU | 1036 |
| miR-367 | AAUUGCACUUUAGCAAUGGUGA | 1037 |
| miR-368 | ACAUAGAGGAAAUUCCACGUUU | 1038 |
| miR-369 | AAUAAUACAUGGUUGAUCUUU | 1039 |
| miR-371 | GUGCCGCCAUCUUUUGAGUGU | 1040 |
| miR-372 | AAAGUGCUGCGACAUUUGAGCGU | 1041 |
| miR-373 | GAAGUGCUUCGAUUUUGGGGUGU | 1042 |
| miR-374 | UUAUAAUACAACCUGAUAAGUG | 1043 |
| miR-320c | AAAAGCUGGGUUGAGAGGGU | 2692 |

**Table 6**

| **Up-requlated target RNAs for detection of non-small cell lung cancer** | | | | | | | | | | | |
|---|---|---|---|---|---|---|---|---|---|---|---|
| | | **Fold changes v. normal Lung** | | | | | | | | | |
| **Array Probe** | **Probe SEQ ID NO.** | **Epi4** | **Epi7** | **Epi5** | **Adk1** | **Adk3** | **Adk11** | **Adk8** | **Adk9** | **Adk2** | **Adk10** |
| miR-21 | 196 | -2.52 | 1.77 | -1.51 | 1.45 | 1.41 | **3.51** | **2.27** | **3.43** | **5.38** | 1.25 |
| miR-765 | 246 | **25.46** | **3.15** | **3.08** | 6.06 | -1.58 | **3.70** | 1.82 | **3.00** | -1.58 | 1.02 |
| 4037-R3-2 | 15 | **6.57** | 1.44 | -1.28 | **2.17** | -1.28 | **2.17** | **2.34** | **2.64** | **6.81** | -1.06 |
| miR-27b | 205 | -1.11 | **2.20** | **2.52** | -1.91 | 1.18 | 1.64 | 1.24 | **2.05** | **6.50** | -1.85 |
| miR-29a | 207 | -2.68 | 1.00 | **3.18** | 1.50 | -1.90 | **2.13** | 1.41 | 1.23 | **2.18** | -2.96 |
| miR-923 | 248 | **6.70** | 1.19 | 1.24 | 1.35 | -2.18 | 1.11 | -1.43 | 1.19 | **2.00** | -2.79 |
| miR-199a-3p | 191 | -3.52 | 1.40 | 1.43 | -1.94 | 1.72 | 1.56 | 1.33 | 1.59 | **2.97** | -4.20 |
| miR-331-3p | 219 | -1.21 | -1.21 | **2.17** | 1.42 | 1.24 | 1.47 | 1.27 | -1.21 | -1.21 | 1.36 |
| miR-23a | 197 | 1.38 | **2.90** | -4.40 | 1.37 | -4.40 | **2.28** | -4.40 | **2.38** | **7.76** | -2.09 |
| miR-146b-5p | 184 | -1.89 | 1.12 | **2.44** | -1.89 | -1.89 | **2.71** | 1.07 | 1.30 | **2.48** | -1.21 |
| miR-483-5p | 225 | **24.38** | 1.51 | 1.68 | **5.17** | -1.54 | 1.11 | -1.64 | **3.19** | -1.54 | -1.03 |
| 9733-L3-1 | 129 | **7.61** | **3.56** | -1.48 | **2.44** | -1.48 | -1.48 | 1.07 | 1.69 | -1.48 | 1.33 |
| miR-30c-1* | 214 | **39.98** | -1.58 | 1.57 | **7.84** | -1.58 | -1.58 | -1.42 | **2.67** | -1.58 | -1.58 |
| 4593-R3-1 | 26 | **8.31** | **2.80** | 1.23 | 1.93 | -4.73 | -2.65 | -5.00 | 1.79 | -9.77 | 1.20 |
| 8433 C-R4-1 | 164 | **24.29** | 1.73 | 1.87 | **5.70** | -1.45 | -1.45 | -1.45 | 1.95 | -1.45 | 1.13 |
| 4855-R3-1 | 30 | **8.26** | 1.91 | 1.44 | 1.78 | -3.56 | -1.50 | -1.81 | 1.87 | -2.80 | 1.20 |
| 4666-R4-1 | 27 | **24.77*** | 2.27 | **3.49** * | **7.40** * | -2.36 | -1.70 | -2.05 | 2.37 | -3.62 | -1.01 |

**Table 7: Target RNAs more frequently present at elevated levels in squamous cell carcinoma (SCC)**

| **Gene** | **Fold change average in SCC** | **Number of adenocarcino mas with increased levels** | **Number of SCC with increased levels** | **Probe SEQ ID NO** | **Pre-microRNA SEQ ID NO** | **microRNA SEQ ID NO** |
|---|---|---|---|---|---|---|
| 10366-R3-2 | 4.1 | 1 | 6 | 145 | 539 | |
| 12223-L5-1 | 3.2 | 2 | 5 | 2110 | 2234 | 2584 |
| 12907-L5-1 | 6.4 | 0 | 5 | 1106 | 1228 | |
| 12911-L5-1 | 3.2 | 2 | 5 | 2115 | 2239 | 2590 |
| 12917-R5-2 | 6.0 | 1 | 6 | 1108 | 1230 | |
| 13108-L5-2 | 3.3 | 1 | 5 | 2673 | 2681 | 2595 |
| 13122-L5-1 | 11.5 | 2 | 7 | 1066 | 1242 | 2597 |
| 13272-R5-2 | 4.0 | 1 | 5 | 2674 | 2682 | 2611 |
| 13316-R5-2 | 2.8 | 0 | 5 | 2675 | 2683 | 2616 |
| 13331-L5-2 | 4.1 | 1 | 5 | 2676 | 2684 | 2617 |
| 13499-R5-1 | 3.3 | 2 | 5 | 2677 | 2685 | 2634 |
| 3923-R5-1 | 5.3 | 3 | 7 | 1070 | 1273 | 2647 |
| 4261-R5-1 | 6.4 | 0 | 5 | 1148 | 1277 | |
| 4479-R3-1 | 4.3 | 1 | 6 | 25 | 421 | |
| 5232-L5-2 | 5.2 | 0 | 5 | 1154 | 1287 | 2653 |
| 5392-R5-1 | 7.0 | 2 | 5 | 1155 | 1288 | 2654 |
| 5971-R5-2 | 2.6 | 1 | 5 | 2678 | 2686 | 2657 |
| 6183-R5-1 | 2.8 | 0 | 5 | 2149 | 2274 | |
| 7026-L3-1 | 2.8 | 1 | 5 | 2679 | 2687 | |
| 7292-L3-2 | 3.5 | 2 | 6 | 80 | 476 | |
| 7471-L5-1 | 4.5 | 1 | 5 | 2680 | 2688 | |
| 8004-R3-2 | 11.0 | 2 | 6 | 97 | 492 | |
| 8316-R5-1 | 20.3 | 1 | 5 | 1177 | 1315 | |
| 9349-R5-2 | 24.4 | 3 | 6 | 1080 | 1325 | 2672 |
| 9594-R5-1 | 9.7 | 2 | 6 | 1081 | 1328 | |
| miR-1323 | 4.9 | 2 | 6 | 1197 | 1338 | 923 |
| miR-205 | 21.2 | 3 | 7 | 195 | 694 | 841 |
| miR-675 | 2.9 | 2 | 5 | 243 | 743 | 902 |
| miR-923 | 3.5 | 3 | 6 | 248 | 748 | 908 |

**Table 8: Target RNAs more frequently present at elevated levels in adenocarcinoma**

| **Gene** | **Fold change average in adenocarcinoma** | **Number of adenocarcinomas with increased levels** | **Number of SCC with increased levels** | **Probe SEQ ID NO** | **Pre-microRNA SEQ ID NO** | **microRNA SEQ ID NO** |
|---|---|---|---|---|---|---|
| 13252-L5-3 | 4.9 | 5 | 1 | 1123 | 1249 | 2609 |
| 13373-R5-2 | 5.1 | 5 | 3 | 1130 | 1257 | 2624 |
| 9798-L5-1 | 9.9 | 5 | 3 | 1188 | 1329 | |
| miR-106b | 4.5 | 5 | 2 | 363 | 757 | 805 |
| miR-20b | 4.8 | 6 | 2 | 1086 | 1349 | 843 |
| miR-92a | 4.0 | 4 | 0 | 2180 | 2307 | 909 |
| miR-93 | 3.8 | 5 | 1 | 251 | 751 | 912 |

**Table 9: Target RNAs present at increased levels in aggressive forms of lung cancer**

| **Gene** | **Probe SEQ ID NO** | **Pre-microRNA SEQ ID NO(s)** | **microRNA SEQ ID NO** |
|---|---|---|---|
| 10083-L5-1 | 1090 | 1211 | |
| 10233-R5-1 | 1091 | 1212 | 2576 |
| 10455-L5-1 | 1063 | 1215 | 2578 |
| 11444-L5-3 | 1097 | 1219 | |
| 12729-R5-1 | 1103 | 1225 | |
| 12888-L5-2 | 1105 | 1227 | |
| 12907-L5-1 | 1106 | 1228 | |
| 12917-R5-2 | 1108 | 1230 | |
| 12947-L5-4 | 1064 | 1231 | |
| 12974-R5-2 | 1110 | 1233 | 2592 |
| 12979-R5-2 | 1111 | 1234 | |
| 13001-L5-1 | 1113 | 1236 | |
| 13070-R5-3 | 1115 | 1238 | |
| 13122-L5-1 | 1066 | 1242 | 2597 |
| 13185-L5-3 | 1118 | 1243 | 2603 |
| 13219-L5-1 | 1067 | 1245 | |
| 13245-L5-4 | 1122 | 1248 | 2607 |
| 13274-L5-3 | 1124 | 1251 | 2612 |
| 13357-L5-4 | 1128 | 1255 | 2621 |
| 13398-R5-4 | 1132 | 1259 | 2627 |
| 13467-L5-1 | 1069 | 1262 | 2630 |
| 13468-L5-1 | 1135 | 1263 | 2631 |
| 13470-R5-1 | 1136 | 1264 | |
| 13473-L5-3 | 1137 | 1265 | 2633 |
| 13500-L5-3 | 138 | 1266 | 2635,2636 |
| 3744-R5-1 | 1143 | 1271 | 2645,2646 |
| 3875-R5-2 | 1144 | 1272 | |
| 3992-R5-1 | 1146 | 1275 | |
| 4790-L5-2 | 1150 | 1282 | |
| 5080-R3-1 | 1073 | 1285 | 2650 |
| 5108-R5-2 | 1153 | 1286 | |
| 5392-R5-1 | 1155 | 1288 | 2654 |
| 6037-R3-2 | 1159 | 1292 | 2658 |
| 6181-L5-1 | 1160 | 1293 | |
| 6233-L5-2 | 1161 | 1295 | |
| 6235-R5-2 | 1075 | 1296 | 2661 |
| 6474-L5-1 | 1164 | 1299 | |
| 6602-R3-1 | 1165 | 1300 | |
| 6683-R5-1 | 1167 | 1302 | |
| 6906-L5-1 | 1172 | 1307 | 2666 |
| 6930-R5-1 | 1173 | 1308 | 2667 |
| 7764-R3-2 | 1175 | 1312 | |
| 8004-R3-2 | 97 | 492 | |
| 8316-R5-1 | 1177 | 1315 | |
| 836-R5-2 | 1079 | 1316 | |
| 8433_C-R4-1 | 1177 | 1317 | |
| 8808-R5-1 | 1183 | 1322 | |
| 9349-R5-2 | 1080 | 1325 | 2672 |
| 9594-R5-1 | 1081 | 1328 | |
| miR-198 | 1202 | 1343 | 834 |
| miR-298 | 1088 | 1351 | 858 |
| miR-30c-1* | 1204 | 1352 | 867 |
| miR-320c | 2689 | 2690, 2691 | 2692 |
| miR-516a-5p | 1209 | 1357 | 890 |
| miR-765 | 246 | 746 | 906 |

In some embodiments, target RNAs can be measured in samples collected at one or more times from a patient to monitor the status or progress of lung cancer in the patient.

In some embodiments, a sample to be tested is obtained using one or more techniques commonly used for collecting lung tissue, e.g., bronchoscopy, bronchial washing, brushing, or transbronchial needle aspiration. In some embodiments, the sample is obtained from a patient without lesions by bronchoalveolar lavage, i.e., washing the airways with saline, to obtain cells. In some embodiments, the sample is obtained by biopsy, such as computed tomography (CT)-aided needle biopsy.

In some embodiments, the sample to be tested is a bodily fluid, such as blood, sputum, mucus, saliva, urine, semen, etc. In some embodiments, a sample to be tested is a blood sample. In some embodiments, the blood sample is whole blood. In some embodiments, the blood sample is a sample of blood cells. In some embodiments, the blood sample is plasma. In some embodiments, the blood sample is serum.

The clinical sample to be tested is, in some embodiments, freshly obtained. In other embodiments, the sample is a fresh frozen specimen. In some embodiments, the sample is a tissue sample, such as a formalin-fixed paraffin embedded sample. In some embodiments, the sample is a liquid cytology sample.

In some embodiments, the methods described herein are used for early detection of lung cancer in a sample of lung cells, such as those obtained by routine bronchoscopy. In some embodiments, the methods described herein are used for early detection of lung cancer in a sample of blood or serum.

In some embodiments, the clinical sample to be tested is obtained from individuals who have one or more of the following risk factors: history of smoking, over 45 years of age, exposure to radon gas, secondhand smoke or occupational carcinogens (e.g., asbestos, radiation, arsenic, chromates, nickel, chloromethyl ethers, mustard gas, or coke-oven emissions), or lungs scarred by prior disease such as tuberculosis. In some embodiments, the clinical sample is obtained from individuals who have diagnostic signs or clinical symptoms that may be associated with lung cancer, such as abnormal chest x-ray and/or computed tomography ("CT") scan, cough, localized chest pain, or hoarseness.

Thus, in some embodiments, methods described herein can be used for routine screening of healthy individuals with no risk factors. In some embodiments, methods described herein are used to screen asymptomatic individuals having one or more of the above-described risk factors.

In some embodiments, the methods described herein can be used to assess the effectiveness of a treatment for lung cancer in a patient. In some embodiments, the target RNA expression levels are determined at various times during the treatment, and are compared to target RNA expression levels from an archival sample taken from the patient, e.g., by bronchoscopy, before the manifestation of any signs of lung cancer or before beginning treatment. In some embodiments, the target RNA expression levels are compared to target RNA expression levels from an archival sample of normal tissue taken from the patient, i.e., a sample of tissue taken from a tumor-free part of the patient's lung by biopsy. Ideally, target RNA expression levels in the normal sample evidence no aberrant changes in target RNA expression levels. Thus, in such embodiments, the progress of treatment of an individual with lung cancer can be assessed by comparison to a sample of lung cells from the same individual when he was healthy or prior to beginning treatment, or by comparison to a sample of healthy lung cells from the same individual.

In embodiments in which the method comprises detecting expression of more than one target RNA, the expression levels of the plurality of target RNAs may be detected concurrently or simultaneously in the same assay reaction. In some embodiments, expression levels are detected concurrently or simultaneously in separate assay reactions. In some embodiments, expression levels are detected at different times, e.g., in serial assay reactions.

In some embodiments, a method comprises detecting the level of at least one target RNA in a sample from a subject, wherein detection of a level of at least one target RNA that is greater than a normal level of the at least one target RNA indicates the presence of lung cancer in the subject. In some embodiments, a method comprises detecting the level of at least one target RNA in a sample from a subject and comparing the level of the at least one target RNA in the sample to a normal level of the at least one target RNA, wherein a level of at least one target RNA in the sample that is greater than a normal level of the at least one target RNA indicates the presence of lung cancer in the subj ect.

In some embodiments, a method of facilitating diagnosis of lung cancer in a subject is provided. Such methods comprise detecting the level of at least one target RNA in a sample from the subject. In some embodiments, information concerning the level of at least one target RNA in the sample from the subject is communicated to a medical practitioner. A "medical practitioner," as used herein, refers to an individual or entity that diagnoses and/or treats patients, such as a hospital, a clinic, a physician's office, a physician, a nurse, or an agent of any of the aforementioned entities and individuals. In some embodiments, detecting the level of at least one target RNA is carried out at a laboratory that has received the subject's sample from the medical practitioner or agent of the medical practitioner. The laboratory carries out the detection by any method, including those described herein, and then communicates the results to the medical practitioner. A result is "communicated," as used herein, when it is provided by any means to the medical practitioner. In some embodiments, such communication may be oral or written, may be by telephone, in person, by e-mail, by mail or other courier, or may be made by directly depositing the information into, e.g., a database accessible by the medical practitioner, including databases not controlled by the medical practitioner. In some embodiments, the information is maintained in electronic form. In some embodiments, the information can be stored in a memory or other computer readable medium, such as RAM, ROM, EEPROM, flash memory, computer chips, digital video discs (DVD), compact discs (CDs), hard disk drives (HDD), magnetic tape, etc.

In some embodiments, methods of detecting the presence lung cancer are provided. In some embodiments, methods of diagnosing lung cancer are provided. In some embodiments, the method comprises obtaining a sample from a subject and providing the sample to a laboratory for detection of at least one target RNA level in the sample. In some embodiments, the method further comprises receiving a communication from the laboratory that indicates the at least one target RNA level in the sample. In some embodiments, lung cancer is present if the level of at least one target RNA in the sample is greater than a normal level of the at least one target RNA. A "laboratory," as used herein, is any facility that detects the level of at least one target RNA in a sample by any method, including the methods described herein, and communicates the level to a medical practitioner. In some embodiments, a laboratory is under the control of a medical practitioner. In some embodiments, a laboratory is not under the control of the medical practitioner.

When a laboratory communicates the level of at least one target RNA to a medical practitioner, in some embodiments, the laboratory communicates a numerical value representing the level of at least one target RNA in the sample, with or without providing a numerical value for a normal level. In some embodiments, the laboratory communicates the level of at least one target RNA by providing a qualitative value, such as "high," "elevated," etc.

As used herein, when a method relates to detecting lung cancer, determining the presence of lung cancer, and/or diagnosing lung cancer, the method includes activities in which the steps of the method are carried out, but the result is negative for the presence of lung cancer. That is, detecting, determining, and diagnosing lung cancer include instances of carrying out the methods that result in either positive or negative results (e.g., whether target RNA levels are normal or greater than normal).

As used herein, the term "subject" means a human. In some embodiments, the methods described herein may be used on samples from non-human animals.

The common, or coordinate, expression of target RNAs that are physically proximal to one another in the genome permits the informative use of such chromosome-proximal target RNAs in methods herein.

Table 3 identifies the chromosomal location of each of the 397 target RNAs capable of specifically hybridizing to a nucleic acid comprising a sequence that is identically present in one of SEQ ID NOs: 1 to 397 in Tables 1 and 2. Table 22 identifies the chromosomal location of the target RNAs capable of specifically hybridizing to a nucleic acid comprising a sequence that is identically present in one of SEQ ID NOs: 1063 to 1210 in Tables 18 and 20. Table 25 identifies the chromosomal location of the target RNAs capable of specifically hybridizing to a nucleic acid comprising a sequence that is identically present in one of SEQ ID NOs: 1363 to 1707 in Table 23. Table 29 identifies the chromosomal location of the target RNAs capable of specifically hybridizing to a nucleic acid comprising a sequence that is identically present in one of SEQ ID NOs: 2064 to 2183 in Tables 27 and 28. Table 31 identifies the chromosomal location of the target RNAs capable of specifically hybridizing to a nucleic acid comprising a sequence that is identically present in one of SEQ ID NOs: 2312 to 2452 in Table 30. Thus, in some embodiments, the level of expression of one or more target RNAs located within about 1 kilobase (kb), within about 2 kb, within about 5 kb, within about 10 kb, within about 20 kb, within about 30 kb, within about 40 kb, and even within about 50 kb of the chromosomal locations in Tables 3, 22, 25, 29, and 31 is detected in lieu of, or in addition to, measurement of expression of the respective tabulated target RNAs in the methods described herein. *See* Baskerville, S. and Bartel D.P. (2005) RNA 11:241-247.

In some embodiments, in combination with detecting one or more target RNAs capable of specifically hybridizing to a nucleic acid comprising a sequence selected from SEQ ID NOs: 1 to 397, 1063 to 1210, 1363 to 1707, 2064 to 2183, 2312 to 2452, 2673 to 2680, and 2689 and/or detecting one or more target RNAs comprising at least 15 contiguous nucleotides of a sequence selected from SEQ ID NOs: 794 to 1043, 2576 to 2672, and 2692 and/or detecting one or more target RNAs that comprise a sequence that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689, methods herein further comprise detecting the level(s) of expression of at least one microRNA from the human miRNome.

In some embodiments, at least one target RNA is capable of specifically hybridizing to a nucleic acid comprising a sequence selected from SEQ ID NOs: 1 to 397, 1063 to 1210, 1363 to 1707, 2064 to 2183, 2312 to 2452, 2673 to 2680, and 2689. In some embodiments, at least one target RNA comprises at least 15 contiguous nucleotides that are complementary to at least a portion of a sequence selected from SEQ ID NOs: 1 to 397, 1063 to 1210, 1363 to 1707, 2064 to 2183, 2312 to 2452, 2673 to 2680, and 2689. In some embodiments, at least one target RNA comprises at least 15 contiguous nucleotides of a sequence selected from SEQ ID NOs: 794 to 1043, 2576 to 2672, and 2692. In some embodiments, a target RNA, in its mature form, comprises fewer than 30 nucleotides. In some embodiments, a target RNA is a microRNA.

In some embodiments, more than one target RNA is detected simultaneously in a single reaction. In some embodiments, at least 2, at least 3, at least 5, or at least 10 target RNAs are detected simultaneously in a single reaction. In some embodiments, all target RNAs are detected simultaneously in a single reaction.

In some embodiments, an increased level of one or more target RNAs capable of specifically hybridizing to a nucleic acid comprising a sequence selected from SEQ ID NOs: 1 to 397, 1063 to 1210, 2064 to 2183, 2673 to 2680, and 2689 in a sample is indicative of the presence of lung cancer in an individual from whom the sample has been taken. In some embodiments, an increased level of one or more target RNAs that comprise at least 15 contiguous nucleotides that are complementary to at least a portion of a sequence selected from SEQ ID NO: 1 to 397, 1063 to 1210, 2064 to 2183, 2673 to 2680, and 2689 in a sample is indicative of the presence of lung cancer in an individual from whom the sample has been taken. In some embodiments, an increased level of one or more target RNAs that comprise at least 15 contiguous nucleotides of a sequence selected from SEQ ID NOs: 794 to 1043, 2576 to 2672, and 2692 in a sample is indicative of the presence of lung cancer in an individual from whom the sample has been taken. In some embodiments, a decreased level of one or more target RNAs capable of specifically hybridizing to a nucleic acid comprising a sequence selected from SEQ ID NOs: 1 to 397, 1363 to 1707, and 2312 to 2452 in a sample is indicative of the presence of lung cancer in an individual from whom the sample has been taken. In some embodiments, a decreased level of one or more target RNAs that comprise at least 15 contiguous nucleotides that are complementary to at least a portion of a sequence selected from SEQ ID NO: 1 to 397, 1363 to 1707, and 2312 to 2452 in a sample is indicative of the presence of lung cancer in an individual from whom the sample has been taken. In some embodiments, a decreased level of one or more target RNAs that comprise at least 15 contiguous nucleotides of a sequence selected from SEQ ID NOs: 794 to 1043, 2576 to 2672, and 2692 in a sample is indicative of the presence of lung cancer in an individual from whom the sample has been taken.

In some embodiments, an increased level of one or more target RNAs capable of specifically hybridizing to a polynucleotide sequence in Table 6 is indicative of the presence of non-small cell lung cancer.

In some embodiments, an increased level of one or more target RNAs capable of specifically hybridizing to a polynucleotide sequence in Table 7 is indicative of squamous cell carcinoma.

In some embodiments, an increased level of one or more target RNAs capable of specifically hybridizing to a polynucleotide sequence in Table 8 is indicative of adenocarcinoma.

In some embodiments, an increased level of one or more target RNAs capable of specifically hybridizing to a polynucleotide sequence in Table 9 is indicative of aggressive lung cancer.

In some embodiments, an increased level of one or more target RNAs capable of specifically hybridizing to a polynucleotide sequence in Table 32 or 33 is indicative of lung cancer.

In some embodiments, a decreased level of one or more target RNAs capable of specifically hybridizing to a polynucleotide sequence in Table 34 is indicative of lung cancer.

In some embodiments, an increased level of one or more target RNAs capable of specifically hybridizing to a sequence selected from SEQ ID NOs: 15, 26, 27, 30, 129, 164, 184, 191, 196, 197, 205, 207, 214, 219, 225, 246, and 248 is indicative of non-small cell lung cancer.

In some embodiments, an increased level of one or more target RNAs capable of specifically hybridizing to a sequence selected from SEQ ID NOs: 15, 26, 27 and 191 and decreased level of one or more target RNAs capable of specifically hybridizing to a sequence selected from SEQ ID NOs: 92 and 171 is indicative of squamous cell carcinoma or adenocarcinoma.

In some embodiments, an increased level of one or more target RNAs capable of specifically hybridizing to a sequence selected from SEQ ID NOs: 4, 36, 50, 93, 122, 125, 139, 140, 144, 146, 159, 226, 239 and 241 is indicative of squamous cell carcinoma or adenocarcinoma.

In some embodiments, an increased level of one or more target RNAs capable of specifically hybridizing to a sequence selected from SEQ ID NOs: 19, 27, 33, 48, 55, 72, 73, 94, 101, 105, 112, 117, 130, 131, 133, 134, 135, 143, 155, 158, 160, 161, 163, 165, 221, 238, 240 and 246 is indicative of squamous cell carcinoma.

In some embodiments, a decreased level of one or more target RNAs capable of specifically hybridizing to a sequence selected from SEQ ID NOs: 19, 27, 33, 48, 55, 72, 73, 94, 101, 105, 112, 117, 130, 131, 133, 134, 135, 143, 155, 158, 160, 161, 163, 165, 221, 238, 240 and 246 is indicative of adenocarcinoma.

In some embodiments, an increased level of one or more target RNAs capable of specifically hybridizing to a sequence selected from SEQ ID NOs: 27, 72, 73, 161 or 246 is indicative of an aggressive form of adenocarcinoma.

### 4.1.2. Exemplary controls

In some embodiments, a normal level (a "control") for each target RNA can be determined as an average level or range that is characteristic of normal human lung cells or other reference material, against which the level measured in the sample can be compared. The determined average or range of target RNA in normal subjects can be used as a benchmark for detecting above-normal or below-normal levels of target RNA indicative of lung cancer. In some embodiments, normal levels of target RNA can be determined using individual or pooled RNA-containing samples from one or more individuals, such as from normal lung tissue from patients undergoing surgical resection for stage I, II or IIIA non-small cell lung cancer.

In some embodiments, determining a normal level of expression of a target RNA comprises detecting a complex comprising a probe hybridized to a nucleic acid selected from a target RNA, a DNA amplicon of the target RNA, and a complement of the target RNA. That is, in some embodiments, a normal level of expression can be determined by detecting a DNA amplicon of the target RNA, or a complement of the target RNA rather than the target RNA itself. In some embodiments, a normal level of such a complex is determined and used as a control. The normal level of the complex, in some embodiments, correlates to the normal level of the target RNA. Thus, when a normal level of a target is discussed herein, that level can, in some embodiments, be determined by detecting such a complex.

In some embodiments, a control comprises RNA from cells of a single individual, *e.g*., from normal tissue from a patient undergoing surgical resection for stage I, II or IIIA non-small cell lung cancer. In some embodiments, the control is drawn from anatomically and/or cytologically normal areas of the lung of the individual from whom the test sample was obtained. In some embodiments, a control comprises RNA from a pool of cells from multiple individuals. In some embodiments, a control comprises RNA from a pool of blood, such as whole blood or serum, from multiple individuals. In some embodiments, a control comprises commercially-available human RNA, such as, for example, human lung total RNA (Ambion; AM7968). In some embodiments, a normal level or normal range has already been predetermined prior to testing a sample for an elevated level.

In some embodiments, the normal level of target RNA can be determined from one or more continuous cell lines, typically cell lines previously shown to have expression levels of the at least one target RNA that approximate the level of expression in normal human lung cells.

In some embodiments, a method comprises detecting the level of expression of at least one target RNA. In some embodiments, a method further comprises comparing the level of expression of at least one target RNA to a normal level of expression of the at least one target RNA. In some embodiments, a method further comprises comparing the level of expression of at least one target RNA to a control level of expression of the at least one target RNA. A control level of expression of the at least one target RNA is, in some embodiments, the level of expression of the at least one target RNA in a normal cell. In some such embodiments, a control level may be referred to as a normal level. In some embodiments, a greater level of expression of the at least one target RNA relative to the level of expression of the at least one target RNA in a normal cell indicates lung cancer. In some embodiments, a reduced level of expression of the at least one target RNA relative to the level of expression of the at least one target RNA in a normal cell indicates lung cancer.

In some embodiments, the level of expression of the at least one target RNA is compared to a reference level of expression, e.g., from a patient with a confirmed lung cancer. In some such embodiments, a similar level of expression of the at least one target RNA relative to the reference sample indicates lung cancer.

In some embodiments, a level of expression of at least one target RNA that is at least about two-fold greater than a normal level of expression of the respective at least one target RNA indicates the presence of lung cancer. In some embodiments, a level of expression of at least one target RNA that is at least about two-fold greater than the level of the respective at least one target RNA in a control sample comprised of normal cells indicates the presence of a lung cancer. In various embodiments, a level of expression of at least one target RNA that is at least about 3-fold, at least about 4-fold, at least about 5-fold, at least about 6-fold, at least about 7-fold, at least about 8-fold, at least about 9-fold, or at least about 10-fold greater than the level of expression of the respective at least one target RNA in a control sample comprised of normal cells indicates the presence of lung cancer. In various embodiments, a level of expression of at least one target RNA that is at least about 3-fold, at least about 4-fold, at least about 5-fold, at least about 6-fold, at least about 7-fold, at least about 8-fold, at least about 9-fold, or at least about 10-fold greater than a normal level of expression of the at least one target RNA indicates the presence of lung cancer.

In some embodiments, a level of expression of at least one target RNA that is reduced by at least about two-fold relative to a normal level of expression of the respective at least one target RNA indicates the presence of lung cancer. In some embodiments, a level of expression of at least one target RNA that is reduced by at least about two-fold as compared to the level of the respective at least one target RNA in a control sample comprised of normal cells indicates the presence of a lung cancer. In various embodiments, a level of expression of at least one target RNA that is reduced by at least about 3-fold, at least about 4-fold, at least about 5-fold, at least about 6-fold, at least about 7-fold, at least about 8-fold, at least about 9-fold, or at least about 10-fold as compared to the level of expression of the respective at least one target RNA in a control sample comprised of normal cells indicates the presence of lung cancer. In various embodiments, a level of expression of at least one target RNA that is reduced by at least about 3-fold, at least about 4-fold, at least about 5-fold, at least about 6-fold, at least about 7-fold, at least about 8-fold, at least about 9-fold, or at least about 10-fold as compared to a normal level of expression of the at least one target RNA indicates the presence of lung cancer.

In some embodiments, a control level of expression of a target RNA is determined contemporaneously, such as in the same assay or batch of assays, as the level of expression of the target RNA in a sample. In some embodiments, a control level of expression of a target RNA is not determined contemporaneously as the level of expression of the target RNA in a sample. In some such embodiments, the control level of expression has been determined previously.

In some embodiments, the level of expression of a target RNA is not compared to a control level of expression, for example, when it is known that the target RNA is expressed at very low levels, or not at all, in normal cells. In some such embodiments, detection of a high level of the target RNA in a sample is indicative of lung cancer. Alternatively, if the target RNA is known to be expressed at high levels in normal cells, then detection of a very low level of the target RNA in a sample is indicative of lung cancer.

### 4.1.3. Exemplary methods of preparing RNAs

Target RNA can be prepared by any appropriate method. Total RNA can be isolated by any method, including, but not limited to, the protocols set forth in Wilkinson, M. (1988) Nucl. Acids Res. 16(22):10,933; and Wilkinson, M. (1988) Nucl. Acids Res. 16(22): 10934, or by using commercially-available kits or reagents, such as the TRIzoI® reagent (Invitrogen™), Total RNA Extraction Kit (iNtRON Biotechnology), Total RNA Purification Kit (Norgen Biotek Corp.), RNAqueous™ (Ambion), MagMAX™ (Ambion), RecoverAll™ (Ambion), RNeasy (Qiagen), etc.

In some embodiments, small RNAs are isolated or enriched. In some embodiments "small RNA" refers to RNA molecules smaller than about 200 nucleotides (nt) in length. In some embodiments, "small RNA" refers to RNA molecules smaller than about 100 nt, smaller than about 90 nt, smaller than about 80 nt, smaller than about 70 nt, smaller than about 60 nt, smaller than about 50 nt, or smaller than about 40 nt.

Enrichment of small RNAs can be accomplished by method. Such methods include, but are not limited to, methods involving organic extraction followed by adsorption of nucleic acid molecules on a glass fiber filter using specialized binding and wash solutions, and methods using spin column purification. Enrichment of small RNAs may be accomplished using commercially-available kits, such as mirVana™ Isolation Kit (Applied Biosystems), mirPremier™ microRNA Isolation Kit (Sigma-Aldrich), PureLink™ miRNA Isolation Kit (Invitrogen), miRCURY™ RNA isolation kit (Exiqon), microRNA Purification Kit (Norgen Biotek Corp.), miRNeasy kit (Qiagen), etc. In some embodiments, purification can be accomplished by the TRIzol® (Invitrogen) method, which employs a phenol/isothiocyanate solution to which chloroform is added to separate the RNA-containing aqueous phase. Small RNAs are subsequently recovered from the aqueous by precipitation with isopropyl alcohol. In some embodiments, small RNAs can be purified using chromatographic methods, such as gel electrophoresis using the flashPAGE™ Fractionator available from Applied Biosystems.

In some embodiments, small RNA is isolated from other RNA molecules to enrich for target RNAs, such that the small RNA fraction (e.g., containing RNA molecules that are 200 nucleotides or less in length, such as less than 100 nucleotides in length, such as less than 50 nucleotides in length, such as from about 10 to about 40 nucleotides in length) is substantially pure, meaning it is at least about 80%, 85%, 90%, 95% pure or more, but less than 100% pure, with respect to larger RNA molecules. Alternatively, enrichment of small RNA can be expressed in terms of fold-enrichment. In some embodiments, small RNA is enriched by about, at least about, or at most about 5X, 10X, 20X, 30X, 40X, 50X, 60X, 70X, 80X, 90X, 100X, 110X, 120X, 130X, 140X, 150X, 160X, 170X, 180X, 190X, 200X, 210X, 220X, 230X, 240X, 250X, 260X, 270X, 280X, 290X, 300X, 310X, 320X, 330X, 340X, 350X, 360X, 370X, 380X, 390X, 400X, 410X, 420X, 430X, 440X, 450X, 460X, 470X, 480X, 490X, 500X, 600X, 700X, 800X, 900X, 1000X, 1100X, 1200X, 1300X, 1400X, 1500X, 1600X, 1700X, 1800X, 1900X, 2000X, 3000X, 4000X, 5000X, 6000X, 7000X, 8000X, 9000X, 10,000X or more, or any range derivable therein, with respect to the concentration of larger RNAs in an RNA isolate or total RNA in a sample.

In yet other embodiments, expression is measured in a sample in which RNA has not first been purified from the cells.

In some embodiments, RNA is modified before target RNAs are detected. In some embodiments, the modified RNA is total RNA. In other embodiments, the modified RNA is small RNA that has been purified from total RNA or from cell lysates, such as RNA less than 200 nucleotides in length, such as less than 100 nucleotides in length, such as less than 50 nucleotides in length, such as from about 10 to about 40 nucleotides in length. RNA modifications that can be utilized in the methods described herein include, but are not limited to, the addition of a poly-dA or a poly-dT tail, which can be accomplished chemically or enzymatically, and/or the addition of a small molecule, such as biotin.

In some embodiments, one or more target RNAs are reverse transcribed. In some embodiments, where present, RNA is modified when it is reverse transcribed, such as when a poly-dA or a poly-dT tail is added to the cDNA during reverse transcription. In other embodiments, RNA is modified before it is reverse transcribed. In some embodiments, total RNA is reverse transcribed. In other embodiments, small RNAs are isolated or enriched before the RNA is reverse transcribed.

When a target RNA is reverse transcribed, a complement of the target RNA is formed. In some embodiments, the complement of the target RNA is detected rather than the target RNA itself (or a DNA copy thereof). Thus, when the methods discussed herein indicate that a target RNA is detected, or the level of a target RNA is determined, such detection or determination may be carried out on a complement of the target RNA instead of, or in addition to, the target RNA itself. In some embodiments, when the complement of the target RNA is detected rather than the target RNA, a probe is used that is complementary to the complement of the target RNA. In such embodiments, the probe comprises at least a portion that is identical in sequence to the target RNA, although it may contain thymidine in place of uridine, and/or comprise other modified nucleotides.

In some embodiments, the method of detecting one or more target RNAs comprises amplifying cDNA complementary to said target RNA. Such amplification can be accomplished by any method. Exemplary methods include, but are not limited to, real time PCR, endpoint PCR, and amplification using T7 polymerase from a T7 promoter annealed to a cDNA, such as provided by the SenseAmp PIus™ Kit available at Implen, Germany.

When a target RNA or a cDNA complementary to a target RNA is amplified, in some embodiments, a DNA amplicon of a target RNA is formed. A DNA amplicon may be single stranded or double-stranded. In some embodiments, when a DNA amplicon is single-stranded, the sequence of the DNA amplicon is related to the target RNA in either the sense or antisense orientation. In some embodiments, the DNA amplicon of the target RNA is detected rather than the target RNA itself. Thus, when the methods discussed herein indicate that a target RNA is detected, or the level of a target RNA is determined, such detection or determination may be carried out on a DNA amplicon of the target RNA instead of, or in addition to, the target RNA itself. In some embodiments, when the DNA amplicon of the target RNA is detected rather than the target RNA, a probe is used that is complementary to the complement of the target RNA. In some embodiments, when the DNA amplicon of the target RNA is detected rather than the target RNA, a probe is used that is complementary to the target RNA. Further, in some embodiments, multiple probes may be used, and some probes may be complementary to the target RNA and some probes may be complementary to the complement of the target RNA.

In some embodiments, the method of detecting one or more target RNAs comprises RT-PCR, as described below. In some embodiments, detecting one or more target RNAs comprises real-time monitoring of an RT-PCR reaction, which can be accomplished by any method. Such methods include, but are not limited to, the use of TaqMan®, Molecular beacon, or Scorpion probes (i.e., FRET probes) and the use of intercalating dyes, such as SYBR green, EvaGreen, thiazole orange, YO-PRO, TO-PRO, etc.

### 4.1.4. Exemplary analytical methods

As described above, methods are presented for detecting lung cancer in a sample from a patient. In some embodiments, the method comprises detecting a level of expression of at least one target RNA capable of specifically hybridizing to a nucleic acid comprising a sequence selected from SEQ ID NOs: 1 to 397, 1063 to 1210, 2064 to 2183, 2673 to 2680, and 2689 that is greater in the sample than a normal level of expression of the at least one target RNA in a control sample, such as a sample derived from normal lung cells. In some embodiments, a method comprises detecting a level of one or more target RNAs that comprise a sequence that is complementary to at least 15 contiguous nucleotides of a sequence selected from SEQ ID NOs: 1 to 397, 1063 to 1210, 2064 to 2183, 2673 to 2680, and 2689 that is greater in the sample than a normal level of expression of the at least one target RNA in a control sample. In some embodiments, a method comprises detecting a level of one or more target RNAs that comprise at least 15 contiguous nucleotides of a sequence selected from SEQ ID NOs: 794 to 1043, 2576 to 2672, and 2692 that is greater in the sample than a normal level of expression of the at least one target RNA in a control sample.

In some embodiments, the method comprises detecting a level of expression of at least one target RNA capable of specifically hybridizing to a nucleic acid comprising a sequence selected from SEQ ID NOs: 1 to 397, 1363 to 1707, and 2312 to 2452 that is reduced in the sample relative to a normal level of expression of the at least one target RNA in a control sample, such as a sample derived from normal lung cells. In some embodiments, a method comprises detecting a level of one or more target RNAs that comprise a sequence that is complementary to at least 15 contiguous nucleotides of a sequence selected from SEQ ID NOs: 1 to 397, 1363 to 1707, and 2312 to 2452 that is reduced in the sample relative to a normal level of expression of the at least one target RNA in a control sample. In some embodiments, a method comprises detecting a level of one or more target RNAs that comprise at least 15 contiguous nucleotides of a sequence selected from SEQ ID NOs: 794 to 1043, 2576 to 2672, and 2692 that is reduced in the sample relative a normal level of expression of the at least one target RNA in a control sample.

In some embodiments, a target RNA, in its mature form, comprises fewer than 30 nucleotides. In some embodiments, a target RNA is a microRNA.

In some embodiments, such as those described above, the method further comprises detecting a level of expression of at least one target RNA of the human miRNome that does not specifically hybridize to a nucleic acid comprising a sequence selected from SEQ ID NOs: 1 to 397, 1063 to 1210, 1363 to 1707, 2064 to 2183, 2312 to 2452, 2673 to 2680, and 2689 and does not comprise at least 15 contiguous nucleotides of a sequence selected from SEQ ID NOs: 794 to 1043, 2576 to 2672, and 2692, that is altered in the sample relative to a normal level of expression of the at least one target RNA in a control sample. As used herein, the term "human miRNome" refers to all microRNA genes in a human cell and the mature microRNAs produced therefrom.

Any analytical procedure capable of permitting specific and quantifiable (or semi-quantifiable) detection of the desired at least one target RNA may be used in the methods herein presented. Such analytical procedures include, but are not limited to, the microarray methods set forth in Examples 1, 2, 4, and 5, the microbead methods set forth in Example 3, and methods known to those skilled in the art.

In some embodiments, detection of a target RNA comprises forming a complex comprising a polynucleotide that is complementary to a target RNA or to a complement thereof, and a nucleic acid selected from the target RNA, a DNA amplicon of the target RNA, and a complement of the target RNA. Thus, in some embodiments, the polynucleotide forms a complex with a target RNA. In some embodiments, the polynucleotide forms a complex with a complement of the target RNA, such as a cDNA that has been reverse transcribed from the target RNA. In some embodiments, the polynucleotide forms a complex with a DNA amplicon of the target RNA. When a double-stranded DNA amplicon is part of a complex, as used herein, the complex may comprise one or both strands of the DNA amplicon. Thus, in some embodiments, a complex comprises only one strand of the DNA amplicon. In some embodiments, a complex is a triplex and comprises the polynucleotide and both strands of the DNA amplicon. In some embodiments, the complex is formed by hybridization between the polynucleotide and the target RNA, complement of the target RNA, or DNA amplicon of the target RNA. The polynucleotide, in some embodiments, is a primer or probe.

In some embodiments, a method comprises detecting the complex. In some embodiments, the complex does not have to be associated at the time of detection. That is, in some embodiments, a complex is formed, the complex is then dissociated or destroyed in some manner, and components from the complex are detected. An example of such a system is a TaqMan® assay. In some embodiments, when the polynucleotide is a primer, detection of the complex may comprise amplification of the target RNA, a complement of the target RNA, or a DNA amplicon of a target RNA.

In some embodiments the analytical method used for detecting at least one target RNA in the methods set forth herein includes real-time quantitative RT-PCR. See Chen, C. et al. (2005) Nucl. Acids Res. 33:e179 and PCT Publication No. WO 2007/117256, which are incorporated herein by reference in its entirety. In some embodiments, the analytical method used for detecting at least one target RNA includes the method described in U.S. Publication No. US2009/0123912 A1, which is incorporated herein by reference in its entirety. In an exemplary method described in that publication, an extension primer comprising a first portion and second portion, wherein the first portion selectively hybridizes to the 3' end of a particular microRNA and the second portion comprises a sequence for universal primer, is used to reverse transcribe the microRNA to make a cDNA. A reverse primer that selectively hybridizes to the 5' end of the microRNA and a universal primer are then used to amplify the cDNA in a quantitative PCR reaction.

In some embodiments, the analytical method used for detecting at least one target RNA includes the use of a TaqMan® probe. In some embodiments, the analytical method used for detecting at least one target RNA includes a TaqMan® assay, such as the TaqMan® MicroRNA Assays sold by Applied Biosystems, Inc. In an exemplary TaqMan® assay, total RNA is isolated from the sample. In some embodiments, the assay can be used to analyze about 10 ng of total RNA input sample, such as about 9 ng of input sample, such as about 8 ng of input sample, such as about 7 ng of input sample, such as about 6 ng of input sample, such as about 5 ng of input sample, such as about 4 ng of input sample, such as about 3 ng of input sample, such as about 2 ng of input sample, and even as little as about 1 ng of input sample containing microRNAs.

The TaqMan® assay utilizes a stem-loop primer that is specifically complementary to the 3'-end of a target RNA. In an exemplary TaqMan® assay, hybridizing the stem-loop primer to the target RNA is followed by reverse transcription of the target RNA template, resulting in extension of the 3' end of the primer. The result of the reverse transcription is a chimeric (DNA) amplicon with the step-loop primer sequence at the 5' end of the amplicon and the cDNA of the target RNA at the 3' end. Quantitation of the target RNA is achieved by real time RT-PCR using a universal reverse primer having a sequence that is complementary to a sequence at the 5' end of all stem-loop target RNA primers, a target RNA-specific forward primer, and a target RNA sequence-specific TaqMan® probe.

The assay uses fluorescence resonance energy transfer ("FRET") to detect and quantitate the synthesized PCR product. Typically, the TaqMan® probe comprises a fluorescent dye molecule coupled to the 5'-end and a quencher molecule coupled to the 3'-end, such that the dye and the quencher are in close proximity, allowing the quencher to suppress the fluorescence signal of the dye via FRET. When the polymerase replicates the chimeric amplicon template to which the TaqMan® probe is bound, the 5'-nuclease of the polymerase cleaves the probe, decoupling the dye and the quencher so that FRET is abolished and a fluorescence signal is generated. Fluorescence increases with each RT-PCR cycle proportionally to the amount of probe that is cleaved.

Additional exemplary methods for RNA detection and/or quantification are described, e.g., in U.S. Publication No. US 2007/0077570 (Lao et al.), PCT Publication No. WO 2007/025281 (Tan et al.), U.S. Publication No. US2007/0054287 (Bloch), PCT Publication No. W02006/0130761 (Bloch), and PCT Publication No. WO 2007/011903 (Lao et al.), which are incorporated by reference herein in their entireties for any purpose.

In some embodiments, quantitation of the results of real-time RT-PCR assays is done by constructing a standard curve from a nucleic acid of known concentration and then extrapolating quantitative information for target RNAs of unknown concentration. In some embodiments, the nucleic acid used for generating a standard curve is an RNA (e.g., microRNA) of known concentration. In some embodiments, the nucleic acid used for generating a standard curve is a purified double-stranded plasmid DNA or a single-stranded DNA generated in vitro.

In some embodiments, where the amplification efficiencies of the target nucleic acids and the endogenous reference are approximately equal, quantitation is accomplished by the comparative Ct (cycle threshold, e.g., the number of PCR cycles required for the fluorescence signal to rise above background) method. Ct values are inversely proportional to the amount of nucleic acid target in a sample. In some embodiments, Ct values of the target RNA of interest can be compared with a control or calibrator, such as RNA (e.g., microRNA) from normal tissue. In some embodiments, the Ct values of the calibrator and the target RNA samples of interest are normalized to an appropriate endogenous housekeeping gene.

In addition to the TaqMan® assays, other real-time RT-PCR chemistries useful for detecting and quantitating PCR products in the methods presented herein include, but are not limited to, Molecular Beacons, Scorpion probes and intercalating dyes, such as SYBR Green, EvaGreen, thiazole orange, YO-PRO, TO-PRO, etc., which are discussed below.

In some embodiments, real-time RT-PCR detection is performed specifically to detect and quantify the expression of a single target RNA. The target RNA, in some embodiments, is selected from a target RNA capable of specifically hybridizing to a nucleic acid comprising a sequence selected from SEQ ID NOs: 1 to 397, 1063 to 1210, 1363 to 1707, 2064 to 2183, 2312 to 2452, 2673 to 2680, and 2689. In some embodiments, the target RNA comprises a sequence that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689. The target RNA, in some embodiments, comprises at least 15 contiguous nucleotides of a sequence selected from SEQ ID NOs.: 794 to 1043, 2576 to 2672, and 2692. In some embodiments, the target RNA specifically hybridizes to a nucleic acid comprising a sequence selected from the probe sequences in Table 6. In some embodiments, the target RNA specifically hybridizes to a nucleic acid comprising a sequence selected from the probe sequences in Table 7. In some embodiments, the target RNA specifically hybridizes to a nucleic acid comprising a sequence selected from the probe sequences in Table 8. In some embodiments, the target RNA specifically hybridizes to a nucleic acid comprising a sequence selected from the probe sequences in Table 9. In some embodiments, the target RNA specifically hybridizes to a nucleic acid comprising a sequence selected from the probe sequences in Tables 32 and 33. In some embodiments, the target RNA specifically hybridizes to a nucleic acid comprising a sequence selected from the probe sequences in Table 34.

In some embodiments, a target RNA, in its mature form, comprises fewer than 30 nucleotides. In some embodiments, a target RNA is a microRNA.

In various embodiments, real-time RT-PCR detection is utilized to detect, in a single multiplex reaction, at least 2, at least 3, at least 4, at least 5, at least 6, at least 7, or at least 8 target RNAs. At least one target RNA, in some embodiments, is capable of specifically hybridizing to a nucleic acid comprising a sequence selected from SEQ ID NOs: 1 to 397, 1063 to 1210, 1363 to 1707, 2064 to 2183, 2312 to 2452, 2673 to 2680, and 2689. In some embodiments, at least one target RNA comprises at least 15 contiguous nucleotides of a sequence selected from SEQ ID NOs: 794 to 1043, 2576 to 2672, and 2692. In some embodiments, at least one target RNA comprises a sequence that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689. In some embodiments, a target RNA, in its mature form, comprises fewer than 30 nucleotides. In some embodiments, a target RNA is a microRNA.

In some embodiments, the method comprises detecting expression in a multiplex RT-PCR reaction of at least 2, at least 3, at least 5, at least 10, or at least 15 target RNAs, wherein each target RNA is capable of specifically hybridizing to a probe sequence in Table 6. In some embodiments, the method comprises detecting expression, using a single multiplex RT-PCR reaction, of at least 2, at least 3, at least 4, at least 5, at least 6, at least 7, at least 8, at least 9, at least 10, at least 12, at least 15, at least 20, or at least 25 target RNAs, wherein each target RNA is capable of specifically hybridizing to a probe sequence in Table 7. In some embodiments, the method comprises detecting expression, using a single multiplex RT-PCR reaction, of at least 2, at least 3, at least 4, at least 5, at least 6, or at least 7 target RNAs, wherein each target RNA is capable of specifically hybridizing to a probe sequence in Table 8. In some embodiments, the method comprises detecting expression in a multiplex RT-PCR reaction of at least 2, at least 3, at least 5, at least 10, at least 15, at least 20, at least 25, at least 30, at least 40, or at least 50 target RNAs, wherein each target RNA is capable of specifically hybridizing to a probe sequence in Table 9. In some embodiments, the method comprises detecting expression in a multiplex RT-PCR reaction of at least two, at least five, at least 10, at least 15, at least 20, at least 25, at least 30, or at least target RNAs, wherein each target RNA is capable of specifically hybridizing to a probe sequence in one of Tables 32 or 33. In some embodiments, the method comprises detecting expression in a multiplex RT-PCR reaction of at least two, at least five, at least 10, at least 15, at least 20, at least 30, at least 40, at least 50, or at least 60 target RNAs, wherein each target RNA is capable of specifically hybridizing to a probe sequence in Table 34.

In some multiplex embodiments, a plurality of probes, such as TaqMan® probes, each specific for a different RNA target, is used. In some embodiments, each target RNA-specific probe is spectrally distinguishable from the other probes used in the same multiplex reaction.

In some embodiments, quantitation of real-time RT PCR products is accomplished using a dye that binds to double-stranded DNA products, such as SYBR Green, EvaCrreen, thiazole orange, YO-PRO, TO-PRO, etc. In some embodiments, the assay is the QuantiTect SYBR Green PCR assay from Qiagen. In this assay, total RNA is first isolated from a sample. Total RNA is subsequently poly-adenylated at the 3'-end and reverse transcribed using a universal primer with poly-dT at the 5'-end. In some embodiments, a single reverse transcription reaction is sufficient to assay multiple target RNAs. Real-time RT-PCR is then accomplished using target RNA-specific primers and an miScript Universal Primer, which comprises a poly-dT sequence at the 5'-end. SYBR Green dye binds non-specifically to double-stranded DNA and upon excitation, emits light. In some embodiments, buffer conditions that promote highly-specific annealing of primers to the PCR template (e.g., available in the QuantiTect SYBR Green PCR Kit from Qiagen) can be used to avoid the formation of non-specific DNA duplexes and primer dimers that will bind SYBR Green and negatively affect quantitation. Thus, as PCR product accumulates, the signal from SYBR Green increases, allowing quantitation of specific products.

Real-time RT-PCR is performed using any RT-PCR instrumentation available in the art. Typically, instrumentation used in real-time RT-PCR data collection and analysis comprises a thermal cycler, optics for fluorescence excitation and emission collection, and optionally a computer and data acquisition and analysis software.

In some embodiments, the analytical method used in the methods described herein is a DASL® (cDNA-mediated Annealing, Selection, Extension, and Ligation) Assay, such as the MicroRNA Expression Profiling Assay available from Illumina, Inc. (See http://www.illumina.com/downloads/MicroRNAAssayWorkflow.pdf). In some embodiments, total RNA is isolated from a sample to be analyzed by any method. Additionally, in some embodiments, small RNAs are isolated from a sample to be analyzed by any method. Total RNA or isolated small RNAs may then be polyadenylated (> 18 A residues are added to the 3'-ends of the RNAs in the reaction mixture). The RNA is reverse transcribed using a biotin-labeled DNA primer that comprises from the 5' to the 3' end, a sequence that includes a PCR primer site and a poly-dT region that binds to the poly-dA tail of the sample RNA. The resulting biotinylated cDNA transcripts are then hybridized to a solid support via a biotin-streptavidin interaction and contacted with one or more target RNA-specific polynucleotides. The target RNA-specific polynucleotides comprise, from the 5'-end to the 3'-end, a region comprising a PCR primer site, region comprising an address sequence, and a target RNA-specific sequence.

In some DASL® embodiments, the target RNA-specific sequence comprises at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides having a sequence identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, the target RNA-specific sequence comprises a probe sequence that is complementary to at least a portion of a microRNA of the human miRNome.

After hybridization, the target RNA-specific polynucleotide is extended, and the extended products are then eluted from the immobilized cDNA array. A second PCR reaction using a fluorescently-labeled universal primer generates a fluorescently-labeled DNA comprising the target RNA-specific sequence. The labeled PCR products are then hybridized to a microbead array for detection and quantitation.

In some embodiments, the analytical method used for detecting and quantifying the expression of the at least one target RNA in the methods described herein is a bead-based flow cytometric assay. See Lu J. et al. (2005) Nature 435:834-838, which is incorporated herein by reference in its entirety. An example of a bead-based flow cytometric assay is the xMAP® technology of Luminex, Inc. (See http://www.luminexcorp.com/ technology/index.html). In some embodiments, total RNA is isolated from a sample and is then labeled with biotin. The labeled RNA is then hybridized to target RNA-specific capture probes (e.g., FlexmiR™ products sold by Luminex, Inc. at http://www.luminexcorp.com/products/assays/index.html ) that are covalently bound to microbeads, each of which is labeled with 2 dyes having different fluorescence intensities. A streptavidin-bound reporter molecule (e.g., streptavidin-phycoerythrin, also known as "SAPE") is attached to the captured target RNA and the unique signal of each bead is read using flow cytometry. In some embodiments, the RNA sample (total RNA or enriched small RNAs) is first polyadenylated, and is subsequently labeled with a biotinylated 3DNA™ dendrimer (i.e., a multiple-arm DNA with numerous biotin molecules bound thereto), such as those sold by Marligen Biosciences as the Vantage™ microRNA Labeling Kit, using a bridging polynucleotide that is complementary to the 3'-end of the poly-dA tail of the sample RNA and to the 5'-end of the polynucleotide attached to the biotinylated dendrimer. The streptavidin-bound reporter molecule is then attached to the biotinylated dendrimer before analysis by flow cytometry. See http://www.marligen.com/vantage-microrna-labeling-kit.html. In some embodiments, biotin-labeled RNA is first exposed to SAPE, and the RNA/SAPE complex is subsequently exposed to an anti-phycoerythrin antibody attached to a DNA dendrimer, which can be bound to as many as 900 biotin molecules. This allows multiple SAPE molecules to bind to the biotinylated dendrimer through the biotin-streptavidin interaction, thus increasing the signal from the assay.

In some embodiments, the analytical method used for detecting and quantifying the expression of the at least one target RNA in the methods described herein is by gel electrophoresis and detection with labeled probes (e.g., probes labeled with a radioactive or chemiluminescent label), such as by Northern blotting. In some embodiments, total RNA is isolated from the sample, and then is size-separated by SDS polyacrylamide gel electrophoresis. The separated RNA is then blotted onto a membrane and hybridized to radiolabeled complementary probes. In some embodiments, exemplary probes contain one or more affinity-enhancing nucleotide analogs as discussed below, such as locked nucleic acid ("LNA") analogs, which contain a bicyclic sugar moiety instead of deoxyribose or ribose sugars. See, e.g., Várallyay, E. et al. (2008) Nature Protocols 3(2):190-196, which is incorporated herein by reference in its entirety. In some embodiments, the total RNA sample can be further purified to enrich for small RNAs. In some embodiments, target RNAs can be amplified by, e.g., rolling circle amplification using a long probe that is complementary to both ends of a target RNA ("padlocked probes"), ligation to circularize the probe followed by rolling circle replication using the target RNA hybridized to the circularized probe as a primer. See, e.g., Jonstrup, S.P. et al. (2006) RNA 12:1-6, which is incorporated herein by reference in its entirety. The amplified product can then be detected and quantified using, e.g., gel electrophoresis and Northern blotting.

In alternative embodiments, labeled probes are hybridized to isolated total RNA in solution, after which the RNA is subjected to rapid ribonuclease digestion of single-stranded RNA, e.g., unhybridized portions of the probes or unhybridized target RNAs. In these embodiments, the ribonuclease treated sample is then analyzed by SDS-PAGE and detection of the radiolabeled probes by, e.g., Northern blotting. See mirVana™ miRNA Detection Kit sold by Applied Biosystems, Inc. product literature at http://www.ambion.com/catalog/CatNum.php?1552.

In some embodiments, the analytical method used for detecting and quantifying the at least one target RNA in the methods described herein is by hybridization to a microarray. See, e.g., Liu, C.G. et al. (2004) Proc. Nat'l Acad. Sci. USA 101:9740-9744; Lim, L.P. et al. (2005) Nature 433:769-773, each of which is incorporated herein by reference in its entirety, and Examples 1, 2, 4, and 5.

In some embodiments, detection and quantification of a target RNA using a microarray is accomplished by surface plasmon resonance. See, e.g., Nanotech News (2006), available at http://nano.cancer.gov/news_center/nanotech_news_2006-10-30b.asp. In these embodiments, total RNA is isolated from a sample being tested. Optionally, the RNA sample is further purified to enrich the population of small RNAs. After purification, the RNA sample is bound to an addressable microarray containing probes at defined locations on the microarray. Nonlimiting exemplary probes include probes comprising sequences set forth in SEQ ID NOs: 1 to 397, 1063 to 1210, 1363 to 1707, 2064 to 2183, 2312 to 2452, 2673 to 2680, and 2689. Exemplary probes also include, but are not limited to, probes comprising a region that is complementary to at least 15 contiguous nucleotides of a sequence selected from SEQ ID NOs: 794 to 1043, 2576 to 2672, and 2692. Exemplary probes also include, but are not limited to, probes comprising at least 15 contiguous nucleotides of a sequence selected from SEQ ID NOs: 1 to 397, 1063 to 1210, 1363 to 1707, 2064 to 2183, 2312 to 2452, 2673 to 2680, and 2689. In some embodiments, the probes contain one or more affinity-enhancing nucleotide analogs as discussed below, such as locked nucleic acid ("LNA") nucleotide analogs. After hybridization to the microarray, the RNA that is hybridized to the array is first polyadenylated, and the array is then exposed to gold particles having poly-dT bound to them. The amount of bound target RNA is quantitated using surface plasmon resonance.

In some embodiments, microarrays are utilized in a RNA-primed, Array-based Klenow Enzyme ("RAKE") assay. See Nelson, P.T. et al. (2004) Nature Methods 1(2):1-7; Nelson, P.T. et al. (2006) RNA 12(2):1-5, each of which is incorporated herein by reference in its entirety. In some embodiments, total RNA is isolated from a sample. In some embodiments, small RNAs are isolated from a sample. The RNA sample is then hybridized to DNA probes immobilized at the 5'-end on an addressable array. The DNA probes comprise, in some embodiments, from the 5'-end to the 3'-end, a first region comprising a "spacer" sequence which is the same for all probes, a second region comprising three thymidine-containing nucleosides, and a third region comprising a sequence that is complementary to a target RNA of interest.

Exemplary target RNAs of interest include, but are not limited to, target RNAs capable of specifically hybridizing to a nucleic acid comprising a sequence selected from SEQ ID NOs: 1 to 397, 1063 to 1210, 1363 to 1707, 2064 to 2183, 2312 to 2452, 2673 to 2680, and 2689; target RNAs comprising a region that is identical to at least 15 contiguous nucleotides of a sequence selected from SEQ ID NOs: 794 to 1043, 2576 to 2672, and 2692; and target RNAs comprising a region that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689. Target RNAs also include target RNAs in the miRNome that do not specifically hybridize to a nucleic acid comprising a sequence selected from SEQ ID NOs: 1 to 397, 1063 to 1210, 1363 to 1707, 2064 to 2183, 2312 to 2452, 2673 to 2680, and 2689. In some embodiments, a target RNA, in its mature form, comprises fewer than 30 nucleotides. In some embodiments, a target RNA is a microRNA.

After the sample is hybridized to the array, it is exposed to exonuclease I to digest any unhybridized probes. The Klenow fragment of DNA polymerase I is then applied along with biotinylated dATP, allowing the hybridized target RNAs to act as primers for the enzyme with the DNA probe as template. The slide is then washed and a streptavidin-conjugated fluorophore is applied to detect and quantitate the spots on the array containing hybridized and Klenow-extended target RNAs from the sample.

In some embodiments, the RNA sample is reverse transcribed. In some embodiments, the RNA sample is reverse transcribed using a biotin/poly-dA random octamer primer. When than primer is used, the RNA template is digested and the biotin-containing cDNA is hybridized to an addressable microarray with bound probes that permit specific detection of target RNAs. In some embodiments, the microarray includes at least one probe comprising at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides identically present in, or complementary to a region of, a sequence selected from SEQ ID NOs: 1 to 397, 1063 to 1210, 1363 to 1707, 2064 to 2183, 2312 to 2452, 2673 to 2680, and 2689. After hybridization of the cDNA to the microarray, the microarray is exposed to a streptavidin-bound detectable marker, such as a fluorescent dye, and the bound cDNA is detected. See Liu C.G. et al. (2008) Methods 44:22-30, which is incorporated herein by reference in its entirety.

In some embodiments, target RNAs are detected and quantified in an ELISA-like assay using probes bound in the wells of microtiter plates. See Mora J.R. and Getts R.C. (2006) BioTechniques 41:420-424 and supplementary material in BioTechniques 41(4):1-5; U.S. Patent Publication No. 2006/0094025 to Getts et al., each of which is incorporated by reference herein in its entirety. In these embodiments, a sample of RNA that is enriched in small RNAs is either polyadenylated, or is reverse transcribed and the cDNA is polyadenylated. The RNA or cDNA is hybridized to probes immobilized in the wells of a microtiter plates, wherein each of the probes comprises a sequence that is identically present in, or complementary to a region of, one of SEQ ID NOs: 1 to 397, 1063 to 1210, 1363 to 1707, 2064 to 2183, 2312 to 2452, 2673 to 2680, and 2689, or a sequence such as one or more sequences of target RNAs (or the reverse complement thereof) of the human miRNome, depending on whether RNA or cDNA is hybridized to the array. In some embodiments, the hybridized RNAs are labeled using a capture sequence, such as a DNA dendrimer (such as those available from Genisphere, Inc., http://www.genisphere.com/about_3dna.html) that is labeled with a plurality of biotin molecules or with a plurality of horseradish peroxidase molecules, and a bridging polynucleotide that contains a poly-dT sequence at the 5'-end that binds to the poly-dA tail of the captured nucleic acid, and a sequence at the 3'-end that is complementary to a region of the capture sequence. If the capture sequence is biotinylated, the microarray is then exposed to streptavidin-bound horseradish peroxidase. Hybridization of target RNAs is detected by the addition of a horseradish peroxidase substrate such as tetramethylbenzidine (TMB) and measurement of the absorbance of the solution at 450nM.

In still other embodiments, an addressable microarray is used to detect a target RNA using quantum dots. See Liang, R.Q. et al. (2005) Nucl. Acids Res. 33(2):e17, available at http://www.pubmedcentral.nih.gov/articlerender.fcgi?artid= 548377, which is incorporated herein by reference in its entirety. In some embodiments, total RNA is isolated from a sample. In some embodiments, small RNAs are isolated from the sample. The 3'-ends of the target RNAs are biotinylated using biotin-X-hydrazide. The biotinylated target RNAs are captured on a microarray comprising immobilized probes comprising sequences that are identically present in, or complementary to a region of, one or more of SEQ ID NOs: 1 to 397, 1063 to 1210, 1363 to 1707, 2064 to 2183, 2312 to 2452, 2673 to 2680, and 2689 and/or probes comprising sequences other than those that are complementary to one or more microRNAs of the human miRNome. The hybridized target RNAs are then labeled with quantum dots via a biotin-streptavidin binding. A confocal laser causes the quantum dots to fluoresce and the signal can be quantified. In alternative embodiments, small RNAs can be detected using a colorimetric assay. In these embodiments, small RNAs are labeled with streptavidin-conjugated gold followed by silver enhancement. The gold nanoparticles bound to the hybridized target RNAs catalyze the reduction of silver ions to metallic silver, which can then be detected colorimetrically with a CCD camera.

In some embodiments, detection and quantification of one or more target RNAs is accomplished using microfluidic devices and single-molecule detection. In some embodiments, target RNAs in a sample of isolated total RNA are hybridized to two probes, one which is complementary to nucleic acids at the 5'-end of the target RNA and the second which is complementary to the 3'-end of the target RNA. Each probe comprises, in some embodiments, one or more affinity-enhancing nucleotide analogs, such as LNA nucleotide analogs and each is labeled with a different fluorescent dye having different fluorescence emission spectra. The sample is then flowed through a microfluidic capillary in which multiple lasers excite the fluorescent probes, such that a unique coincident burst of photons identifies a particular target RNA, and the number of particular unique coincident bursts of photons can be counted to quantify the amount of the target RNA in the sample. See U.S. Patent Publication No. 2006/0292616 to Neely et al., which is hereby incorporated by reference in its entirety. In some alternative embodiments, a target RNA-specific probe can be labeled with 3 or more distinct labels selected from, e.g., fluorophores, electron spin labels, etc., and then hybridized to an RNA sample, such as total RNA, or a sample that is enriched in small RNAs. The target RNA/probe duplex is then passed through channels in a microfluidic device and that comprise detectors that record the unique signal of the 3 labels. In this way, individual molecules are detected by their unique signal and counted. *See* U.S. Patent Nos. 7,402,422 and 7,351,538 to Fuchs et al*.,* U.S. Genomics, Inc., each of which is incorporated herein by reference in its entirety.

Nonlimiting exemplary target RNA-specific probes include probes comprising sequences selected from of SEQ ID NOs: 1 to 397. Nonlimiting exemplary target RNA-specific probes include probes comprising sequences that are complementary to sequences selected from of SEQ ID NOs: 1 to 397. Nonlimiting exemplary target RNA-specific probes also include probes comprising at least 15 contiguous nucleotides of, or the complement of at least 15 contiguous nucleotides of, a sequence selected from SEQ ID NOs: 1 to 397, 1063 to 1210, 1363 to 1707, 2064 to 2183, 2312 to 2452, 2673 to 2680, and 2689.

Optionally, the sample RNA is modified before hybridization. The target RNA/probe duplex is then passed through channels in a microfluidic device and that comprise detectors that record the unique signal of the 3 labels. In this way, individual molecules are detected by their unique signal and counted. See U.S. Patent Nos. 7,402,422 and 7,351,538 to Fuchs et al., U.S. Genomics, Inc., each of which is incorporated herein by reference in its entirety.

In some embodiments, the detection and quantification of one or more target RNAs is accomplished by a solution-based assay, such as a modified Invader assay. See Allawi H.T. et al. (2004) RNA 10:1153-1161, which is incorporated herein by reference in its entirety. In some embodiments, the modified invader assay can be performed on unfractionated detergent lysates of cells. In other embodiments, the modified invader assay can be performed on total RNA isolated from cells or on a sample enriched in small RNAs. The target RNAs in a sample are annealed to two probes which form hairpin structures. A first probe has a hairpin structure at the 5' end and a region at the 3'-end that has a sequence that is complementary to the sequence of a region at the 5'-end of a target RNA. The 3'-end of the first probe is the "invasive polynucleotide". A second probe has, from the 5' end to the 3'-end a first "flap" region that is not complementary to the target RNA, a second region that has a sequence that is complementary to the 3'-end of the target RNA, and a third region that forms a hairpin structure. When the two probes are bound to a target RNA target, they create an overlapping configuration of the probes on the target RNA template, which is recognized by the Cleavase enzyme, which releases the flap of the second probe into solution. The flap region then binds to a complementary region at the 3'-end of a secondary reaction template ("SRT"). A FRET polynucleotide (having a fluorescent dye bound to the 5'-end and a quencher that quenches the dye bound closer to the 3' end) binds to a complementary region at the 5'-end of the SRT, with the result that an overlapping configuration of the 3'-end of the flap and the 5'-end of the FRET polynucleotide is created. Cleavase recognizes the overlapping configuration and cleaves the 5'-end of the FRET polynucleotide, generates a fluorescent signal when the dye is released into solution.

### 4.1.5. Exemplary polynucleotides

In some embodiments, polynucleotides are provided. In some embodiments, synthetic polynucleotides are provided. Synthetic polynucleotides, as used herein, refer to polynucleotides that have been synthesized in vitro either chemically or enzymatically. Chemical synthesis of polynucleotides includes, but is not limited to, synthesis using polynucleotide synthesizers, such as OligoPilot (GE Healthcare), ABI 3900 DNA Synthesizer (Applied Biosystems), and the like. Enzymatic synthesis includes, but is not limited, to producing polynucleotides by enzymatic amplification, e.g., PCR.

In some embodiments, a polynucleotide is provided that comprises at least 8, at least 9, at least 10, at least 11, at least 12, at least 13, at least 14, or at least 15 contiguous nucleotides of a sequence selected from SEQ ID NOs: 1 to 397, 1063 to 1210, 1363 to 1707, 2064 to 2183, 2312 to 2452, 2673 to 2680, and 2689 and sequences complementary to SEQ ID NOs: 1 to 397, 1063 to 1210, 1363 to 1707, 2064 to 2183, 2312 to 2452, 2673 to 2680, and 2689. In some embodiments, the polynucleotide further comprises a region having a sequence that is not found in, or complementary to, any of SEQ ID NOs: 1 to 397, 1063 to 1210, 1363 to 1707, 2064 to 2183, 2312 to 2452, 2673 to 2680, and 2689. In some embodiments, a polynucleotide is provided that comprises at least 8, at least 9, at least 10, at least 11, at least 12, at least 13, at least 14, or at least 15 contiguous nucleotides of a sequence selected from SEQ ID NOs: 794 to 1043, 2576 to 2672, and 2692, and sequences complementary to SEQ ID NOs: 794 to 1043, 2576 to 2672, and 2692. In some embodiments, the polynucleotide further comprises a region having a sequence that is not found in, or complementary to, any of SEQ ID NOs: 794 to 1043, 2576 to 2672, and 2692.

A "region" can comprise the full-length sequence, or the complement of the full-length sequence, of a particular sequence, such as any of SEQ ID NOs: 1 to 397, 1063 to 1210, 1363 to 1707, 2064 to 2183, 2312 to 2452, 794 to 1043, and 2576 to 2672 or it can comprise a subsequence, or the complement of a subsequence, of a particular sequence, such as any of SEQ ID NOs: 1 to 397, 1063 to 1210, 1363 to 1707, 2064 to 2183, 2312 to 2452, 794 to 1043, and 2576 to 2672. Such subsequences may comprise, in some embodiments, 8, 9, 10, 11, 12, 13, 14, 15, 16, 17, 18, 19, 20, 21, 22, 23, 24, 25, 26, 27, 28, 29, or more contiguous nucleotides from a particular SEQ ID NO or its complement.

In various embodiments, a polynucleotide comprises fewer than 500, fewer than 300, fewer than 200, fewer than 150, fewer than 100, fewer than 75, fewer than 50, fewer than 40, or fewer than 30 nucleotides. In various embodiments, a polynucleotide is between 8 and 200, between 8 and 150, between 8 and 100, between 8 and 75, between 8 and 50, between 8 and 40, or between 8 and 30 nucleotides long.

In some embodiments, the polynucleotide is a primer. In some embodiments, the primer is labeled with a detectable moiety. In some embodiments, a primer is not labeled. A primer, as used herein, is a polynucleotide that is capable of specifically hybridizing to a target RNA or to a cDNA reverse transcribed from the target RNA or to an amplicon that has been amplified from a target RNA or a cDNA (collectively referred to as "template"), and, in the presence of the template, a polymerase and suitable buffers and reagents, can be extended to form a primer extension product.

In some embodiments, the polynucleotide is a probe. In some embodiments, the probe is labeled with a detectable moiety. A detectable moiety, as used herein, includes both directly detectable moieties, such as fluorescent dyes, and indirectly detectable moieties, such as members of binding pairs. When the detectable moiety is a member of a binding pair, in some embodiments, the probe can be detectable by incubating the probe with a detectable label bound to the second member of the binding pair. In some embodiments, a probe is not labeled, such as when a probe is a capture probe, e.g., on a microarray or bead. In some embodiments, a probe is not extendable, e.g., by a polymerase. In other embodiments, a probe is extendable.

In some embodiments, the polynucleotide is a FRET probe that in some embodiments is labeled at the 5'-end with a fluorescent dye (donor) and at the 3'-end with a quencher (acceptor), a chemical group that absorbs (i.e., suppresses) fluorescence emission from the dye when the groups are in close proximity (i.e., attached to the same probe). In other embodiments, the donor and acceptor are not at the ends of the FRET probe. Thus, in some embodiments, the emission spectrum of the donor moiety should overlap considerably with the absorption spectrum of the acceptor moiety.

### 4.1.5.1. Exemplary polynucleotide modifications

In some embodiments, the methods of detecting at least one target RNA described herein employ one or more polynucleotides that have been modified, such as polynucleotides comprising one or more affinity-enhancing nucleotide analogs. Modified polynucleotides useful in the methods described herein include primers for reverse transcription, PCR amplification primers, and probes. In some embodiments, the incorporation of affinity-enhancing nucleotides increases the binding affinity and specificity of a polynucleotide for its target nucleic acid as compared to polynucleotides that contain only deoxyribonucleotides, and allows for the use of shorter polynucleotides or for shorter regions of complementarity between the polynucleotide and the target nucleic acid.

In some embodiments, affinity-enhancing nucleotide analogs include nucleotides comprising one or more base modifications, sugar modifications and/or backbone modifications.

In some embodiments, modified bases for use in affinity-enhancing nucleotide analogs include 5-methylcytosine, isocytosine, pseudoisocytosine, 5-bromouracil, 5-propynyluracil, 6-aminopurine, 2-aminopurine, inosine, diaminopurine, 2-chloro-6-aminopurine, xanthine and hypoxanthine.

In some embodiments, affinity-enhancing nucleotide analogs include nucleotides having modified sugars such as 2'-substituted sugars, such as 2'-O-alkyl-ribose sugars, 2'-amino-deoxyribose sugars, 2'-fluoro- deoxyribose sugars, 2'-fluoro-arabinose sugars, and 2'-O-methoxyethyl-ribose (2'MOE) sugars. In some embodiments, modified sugars are arabinose sugars, or d-arabino-hexitol sugars.

In some embodiments, affinity-enhancing nucleotide analogs include backbone modifications such as the use of peptide nucleic acids (PNA; e.g., an oligomer including nucleobases linked together by an amino acid backbone). Other backbone modifications include phosphorothioate linkages, phosphodiester modified nucleic acids, combinations of phosphodiester and phosphorothioate nucleic acid, methylphosphonate, alkylphosphonates, phosphate esters, alkylphosphonothioates, phosphoramidates, carbamates, carbonates, phosphate triesters, acetamidates, carboxymethyl esters, methylphosphorothioate, phosphorodithioate, p-ethoxy, and combinations thereof.

In some embodiments, a polynucleotide includes at least one affinity-enhancing nucleotide analog that has a modified base, at least nucleotide (which may be the same nucleotide) that has a modified sugar, and/or at least one internucleotide linkage that is non-naturally occurring.

In some embodiments, an affinity-enhancing nucleotide analog contains a locked nucleic acid ("LNA") sugar, which is a bicyclic sugar. In some embodiments, a polynucleotide for use in the methods described herein comprises one or more nucleotides having an LNA sugar. In some embodiments, a polynucleotide contains one or more regions consisting of nucleotides with LNA sugars. In other embodiments, a polynucleotide contains nucleotides with LNA sugars interspersed with deoxyribonucleotides. *See, e.g.,* Frieden, M. et al. (2008) Curr. Pharm. Des. 14(11):1138-1142.

### 4.1.5.2. Exemplary primers

In some embodiments, a primer is provided. In some embodiments, a primer is identical or complementary to at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides of a target RNA. In some embodiments, a primer may also comprise portions or regions that are not identical or complementary to the target RNA. In some embodiments, a region of a primer that is identical or complementary to a target RNA is contiguous, such that any region of a primer that is not identical or complementary to the target RNA does not disrupt the identical or complementary region.

In some embodiments, a primer comprises a portion that is identically present in a target RNA. In some such embodiments, a primer that comprises a region that is identically present in the target RNA is capable of selectively hybridizing to a cDNA that has been reverse transcribed from the RNA, or to an amplicon that has been produced by amplification of the target RNA or cDNA. In some embodiments, the primer is complementary to a sufficient portion of the cDNA or amplicon such that it selectively hybridizes to the cDNA or amplicon under the conditions of the particular assay being used.

As used herein, "selectively hybridize" means that a polynucleotide, such as a primer or probe, will hybridize to a particular nucleic acid in a sample with at least 5-fold greater affinity than it will hybridize to another nucleic acid present in the same sample that has a different nucleotide sequence in the hybridizing region. Exemplary hybridization conditions are discussed in Example 1. In some embodiments, a polynucleotide will hybridize to a particular nucleic acid in a sample with at least 10-fold greater affinity than it will hybridize to another nucleic acid present in the same sample that has a different nucleotide sequence in the hybridizing region.

Nonlimiting exemplary primers include primers comprising sequences that are identically present in, or complementary to a region of, sequences selected from SEQ ID NOs: 1 to 397, 1063 to 1210, 1363 to 1707, 2064 to 2183, 2312 to 2452, 2673 to 2680, and 2689. Exemplary primers also include, but are not limited to, primers comprising regions that are identical or complementary to at least 15 contiguous nucleotides of sequences selected from SEQ ID NOs: 1 to 397, 1063 to 1210, 1363 to 1707, 2064 to 2183, 2312 to 2452, 2673 to 2680, and 2689. Exemplary primers also include, but are not limited to, primers comprising regions that are identical or complementary to at least 15 contiguous nucleotides of sequences selected from SEQ ID NOs: 794 to 1043, 2576 to 2672, and 2692.

In some embodiments, a primer is used to reverse transcribe a target RNA, for example, as discussed herein. In some embodiments, a primer is used to amplify a target RNA or a cDNA reverse transcribed therefrom. Such amplification, in some embodiments, is quantitative PCR, for example, as discussed herein. In some embodiments, a primer comprises a detectable moiety.

### 4.1.5.3. Exemplary probes

In various embodiments, methods of detecting the presence of a lung cancer comprise hybridizing nucleic acids of a human sample with a probe. In some embodiments, the probe comprises a portion that is complementary to a target RNA. In some embodiments, the probe comprises a portion that is identically present in the target RNA. In some such embodiments, a probe that is complementary to a target RNA is complementary to a sufficient portion of the target RNA such that it selectively hybridizes to the target RNA under the conditions of the particular assay being used. In some embodiments, a probe that is complementary to a target RNA is complementary to at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides of the target RNA. In some embodiments, a probe that is complementary to a target RNA comprises a region that is complementary to at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides of the target RNA. That is, a probe that is complementary to a target RNA may also comprise portions or regions that are not complementary to the target RNA. In some embodiments, a region of a probe that is complementary to a target RNA is contiguous, such that any region of a probe that is not complementary to the target RNA does not disrupt the complementary region.

In some embodiments, the probe comprises a portion that is identically present in the target RNA. In some such embodiments, a probe that comprises a region that is identically present in the target RNA is capable of selectively hybridizing to a cDNA that has been reverse transcribed from the RNA, or to an amplicon that has been produced by amplification of the target RNA or cDNA. In some embodiments, the probe is complementary to a sufficient portion of the cDNA or amplicon such that it selectively hybridizes to the cDNA or amplicon under the conditions of the particular assay being used. In some embodiments, a probe that is complementary to a cDNA or amplicon is complementary to at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides of the cDNA or amplicon. In some embodiments, a probe that is complementary to a target RNA comprises a region that is complementary to at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides of the cDNA or amplicon. That is, a probe that is complementary to a cDNA or amplicon may also comprise portions or regions that are not complementary to the cDNA or amplicon. In some embodiments, a region of a probe that is complementary to a cDNA or amplicon is contiguous, such that any region of a probe that is not complementary to the cDNA or amplicon does not disrupt the complementary region.

Nonlimiting exemplary probes include probes comprising sequences set forth in SEQ ID NOs: 1 to 397. Nonlimiting exemplary probes include probes comprising sequences that are identically present in, or complementary to a region of, sequences selected from SEQ ID NOs: 1 to 397, 1063 to 1210, 1363 to 1707, 2064 to 2183, 2312 to 2452, 2673 to 2680, and 2689. Exemplary probes also include, but are not limited to, probes comprising regions that are identical or complementary to at least 15 contiguous nucleotides of sequences selected from SEQ ID NOs: 1 to 397, 1063 to 1210, 1363 to 1707, 2064 to 2183, 2312 to 2452, 2673 to 2680, and 2689.

In some embodiments, the method of detectably quantifying one or more target RNAs comprises: (a) isolating total RNA; (b) reverse transcribing a target RNA to produce a cDNA that is complementary to the target RNA; (c) amplifying the cDNA from (b); and (d) detecting the amount of a target RNA using real time RT-PCR.

As described above, in some embodiments, the real time RT-PCR detection is performed using a FRET probe, which includes, but is not limited to, a TaqMan® probe, a Molecular beacon probe and a Scorpion probe. In some embodiments, the real time RT-PCR detection and quantification is performed with a TaqMan® probe, *i.e.,* a linear probe that typically has a fluorescent dye covalently bound at one end of the DNA and a quencher molecule covalently bound at the other end of the DNA. The FRET probe comprises a sequence that is complementary to a region of the cDNA such that, when the FRET probe is hybridized to the cDNA, the dye fluorescence is quenched, and when the probe is digested during amplification of the cDNA, the dye is released from the probe and produces a fluorescence signal. In such embodiments, the amount of target RNA in the sample is proportional to the amount of fluorescence measured during cDNA amplification.

The TaqMan® probe typically comprises a region of contiguous nucleotides having a sequence that is complementary to a region of a target RNA or its complementary cDNA that is reverse transcribed from the target RNA template *(i.e.,* the sequence of the probe region is complementary to or identically present in the target RNA to be detected) such that the probe is specifically hybridizable to the resulting PCR amplicon. In some embodiments, the probe comprises a region of at least 6 contiguous nucleotides having a sequence that is fully complementary to or identically present in a region of a cDNA that has been reverse transcribed from a target RNA template, such as comprising a region of at least 8 contiguous nucleotides, at least 10 contiguous nucleotides, at least 12 contiguous nucleotides, at least 14 contiguous nucleotides, or at least 16 contiguous nucleotides having a sequence that is complementary to or identically present in a region of a cDNA reverse transcribed from a target RNA to be detected.

In some embodiments, the region of the cDNA that has a sequence that is complementary to the TaqMan® probe sequence is at or near the center of the cDNA molecule. In some embodiments, there are independently at least 2 nucleotides, at least 3 nucleotides, at least 4 nucleotides, at least 5 nucleotides of the cDNA at the 5'-end and at the 3'-end of the region of complementarity.

In some embodiments, Molecular Beacons can be used to detect and quantitate PCR products. Like TaqMan® probes, Molecular Beacons use FRET to detect and quantitate a PCR product via a probe having a fluorescent dye and a quencher attached at the ends of the probe. Unlike TaqMan® probes, Molecular Beacons remain intact during the PCR cycles. Molecular Beacon probes form a stem-loop structure when free in solution, thereby allowing the dye and quencher to be in close enough proximity to cause fluorescence quenching. When the Molecular Beacon hybridizes to a target, the stem-loop structure is abolished so that the dye and the quencher become separated in space and the dye fluoresces. Molecular Beacons are available, e.g., from Gene Link™ (see http://www.genelink.com/newsite/products/mbintro.asp).

In some embodiments, Scorpion probes can be used as both sequence-specific primers and for PCR product detection and quantitation. Like Molecular Beacons, Scorpion probes form a stem-loop structure when not hybridized to a target nucleic acid. However, unlike Molecular Beacons, a Scorpion probe achieves both sequence-specific priming and PCR product detection. A fluorescent dye molecule is attached to the 5'-end of the Scorpion probe, and a quencher is attached to the 3'-end. The 3' portion of the probe is complementary to the extension product of the PCR primer, and this complementary portion is linked to the 5'-end of the probe by a non-amplifiable moiety. After the Scorpion primer is extended, the target-specific sequence of the probe binds to its complement within the extended amplicon, thus opening up the stem-loop structure and allowing the dye on the 5'-end to fluoresce and generate a signal. Scorpion probes are available from, e.g, Premier Biosoft International (see http://www.premierbiosoft.com/tech_notes/Scorpion.html).

In some embodiments, labels that can be used on the FRET probes include colorimetric and fluorescent labels such as Alexa Fluor dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and CyS; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; fluorescent energy transfer dyes, such as thiazole orange-ethidium heterodimer; and, TOTAB.

Specific examples of dyes include, but are not limited to, those identified above and the following: Alexa Fluor 350, Alexa Fluor 405, Alexa Fluor 430, Alexa Fluor 488, Alexa Fluor 500. Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, and, Alexa Fluor 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2', 4',5',7'-Tetrabromosulfonefluorescein, and TET.

Specific examples of fluorescently labeled ribonucleotides useful in the preparation of RT-PCR probes for use in some embodiments of the methods described herein are available from Molecular Probes (Invitrogen), and these include, Alexa Fluor 488-5-UTP, Fluorescein-12-UTP, BODIPY FL-14-UTP, BODIPY TMR-14-UTP, Tetramethylrhodamine-6-UTP, Alexa Fluor 546-14-UTP, Texas Red-5-UTP, and BODIPY TR-14-UTP. Other fluorescent ribonucleotides are available from Amersham Biosciences (GE Healthcare), such as Cy3-UTP and Cy5-UTP.

Examples of fluorescently labeled deoxyribonucleotides useful in the preparation of RT-PCR probes for use in the methods described herein include Dinitrophenyl (DNP)-1'-dUTP, Cascade Blue-7-dUTP, Alexa Fluor 488-5-dUTP, Fluorescein-12-dUTP, Oregon Green 488-5-dUTP, BODIPY FL-14-dUTP, Rhodamine Green-5-dUTP, Alexa Fluor 532-5-dUTP, BODIPY TMR-14-dUTP, Tetramethylrhodamine-6-dUTP, Alexa Fluor 546-14-dUTP, Alexa Fluor 568-5-dUTP, Texas Red-12-dUTP, Texas Red-5-dUTP, BODIPY TR-14-dUTP, Alexa Fluor 594-5-dUTP, BODIPY 630/650-14-dUTP, BODIPY 650/665-14-dUTP; Alexa Fluor 488-7-OBEA-dCTP, Alexa Fluor 546-16-OBEA-dCTP, Alexa Fluor 594-7-OBEA-dCTP, Alexa Fluor 647-12-OBEA-dCTP. Fluorescently labeled nucleotides are commercially available and can be purchased from, e.g., Invitrogen.

In some embodiments, dyes and other moieties, such as quenchers, are introduced into polynucleotide used in the methods described herein, such as FRET probes, via modified nucleotides. A "modified nucleotide" refers to a nucleotide that has been chemically modified, but still functions as a nucleotide. In some embodiments, the modified nucleotide has a chemical moiety, such as a dye or quencher, covalently attached, and can be introduced into a polynucleotide, for example, by way of solid phase synthesis of the polynucleotide. In other embodiments, the modified nucleotide includes one or more reactive groups that can react with a dye or quencher before, during, or after incorporation of the modified nucleotide into the nucleic acid. In specific embodiments, the modified nucleotide is an amine-modified nucleotide, i.e., a nucleotide that has been modified to have a reactive amine group. In some embodiments, the modified nucleotide comprises a modified base moiety, such as uridine, adenosine, guanosine, and/or cytosine. In specific embodiments, the amine-modified nucleotide is selected from 5-(3-aminoallyl)-UTP; 8-[(4-amino)butyl]-amino-ATP and 8-[(6-amino)butyl]-amino-ATP; N6-(4-amino)butyl-ATP, N6-(6-amino)butyl-ATP, N4-[2,2-oxy-bis-(ethylamine)]-GTP; N6-(6-Amino)hexyl-ATP; 8-[(6-Amino)hexyl]-amino-ATP; 5-propargylamino-CTP, 5-propargylamino-UTP. In some embodiments, nucleotides with different nucleobase moieties are similarly modified, for example, 5-(3-aminoallyl)-GTP instead of 5-(3-aminoallyl)-UTP. Many amine modified nucleotides are commercially available from, e.g., Applied Biosystems, Sigma, Jena Bioscience and TriLink.

Exemplary detectable moieties also include, but are not limited to, members of binding pairs. In some such embodiments, a first member of a binding pair is linked to a polynucleotide. The second member of the binding pair is linked to a detectable label, such as a fluorescent label. When the polynucleotide linked to the first member of the binding pair is incubated with the second member of the binding pair linked to the detectable label, the first and second members of the binding pair associate and the polynucleotide can be detected. Exemplary binding pairs include, but are not limited to, biotin and streptavidin, antibodies and antigens, etc.

In some embodiments, multiple target RNAs are detected in a single multiplex reaction. In some such embodiments, each probe that is targeted to a unique cDNA is spectrally distinguishable when released from the probe. Thus, each target RNA is detected by a unique fluorescence signal.

One skilled in the art can select a suitable detection method for a selected assay, e.g., a real-time RT-PCR assay. The selected detection method need not be a method described above, and may be any method.

### 4.2. Exemplary compositions and kits

In another aspect, compositions are provided. In some embodiments, compositions are provided for use in the methods described herein.

In some embodiments, a composition comprises at least one polynucleotide. In some embodiments, a composition comprises at least one primer. In some embodiments, a composition comprises at least one probe. In some embodiments, a composition comprises at least one primer and at least one probe.

In some embodiments, compositions arc provided that comprise at least one target RNA-specific primer. The term "target RNA-specific primer" encompasses primers that have a region of contiguous nucleotides having a sequence that is (i) identically present in one of SEQ ID NOs: 1 to 397, 1063 to 1210, 1363 to 1707, 2064 to 2183, 2312 to 2452, 2673 to 2680, and 2689, or (ii) complementary to the sequence of a region of contiguous nucleotides found in one of SEQ ID NOs: 1 to 397, 1063 to 1210, 1363 to 1707, 2064 to 2183, 2312 to 2452, 2673 to 2680, and 2689.

In some embodiments, compositions are provided that comprise at least one target RNA-specific probe. The term "target RNA-specific probe" encompasses probes that have a region of contiguous nucleotides having a sequence that is (i) identically present in one of SEQ ID NOs: 1 to 397, 1063 to 1210, 1363 to 1707, 2064 to 2183, 2312 to 2452, 2673 to 2680, and 2689, or (ii) complementary to the sequence of a region of contiguous nucleotides found in one of SEQ ID NOs: 1 to 397, 1063 to 1210, 1363 to 1707, 2064 to 2183, 2312 to 2452, 2673 to 2680, and 2689.

In some embodiments, target RNA-specific primers and probes comprise deoxyribonucleotides. In other embodiments, target RNA-specific primers and probes comprise at least one nucleotide analog. Nonlimiting exemplary nucleotide analogs include, but are not limited to, analogs described herein, including LNA analogs and peptide nucleic acid (PNA) analogs. In some embodiments, target RNA-specific primers and probes comprise at least one nucleotide analog which increases the hybridization binding energy (e.g., an affinity-enhancing nucleotide analog, discussed above). In some embodiments, a target RNA-specific primer or probe in the compositions described herein binds to one target RNA in the sample. In some embodiments, a single primer or probe binds to multiple target RNAs, such as multiple isomirs.

In some embodiments, more than one primer or probe specific for a single target RNA is present in the compositions, the primers or probes capable of binding to overlapping or spatially separated regions of the target RNA.

It will be understood, even if not explicitly stated hereinafter, that in some embodiments in which the compositions described herein are designed to hybridize to cDNAs reverse transcribed from target RNAs, the composition comprises at least one target RNA-specific primer or probe (or region thereof) having a sequence that is identically present in a target RNA (or region thereof).

In some embodiments, a target RNA is capable of specifically hybridizing to at least one probe sequence in one of Tables 6, 7, 8, 9, 32, 33, or 34. In some embodiments, a target RNA comprises at least 15 contiguous nucleotides of a sequence selected from SEQ ID NOs: 794 to 1043, 2576 to 2672, and 2692. In some embodiments, a target RNA comprises a sequence that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689. In some embodiments, a target RNA, in its mature form, comprises fewer than 30 nucleotides. In some embodiments, a target RNA is a microRNA.

In some embodiments, the composition comprises a plurality of target RNA-specific primers and/or probes for each of at least 2, at least 3, at least 4, at least 5, at least 6, at least 7, or at least 8 target RNAs, the target RNAs comprising a region of contiguous nucleotides having a sequence that is identically present in one of SEQ ID NOs: 794 to 1043, 2576 to 2672, and 2692. In some embodiments, the plurality includes a target RNA-specific primer and/or probe specific for each of at least 2, at least 3, at least 4, at least 5, at least 6, at least 7, at least 8, at least 9, at least 10, at least 11, or at least 12 target RNAs, the target RNAs comprising a region of contiguous nucleotides having a sequence that is identically present in one of SEQ ID NOs: 794 to 1043, 2576 to 2672, and 2692. In some embodiments, the plurality includes a target RNA-specific primer and/or probe specific for each of at least 2, at least 3, at least 4, at least 5, at least 6, at least 7, at least 8, at least 9, at least 10, at least 15, at least 20, at least 25, at least 30, at least 40, at least 50, at least 75, or at least 100 target RNAs comprising a region of contiguous nucleotides having a sequence that is identically present in one of SEQ ID NOs: 794 to 1043, 2576 to 2672, and 2692. It will be understood that, in some embodiments, target RNAs described herein comprise a sequence identically present in a sequence set forth in at least one of Tables 4, 5, and 38. It is understood that where a sequence includes thymine (T) bases, a target RNA may contain uracil (U) bases instead.

In some embodiments, a composition is an aqueous composition. In some embodiments, the aqueous composition comprises a buffering component, such as phosphate, tris, HEPES, etc., and/or additional components, as discussed below. In some embodiments, a composition is dry, for example, lyophilized, and suitable for reconstitution by addition of fluid. A dry composition may include a buffering component and/or additional components.

In some embodiments, a composition comprises one or more additional components. Additional components include, but are not limited to, salts, such as NaCl, KCI, and MgCl₂; polymerases, including thermostable polymerases; dNTPs; RNase inhibitors; bovine serum albumin (BSA) and the like; reducing agents, such as β-mercaptoethanol; EDTA and the like; etc. One skilled in the art can select suitable composition components depending on the intended use of the composition.

In some embodiments, an addressable microarray component is provided that comprises target RNA-specific probes attached to a substrate.

Microarrays for use in the methods described herein comprise a solid substrate onto which the probes are covalently or non-covalently attached. In some embodiments, probes capable of hybridizing to one or more target RNAs or cDNAs are attached to the substrate at a defined location ("addressable array"). Probes can be attached to the substrate in a wide variety of ways, as will be appreciated by those in the art. In some embodiments, the probes are synthesized first and subsequently attached to the substrate. In other embodiments, the probes are synthesized on the substrate. In some embodiments, probes are synthesized on the substrate surface using techniques such as photopolymerization and photolithography.

In some embodiments, the solid substrate is a material that is modified to contain discrete individual sites appropriate for the attachment or association of the probes and is amenable to at least one detection method. Representative examples of substrates include glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses and plastics. In some embodiments, the substrates allow optical detection without appreciably fluorescing.

In some embodiments, the substrate is planar. In other embodiments, probes are placed on the inside surface of a tube, such as for flow-through sample analysis to minimize sample volume. In other embodiments, probes can be in the wells of multi-well plates. In still other embodiments, probes can be attached to an addressable microbead array. In yet other embodiments, the probes can be attached to a flexible substrate, such as a flexible foam, including closed cell foams made of particular plastics.

The substrate and the probe can each be derivatized with functional groups for subsequent attachment of the two. For example, in some embodiments, the substrate is derivatized with one or more chemical functional groups including, but not limited to, amino groups, carboxyl groups, oxo groups and thiol groups. In some embodiments, probes are attached directly to the substrate through one or more functional groups. In some embodiments, probes are attached to the substrate indirectly through a linker (*i.e.,* a region of contiguous nucleotides that space the probe regions involved in hybridization and detection away from the substrate surface). In some embodiments, probes are attached to the solid support through the 5' terminus. In other embodiments, probes are attached through the 3' terminus. In still other embodiments, probes are attached to the substrate through an internal nucleotide. In some embodiments the probe is attached to the solid support non-covalently, e.g., via a biotin-streptavidin interaction, wherein the probe biotinylated and the substrate surface is covalently coated with streptavidin.

In some embodiments, the compositions comprise a microarray having probes attached to a substrate, wherein at least one of the probes (or a region thereof) comprises a sequence that is identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, at least 2, at least 5, at least 10, at least 15, at least 20, at least 25, at least 30, at least 40, at least 50, or at least 100 of the probes comprise a sequence that is identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, the microarray comprises at least one target RNA-specific probe comprising a sequence that is identically present in, or complementary to a region of, one of SEQ ID NOs: 1 to 397, 1063 to 1210, 1363 to 1707, 2064 to 2183, 2312 to 2452, 2673 to 2680, and 2689 and at least one target RNA-specific probe comprising a sequence that is identically present in, or complementary to a region of, a target RNA of the human miRNome. In some embodiments, the microarray comprises each target RNA-specific probe at only one location on the microarray. In some embodiments, the microarray comprises at least one target RNA-specific probe at multiple locations on the microarray.

As used herein, the terms "complementary" or "partially complementary" to a target RNA (or target region thereof), and the percentage of "complementarity" of the probe sequence to that of the target RNA sequence is the percentage "identity" to the reverse complement of the sequence of the target RNA. In determining the degree of "complementarity" between probes used in the compositions described herein (or regions thereof) and a target RNA, such as those disclosed herein, the degree of "complementarity" is expressed as the percentage identity between the sequence of the probe (or region thereof) and the reverse complement of the sequence of the target RNA that best aligns therewith. The percentage is calculated by counting the number of aligned bases that are identical as between the 2 sequences, dividing by the total number of contiguous nucleotides in the probe, and multiplying by 100.

In some embodiments, the microarray comprises at least one probe having a region with a sequence that is fully complementary to a target region of a target RNA. In other embodiments, the microarray comprises at least one probe having a region with a sequence that comprises one or more base mismatches when compared to the sequence of the best-aligned target region of a target RNA.

As noted above, a "region" of a probe or target RNA, as used herein, may comprise or consist of 8, 9, 10, 11, 12, 13, 14, 15, 16, 17, 18, 19, 20, 21, 22, 23, 24, 25, 26, 27, 28, 29 or more contiguous nucleotides from a particular SEQ ID NO or the complement thereof. In some embodiments, the region is of the same length as the probe or the target RNA. In other embodiments, the region is shorter than the length of the probe or the target RNA.

In some embodiments, the microarray comprises at least one probe having a region of at least 10, at least 11, at least 13, at least 14, at least 15, at least 16, at least 17, at least 18, at least 19, at least 20, at least 21, at least 22, at least 23, at least 24, or at least 25 contiguous nucleotides with a sequence that is identically present in, or complementary to a region of, one of SEQ ID NOs: 1 to 397, 1063 to 1210, 1363 to 1707, 2064 to 2183, 2312 to 2452, 2673 to 2680, and 2689.

In some embodiments, the microarray comprises at least one probe having a region with a sequence that is identically present in, or complementary to a region of, a probe sequence in one of Tables 6, 7, 8, 9, 32, 33, and 34. In some embodiments, a microarray further comprises at least one probe that does not have a region that is identically present in, or complementary to a region of, a probe sequence in one of Tables 6, 7, 8, 9, 32, 33, and 34.

In some embodiments, the microarray comprises at least one, at least two, at least three, at least five, at least 10, or at least 15 probes that each comprise a region with a sequence that is identically present in, or complementary to a region of, a probe sequence in Table 6. In some embodiments, the microarray comprises at least one, at least two, at least three, at least five, at least eight, at least 10, at least 12, at least 15, at least 20, or at least 25 probes that each comprise a region with a sequence that is identically present in, or complementary to a region of, a probe sequence in Table 7. In some embodiments, the microarray comprises at least one, at least two, at least three, at least five, at least six, or at least seven probes that each comprise a region with a sequence that is identically present in, or complementary to a region of, a probe sequence in Table 8. In some embodiments, the microarray comprises at least one, at least two, at least three, at least four, at least five, at least ten, at least 15, at least 20, at least 25, at least 30, at least 40, or at least 50 probes that each comprise a region with a sequence that is identically present in, or complementary to a region of, a probe sequence in Table 9. In some embodiments, the microarray comprises at least one, at least two, at least five, at least 10, at least 15, at least 20, at least 25, at least 30, or at least 40 probes that each comprise a region with a sequence that is identically present in, or complementary to a region of, a probe sequence in one of Tables 32 or 33. In some embodiments, the microarray comprises at least one, at least two, at least five, at least 10, at least 15, at least 20, at least 30, at least 40, at least 50, at least 60, or at least 70 probes that each comprise a region with a sequence that is identically present in, or complementary to a region of, a probe sequence in Table 34.

In some embodiments, the microarrays comprise probes having a region with a sequence that is complementary to target RNAs that comprise a substantial portion of the human miRNome (*i.e*., the publicly known microRNAs that have been accessioned by others into miRBase (http://micromn.sanger.ac.uk/ at the time the microarray is fabricated), such as at least about 60%, at least about 70%, at least about 80%, at least about 90%, or at least about 95% of the human miRNome. In some embodiments, the microarrays comprise probes that have a region with a sequence that is identically present in target RNAs that comprise a substantial portion of the human miRNome, such as at least about 60%, at least about 70%, at least about 80%, at least about 90%, or at least about 95% of the human miRNome.

In some embodiments, components are provided that comprise probes attached to microbeads, such as those sold by Luminex, each of which is internally dyed with red and infrared fluorophores at different intensities to create a unique signal for each bead. In some embodiments, the compositions useful for carrying out the methods described herein include a plurality of microbeads, each with a unique spectral signature. Each uniquely labeled microbead is attached to a unique target RNA-specific probe such that the unique spectral signature from the dyes in the bead is associated with a particular probe sequence. Nonlimiting exemplary probe sequences include SEQ ID NOs: 1 to 397, 1063 to 1210, 1363 to 1707, 2064 to 2183, 2312 to 2452, 2673 to 2680, and 2689. Nonlimiting exemplary probe sequences also include probes comprising a region that is identically present in, or complementary to, a sequence selected from SEQ ID NOs: 1 to 397, 1063 to 1210, 1363 to 1707, 2064 to 2183, 2312 to 2452, 2673 to 2680, and 2689. In some embodiments, a probe sequence comprises at least 8, at least 9, at least 10, at least 11, at least 12, at least 13, at least 14, at least 15, at least 16, at least 17, at least 18, at least 19, at least 20, at least 21, at least 22, at least 23, or at least 24 contiguous nucleotides that are identically present in, or complementary to a region of, one of SEQ ID NOs: 1 to 397, 1063 to 1210, 1363 to 1707, 2064 to 2183, 2312 to 2452, 2673 to 2680, and 2689.

In some embodiments, a uniquely labeled microbead has attached thereto a probe having a region with a sequence that is identically present in, or complementary to a region of, one of SEQ ID NOs: 1 to 397, 1063 to 1210, 1363 to 1707, 2064 to 2183, 2312 to 2452, 2673 to 2680, and 2689. In other embodiments, the uniquely labeled microbead has attached thereto a probe having a region with a sequence that comprises one or more base mismatches when compared to the most similar sequence selected from SEQ ID NOs: 1 to 397, 1063 to 1210, 1363 to 1707, 2064 to 2183, 2312 to 2452, 2673 to 2680, and 2689, and sequences complementary to SEQ ID NOs: 1 to 397, 1063 to 1210, 1363 to 1707, 2064 to 2183, 2312 to 2452, 2673 to 2680, and 2689.

In some embodiments, a composition is provided that comprises a plurality of uniquely labeled microbeads, wherein at least one microbead has attached thereto a probe having a region of at least 10, at least 11, at least 13, at least 14, at least 15, at least 16, at least 17, at least 18, at least 19, at least 20, at least 21, at least 22, at least 23, at least 24, or at least 25 contiguous nucleotides with a sequence that is identically present in, or complementary to a region of, one of SEQ ID NOs: 1 to 397, 1063 to 1210, 1363 to 1707, 2064 to 2183, 2312 to 2452, 2673 to 2680, and 2689.

In some embodiments, a composition is provided that comprises a plurality of uniquely labeled microbeads, wherein at least one microbead has attached thereto a probe having a region of at least 10, at least 11, at least 13, at least 14, at least 15, at least 16, at least 17, at least 18, at least 19, at least 20, at least 21, at least 22, at least 23, at least 24, or at least 25 contiguous nucleotides with a sequence that is identically present in, or complementary to a region of, a probe sequence in one of Tables 6, 7, 8, 9, 32, 33, and 34. In some embodiments, the composition further comprises at least one uniquely labeled microbead having attached thereto a probe that does not have a region that is identically present in, or complementary to a region of, a probe sequence in one of Tables 6, 7, 8, 9, 32, 33, and 34.

In some embodiments, the compositions comprise at least one, at least two, at least three, at least five, at least 10, or at least 15 uniquely labeled microbeads that each have attached thereto a unique target RNA-specific probe having a region with a sequence that is identically present in, or complementary to a region of, a probe sequence in Table 6. In some embodiments, the compositions comprise at least one, at least two, at least three, at least five, at least eight, at least 10, at least 12, at least 15, at least 20, or at least 25 uniquely labeled microbeads that each have attached thereto a unique target RNA-specific probe having a region with a sequence that is identically present in, or complementary to a region of, a probe sequence in Table 7. In some embodiments, the compositions comprise at least one, at least two, at least three, at least five, at least six, or at least seven uniquely labeled microbeads that each have attached thereto a unique target RNA-specific probe having a region with a sequence that is identically present in, or complementary to a region of, a probe sequence in Table 8. In some embodiments, the compositions comprise at least one, at least two, at least three, at least four, at least five, at least ten, at least 15, at least 20, at least 25, at least 30, at least 40, or at least 50 uniquely labeled microbeads that each have attached thereto a unique target RNA-specific probe having a region with a sequence that is identically present in, or complementary to a region of, a probe sequence in Table 9. In some embodiments, the compositions comprise at least one, at least two, at least five, at least 10, at least 15, at least 20, at least 25, at least 30, or at least 40 uniquely labeled microbeads that each have attached thereto a unique target RNA-specific probe having a region with a sequence that is identically present in, or complementary to a region of, a probe sequence in one of Tables 32 or 33. In some embodiments, the compositions comprise at least one, at least two, at least five, at least 10, at least 15, at least 20, at least 30, at least 40, at least 50, at least 60, or at least 70 uniquely labeled microbeads that each have attached thereto a unique target RNA-specific probe having a region with a sequence that is identically present in, or complementary to a region of, a probe sequence in Table 34.

In some embodiments, the compositions comprise a plurality of uniquely labeled microbeads, wherein the plurality comprises at least one microbead having attached thereto a probe having a region with a sequence that is identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, the plurality comprises at least two, at least five, at least 10, at least 15, at least 20, at least 25, at least 30, at least 40, at least 50, at least 60, at least 75, or at least 100 microbeads each of which having attached thereto a probe having a region with a sequence that is identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, a composition comprises at least one uniquely labeled microbead having attached thereto a target RNA-specific probe having a region with a sequence that is not present in, or complementary to a region of, any of SEQ ID NOs: 1 to 397, 1063 to 1210, 1363 to 1707, 2064 to 2183, 2312 to 2452, 2673 to 2680, and 2689.

In some embodiments, the compositions comprise a plurality of uniquely labeled microbeads, at least one of which has attached thereto a probe having a region with a sequence that identically present in, or complementary to a region of, one of SEQ ID NOs: 1 to 397, 1063 to 1210, 1363 to 1707, 2064 to 2183, 2312 to 2452, 2673 to 2680, and 2689 and at least a second bead that has attached thereto a probe having a region with a sequence that is identically present in, or complementary to a region of, a target RNA from the human miRNome.

In some embodiments, the compositions comprise a plurality of uniquely labeled microbeads, each of which has attached thereto a unique probe having a region that is complementary to target RNAs that comprise a substantial portion of the human miRNome, such as at least about 60%, at least about 70%, at least about 80%, at least about 90%, or at least about 95% of the human miRNome. In some embodiments, the compositions comprise a plurality of uniquely labeled microbeads having attached thereto a unique probe having a region with a sequence that is identically present in target RNAs that comprise a substantial portion of the human miRNome, such as at least about 60%, at least about 70%, at least about 80%, at least about 90%, or at least about 95% of the human miRNome.

In some embodiments, compositions are provided that comprise at least one polynucleotide for detecting at least one target RNA. In some embodiments, the polynucleotide is used as a primer for a reverse transcriptase reaction. In some embodiments, the polynucleotide is used as a primer for amplification. In some embodiments, the polynucleotide is used as a primer for RT-PCR. In some embodiments, the polynucleotide is used as a probe for detecting at least one target RNA. In some embodiments, the polynucleotide is detectably labeled. In some embodiments, the polynucleotide is a FRET probe. In some embodiments, the polynucleotide is a TaqMan® probe, a Molecular Beacon, or a Scorpion probe.

In some embodiments, a composition comprises at least one FRET probe having a sequence that is identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, a composition comprises at least two, at least five, at least 10, at least 15, at least 20, at least 25, at least 30, at least 40, at least 50, at least 60, at least 75, or at least 100 FRET probes, each of which has a sequence that is identically present in, or complementary to a region of, a different one of SEQ ID NOs: 1 to 397, 1063 to 1210, 1363 to 1707, 2064 to 2183, 2312 to 2452, 2673 to 2680, and 2689.

In some embodiments, a FRET probe is labeled with a donor/acceptor pair such that when the probe is digested during the PCR reaction, it produces a unique fluorescence emission that is associated with a specific target RNA. In some embodiments, when a composition comprises multiple FRET probes, each probe is labeled with a different donor/acceptor pair such that when the probe is digested during the PCR reaction, each one produces a unique fluorescence emission that is associated with a specific probe sequence and/or target RNA. In some embodiments, the sequence of the FRET probe is complementary to a target region of a target RNA. In other embodiments, the FRET probe has a sequence that comprises one or more base mismatches when compared to the sequence of the best-aligned target region of a target RNA.

In some embodiments, a composition comprises a FRET probe consisting of at least 8, at least 9, at least 10, at least 11, at least 13, at least 14, at least 15, at least 16, at least 17, at least 18, at least 19, at least 20, at least 21, at least 22, at least 23, at least 24, or at least 25 nucleotides, wherein at least a portion of the sequence is identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, at least 8, at least 9, at least 10, at least 11, at least 13, at least 14, at least 15, at least 16, at least 17, at least 18, at least 19, at least 20, at least 21, at least 22, at least 23, at least 24, or at least 25 nucleotides of the FRET probe are identically present in, or complementary to a region of, one of SEQ ID NOs: 1 to 397, 1063 to 1210, 1363 to 1707, 2064 to 2183, 2312 to 2452, 2673 to 2680, and 2689. In some embodiments, the FRET probe has a sequence with one, two or three base mismatches when compared to the sequence or complement of one of SEQ ID NOs: 1 to 397, 1063 to 1210, 1363 to 1707, 2064 to 2183, 2312 to 2452, 2673 to 2680, and 2689.

In some embodiments, a composition comprises a FRET probe consisting of at least 8, at least 9, at least 10, at least 11, at least 13, at least 14, at least 15, at least 16, at least 17, at least 18, at least 19, at least 20, at least 21, at least 22, at least 23, at least 24, or at least 25 nucleotides, wherein at least a portion of the sequence is identically present in, or complementary to a region of, a probe sequence in one of Tables 6, 7, 8, 9, 32, 33, and 34. In some embodiments, a composition comprises at least one FRET probe that does not comprise a portion that is identically present in, or complementary to a region of, a probe sequence in one of Tables 6, 7, 8, 9, 32, 33, and 34.

In some embodiments, the compositions comprise at least one, at least two, at least three, at least five, at least 10, or at least 15 uniquely labeled target RNA-specific FRET probes, each comprising a sequence that is identically present in, or complementary to a region of, a probe sequence in Table 6. In some embodiments, the compositions comprise at least one, at least two, at least three, at least five, at least eight, at least 10, at least 12, at least 15, at least 20, or at least 25 uniquely labeled target RNA-specific FRET probes, each of which comprises a sequence that is identically present in, or complementary to a region of, a probe sequence in Table 7. In some embodiments, the compositions comprise at least one, at least two, at least three, at least five, at least six, or at least seven uniquely labeled target RNA-specific FRET probes, each of which comprises a sequence that is identically present in, or complementary to a region of, a probe sequence in Table 8. In some embodiments, the compositions comprise at least one, at least two, at least three, at least four, at least five, at least ten, at least 15, at least 20, at least 25, at least 30, at least 40, or at least 50 uniquely labeled target RNA-specific FRET probes, each of which comprises a sequence that is identically present in, or complementary to a region of, a probe sequence in Table 9. In some embodiments, the compositions comprise at least one, at least two, at least five, at least 10, at least 15, at least 20, at least 25, at least 30, or at least 40 uniquely labeled target RNA-specific FRET probes, each of which comprises a sequence that is identically present in, or complementary to a region of, a probe sequence in one of Tables 32 or 33. In some embodiments, the compositions comprise at least one, at least two, at least five, at least 10, at least 15, at least 20, at least 30, at least 40, at least 50, at least 60, or at least 70 uniquely labeled target RNA-specific FRET probes, each of which comprises a sequence that is identically present in, or complementary to a region of, a probe sequence in Table 34.

In some embodiments, a kit comprises a polynucleotide discussed above. In some embodiments, a kit comprises at least one primer and/or probe discussed above. In some embodiments, a kit comprises at least one polymerase, such as a thermostable polymerase. In some embodiments, a kit comprises dNTPs. In some embodiments, kits for use in the real time RT-PCR methods described herein comprise one or more target RNA-specific FRET probes and/or one or more primers for reverse transcription of target RNAs and/or one or more primers for amplification of target RNAs or cDNAs reverse transcribed therefrom.

In some embodiments, one or more of the primers and/or probes is "linear". A "linear" primer refers to a polynucleotide that is a single stranded molecule, and typically does not comprise a short region of, for example, at least 3, 4 or 5 contiguous nucleotides, which are complementary to another region within the same polynucleotide such that the primer forms an internal duplex. In some embodiments, the primers for use in reverse transcription comprise a region of at least 4, at least 5, at least 6, at least 7 or more contiguous nucleotides at the 3'-end that has a sequence that is complementary to region of at least 4, at least 5, at least 6, at least 7 or more contiguous nucleotides at the 5'-end of a target RNA.

In some embodiments, a kit comprises one or more pairs of linear primers (a "forward primer" and a "reverse primer") for amplification of a cDNA reverse transcribed from a target RNA. Accordingly, in some embodiments, a first primer comprises a region of at least 4, at least 5, at least 6, at least 7, at least 8, at least 9, or at least 10 contiguous nucleotides having a sequence that is identical to the sequence of a region of at least 4, at least 5, at least 6, at least 7, at least 8, at least 9, or at least 10 contiguous nucleotides at the 5'-end of a target RNA. Furthermore, in some embodiments, a second primer comprises a region of at least 4, at least 5, at least 6, at least 7, at least 8, at least 9, or at least 10 contiguous nucleotides having a sequence that is complementary to the sequence of a region of at least 4, at least 5, at least 6, at least 7, at least 8, at least 9, or at least 10 contiguous nucleotides at the 3'-end of a target RNA. In some embodiments, the kit comprises at least a first set of primers for amplification of a cDNA that is reverse transcribed from a target RNA capable of specifically hybridizing to a nucleic acid comprising a sequence identically present in one of SEQ ID NOs: 1 to 397, 1063 to 1210, 1363 to 1707, 2064 to 2183, 2312 to 2452, 2673 to 2680, and 2689 and/or a cDNA that is reverse transcribed from a target RNA that comprises at least 15 contiguous nucleotides of a sequence selected from SEQ ID NOs: 794 to 1043, 2576 to 2672, and 2692.

In some embodiments, the kit comprises at least two, at least five, at least 10, at least 15, at least 20, at least 25, at least 30, at least 40, at least 50, at least 60, at least 75, or at least 100 sets of primers, each of which is for amplification of a cDNA that is reverse transcribed from a different target RNA capable of specifically hybridizing to a sequence selected from SEQ ID NOs: 1 to 397, 1063 to 1210, 1363 to 1707, 2064 to 2183, 2312 to 2452, 2673 to 2680, and 2689 and/or a cDNA that is reverse transcribed from a target RNA that comprises at least 15 contiguous nucleotides of a sequence selected from SEQ ID NOs: 794 to 1043, 2576 to 2672, and 2692. In some embodiments, the kit comprises at least one set of primers that is capable of amplifying more than one cDNA reverse transcribed from a target RNA in a sample.

In some embodiments, probes and/or primers for use in the compositions described herein comprise deoxyribonucleotides. In some embodiments, probes and/or primers for use in the compositions described herein comprise deoxyribonucleotides and one or more nucleotide analogs, such as LNA analogs or other duplex-stabilizing nucleotide analogs described above. In some embodiments, probes and/or primers for use in the compositions described herein comprise all nucleotide analogs. In some embodiments, the probes and/or primers comprise one or more duplex-stabilizing nucleotide analogs, such as LNA analogs, in the region of complementarity.

In some embodiments, the compositions described herein also comprise probes, and in the case of RT-PCR, primers, that are specific to one or more housekeeping genes for use in normalizing the quantities of target RNAs. Such probes (and primers) include those that are specific for one or more products of housekeeping genes selected from U6 snRNA, RNU44, RNU48, U47, 7SL scRNA, U1 snRNA, 5.8S rRNA, and U87 scaRNA.

In some embodiments, the kits for use in real time RT-PCR methods described herein further comprise reagents for use in the reverse transcription and amplification reactions. In some embodiments, the kits comprise enzymes such as reverse transcriptase, and a heat stable DNA polymerase, such as Taq polymerase. In some embodiments, the kits further comprise deoxyribonucleotide triphosphates (dNTPs) for use in reverse transcription and amplification. In further embodiments, the kits comprise buffers optimized for specific hybridization of the probes and primers.

### 4.2.1. Exemplary normalization of RNA levels

In some embodiments, quantitation of target RNA expression levels requires assumptions to be made about the total RNA per cell and the extent of sample loss during sample preparation. In order to correct for differences between different samples or between samples that are prepared under different conditions, the quantities of target RNAs in some embodiments are normalized to the expression of at least one endogenous housekeeping gene.

Appropriate genes for use as reference genes in the methods described herein include those as to which the quantity of the product does not vary between normal samples and samples from lung cancer patients, or between different cell lines or under different growth and sample preparation conditions. In some embodiments, endogenous housekeeping genes useful as normalization controls in the methods described herein include, but are not limited to, U6 snRNA, RNU44, RNU48, U47, 7SL scRNA, U1 snRNA, 5.8S rRNA, and U87 scaRNA. In typical embodiments, the at least one endogenous housekeeping gene for use in normalizing the measured quantity of microRNAs is selected from U6 snRNA, RNU44, RNU48, U47, 7SL scRNA, U1 snRNA, 5.8S rRNA, and U87 scaRNA. In some embodiments, one housekeeping gene is used for normalization. In some embodiments, more than one housekeeping gene is used for normalization.

### 4.2.2. Exemplary qualitative methods

In some embodiments, methods comprise detecting a qualitative change in a target RNA profile generated from a human sample as compared to a normal target RNA profile (in some exemplary embodiments, a target RNA profile of a control sample). Some qualitative changes in the expression profile are indicative of the presence of lung cancer in a sample from a subject. The term "target RNA profile" refers to a set of data regarding the concurrent expression of a plurality of target RNAs in the same sample.

In some embodiments, at least one, at least two, at least three, at least five, at least 10, or at least 15 of the target RNAs of the plurality of target RNAs are capable of specifically hybridizing to a probe sequence in Table 6. In some embodiments, at least one, at least two, at least three, at least five, at least eight, at least 10, at least 12, at least 15, at least 20, or at least 25 of the target RNAs of the plurality of target RNAs are capable of specifically hybridizing to a a probe sequence in Table 7. In some embodiments, at least one, at least two, at least three, at least five, at least six, or at least seven of the target RNAs of the plurality of target RNAs are capable of specifically hybridizing to a probe sequence in Table 8. In some embodiments, at least one, at least two, at least three, at least four, at least five, at least ten, at least 15, at least 20, at least 25, at least 30, at least 40, or at least 50 of the target RNAs of the plurality of target RNAs are capable of specifically hybridizing to a probe sequence in Table 9. In some embodiments, at least one, at least two, at least five, at least 10, at least 15, at least 20, at least 25, at least 30, or at least 40 of the target RNAs of the plurality of target RNAs are capable of specifically hybridizing to a probe sequence in one of Table 32 or 33. In some embodiments, at least one, at least two, at least five, at least 10, at least 15, at least 20, at least 30, at least 40, at least 50, at least 60, or at least 70 of the target RNAs of the plurality of target RNAs is capable of specifically hybridizing to a probe sequence in Table 34.

In some embodiments, at least one, at least two, at least five, at least 10, at least 15, at least 20, at least 25, at least 30, at least 40, at least 50, at least 60, or at least 75 of the plurality of target RNAs comprises a sequence that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689. In some embodiments, at least one, at least two, at least five, at least 10, at least 15, at least 20, at least 25, at least 30, at least 40, at least 50, at least 60, or at least 70 of the plurality of target RNAs comprises at least 15 contiguous nucleotides of a sequence selected from SEQ ID NOs: 794 to 1043, 2576 to 2672, and 2692. In some embodiments, a target RNA, in its mature form, comprises fewer than 30 nucleotides. In some embodiments, a target RNA is a microRNA.

Qualitative expression data for use in preparing target RNA expression profiles is obtained using any suitable analytical method, including the analytical methods presented herein.

In some embodiments, for example, concurrent expression data are obtained using, e.g., a microarray, as described above. Thus, in addition to use for quantitative expression level assays of specific target RNAs as described above, a microarray comprising probes having sequences that are complementary to a substantial portion of the miRNome may be employed to carry out target RNA gene expression profiling, for analysis of target RNA expression patterns.

In some embodiments, distinct target RNA signatures are associated with established markers for lung cancer. In some embodiments, distinct target RNA signatures are associated with established markers for lung cancer caused by bacterial infection, such as for lung cancer caused by gram-positive bacterial infection, lung cancer caused by gram-negative bacterial infection or lung cancer caused by mycobacterial infection. In some embodiments, distinct target RNA signatures are associated with established markers for lung cancer caused by viral infection. In some embodiments, distinct target RNA signatures are associated with established markers for lung cancer caused by multiple infection, such as by co-infection with bacteria and viruses, or by co-infection with more than one viral or more than one bacterial strain. In some embodiments, distinct target RNA signatures are associated directly with the level of severity of the lung cancer.

According to the expression profiling method, in some embodiments, total RNA from a sample from a subject suspected of having lung cancer is quantitatively reverse transcribed to provide a set of labeled oligonucleotides complementary to the RNA in the sample. The oligonucleotides are then hybridized to a microarray comprising target RNA-specific probes to provide a hybridization profile for the sample. The result is a hybridization profile for the sample representing the expression pattern of target RNAs in the sample. The hybridization profile comprises the signal from the binding of the oligonucleotides reverse transcribed from the sample to the target RNA-specific probes in the microarray. In some embodiments, the profile is recorded as the presence or absence of binding (signal vs. zero signal). In some embodiments, the profile recorded includes the intensity of the signal from each hybridization. The profile is compared to the hybridization profile generated from a normal, i.e., nonseptic sample, or in some embodiments, a control sample. An alteration in the signal is indicative of the presence of lung cancer in the subject.

### 4.3. Exemplary additional target RNAs

In some embodiments, in combination with detecting one or more target RNAs that are capable of specifically hybridizing to a nucleic acid comprising a sequence selected from SEQ ID NOs: 1 to 397, 1063 to 1210, 1363 to 1707, 2064 to 2183, 2312 to 2452, 2673 to 2680, and 2689 and/or detecting one or more target RNAs comprising at least 15 contiguous nucleotides of a sequence selected from SEQ ID NOs: 794 to 1043, 2576 to 2672, and 2692 and/or detecting one or more target RNAs that comprise a sequence that is complementary to at least 15 contiguous nucleotides of a sequence selected from SEQ ID NOs: 1 to 397, 1063 to 1210, 1363 to 1707, 2064 to 2183, 2312 to 2452, 2673 to 2680, and 2689, methods herein further comprise detecting the level(s) of expression of at least one other marker associated with lung cancer.

In some embodiments, the methods described herein further comprise detecting altered expression of lung cancer-associated small RNAs with non-canonical hairpins.

In alternative embodiments, the methods described herein further comprise detecting chromosomal codependents, i.e., target RNAs clustered near each other in the human genome which tend to be regulated together. Accordingly, in further embodiments, the methods comprise detecting the expression of one or more target microRNAs, each situated within the chromosome no more than 50,000 bp from the chromosomal location of the pre-microRNA sequences in Tables 3, 22, 25, 29, and 31.

In some embodiments, the methods comprise detecting the expression of one or more target RNAs clustered on chromosome 8 at 8p21.3-21.2, at 8p12, at 8p11.21, at 8q11.21, at 8q21.11, or at 8q24.22-24.3; on chromosome 9 at 9p21.3, at 9p13.3, at 9p11.2, at 9q22.32, at 9q31.3-34.11, or at 9q34.3; on chromosome 10 at 10q21.3-23.31, at 10q24.1, at 10q24.32, at 10q25.3, or at 10q26.3; on chromosome 11 at 11p15.5, at 11p14.1, at 11q12.1-23.2, at 11q13.4-14.1, at 11q23.2-24.1, or at 11q25; on chromosome 12 at 12p13.32-13.31, at 12p13.1, at 12p12.1, at 12q12-14.1, at 12q14.3-21.1, at 12q21.32-23.2, or at 12q24.23; on chromosome 13 at13q13.3, at 13q14.2, at 13q21.33, at 13q22.3, or at 13q31.3; at chromosome 14 at 14q11.1-13.2, at 14q24.3-31.1, or at 14q32.2-32.31; on chromosome 15 at 15q3, at15q23-24.32, or at 15q25.3-26.2; on chromosome 16 at 16p13.3-13.2, at 16p11.2, or at 16q12.1-22.3; on chromosome 17 at 17p13.3, at 17p13.1-11.2, at 17q12-21.1, at 17q22-23.3, or at 17q25.31; on chromosome 18 at 18q11.2, at 18q21.31-33, or at 18q22.3; on chromosome 19 at 19p13.3-13.2, at 19p13.12-12, at 19q12, or at 19q13.32-13.42; on chromosome 20 at 20p13, at 20p11.1, at 20q12, or at 20q13.32; on chromosome 21 at 21q21.1-22.11; on chromosome 22 at 22q11.21, or at 22q12.1-13.31; or on the X chromosome at Xp11.4-11.22, at Xq 13.1-13.3, at Xq22.3, at Xq25-27.1, or at Xq27.3-28.

### 4.4. Pharmaceutical compositions and methods of treatment

In some embodiments, the disclosure relates to methods of treating lung cancer in which expression of a target RNA is deregulated, e.g., either down-regulated or up-regulated in the lung cancer cells of an individual. When at least one target RNA is up-regulated in the cancer cells, the method comprises administering to the individual an effective amount of at least one compound that inhibits the expression of the at least one target RNA, such that proliferation of lung cancer cells is inhibited. Alternatively, in some embodiments, when at least one target RNA is up-regulated in the cancer cells, the method comprises administering to the individual an effective amount of at least one compound that inhibits the activity of the at least one target RNA, such that proliferation of lung cancer cells is inhibited. Such a compound may be, in some embodiments, a polynucleotide, including a polynucleotide comprising modified nucleotides.

When at least one target RNA is down-regulated in the lung cancer cells, the method comprises administering an effective amount of an isolated target RNA (*i.e.,* in some embodiments, a target RNA that is chemically synthesized, recombinantly expressed or purified from its natural environment), or an isolated variant or biologically-active fragment thereof, such that proliferation of cancer cells in the individual is inhibited.

The disclosure further provides pharmaceutical compositions for treating lung cancer. In some embodiments, the pharmaceutical compositions comprise at least one isolated target RNA, or an isolated variant or biologically-active fragment thereof, and a pharmaceutically-acceptable carrier. In some embodiments, the at least one isolated target RNA corresponds to a target RNA that exhibits a decreased level of expression in lung cancer cells relative to normal levels (in some exemplary embodiments, relative to the level of the target RNA in a control sample). In some embodiments, the isolated target RNA is a target RNA that is capable of selectively hybridizing to at least one nucleic acid probe comprising a sequence that is identically present in one of SEQ ID NOs: 1 to 397, 1363 to 1707, and 2312 to 2452. In some embodiments, the isolated target RNA that has a decreased level of expression in lung cancer relative to normal levels is capable of selectively hybridizing to at least one nucleic acid probe comprising a sequence that is identically present in one of SEQ ID NOs: 92 or 171, or a sequence that is identically present in the probe sequences of Table 34.

In some embodiments, the pharmaceutical compositions are useful for treating adenocarcinoma and comprise an isolated target RNA that is capable of selectively hybridizing to at least one nucleic acid probe comprising a sequence that is identically present in one of SEQ ID NOs: 4, 36, 50, 93, 122, 125, 139, 140, 144, 146, 159, 226, 239 or 241. In some embodiments, the pharmaceutical compositions are useful for treating adenocarcinoma and comprise an isolated target RNA that is capable of selectively hybridizing to at least one nucleic acid probe comprising a sequence that is identically present in one of SEQ ID NOs: 19, 27, 33, 48, 55, 72, 73, 94, 101, 105, 112, 117, 130, 131, 133, 134, 135, 143, 155, 158, 160, 161, 163, 165, 221, 238, 240 or 246.

In some embodiments the isolated target RNA is identical to an endogenous wild-type target RNA gene product (such as a product of a gene set forth in Table 3) that is down-regulated in the cancer cell. In some embodiments, the isolated target RNA is a variant target RNA or biologically active fragment thereof. As used herein, a "variant" refers to a target RNA gene product that has less than 100% sequence identity to the corresponding wild-type target RNA, but still possesses one or more biological activities of the wild-type target RNA (e.g., ability to inhibit expression of a target RNA molecule and cellular processes associated with lung cancer). A "biologically active fragment" of a target RNA is a fragment of the target RNA gene product that possesses one or more biological activities of the wild-type target RNA. In some embodiments, the isolated target RNA can be administered with one or more additional anti-cancer treatments including, but not limited to, chemotherapy, radiation therapy and combinations thereof. In some embodiments, the isolated target RNA is administered concurrently with additional anti-cancer treatments. In some embodiments, the isolated target RNA is administered sequentially to additional anti-cancer treatments.

In some embodiments, the pharmaceutical compositions comprise at least one compound that inhibits expression of the target RNA. In some embodiments, the compound is specific for one or more target RNAs, the expression of which is increased in lung cancer cells relative to normal levels (in some exemplary embodiments, relative to the level of the target RNA in a control sample). In some embodiments, the target RNA expression inhibitor is specific for at least one target RNA that is capable of selectively hybridizing to at least one nucleic acid probe comprising a sequence that is identically present in one of SEQ ID NOs: 1 to 397, 1063 to 1210, 2064 to 2183, 2673 to 2680, and 2689. In some embodiments, the target RNA expression inhibitor is specific for at least one target RNA that is capable of selectively hybridizing to at least one nucleic acid probe comprising a sequence that is identically present in one of SEQ ID NOs: 15, 26, 27 or 191. In some embodiments, the target RNA expression inhibitor is specific for at least one target RNA that is capable of selectively hybridizing to at least one nucleic acid probe comprising a sequence that is identically present in one of SEQ ID NOs: 15, 26, 27, 30, 129, 164, 184, 191, 196, 205, 207, 214, 219, 225, 246 or 248. In some embodiments, the target RNA expression inhibitor is specific for at least one target RNA that is capable of selectively hybridizing to at least one nucleic acid probe in one of Tables 6, 7, 8, 9, 32, and 33. In some embodiments, a target RNA expression inhibitor that is specific for at least one target RNA that is capable of selectively hybridizing to at least one nucleic acid probe in Table 6 is useful for treating non-small cell lung cancer. In some embodiments, a target RNA expression inhibitor that is specific for at least one target RNA that is capable of selectively hybridizing to at least one nucleic acid probe in Table 7 is useful for treating squamous cell carcinoma. In some embodiments, a target RNA expression inhibitor that is specific for at least one target RNA that is capable of selectively hybridizing to at least one nucleic acid probe in Table 8 is useful for treating adenocarcinoma. In some embodiments, a target RNA expression inhibitor that is specific for at least one target RNA that is capable of selectively hybridizing to at least one nucleic acid probe in Table 9 is useful for treating aggressive forms of lung cancer.

In some embodiments, the pharmaceutical compositions are useful for treating squamous cell carcinoma and comprise a target RNA expression inhibitor that is specific to a target RNA capable of selectively hybridizing to at least one nucleic acid probe comprising a sequence that is identically present in one of SEQ ID NOs: 4, 36, 50, 93, 122, 125, 139, 140, 144, 146, 159, 226, 239 and 241. In some embodiments, the pharmaceutical compositions are useful for treating squamous cell carcinoma and comprise a target RNA expression inhibitor that is specific to a target RNA capable of selectively hybridizing to at least one nucleic acid probe comprising a sequence that is identically present in one of SEQ ID NOs: 19, 27, 19, 27, 33, 48, 55, 72, 73, 94, 101, 105, 112, 117, 130, 131, 133, 134, 135, 143, 155, 158, 160, 161, 163, 165, 221, 238, 240 or 246. In some embodiments, the pharmaceutical compositions are useful for treating squamous cell carcinoma and comprise a target RNA expression inhibitor that is specific to a target RNA capable of selectively hybridizing to at least one nucleic acid probe in Table 7. In some embodiments, the pharmaceutical compositions are useful for treating adenocarcinoma and comprise a target RNA expression inhibitor that is specific to a target RNA capable of selectively hybridizing to at least one nucleic acid probe in Table 8. In some embodiments, the pharmaceutical compositions are useful for treating aggressive forms of lung cancer and comprise a target RNA expression inhibitor that is specific to a target RNA capable of selectively hybridizing to at least one nucleic acid probe in Table 9.

In some embodiments, the target RNA inhibitor is selected from double-stranded RNA, antisense nucleic acids and enzymatic RNA molecules. In some embodiments, the target RNA inhibitor is a small molecule inhibitor. In some embodiments, the target RNA inhibitor can be administered in combination with other anti-cancer treatments, including but not limited to, chemotherapy, radiation therapy and combinations thereof. In some embodiments, the target RNA inhibitor is administered concurrently with other anti-cancer treatments. In some embodiments, the target RNA inhibitor is administered sequentially to other anti-cancer treatments.

In some embodiments, a pharmaceutical composition is formulated and administered according to Semple et al., Nature Biotechnology advance online publication, 17 January 2010 (doi:10.1038/nbt.1602)), which is incorporated by reference herein in its entirety for any purpose.

The terms "treat," "treating" and "treatment" as used herein refer to ameliorating symptoms associated with lung cancer, including preventing or delaying the onset of symptoms and/or lessening the severity or frequency of symptoms of the lung cancer.

The term "effective amount" of a target RNA or an inhibitor of target RNA expression or activity is an amount sufficient to inhibit proliferation of cancer cells in an individual suffering from lung cancer. An effective amount of a compound for use in the pharmaceutical compositions disclosed herein is readily determined by a person skilled in the art, e.g., by taking into account factors such as the size and weight of the individual to be treated, the stage of the disease, the age, health and gender of the individual, the route of administration and whether administration is localized or systemic.

In addition to an isolated target RNA or a target RNA inhibitor, or a pharmaceutically acceptable salt thereof, the pharmaceutical compositions disclosed herein further comprise a pharmaceutically acceptable carrier, including but not limited to, water, buffered water, normal saline, 0.4% saline, 0.3% glycine, and hyaluronic acid. In some embodiments, the pharmaceutical compositions comprise an isolated target RNA or a target RNA expression inhibitor that is encapsulated, *e.g.,* in liposomes. In some embodiments, the pharmaceutical compositions comprise an isolated target RNA or a target RNA expression inhibitor that is resistant to nucleases, *e.g.,* by modification of the nucleic acid backbone as described above in Section 4.1.5. In some embodiments, the pharmaceutical compositions further comprise pharmaceutically acceptable excipients such as stabilizers, antioxidants, osmolality adjusting agents and buffers. In some embodiments, the pharmaceutical compositions further comprise at least one chemotherapeutic agent, including but not limited to, alkylating agents, anti-metabolites, epipodophyllotoxins, anthracyclines, vinca alkaloids, plant alkaloids and terpenoids, monoclonal antibodies, taxanes, topoisomerase inhibitors, platinum compounds, protein kinase inhibitors, and antisense nucleic acids.

Pharmaceutical compositions can take the form of solutions, suspensions, emulsions, tablets, pills, pellets, capsules, capsules containing liquids, powders, sustained-release formulations, suppositories, emulsions, aerosols, sprays, suspensions, or any other form suitable for use. Methods of administration include, but are not limited to, oral, parenteral, intravenous, oral, and by inhalation.

The following examples are for illustration purposes only, and are not meant to be limiting in any way.

### 5. EXAMPLES

### 5.1 Example 1: MicroRNAs from Primary Lung Tumors

Using microarray analysis, distinct microRNAs were demonstrated to be deregulated (e.g., either over-expressed or under-expressed) in primary lung tumors.

Total RNA was extracted from each of the primary tumors in Table 10 by preparation from frozen tissue samples as described below.

### RNA Preparation from frozen tissue samples

Archived or freshly snap-frozen tumor specimens were homogenized by mortar and pestle in TRIzol® Reagent, Invitrogen (Carlsbad, CA) and RNA was further extracted according to the manufacturer's protocol. All RNA samples were diluted in RNase-free water and stored at -80°C.

**Table 10**

| **Tumor designation** | **Sex** | **Age** | **Smoking Risk Factor** | **Cancer type** | **Stage** |
|---|---|---|---|---|---|
| Adk-1 | M | 59 | Smoker | Non Squamous, adenocarcinoma | YT2N2 |
| Adk-2 | M | 67 | Smoker | Non Squamous, adenocarcinoma | PT2N0 |
| Adk-3 | M | 78 | na | Non Squamous, adenocarcinoma | PT1N0 |
| Adk-8 | F | 51 | na | Non Squamous, adenocarcinoma | PT3N0 |
| Adk-9 | M | 55 | Smoker | Non Squamous, adenocarcinoma | PT1N0 |
| Adk-10 | M | 49 | Smoker | Non Squamous, adenocarcinoma | PT3N0 |
| Adk-11 | M | 73 | na | Non Squamous, adenocarcinoma | PT1N0 |

**Table 10**

| **Tumor designation** | **Sex** | **Age** | **Smoking Risk Factor** | **Cancer type** | **Stage** |
|---|---|---|---|---|---|
| Epi-4 | M | 78 | Smoker | Squamous, Epidermoid carcinoma | PT2NX |
| Epi-5 | M | 78 | Smoker | Squamous, Epidermoid carcinoma | PT2NX |
| Epi-7 | F | 84 | Non-smoker | Squamous, Epidermoid carcinoma | PT2NX |
| Normal-lung_1 | M | 81 | na | Normal tissue from patient | PT2N0 |
| Normal-lung_2 | na | na | na | Normal tissue from Ambion | na |

Epi4, Epi7, Epi5, Adk1, Adk3, Adk11, Adk8, Adk9 and Adk2 identify primary tumors resected from 10 patients. Epi (epidermoid) is squamous cell carcinoma, while Adk (adenocarcinoma) is non-squamous cell carcinoma.

In Table 10, above, "na" indicates that the information was not available. "Y" in the staging column indicates that the patient was treated before surgical resection. "P" indicates that the patient was not treated before surgical resection.

Staging is according to the "TNM" staging system for NSCLC. "T" categories for NSCLC indicate the following: (a) T1 indicates that the tumor is no larger than 3 centimeters across, has not reached the membranes that surround the lungs, and does not affect the main branches of the bronchi; (b) T2 tumors possess one or more of the following features: (i) larger than 3 centimeters across; (ii) involves a main bronchus, but is not closer than 2 cm to the carina (the point where the windpipe splits into the left and right main bronchi); (iii) has grown into the membranes that surround the lungs; or (iv) partially clogs the airways, but has not caused the entire lung to collapse or develop pneumonia; (c) T3 indicates a tumor of any size having one or more of the following features: (i) tumor has grown into the chest wall, the diaphragm, the membranes surrounding the space between the two lungs, or membranes of the sac surrounding the heart; (ii) tumor invades a main bronchus and is closer than 2 cm to the carina, but it does not involve the carina itself; or (iii) tumor has grown into the airways enough to cause an entire lung to collapse or to cause pneumonia in the entire lung. The "N" number indicates involvement of the lymph nodes as follows: (a) N0 indicates that the cancer has not spread to nearby lymph nodes; (b) N2 indicates spread to lymph nodes around the carina or in the space behind the breastbone and in front of the mediastinum. Affected lymph nodes are on the same side as the primary tumor; and (c) NX indicates that nearby lymph nodes could not be accessed.

Total RNA from normal lung tissue resected from a patient (in this case from a location in the lung as distal as possible from the tumor) and normal lung tissue from a location adjacent to a lung tumor (Ambion) were used as the control.

### Total RNA preparation and analysis

Total RNA was isolated by using standard TRIzol^{®} protocol (Invitrogen). Cells from two confluent 75cm² flasks were harvested (= approx 10⁷ cells). Total RNA was prepared using TRIzol^{®} Reagent, Invitrogen (Carlsbad, CA) according to the manufacturer's protocol. All RNA samples were diluted in RNase-free water and stored in -80°C (-112°F).

RNA quality was assessed by calculating OD 260/280 ratios. The quality of all RNA samples was high as assessed using an Agilent Bioanalyser 2100, as exemplified by the electropherogram shown in FIG. 1 obtained for total RNA from A549 human adenocarcinoma cell line. Similar electropherograms were obtained for total RNA from the other primary tumor samples as well.

### MicroRNA Purification

MicroRNA purification was performed using a Flash PAGE Fractionator (Ambion). The Ambion gel purification protocol enriches for small RNAs less than 40 nucleotides (nt) long, including microRNAs. Briefly, a total RNA sample was loaded onto a pre-cast gel using the Flash PAGE Fractionator. The total RNA fraction smaller than 40 nt (the "microRNA fraction") was recovered after gel migration and resuspended into nuclease free water.

### Microarray Analysis

### Probe design and spotting

The oligonucleotide probes used for microarray preparation had the configuration 5'-NH₂-(C)₆-(spacer)-(oligomer probe sequence)-3'. The 5'-amino group allowed chemical bonding onto the array support. Each also included an identical spacer sequence of 15 nt, as shown below, to prevent non-specific interactions of the oligonucleotide probes with the array support:
5'AminoC6-TTGTAATACGACTCA - Oligo probe sequence (SEQ ID NO: 1044)

### Probe sequences given in Table 1 and Table 2 omit the linker.

The probes were synthesized according to standard protocols by Eurofins MWG Operon (Ebersberg, Germany). Nexterion (Schott) microarray glass slides were used as the solid support for the microarray.

The oligonucleotide probe concentration used for the spotting was 25 µmol. The probes were spotted in duplicate using the Nexterion spotting buffer provided with the array glass support by Schott with 1 % SDS (sodium dodecyl sulfate) added to allow larger spot sizes *(e.g.,* 100-150 microns compared to 70-100 microns without SDS). The spotter used was the QArray mini (Genetix) equipped with Stealth SMP3 pins (Telechem). After deposition of one series of spots, the spotting needle was washed 5 times with 60mM NaOH before spotting the next series of probes. Each slide is designed with 32 blocks of spotted probes, with each block being a 20x20 square of spotted probes. Each probe was spotted in duplicate. Spotted glass slides were stored at 4°C until use.

### MicroRNA labelling

The labelling of the microRNA fraction was adapted from a published protocol developed at EMBL (Heidelburg, Germany) by the European Molecular Biology Group (Castoldi et al., "A sensitive array for microRNA expression profiling (miChip) based on locked nucleic acids (LNA)," RNA 2006 May;12(5):913-20. Epub 2006 Mar 15, incorporated herein by reference in its entirety). Briefly, the microRNA fraction was incubated for 6 hours at 4°C with a mixture containing 10 µM of dye-labelled tetra-nucleotide (5'-rUrUrUrU -Cy5- 3') (or alternatively, 5'-rUrUrUrU - Cy3-3') (Biospring, Germany) in Ambion buffer diluted to 1X with RNase free water, 8% polyethylene glycol (PEG), 2 mM adenosine triphosphate (ATP), and T4 RNA ligase (0.7U/µl). The labelling reaction was run by heating the mixture for 15 minutes at 65°C. This procedure ligated the poly-U dye-labelled tail to the 3'end of all the microRNAs. Labelled samples were stored at 4°C before hybridization.

### Array Hybridization

The labelled microRNA fraction was hybridized to the spotted arrays using a Discovery hybridization station (Ventana, Tucson, AZ). Briefly, 2 mL of a mixture of 1% BSA, 2X SSC, and 0.2 % SDS was incubated with the chips for 30 min at 42°C. Then the chips were washed once using EZ Prep buffer (Ventana) and then three more times with Ribowash (Ventana). Next, 20 µl of the labelled microRNA mixture and 180µl of ChipHybe Reagent (Ventana) were added to the array. The arrays were heated for 6 minutes at 37°C, then were incubated at 42°C for 8 hours, after which the heating was stopped. The chips were washed once with Ribowash (Ventana) and then heated for 2 minutes at 37°C. The chips were washed again with Ribowash (Ventana) with one drop of Cheap Clean (Ventana) added, and incubated for 2 minutes at 37°C. The chips were washed two more times using Ribowash (Ventana). The chips were stored dry overnight. On the following day, the final washes were done according to Ventana's instructions for the Discovery hybridization station. The slides were washed twice with 2X SSC + 0.2X SDS buffer and then one more time with 0.1X SSC. All the slides were dried using a speed centrifuge from Arrayit (TeleChem International, Sunnyvale, CA) at room temperature and kept in the dark before scanning.

As an alternative to the ChipHybe Reagent solution (solution 1), the following solution may be used for array hybridization (solution 2) to form probe:target RNA hybrids by mixing 2 parts of the 1.5X TMAC Hybridization Solution to 1 part (v:v) sample, so that the final component concentrations are 3M TMAC, 0.10% Sarkosyl, 50 mM Tris, and 4 mM EDTA, and incubating on the array at 42°C for 8 hours:

### 1.5X TMAC Hybridization Solution

| **Reagent** | **Catalog Number** | **Final Conc** | **Amount/ 250 mL** |
|---|---|---|---|
| 5 M TMAC* | Sigma T3411 | 4.5 M | 225 mL |
| 20% Sarkosyl | ------ | 0.15% | 1.88 mL |
| 1 M Tris-HCl, pH 8.0 | Sigma T3038 | 75mM | 18.75 mL |
| 0.5 M EDTA, pH 8.0 | Invitrogen 15575-020 | 6mM | 3.0 mL |
| H₂O | ------ | ------ | 1.37 mL |

| | | | |
|---|---|---|---|
| *TMAC is tetramethyl ammonium chloride | | | |

The number of technical replicates hybridized for each biological sample is shown below in Table 11.

**Table 11**

| | **Normal** | | **Squamous cell carcinoma** | | | **Non-squamous carcinoma** | | | | | | |
|---|---|---|---|---|---|---|---|---|---|---|---|---|
| Samples | Lung1 | Lung2 | Epi4 | Epi5 | Epi7 | Adk1 | Adk2 | Adk3 | Adk8 | Adk9 | Adk10 | Adk11 |
| G2 | 3 | 4 | 4 | 1 | 2 | 3 | | 2 | 3 | | 5 | 2 |
| G3 | | | 2 | | | | 2 | | | 1 | 2 | |

### Array Image Acquisition

The arrays were scanned using an Axon™ scanner (Molecular Devices, Sunnyvale, CA) and their Genepix™ software. The image was formatted in tif format, defined by an image color depth of 16 bits/pixel (1600*1600). At such setting, pixels can assume intensity values ranging from 0 to 65,535. Pixels exhibiting the maximum intensity value are "saturated" and were assigned the value of 65,535. The resolution of the array scan was set at 10 µm/pixel. For hybridization experiments using different fluorescent dyes (e.g., Cy5 and Cy3) the photomultiplier tube (PMT) was adjusted to the higher intensity spot (Cy3 is scanned at lower PMT settings than Cy5).

### Array Image Analysis

The PMT of the laser scanner digitized the captured fluorescence intensity for each given "point" of a slide and stored the numerical value as a pixel corresponding to that point. A picture composed of such pixels was then analyzed.

The first task for image analysis was to detect the spot position, using a process called segmentation. Spots were segmented by circles of adaptable or fixed radius. To be reliably segmented and quantified, the spot diameter was required to be more than 5 - 6 pixels. Before segmentation an indexing grid was provided giving the approximate positions of the spots. The segmentation itself detected the limits of spots near the grid circles. Briefly, the Genepix software assigns a circle to each spot on the array (segmentation). The segmentation had to be conducted in a somewhat flexible way due to spotting imperfections and/or support deformation, as the spots were almost never on a perfectly rectangular grid.

After segmentation by the software, the circles were modified manually and adjusted onto the spots until all the spots on the array were clearly identified. At this stage, if the array presented high background noise preventing real spots from being distinguished from the background, the array was rejected for further analysis.

The second task of image analysis was to quantify spots and export the data into a result file. This was a relatively easy and well-defined task once the spots were located on the image. The statistical approach used most frequently to quantify spot intensity was the mean or median of pixels belonging to a spot. The median approach was more robust than the mean value in the presence of outlier pixels. In practice, however, there was little difference in the results obtained using mean or median.

### Array Data Analysis

All the array data were analysed using the R bioconductor package ("Bioconductor: open software development for computational biology and bioinformatics," Genome Biol. 2004;5(10):R80. Epub 2004 Sep 15, which is incorporated herein by reference in its entirety).

Array data were first tested for quality by comparing the spot intensities for the internal controls. (Table 12) One internal control (SEQ ID NO: 1046) was used as a labelling control (this synthetic RNA is added to the purified microRNA fraction before labelling), and 7 other internal controls (SEQ ID NOs: 1047 to 1052 and 1045) were used for the normalization of the data (these synthetic RNA controls are added to the total RNA fraction before hybridization at 520 fmol each/array).

**Table 12**

| **Internal controls added to total RNA or microRNA fraction** | |
|---|---|
| CGCGCGUCGCUUUAUCUACUGU | SEQ ID NO: 1046; CTL30_COMP |
| UUAUCGUUCGAUAAGUCGCGUU | SEQ ID NO: 1047; CTL11_COMP |
| GAAGUUACUAUGUAGGCAACCU | SEQ ID NO: 1048; CTL23_COMP |
| CGCGGGACUAAUUGUUACCGGG | SEQ ID NO: 1049; CTL26_COMP |
| UCGCGUCGAACUCCGCAACCGA | SEQ ID NO: 1050; CTL29_COMP |
| ACCGAACGCCGUACCCAUCGGG | SEQ ID NO: 1051; CTL31_COMP |
| CGAGGGUAACGACUCUCGUGUC | SEQ ID NO: 1052; CTL36_COMP |
| GCGUACCGACGCGUAGACGGAC | SEQ ID NO: 1045; CTL13_COMP |

**Table 13**

| **Probes for hybridization of control sequences in microarray experiments** | |
|---|---|
| **Sequence (5'-3')** | **Sequence identification number** |
| TTGTAATACGACTCAACAGTAGATAAAGCGACGCGCG | SEQ ID NO: 1054; CTL30 |
| TTGTAATACGACTCAAACGCGACTTATCGAACGATAA | SEQ ID NO: 1055; CTL11 |
| TTGTAATACGACTCAAGGTTGCCTACATAGTAACTTC | SEQ ID NO: 1056; CTL23 |
| TTGTAATACGACTCACCCGGTAACAATTAGTCCCGCG | SEQ ID NO: 1057; CTL26 |
| TTGTAATACGACTCATCGGTTGCGGAGTTCGACGCGA | SEQ ID NO: 1058; CTL29 |
| TTGTAATACGACTCACCCGATGGGTACGGCGTTCGGT | SEQ ID NO: 1059; CTL31 |
| TTGTAATACGACTCAGACACGAGAGTCGTTACCCTCG | SEQ ID NO: 1060; CTL36 |
| TTGTAATACGACTCACCCGGTAACAATTAGACCCGCG | SEQ ID NO: 1061; CTL26_MUT |
| TTGTAATACGACTCAGTCCGTCTACGCGTCGGTACGC | SEQ ID NO: 1062; CTL13 |
| TTGTAATACGACTCAGGCCGTCTACGCGTCGGTACGC | SEQ ID NO: 1053; CTL13_MUT |

All sequences for which the intensity of the spot was higher than the mean local background intensity plus 1.5 times its standard deviation were categorized as expressed microRNAs. The following criteria were required to be met in order consider the array intensity data valid for further analysis:
1. Specificity of the hybridization controls had to be within acceptance criteria (e.g. CTL26 vs. its corresponding single base mutant, CTL26_MUT, or CTL13 vs. its corresponding single base mutant, CTL13_MUT).
2. Approximate equality of the signal intensity of the replicates of the positive controls
3. Approximate equality between median block signal intensities based on the positive controls for each block
4. Approximate equality between median array signals based on all sequences detected
5. Signal intensity for the purification and labelling control (CTL30).

Statistical normalization of the data was done by computing the Log2ratio where the Log2ratio equals average intensity signal of the duplicated spots/median intensity of all positives controls for the block. The normalization was done per block to avoid non-homogenous labelling of all blocks of the array. This block-by-block normalization has been shown to be more efficient then using overall normalization of the slide. The obtained values are Log2 values.

The intensities of the spots for each oligonucleotide probe were compared in the sample from the primary lung tumors versus normal lung tissue, resulting in an evaluation of the relative expression for each microRNA.

The expression fold-change corresponds to 2^{(Log2ratio)}. The Log2ratio is the ratio between the two conditions compared, or log2(Xcell-line/Xnormal), which is the same as (log2Xcell-line - log2Xnormal), where X is the measured intensity value. In cases where there was no signal from the "normal" condition, the lowest measured intensity value in the experiment was used as the baseline from which a fold-change expression value was calculated. A fold-change value of less than zero corresponds to a down-regulation of (1/fold-change) times.

Data are tabulated in Table 1. The numerical values set forth in Table 1 indicate the fold-change calculated by comparing the signal intensity measured for hybridization to a particular probe in each tumor cell sample with the signal intensity measured for hybridization to the same probe in the control sample (NHBE cells). When the signal detected for hybridization to a particular probe in both the control sample and the tumor cell sample was below the threshold value, the indication "nd" is given in the Table and no fold-change was calculated. Likewise, when hybridization to a particular array probe was detected in the control sample but not in a tumor cell sample, the fold-change calculation is preceded by "nd" in the Table. When no signal was detected in the control sample, the threshold value was retained for the fold-change calculation. This results in an underestimation of the fold-change in the tumor cell sample as compared to the control sample, which is indicated respectively by a ">" or "<" sign in Table 1 for up-regulation and down-regulation of a target RNA.

### 5.2 Example 2: Analysis of target RNA from Lung Cancer Cell Lines

### Cell lines

Total RNA was prepared from three different lung cell lines that are commonly used in studies of lung cancer. The RNA was used for target RNA array profiling.

As set forth in Table 14 below, cell lines were selected for diversity, deriving from adenocarcinomas, squamous cell carcinomas and large cell carcinomas. All cell lines were purchased from LGC Promochem (ATCC) and cultured according to ATCC's guidelines.

**Table 14**

| **Cell Line** | **ATCC Number** | **Cancer type** |
|---|---|---|
| NHBE | CC-2540 | Normal bronchial epithelial cells |
| BEA2B | CRL-9609 | Immortalized bronchial epithelial cells - normal phenotype |
| A549 | CCL-185 | Adenocarcinoma |
| H1703 | CRL-5889 | Adenocarcinoma |
| H460 | HTB-177 | Carcinoma (big neuroendocrine cells) |

Microarray data acquisition and analysis were conducted as described in Example 1.

Total RNA from normal bronchial epithelial cells (NHBE, ATCC no. CC-2540) was used as a control.

Data are tabulated in Table 2. The tabulated values correspond to the fold-changes calculated by comparing the signal intensity measured for hybridization to a particular array probe in a primary tumor sample with the mean signal intensity for hybridization to the same probe in the control samples. When the signal was below the threshold value for both the control sample and the primary tumor sample, no fold-change was calculated (indicated by "nd" in Table 2). When hybridization to a particular probe was detected in the control sample, but not in a primary tumor sample, the fold-change calculation is preceded by "nd". When no signal was detected for hybridization to an array probe in the control samples, the threshold value was retained for the fold-change calculation, which resulted in an underestimation of the fold-change in the primary tumor sample as compared to the control sample. This is indicated in Table 2 respectively by a ">" or "<" sign for up-regulated and down-regulated target RNAs.

### 5.3 Example 3: Analysis of target RNA on Luminex Platform

The Luminex technology (Luminex Corp., Austin, TX) is based on liquid phase hybridization to probe-labelled beads, followed by flow cytometry detection of beads with differing ratios of fluorescent dyes. Beads with up to 100 different dye ratios are available, making it possible to interrogate a single sample for up to 100 analytes simultaneously.

### Coupling of Probes to Luminex Beads

Aliquots of each 5'-amino-modified probe having sequences as set forth in Example 1 and Table 1 are prepared at a concentration of 0.1nmol/µL in molecular biology grade water. The probes are coupled to the beads using carbodiimide chemistry according to the manufacturer's protocol (Luminex bead coupling protocol). The probe-coupled beads are stored at 4°C.

### Total RNA Preparation for Luminex Analysis

Fifty fmoles of each of 7 internal controls (the same synthetic RNAs used for the array controls) are added to the total RNA fraction isolated from the biological samples. Prior to hybridization with Luminex beads, the total RNA preparation is treated to avoid the formation of dendrimers, which result from the circularization of a single RNA molecule, or concatenation to another RNA molecule. To avoid the formation of dendrimers, the RNA is pre-treated with calf intestinal phosphatase (CIP) to remove the 5'-phosphate groups. The CIP reagent can be obtained from Invitrogen (Carlsbad, CA) and the CIP reaction is run according to the manufacturer's protocol.

### Bead Labelling and Hybridization

After CIP treatment, the total RNA fraction is then labelled with biotin using the Vantage microRNA Labelling Kit (Marligen). The labelled fraction is hybridized to the Luminex beads using the Marligen protocol. Briefly, the oligonucleotide beads are mixed with the Marligen hybridization solution (1.5 X TMAC) and the labelled total RNA. The hybridization is performed at 60°C for an hour in the dark. After hybridization, the beads are washed using the Luminex standard 6X SSPET wash buffer (sodium phosphate, sodium chloride, EDTA, Triton X-100, pH 7.4).

### Detection of Bead Hybridization

The detection of the Luminex beads is done using streptavidin phycoerythrin (SAPE) (Europa Bioproducts, Cambridge, UK). The SAPE is added to the washed beads according to the Luminex protocol. The beads are then read using the Luminex IS-200 instrument using the high gain setting for better resolution

### Data Acquisition and Analysis

The Luminex IS-200 reads at least 25 beads of each dye-ratio in the reaction mix. Each dye-ratio bead corresponds to a particular probe sequence, and the intensity value is returned as an average value of all read beads. The mean fluorescence intensity (MFI) data is normalized using synthetic RNA controls, and fold changes between normal and diseased samples are computed using the Bioplex software (Bio-Rad, Hercules, CA) and the R bioconductor package (Bioconductor: open software development for computational biology and bioinformatics, Genome Biol. 2004;5(10):R80. Epub 2004 Sep 15).

### 5.4 Example 4: Analysis of target RNA from Additional Primary Lung Tumors

Three of the primary lung tumors analyzed in Example 1 were included in this analysis (Adk-9, Adk-10, and Epi-4). The microRNA fractions isolated from those tumors in Example 1 were used in the microarray experiment described below.

### Patient cohort

In addition to the three tumors listed above (and included in Table 15 in the first three lines), fifteen patients diagnosed with lung cancer were included in the cohort, nine men and six women. Normal tissue from an additional patient (Ksarc-17) was also included in the study. Because squamous cell lung cancer and non-squamous cell lung cancer are two of the most frequent lung cancer types (more than 70% of all lung cancers), four patients diagnosed with squamous cell carcinoma (Kmalp-21, Kmalp-25, Epi-42, and Kmalp-44) and seven patients diagnosed with non-squamous cell carcinoma (Adk-40, Adk-41, Adk-48, Adk-49, Adk-15, Adk-23, and Adk-29) were included, in addition to the previous squamous cell carcinoma (Epi-4) and non-squamous cell carcinoma (Adk-9, and Adk-10) patients. In addition, one patient diagnosed with a carcinoid (Car-13), one diagnosed with a carcinoma sarcomatoid (Ksarc-19), one diagnosed with a small cell lung cancer (Scc-27), and one diagnosed with a large cell neuroendocrine cancer (Lcnec-31) were also selected. Table 15 shows a list of the patients and various clinical characteristics of each patient.

**Table 15. Clinical characteristics of patients in cohort.**

| **Patient ID** | **histology** | **Tissue collected** | **gender** | **Birth year or Age** | **Smoking history** | **TNM staging** | **Stage group** |
|---|---|---|---|---|---|---|---|
| Adk-9 | adenocarcinoma | PT | M | 1953 | 40 smokes / day | T1N0M0 | IA |
| Adk-10 | adenocarcinoma | PT | M | 1959 | 25 smokes / day | T3N0M0 | IIB |
| Epi-4 | sqamous | PT | M | 1930 | 30 smokes / day | T2NxM0 | IIIA |
| Adk-29 | adenocarcinome | PT & DNT | M | 1932 | ? smokes / day | T4N2M1 | IV |
| Adk-15 | adenocarcinoma | PT & DNT | M | 1947 | ? smokes / day | T2N0M0 | IB |
| Adk-23 | adenocarcinome | PT & DNT | F | 1971 | 0 | T3N0M0 | IIB |
| Ksarc-19 | carcinome sarcomatoide | PT & DNT | M | 1949 | 0 | T4N1M0 | IIIB |
| Kmalp-25 | Epidermoide | PT & DNT | M | 1928 | 0 | T2N0M0 | IB |
| Kmalp-21 | Epidermoide | PT & DNT | F | 1942 | 0 | T3N1M0 | IIIA |
| Car-13 | Carcinoide | PT & DNT | F | 1952 | 0 | T3N1M0 | IIIA |
| Lcnec-31 | neuroendocrine GC | PT & DNT | M | 1946 | 0 | T3N2M0 | IIIA |
| Scc-27 | petites cellules | PT & DNT | F | 1943 | 0 | T2N0M0 | IB |
| Adk-40 | Non Squamous, adenocarcinoma | PT | M | 76 | NA | T1aN0 | IA |
| Adk-41 | Non Squamous, adenocarcinoma | PT | F | 61 | Smoker | T2N0M0 | IB |
| Adk-48 | Non Squamous, adenocarcinoma | PT | F | 56 | NA | T1bN0M0 | IA |
| Adk-49 | Non Squamous, adenocarcinoma | PT | M | 53 | NA | T3N2 | IIIA |
| Epi-42 | Squamous, Epidermoid carcinoma | PT | M | 59 | NA | T3N1M0 | IIIA |
| Kmalp-44 | Squamous, Epidermoid carcinoma | PT | M | na | NA | T2bN0 | IB |
| Ksarc-17 | Carcinome Sarcomatoide | DNT | M | 53 | NA | T2N0M0 | IB |

| | | | | | | | |
|---|---|---|---|---|---|---|---|
| PT = Primary Tumor DNT = Distal Normal Tissue NA= information not available | | | | | | | |

Five of the patients had very aggressive forms of lung cancer, Ksarc-19 (carcinoma sarcomatoide), Adk-9, Adk-10, Adk-29 (all adenocarcinoma), and Lcnec-31. Patient Ksarc-19 relapsed one month after surgery and died six months later. Patient Car-13 (carcinoide) had a slow growing form of lung cancer.

The primary tumors only were collected from six of the patients (as well as the four previous patients, for a total of 10 patients), while the primary tumor and distal normal tissue were collected from nine of the patients. Distal normal tissue only was used from one of the patients (Ksarc-17). The relative amount of tumor cells versus normal cells in primary tumors was determined to be between 90% and 100% for all patients. The distal normal tissue was collected from a location as far as possible from the primary tumor, and did not contain detectable tumor cells.

### Tissue samples

Archived or freshly snap-frozen specimens from lung primary tumors were used. Tissue samples were homogenized by mortar and pestle in TRIzol® Reagent (Invitrogen; Carlsbad, CA) and RNA was extracted according to manufacturer's protocol. RNA samples were diluted in RNase-free water and stored in -80°C (-112°F).

### MicroRNA Preparation:

All samples were enriched for the microRNA fraction using a Flash PAGE Fractionator (Ambion). Briefly, a total RNA sample was loaded onto a pre-cast gel using the Flash PAGE Fractionator. The total RNA fraction smaller than 40 nt (the "microRNA fraction") was recovered after gel migration and resuspended into nuclease free water.

### Microarray Analysis

### Probe design and spotting

The polynucleotide probes used for microarray preparation had the configuration 5'-NH₂-(C)₆-(spacer)-(oligomer probe sequence)-3'. The 5'-amino group allowed chemical bonding onto the array support. Each also included an identical spacer sequence of 15 nt, as shown below, to prevent non-specific interactions of the polynucleotide probes with the array support:
5'AminoC6-TTGTAATACGACTCA - Oligo probe sequence. (SEQ ID NO: 1044)

### Probe sequences given in the Tables herein omit the linker.

The probes were synthesized according to standard protocols by Eurofins MWG Operon (Ebersberg, Germany). Nexterion (Schott) microarray glass slides were used as the solid support for the microarray.

The polynucleotide probe concentration used for the spotting was 25 µmol. The probes were spotted in duplicate using the Nexterion spotting buffer provided with the array glass support by Schott with 1 % SDS (sodium dodecyl sulfate) added to allow larger spot sizes (e.g., 100-150 microns compared to 70-100 microns without SDS). The spotter used was the QArray mini (Genetix) equipped with Stealth SMP3 pins (Telechem). After deposition of one series of spots, the spotting needle was washed 5 times with 60mM NaOH before spotting the next series of probes. Each slide is designed with 48 blocks of spotted probes, with each block being a 20x18 square of spotted probes. Each probe was spotted in duplicate. Spotted glass slides were stored at 4°C until use.

### MicroRNA labelling

The labelling of the microRNA fraction was adapted from a published protocol developed at EMBL (Heidelburg, Germany) by the European Molecular Biology Group (Castoldi et al., "A sensitive array for microRNA expression profiling (miChip) based on locked nucleic acids (LNA)," RNA 2006 May;12(5):913-20. Epub 2006 Mar 15, incorporated herein by reference in its entirety). Briefly, the microRNA fraction was incubated for 6 hours at 4°C with a mixture containing 10 µM of dye-labelled tetra-nucleotide (5'-rUrUrUrU -Cy5- 3') (or alternatively, 5'-rUrUrUrU - Cy3-3') (Biospring, Germany) in Ambion buffer diluted to 1X with RNase free water, 8% polyethylene glycol (PEG), 2 mM adenosine triphosphate (ATP), and T4 RNA ligase (0.7U/µl). The labelling reaction was run by heating the mixture for 15 minutes at 65°C. This procedure ligated the poly-U dye-labelled tail to the 3'end of all the microRNAs. Labelled samples were stored at 4°C before hybridization.

### Array Hybridization

Table 16 shows the number of array replicates carried out for each tumor sample.

**Table 16: Number of Replicates for Sample**

| **Sample** | **Tumor type** | **Number replicates** |
|---|---|---|
| Adk-9 | Non-squamous | 1 |
| Adk-10 | Non-squamous | 2 |
| Epi-4 | Squamous | 2 |
| Adk-29 | Non-squamous | 3 |
| Adk-15 | Non-squamous | 3 |
| Adk-23 | Non-squamous | 3 |
| Ksarc-19 | Squamous | 2 |
| Kmalp-25 | Squamous | 1 |
| Kmalp-21 | Squamous | 3 |
| Car-13 | Carcinoid | 3 |
| Lcnec-31 | Large cell neuroendocrine | 2 |
| Scc-27 | Small cell | 2 |
| Adk-40 | Non-squamous | 2 |
| Adk-41 | Non-squamous | 3 |
| Adk-48 | Non-squamous | 2 |
| Adk-49 | Non-squamous | 3 |
| Epi-42 | Squamous | 3 |
| Kmalp-44 | Squamous | 3 |
| Ksarc-17 | Normal | 3 |
| Adk-29 | Normal | 1 |
| Adk-15 | Normal | 2 |
| Adk-23 | Normal | 3 |
| Ksarc-19 | Normal | 1 |
| Kmalp-25 | Normal | 1 |
| Kmalp-21 | Normal | 1 |
| Lcnec31 | Normal | 1 |
| Scc27 | Normal | 1 |

The labelled microRNA fraction was hybridized to the spotted arrays using a Discovery hybridization station (Ventana, Tucson, AZ). Briefly, 2 mL of a mixture of 1% BSA, 2X SSC, and 0.2 % SDS was incubated with the chips for 30 min at 42°C. Then the chips were washed once using EZ Prep buffer (Ventana) and then three more times with Ribowash (Ventana). Next, 20 µl of the labelled microRNA mixture and 180µl of ChipHybe Reagent (Ventana) were added to the array. The arrays were heated for 6 minutes at 37°C, then were incubated at 42°C for 8 hours, after which the heating was stopped. The chips were washed once with Ribowash (Ventana) and then heated for 2 minutes at 37°C. The chips were washed again with Ribowash (Ventana) with one drop of CheapClean (Ventana) added, and incubated for 2 minutes at 37°C. The chips were washed two more times using Ribowash (Ventana). The chips were stored dry overnight. On the following day, the final washes were done according to Ventana's instructions for the Discovery hybridization station. The slides were washed twice with 2X SSC + 0.2X SDS buffer and then one more time with 0.1X SSC. All the slides were dried using a speed centrifuge from Arrayit (TeleChem International, Sunnyvale, CA) at room temperature and kept in the dark before scanning.

### Array Image Acquisition

The arrays were scanned using an Axon™ scanner (Molecular Devices, Sunnyvale, CA) and their Genepix™ software. The image was formatted in tif format, defined by an image color depth of 16 bits/pixel (1600*1600). At such setting, pixels can assume intensity values ranging from 0 to 65,535. Pixels exhibiting the maximum intensity value are "saturated" and were assigned the value of 65,535. The resolution of the array scan was set at 10 µm/pixel. For hybridization experiments using different fluorescent dyes (e.g., Cy5 and Cy3) the photomultiplier tube (PMT) was adjusted to the higher intensity spot (Cy3 is scanned at lower PMT settings than Cy5).

### Array Image Analysis

The PMT of the laser scanner digitized the captured fluorescence intensity for each given "point" of a slide and stored the numerical value as a pixel corresponding to that point. A picture composed of such pixels was then analyzed. The first task for image analysis was to detect the spot position, using a process called segmentation. Spots were segmented by circles of adaptable or fixed radius. To be reliably segmented and quantified, the spot diameter was required to be more than 5 - 6 pixels. Before segmentation an indexing grid was provided giving the approximate positions of the spots. The segmentation itself detected the limits of spots near the grid circles. Briefly, the Genepix software assigns a circle to each spot on the array (segmentation). The segmentation had to be conducted in a somewhat flexible way due to spotting imperfections and/or support deformation, as the spots were almost never on a perfectly rectangular grid.

After segmentation by the software, the circles were modified manually and adjusted onto the spots until all the spots on the array were clearly identified. At this stage, if the array presented high background noise preventing real spots from being distinguished from the background, the array was rejected for further analysis.

The second task of image analysis was to quantify spots and export the data into a result file. This was a relatively easy and well-defined task once the spots were located on the image. The statistical approach used most frequently to quantify spot intensity was the mean or median of pixels belonging to a spot. The median approach was more robust than the mean value in the presence of outlier pixels. In practice, however, there was little difference in the results obtained using mean or median.

### Array Data Analysis

All the array data were analysed using the R bioconductor package ("Bioconductor: open software development for computational biology and bioinformatics," Genome Biol. 2004;5(10):R80. Epub 2004 Sep 15, which is incorporated herein by reference in its entirety).

Array data were first tested for quality by comparing the spot intensities for the internal controls. One internal control (SEQ ID NO: 1046; Table 17) was used as a labelling control (this synthetic RNA is added to the purified microRNA fraction before labelling), and 6 other internal controls (SEQ ID NOs: 1047 to 1052) were used for the normalization of the data (these synthetic RNA controls are added to the total RNA fraction before hybridization at 520 fmol each/array). The probe sequences that bind to the synthetic RNAs, and a mutant probe sequence, are also shown in Table 17 (SEQ ID NOs: 1054 to 1061).

**Table 17**

| **Control Sequences used in microarray experiments** | |
|---|---|
| **Sequence (5'-3')** | **Sequence identification number** |
| CGCGCGUCGCUUUAUCUACUGU | SEQ ID NO: 1046; CTL30_COMP |
| UUAUCGUUCGAUAAGUCGCGUU | SEQ ID NO: 1047; CTL11_COMP |
| GAAGUUACUAUGUAGGCAACCU | SEQ ID NO: 1048; CTL23_COMP |
| CGCGGGACUAAUUGUUACCGGG | SEQ ID NO: 1049; CTL26_COMP |
| UCGCGUCGAACUCCGCAACCGA | SEQ ID NO: 1050; CTL29_COMP |
| ACCGAACGCCGUACCCAUCGGG | SEQ ID NO: 1051; CTL31_COMP |
| CGAGGGUAACGACUCUCGUGUC | SEQ ID NO: 1052; CTL36_COMP |
| TTGTAATACGACTCAACAGTAGATAAAGCGACGCGCG | SEQ ID NO: 1054; CTL30 |
| TTGTAATACGACTCAAACGCGACTTATCGAACGATAA | SEQ ID NO: 1055; CTL11 |
| TTGTAATACGACTCAAGGTTGCCTACATAGTAACTTC | SEQ ID NO: 1056; CTL23 |
| TTGTAATACGACTCACCCGGTAACAATTAGTCCCGCG | SEQ ID NO: 1057; CTL26 |
| TTGTAATACGACTCATCGGTTGCGGAGTTCGACGCGA | SEQ ID NO: 1058; CTL29 |
| TTGTAATACGACTCACCCGATGGGTACGGCGTTCGGT | SEQ ID NO: 1059; CTL31 |
| TTGTAATACGACTCAGACACGAGAGTCGTTACCCTCG | SEQ ID NO: 1060; CTL36 |
| TTGTAATACGACTCACCCGGTAACAATTAGACCCGCG | SEQ ID NO: 1061; CTL26_MUT |

All sequences for which the intensity of the spot was higher than the mean local background intensity plus 1.5 times its standard deviation were categorized as expressed microRNAs. The following criteria were required to be met in order consider the array intensity data valid for further analysis:
1. Specificity of the hybridization controls had to be within acceptance criteria (e.g. CTL26 vs. its corresponding single base mutant, CTL26_MUT).
2. Approximate equality of the signal intensity of the replicates of the positive controls
3. Approximate equality between median block signal intensities based on the positive controls for each block
4. Approximate equality between median array signals based on all sequences detected
5. Signal intensity for the purification and labelling control (CTL30).

Statistical normalization of the data was done by computing the Log2ratio where the Log2ratio equals average intensity signal of the duplicated spots/median intensity of all positives controls for the block. The normalization was done per block to avoid non-homogenous labelling of all blocks of the array. This block-by-block normalization has been shown to be more efficient then using overall normalization of the slide. The obtained values are Log2 values.

The intensities of the spots for each polynucleotide probe were compared in the sample from the tumors versus normal tissue, resulting in an evaluation of the relative expression for each microRNA.

The expression fold-change corresponds to 2^{(Log2ratio)}. The Log2ratio is the ratio between the two conditions compared, or log2(Xcell-line/Xnormal), which is the same as (log2Xcell-line - log2Xnormal), where X is the measured intensity value. In cases where there was no signal from the "normal" condition, the lowest measured intensity value in the experiment was used as the baseline from which a fold-change expression value was calculated. A fold-change value of less than zero corresponds to a down-regulation of (1/fold-change) times.

### Results

The microarray data was analyzed to identify target RNAs that were present at increased levels in more than half of the tumor samples tested. Tables 18 and 19 show the target RNAs, in what percentage of tumor samples they were present in increased levels, and the average fold-increase in those tumor samples in which they were present at increased levels. Data for six of the tumors are shown in Table 18 and data for the remaining 13 tumors are shown in Table 19. Table 18 also shows the probe sequences used to detect the target RNAs.

The microarray data was further analyzed to identify target RNAs that are present at levels at least 5-fold higher than normal levels in less than 50% of the tumor samples. Tables 20 and 21 show the target RNAs, in what percentage of tumor samples they were present in increased levels, and the average fold-increase in those tumor samples in which they were present at increased levels. Data for six of the tumors are shown in Table 20 and data for the remaining 13 tumors are shown in Table 21. Table 20 also shows the probe sequences used to detect the target RNAs. Finally, Table 22 shows the pre-microRNA sequences and chromosomal location of the pre-microRNA gene for each of the target RNAs in Tables 18 to 21.

The microarray data was next analyzed to identify target RNAs that were present at at least 5-fold decreased levels in more than half of the tumor samples tested. Tables 23 and 24 show the target RNAs, the probe sequences used to detect the target RNAs, and in what percentage of tumor samples they were present in increased levels. Data for six of the tumors are shown in Table 23 and data for the remaining 13 tumors are shown in Table 24. Table 25 shows the pre-microRNA sequences and chromosomal location of the pre-microRNA gene for each of the target RNAs in Tables 23 and 24.

Finally, Table 9, above, shows target RNAs that were present at higher levels in at least three of the five most aggressive cancers (Ksarc-19, Adk-9, Adk-10, Adk-29, and Lnec-31).

**Table 18: Target RNAs present at increased levels in at least 50% of tumor samples**

| **Gene** | **Probe sequence** | **SEQ ID NO** | **% tumors increased** | **Fold Chng Avg** | **ADK9** | **ADK10** | **Adk29** | **Adk15** | **Adk23** | **ADK48** |
|---|---|---|---|---|---|---|---|---|---|---|
| 10455-L5-1 | TAACAACTGCAATTGCGCCAAACATCCCTCTCC | 1063 | 58 | 6.4 | 2.05 | -3.86 | 1.02 | 5.18 | 2.20 | -3.86 |
| 12947-L5-4 | GTTCCCTTCTCCACAGCCTCTAAATCTCCCATCCG | 1064 | 63 | 5.0 | 3.17 | 2.58 | -1.79 | 1.50 | 2.10 | 1.18 |
| 13098-L5-1 | TGCCGGGGCGCTGTTGGCGGTTCCTCCGGGGGGT | 1065 | 58 | 4.4 | -2.86 | -2.86 | -2.86 | 1.70 | -1.49 | 5.93 |
| 13122-L5-1 | TTAGGAAATTCCATCTCACCTGCTCCAGTCC | 1066 | 58 | 11.5 | 1.32 | 1.17 | 1.40 | 1.68 | 1.56 | 2.09 |
| 13219-L5-1 | CTCTGACTCCCTCACTCAGTCTCTCTGCTCCAGC | 1067 | 68 | 7.8 | 1.54 | -1.85 | 1.30 | 1.90 | 1.44 | 2.19 |
| 13254-R5-1 | CAATGAACCACTGAACCACTCATGCACTGAACC | 1068 | 63 | 5.0 | 2.21 | 6.72 | 2.48 | 2.40 | 1.52 | 4.02 |
| 13467-L5-1 | GTGACAGTCAGACCCTCCTTGCTCCAAGTCAAA | 1069 | 53 | 17.7 | 2.83 | 3.05 | -1.83 | 3.62 | 2.22 | 1.35 |
| 3923-R5-1 | TCACAAAGGATCTCCTTCATCCCTCTCCAG | 1070 | 58 | 5.3 | -6.96 | -3.57 | -1.11 | 6.71 | 2.53 | 1.59 |
| 4440-L3-2* | TTTGACATTCAGAGCACTGGGCAGAAATCACA | 1071 | 68 | 6.9 | 2.32 | -1.15 | 1.65 | 2.00 | -1.34 | 1.29 |
| 4479-R3-1 | AGCCCCCTGCCCGGAAATTCAAAACAACTGC | 1072 | 53 | 4.3 | 1.27 | 1.08 | -1.38 | 1.11 | 1.55 | 1.75 |
| 5080-R3-1 | CTTGCAAAGGGTCTCCTTCATCCCTCTCCA | 1073 | 68 | 7.2 | 2.60 | -3.84 | 1.58 | 8.83 | 4.24 | -1.12 |
| 6216-L1-1 | GACATTCAGAGCACTGGGCAGAAATCACATG | 1074 | 53 | 8.0 | -1.89 | -1.89 | 1.27 | 2.22 | -1.89 | -1.89 |
| 6235-R5-2 | TCTGCTCCAAAAATCCATTTAATATATTGT | 1075 | 74 | 13.5 | 2.51 | 1.63 | 1.29 | 3.07 | 2.15 | 3.39 |
| 7426-L5-1 | AGACGAACACCTCCTGCTGTGCTTGATTT | 1076 | 63 | 5.1 | 2.59 | -1.45 | 3.22 | 2.64 | 1.21 | -1.45 |
| 7726-R3-2 | CATCCCTCTCCAGAAGAGGAGAAGAGGAAACA | 1077 | 68 | 5.7 | 2.18 | -2.52 | 1.66 | 7.34 | 3.64 | -2.52 |
| 8004-R3-2 | GGAACTGCTTCTCCTTGCTCCAGTCATTGAAG | 1078 | 63 | 11.0 | -5.32 | 1.60 | 1.51 | 1.80 | 1.80 | 2.37 |
| 836-R5-2 | AAATAATCATTCCAAATGGTTCTCCCTGCTAT | 1079 | 79 | 8.7 | 2.22 | 2.37 | 1.49 | 1.80 | 1.93 | 4.05 |
| 9349-R5-2 | GAACACAGTGATGCAGAGGACTTCCTGCTCCA | 1080 | 58 | 24.4 | -1.44 | 4.53 | 1.39 | 7.28 | 1.65 | -1.44 |
| 9594-R5-1 | ACTTAGACTTCCTTCCCACTCCCTGCATCCT | 1081 | 53 | 9.7 | 1.32 | 1.20 | -1.03 | 1.68 | 1.46 | 1.95 |
| miR-200a | ACATCGTTACCAGACAGTGTTA | 1082 | 58 | 6.2 | 4.00 | -1.98 | -1.98 | 1.89 | 2.18 | 4.85 |
| miR-200b | TCATCATTACCAGGCAGTATTA | 1083 | 63 | 4.6 | 3.65 | 1.66 | 1.01 | 2.11 | 2.43 | 2.54 |
| miR-200c | TCCATCATTACCCGGCAGTATTA | 1084 | 68 | 3.6 | 3.05 | 1.53 | -1.02 | 2.00 | 3.71 | 2.39 |
| miR-205 | CAGACTCCGGTGGAATGAAGGA | 1085 | 53 | 21.2 | 6.16 | -1.05 | 7.09 | -1.05 | -1.05 | -1.05 |
| miR-20b | CTACCTGCACTATGAGCACTTTG | 1086 | 53 | 4.8 | 3.82 | 4.97 | -2.24 | 5.93 | 1.03 | 2.35 |
| miR-21 | TCAACATCAGTCTGATAAGCTA | 1087 | 68 | 6.7 | 10.37 | 4.28 | -41.68 | 8.21 | 4.78 | 4.58 |
| miR-298 | TGGGAGAACCTCCCTGCTTCTGCT | 1088 | 68 | 9.8 | 1.96 | 1.55 | 1.87 | 2.23 | 2.18 | 1.30 |
| miR-720 | TGGAGGCCCCAGCGAGA | 1089 | 63 | 4.2 | 9.81 | 4.77 | 3.06 | 2.73 | 5.03 | 4.82 |

**Table 19: Target RNAs present at increased levels in at least 50% of tumor samples (con't)**

| **Gene** | **% tumors increased** | **Fold Chng Avg** | **Adk40** | **Adk41** | **Adk49** | **Epi42** | **Ksarc19** | **Kmalp21** | **Kmalp 25** | **EPI-4** | **Kmalp 44** | **Scc27** | **Lcnec31** | **Car13** |
|---|---|---|---|---|---|---|---|---|---|---|---|---|---|---|
| 10455-L5-1 | 58 | 6.4 | -3.86 | -3.86 | 4.88 | 6.15 | 11.22 | 8.61 | 2.50 | 20.65 | 3.92 | 1.74 | 1.71 | -3.86 |
| 12947-L5-4 | 63 | 5.0 | 7.18 | -2.89 | 1.75 | 2.00 | 10.83 | 4.19 | 2.17 | 15.66 | 4.39 | 3.14 | 2.39 | -2.89 |
| 13098-L5-1 | 58 | 4.4 | 6.04 | 2.12 | 4.93 | 7.99 | 2.92 | 2.06 | 1.19 | 5.45 | 5.13 | 1.99 | 1.68 | -2.86 |
| 13122-L5-1 | 58 | 11.5 | -2.23 | -3.30 | 11.06 | 3.00 | 45.07 | 8.66 | 12.05 | 25.75 | 4.32 | 8.92 | 4.14 | -6.80 |
| 13219-L5-1 | 68 | 7.8 | 2.12 | -3.09 | 4.87 | 2.37 | 29.25 | 8.14 | 10.45 | 23.80 | 3.38 | 7.05 | 3.81 | -5.56 |
| 13254-R5-1 | 63 | 5.0 | -5.53 | -5.53 | 4.42 | 3.62 | 1.67 | 2.00 | 5.81 | 14.41 | 2.64 | 1.20 | -1.16 | -2.34 |
| 13467-L5-1 | 53 | 17.7 | -3.86 | -3.86 | 6.58 | -1.54 | 47.10 | 19.28 | 11.18 | 87.67 | -1.56 | 6.95 | 4.54 | -3.86 |
| 3923-R5-1 | 58 | 5.3 | 1.69 | 1.36 | 5.55 | 6.66 | 7.94 | 6.74 | 2.87 | 12.31 | 3.49 | 1.89 | 1.78 | -6.96 |
| 4440-L3-2* | 68 | 6.9 | 14.22 | -2.38 | 4.89 | 5.53 | 4.84 | 4.40 | 4.36 | 20.54 | 7.80 | 5.37 | 9.54 | -2.38 |
| 4479-R3-1 | 53 | 4.3 | -14.83 | 1.85 | -1.15 | 5.75 | 5.60 | 6.31 | -14.83 | 10.98 | 1.91 | 4.07 | 1.95 | 2.02 |
| 5080-R3-1 | 68 | 7.2 | -3.84 | -1.79 | 8.12 | 10.22 | 12.60 | 9.22 | 3.75 | 18.91 | 6.17 | 2.71 | 2.74 | -3.84 |
| 6216-L1-1 | 53 | 8.0 | 15.02 | -1.89 | 2.61 | -1.89 | 5.37 | 4.69 | 3.76 | 22.08 | 9.22 | 5.16 | 9.56 | -1.89 |
| 6235-R5-2 | 74 | 13.5 | -2.30 | -1.76 | 11.09 | 4.52 | 53.43 | 16.58 | 19.84 | 39.60 | 6.59 | 11.81 | 11.43 | -8.31 |
| 7426-L5-1 | 63 | 5.1 | 2.23 | -1.45 | 6.10 | -1.45 | 6.82 | 4.16 | 3.33 | 11.69 | 5.48 | 5.61 | 7.41 | -1.45 |
| 7726-R3-2 | 68 | 5.7 | -2.52 | -2.52 | 8.06 | 9.94 | 8.81 | 7.20 | 3.54 | 11.79 | 2.24 | 2.37 | 2.77 | -2.52 |
| 8004-R3-2 | 63 | 11.0 | -1.69 | -5.32 | 6.36 | 3.58 | 48.62 | 7.86 | 12.44 | 28.62 | 4.41 | 9.48 | 5.21 | -5.32 |
| 836-R5-2 | 79 | 8.7 | 5.02 | -2.64 | 5.63 | 4.02 | 40.75 | 6.59 | 10.21 | 26.72 | 4.38 | 8.08 | 5.16 | -2.64 |
| 9349-R5-2 | 58 | 24.4 | -1.44 | -1.44 | 10.49 | 3.33 | 49.05 | 33.90 | 19.27 | 112.09 | 8.79 | 10.70 | 8.85 | -1.44 |
| 9594-R5-1 | 53 | 9.7 | -6.58 | -6.58 | 4.10 | 2.60 | 30.50 | 7.67 | 10.86 | 24.28 | 3.32 | 6.82 | 4.54 | -6.58 |
| miR-200a | 58 | 6.2 | -1.98 | -1.98 | 5.20 | 4.55 | -1.98 | 1.86 | 1.17 | 3.60 | 3.49 | 4.09 | 3.32 | 26.93 |
| miR-200b | 63 | 4.6 | -1.76 | 2.73 | 3.27 | 2.23 | -4.79 | 1.16 | 1.30 | 2.97 | 2.05 | 4.07 | 2.98 | 24.67 |
| miR-200c | 68 | 3.6 | -8.29 | 2.84 | 1.30 | 2.38 | -8.29 | -1.03 | 2.11 | 3.38 | 2.05 | 4.09 | 4.89 | 11.97 |
| miR-205 | 53 | 21.2 | -1.05 | -1.05 | 8.11 | 13.68 | 3.20 | 27.74 | 56.45 | 30.05 | 46.50 | -1.05 | -1.05 | -1.05 |
| miR-20b | 53 | 4.8 | -2.24 | 2.99 | 8.37 | 4.56 | -1.45 | -1.10 | 1.71 | -2.24 | -2.24 | 6.46 | 3.10 | -2.24 |
| miR-21 | 68 | 6.7 | -6.36 | 6.76 | 17.86 | 8.91 | 3.81 | -41.68 | 4.64 | 1.92 | 7.46 | 3.38 | 1.19 | 1.41 |
| miR-298 | 68 | 9.8 | -2.56 | -2.56 | 6.60 | 5.82 | 36.81 | 9.96 | 10.86 | 31.32 | 5.07 | 7.72 | 4.43 | -2.56 |
| miR-720 | 63 | 4.2 | 6.70 | -1.87 | 2.31 | -1.87 | 2.72 | 1.99 | 1.95 | -1.87 | 4.11 | 1.35 | -1.87 | -1.87 |

**Table 20: Target RNAs present at increased levels in less than 50% of tumor samples, but with an average increase of at least 5-fold relative to normal levels**

| **Gene** | **Probe sequence** | **SEQ ID NO** | **% tumors increased** | **Fold Change Average** | **ADK9** | **ADK10** | **Adk29** | **Adk15** | **Adk23** |
|---|---|---|---|---|---|---|---|---|---|
| 10083-L5-1 | CCCTCTTCCTTTCTACCCCCTCTCTCCACCC | 1090 | 16 | 11.2 | -1.64 | -4.49 | -10.22 | -3.32 | -1.50 |
| 10233-R5-1 | ACCCTCTCCCCTTGGATCTGGAGCAGCAGGCAGTAGA | 1091 | 16 | 13.1 | -1.55 | -7.07 | -4.19 | -1.46 | -1.77 |
| 10335-L5-2 | CCACTCCCCTCCTTTTTAATTAGAAAGCAC | 1092 | 16 | 5.3 | -1.72 | -8.23 | -8.23 | -2.21 | -3.36 |
| 10357-R5-1 | CTCTCCACTGTCTGAATATTCCATGTGTTTGTTTCCCTAG | 1093 | 16 | 6.0 | -1.13 | -1.13 | 1.25 | 2.75 | 1.29 |
| 10520-L5-1 | CAGCATCTCCTTGCTACCTCCTC | 1094 | 26 | 5.9 | -1.42 | -1.42 | -1.42 | -1.42 | 1.14 |
| 10844-R4-1 | AGGGGGTAGCATATTCAGGACTCTACTTTCAAA | 1095 | 26 | 5.4 | -2.58 | -2.58 | -2.58 | 1.36 | -2.00 |
| 11358-R5-2 | ATGGAGGGTGGGAAACTAGGCTGACATGGCTC | 1096 | 21 | 5.3 | -2.77 | -2.77 | -2.77 | 1.77 | -2.18 |
| 11444-L5-3 | GCACTTGTGTTCAGGCACCCACTCCTCCAGCAAAG | 1097 | 21 | 8.8 | -1.20 | -1.20 | -1.20 | -1.20 | -1.20 |
| 11547-R4-1 | GCCCCCTGCTCTGGCTGGTCAAACGGAACCAAGTCCGTCT | 1098 | 16 | 7.6 | -2.09 | -2.09 | -2.09 | -2.09 | -1.66 |
| 11605-L5-4 | TCCCTTCTTTAATTCCCTTCCCCTCAATTCCATAA | 1099 | 26 | 5.0 | -2.54 | -2.54 | -2.54 | -2.54 | -1.37 |
| 11688-R5-3 | GAGACGCCGACTGGCTGAGACTCAAGGCGGGG | 1100 | 32 | 6.0 | -1.33 | -1.33 | -1.33 | -1.33 | -1.33 |
| 12699-R5-2 | AGGGTGGTGGCGGTGGTGGCGGGAACGCTGGGGGG | 1101 | 26 | 6.0 | -1.96 | -1.96 | -1.96 | 1.14 | -1.60 |
| 12707-L5-2 | CATTCTCCTCTGCAATCCAACAACTCTAC | 1102 | 42 | 5.0 | -1.91 | -1.91 | -1.20 | 3.36 | -1.40 |
| 12729-R5-1 | CGACGAAAATGCAATTGTGTGCCTTCTCCCTCC | 1103 | 16 | 11.8 | -1.10 | -5.55 | -5.55 | -1.51 | -2.63 |
| 12730-R5-1 | TTGTGCTCCGCTCTCCGGGAAATGCCATCACTAAT | 1104 | 37 | 7.3 | -3.66 | -3.66 | -2.24 | 1.55 | -2.46 |
| 12888-L5-2 | TTTCCTACATTGTATGGTTCTCCCAGCTCCT | 1105 | 47 | 21.7 | 4.55 | 1.76 | 1.24 | 5.05 | 1.51 |
| 12907-L5-1 | ATGCCCTCTCTACCACGTCCTAGACACTGAGCC | 1106 | 26 | 6.4 | -3.23 | -3.23 | -3.23 | -1.53 | -1.46 |
| 12912-L5-2 | TCAAGTTTTCTGGCACCTTCCACCCACAGAGGCT | 1107 | 37 | 5.3 | -2.26 | -2.26 | -1.29 | 2.16 | -2.26 |
| 12917-R5-2 | GGACCTATGGGCCCTTCCCTTCCCCCAACATTG | 1108 | 47 | 6.0 | 1.03 | -3.76 | -1.02 | 1.54 | 2.26 |
| 12958-R5-1 | TCTTCCCCTGGGTGCATCTATAACGACTACAGA | 1109 | 32 | 5.1 | -1.32 | -1.32 | -1.32 | -1.32 | -1.32 |
| 12974-R5-2 | ACCCTCTGTGCCCCCAAATTCCTAGTCCCTTGGT | 1110 | 21 | 16.1 | -1.94 | -1.94 | -1.94 | -1.94 | -1.30 |
| 12979-R5-2 | TCCAGGTCCTTCCCCAGTAGCTAGATGAAAGAAAA | 1111 | 42 | 16.3 | -1.39 | -1.39 | 1.39 | 6.66 | 4.45 |
| 12992-L5-1 | CATACCTGCTTCCCTCCACCCCCATCTCTA | 1112 | 16 | 6.7 | -9.54 | -2.35 | -9.54 | -2.64 | -3.60 |
| 13001-L5-1 | TTCCTAGATACCACTCCCAGCTCCA | 1113 | 32 | 15.5 | -1.83 | -1.83 | -1.09 | 1.61 | 1.27 |
| 13053-R5-4 | GCCTTCCCTAAGATCAGGCTCATCAGGCAGCCCCA | 1114 | 26 | 5.6 | -1.32 | -1.32 | -1.32 | -1.32 | 1.25 |
| 13070-R5-3 | CTGTCTCCTCTCCCCAGTCCAAAGGACCTAATGC | 1115 | 16 | 20.1 | -1.13 | -4.70 | -4.70 | -1.17 | -2.22 |
| 13071-L5-3 | AGAGAAGGGGTGAGGATCTCCAGGGGTGACTGCT | 1116 | 32 | 7.2 | -2.11 | -2.11 | -2.11 | 1.69 | -2.11 |
| 13078-L5-1 | GCCCTCATTTCATAAGCCTGCTAGACAGGAGA | 1117 | 32 | 5.0 | -1.28 | -1.28 | -1.28 | -1.28 | 1.18 |
| 13185-L5-3 | TTCTGTTTTTTTTCTTCCCTCTCTCCCCTCTT | 1118 | 21 | 13.4 | -1.02 | -1.33 | -6.52 | -1.77 | -1.16 |
| 13216-R5-2 | ATATATTCCTCCTTTCTCCTCTCTGGCAGCTGA | 1119 | 26 | 5.1 | -1.79 | -1.79 | -1.79 | -1.79 | -1.03 |
| 13225-L5-1 | CTGCCCACTTCTGGGACAGCCCCTTCCATCTTC | 1120 | 16 | 5.9 | -3.37 | -3.37 | -3.37 | -1.24 | -3.37 |
| 13235-R5-1 | CCTGGGCTTTCTAGTCTCAGCTCTCCTCCAGCT | 1121 | 42 | 5.1 | 1.75 | 2.72 | -2.00 | -2.00 | -1.22 |
| 13245-L5-4 | ATTCCAAATAGTCTTTCCCTTCTACTCCCTTTAAA | 1122 | 16 | 12.2 | -2.87 | -2.87 | -2.87 | -2.87 | -2.25 |
| 13252-L5-3 | ACGTGCCTTCCTGACTGTGAGCTCCTTGAGAGC | 1123 | 32 | 5.0 | 3.73 | 2.50 | -1.79 | 3.92 | 1.23 |
| 13274-L5-3 | CCTTCTCTTCTCCCGTGCTCCCACCCTCCCTCAGGG | 1124 | 26 | 6.2 | -1.53 | -1.73 | -2.51 | -1.80 | -1.69 |
| 13300-L5-1 | TTACACACAGAATTATCTCCCCTCCTACTTACCC | 1125 | 16 | 7.4 | -1.04 | -6.08 | -6.08 | -2.44 | -3.08 |
| 13309-R5-1 | TCCCCCTTGTCTTCAGCTACAATTTTTCTGTG | 1126 | 16 | 8.2 | 1.66 | -3.05 | -3.05 | -3.05 | -3.05 |
| 13352-R5-1 | CCGGAACCTCAGCGCTGCATCTGTGAAATGGGG | 1127 | 37 | 5.8 | -3.13 | -3.13 | -3.13 | 1.36 | -2.38 |
| 13357-L5-4 | AGCGGACCTTCTCCCCACACCTCCCTGCAGCCTC | 1128 | 16 | 10.3 | -1.89 | -1.91 | -6.27 | -2.80 | -2.38 |
| 13366-R5-3 | CAGGCCTCACCCCAGTGCCCTCTCCTATTCCCAC | 1129 | 16 | 6.5 | -1.56 | -5.82 | -11.70 | -1.40 | -1.25 |
| 13373-R5-2 | TGTGGGGGGAGGGTGGTCAGCAGGTGGGGCAG | 1130 | 42 | 5.1 | -1.86 | -1.86 | -1.86 | 3.87 | -1.44 |
| 13375-R5-4 | GTACCAGCTCTCAGACCCTCACCACAGCCTGGGGG | 1131 | 32 | 5.8 | -7.06 | -7.06 | -7.06 | -2.47 | -1.39 |
| 13398-R5-4 | TTCTCTCTCCCTCCACCTTTTGTGCCACCACTT | 1132 | 16 | 15.6 | -5.01 | -2.59 | -5.01 | -1.56 | -2.35 |
| 13417-R5-4 | TGCCCTTCCTTCTGTTAACACCAGCCAGATCCCC | 1133 | 32 | 6.4 | -1.82 | -1.82 | -1.82 | 2.57 | 1.34 |
| 13436-L5-1 | AGCTCAATGAAGCCCCCAGCTAGGTCATGCCTC | 1134 | 21 | 5.3 | -2.80 | -8.31 | -20.29 | -2.23 | -2.79 |
| 13468-L5-1 | CAAGTGGCCATCAGACCCTCTTTGCCCCAAGTC | 1135 | 21 | 25.5 | -1.16 | -1.16 | -1.16 | -1.16 | 1.07 |
| 13470-R5-1 | TCCCACCCTCTCCACCTCAGGGACCAGAATCCT | 1136 | 21 | 13.2 | -1.29 | -3.12 | -6.50 | -1.24 | -1.23 |
| 13473-L5-3 | TATGTTTGCCCTTCTACCACCTATCCTGATACA | 1137 | 21 | 9.2 | -1.57 | -1.57 | -1.57 | 1.32 | 1.04 |
| 13500-L5-3 | CCAAGATCTTCCAGGCCTCTCTCTCCATTGAGT | 1138 | 37 | 6.4 | -1.38 | -1.38 | -1.38 | 2.14 | 1.20 |
| 13522-L5-4 | CTGCCTCACCTGAGCTCCCGTGCCTGTGCACCCTC | 1139 | 16 | 5.3 | -3.11 | -3.11 | -3.11 | -3.11 | -2.36 |
| 13523-R5-1 | GTGGAATAGTAAGGCTTCTTCCCCTGCCTGGC | 1140 | 26 | 5.3 | -3.75 | -3.75 | -1.20 | 1.11 | 1.61 |
| 13545-L5-1 | GCCTGTGGCGCAAGGGAGGCTGTGAGTCTGGGG | 1141 | 21 | 5.3 | -2.13 | -2.13 | -2.13 | 1.70 | -2.13 |
| 227-L5-1 | ACACCTGTCTCTCCCCAGTGCTTCCGCCCCTCA | 1142 | 16 | 5.0 | -2.46 | -3.09 | -2.60 | -3.09 | -3.20 |
| 3744-R5-1 | CTTCTCCTTCCTCCCTGCTCCCCTCCCACTAATGCCAAAT | 1143 | 37 | 6.7 | -1.38 | -2.00 | -2.67 | -1.30 | -1.23 |
| 3875-R5-2 | GACTGATTCAACCTCTCTCTCCCACTTTA | 1144 | 32 | 19.0 | -2.72 | -2.72 | -2.72 | 1.47 | -1.32 |
| 3926-L5-1 | ACTGATAATTCATTAGCATTCTTGAACGTGCCCCCACT | 1145 | 16 | 5.1 | -2.22 | -2.22 | -2.22 | -2.22 | -2.22 |
| 3992-R5-1 | GCCCCTTTCTCTTCTGTCAGTATCTGAGCTGATGGTTG | 1146 | 32 | 6.7 | -1.27 | -1.27 | -1.27 | -1.27 | -1.27 |
| 4203-R3-2 | GCACATTCCCACTTCCCCAGAGGCAGGCTCCATAT | 1147 | 16 | 6.0 | -2.01 | -2.01 | -2.01 | -1.45 | -2.01 |
| 4261-R5-1 | AATAACCCTCCTTCCTTGATAGATTTCAC | 1148 | 21 | 6.4 | -2.52 | -2.52 | -2.52 | -2.52 | -1.56 |
| 4291-R5-1 | GATCAGGTAGTTACCAGGAGCCATGGGGGTGTACA | 1149 | 37 | 6.0 | -1.67 | -1.67 | -1.67 | -1.25 | -1.67 |
| 4790-L5-2 | TAACCTGTCTCCCTCATTACTAGAATTCT | 1150 | 21 | 12.6 | -1.56 | -1.56 | -1.56 | -1.56 | -1.32 |
| 4958-L5-1 | TAACCTGTCTCCCTCATTACTAGAATTCT | 1151 | 32 | 5.0 | -2.14 | -2.14 | -2.14 | -1.00 | -2.14 |
| 4988-R5-2 | GAGAATTAATAAACCATGGCATCTTCTGAAAATGGGG | 1152 | 16 | 5.3 | -2.97 | -3.81 | -7.10 | -2.52 | -2.60 |
| 5108-R5-2 | CTCCTCCTCCCCGTCTTTGGATACCAAACAC | 1153 | 21 | 16.5 | -1.14 | -1.67 | -4.77 | -1.11 | -1.10 |
| 5232-L5-2 | CCCTCTCCTCCCACACCGTCACTCACAATAACCC | 1154 | 32 | 5.2 | -1.81 | -1.81 | 1.81 | -1.34 | -1.44 |
| 5392-R5-1 | TCCTCCTCCTTCATTTGCAGGACATCAAGG | 1155 | 42 | 7.0 | -2.12 | -2.12 | -2.12 | 2.25 | -1.14 |
| 5723-R5-1 | CCCTCTCCGACAGTATCTCATTACAATAATTGCTAATCTC | 1156 | 16 | 6.9 | -1.96 | -3.89 | -8.51 | -5.58 | -3.53 |
| 5836-L5-1 | CCTCCTCCCCTTTCTCCAGCAGTAGCCTTCTTAA | 1157 | 26 | 5.0 | -2.14 | -2.14 | -2.14 | 1.48 | -2.14 |
| 5842-R5-1 | TCTTGGGAACCAAGGGGGCTACAG | 1158 | 32 | 6.0 | 2.40 | -5.08 | -5.08 | 2.54 | 1.13 |
| 6037-R3-2 | GCAATTCCCTTTCCTCCATCTCCAATTTTCCTC | 1159 | 32 | 7.4 | -1.71 | -1.71 | -1.71 | -1.71 | -1.32 |
| 6181-L5-1 | CATGGTAAACTTAGATGACTCTTCCCCCTGGATTT | 1160 | 26 | 8.6 | -1.40 | -1.40 | -1.40 | -1.40 | -1.40 |
| 6233-L5-2 | CAAAATTAGATTTCCACTTTATCCTTCTCCC | 1161 | 16 | 10.1 | -3.02 | -3.02 | -3.02 | -1.89 | -1.92 |
| 6395-L5-1 | TCTCCCCTGCAGAGAGGAGGAAAGCCTGCCTCGGAAC | 1162 | 21 | 8.4 | -3.73 | -3.73 | -3.73 | 1.09 | -1.93 |
| 6405-R5-1 | TCAAAGAAAAATAAGTGAAAGCATATCTCATCATGGGG | 1163 | 32 | 5.7 | -2.10 | -2.10 | -2.10 | -2.10 | -2.10 |
| 6474-L5-1 | CCCTAATTAAAGCCATCCCCTCTTCCCCCTTCACC | 1164 | 16 | 11.9 | -11.16 | -5.02 | -11.16 | -1.96 | -1.85 |
| 6602-R3-1 | AGAGCCCCAGTGGAAATCTCTCCTCCAAATCCAT | 1165 | 32 | 8.8 | -1.36 | -1.36 | -1.36 | -1.36 | -1.06 |
| 6681-R2-1 | CCTGTTTTCTCCCCTCTCTCTCTGCCCCTCC | 1166 | 16 | 5.0 | -1.60 | -2.15 | -3.96 | -1.87 | -1.45 |
| 6683-R5-1 | TCATCCCAAAATAAACTCTTCCTGCTCAAG | 1167 | 37 | 11.9 | -1.39 | -1.39 | -1.39 | 1.04 | 1.29 |
| 6752-R5-2 | CCCTCCTTTCCCCACCTCAGTCGGGCCACTGCT | 1168 | 16 | 6.4 | -7.01 | -7.01 | -4.90 | -2.94 | -2.85 |
| 6803-R5-2 | GCTCCCTCTCTGGTTGGACCTCACCCAAA | 1169 | 21 | 12.2 | 1.30 | -2.22 | -1.17 | -1.05 | -1.19 |
| 6864-R4-1 | GACACACAATTACACTCCCCTGGAG | 1170 | 21 | 12.5 | -1.50 | -1.50 | -1.50 | -1.50 | -1.50 |
| 6872-L5-1 | AAGACTCTGTCACAGTCTGTGACCCGGTGGGG | 1171 | 42 | 5.2 | -1.96 | -1.96 | -1.96 | -1.00 | -1.96 |
| 6906-L5-1 | TCTTCTCCCCAACCAGCCAGCTCTCCTGG | 1172 | 21 | 8.0 | -2.17 | -2.17 | -2.17 | -2.17 | -2.17 |
| 6930-R5-1 | TTAATCCTTCTCTCCCCTCTGATCTTGCAG | 1173 | 16 | 14.8 | -1.27 | -1.66 | -4.45 | -1.91 | -1.65 |
| 7631-L3-2 | CCTAGACGCCAGCTGCCTGCATGAAGCCTGCCCAA | 1174 | 26 | 5.2 | -2.68 | -2.68 | -2.68 | 1.68 | 2.02 |
| 7764-R3-2 | CCCTCTCTGCCTCTCTCATCACCAATAACAGAC | 1175 | 16 | 9.3 | -1.03 | -3.64 | -3.64 | -1.33 | 1.24 |
| 7849-L2-2 | GAAATATCAAGACAAATAAGATGTTTGGGGGCACA | 1176 | 16 | 6.1 | -2.04 | -2.04 | -2.04 | -1.42 | -2.04 |
| 8316-R5-1 | CAGGGTATCCTCTCCCCACGTGATGCC | 1177 | 42 | 20.3 | 2.51 | -1.81 | -1.81 | 1.36 | 1.12 |
| 8433_C-R4-1 | AAACCAAAAAAAAAAAATTAAAAAGCGACGAAAATGCAATTGTGTGC CTTCTCCCTCC | 1178 | 16 | 14.3 | -1.03 | -2.46 | -5.42 | -1.38 | -1.21 |
| 8433-L3-1 | AAATGGCTCCTTTCCCCTTTCCCTCCACCG | 1179 | 16 | 6.9 | -1.70 | -1.71 | -5.48 | -2.04 | -1.62 |
| 8452-L3-1 | ATGGCCACATACATGGCTGGGGTGTTGAA | 1180 | 21 | 7.6 | -1.08 | -1.08 | -1.08 | -1.08 | -1.08 |
| 8724-R5-2 | GGCCAAGCTTGGAACCTCTCCCTGCCAGCA | 1181 | 16 | 6.0 | -1.71 | -2.12 | -4.29 | -1.92 | -1.69 |
| 8746-R5-1 | TAAGCGCAGGATGGGGGTATTCAAGTCCAAAGC | 1182 | 37 | 5.4 | -2.03 | -2.03 | -2.03 | 1.54 | -2.03 |
| 8808-R5-1 | GACCCCTTTCTCCCAGCCTGTTTCTGCAA | 1183 | 21 | 8.4 | -1.07 | -2.31 | -4.54 | -4.54 | -2.03 |
| 8832-R5-1 | TGGAGTACCACCTGTTTTTCCCCCACTT | 1184 | 32 | 6.0 | -1.37 | -1.37 | -1.37 | -1.37 | -1.06 |
| 8879-L5-1 | TGGGGGCTGGGGCGCAGGTCGAGAGCCCAGGGG | 1185 | 32 | 5.2 | -1.79 | -1.79 | -1.79 | -1.30 | -1.79 |
| 9485-L5-1 | TGCTGCACTCCATCCTCCCACAATGGCCCCATTGTTCCCAG | 1186 | 26 | 5.2 | -3.02 | -3.02 | -3.02 | 1.26 | -3.02 |
| 9507-L5-1 | GTCTCCCTCATCCATCATCCACCA | 1187 | 26 | 6.0 | -1.45 | -1.45 | -1.45 | -1.45 | 1.16 |
| 9798-L5-1 | CTCTATATATACCTGGTATGACTTCTTTGGGGGGAAAGAAAAGCAC | 1188 | 42 | 9.9 | -1.40 | -1.40 | -1.40 | 3.74 | 1.36 |
| 999996-L4-1 | GGGAGGAGTCAGGTGTGTGCTGTGGGTTGGGGGAAGAC | 1189 | 21 | 5.3 | -4.63 | -4.63 | -4.63 | 1.35 | -1.12 |
| 999999-R4-1 | CCCCTTGTCACCCCCAGCCCCTTCCTGGCCAGGACCCCAGCGAGG CCCAGAGAA | 1190 | 16 | 5.2 | -1.71 | -9.15 | -23.66 | -3.15 | -2.48 |
| miR-103 | TCATAGCCCTGTACAATGCTGCT | 1191 | 26 | 5.0 | 1.81 | -3.09 | 1.10 | 1.01 | 1.33 |
| miR-1202 | CTCCCCCACTGCAGCTGGCAC | 1192 | 16 | 5.4 | -1.58 | -7.52 | -7.52 | -5.40 | -4.07 |
| miR-1249 | TGAAGAAGGGGGGGAAGGGCGT | 1193 | 32 | 5.0 | -2.26 | -2.26 | -2.26 | 1.48 | -1.92 |
| miR-1275 | GACAGCCTCTCCCCCAC | 1194 | 16 | 12.7 | 1.07 | -1.12 | -12.61 | -1.07 | -2.42 |
| miR-129-3p | ATGCTTTTTGGGGTAAGGGCTT | 1195 | 11 | 21.4 | -1.00 | -1.00 | -1.00 | -1.00 | -1.00 |
| miR-1321 | ATCACATTCACCTCCCTG | 1196 | 16 | 5.9 | -2.14 | -2.14 | -2.14 | -1.05 | -2.14 |
| miR-1323 | AGAAAATGCCCCTCAGTTTTGA | 1197 | 47 | 5.0 | 2.09 | -1.57 | -3.13 | 1.70 | -1.05 |
| miR-141 | CCATCTTTACCAGACAGTGTTA | 1198 | 42 | 5.6 | 4.01 | -1.96 | -1.37 | 2.58 | 3.17 |
| miR-143 | GAGCTACAGTGCTTCATCTCA | 1199 | 42 | 5.8 | 1.65 | -4.11 | 1.63 | 1.38 | 1.30 |
| miR-182 | AGTGTGAGTTCTACCATTGCCAAA | 1200 | 37 | 8.7 | -1.00 | -1.00 | -1.00 | 2.28 | 1.51 |
| miR-183 | AGTGAATTCTACCAGTGCCATA | 1201 | 21 | 10.0 | -1.00 | -1.00 | -1.00 | -1.00 | -1.00 |
| miR-198 | GAACCTATCTCCCCTCTGGACC | 1202 | 21 | 12.6 | -4.73 | -4.73 | -4.73 | -1.31 | -2.54 |
| miR-19a | TCAGTTTTGCATAGATTTGCACA | 1203 | 47 | 5.0 | 2.88 | -1.76 | -1.23 | 2.71 | -1.03 |
| miR-30c-1* | GGAGTAAACAACCCTCTCCCAG | 1204 | 26 | 11.2 | -1.05 | -8.25 | -8.25 | -1.14 | -1.53 |
| miR-375 | TCACGCGAGCCGAACGAACAAA | 1205 | 32 | 16.2 | -1.60 | 4.80 | -1.60 | 2.88 | -1.60 |
| miR-376c | ACGTGGAATTTCCTCTATGTT | 1206 | 16 | 62.7 | -1.12 | -1.12 | -1.12 | -1.12 | -1.12 |
| miR-429 | ACGGTTTTACCAGACAGTATTA | 1207 | 16 | 8.1 | 3.25 | -1.00 | -1.00 | -1.00 | -1.00 |
| miR-483-5p | CTCCCTTCTTTCCTCCCGTCTT | 1208 | 42 | 10.1 | 2.71 | -1.10 | -3.38 | 2.43 | 1.23 |
| miR-516a-5p | GAAAGTGCTTCTTTCCTCGAGAA | 1209 | 47 | 8.8 | 2.57 | -1.65 | -1.65 | 1.35 | -1.19 |
| miR-7 | ACAACAAAATCACTAGTCTTCCA | 1210 | 21 | 86.3 | -1.00 | -1.00 | 1.53 | -1.00 | 1.56 |

**Table 21: Target RNAs present at levels at least 5-fold higher than nonnal levels in less than 50% of tumor samples (con't)**

| **Gene** | **% tumors increased** | **Fold Change Average** | **ADK48** | **Adk40** | **Adk41** | **Adk49** | **Epi42** | **Ksarc19** | **Kmalp 21** | **Kmalp 25** | **EPI-4** | **Kmalp 44** | **Scc27** | **Lcnec31** | **Car13** |
|---|---|---|---|---|---|---|---|---|---|---|---|---|---|---|---|
| 10083-L5-1 | 16 | 11.2 | -3.12 | -10.22 | -10.22 | -4.85 | 1.60 | 14.20 | 6.04 | -1.20 | 13.39 | -1.15 | 1.20 | -1.16 | -10.22 |
| 10233-R5-1 | 16 | 13.1 | -1.29 | -15.77 | -1.70 | 1.17 | 1.33 | 17.61 | 6.52 | -1.39 | 15.23 | 1.27 | 1.51 | 1.10 | -6.54 |
| 10335-L5-2 | 16 | 5.3 | -2.42 | -8.23 | -8.23 | -8.23 | 1.82 | 5.00 | 2.55 | -1.85 | 8.20 | -8.23 | -1.15 | -1.71 | -8.23 |
| 10357-R5-1 | 16 | 6.0 | -1.13 | 12.33 | -1.13 | 1.73 | -1.13 | 2.98 | -1.13 | 1.86 | -1.13 | -1.13 | 1.37 | -1.13 | -1.13 |
| 10520-L5-1 | 26 | 5.9 | -1.42 | -1.42 | -1.42 | -1.42 | -1.42 | 7.33 | 3.18 | 4.71 | 10.86 | -1.42 | 3.49 | 1.34 | -1.42 |
| 10844-R4-1 | 26 | 5.4 | 5.98 | 4.89 | 1.80 | 4.75 | 6.90 | 1.69 | 1.36 | 1.08 | -2.58 | 4.49 | 1.09 | -2.58 | -2.58 |
| 11358-R5-2 | 21 | 5.3 | 4.38 | -2.77 | 1.50 | 7.69 | 5.96 | 1.31 | -1.16 | 1.17 | -2.77 | 2.99 | 1.18 | -2.77 | -2.77 |
| 11444-L5-3 | 21 | 8.8 | -1.20 | -1.20 | -1.20 | -1.20 | -1.20 | 13.50 | 5.64 | -1.20 | 13.27 | -1.20 | 2.62 | -1.20 | -1.20 |
| 11547-R4-1 | 16 | 7.6 | -2.09 | -2.09 | -2.09 | -2.09 | 1.98 | 2.11 | 5.05 | 1.30 | 15.60 | -2.09 | 1.36 | -1.23 | -2.09 |
| 11605-L5-4 | 26 | 5.0 | -2.54 | -2.54 | -2.54 | -2.54 | -2.54 | 7.43 | 3.53 | 1.60 | 9.69 | -2.54 | 2.07 | 1.86 | -2.54 |
| 11688-R5-3 | 32 | 6.0 | 6.88 | 2.76 | 1.49 | 5.01 | 7.47 | 1.72 | 1.11 | -1.33 | -1.33 | 4.67 | -1.33 | -1.33 | -1.33 |
| 12699-R5-2 | 26 | 6.0 | 1.74 | 7.41 | 5.23 | 5.07 | 7.67 | 1.00 | 1.66 | 1.16 | -1.96 | 4.62 | -1.16 | 1.61 | -1.96 |
| 12707-L5-2 | 42 | 5.0 | -1.91 | -1.91 | -1.91 | 6.52 | -1.91 | 7.16 | 4.03 | 2.78 | -1.91 | 7.26 | 4.33 | 4.33 | -1.91 |
| 12729-R5-1 | 16 | 11.8 | -5.55 | -5.55 | -5.55 | -5.55 | -5.55 | 15.59 | 5.64 | -1.24 | 14.17 | 1.67 | 1.74 | 1.06 | -5.55 |
| 12730-R5-1 | 37 | 7.3 | -1.10 | -3.66 | -3.66 | 3.20 | -1.72 | 8.17 | 6.34 | 1.57 | 23.70 | 5.24 | 2.24 | 2.37 | -3.66 |
| 12888-L5-2 | 47 | 21.7 | -1.64 | -1.64 | -1.64 | 7.18 | 1.22 | 43.87 | 23.04 | 11.51 | 82.88 | 1.23 | 11.54 | 5.75 | -1.64 |
| 12907-L5-1 | 26 | 6.4 | -3.23 | -3.23 | -3.23 | -1.68 | 4.63 | 10.17 | 3.99 | 1.05 | 11.02 | 2.09 | 1.42 | 1.32 | -3.23 |
| 12912-L5-2 | 37 | 5.3 | -2.26 | -2.26 | -2.26 | -1.02 | -2.26 | 7.22 | 2.14 | 2.86 | 14.96 | -2.26 | 3.88 | 4.19 | -2.26 |
| 12917-R5-2 | 47 | 6.0 | -3.76 | -3.76 | -3.76 | -3.76 | 2.93 | 14.76 | 7.83 | 2.46 | 16.79 | 2.54 | 2.64 | 2.10 | -3.76 |
| 12958-R5-1 | 32 | 5.1 | -1.32 | -1.32 | -1.32 | -1.32 | -1.32 | 6.38 | 4.19 | 1.98 | 12.26 | -1.32 | 2.45 | 3.54 | -1.32 |
| 12974-R5-2 | 21 | 16.1 | -1.94 | -1.94 | -1.94 | -1.94 | -1.94 | 24.20 | 12.93 | 1.29 | 29.06 | -1.94 | 1.81 | 1.64 | -1.94 |
| 12979-R5-2 | 42 | 16.3 | -1.39 | -1.39 | -1.39 | -1.39 | -1.39 | 10.78 | 36.28 | 4.84 | 58.47 | 1.46 | 5.16 | 4.12 | -1.39 |
| 12992-L5-1 | 16 | 6.7 | -9.54 | -9.54 | -9.54 | -4.91 | 1.18 | 9.52 | 3.16 | -2.51 | 7.29 | -1.36 | -1.30 | -1.84 | -3.31 |
| 13001-L5-1 | 32 | 15.5 | -1.83 | -1.83 | -1.83 | 1.30 | -1.83 | 42.81 | 17.02 | 13.68 | 4.38 | -1.83 | 8.78 | 6.48 | -1.83 |
| 13053-R5-4 | 26 | 5.6 | -1.32 | -1.32 | -1.32 | -1.32 | -1.32 | 3.53 | 13.64 | 2.00 | 6.49 | -1.32 | 2.42 | 1.51 | -1.32 |
| 13070-R5-3 | 16 | 20.1 | -1.56 | -1.56 | -4.70 | -1.74 | -2.22 | 28.78 | 10.28 | -1.18 | 21.26 | 1.71 | 1.74 | 1.44 | -4.70 |
| 13071-L5-3 | 32 | 7.2 | 9.95 | 11.19 | -2.11 | 2.59 | 8.14 | 1.71 | -1.39 | 1.41 | -2.11 | 9.31 | -1.31 | -2.11 | 1.49 |
| 13078-L5-1 | 32 | 5.0 | -1.28 | -1.28 | -1.28 | -1.28 | -1.28 | 5.67 | 3.96 | 2.12 | 9.20 | -1.28 | 4.09 | 4.68 | -1.28 |
| 13185-L5-3 | 21 | 13.4 | 1.19 | -10.17 | -4.63 | -2.11 | 1.40 | 22.48 | 9.50 | -1.19 | 19.78 | 1.34 | 1.88 | 1.66 | -1.67 |
| 13216-R5-2 | 26 | 5.1 | -1.79 | -1.79 | -1.79 | -1.79 | -1.79 | 9.13 | 3.36 | 1.72 | 8.24 | -1.79 | 2.44 | 2.56 | -1.79 |
| 13225-L5-1 | 16 | 5.9 | -3.37 | -3.37 | -3.37 | -1.14 | -1.38 | 3.98 | 4.43 | 1.04 | 9.41 | -3.37 | 1.69 | 1.75 | -3.37 |
| 13235-R5-1 | 42 | 5.1 | -2.00 | 6.84 | -2.00 | -2.00 | -2.00 | 4.04 | 10.73 | 2.12 | 9.58 | 1.76 | 2.43 | 2.20 | -2.00 |
| 13245-L5-4 | 16 | 12.2 | -2.87 | -2.87 | -2.87 | -2.87 | -2.87 | 14.29 | 12.06 | 1.04 | 10.37 | -2.87 | 1.47 | 1.29 | -2.87 |
| 13252-L5-3 | 32 | 5.0 | 7.58 | -3.66 | -3.66 | 3.75 | -1.66 | -1.06 | -1.78 | 1.28 | 1.36 | -3.66 | 1.14 | 1.12 | -3.66 |
| 13274-L5-3 | 26 | 6.2 | -1.00 | -1.48 | -2.00 | 1.50 | 1.33 | 13.34 | 3.11 | 2.88 | 9.05 | 1.27 | 2.80 | 1.56 | -1.16 |
| 13300-L5-1 | 16 | 7.4 | -1.75 | -6.08 | -6.08 | -1.13 | -2.47 | 5.40 | 3.67 | -1.28 | 13.10 | -6.08 | 1.07 | -1.03 | -6.08 |
| 13309-R5-1 | 16 | 8.2 | -3.05 | -3.05 | -3.05 | -3.05 | -3.05 | 9.45 | 2.79 | 1.01 | 12.42 | -3.05 | 1.39 | -3.05 | -3.05 |
| 13352-R5-1 | 37 | 5.8 | 6.85 | 7.65 | -3.13 | 4.84 | 4.78 | 1.36 | -1.04 | -1.01 | -3.13 | 7.39 | 1.08 | -1.49 | 3.02 |
| 13357-L5-4 | 16 | 10.3 | -4.08 | -13.36 | -13.36 | -1.26 | -1.04 | 13.07 | 4.68 | -1.56 | 13.01 | -1.17 | 1.17 | -1.45 | -13.36 |
| 13366-R5-3 | 16 | 6.5 | -3.19 | -11.70 | -11.70 | 1.49 | -4.14 | 6.00 | 3.36 | -1.62 | 10.24 | -4.94 | -1.07 | -1.49 | -11.70 |
| 13373-R5-2 | 42 | 5.1 | 8.95 | 6.03 | 4.53 | 2.23 | 6.76 | 1.51 | 4.97 | 1.11 | -1.86 | 3.80 | -1.32 | -1.06 | -1.86 |
| 13375-R5-4 | 32 | 5.8 | 1.45 | -1.36 | 3.34 | 5.09 | 3.16 | 2.32 | -1.53 | -1.93 | 4.20 | 16.42 | -1.56 | -3.11 | -7.06 |
| 13398-R5-4 | 16 | 15.6 | -1.64 | -5.01 | -5.01 | -5.01 | -5.01 | 24.60 | 7.38 | -1.41 | 14.84 | -5.01 | 1.57 | 1.33 | -5.01 |
| 13417-R5-4 | 32 | 6.4 | -1.82 | -1.82 | -1.82 | 2.73 | 11.74 | 7.23 | 4.35 | -1.82 | 9.97 | -1.82 | -1.82 | 1.33 | -1.82 |
| 13436-L5-1 | 21 | 5.3 | 10.40 | -20.29 | -20.29 | 3.70 | -3.72 | -1.06 | 2.55 | -2.03 | 4.35 | -1.04 | -1.31 | -2.32 | -20.29 |
| 13468-L5-1 | 21 | 25.5 | -1.16 | -1.16 | -1.16 | -1.16 | -1.16 | 25.58 | 42.79 | -1.16 | 31.50 | -1.16 | 2.25 | -1.16 | -1.16 |
| 13470-R5-1 | 21 | 13.2 | -6.50 | -6.50 | -6.50 | 1.75 | 2.16 | 22.13 | 9.28 | -1.38 | 19.17 | 1.62 | 1.31 | 1.04 | -6.50 |
| 13473-L5-3 | 21 | 9.2 | -1.57 | -1.57 | -1.57 | -1.57 | -1.57 | 12.99 | 5.85 | 1.57 | 15.53 | -1.57 | 2.39 | 1.37 | -1.57 |
| 13500-L5-3 | 37 | 6.4 | -1.38 | -1.38 | -1.38 | -1.38 | -1.38 | 11.94 | 6.95 | 2.11 | 14.71 | -1.38 | 2.73 | 3.87 | -1.38 |
| 13522-L5-4 | 16 | 5.3 | -3.11 | -3.11 | -3.11 | 3.37 | -3.11 | 2.60 | 1.14 | 1.10 | 9.89 | -3.11 | 1.73 | 1.65 | -3.11 |
| 13523-R5-1 | 26 | 5.3 | -3.75 | -3.75 | -3.75 | 1.01 | -3.75 | 7.12 | 4.25 | 1.30 | 11.04 | 1.81 | 2.32 | 1.90 | -3.75 |
| 13545-L5-1 | 21 | 5.3 | 1.56 | 5.75 | 4.10 | 4.33 | 7.14 | 1.80 | -1.46 | 1.04 | -2.13 | -2.13 | -2.13 | -2.13 | -2.13 |
| 227-L5-1 | 16 | 5.0 | -1.10 | -8.03 | -2.54 | -1.26 | -1.22 | 3.33 | 5.25 | -1.86 | 5.98 | 1.00 | -1.14 | -1.09 | -4.63 |
| 3744-R5-1 | 37 | 6.7 | 1.22 | -1.55 | -1.88 | 2.15 | 1.70 | 17.73 | 4.94 | 4.32 | 12.79 | 1.63 | 2.72 | 1.99 | -2.37 |
| 3875-R5-2 | 32 | 19.0 | -2.72 | -2.72 | -2.72 | -2.72 | -2.72 | 47.37 | 21.41 | 1.80 | 36.52 | 3.32 | 2.67 | 2.60 | -2.72 |
| 3926-L5-1 | 16 | 5.1 | -2.22 | -2.22 | -2.22 | -2.22 | 5.38 | 1.86 | 2.06 | 1.37 | 7.91 | -2.22 | 1.57 | -1.06 | -2.22 |
| 3992-R5-1 | 32 | 6.7 | -1.27 | -1.27 | -1.27 | -1.27 | -1.27 | 11.59 | 6.64 | 2.32 | 12.31 | -1.27 | 3.77 | 3.55 | -1.27 |
| 4203-R3-2 | 16 | 6.0 | -2.01 | -2.01 | -2.01 | -2.01 | -2.01 | 7.42 | 3.12 | -2.01 | 7.32 | -2.01 | -1.16 | -2.01 | -2.01 |
| 4261-R5-1 | 21 | 6.4 | 1.22 | -2.52 | -2.52 | 1.50 | 4.19 | 5.56 | 9.03 | 1.23 | 6.64 | -2.52 | 1.69 | 1.33 | -2.52 |
| 4291-R5-1 | 37 | 6.0 | 6.21 | 9.19 | 5.89 | 2.44 | 7.75 | -1.03 | 1.01 | -1.67 | -1.67 | 4.73 | -1.67 | -1.67 | -1.67 |
| 4790-L5-2 | 21 | 12.6 | -1.56 | -1.56 | -1.56 | -1.56 | -1.56 | 21.19 | 7.09 | 1.61 | 19.81 | -1.56 | 2.37 | 1.17 | -1.56 |
| 4958-L5-1 | 32 | 5.0 | 4.94 | 6.00 | 4.13 | 4.23 | 6.24 | -2.14 | -1.19 | -1.04 | -2.14 | 4.24 | -2.14 | -2.14 | -2.14 |
| 4988-R5-2 | 16 | 5.3 | 1.08 | -25.28 | -3.70 | -1.17 | 1.20 | 5.54 | 3.48 | -2.05 | 6.82 | 1.28 | 1.05 | -1.34 | -8.12 |
| 5108-R5-2 | 21 | 16.5 | -2.27 | -7.21 | -7.21 | 1.49 | 1.77 | 31.12 | 11.82 | 1.05 | 21.13 | 1.91 | 2.06 | 1.54 | -1.29 |
| 5232-L5-2 | 32 | 5.2 | -1.81 | -1.81 | -1.81 | -1.81 | 5.58 | 6.39 | 2.75 | 2.65 | 1.47 | -1.81 | 2.30 | -1.81 | -1.81 |
| 5392-R5-1 | 42 | 7.0 | -2.12 | -2.12 | -2.12 | 3.87 | 4.77 | 15.89 | 6.70 | 1.74 | 16.76 | 3.71 | 1.91 | 1.15 | -2.12 |
| 5723-R5-1 | 16 | 6.9 | -2.80 | -8.51 | -8.51 | -2.09 | 1.62 | 8.94 | 2.94 | -1.53 | 8.79 | 1.02 | 1.08 | -1.42 | -8.51 |
| 5836-L5-1 | 26 | 5.0 | 1.59 | 6.47 | 4.54 | 2.07 | 7.39 | 1.66 | -1.54 | -2.14 | -2.14 | 4.43 | -2.14 | -2.14 | -2.14 |
| 5842-R5-1 | 32 | 6.0 | -1.05 | -5.08 | 1.12 | -1.02 | 2.33 | 2.71 | 9.50 | 1.02 | 16.41 | -1.31 | -1.32 | -1.44 | -5.08 |
| 6037-R3-2 | 32 | 7.4 | -1.71 | -1.71 | -1.71 | -1.71 | -1.71 | 16.51 | 6.11 | 1.91 | 14.55 | -1.71 | 2.97 | 2.07 | -1.71 |
| 6181-L5-1 | 26 | 8.6 | -1.40 | -1.40 | -1.40 | -1.40 | -1.40 | 16.60 | 5.10 | 1.57 | 15.61 | -1.40 | 2.68 | 2.92 | -1.40 |
| 6233-L5-2 | 16 | 10.1 | -3.02 | -3.02 | -3.02 | -3.02 | -3.02 | 10.71 | 4.99 | -1.05 | 14.68 | -3.02 | 1.54 | 1.57 | -3.02 |
| 6395-L5-1 | 21 | 8.4 | -3.73 | -3.73 | -3.73 | 1.35 | 4.16 | 7.80 | 6.46 | -1.04 | 15.00 | -3.73 | 1.67 | 1.45 | -3.73 |
| 6405-R5-1 | 32 | 5.7 | 6.13 | 7.07 | 4.77 | 5.11 | 6.86 | -2.10 | 1.46 | -2.10 | -2.10 | 4.48 | -2.10 | -2.10 | -2.10 |
| 6474-L5-1 | 16 | 11.9 | -3.05 | -11.16 | -11.16 | -11.16 | -4.35 | 14.82 | 7.02 | -1.48 | 13.75 | -4.80 | 1.09 | -1.19 | -11.16 |
| 6602-R3-1 | 32 | 8.8 | -1.36 | -1.36 | -1.36 | -1.36 | -1.36 | 19.18 | 6.22 | 3.09 | 17.33 | -1.36 | 3.82 | 2.93 | -1.36 |
| 6681-R2-1 | 16 | 5.0 | -1.47 | -1.37 | -1.55 | -1.23 | 1.40 | 5.10 | 2.76 | -1.63 | 6.93 | -1.55 | 1.43 | -1.17 | -3.04 |
| 6683-R5-1 | 37 | 11.9 | -1.39 | -1.39 | -1.39 | 1.48 | -1.39 | 26.81 | 6.23 | 10.02 | 25.16 | 5.08 | 6.03 | 4.31 | -1.39 |
| 6752-R5-2 | 16 | 6.4 | -2.32 | -7.01 | -1.56 | -7.01 | -1.38 | 7.39 | 3.11 | -1.67 | 8.79 | -7.01 | 1.18 | -1.03 | -7.01 |
| 6803-R5-2 | 21 | 12.2 | 1.33 | 1.11 | -1.53 | 1.68 | 1.59 | 9.14 | 18.88 | 1.50 | 18.71 | 1.83 | 1.99 | 1.71 | -11.71 |
| 6864-R4-1 | 21 | 12.5 | 2.22 | -1.50 | -1.50 | 1.54 | 1.44 | 2.95 | 1.88 | 1.78 | 42.80 | -1.50 | 2.10 | 1.47 | -1.50 |
| 6872-L5-1 | 42 | 5.2 | 5.15 | 7.34 | 4.47 | 4.25 | 5.82 | 1.59 | 6.06 | -1.96 | -1.96 | 3.74 | -1.96 | -1.96 | -1.96 |
| 6906-L5-1 | 21 | 8.0 | -2.17 | -2.17 | -2.17 | -2.17 | -2.17 | 14.47 | 3.55 | 1.13 | 12.00 | -2.17 | 2.01 | 1.54 | -2.17 |
| 6930-R5-1 | 16 | 14.8 | -2.83 | -10.13 | -10.13 | 1.07 | 1.26 | 22.64 | 6.91 | -1.19 | 14.99 | 1.28 | 1.74 | 1.04 | -10.13 |
| 7631-L3-2 | 26 | 5.2 | -2.68 | -2.68 | -2.68 | -1.18 | 7.17 | 3.13 | 5.17 | 1.30 | 8.27 | -2.68 | 1.40 | 1.50 | -2.68 |
| 7764-R3-2 | 16 | 9.3 | -3.64 | -3.64 | -3.64 | -3.64 | -3.64 | 12.47 | 4.37 | -1.09 | 11.00 | -3.64 | 1.53 | 1.54 | -3.64 |
| 7849-L2-2 | 16 | 6.1 | 1.61 | 6.57 | 4.84 | 1.03 | 6.78 | 1.71 | -1.05 | -2.04 | -2.04 | -2.04 | -1.29 | -2.04 | -2.04 |
| 8316-R5-1 | 42 | 20.3 | -1.81 | -1.81 | -1.81 | -1.81 | -1.81 | 66.20 | 16.64 | 2.12 | 60.43 | 6.75 | 3.99 | 3.79 | -1.81 |
| 8433_C-R4-1 | 16 | 14.3 | -1.74 | -5.42 | -5.42 | -1.36 | -2.50 | 18.24 | 6.98 | -1.37 | 17.79 | 1.52 | 1.68 | 1.01 | -5.42 |
| 8433-L3-1 | 16 | 6.9 | 1.35 | -8.08 | -8.08 | -4.05 | 1.17 | 8.10 | 3.41 | -1.39 | 9.18 | -1.06 | 1.19 | 1.05 | -3.51 |
| 8452-L3-1 | 21 | 7.6 | -1.08 | 11.39 | 4.06 | 4.04 | 11.10 | -1.08 | -1.08 | -1.08 | -1.08 | -1.08 | -1.08 | -1.08 | -1.08 |
| 8724-R5-2 | 16 | 6.0 | -2.90 | -9.03 | -4.67 | 1.24 | 1.37 | 6.87 | 3.34 | -1.55 | 7.87 | 1.04 | 1.14 | -1.14 | -9.03 |
| 8746-R5-1 | 37 | 5.4 | 6.65 | 6.64 | 4.26 | 4.91 | 6.70 | 1.49 | 1.68 | 1.04 | -2.03 | 4.06 | -2.03 | -2.03 | -2.03 |
| 8808-R5-1 | 21 | 8.4 | -4.54 | -4.54 | -4.54 | -4.54 | -4.54 | 12.95 | 5.01 | 1.08 | 13.67 | 1.70 | 1.57 | 1.02 | -4.54 |
| 8832-R5-1 | 32 | 6.0 | -1.37 | -1.37 | -1.37 | -1.37 | -1.37 | 4.44 | 3.33 | 2.34 | 19.28 | -1.37 | 3.13 | 3.30 | -1.37 |
| 8879-L5-1 | 32 | 5.2 | 5.93 | 6.75 | 2.28 | 1.04 | 6.13 | -1.79 | -1.07 | -1.79 | -1.79 | 4.14 | -1.79 | -1.79 | -1.79 |
| 9485-L5-1 | 26 | 5.2 | -3.02 | -3.02 | -3.02 | 2.49 | 3.21 | 3.66 | 4.65 | -1.18 | 11.90 | -3.02 | 1.00 | -1.56 | -3.02 |
| 9507-L5-1 | 26 | 6.0 | -1.45 | -1.45 | -1.45 | -1.45 | -1.45 | 8.15 | 1.99 | 2.56 | 14.45 | -1.45 | 2.84 | 1.67 | -1.45 |
| 9798-L5-1 | 42 | 9.9 | 14.66 | 15.37 | 4.13 | 9.21 | 16.73 | 1.77 | 4.26 | -1.40 | -1.40 | 10.93 | -1.40 | -1.40 | -1.40 |
| 999996-L4-1 | 21 | 5.3 | 6.41 | -1.05 | 5.20 | 4.33 | 1.88 | 1.23 | -1.40 | -1.10 | -4.63 | 5.08 | -1.06 | -2.07 | -1.67 |
| 999999-R4-1 | 16 | 5.2 | 9.26 | -23.66 | -1.25 | 2.35 | -7.93 | 1.60 | 1.32 | -2.72 | 3.99 | -7.30 | -1.89 | -2.94 | -23.66 |
| miR-103 | 26 | 5.0 | -1.99 | -6.84 | 1.87 | 3.43 | 2.56 | 1.28 | -1.00 | 1.19 | 1.66 | 1.80 | 3.59 | 1.79 | 12.88 |
| miR-1202 | 16 | 5.4 | -7.52 | -7.52 | -7.52 | -7.52 | 1.62 | 6.96 | 2.14 | -2.10 | 7.21 | 1.01 | -1.32 | -1.79 | -7.52 |
| miR-1249 | 32 | 5.0 | 5.38 | 5.84 | 2.27 | 4.52 | 8.04 | 1.49 | -1.12 | 1.02 | -2.26 | 4.17 | 1.10 | -2.26 | -2.26 |
| miR-1275 | 16 | 12.7 | -12.61 | -4.87 | -12.61 | -1.62 | 1.01 | 9.83 | 7.58 | -2.54 | 20.69 | -1.44 | -1.20 | -1.52 | -12.61 |
| miR-129-3p | 11 | 21.4 | -1.00 | -1.00 | -1.00 | -1.00 | 11.04 | -1.00 | -1.00 | -1.00 | -1.00 | -1.00 | -1.00 | -1.00 | 31.72 |
| miR-1321 | 16 | 5.9 | -2.14 | -2.14 | -2.14 | -2.14 | -2.14 | 8.17 | 2.93 | 1.49 | 6.53 | -2.14 | 1.63 | -2.14 | -2.14 |
| miR-1323 | 47 | 5.0 | 1.07 | 1.16 | -3.13 | 4.58 | 11.61 | 4.63 | 4.00 | 1.86 | 8.00 | 4.21 | 3.41 | 1.75 | -3.13 |
| miR-141 | 42 | 5.6 | 1.66 | -1.96 | 1.02 | 5.85 | 1.14 | -1.96 | -1.96 | 1.49 | -1.96 | 1.83 | 3.28 | 3.22 | 16.77 |
| miR-143 | 42 | 5.8 | 4.04 | -4.11 | 2.93 | 5.90 | 5.59 | -4.11 | 4.76 | -4.11 | -4.11 | 4.40 | -4.11 | -4.11 | 7.28 |
| miR-182 | 37 | 8.7 | -1.00 | -1.00 | -1.00 | 11.30 | -1.00 | -1.00 | 3.30 | 2.12 | -1.00 | -1.00 | 4.01 | 7.56 | 30.62 |
| miR-183 | 21 | 10.0 | -1.00 | -1.00 | -1.00 | 8.60 | -1.00 | -1.00 | 1.85 | -1.00 | -1.00 | -1.00 | 3.52 | 2.83 | 25.18 |
| miR-198 | 21 | 12.6 | -4.73 | -4.73 | -4.73 | -2.51 | 1.98 | 21.91 | 7.44 | -1.47 | 19.01 | 1.51 | 1.52 | 1.17 | -4.73 |
| miR-19a | 47 | 5.0 | 1.80 | -1.76 | 7.21 | 8.91 | 6.26 | -1.04 | 1.14 | 1.68 | 4.45 | -1.76 | 3.38 | 2.05 | -1.76 |
| miR-30c-1* | 26 | 11.2 | -2.57 | -8.25 | -3.89 | 1.91 | 1.94 | 22.43 | 8.60 | -1.36 | 21.06 | 1.72 | 1.31 | -1.01 | -8.25 |
| miR-375 | 32 | 16.2 | -1.60 | -1.60 | -1.60 | -1.60 | -1.60 | -1.60 | -1.60 | 1.32 | -1.60 | -1.60 | 10.44 | 12.39 | 58.80 |
| miR-376c | 16 | 62.7 | -1.12 | -1.12 | -1.12 | -1.12 | -1.12 | -1.12 | -1.12 | 2.50 | -1.12 | -1.12 | 15.41 | -1.12 | 170.06 |
| miR-429 | 16 | 8.1 | -1.00 | -1.00 | -1.00 | -1.00 | -1.00 | -1.00 | -1.00 | -1.00 | -1.00 | -1.00 | 3.48 | 1.76 | 17.65 |
| miR-483-5p | 42 | 10.1 | 1.50 | -1.21 | -3.38 | 1.14 | 2.56 | 9.82 | 7.11 | 1.75 | 52.02 | 2.12 | 1.94 | 1.66 | -3.38 |
| miR-516a-5p | 47 | 8.8 | 6.97 | -1.65 | -1.65 | 1.17 | -1.65 | 9.91 | 4.22 | 2.90 | 39.38 | 5.18 | 4.21 | 3.86 | -1.65 |
| miR-7 | 21 | 86.3 | -1.00 | -1.00 | -1.00 | 4.57 | -1.00 | -1.00 | -1.00 | -1.00 | -1.00 | -1.00 | 11.46 | 7.18 | 321.94 |

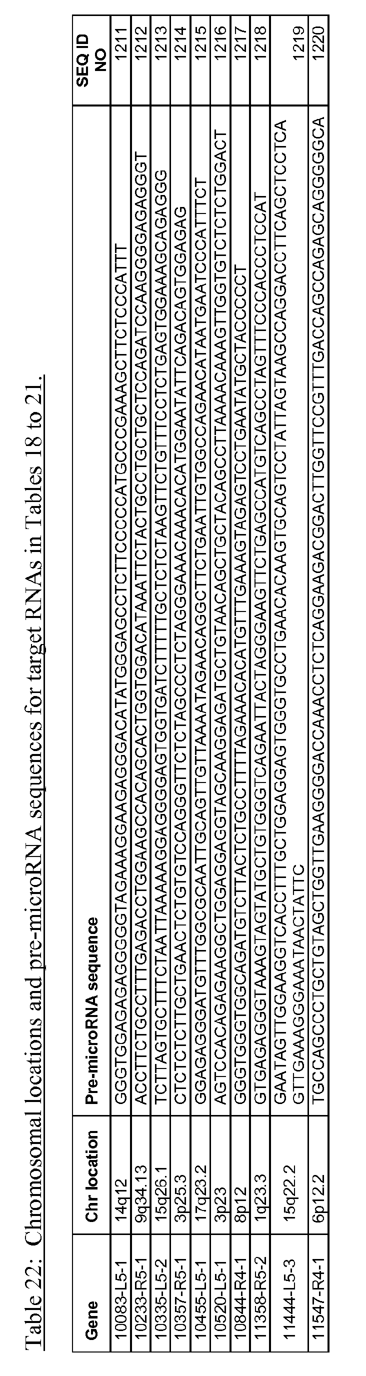

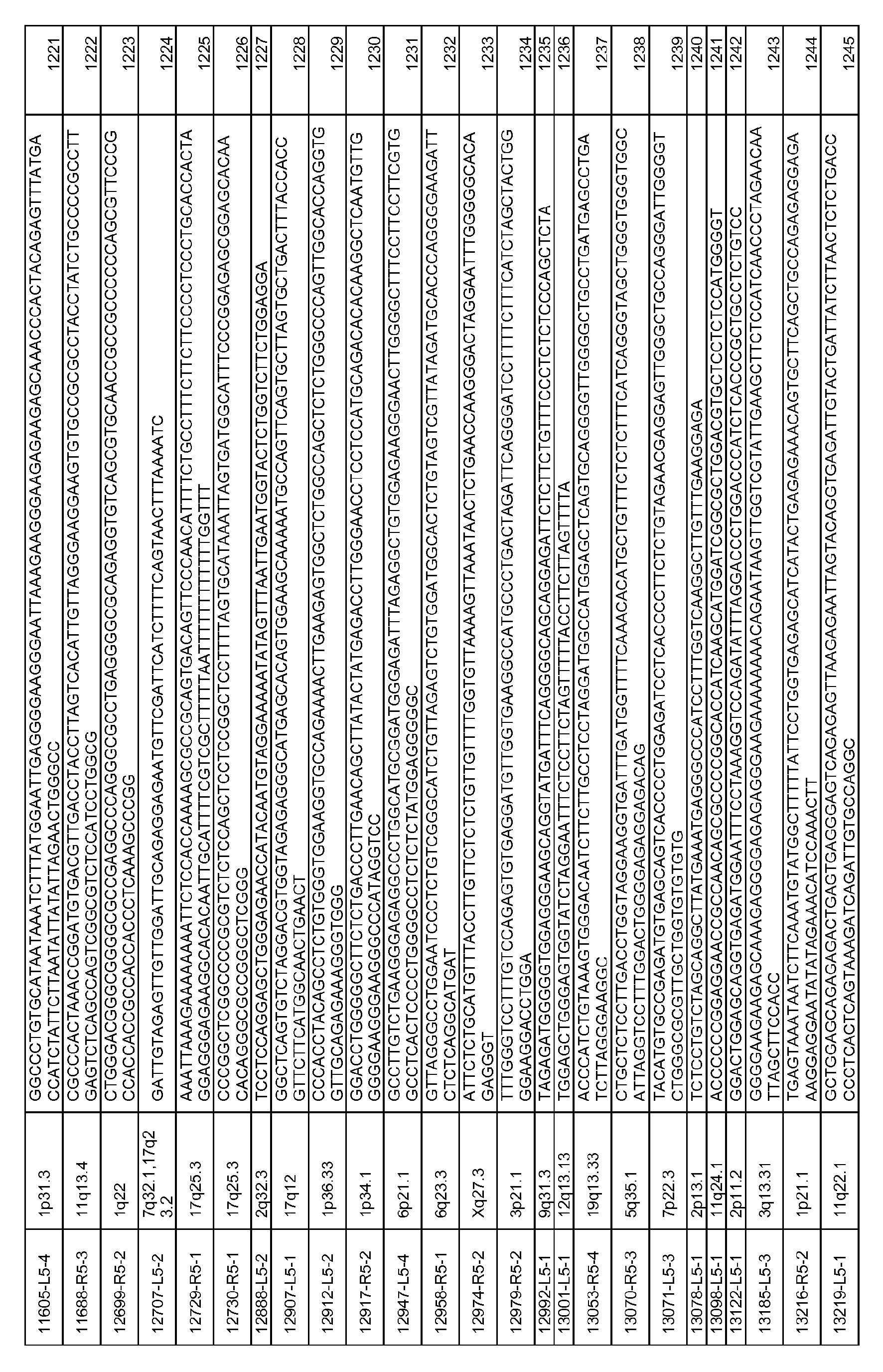

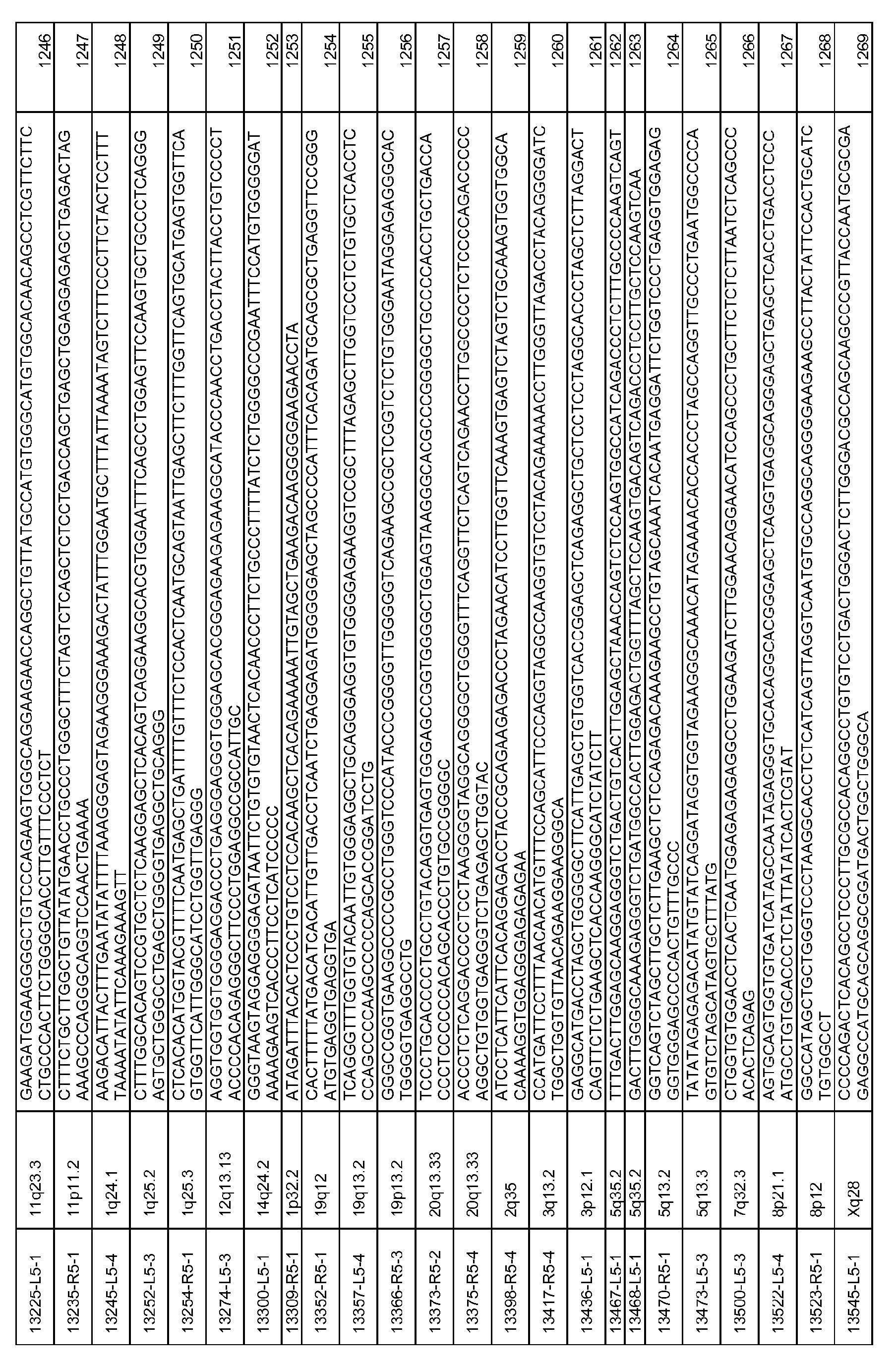

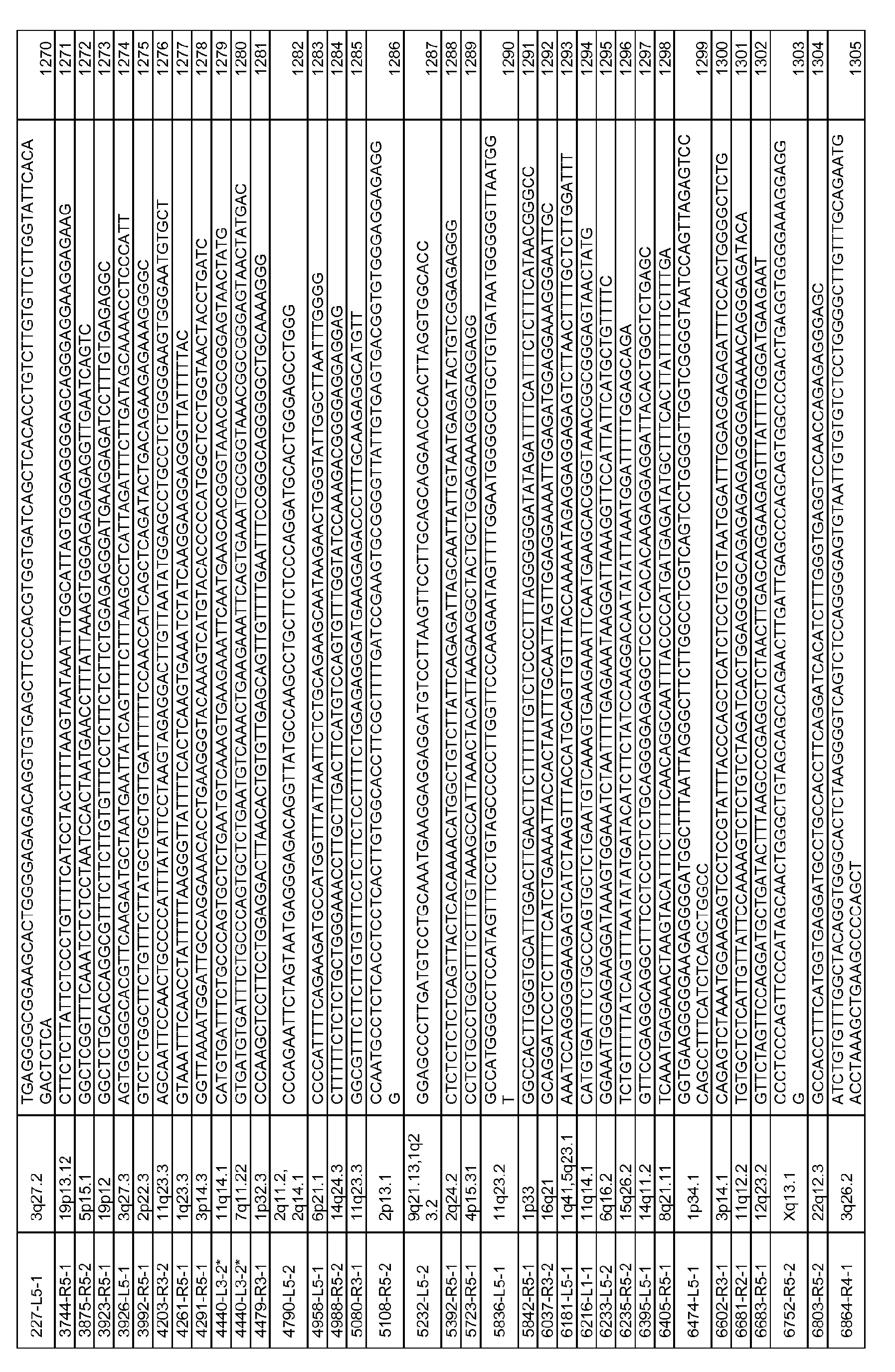

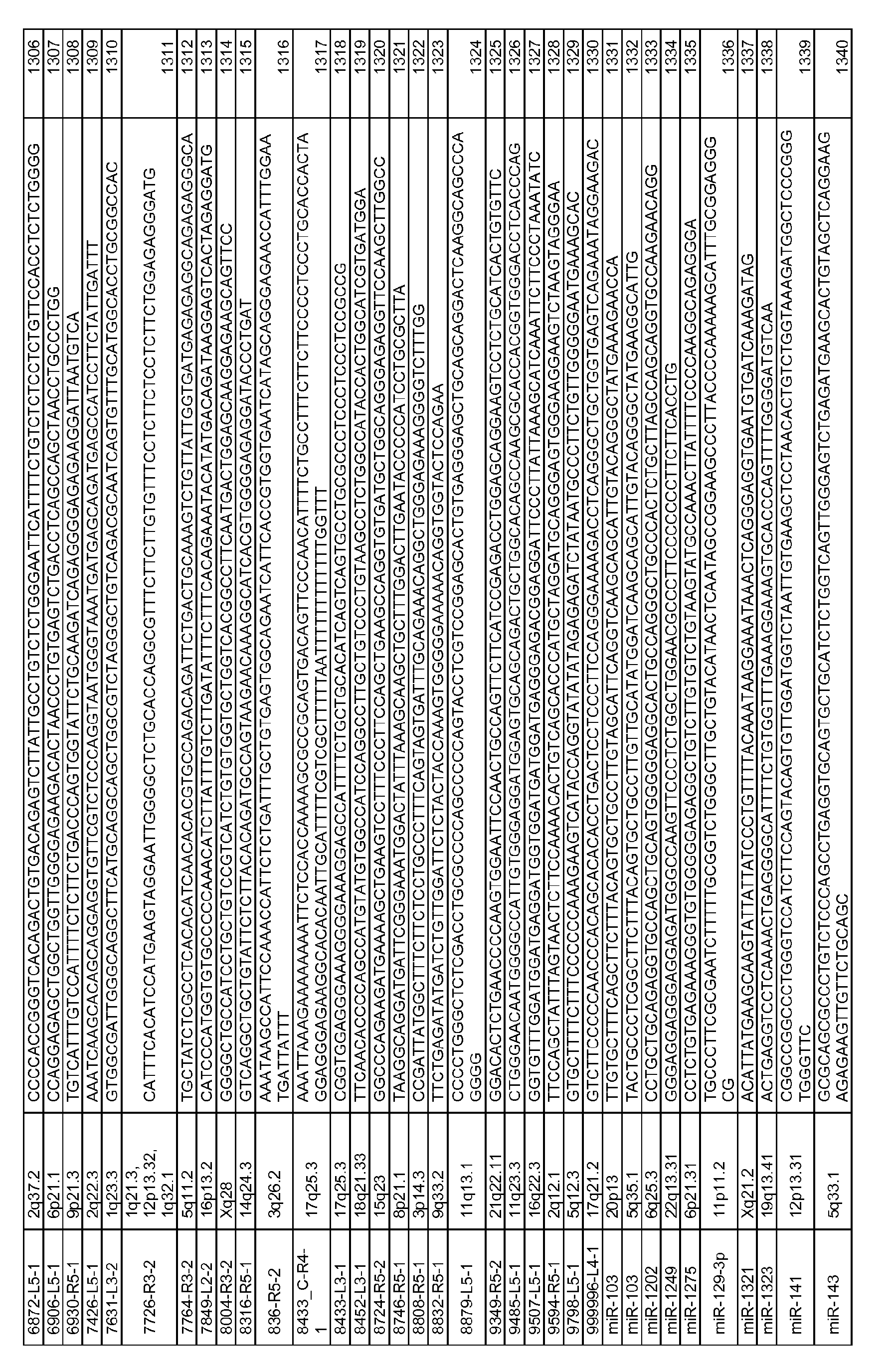

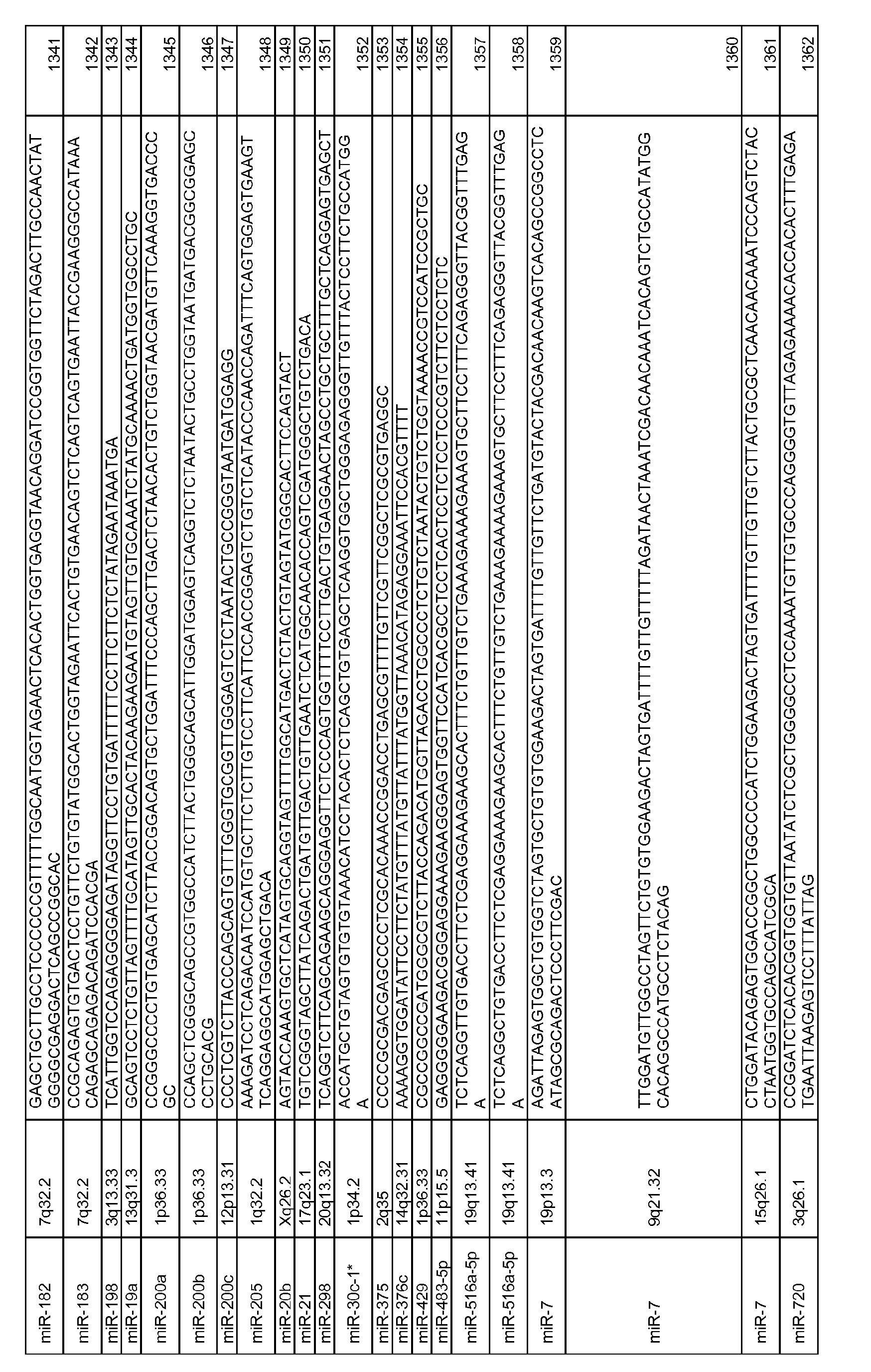

**Table 23: Target RNAs present at at least 5-fold decreased levels in at least 50% of tumor samples**

| **Gene** | **Probe sequence** | **SEQ ID NO** | **% of down-expressed** | **ADK9** | **ADK10** | **Adk29** | **Adk15** | **Adk23** | **ADK48** |
|---|---|---|---|---|---|---|---|---|---|
| 10010 B-L4-1 | GCCGAGCCCCCGCCCCCGCCGGGATGCTGCCCTCCGGAAGGAGG GGCGCTGCCC | 1363 | 67 | -4.54 | -4.10 | -6.89 | -5.28 | -3.68 | -2.54 |
| 10010 D-L4-1 | TGGCGCCCTCCCCCGCCCGGGGCTCAGCCTCTCACCTG | 1364 | 61 | -4.69 | -6.39 | -10.13 | -3.34 | -2.75 | -1.75 |
| 10010-R2-2 | CTCGCCGGCTCCAAACTTTCCCCAACTCCAGG | 1365 | 67 | -10.92 | -10.92 | -7.27 | -7.12 | -4.44 | -2.95 |
| 10030-R5-1 | CCGTGGATGTCAACTCAGCTGCCTTCCGCC | 1366 | 67 | -6.81 | -6.81 | -6.81 | -2.13 | -3.82 | -2.13 |
| 10145-L5-2 | TCAAAAAATTCTTTTTCCTTTGCTCTCTCTTCTGT | 1367 | 56 | -2.03 | -2.03 | -2.03 | -2.03 | -1.18 | -2.03 |
| 10231-R3-1 | TGAACTTTAGCTGGGCCGCCGCCTGTCAGC | 1368 | 72 | -6.47 | -4.83 | -7.60 | -4.34 | -5.39 | -1.70 |
| 10260-L5-2 | ACCTATTGTTCGCCAGGGCCCCCACCCGATGT | 1369 | 67 | -13.31 | -13.31 | -3.91 | -3.96 | -6.75 | -4.01 |
| 10333-L5-1 | TGCCCTGCCCACCCCCTCCCCTGCCCCG | 1370 | 78 | -5.25 | -5.14 | -8.87 | -5.02 | -3.91 | -2.93 |
| 10342-R2-2 | CCCGCCGCCGGAGCATCTCGAAGTTAATTAAA | 1371 | 56 | -4.63 | -3.69 | -4.92 | -3.66 | -4.90 | -1.74 |
| 10345-R5-1 | ACCCTTCTCTCAAAAGCGACCCCTCCCATCCAGTCC | 1372 | 72 | -4.22 | -4.59 | -10.15 | -5.45 | -4.10 | -2.11 |
| 10374-R3-2 | GACACCGCCCGCTACTTTGTTAATGAAAAGCCCCC | 1373 | 67 | -33.82 | -4.71 | -10.79 | -4.92 | -11.40 | -1.26 |
| 10435-R5-1 | TGCTAATTGTGCCCTGTTGTCTTTCTTAAACT | 1374 | 56 | -2.03 | -2.03 | -2.03 | 1.07 | -1.10 | -2.03 |
| 10533-R5-2 | TAACTGTCCTGAGCCCCTTCTTTATATTGC | 1375 | 56 | -2.60 | -2.60 | -2.60 | -1.21 | -1.46 | -2.60 |
| 10543-R5-2 | GCCTTCACCCTTCCCATCCTGGCTCATGAAT | 1376 | 56 | -2.16 | -2.16 | -2.16 | -2.16 | -1.24 | -2.16 |
| 10578-R5-1 | CCACCCCCTCTACGGTCCCCACCAGCCCCG | 1377 | 78 | -5.22 | -4.46 | -6.46 | -4.96 | -4.51 | -1.76 |
| 10818-L5-1 | CTCAGTGATGACAAATGACCGCTGTCAGCCGCC | 1378 | 83 | -21.17 | -21.17 | -8.42 | -4.40 | -9.89 | -1.24 |
| 11370-L5-5 | CCCTCCGCCCCCACACTGCATCCTTGCCCAGTTTGG | 1379 | 78 | -6.80 | -4.03 | -7.05 | -3.30 | -2.15 | -2.30 |
| 11605-L5-4 | TCCCTTCTTTAATTCCCTTCCCCTCAATTCCATAA | 1380 | 61 | -2.54 | -2.54 | -2.54 | -2.54 | -1.37 | -2.54 |
| 12184-L4-1 | GACCTCAGCGTGCCCCCTTTCAACCACAGACGAATATTGTGTACAA | 1381 | 56 | -2.73 | -2.21 | -3.89 | -3.08 | -1.99 | 1.08 |
| 12184-L5-3 | TGACCTCAGCGTGCCCCCTTTCAACCACAGACG | 1382 | 61 | -5.57 | -5.57 | -5.57 | -5.57 | -2.34 | -1.75 |
| 12224-L4-1 | CAAGGCGTCGCCATGAAAATCCACCCCATGGAGTAGCA | 1383 | 78 | -3.22 | -3.22 | -3.22 | -3.22 | -2.54 | -3.22 |
| 12361-R5-1 | ATGTAAATGTGCCATATTGCCGCCCATCT | 1384 | 67 | -3.34 | -4.62 | -6.42 | -3.65 | -3.60 | -1.50 |
| 12691-R5-1 | CCCCGCCCCTGGCGCGCCCCCGACAGGC | 1385 | 78 | -67.73 | -7.29 | -9.93 | -7.32 | -3.57 | -2.81 |
| 12692-L5-1 | GACCCGGCCCCGCAGCCAGCACCCGGCCACCGCGC | 1386 | 89 | -7.80 | -7.12 | -6.21 | -7.94 | -5.87 | -2.09 |
| 12693-L5-1 | GTCGCGGCCGCCCGGCCCTCCCGGTCCCCTCCCC | 1387 | 61 | -84.98 | -6.22 | -8.38 | -2.30 | -2.98 | -1.86 |
| 12694-R5-1 | TCAGCCCCCAGCGCCCCCCGGAGTTCTTGGA | 1388 | 78 | -6.01 | -5.88 | -9.37 | -6.86 | -6.32 | -2.41 |
| 12696-R5-2 | AACCCGGGCTCCCCCACCCGCTCCCTGA | 1389 | 61 | -4.33 | -3.38 | -7.14 | -5.25 | -4.53 | -1.41 |
| 12697-R5-1 | CCGGTGTGCGCCCCCTCCTACCTCTGCCGGCC | 1390 | 67 | -4.28 | -5.08 | -27.65 | -4.83 | -3.72 | -1.76 |
| 12699-L5-1 | GGCGCCCTGGGCCTCGGCGCCCCGCCCGTCCCAG | 1391 | 50 | -4.46 | -29.74 | -4.16 | -3.65 | -2.69 | -1.18 |
| 12701-L5-1 | AAATCCTCGCCATCCTCCACCCCCAGCCCCGG | 1392 | 78 | -4.43 | -12.74 | -7.02 | -6.42 | -4.98 | -1.97 |
| 12703-L5-3 | AGCCGAGCCCCCGCCCCCGCCGGGATGCTGCCCTC | 1393 | 61 | -4.17 | -3.97 | -5.50 | -4.94 | -3.05 | -1.64 |
| 12704-L5-2 | CTGGCGCCCTCCCCCGCCCGGGGCTCAGCCTC | 1394 | 72 | -4.44 | -5.86 | -10.21 | -3.67 | -2.76 | -2.08 |
| 12713-R5-1 | CTCTCGCGACCGACCTGCCGCCGACCGCCACAG | 1395 | 83 | -7.39 | -6.52 | -7.89 | -5.40 | -7.13 | -1.82 |
| 12722-L5-1 | AAACAAAGTACTTCCGACCTCCCCGCCCGCCCGC | 1396 | 56 | -4.21 | -4.38 | -5.37 | -2.81 | -2.86 | -2.02 |
| 12723-R5-2 | CCCCAGCCCCTTCCTGGCCAGGACCCCAGCGAGG | 1397 | 72 | -18.13 | -18.13 | -18.13 | -3.62 | -3.52 | -4.66 |
| 12725-R5-1 | CCACCCCCTCAAGCCCCCAGGAGCTTCCTTAAC | 1398 | 67 | -22.87 | -22.87 | -22.87 | -4.18 | -9.94 | -1.79 |
| 12731-L5-1 | CAGCCGCGCCGGCCGCCAGCGCCCCGGCGCGC | 1399 | 72 | -14.83 | -14.83 | -14.83 | -4.58 | -3.87 | 1.02 |
| 12900-R5-3 | GTGCCCGTGCCCTAAGCGACCCTCCACTCCCTCAA | 1400 | 56 | -8.25 | -1.80 | -8.25 | -1.90 | -3.08 | -2.33 |
| 12904-R5-2 | CTGCCTCCCTGACCCCAAAACACACCTGACTGCC | 1401 | 61 | -2.67 | -4.87 | -4.22 | -2.80 | -2.26 | -9.55 |
| 12910-R5-2 | GCTCCATTTCCCCACTGAAATGACAAAATCCA | 1402 | 61 | -4.61 | -4.61 | -4.61 | -4.61 | -4.61 | -1.30 |
| 12925-L5-3 | GAACACCTCCTCCTTTTCCCTTTGGTGAATAAAGA | 1403 | 61 | -2.02 | -2.02 | -2.02 | -2.02 | -1.68 | -2.02 |
| 12932-L5-3 | GACTCCCTTCCACCCCCATTTCCCACCTACTAAT | 1404 | 67 | -8.22 | -8.22 | -8.22 | -3.88 | -4.15 | -8.22 |
| 12939-L5-2 | CTGATTCAAAACCCACCTCTACAACTTAATAGCT | 1405 | 72 | -2.19 | -2.19 | -2.19 | -2.19 | -2.19 | -2.19 |
| 12947-R5-3 | GCCCCCTCCATAGAGAGAGGCCCCAGGGGAGTGA | 1406 | 72 | -28.53 | -28.53 | -28.53 | -5.22 | -4.41 | -2.39 |
| 12975-L5-1 | GGAGTCTACAACATCCCTCCCGCCT | 1407 | 50 | -2.29 | -3.08 | -4.02 | -2.31 | -2.47 | -1.53 |
| 12981-L5-1 | AATGATGTCACCTTGGTCTGGTTGCCCCCACCC | 1408 | 61 | -4.08 | -4.08 | -4.08 | -4.08 | -2.35 | -1.27 |
| 12981-R5-1 | GGGTGAAGACAGACAGAACCCAGACCCTCCCATAG | 1409 | 50 | -2.22 | -9.73 | -9.73 | -2.36 | -3.84 | 1.96 |
| 12998-R5-1 | CAGGCCCCAGTGCCACCGCCAAGGCTATC | 1410 | 89 | -12.85 | -12.85 | -12.85 | -12.85 | -10.71 | -3.87 |
| 13004-R5-1 | CTCCAACCCCCGCAATTCTCGCTCCCTTCACCTGA | 1411 | 56 | -2.79 | -4.11 | -6.82 | -4.51 | -3.96 | -2.17 |
| 13047-R5-2 | GCACAGCAGACCCCATGCACTAGCCCCGGGCAC | 1412 | 50 | -2.69 | -2.69 | -2.69 | 1.15 | -1.55 | 1.18 |
| 13050-R5-4 | ACGCACCCCTATCTCCCACCCCCCGCAAGCCAAGCA | 1413 | 78 | -4.94 | -6.60 | -11.56 | -5.96 | -5.41 | -2.49 |
| 13052-L5-1 | TCCCCGGACCTAAGCATCTCCCCCACCCGCCAACC | 1414 | 61 | -2.01 | -3.27 | -4.41 | -3.51 | -3.73 | -1.83 |
| 13066-R5-2 | GGAGGTAGCCCCCAAATCTAGTTGAACCAATTC | 1415 | 61 | -2.42 | -2.42 | -2.42 | -2.42 | -2.42 | -2.42 |
| 13072-L5-2 | GGATTTCACCAAACGTATAGCCCCCACCAGTA | 1416 | 78 | -6.78 | -30.88 | -30.88 | -7.56 | -5.70 | -2.19 |
| 13075-L5-1 | TGAAAGCTGAAGTCCAGCCCAGCCCTCT | 1417 | 56 | -6.29 | -6.29 | -6.29 | -2.93 | -2.98 | -2.05 |
| 13089-L5-2 | ATCACCAACAACCTTGCCCCACTCCAAGCCCTG | 1418 | 61 | -2.76 | -2.76 | -2.76 | -2.76 | -2.23 | -2.76 |
| 13091-L5-2 | CCTGAGAACCCCCGACCCTCAAGTGTTCAGCAGGC | 1419 | 78 | -3.59 | -8.53 | -11.47 | -12.31 | -8.16 | -1.70 |
| 13093-L5-2 | CGACCCCGCAGAACCCCACCGCGCCCCGCGCAG | 1420 | 67 | -4.46 | -10.00 | -7.28 | -7.97 | -7.96 | -1.06 |
| 13095-R5-1 | GCAGGACCCACCTCCCTACTCCTGGCCCAGGT | 1421 | 67 | -10.16 | -10.16 | -4.31 | -2.54 | -5.00 | -2.96 |
| 13097-L5-2 | AGCCTCAGCCCCACCTCCAGCCCCACCCTAGGG | 1422 | 56 | -3.53 | -3.87 | -4.76 | -4.17 | -3.23 | -1.41 |
| 13110-R5-1 | AACAGCAGACAGGGTCCCAGGGCCCTCCCTTGT | 1423 | 56 | -4.98 | -4.98 | -4.98 | -1.26 | -2.35 | -1.54 |
| 13115-L5-3 | ACCCCCTCCAGGGCCGACCACCCCAACCCAAAC | 1424 | 78 | -8.12 | -18.66 | -32.34 | -7.11 | -5.65 | -2.64 |
| 13119-R5-2 | GACTCTGCCGCTCCCGCCCGGCCACCTCCCTGT | 1425 | 61 | -3.93 | -3.70 | -4.56 | -3.71 | -3.93 | -1.95 |
| 13124-L5-1 | TCCTCCCCTCCGCGAAAGCCTAAACTTACCCCTCA | 1426 | 50 | -2.05 | -2.84 | -5.18 | -3.12 | -2.71 | -1.10 |
| 13129-L5-3 | AGCCTTCCTGTCCCCTGGCCCCCGACCTGCTCCA | 1427 | 78 | -2.06 | -2.16 | -2.55 | -2.91 | -3.95 | -6.35 |
| 13130-L5-1 | AGCCCGCCCCAACCCACCTCGATCTTTTCCTC | 1428 | 56 | -2.86 | -5.27 | -3.54 | -2.26 | -1.75 | -2.84 |
| 13135-R5-1 | CGAGGAGGCCCTGAGCACTGCCCACCCCCACACC | 1429 | 72 | -4.99 | -4.99 | -6.04 | -4.00 | -7.63 | -1.74 |
| 13136-L5-3 | ACACTTGCCCCTGCCAGCCCTGGTCAGGGCCACCCT | 1430 | 78 | -6.86 | -6.86 | -6.86 | -6.86 | -2.82 | -2.13 |
| 13137-L5-1 | GCTCTAACCCCCGCAACCCCACCTCCCCATGCC | 1431 | 61 | -2.47 | -3.81 | -5.83 | -3.96 | -3.31 | -1.66 |
| 13138-R5-1 | TTCGCCACGCCCCGCCACCCGAGCTGCCTCCC | 1432 | 78 | -5.42 | -5.75 | -7.42 | -6.65 | -4.73 | -2.51 |
| 13163-R5-1 | AGACCAAAGCCCTCCCACCCCTTCCCCTCCCAGC | 1433 | 72 | -5.88 | -16.87 | -21.10 | -2.25 | -2.25 | -2.13 |
| 13164-L5-1 | GAAAACAAACATCTAATAAACTCTCAGGATACC | 1434 | 50 | -5.10 | -5.10 | -5.10 | -1.16 | -2.10 | -1.52 |
| 13166-L5-1 | TCAGAGGACCCCCCGACCCACCCCGCGAGGC | 1435 | 83 | -6.08 | -6.39 | -9.71 | -13.71 | -5.78 | -2.21 |
| 13181-L5-1 | GAACAGTCACTGCCTGCCCCAACCTCTTTCAGGA | 1436 | 67 | -7.92 | -7.92 | -4.10 | -1.01 | -3.99 | -7.92 |
| 13184-L5-1 | CAAAAAATAAACCAAAAACCCACCACCTAATG | 1437 | 56 | -2.31 | -2.31 | -2.31 | -1.65 | -2.31 | -2.31 |
| 13186-R5-2 | ACATTCATCTCTGTCAGATCCCTCCCTCACCA | 1438 | 67 | -4.00 | -9.93 | -26.53 | -3.44 | -2.79 | -1.55 |
| 13195-L5-3 | TCTATATTCCCCCTCCATGACCATTTGTATTAG | 1439 | 78 | -3.32 | -11.15 | -8.93 | -3.70 | -2.85 | -5.82 |
| 13199-R5-1 | GCTGGGGGATTCTGCCCATGCTTCACCTCCC | 1440 | 50 | -2.48 | -8.02 | -5.59 | -2.40 | -6.24 | 1.53 |
| 13202-L5-1 | GGGTCTCCTGCCACCTCCTCTGAGAAGCCCCACCA | 1441 | 61 | -10.20 | -10.20 | -10.20 | -4.67 | -4.70 | -3.14 |
| 13202-R5-2 | GGTGGGGAGACTGGGAGAGAGCCCTCCTAATG | 1442 | 56 | -3.89 | -14.39 | -14.39 | -2.07 | -2.32 | -1.06 |
| 13209-L5-2 | TAACTCGCCTGCTGCCCCGGCGGCCTGCCCGCCG | 1443 | 61 | -45.23 | -4.90 | -5.49 | -3.79 | -3.36 | -1.44 |
| 13209-R5-3 | TGGTCGCCGCCGCAGGCGCCTGAAGGGCACGGCGG | 1444 | 61 | -12.46 | -12.46 | -3.05 | -3.57 | -6.27 | 1.13 |
| 13211-L5-1 | ACGCGCCCCGCCGCTCTCTGACCGACCGGAGGCGC | 1445 | 61 | -7.25 | -6.22 | -5.47 | -5.00 | -4.38 | -1.43 |
| 13220-L5-3 | CCTGTAGCTGCCACTGCCCCTTCCTCACTCAACC | 1446 | 72 | -5.92 | -19.89 | -9.41 | -4.26 | -3.98 | -1.61 |
| 13229-L5-1 | ACCCGTCCCTGCCCCTTTACCCCTTGGGCCAGCA | 1447 | 61 | -5.10 | -4.62 | -6.00 | -4.16 | -3.60 | -1.59 |
| 13230-L5-4 | TAGCTCCAGTGCCTCCAGCTCCAACCACCTGAA | 1448 | 50 | -2.05 | -2.05 | -2.05 | -2.05 | -1.39 | -2.05 |
| 13231-L5-2 | GGGGCCGCTCCCCAGCACCGACGCCAGCATCATCG | 1449 | 56 | -16.99 | -16.99 | -3.88 | -4.81 | -8.33 | 1.00 |
| 13237-L5-4 | ACCCCCTCCAGGGCCGACCACCCCAACCCAAACAA | 1450 | 72 | -35.36 | -16.82 | -35.36 | -6.88 | -5.65 | -2.41 |
| 13239-L5-2 | CAGAGCTCCCCCCATCTCCCCAGACTTACCCCT | 1451 | 72 | -4.10 | -15.67 | -39.77 | -6.16 | -5.51 | -1.58 |
| 13240-L5-2 | AATCGCCGTCCCCGCCGCGGCATTCCCGGCCCCAA | 1452 | 72 | -60.67 | -6.31 | -7.99 | -5.64 | -4.93 | -1.84 |
| 13241-L5-2 | ACACCCCTCCAAAACCACACAGAGCAAGCAAGG | 1453 | 72 | -10.26 | -10.26 | -10.26 | -3.74 | -4.35 | -10.26 |
| 13251-R5-2 | TTGTGCCCCCTCCTCTGCCATGGCTGGTCCCTGG | 1454 | 78 | -5.70 | -7.57 | -14.60 | -5.42 | -4.16 | -2.77 |
| 13259-R5-1 | GCCAGAATTACCACTGTATCTGTCCCCACCCC | 1455 | 72 | -3.29 | -3.29 | -3.29 | -3.29 | -2.05 | -3.29 |
| 13267-L5-1 | CACTCCCTGCTGGCCCCCACCTCACCTATGGTG | 1456 | 61 | -1.87 | -2.26 | -13.85 | -2.21 | -3.68 | 1.35 |
| 13281-L5-3 | CACCCCCACCCCACAGGACAGAGGAAGTGACGAG | 1457 | 56 | -3.70 | -9.33 | -5.75 | -7.17 | -4.05 | -6.04 |
| 13283-L5-3 | GGACCCCTGCCTTCCTTGCTGCCACCCTTTGCACA | 1458 | 50 | -3.60 | -7.29 | -3.33 | -3.32 | -2.19 | -1.11 |
| 13285-L5-3 | GCTATGCACCCAGCCGCCCAGCTCAGCCCCTGC | 1459 | 78 | -6.84 | -6.59 | -7.59 | -4.39 | -4.35 | -1.93 |
| 13287-L5-3 | GGAGCCACCCCACCCTCCTCCCAAGACCCACAT | 1460 | 56 | -3.34 | -3.34 | -4.88 | -3.81 | -2.82 | -2.04 |
| 13291-L5-1 | CCCAAGCGCCCCTTCCTCCCTCCTTCCCTCCCG | 1461 | 50 | -2.07 | -2.35 | -3.92 | -2.62 | -2.12 | -1.10 |
| 13293-L5-1 | TGCCTGGCCAGGCCTCCGCCCCTTGGCTCGCCAC | 1462 | 61 | -3.68 | -3.42 | -6.95 | -3.41 | -3.34 | -1.42 |
| 13298-R5-1 | CAGGGCTTCAGCCTCCCCACAGCCCCACACTT | 1463 | 78 | -14.82 | -14.82 | -14.82 | -8.93 | -8.41 | -4.71 |
| 13303-L5-3 | CCTCCCCTGAACCCAGTTGCCACAACTTTCCAC | 1464 | 50 | -2.57 | -7.48 | -4.39 | -2.24 | -5.82 | -1.05 |
| 13308-L5-1 | CACTCACATCAGCCCATCCACCTCCACCTCTCCAC | 1465 | 50 | -14.43 | -3.76 | -3.43 | -2.63 | -5.96 | -3.88 |
| 13310-R5-1 | ATCCATTGCCACTACCACCACCAATAATTAAAA | 1466 | 56 | -6.22 | -6.22 | -6.22 | -6.22 | -4.23 | -6.22 |
| 13312-L5-2 | TGCCACCCCACCCCTCCCCCACAGCCCAGCCC | 1467 | 72 | -3.89 | -4.71 | -7.40 | -5.54 | -3.94 | -2.64 |
| 13313-L5-2 | CCTGTCTCCCATGCCGTGTCCCTCCCACTAACC | 1468 | 61 | -3.49 | -4.79 | -7.64 | -2.95 | -2.68 | -1.55 |
| 13316-R5-2 | CCACCACCTTGCTGCTGGCCCACAGCACCAGGCC | 1469 | 50 | -4.30 | -4.30 | -2.50 | -2.88 | -4.30 | -1.25 |
| 13326-L5-2 | CAGAGATTCCGGCTTCCCCCCACCCGCCCTTC | 1470 | 67 | -4.02 | -4.30 | -8.40 | -5.02 | -4.30 | -2.41 |
| 13328-R5-2 | TCACCTGCCCCCACATCTGCAACACACAAGAGT | 1471 | 72 | -14.53 | -14.53 | -14.53 | -4.04 | -7.65 | -4.27 |
| 13332-L5-1 | CCATACTCTCCAGCTGTCTTCCCTCCCAA | 1472 | 50 | -4.39 | -4.39 | -3.09 | -1.46 | -2.15 | -1.38 |
| 13334-L5-3 | TCCTGGCAGGACTCCCCTCCCCTCCCACTGTG | 1473 | 72 | -3.05 | -5.00 | -8.54 | -3.73 | -3.16 | -2.05 |
| 13335-L5-3 | ACCTCAGCCTCCACTGCCCTCCTGCCGCATCCTAT | 1474 | 61 | -4.66 | -4.99 | -9.25 | -3.74 | -2.81 | -2.08 |
| 13337-L5-2 | TCTTTTTCTGACATTCCCTCCCCCAACATGGAA | 1475 | 67 | -3.58 | -4.94 | -25.53 | -3.71 | -3.28 | -1.08 |
| 13339-L5-1 | GACTGAGGGTTTAAAGAAGATGGTGTCCGCCGC | 1476 | 78 | -45.98 | -6.02 | -7.34 | -3.40 | -4.61 | -1.22 |
| 13343-L5-1 | CCTCCACCCCTCCCGCAGCGCCCCTCCCCCTCA | 1477 | 50 | -3.99 | -5.54 | -7.36 | -3.68 | -3.31 | -1.90 |
| 13349-L5-2 | CACCACCTGTGTGGGCTATCCCAGCCGCCTCC | 1478 | 83 | -7.08 | -7.21 | -6.98 | -4.08 | -4.09 | -1.92 |
| 13353-L5-2 | GCTTCGGCCACAGAAATGTTCGCCCTCTGAAATC | 1479 | 50 | -2.24 | -2.24 | -2.24 | -1.67 | -1.24 | -2.24 |
| 13354-L5-1 | CTGCCCCACAAAGCTCCTGGAACCCCCTCAGT | 1480 | 72 | -17.63 | -17.63 | -17.63 | -4.43 | -9.91 | -1.86 |
| 13355-L5-2 | TGACCACACCCACCCCTATCCTTTCCTGCAGTGTG | 1481 | 78 | -5.51 | -5.51 | -5.51 | -5.51 | -3.48 | -5.51 |
| 13356-L5-2 | GGCCCCTGGCCTCCCTGCCACCCAGCACGGTG | 1482 | 56 | -8.37 | -4.34 | -4.14 | -2.41 | -4.28 | 1.04 |
| 13358-L5-2 | CCTCCTCACCACCCCCTCCACACTCCTGGGGAAGT | 1483 | 78 | -23.01 | -23.01 | -23.01 | -5.08 | -7.50 | -6.25 |
| 13361-L5-1 | GGGAAGCAGAGTCAGTGACCCCAGCCCTGCACAC | 1484 | 83 | -12.93 | -12.93 | -12.93 | -9.37 | -12.93 | -3.94 |
| 13363-L5-2 | GGCCCCCACCGTCACCTGCTGACACCCTCACATCC | 1485 | 83 | -20.98 | -20.98 | -20.98 | -5.06 | -8.92 | -6.01 |
| 13364-L5-2 | TCCCAGGACCCTTCCTGAGCCTCAGCCCATT | 1486 | 78 | -7.47 | -7.47 | -7.47 | -7.47 | -7.47 | -7.47 |
| 13365-L5-3 | TCCCCAGAGCCCGCCCCAACCCACCTCGATCTTTT | 1487 | 56 | -3.16 | -5.55 | -6.40 | -2.46 | -1.71 | -2.63 |
| 13370-L5-2 | CATGCCCCACCCTCACCTCTGCTCCACATACT | 1488 | 50 | -3.37 | -4.07 | -4.87 | -4.29 | -3.66 | -2.25 |
| 13373-L5-4 | GCCCTTACTCCAGCCCCACCGGCTCCCACTCACCT | 1489 | 56 | -7.98 | -7.98 | -3.68 | -2.29 | -4.40 | -1.07 |
| 13374-R5-1 | AGCCTTCCTGTCCCCTGGCCCCCGACCTGC | 1490 | 78 | -5.54 | -4.92 | -5.77 | -5.78 | -4.29 | -2.09 |
| 13375-L5-3 | GAGAACCTGAAACCCCAGCCCCTGCCTACCCCTTAG | 1491 | 72 | -4.07 | -4.48 | -7.27 | -6.24 | -3.83 | -1.92 |
| 13376-R5-1 | TCTGCTGCAGGTAGTCTGAATGTCCCCCCAACATC | 1492 | 78 | -4.80 | -21.42 | -28.03 | -11.86 | -5.27 | -3.27 |
| 13380-R5-3 | AACTCCCAGCCCCGGAAGAATGCACACGCAGGAG | 1493 | 61 | -10.07 | -10.07 | -10.07 | -3.28 | -5.31 | 1.13 |
| 13385-L5-1 | AATGACTCCTCCGGCGCCACCTACAGT | 1494 | 50 | -3.98 | -3.98 | -3.98 | -1.32 | -1.86 | -3.98 |
| 13396-L5-2 | GACCTGCCCCGCCCCACTCGGGCTCCTTACCG | 1495 | 78 | -4.91 | -4.88 | -2.43 | -5.34 | -3.01 | -2.26 |
| 13403-L5-1 | TCCCCCCAACCTGGGGCCAGGCCCACCTGGT | 1496 | 67 | -2.41 | -11.03 | -7.77 | -2.10 | -2.32 | -1.72 |
| 13412-L5-1 | AGTCAAGATGGCGCCCCCTGGTCCCAG | 1497 | 61 | -2.15 | -8.32 | -5.84 | -5.72 | -4.18 | -2.68 |
| 13423-L5-3 | GCCCTCCCCTGACTCCCTGAAGCTATTTGTTT | 1498 | 72 | -5.62 | -16.59 | -20.12 | -2.22 | -2.23 | -1.92 |
| 13425-L5-3 | TGCCCTCCCAGTCATATGCGCCGCATCAAGGTT | 1499 | 72 | -5.55 | -18.57 | -19.05 | -2.11 | -2.19 | -1.90 |
| 13430-L5-3 | TTCTTACCTCCCCCCAACCCCCATCCCAGTTACC | 1500 | 72 | -2.66 | -5.22 | -11.00 | -5.21 | -4.82 | -2.37 |
| 13431-L5-3 | CGACACCCACTCACTGCCGCTGCCGCACTCACAGC | 1501 | 72 | -6.02 | -7.11 | -7.16 | -4.36 | -6.77 | -1.47 |
| 13432-R5-1 | ACCTCCCCAAACTCCACGTAATCACCTACTATT | 1502 | 50 | -2.31 | -4.47 | -3.97 | -1.94 | -3.01 | 1.02 |
| 13456-R5-2 | CAGATGCCCCGCTATGAAATCTTTTCCAACC | 1503 | 50 | -1.49 | -5.78 | -14.18 | -2.24 | -1.86 | 1.34 |
| 13458-R5-2 | CCTCCTCGGCACTCCCTCTACCTCACTGTCCAC | 1504 | 56 | -9.56 | -4.35 | -9.56 | -2.05 | -1.81 | -2.58 |
| 13461-L5-4 | TCCCCAGCCCCGTCCCCACCCCCTAGAGAAAGTGAA | 1505 | 78 | -4.88 | -5.69 | -6.76 | -5.00 | -4.09 | -2.33 |
| 13463-L5-2 | ATCCAGGACTCGGACCAGCCCCCCAGCCTGAG | 1506 | 78 | -5.87 | -22.46 | -12.28 | -4.92 | -5.26 | -2.41 |
| 13489-L5-1 | GGGGGAAAGCCTGTGGTCAAGCCAAGGAACAAGA | 1507 | 61 | -2.32 | -2.32 | -2.32 | -1.56 | -2.32 | -2.32 |
| 13497-L5-1 | TGTGCCCCCCTCGCTCCCAGCCCCCAGGGGACCGC | 1508 | 72 | -6.04 | -9.24 | -13.05 | -5.73 | -5.31 | -2.89 |
| 13513-L5-1 | ATCCTCCCTGGGTGGTGCTACTCTCACCAAAG | 1509 | 50 | -3.48 | -3.48 | 1.08 | -3.48 | -3.48 | -3.48 |
| 13519-L5-1 | ACCTTAGCTCTACCCAACCTCGCTTCCCACCCCC | 1510 | 72 | -8.34 | -8.34 | -8.34 | -6.17 | -4.60 | -2.83 |
| 13525-L5-2 | CCATGCTCTCGCGTGATCTCCCCTACCGCCATCGT | 1511 | 78 | -3.26 | -11.76 | -11.76 | -3.63 | -4.94 | -3.67 |
| 25-R5-1 | TTCCCAGAGCCTCACCCCCTCTTTTTCTAACC | 1512 | 50 | -2.21 | -2.37 | -8.78 | -2.46 | -2.10 | -1.26 |
| 266-R5-2 | GTCGCCCCCTCCCCCAAGTTGAGACTTGCA | 1513 | 78 | -3.86 | -6.49 | -10.77 | -4.19 | -3.42 | -2.49 |
| 2786-L5-3 | CACCTCCCGCTCAACTGCCCATACTAATGCTTTT | 1514 | 61 | -7.14 | -7.14 | -7.14 | -5.01 | -4.23 | -7.14 |
| 2811-R5-1 | CCACAGCCACCCCGTGCCACTGTGTCCCAACCC | 1515 | 67 | -5.19 | -5.19 | -5.19 | -5.19 | -5.19 | -5.19 |
| 2819-R5-4 | CAGCCTGCCACCGCCGCTTTTGAAAGAAGCACTTCA | 1516 | 78 | -8.91 | -8.06 | -8.93 | -4.59 | -8.36 | -1.88 |
| 3717-L5-2 | CCGCCCTCCCCATAGCCTCACCCCAAACCCA | 1517 | 61 | 161.90 | -6.67 | -8.36 | -1.07 | -3.17 | -1.51 |
| 3732-R5-1 | TCCCTTTCCCTGCCAGCAAACCCCACCACCCTAAG | 1518 | 50 | -13.70 | -5.71 | -2.96 | -2.29 | -2.86 | -1.31 |
| 3799-R5-1 | CTGAAGATGCTCCCAGAGGCCCCCCGCCGGCC | 1519 | 67 | -3.62 | -5.47 | -7.63 | -3.60 | -3.29 | -2.20 |
| 3897-R5-2 | CCGACCCGCCCGTCAGCCGCCTCCCCCTCAG | 1520 | 72 | -6.31 | -6.25 | -5.66 | -5.22 | -5.39 | -1.95 |
| 3942-L5-1 | TCCCTTCACTCCAGTTGCCAAACAGATCCCCCCACTCCC | 1521 | 61 | -2.19 | -6.40 | -7.64 | -1.72 | -2.42 | -2.05 |
| 3952-L3-1 | GAGCACTCAATCTGACACCCCTCGCCGGGG | 1522 | 72 | -7.09 | -7.09 | -7.09 | -2.41 | -4.47 | 1.53 |
| 3953-R3-2 | ACTCCAGCCTCCGCCGCCTCAGCTTCCCGAGC | 1523 | 67 | 155.65 | -4.18 | -6.49 | -3.80 | -5.23 | -1.96 |
| 3966-L5-1 | ACCCCAGAGCTGTCGCCGCCGCTGCCGCCTTCGCC | 1524 | 78 | -8.01 | -6.99 | -8.17 | -5.56 | -7.00 | -1.82 |
| 3995-L2-2 | CTATAAAACTTCGAAAAGTCCCTCCTCCTCACGT | 1525 | 50 | -1.95 | -3.65 | -12.55 | -2.00 | -2.04 | 7.12 |
| 4013-L4-1 | CTATGGCACTTGCATGGTTGAGCTATCAGCAGGGGGACCAT | 1526 | 67 | -2.19 | -2.19 | -2.19 | 1.07 | -2.19 | -2.19 |
| 4026-R5-1 | GAGAGAAAGCCCCCCTTTGTCTGGCTTTG | 1527 | 83 | -81.69 | -81.69 | -18.32 | -7.08 | -6.50 | -3.10 |
| 4026-R5-2 | GGCGAGAGAGAAAGCCCCCCTTTGTCTGGC | 1528 | 72 | -7.22 | -14.47 | -19.24 | -5.56 | -6.21 | -3.11 |
| 410-R5-1 | CCAGCTCCACTACTCCGTCCCCGAGGAAGCAAAACACGGCACC | 1529 | 67 | -5.18 | -5.18 | -5.18 | -5.18 | -3.55 | -5.18 |
| 4130-L5-1 | TCAGCCAGCACGCCGTCCATGTCCACCAGCACCC | 1530 | 78 | -18.42 | -18.42 | -13.52 | -4.27 | -4.12 | -1.61 |
| 4143-R5-2 | TCAGCGTCTTGCTCTCCTCCTGGTAACAGCAGCC | 1531 | 67 | -3.75 | -3.75 | -3.75 | -3.75 | -3.75 | -3.75 |
| 4258-L5-1 | CCTAGAGAACATATATCTGGTGCCTCTCCTCTTTTCCCGT | 1532 | 56 | -2.02 | -2.02 | -2.02 | -1.46 | -1.05 | -2.02 |
| 4315 C-L4-1 | GCAGCCCCTCCTCCGAGAGGTTGGGGGTCGCGGCCGCCCGGCCC TCCCGGTCCCCTCCCC | 1533 | 72 | -3.12 | -84.00 | -7.08 | -2.18 | -2.69 | -2.05 |
| 4315 D-R4-1 | GGAAAGTCAGCCCCCAGCGCCCCCCGGAGTTCTTGG | 1534 | 72 | -6.78 | -6.28 | -7.97 | -6.33 | -6.20 | -2.25 |
| 4315 E-R4-1 | CCCCCACCAAACCTATTCCCGCATCCTCCCCGGCTCTGG | 1535 | 50 | -4.86 | -4.04 | -4.85 | -3.23 | -4.16 | -1.61 |
| 4315 F-R4-1 | AACCCGGGCTCCCCCACCCGCTCCCTGAGC | 1536 | 67 | -4.81 | -4.70 | -9.00 | -5.38 | -5.04 | -2.05 |
| 4315 I-L4-1 | ACACCTCTGCGCCCCTCAGGCGCCCTGGGCCTCGGCGCCCCGCC CGTCCCAG | 1537 | 56 | -3.26 | -4.17 | -7.04 | -2.60 | -2.59 | -1.37 |
| 4315 K-L4-1 | TCCCAGGGGGCCCTGAACTTGTCAAATCCTCGCCATCCTCCACCCC CAGCCCCGG | 1538 | 67 | -5.56 | -4.67 | -6.69 | -5.88 | -4.81 | -1.81 |
| 4315-R3-2 | TCCCCGGCCCTCTCCATTCTCGGCTCCGGAGCA | 1539 | 56 | -3.38 | -3.19 | -12.44 | -3.43 | -6.47 | -1.41 |
| 4338-L5-2 | CCTGGGGGTGGGCGGGAGCTGGCCCCACTGC | 1540 | 50 | -2.07 | -2.07 | -2.07 | -1.43 | -2.07 | 1.59 |
| 4340-R3-1 | ATTTTCCAGCCCCTTGTCCCCAGGCCAAAC | 1541 | 61 | -5.28 | -5.28 | -5.28 | -2.61 | -3.21 | -5.28 |
| 4346-L5-1 | TTCTTCCCCCGCCCTCGCCGCGGCCGCGCACCGG | 1542 | 67 | -3.82 | -4.80 | -7.88 | -4.88 | -3.22 | -2.13 |
| 4361-R5-1 | CTCAGCGTCTCCCTCCCTCATGTGCACATGT | 1543 | 67 | -2.26 | -2.85 | -16.06 | -2.89 | -2.23 | -16.06 |
| 4498-L3-2 | GAGATCCAGACGGCCGTGCGCCTGCTGCTGCCT | 1544 | 56 | -2.36 | -9.82 | -2.18 | -1.93 | -6.91 | 1.37 |
| 4516-L5-1 | TCCATCACCCCCCAGGCTGACTCTGGCTCCTGCCTGGCTCTGCC | 1545 | 72 | -14.82 | -14.82 | -14.82 | -4.16 | -7.74 | -1.36 |
| 454-R5-1 | CCAGCTCCACTACTCCGTCCCCGAGGAGGCCAAACACGGCACC | 1546 | 67 | -2.62 | -2.62 | -2.62 | -2.62 | -2.62 | -2.62 |
| 4593-R5-1 | CTATAGCAGATGACATAACTCCCCCGGCATCAG | 1547 | 50 | -1.18 | -1.22 | -2.44 | -1.60 | -4.72 | -1.09 |
| 4610-R5-1 | CCTCTGGCCCCTGCCTAATTGGCTGC | 1548 | 72 | -7.69 | -7.69 | -7.69 | -7.69 | -4.10 | -7.69 |
| 4610-R5-2 | GCCCTCTGGCCCCTGCCTAATTGGCTG | 1549 | 56 | -3.23 | -3.23 | -3.23 | -3.23 | -1.70 | -3.23 |
| 4642-R3-2 | CAGCGCTCCCCTTCCTTATTTGAGATCTGTGCA | 1550 | 67 | -10.25 | -10.25 | -10.25 | -10.25 | -5.81 | 1.30 |
| 4666-R5-1 | GACTCCCCCCAACACCTGCGGGTGGCAC | 1551 | 78 | -3.26 | -6.47 | -13.10 | -5.57 | -5.25 | -2.30 |
| 4792-L5-2 | AAGCCAGTTACAGCCCCCACTGCCCCCATAAC | 1552 | 61 | -4.51 | -4.61 | -6.34 | -4.01 | -4.00 | -1.49 |
| 4801-L5-2 | GAACTCTGCCTCCTGTTTGCTACAAAAACA | 1553 | 61 | -4.53 | -4.53 | -4.53 | -4.53 | -2.36 | -4.53 |
| 4813-R3-1 | GATTTTACAATCGGGCTGTTAACCCTCCCG | 1554 | 61 | -3.33 | -3.33 | -3.33 | -3.33 | -2.04 | -1.06 |
| 4875-R2-2 | CACAGCCCCTTCCTGTGACTTCACAC | 1555 | 67 | -2.71 | -25.83 | -7.24 | -2.30 | -2.06 | -1.91 |
| 4912-L5-2 | TACCCCTGGCCCCCAGCTGTGATTGTCTAA | 1556 | 78 | -19.46 | -19.46 | -5.35 | -5.07 | -7.98 | -1.65 |
| 4929-R4-1 | GACGCTGACCCACCGCAGCCCGCACTGTT | 1557 | 89 | -11.74 | -11.74 | -11.74 | -8.30 | -7.20 | -11.74 |
| 5032-R5-1 | CGCACCCGTCCCGTTCGTCCCCGGACGT | 1558 | 72 | -13.13 | -13.13 | -13.13 | -8.64 | -6.34 | -13.13 |
| 5048-L5-1 | CGCCGCTCCTTGTACGCGTACTTCTGCTGCACACC | 1559 | 61 | -11.59 | -11.59 | -11.59 | -3.55 | -5.68 | -1.14 |
| 5071-R5-2 | GACCCCCGCCCCAGTCCCAGCCCAATTAATA | 1560 | 72 | -6.98 | -7.41 | -8.60 | -7.82 | -3.38 | -3.14 |
| 5107-L5-1 | AACTCCCCTTTGACCCCCCAGTACAAACTG | 1561 | 61 | -2.41 | -26.66 | -26.66 | -3.80 | -3.15 | 1.08 |
| 5210-L5-1 | AAAGCTGCCCCATGGGGATCCAACCCCA | 1562 | 67 | -1.97 | -7.99 | -7.99 | -3.58 | -4.09 | -7.99 |
| 5342-L5-1 | CCACCAAACCAAATGCCGCTGCTCTCCTTCCA | 1563 | 56 | -4.62 | -3.77 | -3.56 | -3.08 | -2.92 | -1.28 |
| 5491-R5-1 | GCCGTCGCCCACCAGATCACTCATCAGGCCATGGTGGCA | 1564 | 72 | -5.64 | -4.77 | -6.07 | -3.45 | -4.72 | -1.52 |
| 5521-L5-2 | AGGTCTGTCTTGGGTGGGCCCTCCCCAGAGCAC | 1565 | 50 | -7.60 | -7.60 | -7.60 | -2.62 | -1.22 | -2.35 |
| 554-R5-1 | GGAAAGAGAACAGAGAGAGCCCTCCCAGCAGCC | 1566 | 67 | -5.86 | -17.89 | -18.96 | -2.04 | -2.19 | -1.95 |
| 5554-R5-2 | CCCCACCCCCTCATCAGCTGCTCCCAGATC | 1567 | 61 | -3.72 | -3.98 | -5.48 | -4.08 | -2.80 | -1.59 |
| 5638-R5-2 | GGCCCTCCCCCTGCCTGTGATAGGCTG | 1568 | 78 | -5.39 | -15.00 | -20.91 | -2.61 | -2.48 | -2.16 |
| 5640-L3-1 | GCCATGGAACACCGTGCCTGCCCCTCTCGAGA | 1569 | 61 | -2.75 | -4.95 | -5.84 | -2.71 | -2.38 | -1.80 |
| 5749-R5-1 | ATCATAATCCCCCTTTTGACTTTACATGATC | 1570 | 56 | -3.50 | -3.50 | -3.50 | -1.35 | -1.84 | -3.50 |
| 5757-L5-1 | TGCTTTATTGACTAATGAACCTACCGTGCCGCCAAC | 1571 | 89 | -22.78 | -22.78 | -22.78 | -4.38 | -4.58 | -5.43 |
| 5854-R5-2 | GCCCTCCCTCTCCGAAAGAATGTGTCACCCGGG | 1572 | 72 | -4.59 | -14.74 | -16.09 | -1.96 | -1.94 | -2.01 |
| 5956-L5-1 | GCCTGCTCTGCCAACCCCAAATCCGTCAAGACGCATAG | 1573 | 50 | -1.50 | -3.54 | -8.83 | -2.47 | -1.20 | -8.83 |
| 5995-R5-1 | CCCCCTTGAGGTTCCTACTGAAATCTGACAATCAG | 1574 | 78 | -2.29 | -2.29 | -2.29 | -2.29 | -2.29 | -2.29 |
| 6008-R5-1 | ACCCCCACCTTTTTCCTGTACCTTACCCGGAG | 1575 | 83 | -9.71 | -9.71 | -9.71 | -9.71 | -5.88 | -3.27 |
| 6008-R5-2 | CAACAATACCCCCACCTTTTTCCTGTACCTTA | 1576 | 78 | -5.41 | -5.41 | -5.41 | -5.41 | -5.41 | -5.41 |
| 6016-R2-1 | AAACTCCAGCAGCCCCGTCAGCCTCCTGCT | 1577 | 61 | -3.58 | -3.27 | -3.65 | -3.22 | -2.34 | -3.50 |
| 6023-L5-1 | AAGGCAGAGCCAGATGACTGGTCCAACCCCCCAGAGACC | 1578 | 72 | -4.72 | -19.11 | -19.11 | -5.65 | -9.37 | -1.46 |
| 6087-L4-1 | CTGTCTCCATTACTGCCTGCCACCTTCTCCATC | 1579 | 78 | -8.97 | -8.97 | -8.97 | -6.19 | -5.07 | -8.97 |
| 6096-R5-1 | CTTTCCCTTATGTTTTAATCCTGCCCCGT | 1580 | 89 | -12.65 | -12.65 | -12.65 | -2.15 | -6.96 | -12.65 |
| 6192-L5-1 | AATCCGTGAAGATAAAAAACCACCCACCCAGCAC | 1581 | 50 | -13.47 | -13.47 | -2.70 | -1.88 | -6.10 | 1.34 |
| 6198-R5-2 | GCCGCCGCCGCCGCGTCTTCCCGCGAAGCCT | 1582 | 67 | -4.03 | -2.96 | -5.71 | -4.41 | -4.65 | -1.50 |
| 6242-R5-1 | CCCCCACAGTGGCATATGTGACAAACCCAAAGCCCCTGG | 1583 | 67 | -5.90 | -5.90 | -5.90 | -5.90 | -4.69 | -5.90 |
| 6287-L3-2 | GCCCCGCCCCACCTTTCGGGGCTCACCTGGC | 1584 | 72 | -19.21 | -19.21 | -4.59 | -4.31 | -2.70 | -1.67 |
| 6385-R5-2 | GGCCCTGCCCACGGACCGACTCCCGCGGCCC | 1585 | 67 | -9.70 | -9.70 | -9.70 | -9.70 | -5.59 | -3.26 |
| 6409-L3-1 | CGTTCCCAACCGCACGCGCCGCCTTCTGGAAC | 1586 | 72 | -8.06 | -7.09 | -6.76 | -4.68 | -5.18 | -1.54 |
| 6434-R5-1 | TGCAGCCCTCCCACCAGCCAGCTGCAGTGC | 1587 | 67 | -4.42 | -12.82 | -29.88 | -2.13 | -2.32 | -1.24 |
| 6490-R5-3 | CCCCATCCCCCATATGACGCTTCCCCCTCCTAAC | 1588 | 67 | -3.39 | -4.20 | -23.58 | -4.26 | -6.83 | -1.35 |
| 6496-R5-2 | CCCCCTCCCCCACCCACCACTTCCCCTAGA | 1589 | 67 | -44.85 | -14.21 | -9.47 | -4.89 | -3.70 | -2.49 |
| 6584-L5-1 | GTCGGCCCTGCCTCCTCCTCCTCTCACCAAGC | 1590 | 56 | -13.67 | -5.50 | -13.67 | -2.42 | -2.29 | 1.17 |
| 6590-L5-1 | GGTTAATGAGAAACCAAATAAACAGCCTCTGCCCCAGCTG | 1591 | 56 | -4.65 | -4.65 | -4.65 | -4.65 | -2.56 | -4.65 |
| 6642-R5-1 | GTCCTCCCCTCCCCTCGAGGTGTCACACA | 1592 | 56 | -4.01 | -5.51 | -6.59 | -3.85 | -2.99 | -1.63 |
| 669-R5-2 | CCAGCCCTGAGCCACCCTCCCGGGAAACCCCACA | 1593 | 67 | -10.57 | -10.57 | -10.57 | -1.47 | -6.18 | -2.07 |
| 6718-L3-2 | GCCTCCACCACCATAGGGGCCAGAGCTTCTGCCT | 1594 | 72 | -3.98 | -3.98 | -3.98 | -3.98 | -3.30 | -3.98 |
| 6839-L3-1 | GCCCGCTGGGCCCTGCCACCCCCACCCCT | 1595 | 72 | -5.32 | -4.50 | -6.48 | -4.99 | -4.55 | -2.17 |
| 6880-L3-2 | ACCTCCCCCGCGAAGACATCCACATTCTGCA | 1596 | 61 | -2.19 | -4.09 | -5.43 | -2.08 | -4.56 | -1.52 |
| 6908-L3-2 | AGAGGCTACTGGGGGAACAAGACTGGCAAGCC | 1597 | 50 | -2.84 | -2.84 | -2.84 | -1.73 | -2.84 | 4.40 |
| 6984-R4-1 | CCCCCTGCCCAAGCATTTGCTTGGGCACCAAAGTCCCTGCAA | 1598 | 67 | -3.05 | -18.59 | -18.59 | -4.32 | -7.50 | -1.51 |
| 7029-R5-1 | CAAGAGCCCTGCCCCAGCAGCAGCCGCAC | 1599 | 67 | -10.64 | -10.64 | -3.52 | -2.90 | -2.27 | -3.48 |
| 7061-R5-2 | TCATGGAAACCCCACCCTTCCCATGCCCAACC | 1600 | 56 | -3.93 | -4.19 | -5.53 | -4.35 | -3.52 | -2.45 |
| 7066-R5-1 | TAGCCTGAAAAAAGATGCCCCCACCAGCCCTGCC | 1601 | 78 | -6.87 | -6.68 | -11.13 | -7.24 | -11.42 | -2.31 |
| 7069-R5-1 | CTCGGGCCCGGCCCCCTCCGAGCTCAACAGGCTCCCA | 1602 | 78 | -6.84 | -7.03 | -9.92 | -5.66 | -4.77 | -2.63 |
| 7113-R5-1 | CGCCTAATTAGCCCCCCTGCTCCGGAGGCCTCACC | 1603 | 78 | -10.08 | -16.70 | -18.94 | -7.28 | -6.96 | -3.32 |
| 7126-L3-1 | GCACACCCGCTCTCCGGCCCGCGCCCCTG | 1604 | 61 | -3.58 | -3.60 | -3.88 | -3.21 | -4.09 | -1.48 |
| 7141-R5-1 | ACCCCAATCCTTGTTATGTAACCTACCACCTACCCCTT | 1605 | 72 | -2.31 | -2.31 | -2.31 | -2.31 | -2.31 | -2.31 |
| 7221-R5-1 | GGAGAGAAACCCCGGCCACTTCCCACCACCCTGGTGGC | 1606 | 56 | -2.57 | -2.97 | -3.51 | -3.96 | -6.16 | -1.10 |
| 7313-L5-2 | CTGCCTCTGCCCTGATCATCAAAGCCCTCAAGGA | 1607 | 61 | -2.26 | -2.26 | -2.26 | -2.26 | -1.58 | -2.26 |
| 7352-R3-2 | GCCCCTGCCAGAATCCTCTAACAGCTCTAATTGG | 1608 | 50 | -2.04 | -2.07 | -2.22 | -2.46 | -1.45 | -3.40 |
| 7356A-R4-1 | CAGAGCCCGCTCTCGCGACCGACCTGCCGCCGACCGCCACAG | 1609 | 67 | -7.83 | -6.39 | -7.11 | -4.76 | -6.17 | -1.56 |
| 7356-L5-1 | ATCCGGGCTGCCACCGCGACATAGCCTCGCCCCC | 1610 | 56 | -3.91 | -3.91 | -6.19 | -3.02 | -2.48 | -1.08 |
| 7367-L1-1 | AGGGTTAGAGCTGCCCCCTCTGGGGACCG | 1611 | 50 | -5.61 | -1.12 | -5.61 | -1.54 | -2.24 | -5.61 |
| 7384-R3-1 | CTCGCAAAGGATCTCCTTCATCCCTCCCCA | 1612 | 61 | -3.15 | -7.30 | -4.15 | -3.12 | -2.40 | -1.29 |
| 7411-R3-2 | AGTCCCCTGCCTCATCTGCCACCCCTAATGAC | 1613 | 50 | -2.55 | -2.66 | -13.01 | -2.77 | -1.83 | -3.86 |
| 7569-L5-2 | TTCAGGCCACAAAGCTACCCCCAAGACAG | 1614 | 67 | -3.93 | -3.93 | -3.93 | -3.93 | -3.93 | 2.42 |
| 7571-L5-1 | CAGGGCTAACAGGGCTCCCCCACCCCTAAG | 1615 | 56 | -3.75 | -4.46 | -6.71 | -5.25 | -4.30 | -1.32 |
| 7572-R5-2 | ATCACCCTTCCCCCTCCCAAATAAAGCCAAA | 1616 | 50 | -22.49 | -4.07 | -6.89 | -3.47 | -2.12 | -1.57 |
| 7660-L5-1 | GCCTCCGCCTGGCCCGAGCGATAAAGCTC | 1617 | 56 | -3.55 | -7.83 | -4.16 | -3.75 | -3.08 | -1.29 |
| 7702-L2-1 | CCCAGAGAACCGGAATTCCTCCCCGCCCC | 1618 | 78 | -6.38 | -6.47 | -8.59 | -5.58 | -4.12 | -2.55 |
| 7736-L5-1 | ATCCCCACCACCCACCAGAAGGCGACGGTCTCC | 1619 | 50 | -10.35 | -10.35 | -2.65 | -4.09 | -5.59 | -2.98 |
| 7743-L5-1 | GAGCTCCCCAGCCAGCTCCAGTTCGACCTGCCTT | 1620 | 67 | -7.13 | -7.13 | -7.13 | -7.13 | -5.90 | -7.13 |
| 7781-R5-2 | AGCCTGTGCCTGCCGCTGTCTAGTACTGGT | 1621 | 72 | -22.71 | -12.29 | -6.84 | -3.46 | -3.67 | -1.44 |
| 7824-R5-1 | TGCCAGCTTCATCGCCGCCTCACACACACA | 1622 | 72 | -10.99 | -8.91 | -14.45 | -5.40 | -5.99 | 4.65 |
| 7846-L5-2 | GTCCTCCTCCATCCCATCCCTTCCACCAGCCCT | 1623 | 50 | -2.66 | -2.66 | -2.66 | -2.66 | -1.45 | -2.66 |
| 7883-R5-1 | CTCTCCCCTCCCGCCCTCTCACCCTCCCAACCTTATTTAGAAAC | 1624 | 50 | -3.69 | -4.72 | -7.94 | -3.56 | -2.66 | -2.03 |
| 78-R4-1 | CCAGAGCTTCTCAAGGGGGACTACAACTCCCAAGGTACATACAA | 1625 | 50 | -2.39 | -2.39 | -2.39 | 1.35 | -2.39 | 1.44 |
| 7949-R5-1 | GATGCGCGCGCCGACCGCCGCCAGCTGCAATTCATAC | 1626 | 72 | -4.04 | -3.23 | -5.42 | -3.02 | -5.45 | -1.05 |
| 7971-L5-1 | TGTCACTCCCCTGCCGCTCCCTGGGTGCAGGCT | 1627 | 61 | -7.45 | -7.45 | -7.45 | -3.26 | -5.59 | -2.17 |
| 8016-L3-1 | TCAGCGCAACAAGCCCCGCAGTCACCCCTCT | 1628 | 50 | -3.02 | -3.60 | -2.40 | -2.98 | -2.70 | 1.10 |
| 8062-R5-1 | GGGCAAAGGTCACGGGGTCCAAGGCCTTAAGCACCTCCGCCA | 1629 | 50 | -7.61 | -7.61 | -2.06 | -1.46 | -1.16 | -2.34 |
| 8077-R3-1 | CCATTCCCCACCCTCAGGTAGTAAAAATA | 1630 | 67 | -2.08 | -10.66 | -3.95 | -3.94 | -2.28 | -10.66 |
| 8089-L5-1 | TAGAGACCCTTATAAGCTCAGGGCCACCCCCTCCC | 1631 | 78 | -12.58 | -12.58 | -12.58 | -4.10 | -6.46 | -4.03 |
| 8239-R5-1 | TGATTTTCTTCCCACTTCACCTCCCTCTGAGCTCTCCA | 1632 | 56 | -7.85 | -7.85 | -7.85 | -2.34 | -5.84 | -2.52 |
| 8250-R5-2 | CCGACCCGCCCGTCAGCCGCCTCTCCCTCAG | 1633 | 72 | -8.36 | -6.52 | -12.10 | -5.21 | -5.60 | -2.31 |
| 8281-L5-2 | CAGCCCCTCCCCAGCTGCAGCTGAGGGC | 1634 | 61 | -2.82 | -7.08 | -8.59 | -2.10 | -2.55 | -1.83 |
| 8298-R5-1 | GATGCTGGCGTCCGCCGCAGCCTCTCGCCCCATCCCGG | 1635 | 72 | -7.60 | -6.38 | -5.79 | -4.53 | -4.42 | -1.51 |
| 8329-L5-1 | TCCTTCCAAACGCCCACCCTGGGTCAGCTC | 1636 | 72 | -7.14 | -7.14 | -7.14 | -4.59 | -3.30 | -7.14 |
| 8336-R5-2 | CCTCCCCACTCAGTCCCCACACCCCCAGCCA | 1637 | 56 | -3.54 | -3.08 | -4.97 | -3.00 | -3.92 | -1.33 |
| 8394-L5-2 | TGGGGCCCCCGCCCTGCCCATCTCCGACTATCC | 1638 | 50 | -3.26 | -3.15 | -4.42 | -4.40 | -2.96 | -1.58 |
| 8564-L5-1 | ACCCCAGTTGCCAAACAGACCTCCCACCCCCT | 1639 | 72 | -9.90 | -9.90 | -9.90 | -2.63 | -4.92 | -3.12 |
| 8564-R5-2 | AGGGGCTGGGGGAATCCCAGCAGGGGAA | 1640 | 50 | -6.82 | -6.82 | -6.82 | -1.12 | -3.67 | 3.00 |
| 8898-R5-1 | CAGCCGAGGCGGACGCCCGCTCCCGCCACCATG | 1641 | 72 | -5.76 | -6.58 | -7.08 | -4.99 | -4.72 | -2.23 |
| 9021-L5-2 | GAAACAAACACCCAAGCTCCCCACACCA | 1642 | 50 | -9.46 | -9.46 | -9.46 | -4.34 | -5.86 | 1.27 |
| 9068-R5-2 | GTCTGCCCTCCCTCTTGATCAAGACTGCTCT | 1643 | 67 | -6.33 | -16.55 | -18.60 | -2.35 | -2.21 | -1.89 |
| 9087-L5-2 | AGGAAAAGAAACCCTCCCAGTCCATTCCCT | 1644 | 72 | -6.46 | -10.39 | -14.13 | -5.66 | -5.34 | -1.11 |
| 9134-R5-1 | GAAAGGGTTATCCGCCTGGTTGCGGGGCTGC | 1645 | 50 | -2.19 | -2.19 | -2.19 | -1.49 | -2.19 | 1.49 |
| 9217-L3-2 | CGCTAAACTGACGATCCCCGCCGTGACTAAAGCCA | 1646 | 72 | -4.02 | -10.70 | -3.72 | -4.17 | -2.98 | -1.46 |
| 9245-R5-1 | TCTCATTAGCCAGCCACTCGCTCCCAAG | 1647 | 67 | -7.70 | -7.70 | -7.70 | -5.35 | -4.29 | -7.70 |
| 9287-L5-2 | GATATTCAGAGCCCTCCCCAGCCCACACA | 1648 | 67 | -4.67 | -9.40 | -29.04 | -1.28 | -2.93 | -1.16 |
| 9369-L5-1 | TCACACATTCTTCCACAGAGGGAAATCAGGGGA | 1649 | 50 | -2.53 | -2.53 | -2.53 | 1.38 | -2.53 | 1.22 |
| 9384-R5-2 | AAACCAGCTAGCAAACCGCTCCGTCCGTATTG | 1650 | 78 | -15.00 | -15.00 | -15.00 | -15.00 | -7.95 | -4.59 |
| 9387-R2-2 | TCCATCCTTGCCGTCGCCTTCATCTCAAAGCCATC | 1651 | 67 | -7.04 | -4.60 | -3.03 | -4.14 | -4.99 | 3.25 |
| 9564-R5-2 | GCCGCCCGCCGGGCACCGGGCCGGGCCTGGGC | 1652 | 50 | -3.25 | -3.61 | -4.38 | -3.31 | -2.97 | -1.18 |
| 9605-R5-1 | GCCCCCAAGTGAAAAACAGAGCAAAACTCATTTCCTGA | 1653 | 50 | -2.11 | -2.11 | -2.11 | -1.42 | -2.11 | -2.11 |
| 9691-L5-1 | CATTTCATCCGCATCTCCCTCTTGGCCCCTTGC | 1654 | 56 | -3.33 | -6.89 | -4.05 | -5.66 | -2.65 | -4.01 |
| 9770-R5-2 | CACCTGTTGCCAACCCCAGCCCTATAA | 1655 | 67 | -16.08 | -16.08 | -16.08 | -3.95 | -7.31 | -1.78 |
| 9774-R2-2 | CCGCCCCCTCACCGCCTCCTGCTCCCATCAGGC | 1656 | 83 | -7.02 | -6.70 | -13.75 | -8.18 | -5.97 | -2.86 |
| 9812-L3-1 | AAGCTCTATTTATCTGGGCTCCCCAGCTTGCT | 1657 | 56 | -1.83 | -8.36 | -8.36 | -2.32 | -2.02 | -2.57 |
| 9866-L5-1 | TAGGAGAGGGGCTCCATTGCCAGCCCCAGCCC | 1658 | 94 | -11.22 | -11.22 | -11.22 | -11.22 | -5.31 | -3.72 |
| 999997-R4-1 | TCCTCACTGGGCCCCACCAAAACTGTGCCACCCCCTCAAGCCCCCA GGAGCTTCCTTAAC | 1659 | 72 | -4.04 | -4.22 | -40.37 | -2.33 | -5.94 | -1.62 |
| let-7b | TGAGGTAGTAGGTTGTGTGGTT | 1660 | 56 | -1.48 | -6.50 | -2.74 | -2.48 | -2.61 | -1.23 |
| let-7c | TGAGGTAGTAGGTTGTATGGTT | 1661 | 50 | -1.39 | -5.08 | -2.46 | -2.14 | -2.43 | -1.26 |
| let-7e | TGAGGTAGGAGGTTGTATAGTT | 1662 | 50 | -1.27 | -4.56 | -2.29 | -2.10 | -2.25 | -1.06 |
| miR-100 | AACCCGTAGATCCGAACTTGTG | 1663 | 50 | -1.16 | -15.70 | -1.03 | -3.27 | -2.27 | -15.70 |
| miR-101 | TACAGTACTGTGATAACTGAA | 1664 | 50 | -1.54 | -12.44 | -3.65 | -2.51 | -5.38 | 1.25 |
| miR-1182 | GAGGGTCTTGGGAGGGATGTGAC | 1665 | 50 | -2.49 | -6.37 | -2.91 | -2.61 | -4.75 | 1.04 |
| miR-1207-5p | TGGCAGGGAGGCTGGGAGGGG | 1666 | 50 | -2.88 | -9.91 | -5.91 | -3.63 | -2.81 | -1.28 |
| miR-1224-5p | GTGAGGACTCGGGAGGTGG | 1667 | 61 | -8.27 | -8.27 | -8.27 | -2.05 | -4.54 | -8.27 |
| miR-1225-5p | GTGGGTACGGCCCAGTGGGGGG | 1668 | 72 | -32.61 | -32.61 | -32.61 | -32.61 | -14.04 | -2.24 |
| miR-1228* | GTGGGCGGGGGCAGGTGTGTG | 1669 | 67 | -3.86 | -3.61 | -6.14 | -5.78 | -2.67 | -2.95 |
| miR-1234 | TCGGCCTGACCACCCACCCCAC | 1670 | 61 | -2.04 | -2.04 | -2.04 | -1.47 | -2.04 | 1.67 |
| miR-125a-5p | TCCCTGAGACCCTTTAACCTGTGA | 1671 | 72 | -3.80 | -7.98 | -2.23 | -5.05 | -2.99 | -2.20 |
| miR-126 | TCGTACCGTGAGTAATAATGCG | 1672 | 83 | -6.57 | -7.43 | -7.29 | -5.33 | -20.83 | -2.68 |
| miR-1268 | CGGGCGTGGTGGTGGGGG | 1673 | 56 | -10.67 | -10.67 | -10.67 | -5.01 | -5.42 | -3.17 |
| miR-130b | CAGTGCAATGATGAAAGGGCAT | 1674 | 50 | 1.68 | -2.21 | -2.21 | 1.20 | -1.89 | -2.21 |
| miR-140-3p | TACCACAGGGTAGAACCACGG | 1675 | 67 | -4.44 | -4.44 | -4.44 | -2.32 | -2.82 | -4.44 |
| miR-145 | GTCCAGTTTTCCCAGGAATCCCT | 1676 | 61 | -5.21 | -7.10 | -4.29 | -4.12 | -3.15 | -1.88 |
| miR-149* | AGGGAGGGACGGGGGCTGTGC | 1677 | 56 | -3.51 | -2.89 | -6.31 | -3.80 | -2.13 | -2.15 |
| miR-150 | TCTCCCAACCCTTGTACCAGTG | 1678 | 61 | -3.14 | -3.14 | -3.14 | -3.14 | -3.14 | 1.10 |
| miR-181b | AACATTCATTGCTGTCGGTGGGT | 1679 | 50 | -2.73 | -2.73 | -2.73 | 2.75 | -1.22 | -2.73 |
| miR-181d | AACATTCATTGTTGTCGGTGGGT | 1680 | 56 | -2.47 | -2.47 | -2.47 | -1.78 | -1.37 | -2.47 |
| miR-185* | AGGGGCTGGCTTTCCTCTGGTC | 1681 | 78 | -10.19 | -10.19 | -10.19 | -2.49 | -2.10 | 2.05 |
| miR-214 | ACAGCAGGCACAGACAGGCAGT | 1682 | 56 | -4.43 | -4.43 | -4.43 | -3.17 | -2.21 | -1.30 |
| miR-23a* | GGGGTTCCTGGGGATGGGATTT | 1683 | 78 | -3.92 | -3.92 | -3.92 | -3.92 | -2.50 | -3.92 |
| miR-30a | TGTAAACATCCTCGACTGGAAG | 1684 | 56 | -19.76 | 1.09 | -4.20 | -1.92 | -2.01 | 1.15 |
| miR-30d | TGTAAACATCCCCGACTGGAAG | 1685 | 50 | 1.47 | 1.77 | -16.33 | 1.08 | -1.97 | 2.70 |
| miR-320a | AAAAGCTGGGTTGAGAGGGCGA | 1686 | 56 | -9.70 | -9.70 | -4.14 | -1.93 | -2.89 | -2.92 |
| miR-320b | AAAAGCTGGGTTGAGAGGGCAA | 1687 | 50 | -2.05 | -5.08 | -6.60 | -1.99 | -3.25 | -3.07 |
| miR-335 | TCAAGAGCAATAACGAAAAATGT | 1688 | 67 | -3.24 | -3.24 | -3.24 | 1.00 | -2.68 | -3.24 |
| miR-34a | TGGCAGTGTCTTAGCTGGTTGT | 1689 | 50 | 3.37 | -2.08 | 1.99 | 2.04 | -1.20 | -2.08 |
| miR-34b* | TAGGCAGTGTCATTAGCTGATTG | 1690 | 67 | 1.46 | -3.29 | -1.58 | 1.09 | -2.68 | -3.29 |
| miR-34c-5p | AGGCAGTGTAGTTAGCTGATTGC | 1691 | 94 | -2.54 | -2.54 | -2.54 | -2.54 | -2.54 | -2.54 |
| miR-371-5p | ACTCAAACTGTGGGGGCACT | 1692 | 56 | -3.87 | -3.87 | -3.87 | -3.87 | -3.87 | -3.87 |
| miR-373* | ACTCAAAATGGGGGCGCTTTCC | 1693 | 72 | -2.95 | -3.88 | -34.48 | -2.43 | -4.31 | 1.09 |
| miR-451 | AAACCGTTACCATTACTGAGTT | 1694 | 94 | -5.28 | -7.77 | -12.78 | -4.68 | -16.47 | 1.61 |
| miR-486-3p | CGGGGCAGCTCAGTACAGGAT | 1695 | 72 | -2.72 | -10.16 | -10.16 | -2.65 | -5.72 | 1.09 |
| miR-491-3p | CTTATGCAAGATTCCCTTCTAC | 1696 | 72 | -2.34 | -2.34 | -2.34 | -2.34 | -1.30 | -2.34 |
| miR-498 | TTTCAAGCCAGGGGGCGTTTTTC | 1697 | 83 | -5.68 | -5.82 | -9.56 | -7.20 | -4.74 | -1.67 |
| miR-557 | GTTTGCACGGGTGGGCCTTGTCT | 1698 | 56 | -2.45 | -5.53 | -10.00 | -2.80 | -1.94 | 1.01 |
| miR-638 | AGGGATCGCGGGCGGGTGGCGGCCT | 1699 | 78 | -9.64 | -8.70 | -11.76 | -7.11 | -6.51 | -2.43 |
| miR-663 | AGGCGGGGCGCCGCGGGACCGC | 1700 | 61 | -15.30 | -15.30 | -5.72 | -3.78 | -7.09 | -3.77 |
| miR-671-5p | AGGAAGCCCTGGAGGGGCTGGAG | 1701 | 56 | -2.58 | -9.36 | -9.36 | -2.04 | -1.63 | -3.08 |
| miR-744 | TGCGGGGCTAGGGCTAACAGCA | 1702 | 67 | -3.86 | -14.84 | -3.85 | -4.06 | -6.80 | -1.26 |
| miR-885-3p | AGGCAGCGGGGTGTAGTGGATA | 1703 | 83 | -7.91 | -26.07 | -17.77 | -5.03 | -5.01 | -1.60 |
| miR-92a-2* | GGGTGGGGATTTGTTGCATTAC | 1704 | 56 | -2.01 | -4.24 | -5.49 | -2.24 | -6.01 | -2.74 |
| miR-92b* | AGGGACGGGACGCGGTGCAGTG | 1705 | 50 | -11.88 | -11.88 | -7.35 | -2.61 | -5.68 | -1.07 |
| miR-98 | TGAGGTAGTAAGTTGTATTGTT | 1706 | 50 | -1.51 | -4.47 | -2.57 | -1.99 | -2.20 | 1.13 |
| miR-99a | AACCCGTAGATCCGATCTTGTG | 1707 | 50 | -1.63 | -10.35 | 1.26 | -4.62 | -4.12 | -3.20 |

**Table 24: Target RNAs present at at least 5-fold decreased levels in at least 50% of tumor samples (con't)**

| **Gene** | **Adk40** | **Adk41** | **Adk49** | **Epi42** | **Ksarc19** | **Kmalp21** | **Kmalp25** | **EPI-4** | **Kmalp44** | **Scc27** | **Lcnec31** | **Car13** |
|---|---|---|---|---|---|---|---|---|---|---|---|---|
| 10010_B-L4-1 | -8.97 | -3.24 | -2.01 | -1.22 | -1.01 | 1.17 | -4.47 | 1.82 | -4.06 | -1.50 | -2.18 | -10.58 |
| 10010_D-L4-1 | -6.36 | -2.67 | 1.05 | 1.34 | 1.42 | -1.02 | -4.33 | 1.72 | -3.11 | -1.85 | -2.76 | -2.21 |
| 10010-R2-2 | -10.92 | -10.92 | -10.92 | -10.92 | 1.41 | 1.26 | -2.35 | 1.55 | -1.11 | -1.47 | -1.43 | -10.92 |
| 10030-R5-1 | -1.67 | -6.81 | -6.81 | -6.81 | 1.05 | -1.33 | -1.91 | 3.13 | -6.81 | -1.68 | -1.42 | -6.81 |
| 10145-L5-2 | -2.03 | -2.03 | -1.03 | -2.03 | 3.99 | 1.36 | 2.04 | 8.70 | -2.03 | 2.51 | 2.49 | -2.03 |
| 10231-R3-1 | -5.36 | -4.79 | -1.71 | -1.02 | -2.00 | -3.34 | -3.16 | -1.40 | -2.97 | -2.84 | -3.01 | -1.72 |
| 10260-L5-2 | -13.31 | -13.31 | -1.39 | -1.00 | -1.36 | -1.37 | -2.68 | -13.31 | -1.73 | -1.67 | -1.91 | -13.31 |
| 10333-L5-1 | -6.06 | -5.42 | -2.91 | -1.23 | 2.12 | 1.59 | -3.76 | 3.00 | -3.64 | -2.40 | -2.70 | -2.39 |
| 10342-R2-2 | -21.66 | -4.09 | -1.44 | -1.12 | -1.73 | -1.64 | -2.11 | 1.05 | -2.44 | -1.91 | -1.99 | -2.14 |
| 10345-R5-1 | -38.49 | -2.89 | -2.15 | -1.16 | 2.43 | -1.11 | -3.15 | 2.42 | -2.93 | -1.81 | -2.89 | -2.05 |
| 10374-R3-2 | -33.82 | -2.75 | -1.86 | 1.01 | -1.26 | -1.38 | -4.17 | 1.55 | -2.29 | -2.84 | -2.81 | -1.19 |
| 10435-R5-1 | -2.03 | -2.03 | -2.03 | -2.03 | 2.64 | 1.90 | 1.56 | 1.79 | -2.03 | 1.98 | 2.99 | -2.03 |
| 10533-R5-2 | -2.60 | -2.60 | -2.60 | 4.07 | 3.00 | 2.42 | -2.60 | 5.87 | -2.60 | 1.93 | 1.78 | -2.60 |
| 10543-R5-2 | -2.16 | -2.16 | -2.16 | -2.16 | 6.71 | 3.38 | 1.75 | 2.31 | 3.45 | 2.43 | 2.22 | -2.16 |
| 10578-R5-1 | -21.24 | -21.24 | -1.92 | -1.12 | 1.03 | -1.44 | -3.78 | -2.98 | -2.45 | -2.36 | -2.85 | -21.24 |
| 10818-L5-1 | -21.17 | -21.17 | -4.88 | 1.05 | -2.48 | -3.84 | -3.65 | -2.42 | -21.17 | -3.59 | -3.58 | 1.31 |
| 11370-L5-5 | -5.19 | -2.41 | -1.94 | 1.06 | 2.73 | 1.42 | -3.48 | 2.73 | -2.57 | -1.29 | -2.09 | -2.21 |
| 11605-L5-4 | -2.54 | -2.54 | -2.54 | -2.54 | 7.43 | 3.53 | 1.60 | 9.69 | -2.54 | 2.07 | 1.86 | -2.54 |
| 12184-L4-1 | -10.83 | -2.87 | -1.07 | 1.72 | 1.44 | 1.16 | -2.02 | 2.42 | -10.83 | -1.60 | -1.80 | -10.83 |
| 12184-L5-3 | -5.57 | -5.57 | -1.45 | 2.35 | 1.86 | -5.57 | -1.13 | -5.57 | -5.57 | -1.18 | -1.11 | -5.57 |
| 12224-L4-1 | -3.22 | -3.22 | -3.22 | -3.22 | 2.60 | -1.30 | -1.44 | -1.08 | -3.22 | -2.02 | -3.22 | -3.22 |
| 12361-R5-1 | -23.46 | -2.62 | -1.61 | 1.02 | -1.78 | -2.13 | -3.81 | 1.16 | -2.37 | -2.85 | -3.15 | -1.06 |
| 12691-R5-1 | -67.73 | -4.80 | -3.65 | -1.52 | -1.27 | -1.61 | -5.82 | 1.11 | -3.99 | -2.62 | -3.78 | -7.28 |
| 12692-L5-1 | -44.23 | -4.49 | -2.70 | -1.69 | -2.39 | -2.52 | -4.11 | -1.77 | -4.02 | -3.45 | -3.57 | -15.48 |
| 12693-L5-1 | -7.71 | -3.35 | -1.78 | 1.31 | 1.05 | -1.00 | -4.98 | 2.24 | -3.49 | -2.55 | -2.90 | -1.64 |
| 12694-R5-1 | -47.87 | -4.06 | -2.68 | -1.06 | -1.04 | -1.38 | -3.70 | 2.01 | -3.35 | -2.83 | -3.35 | -47.87 |
| 12696-R5-2 | -3.88 | -3.83 | -1.63 | -1.52 | 1.70 | 1.08 | -2.08 | 2.73 | -2.20 | -1.93 | -2.65 | -1.28 |
| 12697-R5-1 | -27.65 | -2.65 | -2.38 | -1.00 | 1.40 | 2.28 | -3.45 | 2.22 | -2.90 | -2.10 | -3.18 | -1.41 |
| 12699-L5-1 | -29.74 | -5.73 | -1.65 | 1.05 | 1.25 | 1.02 | -2.61 | 2.61 | -1.59 | -1.65 | -1.88 | -29.74 |
| 12701-L5-1 | -3.53 | -21.63 | -2.75 | -1.73 | 1.00 | -1.43 | -4.30 | 1.93 | -3.70 | -2.98 | -3.18 | -5.50 |
| 12703-L5-3 | -5.66 | -2.77 | -1.61 | -1.29 | -1.04 | 1.10 | -3.49 | 1.99 | -2.38 | -1.31 | -2.16 | -2.12 |
| 12704-L5-2 | -23.11 | -2.88 | -1.96 | 1.02 | 1.23 | -1.13 | -3.66 | 1.95 | -3.25 | -1.73 | -2.39 | -3.04 |
| 12713-R5-1 | -8.29 | -6.75 | -2.32 | -1.25 | -2.08 | -3.21 | -5.05 | -1.31 | -3.88 | -3.28 | -3.59 | -2.05 |
| 12722-L5-1 | -10.47 | -4.23 | -1.94 | -1.16 | 1.08 | 1.06 | -3.29 | 1.73 | -2.48 | -1.90 | -1.51 | -1.61 |
| 12723-R5-2 | -18.13 | -18.13 | -1.57 | 1.12 | -1.08 | 1.31 | -3.01 | 3.13 | -18.13 | -2.21 | -2.34 | -18.13 |
| 12725-R5-1 | -22.87 | -10.61 | -1.79 | 1.85 | 1.00 | -1.07 | -3.16 | 2.59 | -1.96 | -2.48 | -4.02 | -22.87 |
| 12731-L5-1 | -14.83 | -14.83 | -3.85 | 1.07 | -1.66 | -1.01 | -3.96 | -1.24 | -14.83 | -3.53 | -3.07 | -2.48 |
| 12900-R5-3 | -8.25 | -8.25 | -8.25 | -8.25 | 3.50 | 1.81 | -1.46 | 7.77 | 1.59 | 1.53 | 1.11 | -8.25 |
| 12904-R5-2 | -9.55 | -9.55 | -1.99 | 1.39 | 2.59 | 1.24 | -1.19 | 3.91 | -2.18 | -1.08 | -1.26 | -9.55 |
| 12910-R5-2 | -4.61 | -4.61 | -4.61 | -4.61 | 3.61 | 3.13 | -1.85 | 4.45 | -4.61 | -1.46 | -1.18 | -4.61 |
| 12925-L5-3 | -2.02 | -2.02 | -2.02 | -2.02 | 5.09 | 2.63 | 1.23 | 1.81 | -2.02 | 2.16 | 1.67 | -2.02 |
| 12932-L5-3 | -2.89 | -8.22 | -8.22 | 1.29 | 4.62 | 1.65 | -2.23 | 4.21 | -8.22 | -1.56 | -1.84 | -8.22 |
| 12939-L5-2 | -2.19 | -2.19 | -2.19 | -1.06 | 2.02 | 3.10 | -1.04 | -2.19 | -2.19 | 1.30 | -2.19 | -2.19 |
| 12947-R5-3 | -1.37 | -13.13 | -2.75 | 1.15 | 1.49 | -1.39 | -4.20 | 1.51 | -28.53 | -2.45 | -3.99 | -28.53 |
| 12975-L5-1 | -2.38 | -1.84 | -1.17 | 1.10 | 1.87 | -1.01 | -2.24 | 3.23 | -2.39 | -1.76 | -2.51 | -1.73 |
| 12981-L5-1 | -4.08 | -4.08 | -4.08 | 3.51 | 1.97 | 1.56 | -1.29 | -4.08 | -4.08 | -1.06 | -1.09 | -4.08 |
| 12981-R5-1 | -9.73 | 1.26 | 1.11 | 3.25 | 1.24 | -1.83 | -2.07 | 1.61 | -2.36 | -1.79 | -2.49 | -1.21 |
| 12998-R5-1 | -12.85 | -12.85 | -12.85 | -1.18 | -2.37 | -4.23 | -4.62 | -12.85 | -12.85 | -4.10 | -7.18 | 1.18 |
| 13004-R5-1 | -1.80 | -3.58 | -1.64 | -1.52 | 1.04 | -1.21 | -2.31 | 3.54 | -1.93 | -1.18 | -1.69 | -40.23 |
| 13047-R5-2 | -2.69 | -2.69 | -2.69 | -2.69 | 1.26 | 1.69 | 1.72 | 3.11 | -2.69 | 1.66 | 1.70 | -2.69 |
| 13050-R5-4 | -15.79 | -3.48 | -3.24 | -1.11 | 2.03 | -1.04 | -4.41 | 2.22 | -3.65 | -3.00 | -3.58 | -7.48 |
| 13052-L5-1 | -26.92 | -2.48 | -1.98 | -9.42 | 3.17 | 1.94 | -3.50 | 4.57 | -5.44 | -1.79 | -2.31 | 1.31 |
| 13066-R5-2 | -2.42 | -2.42 | -1.04 | 12.45 | 2.07 | -1.35 | 1.13 | 3.64 | -2.42 | 1.57 | -2.42 | -2.42 |
| 13072-L5-2 | -30.88 | -3.39 | -4.96 | 1.35 | -1.14 | -1.69 | -4.40 | 1.45 | -3.40 | -2.95 | -4.79 | -30.88 |
| 13075-L5-1 | -6.29 | -6.29 | -3.13 | 1.76 | 1.50 | 1.06 | -1.59 | 3.33 | 1.36 | -1.09 | -1.21 | -6.29 |
| 13089-L5-2 | -2.76 | -2.76 | -2.76 | 1.48 | 2.89 | 1.67 | -1.18 | 4.44 | -2.76 | 1.25 | -1.25 | -2.76 |
| 13091-L5-2 | -17.46 | -17.46 | -17.46 | -1.31 | -1.66 | -2.34 | -4.61 | 1.68 | -2.00 | -2.90 | -3.09 | -17.46 |
| 13093-L5-2 | -20.22 | -3.83 | -1.31 | 1.02 | -1.19 | -1.65 | -4.19 | 1.23 | -2.08 | -2.08 | -2.36 | -20.22 |
| 13095-R5-1 | -10.16 | -4.78 | -2.40 | 1.37 | 3.83 | -1.01 | -2.18 | 2.72 | -10.16 | -1.67 | -1.88 | -10.16 |
| 13097-L5-2 | -7.34 | -1.77 | -1.58 | 1.04 | 1.73 | -1.12 | -3.12 | 2.57 | -1.98 | -1.76 | -2.44 | -8.77 |
| 13110-R5-1 | -4.98 | -4.98 | -4.98 | 4.37 | 1.73 | 1.02 | -1.82 | 2.20 | -4.98 | -1.44 | -2.75 | -4.98 |
| 13115-L5-3 | -32.34 | -4.43 | -16.43 | -1.11 | 1.45 | -1.55 | -5.01 | 1.38 | -4.43 | -3.15 | -4.39 | -32.34 |
| 13119-R5-2 | -6.15 | -3.15 | -2.12 | -1.61 | -1.06 | -1.15 | -2.81 | 1.90 | -2.31 | -1.75 | -1.75 | -1.55 |
| 13124-L5-1 | -24.48 | -1.59 | -1.32 | 1.76 | 3.40 | 1.31 | -2.81 | 9.22 | -1.36 | -1.46 | -2.20 | -24.48 |
| 13129-L5-3 | -47.98 | -6.80 | -1.76 | -15.40 | -1.17 | 3.16 | -3.45 | 7.03 | -18.45 | -2.26 | -2.30 | -13.71 |
| 13130-L5-1 | -8.62 | -2.94 | -1.75 | 1.88 | 1.49 | 3.62 | -2.87 | 1.74 | -2.79 | -1.89 | -2.85 | -1.53 |
| 13135-R5-1 | -23.43 | -23.43 | -2.06 | 1.34 | 1.63 | -1.41 | -3.42 | 1.76 | -2.53 | -2.77 | -3.89 | -3.94 |
| 13136-L5-3 | -6.86 | -6.86 | -6.86 | -6.86 | 1.35 | -2.00 | -2.05 | 1.67 | -6.86 | 1.09 | -1.16 | -6.86 |
| 13137-L5-1 | -11.97 | -2.58 | -1.60 | 1.04 | 1.90 | 1.04 | -2.52 | 3.20 | -2.24 | -1.54 | -2.07 | -5.87 |
| 13138-R5-1 | -5.15 | -3.33 | -3.23 | -1.50 | -1.38 | -1.56 | -5.50 | 1.32 | -4.04 | -2.61 | -3.13 | -4.48 |
| 13163-R5-1 | -10.02 | -3.08 | -1.87 | 2.52 | 1.68 | -1.32 | -3.55 | 1.41 | -4.70 | -2.94 | -4.38 | -3.99 |
| 13164-L5-1 | -5.10 | -5.10 | -5.10 | 4.48 | 4.67 | 1.58 | -1.49 | 3.96 | -5.10 | -1.16 | -2.30 | -5.10 |
| 13166-L5-1 | -37.14 | -3.17 | -2.98 | -1.24 | -1.26 | -2.36 | -4.64 | 1.43 | -3.21 | -3.95 | -4.48 | -2.43 |
| 13181-L5-1 | -7.92 | -7.92 | -7.92 | 1.65 | 1.40 | 2.36 | -2.28 | 5.58 | -2.57 | -2.04 | -1.63 | -7.92 |
| 13184-L5-1 | -2.31 | -2.31 | -2.31 | 4.42 | 1.67 | -1.23 | 1.05 | 5.81 | -2.31 | 1.90 | -1.17 | -2.31 |
| 13186-R5-2 | -26.53 | -4.05 | -9.42 | -1.04 | 2.59 | 1.05 | -2.83 | 1.85 | -9.17 | -2.40 | -4.24 | 1.98 |
| 13195-L5-3 | -23.57 | -2.07 | -2.02 | 1.48 | 2.78 | 1.09 | -2.90 | 1.89 | -2.32 | -2.10 | -2.13 | -2.45 |
| 13199-R5-1 | -8.02 | -2.26 | 1.36 | 1.62 | 3.08 | 1.12 | -2.13 | 4.07 | -1.08 | -1.86 | -1.57 | -8.02 |
| 13202-L5-1 | 1.16 | -10.20 | 1.03 | 1.38 | 1.62 | -1.35 | -2.12 | 2.03 | -10.20 | -1.77 | -2.12 | -10.20 |
| 13202-R5-2 | -14.39 | -1.40 | 1.14 | 3.25 | 1.30 | -1.53 | -2.85 | 1.55 | -1.85 | -2.70 | -3.38 | -14.39 |
| 13209-L5-2 | -45.23 | -3.07 | -1.52 | 1.00 | -1.08 | -1.54 | -3.29 | 1.39 | -2.21 | -1.88 | -2.01 | -4.56 |
| 13209-R5-3 | -12.46 | -6.49 | 1.10 | 1.57 | -1.68 | -2.00 | -1.94 | 1.42 | -1.36 | -1.64 | -2.04 | -12.46 |
| 13211-L5-1 | -31.07 | -2.60 | -1.64 | -1.17 | -1.32 | -1.51 | -4.08 | 1.30 | -2.71 | -1.84 | -2.39 | -10.90 |
| 13220-L5-3 | -6.86 | -10.00 | -2.35 | -1.04 | -1.08 | -2.44 | -4.02 | 1.22 | -2.77 | -2.46 | -2.77 | 1.00 |
| 13229-L5-1 | -29.36 | -2.91 | -1.85 | -1.07 | 1.36 | 1.32 | -2.81 | 2.19 | -2.21 | -1.91 | -2.31 | -1.02 |
| 13230-L5-4 | -2.05 | -2.05 | -1.17 | -2.05 | 3.16 | 2.18 | 1.59 | 6.65 | -1.14 | 2.50 | -1.02 | -2.05 |
| 13231-L5-2 | -16.99 | -3.66 | -1.24 | -1.09 | -1.39 | -1.43 | -3.76 | 1.62 | -1.61 | -1.25 | -2.31 | -16.99 |
| 13237-L5-4 | -35.36 | -35.36 | -3.55 | -1.11 | 1.31 | -1.52 | -5.02 | 1.37 | -4.63 | -3.23 | -4.11 | -35.36 |
| 13239-L5-2 | -39.77 | -7.37 | -3.11 | -1.12 | 2.53 | 1.22 | -3.59 | 3.98 | -3.01 | -2.21 | -3.96 | -39.77 |
| 13240-L5-2 | -18.39 | -4.67 | -2.06 | -1.28 | -1.62 | -1.59 | -3.66 | 1.21 | -3.23 | -2.56 | -2.62 | -2.75 |
| 13241-L5-2 | -10.26 | -4.55 | -4.91 | 1.77 | 1.77 | -1.36 | -2.23 | 3.91 | -3.60 | -1.33 | -4.77 | -10.26 |
| 13251-R5-2 | -20.00 | -3.70 | -3.53 | 1.00 | 1.38 | -1.26 | -4.35 | 1.58 | -4.26 | -2.42 | -3.46 | -2.91 |
| 13259-R5-1 | -3.29 | -3.29 | -3.29 | -3.29 | 2.53 | 1.88 | -1.19 | -3.29 | -3.29 | 1.28 | 1.08 | -3.29 |
| 13267-L5-1 | -30.85 | -13.05 | -1.69 | -10.20 | 1.96 | 3.35 | -1.63 | 8.62 | -12.76 | -1.63 | -2.04 | -9.14 |
| 13281-L5-3 | 1.06 | -1.86 | -1.81 | -1.07 | 1.11 | -1.64 | -3.92 | 1.69 | -1.24 | -2.19 | -3.54 | -6.83 |
| 13283-L5-3 | -13.68 | -3.86 | -2.82 | -1.06 | 1.13 | -1.43 | -1.92 | 2.27 | -1.49 | -1.44 | -1.67 | -6.16 |
| 13285-L5-3 | -11.84 | -4.28 | -2.47 | -1.21 | -1.65 | -1.98 | -4.63 | -1.22 | -3.72 | -2.67 | -3.38 | -1.41 |
| 13287-L5-3 | -27.01 | -1.81 | -1.63 | -1.11 | 2.16 | 1.08 | -3.42 | 2.88 | -2.45 | -1.53 | -2.25 | -1.07 |
| 13291-L5-1 | -5.33 | -8.08 | -1.21 | 1.10 | 5.56 | 2.15 | -2.09 | 6.10 | -1.56 | 1.04 | -1.83 | -5.77 |
| 13293-L5-1 | -18.63 | -18.63 | -1.65 | 1.07 | 1.04 | -1.36 | -3.93 | 2.07 | -2.11 | -1.84 | -2.35 | -18.63 |
| 13298-R5-1 | -14.82 | -14.82 | -3.48 | 1.05 | 1.24 | -1.67 | -3.79 | 1.78 | -14.82 | -2.80 | -3.15 | -14.82 |
| 13303-L5-3 | -14.35 | -7.42 | -1.08 | 1.87 | 3.44 | 1.82 | -2.05 | 3.33 | 1.08 | 1.03 | -1.75 | -14.35 |
| 13308-L5-1 | -14.43 | -1.49 | -1.10 | 1.30 | 3.61 | 1.10 | -2.16 | 2.74 | -1.69 | -1.36 | -1.79 | -14.43 |
| 13310-R5-1 | -6.22 | -6.22 | -6.22 | 1.86 | -1.03 | -1.50 | -1.67 | 3.20 | 1.44 | 2.11 | -1.28 | -6.22 |
| 13312-L5-2 | -14.33 | -2.46 | -2.40 | -1.24 | 3.55 | 1.50 | -3.72 | 3.22 | -3.06 | -2.08 | -2.68 | -1.53 |
| 13313-L5-2 | -8.53 | -2.34 | -1.67 | 1.70 | 1.40 | -1.33 | -2.46 | 2.18 | -2.74 | -1.65 | -2.66 | -4.35 |
| 13316-R5-2 | -4.30 | -4.30 | -2.20 | 2.94 | 1.96 | 3.07 | -1.31 | 4.09 | 1.82 | 2.30 | 1.24 | -4.30 |
| 13326-L5-2 | -52.63 | -3.83 | -2.70 | -1.22 | 1.72 | 1.01 | -3.47 | 2.34 | -3.63 | -2.53 | -3.07 | -1.23 |
| 13328-R5-2 | -14.53 | -14.53 | -3.23 | 1.52 | -1.14 | -1.34 | -2.95 | 1.56 | -1.83 | -2.66 | -2.68 | -14.53 |
| 13332-L5-1 | -4.39 | -4.39 | -2.11 | 2.76 | 2.77 | 1.33 | -1.10 | -1.46 | -4.39 | 1.18 | -1.07 | -1.98 |
| 13334-L5-3 | -43.43 | -2.48 | -4.73 | 1.14 | 3.67 | 1.66 | -3.78 | 3.22 | -3.11 | -1.94 | -2.34 | 1.24 |
| 13335-L5-3 | -4.23 | -3.07 | -1.86 | 1.27 | 1.43 | -1.36 | -3.29 | 1.75 | -2.97 | -1.67 | -2.59 | -1.75 |
| 13337-L5-2 | -42.63 | -15.36 | -1.48 | -11.45 | 3.39 | 1.31 | -3.13 | 2.96 | -16.82 | -2.38 | -3.47 | 1.92 |
| 13339-L5-1 | -3.11 | -3.86 | -2.52 | -1.15 | -2.17 | -2.18 | -3.10 | -1.68 | -3.33 | -2.51 | -2.85 | -1.46 |
| 13343-L5-1 | -5.44 | -2.70 | -1.77 | 1.12 | 2.31 | 1.34 | -2.98 | 3.06 | -2.38 | -1.38 | -1.82 | -1.09 |
| 13349-L5-2 | -10.66 | -3.95 | -2.27 | -1.24 | -1.33 | -2.25 | -4.40 | 1.03 | -3.81 | -2.98 | -3.14 | -1.13 |
| 13353-L5-2 | -2.24 | -2.24 | -2.24 | -2.24 | 1.70 | 5.74 | 1.55 | 1.27 | 2.75 | 2.47 | 1.48 | -2.24 |
| 13354-L5-1 | -17.63 | -10.31 | -17.63 | 1.04 | -1.29 | -1.42 | -4.54 | 1.52 | -3.25 | -3.11 | -4.57 | -17.63 |
| 13355-L5-2 | -5.51 | -5.51 | -5.51 | 1.73 | 1.33 | 2.51 | -2.60 | 1.56 | -5.51 | -2.00 | -5.51 | -5.51 |
| 13356-L5-2 | -8.37 | -8.37 | 1.15 | -8.37 | 1.19 | -1.16 | -1.71 | 2.41 | -2.44 | -1.56 | -1.28 | -8.37 |
| 13358-L5-2 | -23.01 | -4.99 | -4.83 | 1.29 | 1.72 | -1.44 | -3.50 | 1.29 | -10.96 | -2.15 | -3.31 | -23.01 |
| 13361-L5-1 | -12.93 | -12.93 | -12.93 | -1.25 | -1.51 | -2.03 | -3.61 | 1.42 | -1.98 | -2.17 | -1.96 | -12.93 |
| 13363-L5-2 | -20.98 | -20.98 | -20.98 | -1.40 | -1.39 | -2.08 | -4.73 | 1.11 | -2.88 | -2.56 | -2.49 | -20.98 |
| 13364-L5-2 | -7.47 | -7.47 | -3.52 | -7.47 | -1.24 | -2.33 | -2.43 | 3.10 | -7.47 | -1.78 | -1.46 | -7.47 |
| 13365-L5-3 | -7.56 | -3.41 | -1.76 | 1.61 | 1.56 | -1.00 | -3.01 | 1.80 | -2.88 | -1.74 | -2.58 | -1.78 |
| 13370-L5-2 | -27.25 | -1.67 | -1.39 | 1.09 | 1.33 | -1.16 | -2.69 | 2.19 | -2.79 | -1.56 | -2.12 | -1.10 |
| 13373-L5-4 | -3.08 | -7.98 | -2.22 | 1.02 | 2.43 | 1.76 | -2.02 | 3.28 | -1.55 | 1.04 | -1.31 | -7.98 |
| 13374-R5-1 | -35.28 | -7.46 | -2.24 | -1.53 | -1.13 | -1.31 | -3.05 | 1.69 | -2.75 | -2.15 | -2.18 | -5.68 |
| 13375-L5-3 | -41.40 | -3.21 | -2.13 | -1.40 | -1.08 | -1.32 | -4.62 | 1.77 | -3.47 | -1.85 | -2.99 | -15.84 |
| 13376-R5-1 | -46.25 | -2.90 | -3.40 | 1.26 | 1.17 | -1.77 | -3.98 | 1.62 | -5.25 | -4.65 | -5.47 | -46.25 |
| 13380-R5-3 | -10.07 | -10.07 | -1.12 | 1.07 | -1.25 | -1.49 | -2.40 | 1.95 | -10.07 | -2.00 | -1.88 | -10.07 |
| 13385-L5-1 | -3.98 | -3.98 | -3.98 | 2.74 | 3.39 | 3.77 | -1.37 | -1.25 | -3.98 | 1.45 | 1.01 | -3.98 |
| 13396-L5-2 | -51.56 | -3.50 | -3.03 | -1.02 | 1.19 | -1.18 | -3.70 | 2.12 | -3.31 | -2.34 | -2.84 | -19.22 |
| 13403-L5-1 | -21.42 | -1.77 | -1.59 | 2.39 | 2.56 | -1.10 | -2.29 | 1.93 | -2.00 | -2.82 | -3.69 | -21.42 |
| 13412-L5-1 | -8.32 | -8.32 | 1.16 | 1.31 | -1.47 | -1.43 | -2.06 | 1.69 | -8.32 | -1.83 | -1.40 | -8.32 |
| 13423-L5-3 | -9.37 | -2.67 | -1.70 | 2.74 | 1.65 | -1.49 | -3.52 | 1.30 | -3.67 | -2.44 | -3.89 | -4.22 |
| 13425-L5-3 | -8.40 | -2.50 | -1.76 | 3.00 | 1.76 | -1.51 | -3.88 | 1.20 | -4.15 | -2.68 | -4.80 | -4.43 |
| 13430-L5-3 | -3.90 | -3.61 | -2.27 | -1.01 | 3.74 | 1.98 | -3.74 | 5.44 | -1.77 | -2.17 | -3.43 | -18.09 |
| 13431-L5-3 | -4.88 | -6.00 | -2.06 | 1.01 | -2.24 | -3.42 | -4.62 | -1.58 | -3.62 | -3.19 | -4.73 | -1.29 |
| 13432-R5-1 | -17.04 | -1.33 | 1.36 | 3.21 | 2.17 | 1.38 | -2.00 | 2.50 | 1.55 | -1.06 | -2.15 | -17.04 |
| 13456-R5-2 | -14.18 | -14.18 | 2.87 | -6.32 | 1.05 | 1.63 | -2.07 | 5.81 | -4.94 | -1.19 | -1.58 | -14.18 |
| 13458-R5-2 | -9.56 | -9.56 | 1.53 | -4.22 | 4.25 | -1.29 | -1.67 | 10.75 | -3.73 | -1.02 | -1.10 | -9.56 |
| 13461-L5-4 | -70.04 | -4.10 | -2.14 | -1.39 | 1.18 | -1.05 | -3.94 | 2.59 | -2.51 | -2.43 | -2.91 | -2.77 |
| 13463-L5-2 | -18.40 | -3.50 | -2.21 | 1.89 | 1.41 | -1.11 | -3.17 | 2.39 | -3.17 | -2.66 | -4.28 | -25.56 |
| 13489-L5-1 | -2.32 | -2.32 | 4.10 | 7.21 | 1.60 | 1.78 | -1.10 | -2.32 | 1.73 | -2.32 | -2.32 | -2.32 |
| 13497-L5-1 | -7.23 | -4.09 | -3.08 | 1.16 | 1.64 | 1.09 | -4.05 | 1.93 | -4.13 | -2.92 | -4.09 | -3.23 |
| 13513-L5-1 | -3.48 | -3.48 | 4.44 | -3.48 | 1.38 | 2.30 | 1.03 | 4.31 | 2.43 | 1.49 | 2.28 | -3.48 |
| 13519-L5-1 | -8.34 | -8.34 | -4.66 | 1.28 | 1.53 | -1.40 | -2.94 | 2.21 | -8.34 | -2.08 | -1.85 | -8.34 |
| 13525-L5-2 | -11.76 | -11.76 | -2.97 | 1.16 | 1.18 | -1.56 | -3.63 | 1.43 | -11.76 | -1.93 | -2.26 | -4.62 |
| 25-R5-1 | -13.46 | -13.46 | 1.75 | 1.51 | 3.69 | 1.86 | -1.81 | 3.93 | -1.29 | -1.53 | -2.27 | -13.46 |
| 266-R5-2 | -5.40 | -3.19 | -2.35 | 1.20 | 3.10 | 1.60 | -3.19 | 3.09 | -2.31 | -1.95 | -3.02 | -3.07 |
| 2786-L5-3 | -7.14 | -7.14 | -1.80 | 1.48 | 1.86 | -1.31 | -2.37 | 2.47 | -7.14 | -1.82 | -1.48 | -7.14 |
| 2811-R5-1 | -5.19 | -5.19 | -5.19 | -5.19 | 1.07 | -1.12 | -1.90 | 2.46 | -5.19 | -1.53 | -1.19 | -5.19 |
| 2819-R5-4 | -5.51 | -5.80 | -2.51 | -1.25 | -2.51 | -3.80 | -5.86 | -1.79 | -4.40 | -3.96 | -6.14 | -1.09 |
| 3717-L5-2 | -10.28 | -3.32 | -2.43 | 1.83 | 1.05 | 1.36 | -5.47 | 2.60 | -5.09 | -3.62 | -5.62 | -1.15 |
| 3732-R5-1 | -13.70 | -1.52 | -1.09 | 1.37 | 1.34 | 1.51 | -2.32 | -1.64 | -1.65 | -1.02 | -2.09 | -4.30 |
| 3799-R5-1 | -6.12 | -2.70 | -1.95 | 1.54 | 1.37 | 1.25 | -2.90 | 2.68 | -2.69 | -1.77 | -2.48 | -8.74 |
| 3897-R5-2 | -8.76 | -5.74 | -2.75 | -1.70 | -1.02 | 1.56 | -3.91 | 1.41 | -3.43 | -2.66 | -2.84 | -2.33 |
| 3942-L5-1 | -4.60 | -1.85 | -1.50 | 2.56 | 2.76 | 1.21 | -2.11 | 2.61 | -2.13 | -2.30 | -3.87 | -53.73 |
| 3952-L3-1 | -7.09 | -1.94 | -1.94 | 1.81 | -1.05 | 1.07 | -2.42 | 1.66 | -7.09 | -2.27 | -1.92 | -7.09 |
| 3953-R3-2 | -7.36 | -4.86 | -2.14 | -1.41 | -1.64 | -1.73 | -3.38 | 1.20 | -3.51 | -2.26 | -3.09 | -1.13 |
| 3966-L5-1 | -9.29 | -5.96 | -2.17 | -1.13 | -1.86 | -2.86 | -3.59 | -1.09 | -3.21 | -2.58 | -3.27 | -2.45 |
| 3995-L2-2 | -25.12 | -2.66 | 2.34 | 1.82 | 2.48 | 1.95 | -2.10 | 4.87 | 4.02 | -1.04 | -1.85 | -25.12 |
| 4013-L4-1 | -2.19 | -1.14 | -1.19 | 5.14 | 1.52 | -1.25 | -2.19 | -2.19 | -2.19 | -2.19 | -2.19 | -2.19 |
| 4026-R5-1 | -5.56 | -3.29 | -2.88 | 1.85 | 1.58 | -1.41 | -3.53 | -81.69 | -5.46 | -4.37 | -5.89 | -81.69 |
| 4026-R5-2 | -7.79 | -3.53 | -2.44 | 2.12 | 1.69 | -1.02 | -3.26 | 1.98 | -4.14 | -3.33 | -5.34 | -145.60 |
| 410-R5-1 | -5.18 | -5.18 | -5.18 | -5.18 | 2.41 | 1.08 | -1.67 | 4.07 | -5.18 | -1.60 | -1.13 | -5.18 |
| 4130-L5-1 | -18.42 | -18.42 | -4.29 | -1.07 | -2.00 | -3.97 | -4.12 | -1.60 | -18.42 | -3.45 | -3.62 | 1.01 |
| 4143-R5-2 | -3.75 | -3.75 | -3.75 | -3.75 | 3.84 | 1.77 | -1.16 | 6.22 | -3.75 | 1.61 | 1.47 | -3.75 |
| 4258-L5-1 | -2.02 | -2.02 | -2.02 | -2.02 | 5.96 | 3.36 | 1.46 | 8.78 | -2.02 | 2.12 | 2.15 | -2.02 |
| 4315_C-L4-1 | -26.61 | -2.74 | -1.68 | 1.24 | 1.19 | 1.14 | -4.07 | -84.00 | -3.03 | -1.71 | -2.46 | -2.12 |
| 4315_D-R4-1 | -52.47 | -4.05 | -1.59 | -1.06 | -1.09 | -1.34 | -4.58 | 1.76 | -3.29 | -2.93 | -3.35 | -16.76 |
| 4315_E-R4-1 | -14.07 | -3.05 | 1.21 | -1.02 | 1.93 | 1.81 | -2.20 | 4.06 | -1.31 | -1.04 | 1.16 | -2.25 |
| 4315_F-R4-1 | -6.50 | -3.14 | -1.75 | -1.35 | 1.89 | 1.09 | -3.03 | 2.07 | -2.72 | -1.88 | -2.15 | -2.00 |
| 4315_I-L4-1 | -24.12 | -2.69 | -1.62 | -1.14 | 1.34 | 1.53 | -3.47 | 3.10 | -2.18 | -1.55 | -1.78 | -24.12 |
| 4315_K-L4-1 | -11.95 | -3.76 | -1.84 | -1.67 | 1.11 | -1.40 | -5.02 | 1.91 | -2.86 | -2.79 | -3.01 | -1.07 |
| 4315-R3-2 | -18.48 | -18.48 | -1.57 | -1.08 | 1.94 | 1.17 | -2.31 | 3.55 | -1.46 | -1.77 | -1.94 | -18.48 |
| 4338-L5-2 | -2.07 | -1.04 | -2.07 | 6.34 | -1.05 | 2.04 | 1.12 | -2.07 | 3.52 | -1.27 | -2.07 | -2.07 |
| 4340-R3-1 | -5.28 | -5.28 | -2.74 | 1.96 | 1.91 | 1.22 | -1.42 | 3.91 | -5.28 | -1.09 | -1.05 | -5.28 |
| 4346-L5-1 | -4.26 | -3.59 | -2.40 | -1.31 | 1.61 | 1.08 | -2.87 | 2.65 | -2.47 | -1.51 | -1.60 | -2.44 |
| 4361-R5-1 | -16.06 | -3.21 | -2.18 | 1.15 | 2.56 | 1.70 | -1.98 | 4.81 | -16.06 | -1.30 | -1.94 | -5.13 |
| 4498-L3-2 | -9.82 | -5.38 | 1.41 | 1.75 | 1.02 | -1.42 | -1.96 | 2.19 | -1.12 | -1.31 | -1.02 | -9.82 |
| 4516-L5-1 | -14.82 | -6.92 | -14.82 | 1.39 | 1.36 | -1.61 | -3.67 | 1.30 | -14.82 | -3.07 | -3.34 | -14.82 |
| 454-R5-1 | -2.62 | -2.62 | -2.62 | -2.62 | 3.88 | 1.74 | 1.02 | 6.55 | -2.62 | 1.18 | -1.32 | -2.62 |
| 4593-R5-1 | -38.50 | -3.52 | -1.40 | -1.57 | 1.21 | 3.25 | -2.72 | 7.52 | -2.16 | -1.91 | -2.36 | -38.50 |
| 4610-R5-1 | -7.69 | -7.69 | -4.23 | -7.69 | 1.05 | -1.38 | -2.23 | 2.29 | -7.69 | -1.85 | -1.57 | -7.69 |
| 4610-R5-2 | -3.23 | -3.23 | -1.64 | -3.23 | 4.54 | 1.72 | 1.06 | 6.67 | -3.23 | 1.85 | 1.59 | -3.23 |
| 4642-R3-2 | -10.25 | -10.25 | -5.37 | 1.32 | -1.09 | -1.37 | -2.03 | 1.75 | -10.25 | -1.52 | -1.92 | -10.25 |
| 4666-R5-1 | -52.31 | -8.64 | -2.97 | -1.02 | 3.07 | 1.24 | -3.80 | 4.13 | -2.83 | -3.00 | -4.54 | -20.59 |
| 4792-L5-2 | -8.62 | -2.62 | -1.48 | 1.58 | 1.24 | -1.03 | -2.41 | 3.62 | -1.95 | -1.54 | -2.22 | -24.82 |
| 4801-L5-2 | -4.53 | -4.53 | -2.30 | 2.06 | 1.80 | 1.04 | -1.18 | 2.99 | -4.53 | 1.18 | 1.17 | -4.53 |
| 4813-R3-1 | -3.33 | -3.33 | -3.33 | 1.39 | 1.45 | -1.77 | -1.46 | -3.33 | -3.33 | -1.07 | -3.33 | -1.06 |
| 4875-R2-2 | -25.83 | -2.14 | -1.33 | 1.90 | 1.88 | 1.16 | -2.27 | 2.53 | -1.98 | -1.34 | -2.73 | -25.83 |
| 4912-L5-2 | -19.46 | -19.46 | -1.91 | -1.29 | -1.45 | -1.96 | -2.67 | 1.56 | -2.21 | -2.67 | -2.47 | -19.46 |
| 4929-R4-1 | -11.74 | -11.74 | -11.74 | 1.31 | -2.37 | -2.86 | -4.16 | -1.03 | -11.74 | -3.58 | -2.44 | -11.74 |
| 5032-R5-1 | -13.13 | -13.13 | -6.46 | -1.03 | 1.10 | -1.14 | -2.75 | 2.43 | -13.13 | -1.83 | -1.91 | -13.13 |
| 5048-L5-1 | -11.59 | -11.59 | -1.39 | 1.05 | -1.30 | -1.54 | -3.38 | 1.08 | -6.02 | -1.58 | -2.26 | -11.59 |
| 5071-R5-2 | -7.99 | -3.93 | -3.06 | -1.63 | -1.09 | -1.22 | -4.17 | 1.01 | -3.96 | -1.64 | -2.24 | -3.88 |
| 5107-L5-1 | -26.66 | -1.96 | -1.85 | 1.79 | 1.96 | 1.08 | -3.02 | 3.02 | -1.40 | -2.20 | -3.10 | -26.66 |
| 5210-L5-1 | -7.99 | -7.99 | 1.22 | 1.47 | -1.11 | -1.13 | -2.48 | 1.72 | -7.99 | -1.31 | -2.28 | -7.99 |
| 5342-L5-1 | -1.33 | -1.69 | -1.29 | 1.18 | -1.19 | -2.25 | -2.81 | 1.02 | -1.76 | -2.17 | -2.61 | -6.40 |
| 5491-R5-1 | -8.97 | -3.43 | -1.87 | 1.26 | -2.01 | -3.08 | -3.05 | -1.22 | -2.81 | -3.09 | -4.36 | 1.32 |
| 5521-L5-2 | -7.60 | -7.60 | 1.16 | 3.47 | 2.17 | -1.21 | -1.91 | 2.12 | -7.60 | -1.57 | -1.79 | -7.60 |
| 554-R5-1 | -8.55 | -2.59 | -1.73 | 2.95 | 1.77 | -1.55 | -3.52 | 1.23 | -4.27 | -2.77 | -4.42 | -5.30 |
| 5554-R5-2 | -28.10 | -1.55 | -1.48 | 1.18 | 1.34 | -1.27 | -3.63 | 1.83 | -2.03 | -2.07 | -3.04 | -2.37 |
| 5638-R5-2 | -8.67 | -2.30 | -2.11 | 2.48 | 1.70 | -1.46 | -4.40 | 1.36 | -3.47 | -2.78 | -4.60 | -4.20 |
| 5640-L3-1 | -11.16 | -2.70 | -1.45 | 1.39 | 1.59 | 1.12 | -3.47 | 2.10 | -2.10 | -1.71 | -2.50 | -35.95 |
| 5749-R5-1 | -3.50 | -3.50 | -1.65 | 7.88 | 3.53 | 2.22 | 1.19 | -3.50 | -3.50 | -1.07 | -3.50 | -3.50 |
| 5757-L5-1 | -22.78 | -22.78 | -4.13 | 1.16 | -2.40 | -6.68 | -3.91 | -22.78 | -22.78 | -4.48 | -4.14 | 1.71 |
| 5854-R5-2 | -8.37 | -2.42 | -1.59 | 3.10 | 1.90 | -1.20 | -2.79 | 1.86 | -3.32 | -2.33 | -3.25 | -4.60 |
| 5956-L5-1 | -1.50 | -1.19 | -8.83 | -3.12 | 1.38 | 1.74 | -1.28 | -1.96 | -8.83 | -1.21 | -3.33 | 1.37 |
| 5995-R5-1 | -2.29 | -2.29 | -2.29 | -2.29 | 3.85 | 1.13 | -2.29 | 4.79 | -2.29 | -1.51 | -2.29 | -2.29 |
| 6008-R5-1 | -9.71 | -9.71 | -9.71 | 1.21 | -1.27 | -2.19 | -3.80 | 1.01 | -9.71 | -2.70 | -5.17 | -9.71 |
| 6008-R5-2 | -5.41 | -5.41 | -2.83 | 1.90 | 1.17 | -5.41 | -2.24 | -5.41 | -5.41 | -1.58 | -1.86 | -5.41 |
| 6016-R2-1 | -12.02 | -12.02 | -2.83 | -1.03 | 1.09 | -1.29 | -2.43 | 2.59 | -1.53 | -1.37 | -1.49 | -12.02 |
| 6023-L5-1 | -19.11 | -4.43 | -9.06 | 1.64 | -1.55 | -1.76 | -4.04 | 1.19 | -2.84 | -3.49 | -3.47 | -19.11 |
| 6087-L4-1 | -8.97 | -8.97 | -4.56 | -8.97 | 1.20 | -1.22 | -2.10 | 1.95 | -8.97 | -1.82 | -2.11 | -8.97 |
| 6096-R5-1 | -12.65 | -12.65 | -6.35 | -12.65 | -2.12 | 1.96 | -4.37 | 3.74 | -12.65 | -3.78 | -3.17 | -12.65 |
| 6192-L5-1 | -13.47 | -13.47 | 2.69 | 2.33 | 1.51 | -1.93 | -1.74 | 4.14 | -4.25 | -1.11 | -1.06 | -13.47 |
| 6198-R5-2 | -8.02 | -4.03 | -1.28 | -1.07 | -1.73 | -2.33 | -2.25 | 1.04 | -2.46 | -1.83 | -2.17 | -2.31 |
| 6242-R5-1 | -5.90 | -5.90 | -5.90 | -2.78 | 1.99 | 1.41 | -2.49 | 3.61 | -5.90 | -2.04 | -3.28 | -5.90 |
| 6287-L3-2 | -19.21 | -10.03 | -2.08 | 1.30 | 1.40 | -1.38 | -3.39 | 2.27 | -2.02 | -2.10 | -2.68 | -19.21 |
| 6385-R5-2 | -9.70 | -9.70 | -2.46 | 1.00 | -1.10 | -1.39 | -2.34 | 2.61 | -9.70 | -1.58 | -1.52 | -9.70 |
| 6409-L3-1 | -2.84 | -3.81 | -1.89 | -1.05 | -1.55 | -3.11 | -4.36 | -1.21 | -2.13 | -3.04 | -2.46 | -3.30 |
| 6434-R5-1 | -4.10 | -2.03 | -1.40 | 3.14 | 1.59 | -1.08 | -3.58 | 2.01 | -2.31 | -2.55 | -4.04 | -2.74 |
| 6490-R5-3 | -23.58 | -2.43 | -2.36 | -1.08 | 2.40 | -1.34 | -2.61 | 3.20 | -2.47 | -1.71 | -2.00 | -4.00 |
| 6496-R5-2 | -44.85 | -3.00 | -3.09 | -1.17 | 4.58 | 2.18 | -3.23 | 4.50 | -2.86 | -1.62 | -2.20 | -1.51 |
| 6584-L5-1 | -13.67 | -13.67 | -5.86 | 1.18 | 3.62 | 2.49 | -1.83 | 6.84 | -13.67 | -1.23 | -1.55 | -13.67 |
| 6590-L5-1 | -4.65 | -4.65 | -1.25 | 2.00 | 2.52 | 1.66 | -1.27 | 6.29 | -2.59 | 1.13 | -1.09 | -4.65 |
| 6642-R5-1 | -9.51 | -1.99 | -1.73 | 1.59 | 3.00 | -1.02 | -2.64 | 3.60 | -1.98 | -1.60 | -2.83 | -1.73 |
| 669-R5-2 | -10.57 | -10.57 | -2.81 | 1.09 | -1.32 | 1.39 | -3.80 | 3.58 | -1.86 | -2.58 | -2.81 | -10.57 |
| 6718-L3-2 | -3.98 | -3.98 | -2.18 | 2.26 | 1.29 | -2.03 | -1.58 | -1.57 | -3.98 | 1.66 | -3.98 | -3.98 |
| 6839-L3-1 | -27.98 | -2.76 | -2.51 | -1.37 | 1.05 | -1.33 | -4.23 | 2.43 | -2.96 | -2.29 | -2.78 | -1.52 |
| 6880-L3-2 | -5.32 | -2.54 | -1.25 | 1.77 | 2.00 | 1.81 | -2.74 | 3.05 | -1.32 | -1.35 | -2.78 | -2.32 |
| 6908-L3-2 | -2.84 | -2.84 | 3.97 | 6.90 | 1.33 | 1.57 | -1.04 | -2.84 | 3.32 | -1.05 | -2.84 | -2.84 |
| 6984-R4-1 | -18.59 | -5.09 | -1.67 | 1.15 | 3.57 | 1.76 | -3.55 | 5.19 | -1.99 | -2.34 | -3.28 | -18.59 |
| 7029-R5-1 | -10.64 | -10.64 | -2.56 | 1.54 | 1.33 | -1.01 | -2.23 | 3.16 | -10.64 | -1.40 | -1.44 | -10.64 |
| 7061-R5-2 | -51.92 | -1.69 | -1.36 | -1.17 | 1.39 | 1.10 | -3.67 | 1.94 | -2.85 | -1.51 | -2.19 | -1.73 |
| 7066-R5-1 | -31.19 | -3.44 | -2.16 | 1.08 | -1.50 | -1.75 | -4.12 | 1.47 | -3.39 | -3.33 | -4.27 | -31.19 |
| 7069-R5-1 | -42.68 | -3.22 | -2.52 | 1.02 | 1.34 | -1.32 | -3.98 | 1.59 | -3.33 | -2.47 | -3.14 | -18.77 |
| 7113-R5-1 | -8.52 | -4.48 | -3.59 | 1.23 | 1.60 | -1.25 | -3.50 | 1.53 | -5.21 | -3.87 | -5.23 | -49.48 |
| 7126-L3-1 | -21.53 | -3.22 | 1.09 | -1.52 | 1.21 | 1.28 | -2.18 | 2.92 | -1.66 | -8.94 | -3.88 | -2.59 |
| 7141-R5-1 | -2.31 | -2.31 | -2.31 | -2.31 | 4.83 | 1.97 | -1.07 | 5.66 | -2.31 | 1.32 | -2.31 | -2.31 |
| 7221-R5-1 | -11.70 | -6.14 | -1.36 | -1.00 | -1.61 | -1.41 | -2.93 | 1.25 | -1.75 | -1.65 | -2.19 | -11.70 |
| 7313-L5-2 | -2.26 | -2.26 | -2.26 | -2.26 | 2.68 | 2.29 | 1.59 | 5.74 | -2.26 | 2.36 | 2.07 | -2.26 |
| 7352-R3-2 | -11.09 | -5.02 | 1.09 | 1.44 | 1.44 | 1.64 | -1.96 | 2.77 | -1.31 | 1.60 | -1.15 | -11.09 |
| 7356_A-R4-1 | -7.06 | -5.54 | -1.95 | -1.05 | -1.74 | -2.76 | -4.73 | -1.20 | -3.41 | -3.22 | -3.51 | -1.25 |
| 7356-L5-1 | -17.42 | -17.42 | -1.11 | 1.16 | 1.37 | -1.26 | -3.38 | 2.37 | -1.70 | -1.56 | -2.28 | -17.42 |
| 7367-L1-1 | -5.61 | -5.61 | -2.76 | 2.85 | 2.74 | 1.81 | -1.31 | 3.71 | -5.61 | -1.01 | -1.26 | -5.61 |
| 7384-R3-1 | -16.46 | -3.05 | -1.45 | 1.34 | 3.85 | 1.12 | -2.15 | 2.45 | -1.99 | -1.70 | -2.75 | -1.96 |
| 7411-R3-2 | -13.01 | -2.66 | -1.16 | 1.17 | 1.26 | 1.15 | -2.46 | 2.04 | -1.61 | 1.08 | -1.57 | -13.01 |
| 7569-L5-2 | -3.93 | -3.93 | -3.93 | -3.93 | -1.05 | -1.16 | -1.50 | 2.37 | -3.93 | -1.26 | -3.93 | -3.93 |
| 7571-L5-1 | -32.04 | -3.20 | 1.02 | -1.14 | 1.30 | -1.37 | -2.52 | 2.12 | -2.59 | -1.83 | -3.22 | -1.07 |
| 7572-R5-2 | -22.49 | -2.34 | -1.93 | -1.03 | 6.19 | 2.41 | -2.23 | 5.40 | -2.12 | -1.30 | -1.54 | -1.17 |
| 7660-L5-1 | -18.40 | -4.72 | -1.14 | 1.08 | 1.23 | -1.13 | -2.11 | 2.32 | -1.23 | -1.17 | -1.37 | -18.40 |
| 7702-L2-1 | -33.45 | -17.84 | -3.01 | -1.64 | 1.18 | -1.56 | -5.68 | 1.73 | -3.58 | -3.35 | -3.12 | -14.37 |
| 7736-L5-1 | -10.35 | -10.35 | 1.09 | 2.15 | 1.62 | 1.20 | -1.65 | 4.30 | 1.24 | 1.39 | -1.23 | -10.35 |
| 7743-L5-1 | -7.13 | -7.13 | -7.13 | -7.13 | -1.31 | -1.50 | -2.01 | 2.34 | -1.02 | -1.50 | -1.49 | -7.13 |
| 7781-R5-2 | -7.71 | -5.45 | -1.96 | 1.05 | -1.75 | -3.27 | -3.53 | -1.42 | -2.53 | -2.75 | -2.78 | -22.71 |
| 7824-R5-1 | -2.36 | -12.29 | -1.52 | -1.54 | -2.01 | -3.22 | -4.60 | -1.27 | -24.95 | -3.64 | -4.64 | -1.19 |
| 7846-L5-2 | 1.10 | -2.66 | -2.66 | -2.66 | 5.00 | 2.15 | 1.24 | 4.64 | -2.66 | 2.08 | 1.99 | -1.18 |
| 7883-R5-1 | -4.16 | -2.58 | -1.80 | -1.08 | 3.79 | 1.59 | -2.34 | 3.80 | -1.95 | -1.40 | -1.82 | -1.00 |
| 78-R4-1 | -2.39 | -2.39 | -1.21 | 6.26 | 2.28 | 1.91 | -1.02 | 1.00 | 3.43 | 1.26 | -2.39 | -2.39 |
| 7949-R5-1 | -4.01 | -4.36 | -1.30 | 1.15 | -2.21 | -2.42 | -2.64 | 1.16 | -2.75 | -2.23 | -3.15 | 1.33 |
| 7971-L5-1 | -7.45 | -7.45 | -1.61 | -7.45 | 1.29 | -1.04 | -1.90 | 2.29 | -7.45 | 1.31 | -1.06 | -7.45 |
| 8016-L3-1 | -13.40 | -1.65 | 1.05 | 1.08 | 1.03 | 2.34 | -2.26 | 3.25 | -1.63 | -1.53 | -2.21 | -13.40 |
| 8062-R5-1 | -7.61 | -3.70 | -1.70 | 2.18 | 2.59 | 1.56 | -2.32 | 2.34 | -7.61 | 1.60 | -1.20 | -7.61 |
| 8077-R3-1 | -10.66 | -10.66 | -1.94 | 1.52 | 1.52 | 2.13 | -2.53 | 4.22 | -10.66 | -1.39 | -1.79 | -10.66 |
| 8089-L5-1 | -12.58 | -12.58 | -12.58 | 1.14 | 1.34 | -1.19 | -3.58 | 1.73 | -12.58 | -2.50 | -2.89 | -12.58 |
| 8239-R5-1 | -7.85 | -7.85 | -1.71 | 1.38 | 3.30 | 1.25 | -1.74 | 4.30 | -7.85 | -1.16 | -1.28 | -7.85 |
| 8250-R5-2 | -8.86 | -6.17 | -3.27 | -1.52 | 1.20 | -1.96 | -3.94 | 1.31 | -3.73 | -2.78 | -2.56 | -1.90 |
| 8281-L5-2 | -4.41 | -1.86 | -1.49 | 2.18 | 1.25 | -1.11 | -2.92 | 2.00 | -2.11 | -1.90 | -2.99 | -5.52 |
| 8298-R5-1 | -2.06 | -3.00 | -4.52 | -1.25 | 1.18 | -1.68 | -4.67 | 1.41 | -2.94 | -3.38 | -3.35 | -2.74 |
| 8329-L5-1 | -7.14 | -7.14 | -7.14 | -1.04 | 1.64 | -4.12 | -1.88 | -2.09 | -7.14 | -1.69 | -1.50 | -7.14 |
| 8336-R5-2 | -7.05 | -5.15 | -1.26 | 1.44 | 1.78 | -1.12 | -2.29 | 2.53 | -1.47 | -1.45 | -2.17 | -26.66 |
| 8394-L5-2 | -33.24 | -2.09 | -1.40 | -1.37 | 1.26 | 1.30 | -2.51 | 2.74 | -2.20 | -1.19 | -1.38 | -1.20 |
| 8564-L5-1 | -9.90 | -9.90 | -9.90 | -1.75 | 2.20 | -1.21 | -2.57 | 3.01 | -9.90 | -2.15 | -2.29 | -3.34 |
| 8564-R5-2 | 3.57 | -3.01 | 2.65 | 4.26 | -1.18 | -1.95 | -1.65 | -6.82 | -1.16 | -1.87 | -3.25 | -6.82 |
| 8898-R5-1 | -15.47 | -9.30 | -2.03 | -1.44 | 1.04 | -1.40 | -4.53 | 1.61 | -2.95 | -1.90 | -2.58 | -1.63 |
| 9021-L5-2 | -9.46 | -4.89 | -1.02 | 1.42 | 1.38 | 1.13 | -1.84 | 4.21 | -1.28 | -1.21 | -2.15 | -9.46 |
| 9068-R5-2 | -10.19 | -2.29 | -1.93 | 2.59 | 1.47 | -1.63 | -3.56 | 1.03 | -4.24 | -2.77 | -4.29 | -2.77 |
| 9087-L5-2 | -5.65 | -2.03 | -2.17 | 1.21 | -1.62 | -4.32 | -4.61 | -1.82 | -3.78 | -3.28 | -6.39 | 1.58 |
| 9134-R5-1 | -2.19 | 1.85 | -1.06 | 5.55 | 1.62 | 1.08 | 1.58 | -2.19 | -2.19 | 1.28 | -2.19 | -2.19 |
| 9217-L3-2 | -25.69 | -2.49 | -2.14 | -1.16 | -1.07 | 1.71 | -3.81 | 1.45 | -2.41 | -2.14 | -2.41 | -11.60 |
| 9245-R5-1 | -7.70 | -7.70 | -7.70 | -7.70 | 1.21 | -1.21 | -1.48 | -7.70 | 1.20 | -1.58 | -1.57 | -7.70 |
| 9287-L5-2 | -8.66 | -10.47 | -2.11 | 1.95 | 1.22 | 1.28 | -5.10 | 2.39 | -5.82 | -4.19 | -6.03 | -2.02 |
| 9369-L5-1 | -2.53 | 2.54 | 3.26 | 5.80 | -1.36 | 1.32 | -1.20 | -2.53 | 3.39 | -2.53 | -2.53 | -2.53 |
| 9384-R5-2 | -15.00 | -15.00 | -3.18 | -1.02 | -1.40 | -1.71 | -4.41 | 1.20 | -4.03 | -2.13 | -2.58 | -15.00 |
| 9387-R2-2 | -2.27 | -11.24 | 1.24 | -1.66 | -1.77 | -2.13 | -2.67 | 1.46 | -18.94 | -3.05 | -3.88 | -1.22 |
| 9564-R5-2 | -21.32 | -1.78 | -1.27 | 1.30 | -1.36 | -1.75 | -2.96 | 1.23 | -1.50 | -1.51 | -2.02 | -8.68 |
| 9605-R5-1 | -2.11 | -2.11 | 2.05 | 9.59 | 3.34 | 1.71 | 1.35 | 1.73 | -2.11 | 2.19 | 1.04 | -2.11 |
| 9691-L5-1 | -13.91 | -7.43 | -3.19 | -1.18 | 1.46 | -1.16 | -1.81 | 3.62 | -1.45 | -1.71 | -1.56 | -13.91 |
| 9770-R5-2 | -16.08 | -16.08 | -1.38 | -6.49 | -1.54 | -1.04 | -3.18 | 2.79 | -16.08 | -2.24 | -1.93 | -16.08 |
| 9774-R2-2 | -20.32 | -5.37 | -3.62 | -1.47 | -1.04 | -2.24 | -5.14 | 1.19 | -4.78 | -2.77 | -3.92 | -3.06 |
| 9812-L3-1 | -8.36 | -8.36 | 1.41 | -3.81 | -1.10 | -1.30 | -1.61 | 2.18 | -8.36 | -1.63 | -1.54 | -8.36 |
| 9866-L5-1 | -11.22 | -11.22 | -11.22 | -11.22 | -1.94 | -4.41 | -3.65 | 1.13 | -11.22 | -2.98 | -2.07 | -11.22 |
| 999997-R4-1 | -40.37 | -3.22 | -2.37 | 1.31 | -1.44 | 1.32 | -5.01 | 3.93 | -3.66 | -3.76 | -5.46 | -40.37 |
| let-7b | -4.92 | 1.02 | 2.03 | 1.30 | -4.60 | -2.75 | -2.04 | -2.69 | -1.28 | -1.09 | -2.68 | 1.40 |
| let-7c | -6.31 | -1.05 | 1.94 | 1.37 | -4.31 | -2.44 | -1.65 | -3.04 | -1.21 | -1.03 | -2.43 | 1.67 |
| let-7e | -22.08 | 1.03 | 2.47 | 1.47 | -4.26 | -2.47 | -1.58 | -2.23 | -1.12 | -1.04 | -2.53 | 2.01 |
| miR-100 | -15.70 | -1.06 | 2.47 | 1.30 | -1.44 | -2.47 | -2.17 | -1.90 | -1.30 | -1.64 | -1.79 | -15.70 |
| miR-101 | -12.44 | -1.03 | 1.43 | 1.63 | -3.70 | -2.97 | -2.13 | -12.44 | -1.45 | -1.11 | -1.92 | 1.97 |
| miR-1182 | -12.48 | -2.69 | 1.05 | 1.43 | 2.25 | -1.41 | -1.78 | -1.07 | -12.48 | -1.46 | -2.26 | 2.67 |
| miR-1207-5p | -7.81 | -1.85 | -1.68 | 1.60 | 1.80 | -1.18 | -2.41 | 2.42 | -1.87 | -1.78 | -2.38 | -2.36 |
| miR-1224-5p | -8.27 | -4.01 | 1.20 | 1.73 | 3.06 | -1.10 | -2.00 | 2.05 | -8.27 | -1.44 | -1.68 | -8.27 |
| miR-1225-5p | -32.61 | -3.21 | -16.64 | 1.04 | 1.24 | -1.74 | -5.01 | 1.53 | -3.91 | -5.58 | -5.48 | -32.61 |
| miR-1228* | -9.13 | -2.28 | -2.02 | -1.49 | 1.07 | 1.35 | -3.26 | 2.11 | -3.56 | -1.07 | -1.87 | -3.42 |
| miR-1234 | -2.04 | 3.94 | 4.09 | 6.04 | 1.42 | 1.35 | -2.04 | -2.04 | -2.04 | -2.04 | -2.04 | -2.04 |
| miR-125a-5p | -12.05 | -1.57 | 2.48 | -1.11 | -2.90 | -3.80 | -2.32 | -36.39 | -1.43 | -2.16 | -3.93 | 1.84 |
| miR-126 | -41.23 | -1.60 | -1.58 | 1.20 | -9.24 | -16.81 | -5.80 | -17.32 | -3.33 | -5.96 | -5.54 | -2.23 |
| miR-1268 | -10.67 | -10.67 | -10.67 | 1.26 | 1.67 | -1.01 | -1.65 | 4.86 | -1.28 | 1.79 | -1.47 | -10.67 |
| miR-130b | -2.21 | -2.21 | 5.25 | 5.24 | -1.32 | -2.21 | 1.17 | -2.21 | -2.21 | 1.98 | 1.45 | -2.21 |
| miR-140-3p | -4.44 | -4.44 | 1.97 | 2.44 | -1.38 | -2.55 | -1.53 | -4.44 | -1.28 | -1.52 | -2.25 | -4.44 |
| miR-145 | -31.48 | -1.89 | 1.24 | -1.28 | -2.10 | -1.49 | -2.04 | -13.08 | 1.01 | -3.26 | -2.80 | 1.20 |
| miR-149* | -61.43 | -1.79 | -1.40 | 1.02 | 1.46 | 1.72 | -2.47 | 3.20 | -2.22 | 1.19 | -1.22 | -2.53 |
| miR-150 | -3.14 | -3.14 | 3.09 | 4.65 | 1.17 | -1.40 | -1.27 | -3.14 | -3.14 | 2.18 | -3.14 | -3.14 |
| miR-181b | -2.73 | -2.73 | 4.73 | -1.14 | 1.39 | -1.40 | -1.08 | -2.73 | -1.49 | 1.49 | -1.51 | -2.73 |
| miR-181d | -2.47 | -2.47 | 1.60 | 3.76 | 1.17 | 1.29 | -2.47 | -2.47 | -2.47 | 1.49 | 1.28 | -2.47 |
| miR-185* | -10.19 | -10.19 | 1.73 | -3.97 | -1.15 | -2.49 | -2.41 | -10.19 | -10.19 | -1.56 | -2.19 | -10.19 |
| miR-214 | -4.43 | -4.43 | 2.00 | 2.45 | -1.27 | 1.26 | -1.06 | -4.43 | 1.76 | -1.08 | -2.47 | -4.43 |
| miR-23a* | -3.92 | -3.92 | -3.92 | -3.92 | 2.27 | 1.12 | 1.28 | -3.92 | -3.92 | -1.18 | -3.92 | -3.92 |
| miR-30a | -19.76 | -1.04 | -1.44 | 1.58 | -3.89 | -3.05 | -4.56 | -7.27 | -1.86 | -1.24 | -2.08 | 5.69 |
| miR-30d | -1.08 | -16.33 | 1.01 | -5.75 | -3.77 | -3.17 | -4.86 | -16.33 | -7.71 | -1.17 | -2.19 | 12.09 |
| miR-320a | -3.29 | -2.05 | 1.23 | 1.49 | 5.76 | 2.18 | -1.27 | -9.70 | 1.23 | 1.59 | -1.65 | -3.76 |
| miR-320b | -10.09 | -2.35 | 1.00 | 1.90 | 5.22 | 2.10 | -1.35 | 4.46 | -1.05 | 1.28 | -1.58 | -10.09 |
| miR-335 | -3.24 | -3.24 | 1.22 | 3.13 | -3.24 | -3.24 | -1.26 | -3.24 | -3.24 | 5.52 | 1.37 | -3.24 |
| miR-34a | -2.08 | -2.08 | 5.16 | -2.08 | -1.23 | -2.08 | 1.19 | -2.08 | -2.08 | -2.08 | -1.15 | 1.18 |
| miR-34b* | -3.29 | -3.29 | 5.13 | -3.29 | -3.29 | -3.29 | -1.55 | -3.29 | -3.29 | -3.29 | -1.06 | -3.29 |
| miR-34c-5p | -2.54 | -2.54 | 5.54 | -2.54 | -2.54 | -2.54 | -2.54 | -2.54 | -2.54 | -2.54 | -2.54 | -2.54 |
| miR-371-5p | -3.87 | -3.87 | -1.81 | -1.51 | 1.98 | 1.65 | -1.03 | -1.06 | -3.87 | 1.10 | -1.83 | -3.87 |
| miR-373* | -34.48 | -13.72 | -1.65 | -8.74 | 1.28 | 2.21 | -3.58 | 5.31 | -16.32 | -2.81 | -4.50 | -34.48 |
| miR-451 | -14.34 | -2.32 | -4.25 | -2.09 | -10.15 | -7.99 | -3.72 | -9.47 | -9.88 | -7.90 | -2.86 | -3.23 |
| miR-486-3p | -10.16 | -10.16 | -2.27 | 1.10 | -1.59 | -1.36 | -3.64 | 1.72 | -10.16 | -2.14 | -1.92 | -10.16 |
| miR-491-3p | -2.34 | -2.34 | -2.34 | 2.06 | 1.33 | -2.34 | 2.23 | -2.34 | -2.34 | 1.67 | -2.34 | -2.34 |
| miR-498 | -28.12 | -3.04 | -1.96 | 1.11 | -1.41 | -2.01 | -3.75 | 1.36 | -3.41 | -2.64 | -4.35 | -28.12 |
| miR-557 | -10.00 | -1.36 | -1.92 | 1.93 | 1.16 | -1.55 | -2.17 | 1.60 | -1.32 | -2.10 | -2.21 | -1.67 |
| miR-638 | -5.13 | -5.73 | -2.92 | -1.37 | -1.87 | -2.89 | -5.04 | -1.34 | -4.22 | -3.54 | -4.11 | -1.97 |
| miR-663 | -15.30 | -15.30 | -2.65 | -1.03 | -1.31 | -1.69 | -3.07 | 1.69 | -1.47 | -1.68 | -1.53 | -15.30 |
| miR-671-5p | -9.36 | -9.36 | 1.12 | 2.50 | 1.56 | 1.06 | -2.25 | 2.06 | -1.39 | -1.18 | -2.06 | -9.36 |
| miR-744 | -14.84 | -14.84 | -3.03 | 1.02 | -1.26 | -1.33 | -3.71 | 2.36 | -1.77 | -2.34 | -2.27 | -14.84 |
| miR-885-3p | -26.07 | -26.07 | -2.17 | -1.16 | -1.96 | -2.54 | -4.68 | -1.16 | -3.53 | -3.27 | -3.46 | -10.53 |
| miR-92a-2* | -8.53 | -2.11 | 1.23 | 1.61 | 1.34 | 1.10 | -2.40 | 2.65 | -1.15 | -1.39 | -1.82 | -8.53 |
| miR-92b* | -11.88 | -11.88 | -1.16 | 1.21 | 1.35 | 1.19 | -2.46 | 3.28 | -1.42 | -1.41 | -1.46 | -11.88 |
| miR-98 | -45.32 | 1.35 | 2.78 | 1.82 | -4.33 | -2.12 | -1.37 | -9.42 | 1.07 | 1.08 | -2.69 | 1.84 |
| miR-99a | -10.35 | 1.22 | 3.06 | 1.55 | -1.32 | -4.39 | -1.67 | -10.35 | -1.21 | -1.78 | -2.15 | -10.35 |

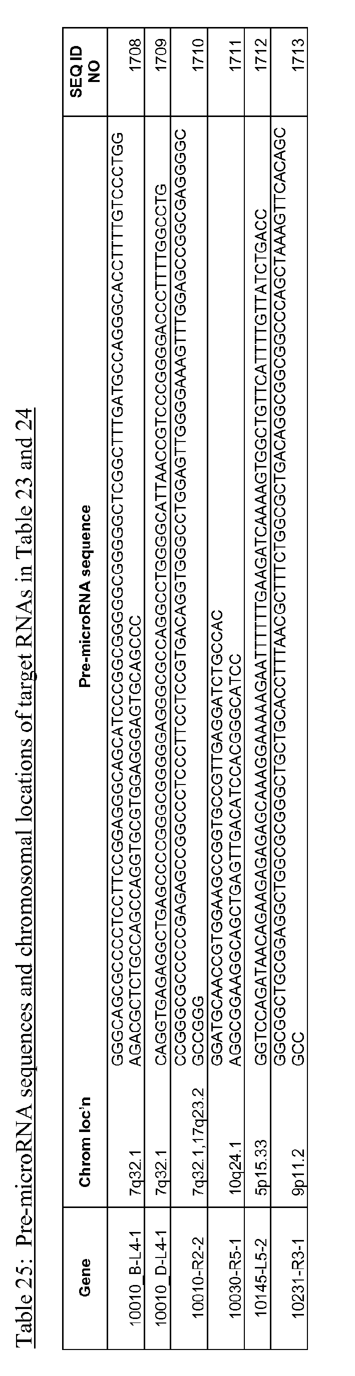

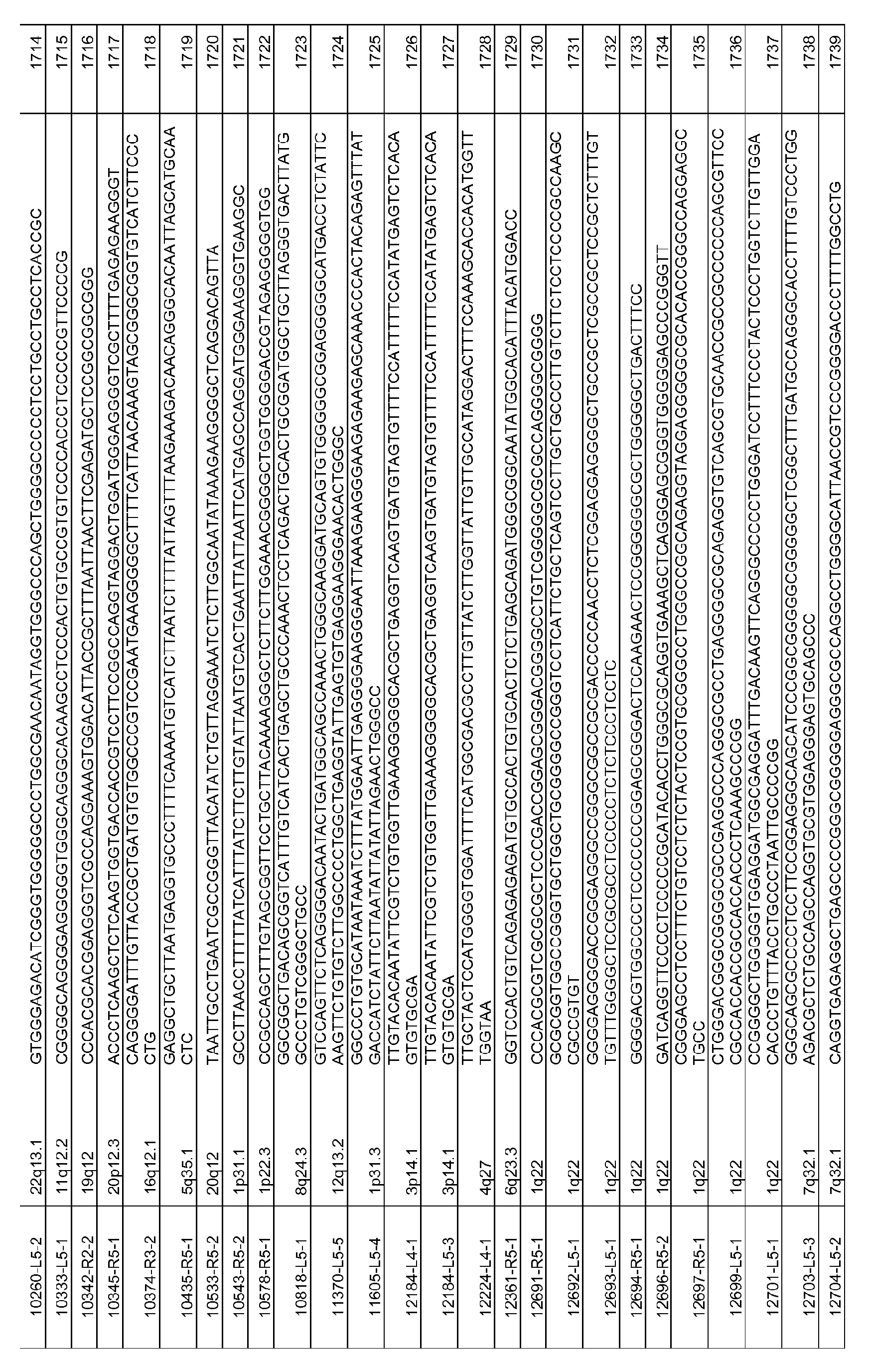

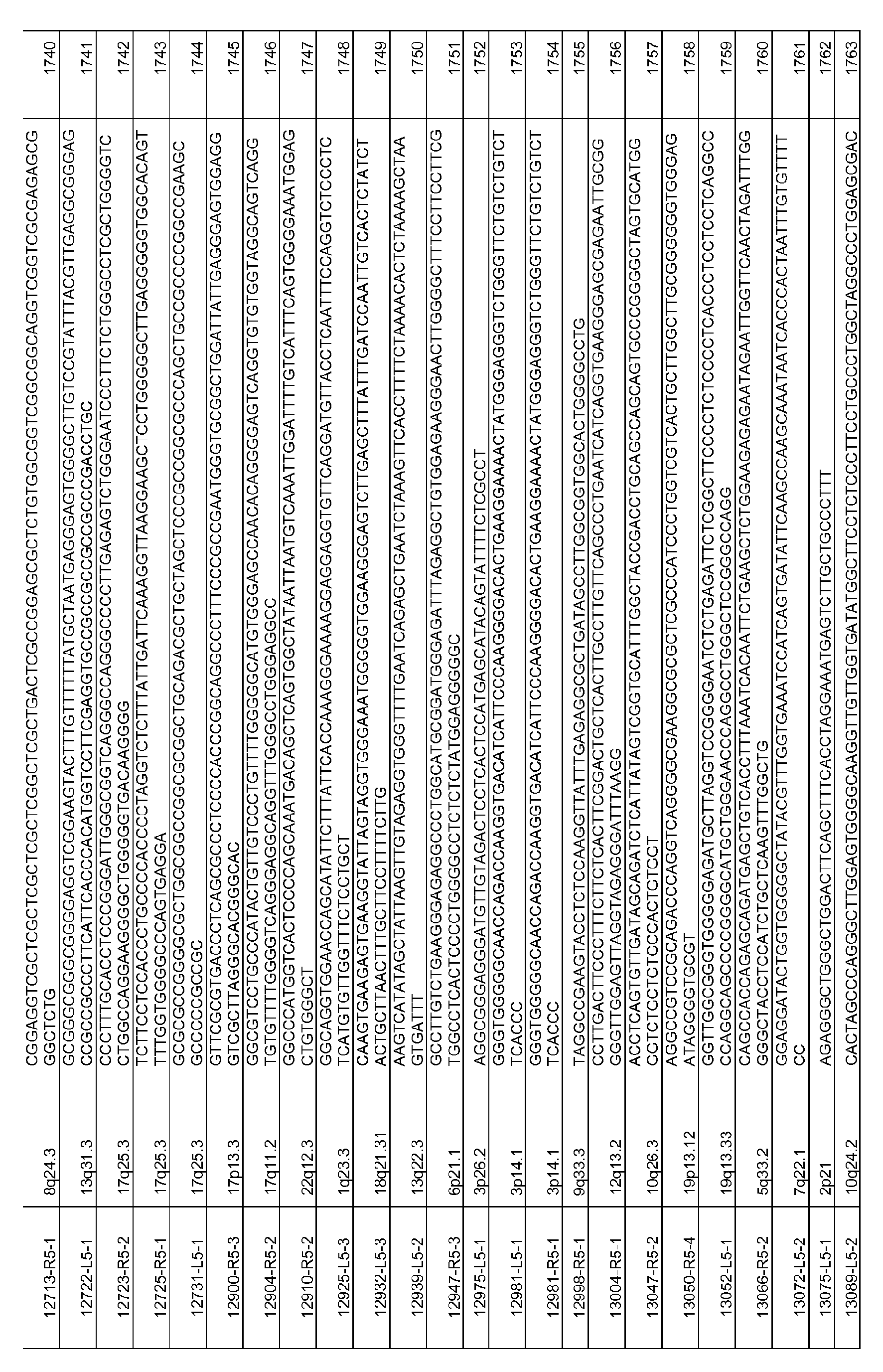

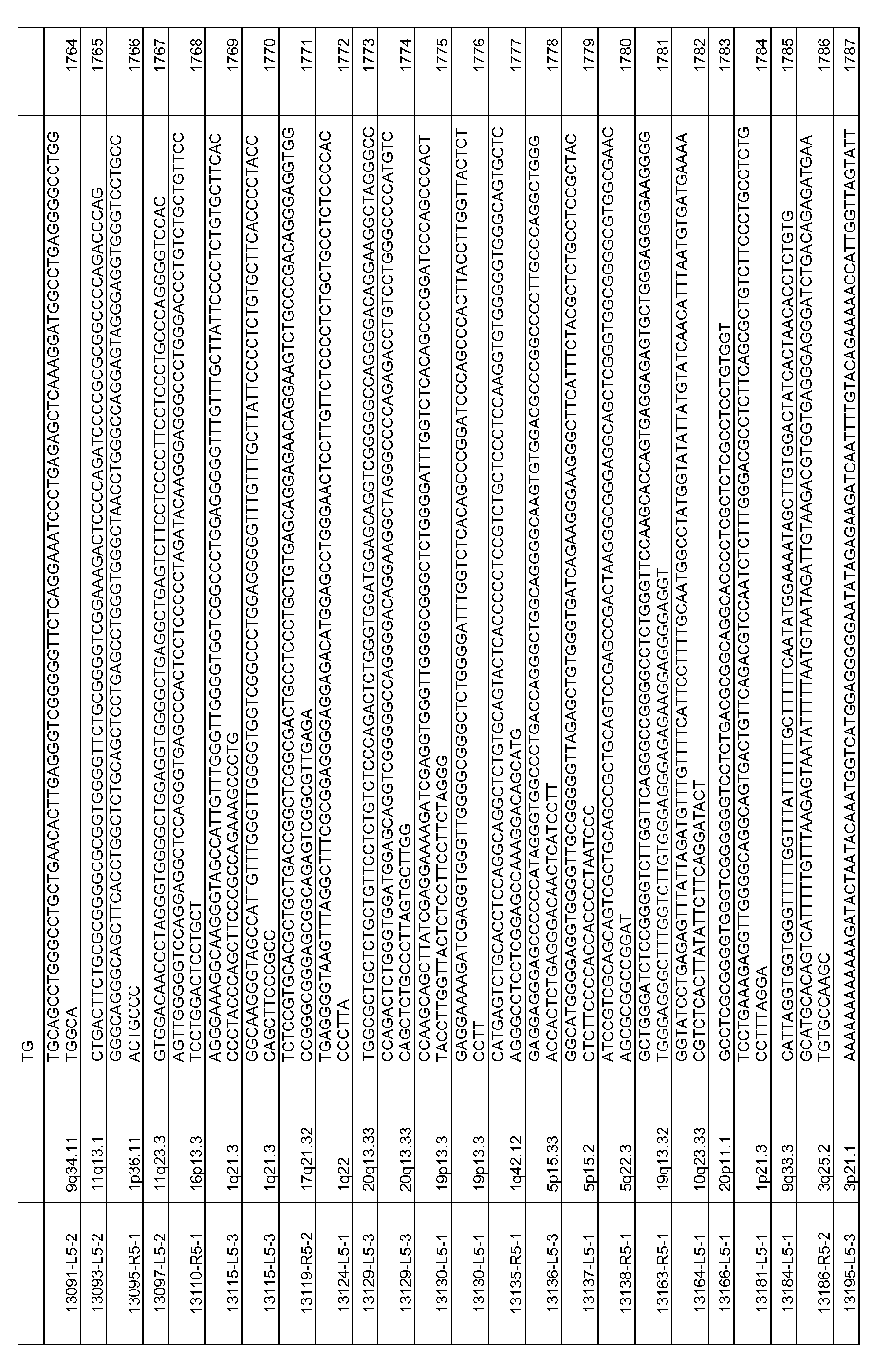

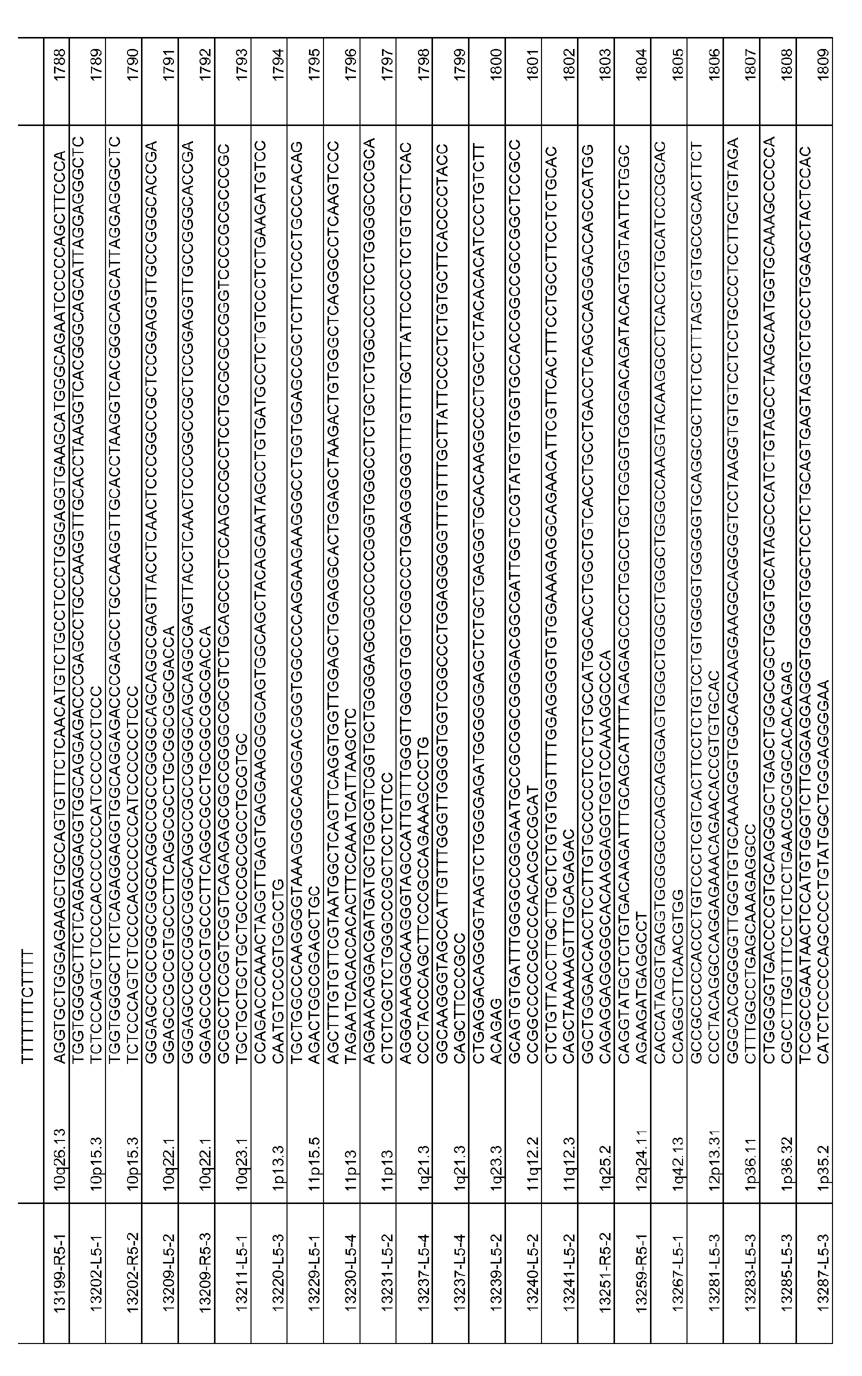

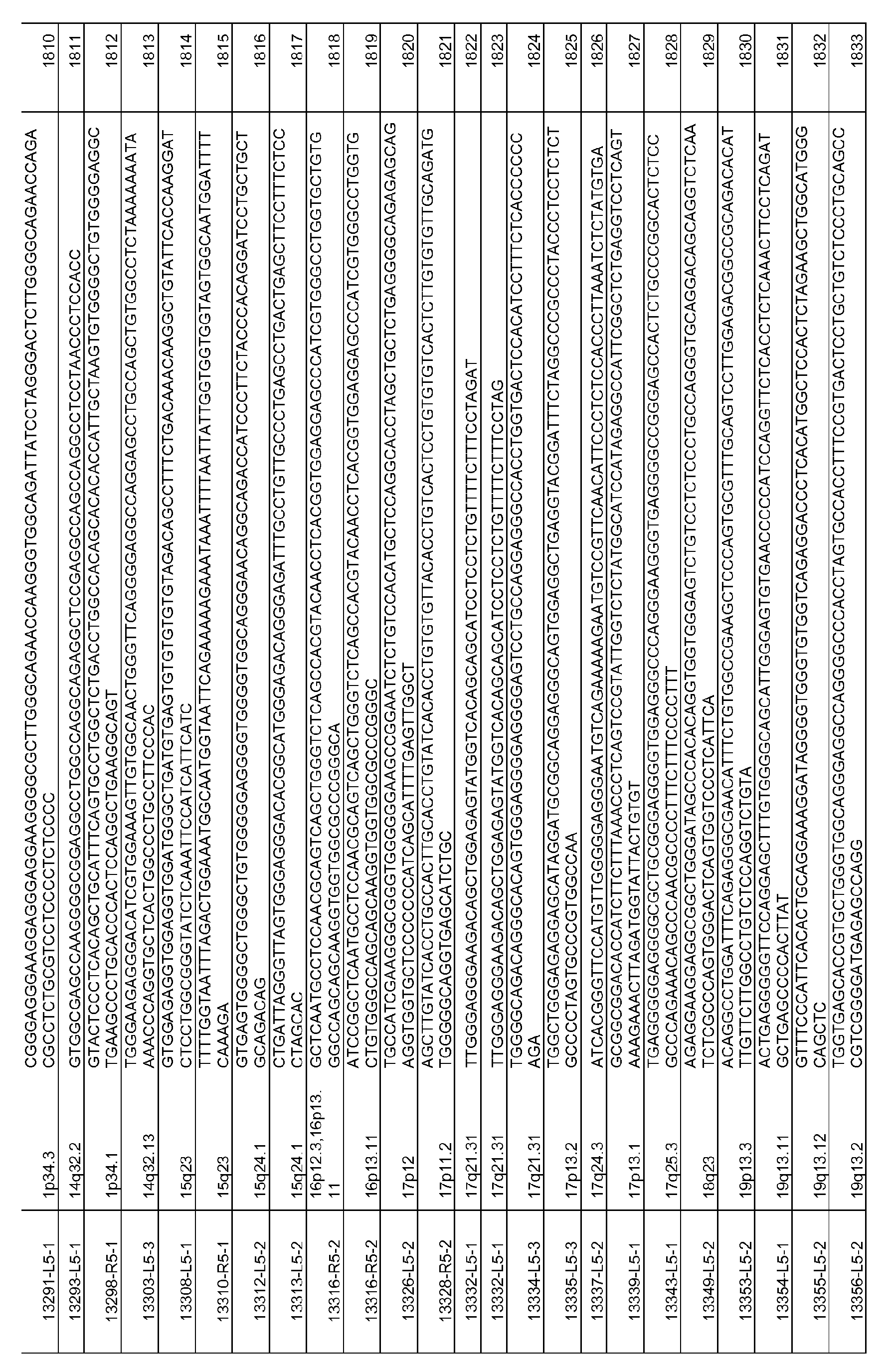

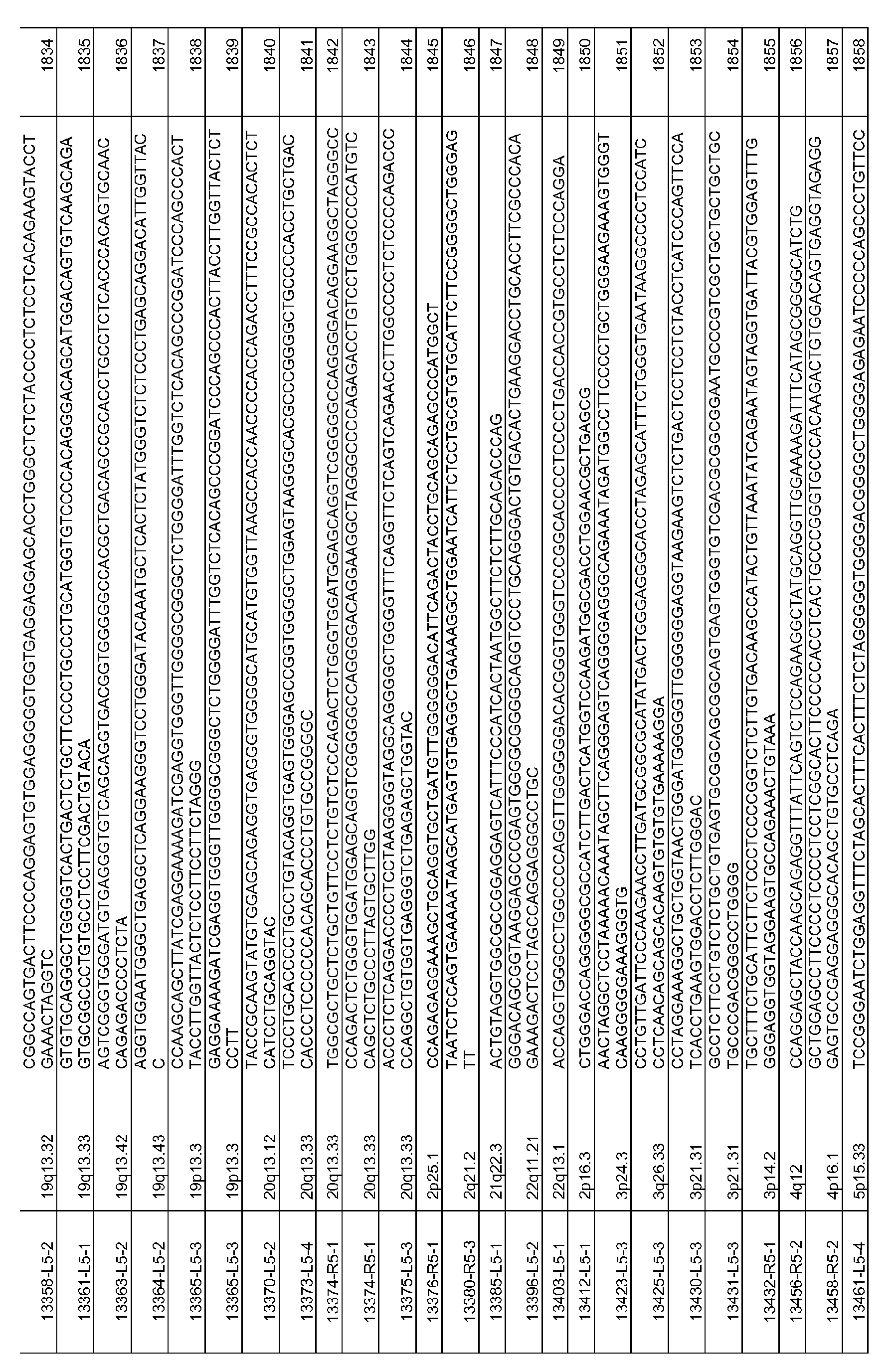

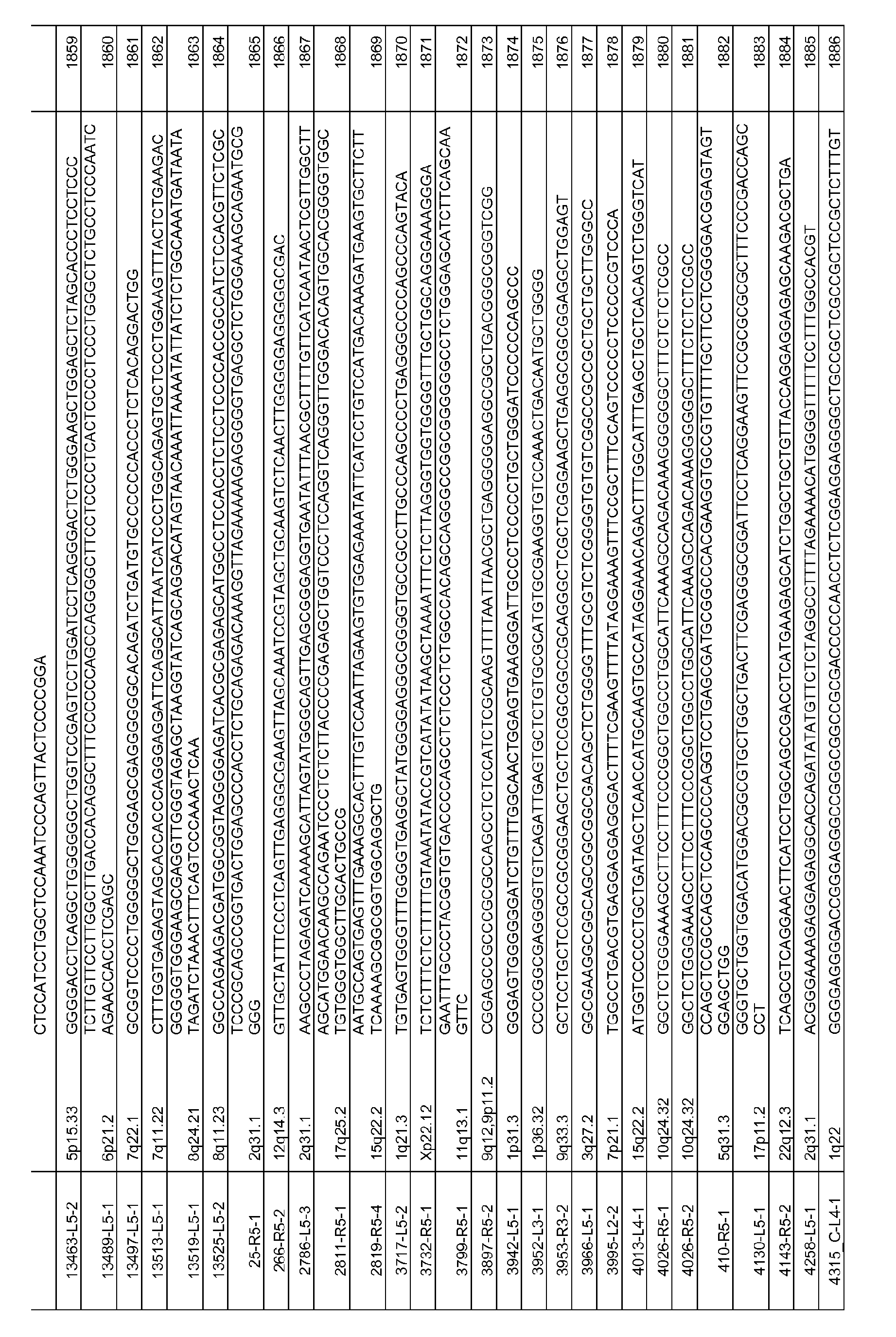

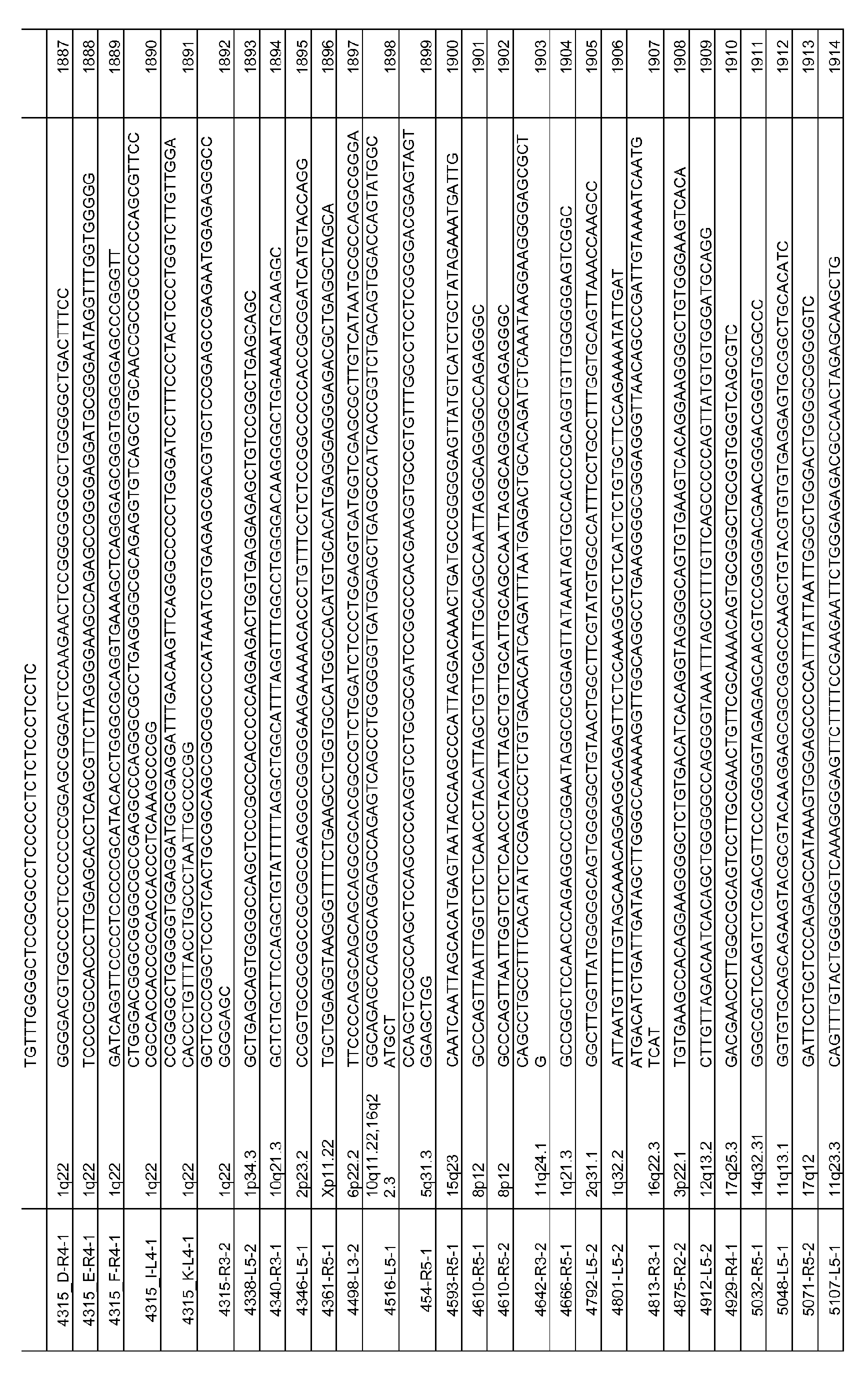

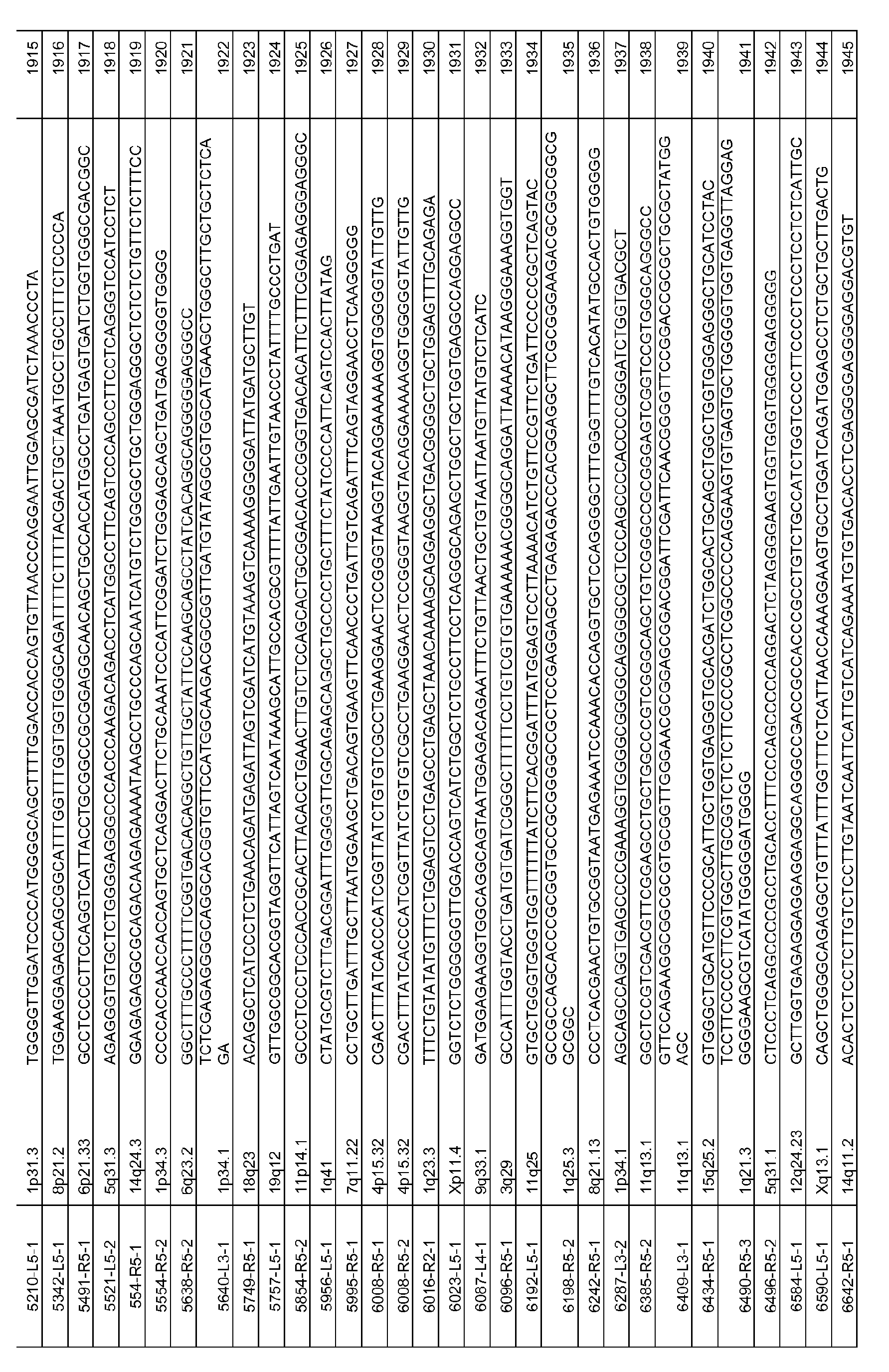

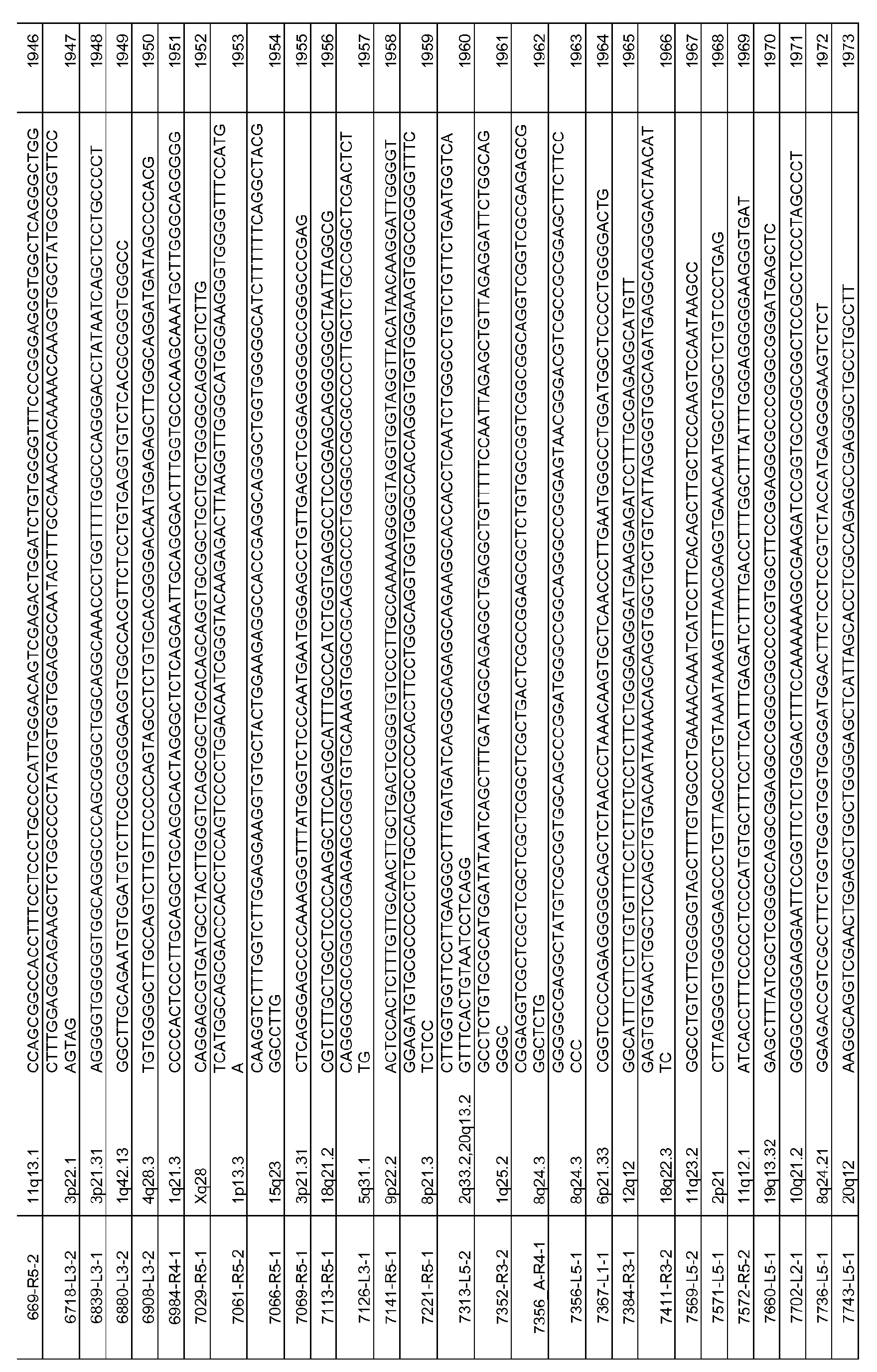

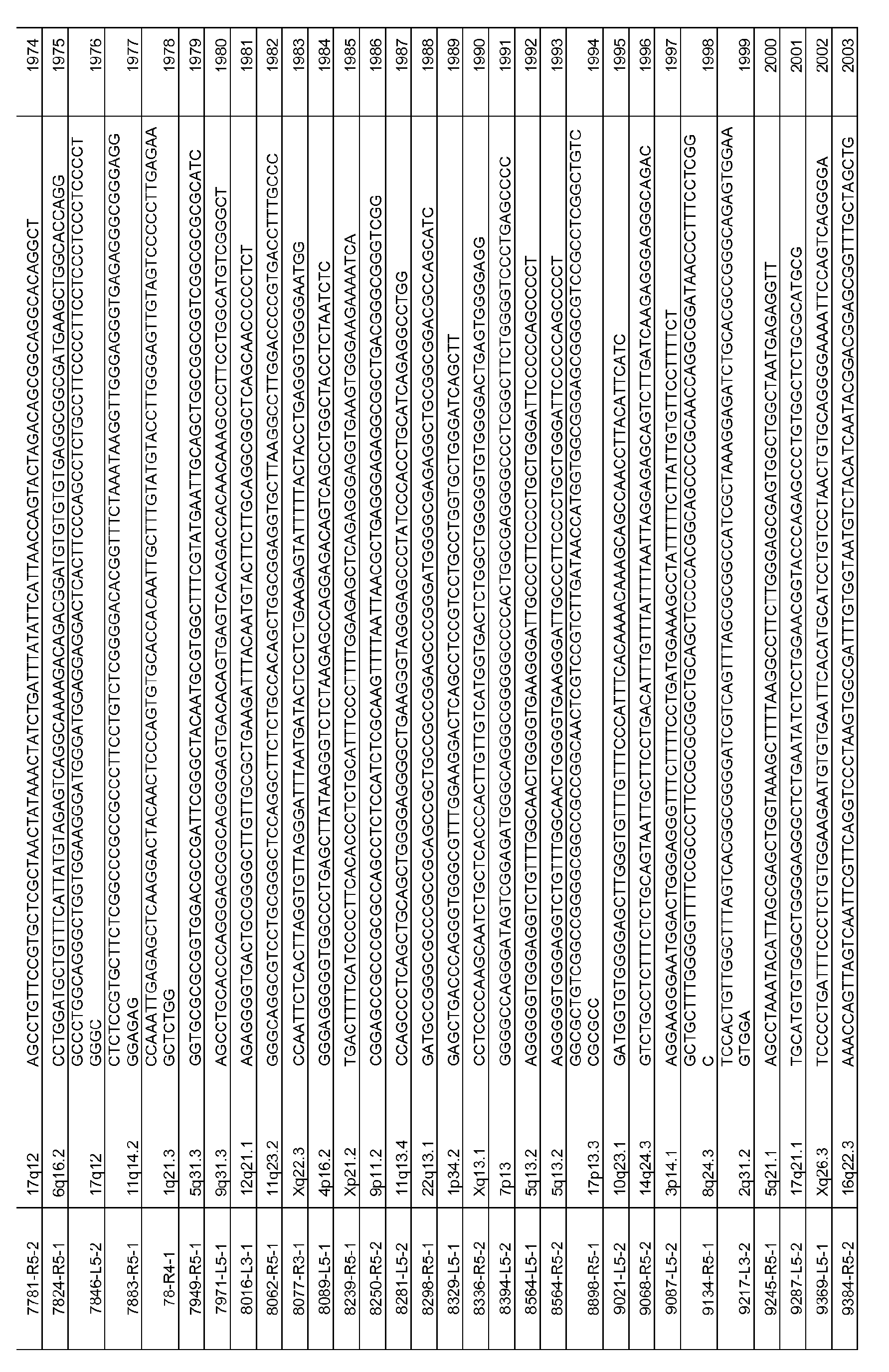

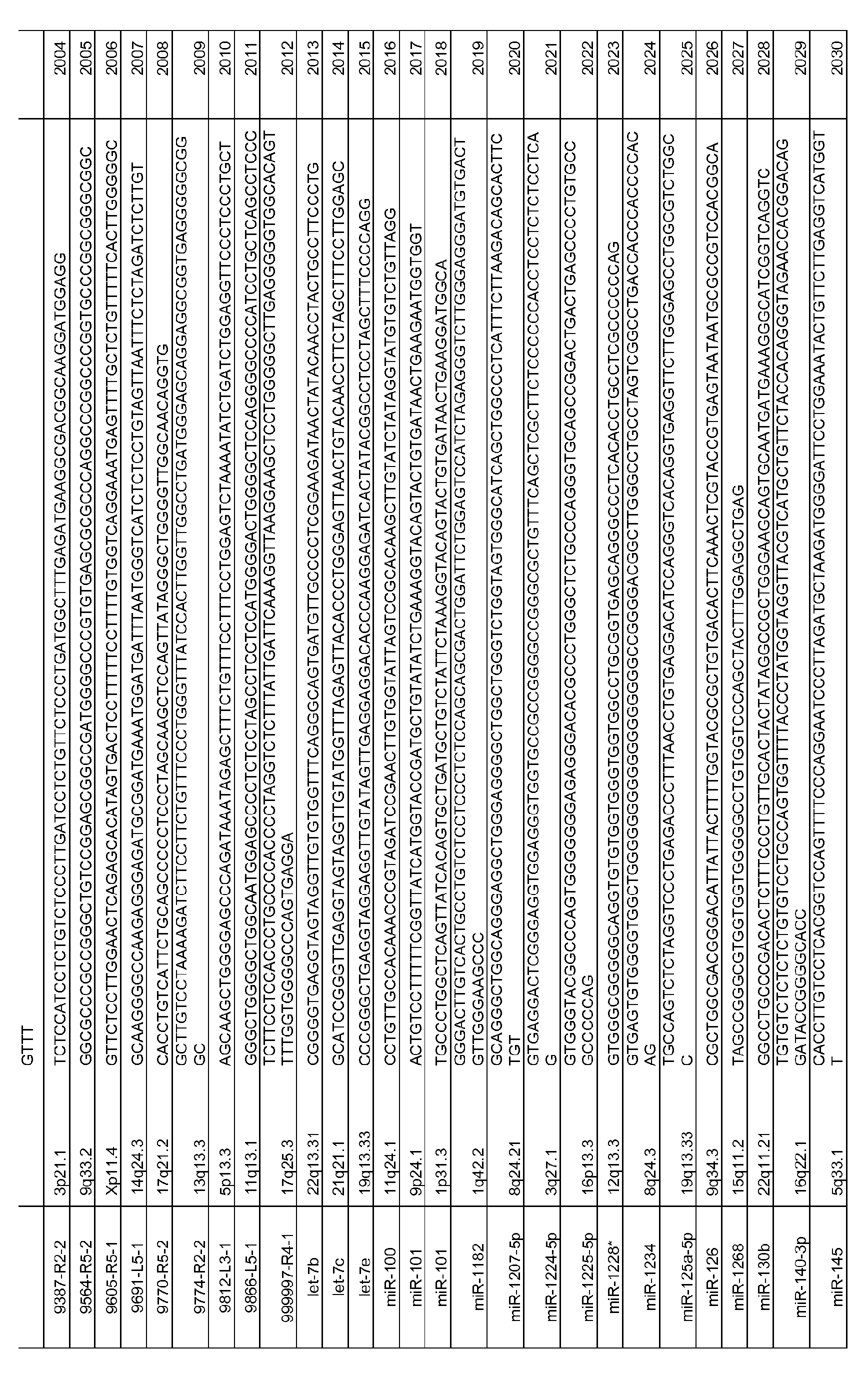

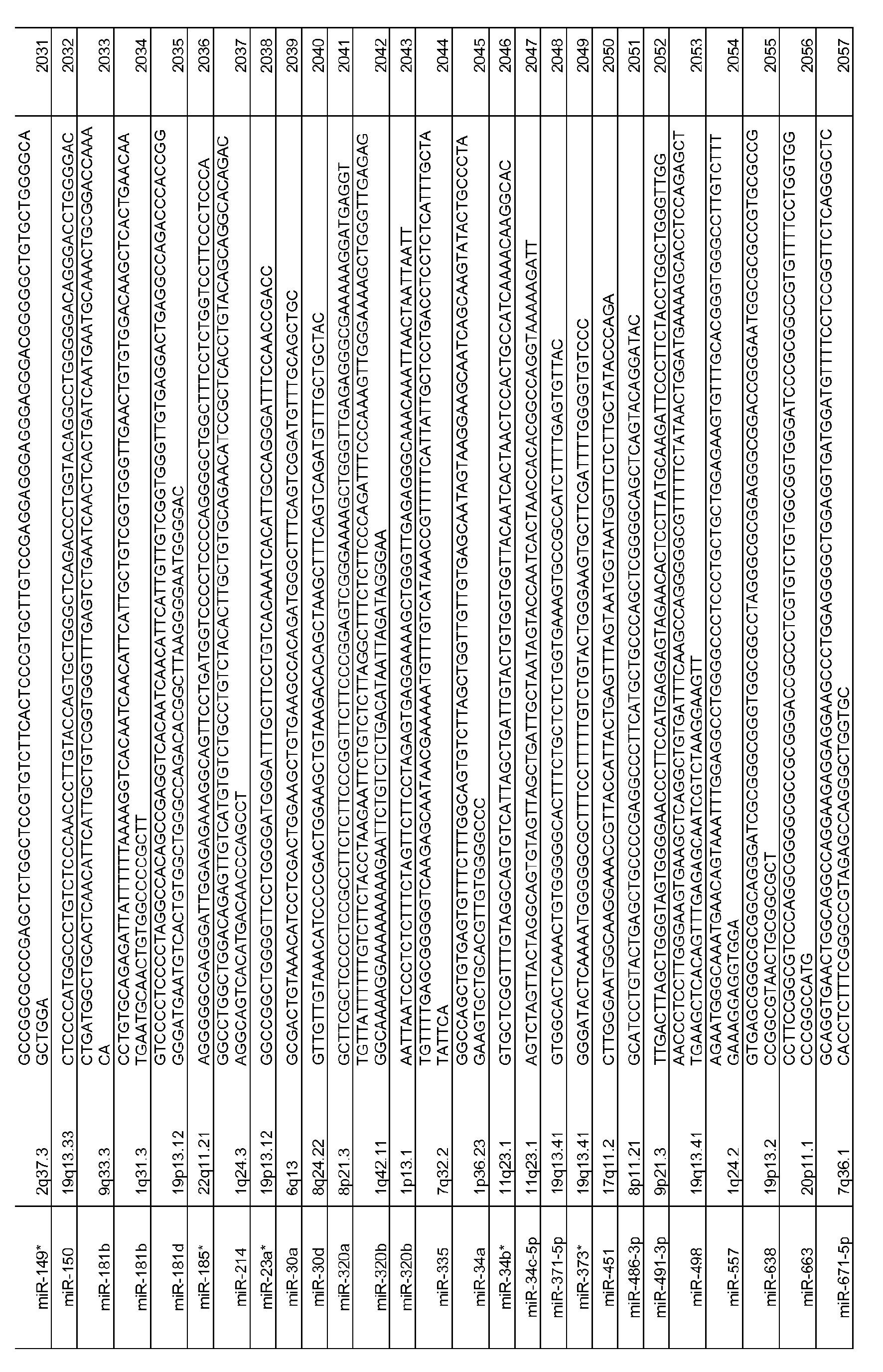

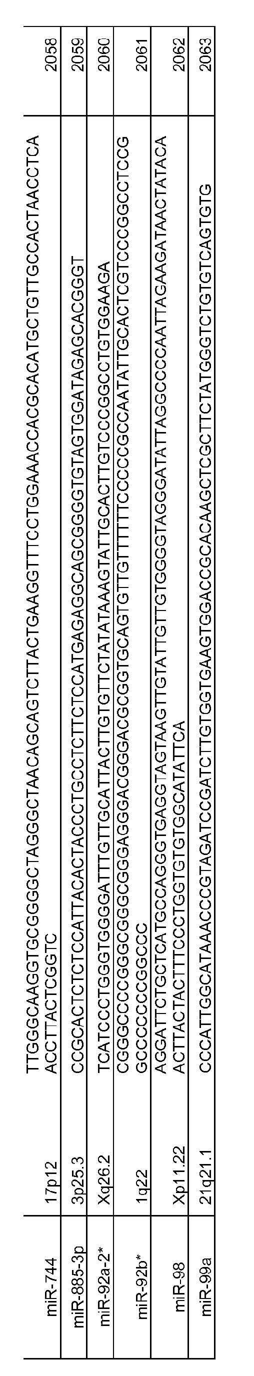

### 5.5 Example 5: Analysis of target RNA from Additional Lung Cancer Cell Lines

Total RNA was prepared from the eight cell lines (seven lung cancer cell lines and one normal lung cell line) listed in Table 26. Cell lines were purchased from LGC promochem (ATCC) and cultured according to ATCC guidelines.

**Table 26: Normal lung and lung tumor cell lines**

| **Cell line** | **ATCC number** | **Cancer type** |
|---|---|---|
| H520 | HTB-182 | squamous cell carcinoma |
| H69 | HTB-119 | small cell lung cancer |
| H146 | HTB-173 | small cell lung cancer |
| H23 | CRL-5800 | adenocarcinoma; non-small cell lung cancer |
| NHBE | CC-2540 | normal Bronchial epithelial cells |
| H187 | CRL-5804 | small cell lung cancer |
| calu 1 | HTB-54 | epidermoid carcinoma |
| calu 3 | HTB-55 | anaplastic carcinoma |

Microarray data acquisition and analysis were conducted as described in Example 4.

Total RNA from normal bronchial epithelial cells (NHBE) was used as a control.

The microarray data was analyzed to identify target RNAs that were present at increased levels in at least four of the cell lines tested. Table 27 shows the target RNAs, the probe sequences used to detect the target RNAs, and the fold-increase in each of the cell lines, relative to NHBE cells.

The microarray data was further analyzed to identify target RNAs that were present at increased levels in two or three of the cell lines, but for which the average increase in the level of the target RNA relative to NHBE cells was at least 5-fold. Table 28 shows the target RNAs, the probe sequences used to detect the target RNAs, and the fold-change in each of the cell lines, relative to NHBE cells. Table 29 shows the pre-microRNA sequences and chromosomal location of the pre-microRNA gene for each of the target RNAs in Tables 27 and 28.

The microarray data was then analyzed to identify target RNAs that were present at at least 5-fold decreased levels in at least four of the cell lines. Table 30 shows the target RNAs, the probe sequences used to detect the target RNAs, and the fold-change in each of the cell lines relative to NHBE cells. Table 30 also shows the number of cell lines in which the same target RNA is present at at least 2-fold increased levels. Table 31 shows the pre-microRNA sequences and chromosomal location of the pre-microRNA gene for each of the target RNAs in Table 30.

**Table 27: Target RNAs present at increased levels in at least four cell lines**

| **Gene** | **Probe sequence** | **SEQ ID NO** | **Calu1 FC** | **calu3 FC** | **H146 FC** | **H187 FC** | **H23 FC** | **H520 FC** | **H69 FC** |
|---|---|---|---|---|---|---|---|---|---|
| 10083-L5-1 | CCCTCTTCCTTTCTACCCCCTCTCTCCACCC | 2064 | -1.00 | -1.00 | 6.68 | 2.01 | 4.16 | 4.70 | 19.19 |
| 10333-L5-1 | TGCCCTGCCCACCCCCTCCCCTGCCCCG | 2065 | -8.68 | -8.68 | 1.38 | 2.28 | 2.04 | 2.09 | 4.83 |
| 10398-R5-1 | TTGCTACCCGTTTCCCCTCCTCTTGAAGTTGCTT | 2066 | -1.00 | -1.00 | 3.24 | 1.35 | 20.09 | 10.86 | 13.61 |
| 13122-L5-1 | TTAGGAAATTCCATCTCACCTGCTCCAGTCC | 2067 | -1.77 | 1.01 | 4.68 | 1.44 | 4.33 | 3.39 | 84.80 |
| 13124-L5-1 | TCCTCCCCTCCGCGAAAGCCTAAACTTACCCCTCA | 2068 | -1.00 | -1.00 | 5.38 | -1.00 | 5.68 | 2.02 | 19.83 |
| 13163-R5-4 | ACCTCCCCTCCTTCTCTCCCTCCCACAAGACCAA | 2069 | -1.18 | -2.47 | 4.54 | 3.26 | 6.06 | 2.05 | 20.32 |
| 13185-L5-3 | TTCTGTTTTTTTTCTTCCCTCTCTCCCCTCTT | 2070 | -1.99 | -1.99 | 5.80 | 3.21 | 12.07 | 3.25 | 16.10 |
| 13195-L5-3 | TCTATATTCCCCCTCCATGACCATTTGTATTAG | 2071 | -1.00 | -1.00 | 2.03 | -1.00 | 23.44 | 12.07 | 6.32 |
| 13219-L5-1 | CTCTGACTCCCTCACTCAGTCTCTCTGCTCCAGC | 2072 | -1.00 | 1.77 | 7.06 | 2.04 | 9.36 | 3.89 | 89.50 |
| 13247-L5-2 | CTTCCCCTCCCGACCTCAGAGCCCTGTTCTTCCT | 2073 | -1.00 | -1.00 | 2.45 | 1.33 | 13.02 | 3.99 | 10.40 |
| 13334-L5-2 | AGGACTCCCCTCCCCTCCCACTGTGCCCTGTC | 2074 | -2.05 | -2.05 | 3.45 | -1.05 | 7.61 | 2.31 | 8.48 |
| 13335-L5-3 | ACCTCAGCCTCCACTGCCCTCCTGCCGCATCCTAT | 2075 | -2.19 | -2.19 | 4.64 | 5.53 | 2.19 | -1.17 | 7.44 |
| 25-R5-2 | TTCTGCTTTCCCAGAGCCTCACCCCCTCTTTT | 2076 | -1.00 | -1.00 | 3.62 | -1.00 | 2.55 | 2.18 | 4.29 |
| 266-R5-2 | GTCGCCCCCTCCCCCAAGTTGAGACTTGCA | 2077 | -2.31 | -2.31 | 3.23 | 1.50 | 9.97 | 5.43 | 10.49 |
| 3249-L5-1 | GCCGCCATCCCCGGAGCCGCCGCCGCCGCCGCC | 2078 | 1.78 | 1.99 | 3.86 | 81.53 | 5.02 | 2.61 | 69.65 |
| 3371-L5-2 | CTTTCCTTTCCTCCCCTCCACACCCCATGACTCCC | 2079 | -1.00 | -1.00 | 4.01 | 1.42 | 12.35 | 5.21 | 16.88 |
| 3744-R5-1 | CTTCTCCTTCCTCCCTGCTCCCCTCCCACTAATGCCAAAT | 2080 | -2.39 | 5.28 | 7.66 | 12.05 | 14.66 | 5.31 | 107.49 |
| 4855-R5-1 | CGGGTCTCCCGCTTCCCCCTCCTGCTCCAAGG | 2081 | -5.66 | -8.69 | 3.89 | 3.18 | 3.61 | 2.49 | 9.85 |
| 5107-L5-1 | AACTCCCCTTTGACCCCCCAGTACAAACTG | 2082 | -1.00 | -1.00 | 5.89 | -1.00 | 6.60 | 3.56 | 35.94 |
| 6235-R5-2 | TCTGCTCCAAAAATCCATTTAATATATTGT | 2083 | -1.00 | 1.77 | 10.45 | 7.18 | 13.80 | 16.55 | 207.50 |
| 6474-L5-1 | CCCTAATTAAAGCCATCCCCTCTTCCCCCTTCACC | 2084 | -1.00 | -1.00 | 7.06 | 2.24 | 8.81 | 2.57 | 8.35 |
| 6490-R5-3 | CCCCATCCCCCATATGACGCTTCCCCCTCCTAAC | 2085 | -2.09 | -2.09 | 2.58 | -2.09 | 9.85 | 6.98 | 8.49 |
| 6681-R2-1 | CCTGTTTTCTCCCCTCTCTCTCTGCCCCTCC | 2086 | -2.21 | -2.21 | 4.83 | 2.74 | 6.51 | 4.82 | 19.47 |
| 7572-R5-2 | ATCACCCTTCCCCCTCCCAAATAAAGCCAAA | 2087 | 1.39 | -1.00 | 7.08 | 4.90 | 27.15 | 15.82 | 29.39 |
| 7883-R5-1 | CTCTCCCCTCCCGCCCTCTCACCCTCCCAACCTTATTTAGAAAC | 2088 | -4.54 | -2.60 | 2.34 | 3.18 | 3.80 | 1.61 | 6.57 |
| 8004-R3-2 | GGAACTGCTTCTCCTTGCTCCAGTCATTGAAG | 2089 | -1.00 | 2.87 | 4.28 | 1.99 | 9.46 | 6.50 | 113.48 |
| 836-R4-1 | AAATAATCATTCCAAATGGTTCTCCCTGCTATGATTCAC | 2090 | -1.00 | -1.00 | 3.82 | -1.00 | 2.16 | 2.67 | 104.03 |
| 9594-R5-1 | ACTTAGACTTCCTTCCCACTCCCTGCATCCT | 2091 | -1.81 | 1.65 | 3.62 | 1.98 | 6.80 | 5.25 | 59.46 |
| let-7c | AACCATACAACCTACTACCTCA | 2092 | 3.65 | 24.65 | 2.65 | 1.35 | 3.24 | 5.11 | 15.37 |
| let-7d | AACTATGCAACCTACTACCTCT | 2093 | 2.02 | 13.30 | 1.78 | -1.94 | 1.37 | 4.41 | 8.00 |
| miR-103 | TCATAGCCCTGTACAATGCTGCT | 2094 | -1.00 | -1.00 | 2.97 | 3.07 | 1.27 | 3.15 | 3.05 |
| miR-106a | CTACCTGCACTGTAAGCACTTTT | 2095 | -1.00 | -1.00 | 13.51 | 23.92 | 4.32 | 6.14 | 17.48 |
| miR-107 | TGATAGCCCTGTACAATGCTGCT | 2096 | -1.00 | 1.59 | 4.64 | 2.94 | 1.85 | 7.68 | 8.76 |
| miR-16 | CGCCAATATTTACGTGCTGCTA | 2097 | -1.00 | 1.78 | 6.76 | 13.51 | 3.05 | 1.91 | 2.91 |
| miR-17 | CTACCTGCACTGTAAGCACTTTG | 2098 | -1.00 | -1.00 | 13.68 | 23.68 | 3.51 | 8.86 | 18.29 |
| miR-200b | TCATCATTACCAGGCAGTATTA | 2099 | -1.00 | 6.04 | 6.46 | 36.53 | -1.00 | 7.67 | 2.72 |
| miR-200c | TCCATCATTACCCGGCAGTATTA | 2100 | -3.97 | 5.00 | 4.38 | 7.25 | -3.97 | 1.02 | 2.02 |
| miR-20a | CTACCTGCACTATAAGCACTTTA | 2101 | 512.00 | -1.00 | 6.67 | 5.00 | 1.25 | 2.43 | 5.02 |
| miR-20b | CTACCTGCACTATGAGCACTTTG | 2102 | -1.00 | -1.00 | 15.49 | 7.38 | 1.90 | 2.68 | 8.04 |
| miR-298 | TGGGAGAACCTCCCTGCTTCTGCT | 2103 | -1.00 | -1.00 | 4.26 | -1.00 | 3.08 | 2.00 | 49.37 |
| miR-320a | TCGCCCTCTCAACCCAGCTTTT | 2104 | -1.00 | -1.00 | 5.63 | 6.24 | 4.27 | -1.00 | 9.68 |
| miR-320b | TTGCCCTCTCAACCCAGCTTTT | 2105 | -1.00 | -1.00 | 3.91 | 3.97 | 3.28 | -1.00 | 3.98 |
| miR-320d | TCCTCTCAACCCAGCTTTT | 2106 | -1.00 | -1.00 | 2.33 | 2.66 | 2.54 | -1.00 | 3.22 |

**Table 28: target RNAs present at increased levels in two or three cell lines, but with an average increase of at least 5-fold relative to normal levels**

| **Gene** | **Probe sequence** | **SEQ ID NO** | **Calu1 FC** | **calu3 FC** | **H146 FC** | **H187 FC** | **H23 FC** | **H520 FC** | **H69 FC** |
|---|---|---|---|---|---|---|---|---|---|
| 10233-R5-1 | ACCCTCTCCCCTTGGATCTGGAGCAGCAGGCAGTAGA | 2107 | -2.04 | -2.04 | 3.75 | 1.08 | -1.08 | -2.04 | 10.41 |
| 10335-L5-2 | CCACTCCCCTCCTTTTTAATTAGAAAGCAC | 2108 | -1.00 | -1.00 | 2.43 | -1.00 | 2.56 | 1.35 | 12.59 |
| 12184-L4-1 | GACCTCAGCGTGCCCCCTTTCAACCACAGACGAATATTGTGTACAA | 2109 | -1.98 | -1.98 | 2.91 | -1.03 | -1.63 | -1.51 | 9.45 |
| 12223-L5-1 | GACATCAGACAGAGTTGTTTCTTCTCCCTCTA | 2110 | -4.85 | -4.85 | 2.43 | -1.45 | -4.85 | -3.28 | 7.69 |
| 12695-R5-2 | CCCCCACCAAACCTATTCCCGCATCCTCCCCGG | 2111 | -2.87 | -2.87 | 2.64 | 1.50 | -1.43 | -2.87 | 7.70 |
| 12721-R5-2 | TCCACTCTGACCAGCTCACCCTCACTGGACTA | 2112 | -1.00 | -1.00 | 2.69 | -1.00 | -1.00 | -1.00 | 19.11 |
| 12729-R5-1 | CGACGAAAATGCAATTGTGTGCCTTCTCCCTCC | 2113 | -1.00 | -1.00 | 2.17 | -1.00 | -1.00 | -1.00 | 16.72 |
| 12888-L5-2 | TTTCCTACATTGTATGGTTCTCCCAGCTCCT | 2114 | -1.00 | -1.00 | 1.29 | -1.00 | 2.69 | 1.51 | 85.37 |
| 12911-L5-1 | CAACTGACACCTCCTTCCTTTTCCTCCTTCGT | 2115 | -1.00 | -1.00 | 7.10 | 2.04 | -1.00 | -1.00 | 10.36 |
| 12917-R5-2 | GGACCTATGGGCCCTTCCCTTCCCCCAACATTG | 2116 | -1.00 | -1.00 | 4.25 | -1.00 | 2.62 | -1.00 | 10.54 |
| 12947-R5-3 | GCCCCCTCCATAGAGAGAGGCCCCAGGGGAGTGA | 2117 | -1.00 | -1.00 | 1.37 | -1.00 | -1.00 | 4.78 | 8.65 |
| 12992-L5-1 | CATACCTGCTTCCCTCCACCCCCATCTCTA | 2118 | -1.00 | -1.00 | 5.12 | 1.36 | 1.24 | -1.00 | 10.93 |
| 13001-L5-1 | TTCCTAGATACCACTCCCAGCTCCA | 2119 | -1.00 | -1.00 | 2.06 | -1.00 | 1.28 | 2.46 | 11.10 |
| 13070-R5-3 | CTGTCTCCTCTCCCCAGTCCAAAGGACCTAATGC | 2120 | -1.00 | -1.00 | 5.17 | 1.43 | 1.89 | -1.00 | 13.57 |
| 13115-L5-3 | ACCCCCTCCAGGGCCGACCACCCCAACCCAAAC | 2121 | -1.00 | -1.00 | 1.59 | -1.00 | 10.07 | 13.09 | 12.60 |
| 13124-L5-2 | GCTCCATGTCTCCTCCCCTCCGCGAAAGCCTAAAC | 2122 | -2.76 | -2.76 | 4.56 | -1.18 | 7.69 | -1.73 | 20.66 |
| 13195-L5-2 | CCCCCTCCATGACCATTTGTATTAGTATCTTTT | 2123 | -1.00 | -1.00 | -1.00 | -1.00 | 12.25 | 13.17 | 10.79 |
| 13237-L5-4 | ACCCCCTCCAGGGCCGACCACCCCAACCCAAACAA | 2124 | -1.00 | -1.00 | 1.35 | -1.00 | 8.49 | 13.59 | 14.13 |
| 13274-L5-3 | CCTTCTCTTCTCCCGTGCTCCCACCCTCCCTCAGGG | 2125 | -4.16 | -3.37 | 1.81 | 1.66 | 3.05 | 2.03 | 18.48 |
| 13278-R5-2 | TCAGACTGTGCTCTCTCCATTCCCCAGGACTCC | 2126 | -1.00 | -1.00 | 4.29 | 1.86 | -1.00 | -1.00 | 22.61 |
| 13287-L5-4 | ACTGCAGAGGAGCCACCCCACCCTCCTCCCAAG | 2127 | -1.00 | -1.00 | 4.08 | -1.00 | 4.47 | -1.00 | 8.90 |
| 13343-L5-4 | CTCCCGGCCCCTCACCCTTCCCTGGGCCCTCCACCC | 2128 | -1.00 | -1.00 | 4.67 | 1.44 | -1.00 | 1.34 | 10.16 |
| 13357-L5-4 | AGCGGACCTTCTCCCCACACCTCCCTGCAGCCTC | 2129 | -2.19 | -2.19 | 3.44 | 1.44 | -1.81 | -2.19 | 15.06 |
| 13358-L5-2 | CCTCCTCACCACCCCCTCCACACTCCTGGGGAAGT | 2130 | -1.00 | -1.00 | 1.41 | -1.00 | 5.44 | 10.83 | 5.16 |
| 13366-R5-3 | CAGGCCTCACCCCAGTGCCCTCTCCTATTCCCAC | 2131 | -1.00 | -1.00 | 2.14 | 1.46 | -1.00 | -1.00 | 19.41 |
| 13395-R5-2 | CGTCAGACTGTGCTCTCTCCATTCCCCAGGACTC | 2132 | -1.00 | -1.00 | 4.13 | -1.00 | -1.00 | -1.00 | 25.57 |
| 13403-L5-2 | CCCGTGTCCCCCCAACCTGGGGCCAGGCCCA | 2133 | -1.00 | -1.00 | 1.56 | -1.00 | 4.85 | 1.85 | 9.80 |
| 13467-L5-1 | GTGACAGTCAGACCCTCCTTGCTCCAAGTCAAA | 2134 | -1.00 | -1.00 | 2.40 | 1.94 | 9.75 | -1.00 | 105.32 |
| 13470-R5-1 | TCCCACCCTCTCCACCTCAGGGACCAGAATCCT | 2135 | -1.00 | -1.00 | 5.33 | 1.97 | -1.00 | -1.00 | 12.11 |
| 13472-L5-1 | CTCAGAGCACTGTGTGTGACGTCCTCCTGTCTGTC | 2136 | 9.44 | 30.30 | -1.00 | -1.00 | -1.00 | -1.00 | -1.00 |
| 13508-L5-3 | GACAGGCTGCTCTCCTTCTGCTCTTAGCTTCCT | 2137 | -1.00 | -1.00 | 2.96 | -1.00 | -1.00 | -1.00 | 18.27 |
| 13530-L5-3 | CCTTCCTCCTTCCCCTGGGTCCCCCTAAGTTCTCC | 2138 | -1.00 | -1.00 | 8.37 | 5.70 | 1.56 | 1.34 | 16.94 |
| 13546-R5-2 | TGCCTCCACCCTCTCCATCCCGTCACCCTCCCA | 2139 | -2.75 | -2.75 | 3.08 | 6.44 | -1.36 | -2.04 | 8.01 |
| 3875-R5-2 | GACTGATTCAACCTCTCTCTCCCACTTTA | 2140 | -1.00 | -1.00 | 3.37 | 1.56 | -1.00 | -1.00 | 8.56 |
| 3923-R5-1 | TCACAAAGGATCTCCTTCATCCCTCTCCAG | 2141 | -1.00 | -1.00 | 1.18 | 3.17 | -1.00 | -1.00 | 8.48 |
| 5108-R5-2 | CCCTCTCCTCCCACACCGTCACTCACAATAACCC | 2142 | -1.94 | -1.94 | 4.49 | 1.77 | 1.53 | -1.94 | 12.32 |
| 5192-L3-2 | CATTTTTCCCCTTCCTTCCTCTATATCAGCAA | 2143 | -1.00 | -1.00 | 1.79 | -1.00 | 24.42 | 13.63 | 4.95 |
| 5306_B-R4-1 | TCCACTCTGACCAGCTCACCCTCACTGGACTATGACCCC | 2144 | -1.00 | -1.00 | 3.27 | -1.00 | -1.00 | -1.00 | 16.02 |
| 5633-L5-1 | CACTCCCTCCCTGGCTCCTGGAGACCCTCTCCAGGACTTG | 2145 | -1.00 | -1.00 | 2.22 | 2.39 | 1.27 | -1.00 | 45.28 |
| 5638-R5-2 | GGCCCTCCCCCTGCCTGTGATAGGCTG | 2146 | -1.57 | -2.40 | 3.23 | -1.15 | 5.85 | -1.33 | 8.54 |
| 5723-R5-1 | CCTCCTCCCCTTTCTCCAGCAGTAGCCTTCTTAA | 2147 | -1.00 | -1.00 | 4.87 | 1.40 | 3.97 | -1.00 | 11.99 |
| 5735-L5-1 | TGCTGTTTCCTCTGGCCTCAAGCCTGTGGGGG | 2148 | -1.00 | -1.00 | 1.18 | 3.00 | -1.00 | -1.00 | 14.30 |
| 6183-R5-1 | ACTTTTCTTAATGACTTTCCCCTCCTTAAG | 2149 | -1.00 | -1.00 | 1.75 | -1.00 | 18.55 | 12.12 | 10.01 |
| 6216-R5-1 | TACTCCCGCCGTTTACCCGTGCTTCATTGAA | 2150 | -3.03 | -3.03 | 2.63 | 13.19 | 1.06 | -3.03 | -1.10 |
| 6490-R5-3 | CCCCATCCCCCATATGACGCTTCCCCCTCCTAAC | 2151 | -3.21 | -4.42 | -2.06 | -1.29 | 5.92 | 3.87 | 5.51 |
| 6496-R5-2 | CCCCCTCCCCCACCCACCACTTCCCCTAGA | 2152 | -2.49 | -5.45 | 1.82 | -1.65 | 4.91 | -1.83 | 10.29 |
| 6584-L5-1 | GTCGGCCCTGCCTCCTCCTCCTCTCACCAAGC | 2153 | -1.00 | -1.00 | 3.33 | -1.00 | -1.00 | -1.00 | 12.02 |
| 6647-R5-1 | CCCCAGCTGGAGAATTTTTCCCCTCATTA | 2154 | -1.00 | -1.00 | -1.00 | -1.00 | 12.90 | 5.66 | 6.72 |
| 6752-R5-2 | CCCTCCTTTCCCCACCTCAGTCGGGCCACTGCT | 2155 | -1.00 | -1.00 | 2.70 | 1.92 | 5.08 | 1.27 | 9.54 |
| 6803-R5-2 | GCTCCCTCTCTGGTTGGACCTCACCCAAA | 2156 | -2.35 | -2.35 | 5.77 | 3.27 | -2.35 | -2.35 | 13.06 |
| 6930-R5-1 | TTAATCCTTCTCTCCCCTCTGATCTTGCAG | 2157 | -1.00 | -1.00 | 7.92 | 1.50 | 2.40 | -1.00 | 28.87 |
| 7113-R5-1 | CGCCTAATTAGCCCCCCTGCTCCGGAGGCCTCACC | 2158 | 1.06 | -2.16 | 2.60 | -2.16 | 1.12 | -1.18 | 12.56 |
| 7192-R5-1 | TTCCCCCTCTAAGTCTGCCTGGGCTCTTGGCACTG | 2159 | -1.00 | -1.00 | -1.00 | -1.00 | 5.96 | 13.37 | 21.66 |
| 7384-R3-1 | CTCGCAAAGGATCTCCTTCATCCCTCCCCA | 2160 | -1.00 | -1.00 | 1.30 | -1.00 | 2.58 | -1.00 | 9.23 |
| 7571-L5-1 | CAGGGCTAACAGGGCTCCCCCACCCCTAAG | 2161 | -2.26 | -2.26 | 4.35 | -2.26 | -1.81 | -2.26 | 6.92 |
| 836-R5-2 | AAATAATCATTCCAAATGGTTCTCCCTGCTAT | 2162 | -1.00 | -1.00 | 3.09 | -1.00 | 2.40 | 1.86 | 51.66 |
| 8433_C-R4-1 | AAACCAAAAAAAAAAAATTAAAAAGCGACGAAAATGCAATTGTGTGCCTTCTCCCTCC | 2163 | -1.00 | -1.00 | 5.09 | -1.00 | -1.00 | -1.00 | 16.86 |
| 8433-L3-1 | AAATGGCTCCTTTCCCCTTTCCCTCCACCG | 2164 | -1.00 | -1.00 | 6.04 | 1.95 | 5.50 | 1.32 | 11.98 |
| 8724-R5-2 | GGCCAAGCTTGGAACCTCTCCCTGCCAGCA | 2165 | -1.89 | -1.89 | 2.12 | 1.04 | -1.89 | -1.89 | 7.98 |
| 8808-R5-1 | GACCCCTTTCTCCCAGCCTGTTTCTGCAA | 2166 | -1.95 | -1.95 | 2.65 | 1.01 | -1.95 | -1.95 | 9.58 |
| 8832-R5-1 | TGGAGTACCACCTGTTTTTCCCCCACTT | 2167 | -1.00 | -1.00 | -1.00 | -1.00 | 5.05 | 1.41 | 21.96 |
| 9349-R5-2 | GAACACAGTGATGCAGAGGACTTCCTGCTCCA | 2168 | -1.00 | -1.00 | 1.84 | -1.00 | 1.99 | 1.61 | 85.75 |
| let-7b | AACCACACAACCTACTACCTCA | 2169 | -1.24 | 11.28 | 1.29 | 1.73 | -1.24 | 2.39 | 6.09 |
| let-7e | AACTATACAACCTCCTACCTCA | 2170 | -1.27 | 12.32 | 1.35 | -1.94 | 1.66 | 1.71 | 7.50 |
| miR-1268 | CCCCCACCACCACGCCCG | 2171 | -1.00 | -1.00 | 2.84 | -1.00 | -1.00 | 1.37 | 11.23 |
| miR-1274b | TGGCGCCCGAACAGGGA | 2172 | 6.00 | -1.00 | 9.59 | -1.00 | 1.21 | -1.00 | -1.00 |
| miR-15b | TGTAAACCATGATGTGCTGCTA | 2173 | -1.00 | -1.00 | 2.40 | 1.66 | -1.00 | 8.53 | -1.00 |
| miR-181a | ACTCACCGACAGCGTTGAATGTT | 2174 | -1.00 | 4.65 | 1.33 | -1.00 | 1.20 | -1.00 | 6.04 |
| miR-198 | GAACCTATCTCCCCTCTGGACC | 2175 | -1.00 | -1.00 | 3.07 | -1.00 | -1.00 | -1.00 | 17.81 |
| miR-222 | ACCCAGTAGCCAGATGTAGCT | 2176 | 2.68 | 9.77 | -1.96 | -1.96 | -1.00 | -1.96 | -1.96 |
| miR-26a | AGCCTATCCTGGATTACTTGAA | 2177 | -1.00 | 4.43 | 1.73 | 6.73 | 1.20 | -1.00 | 4.27 |
| miR-30d | CTTCCAGTCGGGGATGTTTACA | 2178 | -1.00 | 9.69 | 1.71 | 7.03 | -1.00 | -1.00 | -1.00 |
| miR-720 | TGGAGGCCCCAGCGAGA | 2179 | 5.56 | -1.00 | 4.48 | -1.00 | -1.00 | -1.00 | -1.00 |
| miR-92a | ACAGGCCGGGACAAGTGCAATA | 2180 | -1.00 | -1.00 | 3.74 | 10.88 | -1.00 | -1.00 | 7.26 |
| miR-92b* | CACTGCACCGCGTCCCGTCCCT | 2181 | -1.00 | -1.00 | 3.82 | 1.40 | -1.00 | -1.00 | 10.85 |
| miR-936 | CTGCGATTCCTCCCTCTACTGT | 2182 | -1.00 | -1.00 | 3.62 | 1.32 | 1.90 | 1.69 | 18.17 |
| miR-98 | AACAATACAACTTACTACCTCA | 2183 | -1.00 | 17.16 | 1.88 | -1.00 | -1.00 | 1.89 | 10.57 |

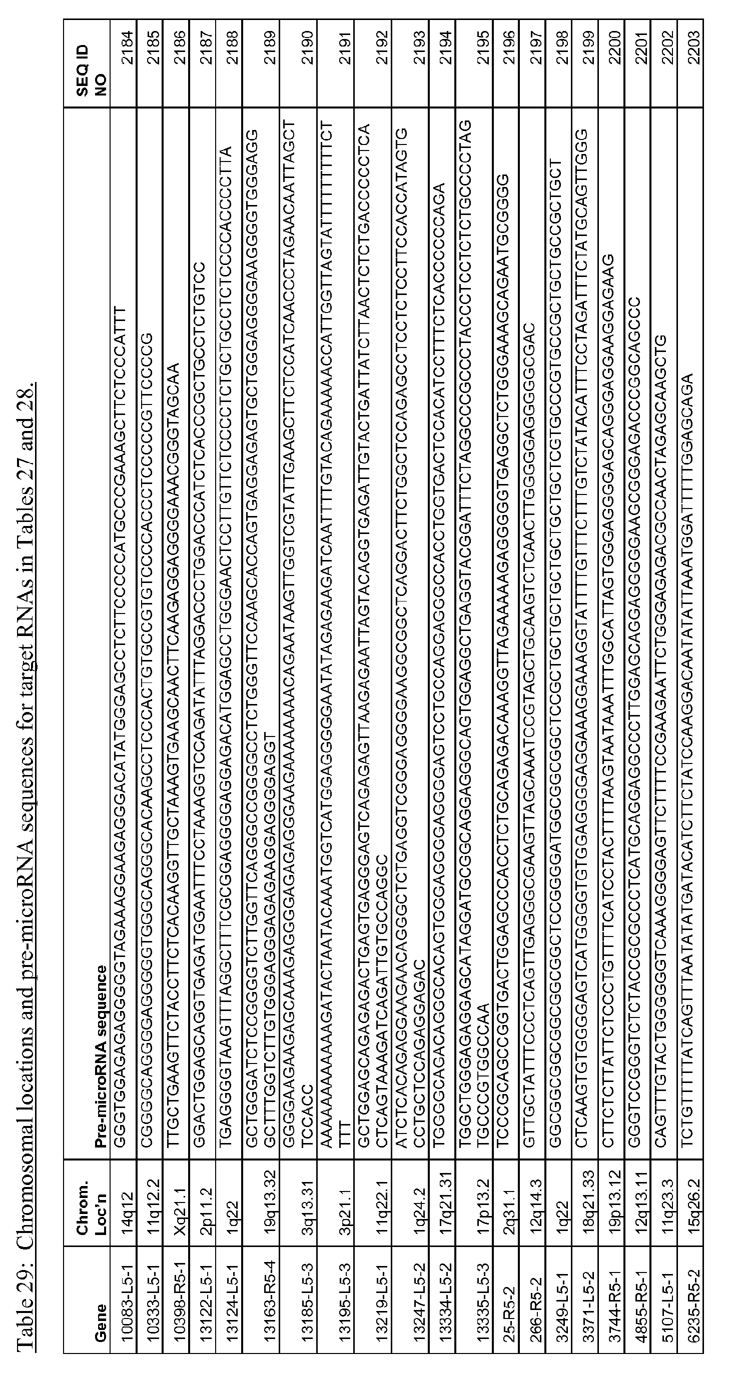

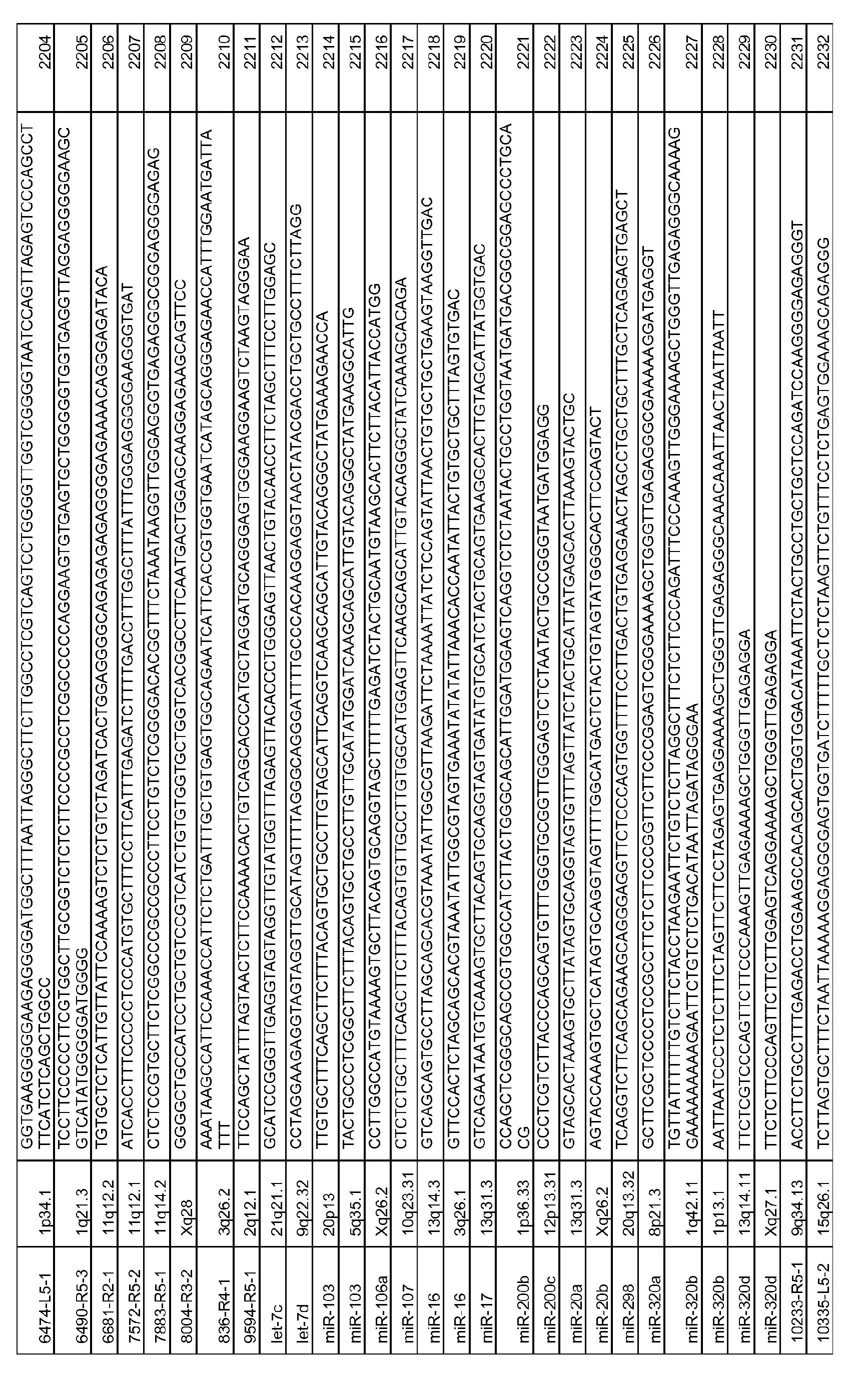

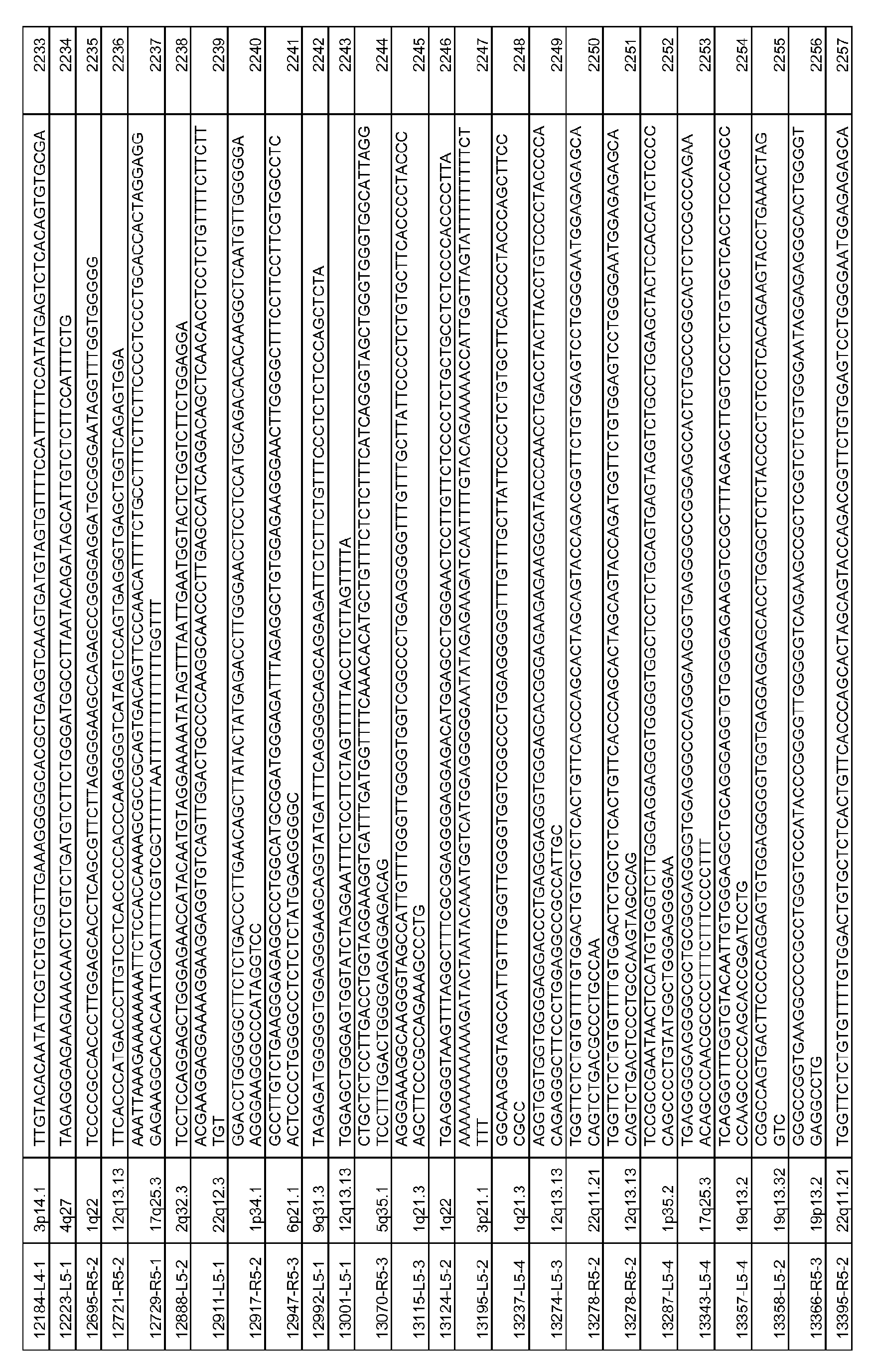

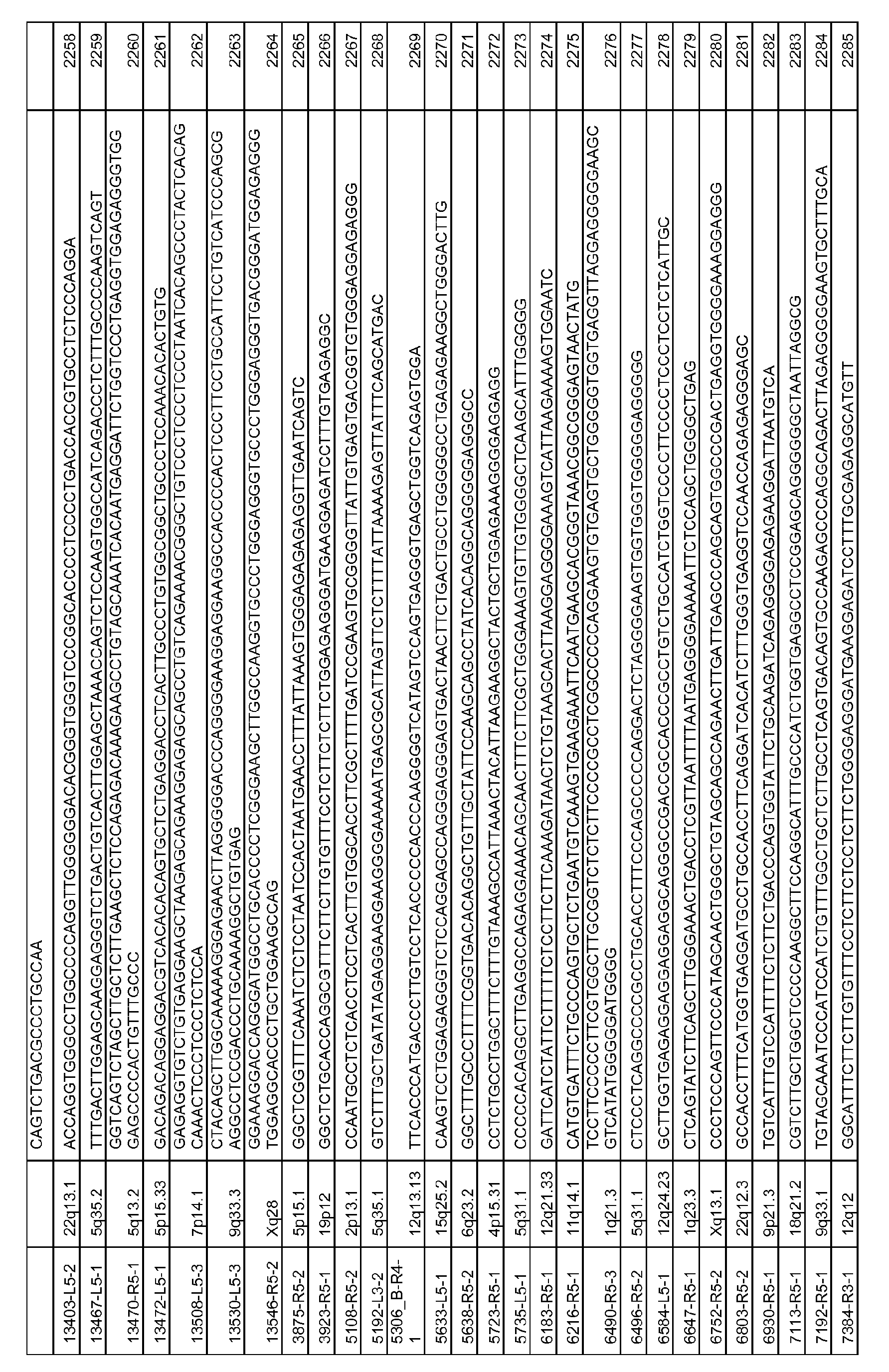

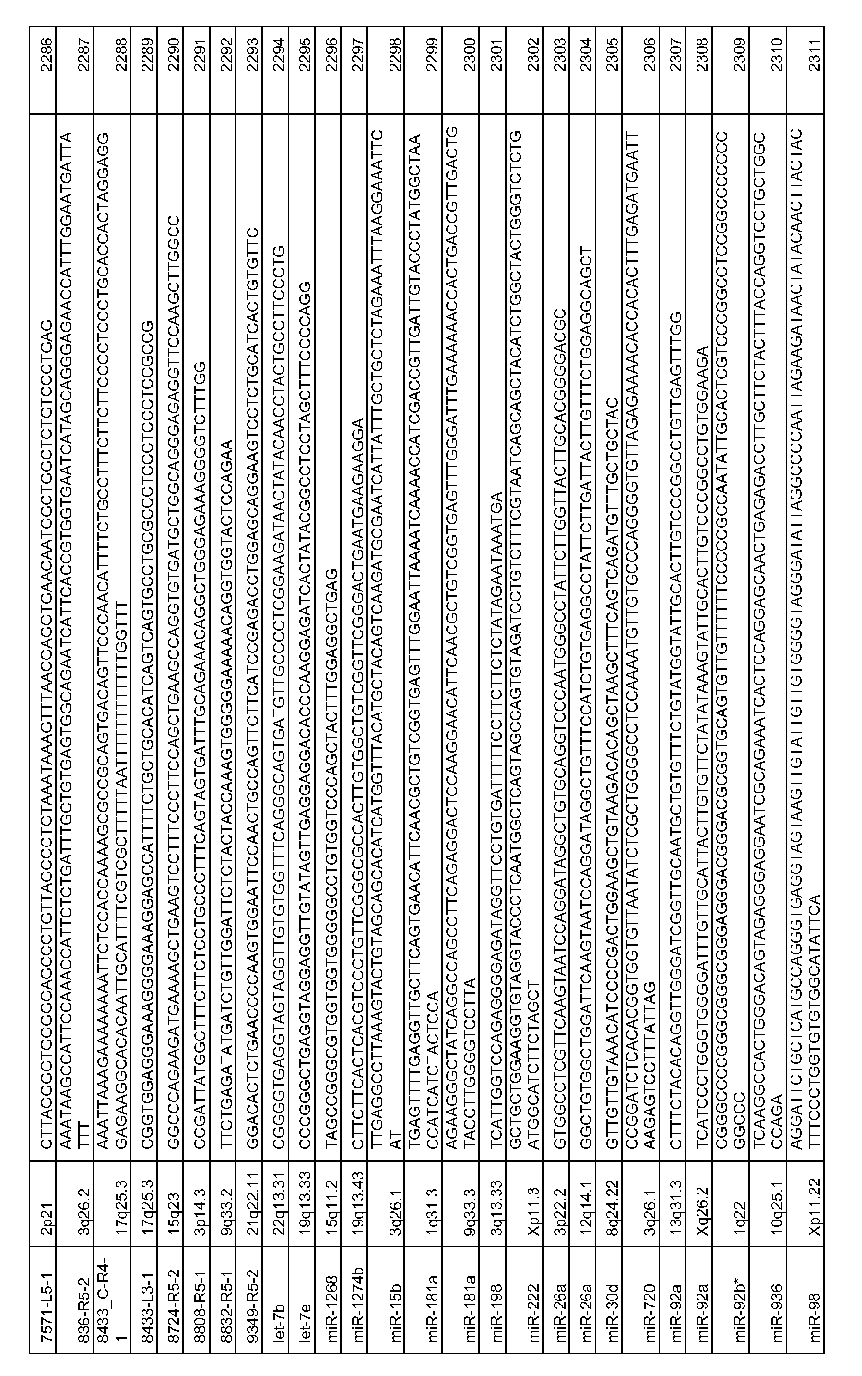

**Table 30: Target RNAs present at at least 5-fold decreased levels in at least four cell lines**

| **Gene Down expressed in Cell lines** | **Probe sequence** | **SEQ ID NO** | **Calu1 FC** | **calu3 FC** | **H146 FC** | **H187 FC** | **H23 FC** | **H520 FC** | **H69 FC** | **Number of cell lines with at least a 2-fold increase** |
|---|---|---|---|---|---|---|---|---|---|---|
| 10010 B-L4-1 | GCCGAGCCCCGCCCCCGCCGGGATGCTGCCCTCCGGAA GGAGGGGCGCTGCCC | 2312 | -16.79 | -8.41 | -4.11 | -2.74 | -7.08 | -22.10 | -2.85 | 0 |
| 10010 D-L4-1 | TGGCGCCCTCCCCCGCCCGGGGCTCAGCCTCTCACCTG | 2313 | -30.70 | -30.70 | -2.08 | -4.60 | -3.76 | -16.38 | -1.22 | 0 |
| 10138-L2-1 | AGCCTGCTCCGCTCTCCCCTCCCACCAGAAAAAGT | 2314 | -7.46 | -7.46 | 1.75 | -1.81 | 1.87 | -2.04 | 7.73 | 1 |
| 10231-R3-1 | TGAACTTTAGCTGGGCCGCCGCCTGTCAGC | 2315 | -29.14 | -29.14 | -3.13 | -4.48 | -29.14 | -29.14 | -5.84 | 0 |
| 10242-R5-1 | GAAGCCCTTCCGCTTCCACCCCGAACAC | 2316 | -25.94 | -25.94 | -14.00 | -25.94 | -25.94 | -25.94 | -8.13 | 0 |
| 10342-R2-2 | CCCGCCGCCGGAGCATCTCGAAGTTAATTAAA | 2317 | -28.71 | -3.68 | -4.01 | -2.19 | -10.93 | -50.54 | -3.01 | 0 |
| 11370-L4-1 | CCTCCGCCCCCACACTGCATCCTTGCCCAGTTTGGCTGCCA TCAGTATTGTCCCCTGAGAACTGGAC | 2318 | -5.20 | -5.20 | 1.61 | -2.49 | -2.57 | -2.80 | 3.46 | 1 |
| 11370-L5-5 | CCCTCCGCCCCCACACTGCATCCTTGCCCAGTTTGG | 2319 | -5.86 | -5.86 | 1.74 | 1.95 | 1.55 | -3.00 | 4.02 | 1 |
| 12223-L4-1 | CCCAGAAGACATCAGACAGAGTTGTTTCTTCTCCCTCTA | 2320 | -5.20 | -5.20 | 2.99 | -2.63 | -5.20 | -5.20 | 5.56 | 2 |
| 12691-R5-1 | CCCCGCCCCTGGCGCGCCCCCGACAGGC | 2321 | -46.43 | -46.43 | -6.02 | -32.36 | -46.43 | -46.43 | -3.50 | 0 |
| 12692-L5-1 | GACCCGGCCCCGCAGCCAGCACCCGGCCACCGCGC | 2322 | -7.57 | -7.57 | 1.00 | -5.34 | -7.57 | -7.57 | 1.21 | 0 |
| 12693-L5-1 | GTCGCGGCCGCCCGGCCCTCCCGGTCCCCTCCCC | 2323 | -34.28 | -34.28 | -4.19 | -6.29 | -2.25 | -14.44 | -1.05 | 0 |
| 12694-R5-1 | TCAGCCCCCAGCGCCCCCCGGAGTTCTTGGA | 2324 | -85.43 | -85.43 | -17.02 | -29.82 | -85.43 | -85.43 | -6.11 | 0 |
| 12695-R5-1 | AAACCTATTCCCGCATCCTCCCCGGCTCTGGC | 2325 | -44.55 | -44.55 | -3.70 | -3.25 | -13.08 | -32.36 | -1.29 | 0 |
| 12696-R5-1 | CCGGGCTCCCCCACCCGCTCCCTGAGC | 2326 | -9.47 | -9.47 | 1.52 | -3.26 | -4.38 | -9.47 | 2.17 | 1 |
| 12696-R5-2 | AACCCGGGCTCCCCCACCCGCTCCCTGA | 2327 | -38.89 | -38.89 | -2.62 | -3.23 | -12.87 | -26.21 | 1.03 | 0 |
| 12697-R5-1 | CCGGTGTGCGCCCCCTCCTACCTCTGCCGGCC | 2328 | -26.89 | -26.89 | -5.74 | -13.37 | -13.11 | -5.78 | -1.74 | 0 |
| 12699-L5-1 | GGCGCCCTGGGCCTCGGCGCCCCGCCCGTCCCAG | 2329 | -9.80 | -9.80 | -2.20 | -4.84 | -9.80 | -9.80 | -1.77 | 0 |
| 12701-L5-1 | AAATCCTCGCCATCCTCCACCCCCAGCCCCGG | 2330 | -24.62 | -24.62 | -2.85 | -2.72 | -5.56 | -12.45 | -1.45 | 0 |
| 12703-L5-3 | AGCCGAGCCCCCGCCCCCGCCGGGATGCTGCCCTC | 2331 | -64.38 | -60.80 | -7.33 | -2.94 | -20.92 | -66.02 | -9.82 | 0 |
| 12704-L5-1 | CCTCCCCCGCCCGGGGCTCAGCCTCTCACCTG | 2332 | -8.28 | -8.28 | 1.19 | -1.16 | -1.79 | -4.47 | 2.96 | 1 |
| 12704-L5-2 | CTGGCGCCCTCCCCCGCCCGGGGCTCAGCCTC | 2333 | -7.28 | -7.28 | -1.14 | -1.77 | -1.61 | -5.51 | 2.57 | 1 |
| 12713-R5-1 | CTCTCGCGACCGACCTGCCGCCGACCGCCACAG | 2334 | -43.69 | -9.13 | -3.01 | -2.22 | -23.87 | -33.93 | -2.33 | 0 |
| 12722-L5-1 | AAACAAAGTACTTCCGACCTCCCCGCCCGCCCGC | 2335 | -34.34 | -6.02 | -2.48 | -2.05 | -6.64 | -8.13 | -1.75 | 0 |
| 13004-R5-1 | CTCCAACCCCCGCAATTCTCGCTCCCTTCACCTGA | 2336 | -35.18 | -35.18 | -5.05 | -10.62 | -35.18 | -35.18 | -2.20 | 0 |
| 13006-R5-4 | CCCGGCTCTCCCTCCCCTGCGCCGCGCTCTCGCC | 2337 | -13.41 | -29.15 | -1.35 | -3.15 | -4.57 | -3.39 | 1.17 | 0 |
| 13044-L5-3 | CCCATCCCAGCTGTCCCTTTCTTTGCTTTCATCA | 2338 | -23.71 | -23.71 | -17.84 | -12.27 | -23.71 | -23.71 | -4.70 | 0 |
| 13044-L5-4 | CCAGGGCTTCTCCCATCCCAGCTGTCCCTTTCTTTG | 2339 | -30.11 | -30.11 | -4.72 | -5.11 | -30.11 | -30.11 | -2.34 | 0 |
| 13047-R5-2 | GCACAGCAGACCCCATGCACTAGCCCCGGGCAC | 2340 | -19.29 | -19.29 | -19.29 | -19.29 | -19.29 | -19.29 | -19.29 | 0 |
| 13052-L5-1 | TCCCCGGACCTAAGCATCTCCCCCACCCGCCAACC | 2341 | -6.46 | -6.46 | 1.30 | -2.05 | -2.52 | -4.72 | 4.47 | 1 |
| 13093-L5-2 | CGACCCCGCAGAACCCCACCGCGCCCCGCGCAG | 2342 | -45.57 | -45.57 | -16.39 | -23.29 | -45.57 | -45.57 | -10.53 | 0 |
| 13097-L5-2 | AGCCTCAGCCCCACCTCCAGCCCCACCCTAGGG | 2343 | -7.94 | -7.94 | -1.16 | -3.99 | -5.23 | -7.94 | 1.33 | 0 |
| 13106-R5-1 | ATCAGAGGCCGACCCCGGCGTCCAGGCCGGCA | 2344 | -37.32 | -37.32 | -26.17 | -37.32 | -37.32 | -37.32 | -24.68 | 0 |
| 13106-R5-2 | TGGACCAATCAGAGGCCGACCCCGGCGTCCAG | 2345 | -42.08 | -42.08 | -35.48 | -42.08 | -42.08 | -42.08 | -9.62 | 0 |
| 13111-L5-3 | TCTCCGCCGGGCCTTCACCCTGCCCTGCTCTTCT | 2346 | -8.41 | -8.41 | 1.41 | 1.94 | -4.26 | -6.40 | -1.19 | 0 |
| 13119-R5-2 | GACTCTGCCGCTCCCGCCCGGCCACCTCCCTGT | 2347 | -6.27 | -5.25 | -3.10 | -2.23 | -8.08 | -8.44 | -2.59 | 0 |
| 13129-L5-2 | GTCCCCTGGCCCCCGACCTGCTCCATCCACCCA | 2348 | -10.72 | -10.72 | 1.69 | -2.03 | -7.75 | -4.28 | 4.56 | 1 |
| 13129-L5-3 | GTCCCCTGGCCCCCGACCTGCTCCATCCACCCA | 2349 | -10.72 | -10.72 | 1.69 | -2.03 | -7.75 | -4.28 | 4.56 | 1 |
| 13129-L5-3 | AGCCTTCCTGTCCCCTGGCCCCCGACCTGCTCCA | 2350 | -8.92 | -15.69 | -1.15 | -1.02 | -5.81 | -15.69 | 3.68 | 1 |
| 13130-L5-1 | AGCCCGCCCCAACCCACCTCGATCTTTTCCTC | 2351 | -2.96 | -5.75 | -1.02 | -2.38 | -7.72 | -7.44 | -1.49 | 0 |
| 13130-L5-2 | TCCCCAGAGCCCGCCCCAACCCACCTCGATCT | 2352 | -19.17 | -11.28 | -2.91 | -4.08 | -19.00 | -28.42 | -1.70 | 0 |
| 13137-L5-1 | GCTCTAACCCCCGCAACCCCACCTCCCCATGCC | 2353 | -6.42 | -6.42 | 1.22 | -1.92 | -2.51 | -6.42 | 2.70 | 1 |
| 13138-R5-1 | TTCGCCACGCCCCGCCACCCGAGCTGCCTCCC | 2354 | -22.78 | -6.06 | -3.57 | -2.48 | -3.75 | -4.73 | -2.04 | 0 |
| 13161-L5-4 | TCCGCCAGGGTCCGCGTGTCAGTCCCCTCTGGTGA | 2355 | -60.36 | -106.72 | -106.33 | -4.55 | 225.31 | -225.31 | 122.88 | 0 |
| 13209-R5-3 | TGGTCGCCGCCGCAGGCGCCTGAAGGGCACGGCGG | 2356 | -23.10 | -23.10 | -23.10 | -23.10 | -23.10 | -23.10 | -14.65 | 0 |
| 13211-L5-1 | ACGCGCCCCGCCGCTCTCTGACCGACCGGAGGCGC | 2357 | -33.55 | -33.55 | -13.40 | -24.17 | -33.55 | -33.55 | -8.31 | 0 |
| 13227-L5-1 | CTCCTCGTCCCCCTTCCCACCTCGGTGTCTTGCTT | 2358 | -25.42 | -25.42 | -3.92 | -12.50 | -2.75 | -12.60 | -4.39 | 0 |
| 13227-L5-2 | GGGCCCAGTCCTCCTCGTCCCCCTTCCCACCTCGG | 2359 | -24.86 | -38.26 | -4.08 | -9.37 | -2.60 | -5.71 | -1.14 | 0 |
| 13229-L5-1 | ACCCGTCCCTGCCCCTTTACCCCTTGGGCCAGCA | 2360 | -6.14 | -6.14 | 1.13 | -3.09 | -3.21 | -4.58 | 2.42 | 1 |
| 13229-L5-2 | CCTGGGGCCACCCGTCCCTGCCCCTTTACCCCTT | 2361 | -7.44 | -7.44 | -1.25 | -7.44 | -7.44 | -7.44 | 3.22 | 1 |
| 13229-R5-3 | GCAGCTCCGCCAGTCTCTGTGGGCAGGGAGAAG | 2362 | -29.88 | -18.41 | -18.21 | -1.79 | -29.88 | -29.88 | -6.56 | 0 |
| 13239-L5-2 | CAGAGCTCCCCCCATCTCCCCAGACTTACCCCT | 2363 | -5.36 | -5.36 | -1.08 | -3.59 | -3.58 | -5.36 | 3.91 | 1 |
| 13240-L5-2 | AATCGCCGTCCCCGCCGCGGCATTCCCGGCCCCAA | 2364 | -28.53 | -28.53 | -2.82 | -2.43 | -28.53 | -21.92 | -1.98 | 0 |
| 13247-L5-3 | TCCTGAGCCGCCTTCCCCTCCCGACCTCAGAGCCCT | 2365 | -13.68 | -19.46 | -1.59 | -1.37 | -1.28 | -2.19 | 1.48 | 0 |
| 13267-L5-1 | CACTCCCTGCTGGCCCCCACCTCACCTATGGTG | 2366 | -58.86 | -58.86 | -9.66 | -17.90 | -7.03 | -39.11 | -1.08 | 0 |
| 13276-L5-4 | CCCCCATTGTGGCTGCTCCCACCCCACCTGCCTTCA | 2367 | -9.13 | -3.05 | -1.92 | -2.52 | -1.28 | -2.58 | -1.50 | 0 |
| 13281-L5-3 | CACCCCCACCCCACAGGACAGAGGAAGTGACGAG | 2368 | -17.40 | -17.40 | -2.72 | -9.25 | -17.40 | -17.40 | -1.76 | 0 |
| 13283-L5-3 | GGACCCCTGCCTTCCTTGCTGCCACCCTTTGCACA | 2369 | -5.08 | -5.08 | -1.49 | -2.73 | -5.08 | -5.08 | 1.94 | 0 |
| 13285-L5-3 | GCTATGCACCCAGCCGCCCAGCTCAGCCCCTGC | 2370 | -13.41 | -13.41 | -2.23 | -7.03 | -13.41 | -13.41 | -1.61 | 0 |
| 13289-L5-3 | GCTCAAAGTTTGCCTCCCATGGCCCCTCTGCCC | 2371 | -5.97 | -5.97 | -1.04 | -2.79 | -5.97 | -5.97 | 1.72 | 0 |
| 13291-L5-2 | TGGTTCTGCCCAAGCGCCCCTTCCTCCCTCCTT | 2372 | -5.00 | -5.00 | 1.36 | -3.54 | 1.37 | -2.63 | 3.53 | 1 |
| 13312-L5-2 | TGCCACCCCACCCCTCCCCCACAGCCCAGCCC | 2373 | -6.79 | -15.24 | -1.49 | -6.97 | -1.11 | -3.12 | 1.59 | 0 |
| 13325-R5-1 | CTGCCTGGCGCTGGGCTCTTCCCCATGAGCG | 2374 | -8.15 | -8.15 | -1.33 | -3.29 | -8.15 | -8.15 | 2.04 | 1 |
| 13325-R5-2 | TCCAACACTGCCTGGCGCTGGGCTCTTCCCCA | 2375 | -8.85 | -8.85 | 1.23 | -1.20 | -8.85 | -8.85 | 2.07 | 1 |
| 13326-L5-1 | TCCGGCTTCCCCCCACCCGCCCTTCGATGGCA | 2376 | -5.52 | -5.52 | 1.33 | -1.60 | 2.24 | -1.93 | 2.59 | 2 |
| 13326-L5-2 | CAGAGATTCCGGCTTCCCCCCACCCGCCCTTC | 2377 | -10.44 | -14.87 | -1.59 | -3.94 | -1.48 | -5.00 | 1.20 | 0 |
| 13343-L5-1 | CCTCCACCCCTCCCGCAGCGCCCCTCCCCCTCA | 2378 | -54.66 | -54.66 | -2.68 | -1.95 | -2.09 | -4.50 | -1.06 | 0 |
| 13349-L5-1 | TGTGTGGGCTATCCCAGCCGCCTCCTTCCTCT | 2379 | -6.04 | -6.04 | 1.05 | -1.36 | -6.04 | -6.04 | 1.40 | 0 |
| 13349-L5-2 | CACCACCTGTGTGGGCTATCCCAGCCGCCTCC | 2380 | -17.13 | -17.13 | -4.48 | -12.67 | -17.13 | -17.13 | -2.49 | 0 |
| 13365-L5-3 | TCCCCAGAGCCCGCCCCAACCCACCTCGATCTTTT | 2381 | -12.64 | -27.91 | -4.88 | -2.98 | -22.68 | -21.98 | -1.53 | 0 |
| 13374-R5-1 | AGCCTTCCTGTCCCCTGGCCCCCGACCTGC | 2382 | -20.45 | -20.45 | -2.48 | -5.82 | -13.37 | -11.72 | -1.59 | 0 |
| 13374-R5-2 | GGCCCTAGCCTTCCTGTCCCCTGGCCCCCGA | 2383 | -5.79 | -5.79 | -1.35 | -2.92 | -4.80 | -5.79 | 1.61 | 0 |
| 13375-L5-3 | GAGAACCTGAAACCCCAGCCCCTGCCTACCCCTTAG | 2384 | -5.86 | -5.86 | 1.24 | -2.87 | -5.86 | -5.86 | 1.67 | 0 |
| 13396-L5-2 | GACCTGCCCCGCCCCACTCGGGCTCCTTACCG | 2385 | -5.03 | -10.37 | -1.25 | -2.62 | -8.50 | -7.78 | 1.54 | 0 |
| 13431-L5-3 | CGACACCCACTCACTGCCGCTGCCGCACTCACAGC | 2386 | -23.38 | -23.38 | -2.69 | -4.01 | -23.38 | -23.38 | -4.52 | 0 |
| 13432-R5-4 | TTTACAGTTTCTGGCACTTCCTACCACCTCCCCA | 2387 | -5.41 | -5.41 | -1.42 | -1.80 | -5.41 | -5.41 | 2.00 | 1 |
| 13456-R5-2 | CAGATGCCCCGCTATGAAATCTTTTCCAACC | 2388 | -16.80 | -16.80 | -13.79 | -16.80 | -16.80 | -16.80 | -16.80 | 0 |
| 13461-L5-4 | TCCCCAGCCCCGTCCCCACCCCCTAGAGAAAGTGAA | 2389 | -19.96 | -28.08 | -3.31 | -5.34 | -4.75 | -16.77 | -1.67 | 0 |
| 13497-L5-1 | TGTGCCCCCCTCGCTCCCAGCCCCCAGGGGACCGC | 2390 | -7.88 | -20.24 | -4.39 | -3.42 | -3.34 | -3.05 | -1.79 | 0 |
| 227-L5-1 | ACACCTGTCTCTCCCCAGTGCTTCCGCCCCTCA | 2391 | -59.95 | -93.40 | -4.00 | -10.53 | -13.51 | -33.21 | -2.73 | 0 |
| 266-R4-1 | GTCGCCCCCTCCCCCAAGTTGAGACTTGCAGCTAC | 2392 | -6.36 | -14.52 | -1.45 | -2.41 | 1.12 | -3.13 | 1.90 | 0 |
| 266-R5-1 | CCCCTCCCCCAAGTTGAGACTTGCAGCTAC | 2393 | -7.35 | -7.35 | 1.06 | -2.08 | 1.10 | -4.92 | 5.32 | 1 |
| 2819-R5-4 | CAGCCTGCCACCGCCGCTTTTGAAAGAAGCACTTCA | 2394 | -8.56 | -8.56 | 1.00 | -5.87 | -8.56 | -8.56 | -1.66 | 0 |
| 3009-L5-1 | ACCTCGGTCTCCTCCACCAGACTTTAAACTCTC | 2395 | -17.19 | -17.19 | -5.92 | -2.82 | -7.48 | -17.19 | -4.70 | 0 |
| 3009-L5-2 | CCAGTGATACCTCGGTCTCCTCCACCAGACTTT | 2396 | -19.46 | -19.46 | -4.10 | -9.16 | -10.45 | -19.46 | -5.93 | 0 |
| 3009-L5-3 | TGTAATAACCAGTGATACCTCGGTCTCCTCCAC | 2397 | -23.73 | -23.73 | -3.74 | -2.02 | -7.39 | -23.73 | -5.75 | 0 |
| 3249-L4-1 | GCGGAGCCGCCGCCATCCCCGGAGCCGCCGCCGCCG CC | 2398 | -8.53 | -6.22 | -3.77 | -2.41 | -14.30 | -15.31 | -2.56 | 0 |
| 3249-L5-2 | AGCGGAGCCGCCGCCATCCCCGGAGCCGCCGCCG | 2399 | -94.34 | -18.11 | -4.95 | -2.32 | -23.40 | -29.20 | -2.57 | 0 |
| 3799-R5-1 | CTGAAGATGCTCCCAGAGGCCCCCCGCCGGCC | 2400 | -7.96 | -8.04 | -1.30 | 1.45 | -8.27 | -13.22 | 1.59 | 0 |
| 3897-R5-2 | CCGACCCGCCCGTCAGCCGCCTCCCCCTCAG | 2401 | -11.04 | -14.48 | -1.65 | -2.21 | -3.50 | -5.62 | -1.55 | 0 |
| 3953-R3-2 | ACTCCAGCCTCCGCCGCCTCAGCTTCCCGAGC | 2402 | -34.43 | -52.88 | -3.53 | -4.10 | -15.19 | -24.22 | -5.59 | 0 |
| 3966-L5-1 | ACCCCAGAGCTGTCGCCGCCGCTGCCGCCTTCGCC | 2403 | -12.50 | -9.10 | -4.67 | -3.33 | -12.90 | -27.25 | -3.64 | 0 |
| 4303-R1-1 | AGTGCCCGCTCCTCCGACCTCCCTGCGCACC | 2404 | -16.94 | -16.94 | -1.82 | -7.37 | -13.90 | -16.94 | 1.02 | 0 |
| 4315_C-L4-1 | GCAGCCCCTCCTCCGAGAGGTTGGGGGTCGCGGCCGCCC GGCCCTCCCGGTC CCCTCCCC | 2405 | -10.99 | -17.02 | -1.97 | -1.53 | -1.97 | -2.96 | 1.32 | 0 |
| 4315 D-R4-1 | GGAAAGTCAGCCCCCAGCGCCCCCCGGAGTTCTTGG | 2406 | -88.80 | -88.80 | -12.25 | -8.23 | -57.77 | -88.80 | -6.18 | 0 |
| 4315 E-R4-1 | CCCCCACCAAACCTATTCCCGCATCCTCCCCGGCTCTGG | 2407 | -7.17 | -8.95 | -1.28 | 1.62 | -3.51 | -8.09 | 2.47 | 1 |
| 4315 F-R4-1 | AACCCGGGCTCCCCCACCCGCTCCCTGAGC | 2408 | -19.29 | -28.54 | -1.97 | -2.12 | -6.85 | -14.36 | -1.32 | 0 |
| 4315 I-L4-1 | ACACCTCTGCGCCCCTCAGGCGCCCTGGGCCTCGGCGCCC CGCCCGTCCCAG | 2409 | -30.88 | -30.88 | -4.30 | -3.56 | -30.88 | -30.88 | -1.71 | 0 |
| 4315 K-L4-1 | TCCCAGGGGGCCCTGAACTTGTCAAATCCTCGCCATCCTCC ACCCCCAGCCCCGG | 2410 | -7.44 | -7.44 | 1.35 | -1.81 | -2.45 | -3.94 | 1.80 | 0 |
| 4440-R3-1 | TACTCCCGCCGTTTACCCGCATTTCACTGAA | 2411 | -10.88 | -1.67 | -1.04 | 1.21 | -4.04 | -11.36 | -1.05 | 0 |
| 4479-R3-1 | AGCCCCCTGCCCGGAAATTCAAAACAACTGC | 2412 | -14.50 | -20.12 | -3.55 | -6.32 | -16.37 | -20.12 | -3.97 | 0 |
| 4593-R5-1 | CTATAGCAGATGACATAACTCCCCCGGCATCAG | 2413 | -12.46 | -12.46 | 1.06 | 1.11 | -12.46 | -12.46 | -1.12 | 0 |
| 4829-R2-1 | TCCCTTTGTGCTGCCCGAGTGCCTTCCCCCTG | 2414 | -9.27 | -9.27 | 2.07 | -1.01 | -3.71 | -2.66 | 6.01 | 2 |
| 4855-R5-2 | GGGCTGCCGGGTCTCCCGCTTCCCCCTCCTGC | 2415 | -10.65 | -15.12 | 1.25 | 1.03 | 1.70 | -1.51 | 1.72 | 0 |
| 4884-R5-1 | TCCCCAGTCTCCCTGTTTCAGCACCTGCCTCA | 2416 | -17.58 | -17.58 | -1.81 | -2.95 | -7.44 | -9.96 | 1.51 | 0 |
| 4988-R4-1 | CTCCTCCTCCCCGTCTTTGGATACCAAACACTGGAC | 2417 | -5.47 | -5.47 | 1.61 | -1.61 | -1.44 | -5.47 | 3.51 | 1 |
| 4988-R5-2 | CTCCTCCTCCCCGTCTTTGGATACCAAACAC | 2418 | -9.43 | -9.43 | 1.16 | -2.18 | -1.17 | -9.43 | 4.95 | 1 |
| 5071-R5-1 | CGCCCCAGTCCCAGCCCAATTAATAAATGGG | 2419 | -6.09 | -6.09 | 1.41 | 2.38 | -6.09 | -6.09 | 1.59 | 0 |
| 5071-R5-2 | GACCCCCGCCCCAGTCCCAGCCCAATTAATA | 2420 | -24.30 | -13.23 | -2.97 | -3.00 | -5.08 | -18.13 | -1.48 | 0 |
| 5640-L3-1 | GCCATGGAACACCGTGCCTGCCCCTCTCGAGA | 2421 | -22.57 | -22.57 | -3.86 | -4.16 | -22.57 | -22.57 | -1.57 | 0 |
| 5707-L5-2 | TCACCATGCGGCCCCGGTGGTCTTCACACAGCA | 2422 | -17.42 | -17.42 | -17.42 | -17.42 | -17.42 | -17.42 | -17.42 | 0 |
| 6198-R5-2 | GCCGCCGCCGCCGCGTCTTCCCGCGAAGCCT | 2423 | -8.01 | -10.31 | -4.02 | -2.44 | -21.45 | -23.60 | -3.04 | 0 |
| 6216-R5-2 | CATAGTTACTCCCGCCGTTTACCCGTGCTTC | 2424 | -15.09 | -10.39 | -1.12 | 1.09 | -5.30 | -18.03 | -1.30 | 0 |
| 6825-R5-1 | TCCTTCTGCTCAGCTGTTCCCGGTGCCAG | 2425 | -28.95 | -28.95 | -21.84 | -14.99 | -28.95 | -28.95 | -6.06 | 0 |
| 6880-L3-2 | ACCTCCCCCGCGAAGACATCCACATTCTGCA | 2426 | -20.08 | -29.17 | -1.83 | -9.68 | -3.55 | -29.17 | -3.36 | 0 |
| 7061-R5-2 | TCATGGAAACCCCACCCTTCCCATGCCCAACC | 2427 | -5.94 | -5.94 | -1.04 | -4.28 | -5.94 | -5.94 | 1.37 | 0 |
| 7126-L3-1 | GCACACCCGCTCTCCGGCCCGCGCCCCTG | 2428 | -25.95 | -6.91 | -4.10 | -2.74 | -11.10 | -14.35 | -2.85 | 0 |
| 7356 A-R4-1 | CAGAGCCCGCTCTCGCGACCGACCTGCCGCCGACCGCCAC AG | 2429 | -18.01 | -26.53 | -3.41 | -4.02 | -22.46 | -25.26 | -3.07 | 0 |
| 7702-L2-1 | CCCAGAGAACCGGAATTCCTCCCCGCCCC | 2430 | -5.27 | -5.27 | -1.83 | -2.91 | -4.35 | -5.27 | 2.04 | 1 |
| 7824-R5-1 | TGCCAGCTTCATCGCCGCCTCACACACACA | 2431 | -8.78 | -8.78 | -2.70 | -8.78 | -8.78 | -8.78 | -3.37 | 0 |
| 7949-R5-1 | GATGCGCGCGCCGACCGCCGCCAGCTGCAATTCATAC | 2432 | -24.23 | -24.23 | -3.02 | -1.84 | -24.23 | -24.23 | -2.16 | 0 |
| 8016-L3-1 | TCAGCGCAACAAGCCCCGCAGTCACCCCTCT | 2433 | -54.19 | -54.19 | -16.08 | -20.80 | -54.19 | -54.19 | -6.74 | 0 |
| 8250-R5-2 | CCGACCCGCCCGTCAGCCGCCTCTCCCTCAG | 2434 | -17.68 | -16.09 | -1.77 | -1.62 | -3.89 | -4.78 | 1.33 | 0 |
| 8394-L5-1 | CCGCCCTGCCCATCTCCGACTATCCCTGGCCCC | 2435 | -7.06 | -7.06 | 1.08 | -1.28 | -7.06 | -7.06 | 2.40 | 1 |
| 8898-R5-1 | CAGCCGAGGCGGACGCCCGCTCCCGCCACCATG | 2436 | -10.42 | -15.12 | -2.07 | -1.34 | -12.35 | -15.12 | -1.31 | 0 |
| 9053-R3-1 | TTCTTGCCCTCCAATCCCCGGGCTCCACCAGCC | 2437 | -10.59 | -10.59 | -1.97 | -1.65 | -8.41 | -10.59 | -2.27 | 0 |
| 9387-R2-2 | TCCATCCTTGCCGTCGCCTTCATCTCAAAGCCATC | 2438 | -8.80 | -8.80 | -1.47 | -8.80 | -8.80 | -8.80 | -3.48 | 0 |
| 9691-L5-1 | CATTTCATCCGCATCTCCCTCTTGGCCCCTTGC | 2439 | -8.33 | -8.33 | -2.45 | -6.14 | -8.33 | -8.33 | 1.53 | 0 |
| 9774-R2-2 | CCGCCCCCTCACCGCCTCCTGCTCCCATCAGGC | 2440 | -7.93 | -16.14 | -1.34 | -2.55 | -4.83 | -3.47 | 1.40 | 0 |
| miR-1228* | CACACACCTGCCCCCGCCCAC | 2441 | -15.38 | -12.90 | -2.54 | -1.74 | -4.13 | -8.82 | -1.45 | 0 |
| miR-1275 | GACAGCCTCTCCCCCAC | 2442 | -8.09 | -8.09 | 2.33 | -1.30 | -2.96 | -8.09 | 2.04 | 2 |
| miR-1308 | CCACTGAACCACCCATGC | 2443 | -18.92 | -18.92 | -5.22 | -18.92 | -4.03 | -18.92 | -5.64 | 0 |
| miR-21 | TCAACATCAGTCTGATAAGCTA | 2444 | -1.68 | -23.57 | -19.14 | -23.57 | 1.39 | -7.45 | -2.50 | 0 |
| miR-373* | GGAAAGCGCCCCCATTTTGAGT | 2445 | -48.87 | -48.87 | -48.87 | -48.87 | -48.87 | -48.87 | -21.32 | 0 |
| miR-423-5p | AAAGTCTCGCTCTCTGCCCCTCA | 2446 | -9.88 | -4.20 | -6.30 | -4.12 | -7.70 | -16.50 | -3.64 | 0 |
| miR-486-3p | ATCCTGTACTGAGCTGCCCCG | 2447 | -40.84 | -40.84 | -36.13 | -40.84 | -40.84 | -40.84 | -7.70 | 0 |
| miR-612 | AAGGAGCTCAGAAGCCCTGCCCAGC | 2448 | -5.79 | -5.79 | -3.09 | -2.94 | -5.79 | -5.79 | -1.13 | 0 |
| miR-638 | AGGCCGCCACCCGCCCGCGATCCCT | 2449 | -11.00 | -23.12 | -2.25 | -1.89 | -19.12 | -23.12 | -1.53 | 0 |
| miR-663 | GCGGTCCCGCGGCGCCCCGCCT | 2450 | -45.02 | -45.02 | -10.68 | -32.17 | -45.02 | -45.02 | -11.52 | 0 |
| miR-744 | TGCTGTTAGCCCTAGCCCCGCA | 2451 | -37.67 | -37.67 | -10.59 | -19.87 | -37.67 | -37.67 | -4.41 | 0 |
| miR-923 | AGTTTCTTTTCCTCCGCTGAC | 2452 | -3.90 | -3.72 | 1.48 | 1.30 | -6.06 | -8.95 | 2.30 | 1 |

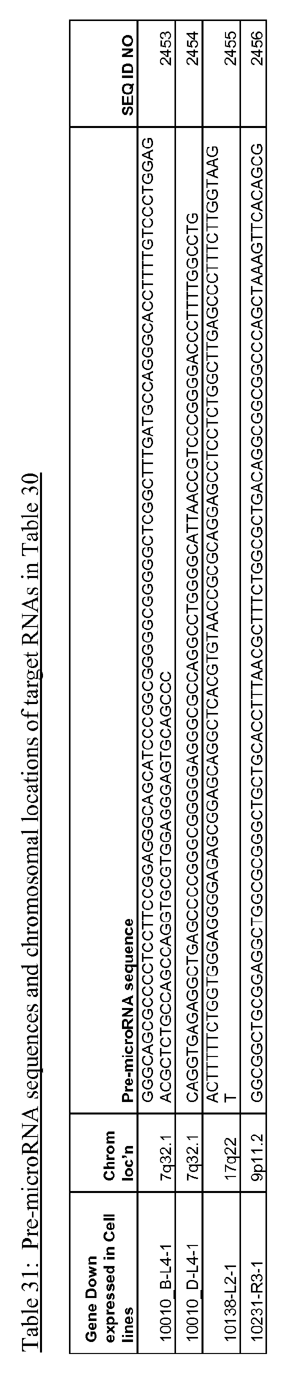

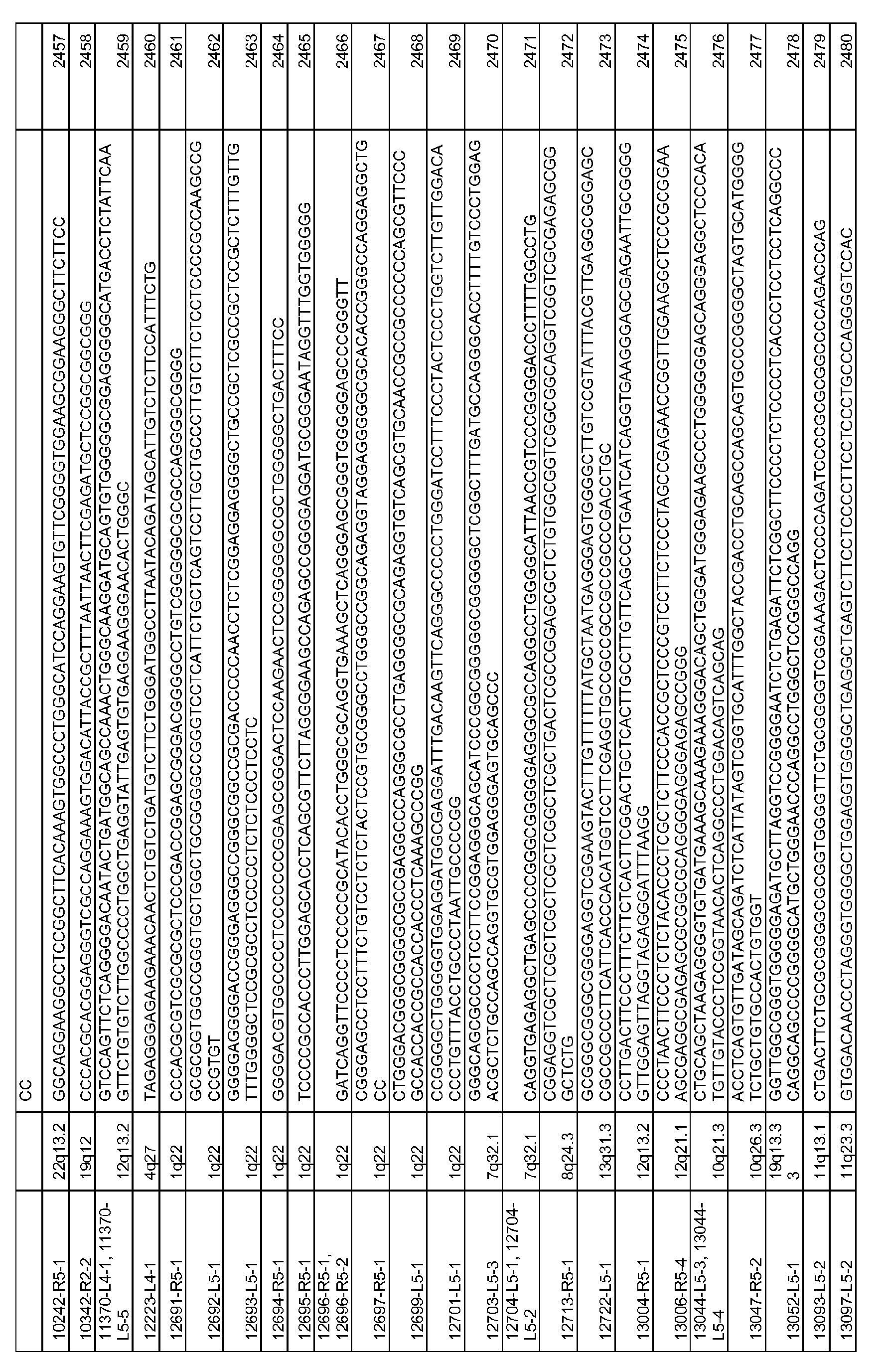

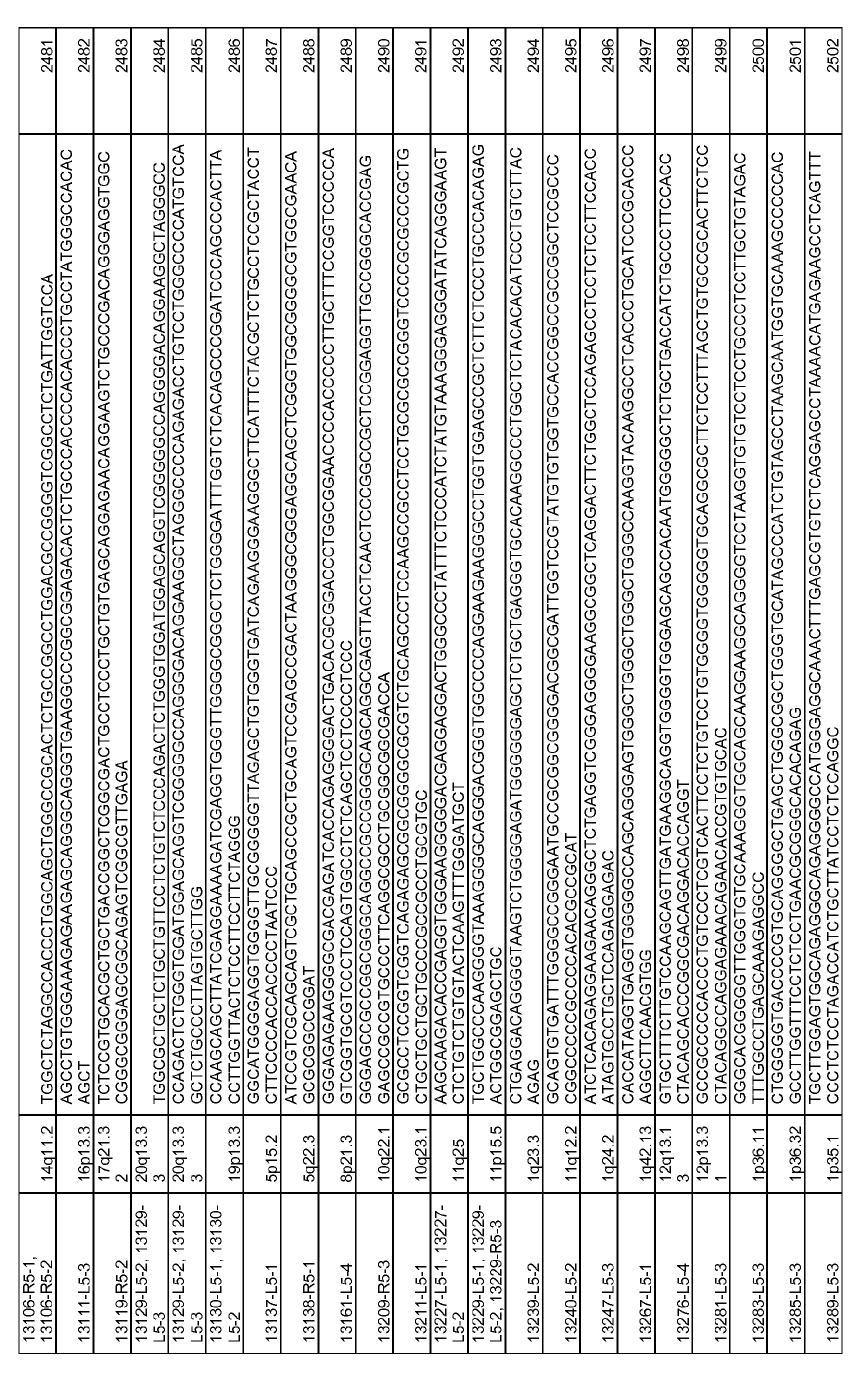

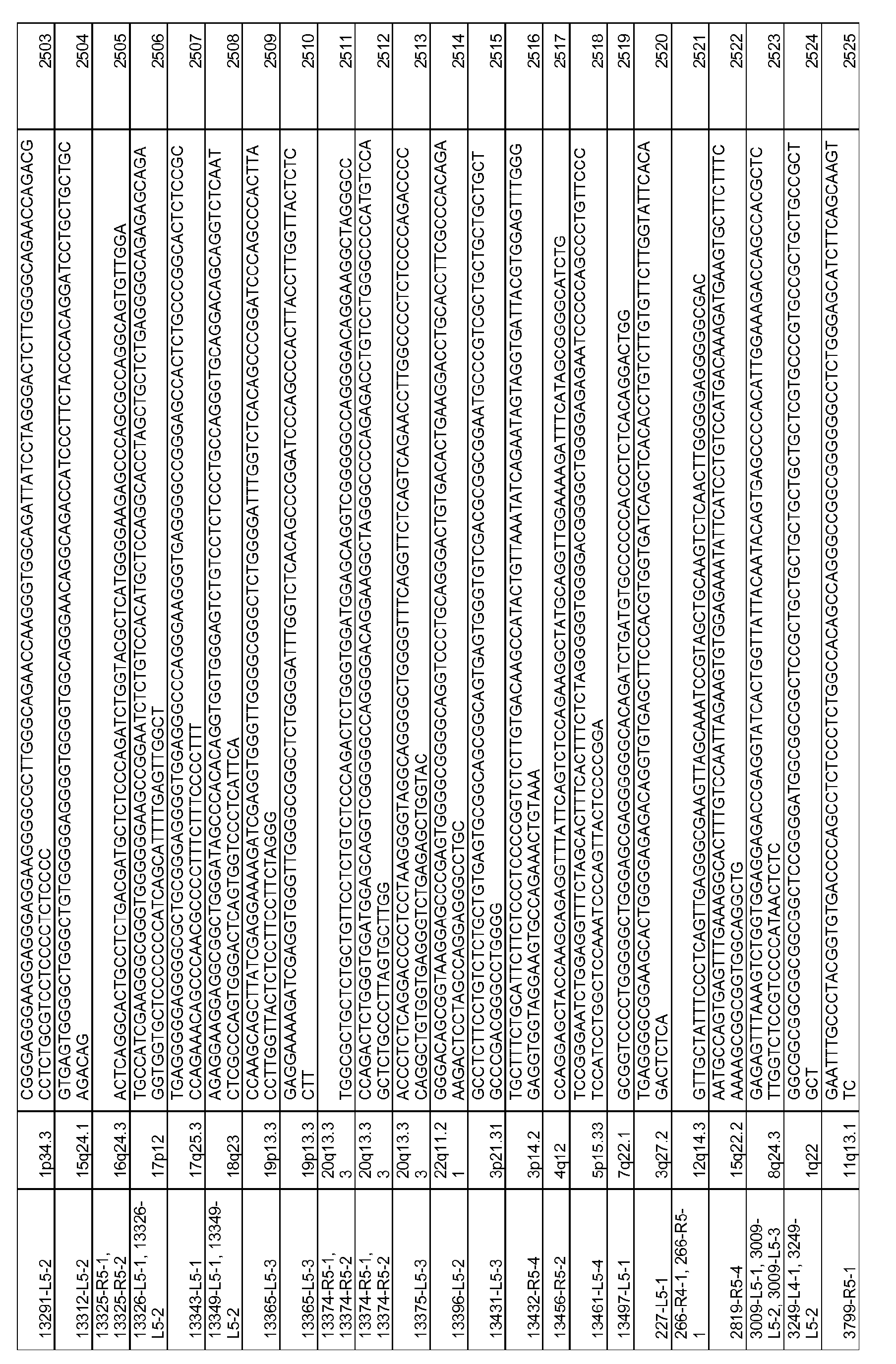

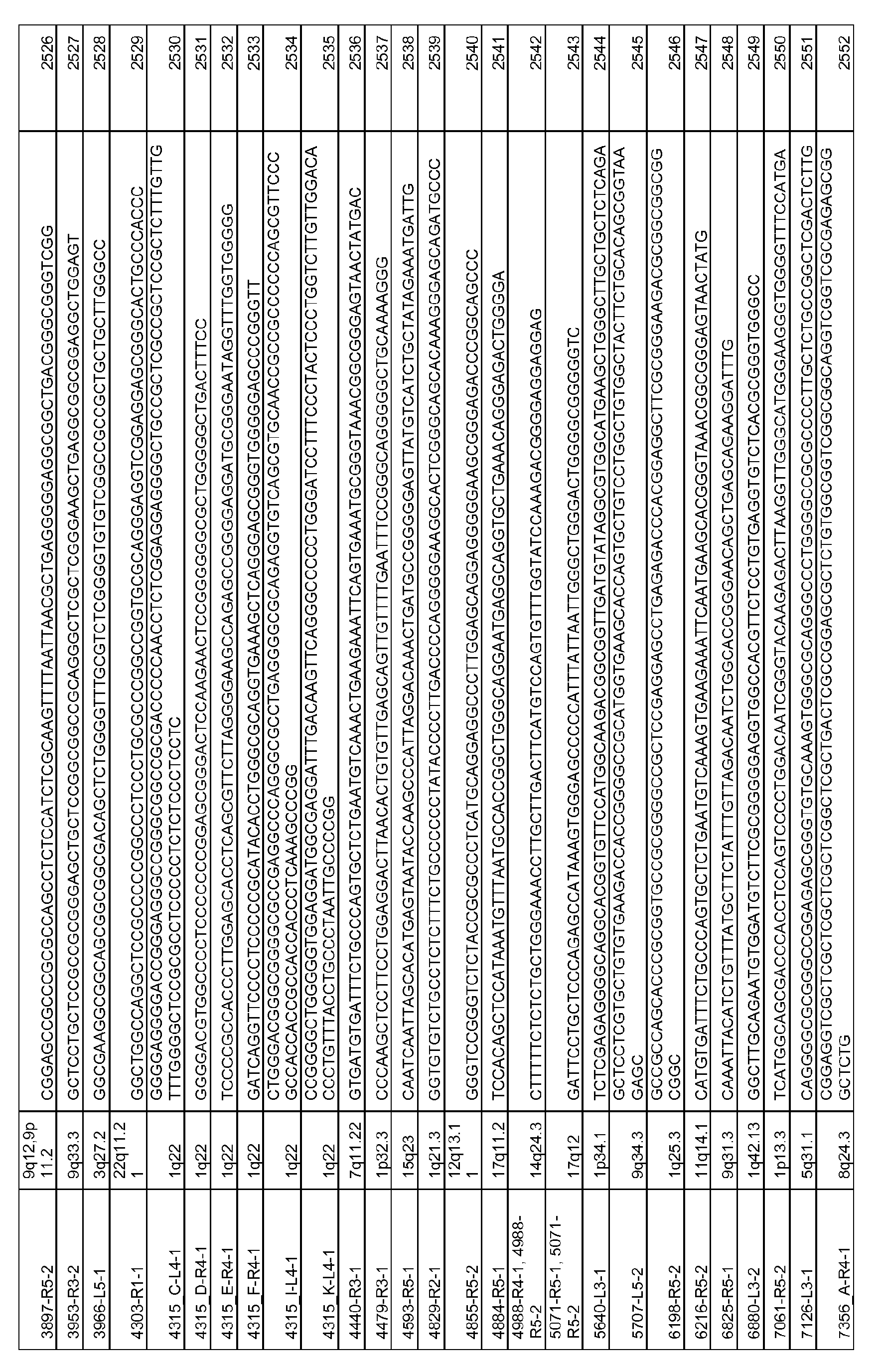

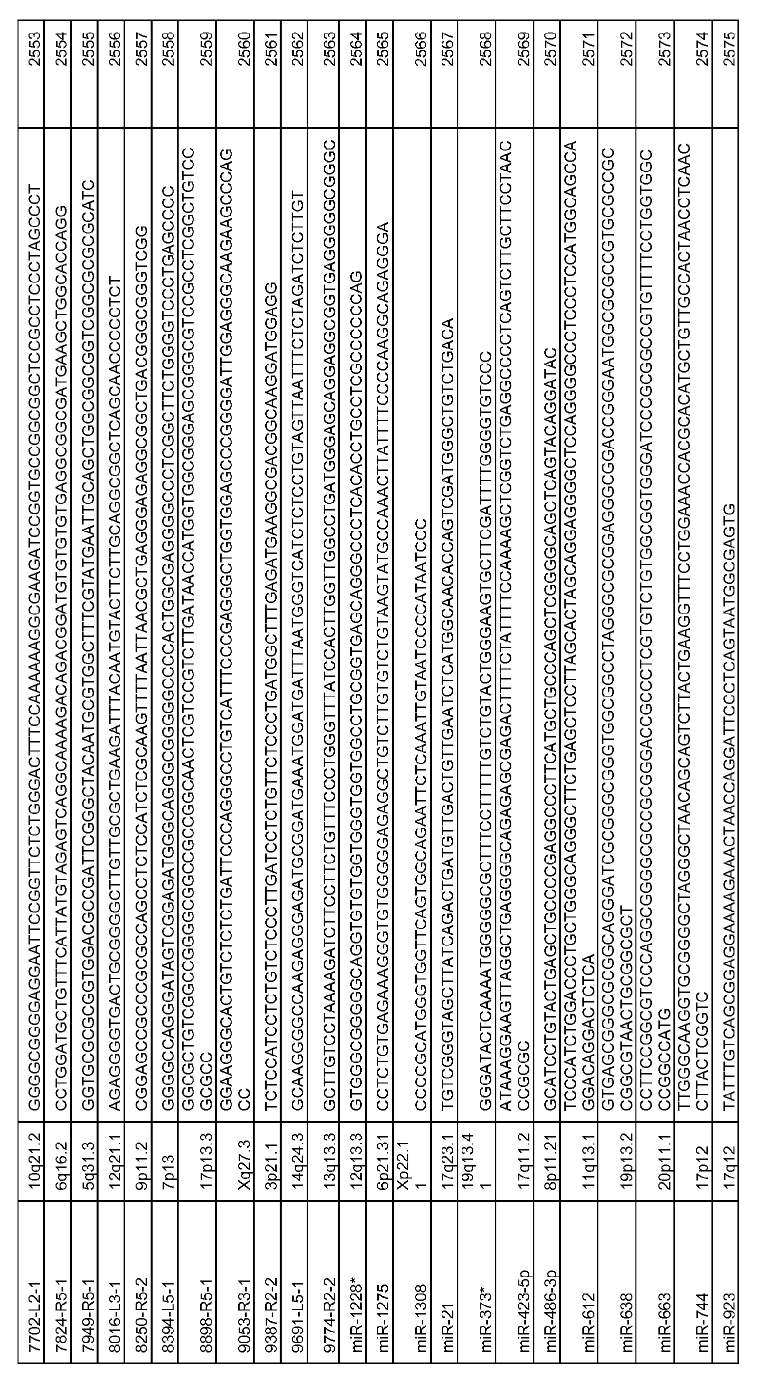

### 5.6 Example 6: Target RNA Levels in Primary Tumors and Cell Lines

Data from Examples 4 and 5 were analyzed to identify target RNAs that were found to be up-expressed in both primary lung tumors and in lung cancer cell lines. Table 32 shows a list of the target RNAs from Table 27 (i.e., target RNAs that are present at increased levels in at least four lung cancer cell lines) that are also in Tables 18 to 21 (i.e., target RNAs that are present at increased levels in more than 50% of primary lung tumors tested, and target RNAs that are present at at least 5-fold increased levels in fewer than 50% of primary lung tumors tested). Table 33 shows a list of the target RNAs from Table 28 (i.e., target RNAs that are present at at least 5-fold increased levels in two or three cell lines) that are also in Tables 18 to 21.

**Table 32: Target RNAs from Table 27 that are also in Tables 18 to 21**

| **target RNA from Table 27 (increased in at least four cell lines)** | **probe SEQ ID NO** | **pre-microRNA SEQ ID NO** | **microRNA SEQ ID NO** | **Present in Tables 18 and 19 (increased in at least 50% tumors)?** | **Present in Tables 20 and 21 (increased at least 5-fold in <50% tumors)?** |
|---|---|---|---|---|---|
| 10083-L5-1 | 1090 | 1211 | | | Yes |
| 13122-L5-1 | 1066 | 1242 | 2587 | Yes | |
| 13185-L5-3 | 1118 | 1243 | 2603 | Yes | |
| 13219-L5-1 | 1067 | 1245 | | Yes | |
| 3744-R5-1 | 1143 | 1271 | 2645, 2646 | | Yes |
| 6235-R5-2 | 1075 | 1296 | 2661 | Yes | |
| 6474-L5-1 | 1164 | 1299 | | | Yes |
| 6681-R2-1 | 1166 | 1301 | | | Yes |
| 8004-R3-2 | 97 | 492 | | Yes | |
| 836-R4-1 | 3 | 400 | | Yes | |
| 9594-R5-1 | 1081 | 1328 | | Yes | |
| miR-103 | 361 | 754, 755 | 803 | | Yes |
| miR-200b | 193 | 692 | 839 | Yes | |
| miR-200c | 194 | 693 | 840 | Yes | |
| miR-20b | 380 | 776 | 843 | Yes | |
| miR-298 | 1088 | 1351 | 858 | Yes | |

**Table 33: Target RNAs from Table 28 that are also in Tables 18 to 21**

| **target RNA from Table 28 (increased 5-fold in 2 or 3 cell lines)** | **probe SEQ ID NO** | **pre-microRNA SEQ ID NO** | **microRNA SEQ ID NO** | **Present in Tables 18 and 19 (increased in at least 50% tumors)?** | **Present in Tables 20 and 21 (increased at least 5-fold in <50% tumors)?** |
|---|---|---|---|---|---|
| 10335-L5-2 | 1092 | 1213 | | | Yes |
| 12729-R5-1 | 1103 | 1186 | | | Yes |
| 12888-L5-2 | 1105 | 1188 | | | Yes |
| 12917-R5-2 | 1108 | 1230 | | | Yes |
| 12992-L5-1 | 1112 | 1235 | | | Yes |
| 13001-L5-1 | 1113 | 1236 | | | Yes |
| 13070-R5-3 | 1115 | 1238 | | | Yes |
| 13274-L5-3 | 1124 | 1251 | 2612 | | Yes |
| 13357-L5-4 | 1128 | 1255 | 2621 | | Yes |
| 13366-R5-3 | 1129 | 1256 | 2623 | | Yes |
| 13467-L5-1 | 1069 | 1262 | 2630 | Yes | |
| 13470-R5-1 | 1136 | 1264 | | | Yes |
| 3875-R5-2 | 1144 | 1272 | | | Yes |
| 3923-R5-1 | 1070 | 1273 | 2647 | Yes | |
| 5108-R5-2 | 1153 | 1286 | | | Yes |
| 5723-R5-1 | 1156 | 1289 | | | Yes |
| 6752-R5-2 | 1168 | 1303 | | | Yes |
| 6803-R5-2 | 1169 | 1304 | | | Yes |
| 6930-R5-1 | 1173 | 1308 | 2667 | | Yes |
| 8433_C-R4-1 | 1177 | 1317 | | | Yes |
| 8433-L3-1 | 1179 | 1318,1319 | | | Yes |
| 8724-R5-2 | 1181 | 1320 | | | Yes |
| 8808-R5-1 | 1183 | 1322 | | | Yes |
| 8832-R5-1 | 1184 | 1323 | | | Yes |
| 9349-R5-2 | 1080 | 1325 | 2672 | Yes | |
| miR-198 | 1202 | 1343 | 834 | | Yes |
| miR-720 | 1089 | 1362 | 917 | Yes | |

Finally, Table 34 shows target RNAs that are present at decreased levels in both primary tumors and cell lines relative to normal tissue or cell lines.

**Table 34: Target RNAs present at decreased levels in primary tumors and cell lines**

| **Gene** | **probe SEQ ID NO** | **pre-microRNA SEQ ID NO** | **microRNA SEQ ID NO** |
|---|---|---|---|
| 10010_B-L4-1 | 157 | 551 | |
| 10010_D-L4-1 | 158 | 552 | |
| 10231-R3-1 | 1368 | 1713 | |
| 10342-R2-2 | 1371 | 1716 | |
| 11370-L5-5 | 1379 | 1724 | |
| 12691-R5-1 | 1385 | 1730 | |
| 12692-L5-1 | 1386 | 1731 | |
| 12693-L5-1 | 1387 | 1732 | |
| 12694-R5-1 | 1388 | 1733 | |
| 12696-R5-2 | 1389 | 1734 | |
| 12697-R5-1 | 1390 | 1735 | |
| 12699-L5-1 | 1391 | 1736 | 2585 |
| 12701-L5-1 | 1392 | 1737 | |
| 12703-L5-3 | 1393 | 1738 | |
| 12704-L5-2 | 1394 | 1739 | |
| 12713-R5-1 | 1395 | 1740 | |
| 12722-L5-1 | 1396 | 1741 | |
| 13004-R5-1 | 1411 | 1756 | |
| 13047-R5-2 | 1412 | 1757 | |
| 13052-L5-1 | 1414 | 1759 | |
| 13093-L5-2 | 1419 | 1765 | |
| 13097-L5-2 | 1422 | 1767 | |
| 13119-R5-2 | 1425 | 1771 | |
| 13129-L5-3 | 1427 | 1773, 1774 | |
| 13130-L5-2 | 2352 | 2486 | |
| 13137-L5-1 | 1431 | 1779 | |
| 13138-R5-1 | 1432 | 1780 | |
| 13209-R5-3 | 1444 | 1792 | |
| 13211-L5-1 | 1445 | 1793 | |
| 13229-L5-1 | 1447 | 1795 | |
| 13239-L5-2 | 1451 | 1800 | |
| 13240-L5-2 | 1452 | 1801 | |
| 13267-L5-1 | 1456 | 1805 | |
| 13281-L5-3 | 1457 | 1806 | |
| 13283-L5-3 | 1458 | 1807 | |
| 13285-L5-3 | 1459 | 1808 | |
| 13291-L5-1 | 1461 | 1810 | |
| 13312-L5-2 | 1467 | 1816 | |
| 13326-L5-2 | 1470 | 1820 | |
| 13343-L5-1 | 1477 | 1828 | 2619 |
| 13349-L5-2 | 1478 | 1829 | |
| 13365-L5-3 | 1487 | 1838 | |
| 13374-R5-1 | 1490 | 1842, 1843 | |
| 13375-L5-3 | 1491 | 1844 | 2625 |
| 13396-L5-2 | 1495 | 1848 | |
| 13431-L5-3 | 1501 | 1854 | |
| 13432-R5-4 | 2387 | 2516 | |
| 13456-R5-2 | 1503 | 1856 | |
| 13461-L5-4 | 1505 | 1858 | |
| 13497-L5-1 | 1508 | 1861 | |
| 266-R5-2 | 1513 | 1866 | |
| 2819-R5-4 | 1516 | 1869 | |
| 3799-R5-1 | 1519 | 1872 | |
| 3897-R5-2 | 1520 | 1873 | |
| 3953-R3-2 | 1523 | 1876 | |
| 3966-L5-1 | 1524 | 1877 | |
| 4315_C-L4-1 | 1533 | 1886 | |
| 4315 D-R4-1 | 1534 | 1887 | |
| 4315 E-R4-1 | 1535 | 1888 | |
| 4315 F-R4-1 | 1536 | 1889 | |
| 4315_I-L4-1 | 1537 | 1890 | |
| 4315 K-L4-1 | 1538 | 1891 | |
| 4593-R5-1 | 1547 | 1900 | |
| 5071-R5-2 | 1560 | 1913 | |
| 5640-L3-1 | 1569 | 1922 | |
| 6198-R5-2 | 1582 | 1935 | |
| 6880-L3-2 | 1596 | 1949 | |
| 7126-L3-1 | 1604 | 1857 | |
| 7356_A-R4-1 | 1609 | 1962 | |
| 7702-L2-1 | 1618 | 1971 | |
| 7824-R5-1 | 1622 | 1975 | |
| 7949-R5-1 | 1626 | 1979 | |
| 8016-L3-1 | 1628 | 1981 | |
| 8250-R5-2 | 1633 | 1986 | |
| 8394-L5-2 | 1638 | 1991 | |
| 8898-R5-1 | 1641 | 1994 | |
| 9387-R2-2 | 1651 | 2004 | |
| 9691-L5-1 | 1654 | 2007 | |
| 9774-R2-2 | 1656 | 2009 | |
| miR-373* | 1693 | 2049 | |
| miR-486-3p | 1695 | 2051 | |
| miR-638 | 1699 | 2055 | |
| miR-663 | 1700 | 2056 | |
| miR-744 | 1702 | 2058 | |

### 5.7 Example 7: Additional Target RNAs from Primary Tumors

In addition to the target RNAs identified in Example 4, certain additional target RNAs were identified in that experiment as being present at elevated levels in squamous cell carcinoma (SCC). Further, one additional target RNA (miR-320c) was identified as being elevated in at least three of the most aggressive tumors in Example 4. Those additional target RNAs, and miR-320c, are shown in Tables 35 and 36, along with the fold-change in each of the primary tumors. Data for six of the tumors are shown in Table 35 and data for the remaining 13 tumors are shown in Table 36. Table 35 also shows the probe sequences used to detect the target RNAs. Table 37 shows the pre-microRNA sequences and chromosomal location of the pre-microRNA gene for each of the target RNAs in Tables 36 and 37.

**Table 35: Additional target RNAs more frequently present at elevated levels in squamous cell carcinoma (SCC), and miR-320c**

| **Gene** | **Probes sequences** | **SEQ ID NO** | **ADK9** | **ADK10** | **Adk29** | **Adk15** | **Adk23** | **ADK48** |
|---|---|---|---|---|---|---|---|---|
| 13108-L5-2 | CTGCTGCCTTCCTTGGTTGAGGGGCCTGAGCACG | 2673 | -5.44 | -5.44 | 1.00 | -1.07 | -2.30 | -1.55 |
| 13272-R5-2 | TCACCGTGCCTCCCTTTGGAAGAGGTAGAAGTCA | 2674 | -2.63 | -2.63 | -2.63 | -2.63 | -1.60 | 5.15 |
| 13316-R5-2 | CCACCACCTTGCTGCTGGCCCACAGCACCAGGCC | 2675 | -4.30 | -4.30 | -2.50 | -2.88 | -4.30 | -1.25 |
| 13331-L5-2 | CCCGAACCCACTCTGAGCACTCGGCACCAGCA | 2676 | 1.69 | -3.04 | -1.84 | 1.35 | -1.38 | -3.04 |
| 13499-R5-1 | ACAAGTTTCTCAGGAAGTCTGAACACTGGGTTT | 2677 | 2.23 | -2.83 | 1.29 | 1.39 | -1.37 | 1.07 |
| 5971-R5-2 | TGCCTGCTCAGCCTCCCACATCTGTTCCTGG | 2678 | 1.36 | -2.71 | -5.23 | -1.20 | -2.32 | 2.33 |
| 7026-L3-1 | GAAAACCTCACCCACCAGATCCGGGAACGA | 2679 | -4.57 | -4.57 | -4.57 | -3.02 | -2.20 | -4.57 |
| 7471-L5-1 | AAATGCAAATGCCCCCTAAGGAGAAGAACTTC | 2680 | -6.11 | -6.11 | -6.11 | -1.41 | -3.03 | 3.29 |
| miR-320c | ACCCTCTCAACCCAGCTTTT | 2689 | -6.09 | -6.09 | -3.75 | -1.52 | 1.03 | -6.09 |

**Table 36: Additional target RNAs more frequently present at elevated levels in squamous cell carcinoma (SCC), and miR-320c (con't)**

| **Gene** | **Adk40** | **Adk41** | **Adk49** | **Epi42** | **Epi43** | **Ksarc19** | **Kmalp21** | **KmaIp25** | **EPI-4** | **Kmalp44** | **Scc27** | **Lcnec31** | **Car13** |
|---|---|---|---|---|---|---|---|---|---|---|---|---|---|
| 13108-L5-2 | -5.44 | -5.44 | 3.75 | 2.76 | 2.52 | 2.92 | 1.32 | 1.28 | 6.13 | 2.43 | 1.71 | 2.26 | -5.44 |
| 13272-R5-2 | -2.63 | -2.63 | -1.32 | -2.63 | 3.89 | 2.75 | 2.27 | 1.53 | 10.30 | 3.13 | 2.20 | 2.16 | -2.63 |
| 13316-R5-2 | -4.30 | -4.30 | -2.20 | 2.94 | 2.28 | 1.96 | 3.07 | -1.31 | 4.09 | 1.82 | 2.30 | 1.24 | -4.30 |
| 13331-L5-2 | -3.04 | -3.04 | 4.06 | -3.04 | 4.60 | 2.19 | 1.84 | 2.46 | 8.67 | 4.20 | 1.80 | 2.26 | -3.04 |
| 13499-R5-1 | -2.83 | -2.83 | 5.02 | 4.08 | 4.17 | 1.08 | 2.12 | 1.95 | -2.83 | 3.61 | 1.34 | 1.21 | -2.83 |
| 5971-R5-2 | -5.23 | -5.23 | -2.44 | 2.15 | 2.01 | 2.54 | 1.93 | 1.48 | 4.62 | -5.23 | 1.20 | 1.38 | -5.23 |
| 7026-L3-1 | -4.57 | 1.04 | 2.37 | -1.93 | 2.63 | 2.98 | 2.00 | -1.19 | 4.29 | 2.31 | 1.36 | 1.10 | -4.57 |
| 7471-L5-1 | -6.11 | -6.11 | -1.05 | 6.18 | 3.37 | 2.29 | 3.21 | -1.10 | 8.46 | -6.11 | -1.14 | -1.42 | -6.11 |
| miR-320c | -6.09 | -6.09 | 1.74 | -2.76 | 2.05 | 11.65 | 2.58 | 1.15 | 5.22 | 1.76 | 2.06 | -1.01 | -6.09 |

**Table 37: Chromosomal locations and pre-microRNA sequences for target RNAs in Tables 36 and 37**

| **Gene** | **Chromosomal Location** | **precursor sequence** | **SEQ ID NO** |
|---|---|---|---|
| 13108-L5-2 | 2p23.1 | TTCCCACACGTGCTCAGGCCCCTCAACCAAGGAAGGCAGCAGGCCCACTGGCCTCCTTATTCAGAGGGGCTGCACT GCACCCTAGGGAG | 2681 |
| 13272-R5-2 | 12q13.11 | GTCACACAGTTATGTTAGGCCATCACAGCATTGGAATAGGGGATATCTCAGCATGTTGAGCCCTGTCTCTGGGGAGC TGACTTCTACCTCTTCCAAAGGGAGGCACGGTGATTGGAAGTGC | 2682 |
| 13316-R5-2 | 16p12.3,16p13.11 | GCTCAATGCCTCCAACGCAGTCAGCTGGGTCTCAGCCACGTACAACCTCACGGTGGAGGAGCCCATCGTGGGCCTG GTGCTGTGGGCCAGCAGCAAGGTGGTGGCGCCCGGGCA | 2683 |
| 13331-L5-2 | 17q21.2 | TGCGGAGTGCTGGTGCCGAGTGCTCAGAGTGGGTTCGGGTTCAGTCCCTGAACCCAAGCATCCTCTGCACCCAGAT CCTGC | 2684 |
| 13499-R5-1 | 7q32.2 | AACAGTCCAGGGAAATGACAGTTGAGTTGCACAAACCCAGTGTTCAGACTTCCTGAGAAACTTGT | 2685 |
| 5971-R5-2 | 1q24.1 | TGCCATCTGCTCTGAAGCCTCCCAAGCTGGGCCTCCCCTCCCACTTCTGGAGCCCAGGAACAGATGTGGGAGGCTG AGCAGGCA | 2686 |
| 7026-L3-1 | 15q15.3 | TCGTTCCCGGATCTGGTGGGTGAGGTTTTCGATCAGGGCAAATACCTGATCACAGACCTTCACAGGATTCTGGATGA | 2687 |
| 7471-L5-1 | 1q21.1 | GAAGTTCTTCTCCTTAGGGGGCATTTGCATTTTAATGGGAATCTTAAAAACCCAAAGGAAATGTTCTCTAATGGTGGG ATTTC | 2688 |
| miR-320c | 18q11.2 | TTTGCATTAAAAATGAGGCCTTCTCTTCCCAGTTCTTCCCAGAGTCAGGAAAAGCTGGGTTGAGAGGGTAGAAAAAAA ATGATGTAGGCTTCTCTTTCCAGTTCTTCCCAGAATTGGGAAAAGCTGGGTTGAGAGGGT | 2690 |
| miR-320c | 18q11.2 | CTTCTCTTTCCAGTTCTTCCCAGAATTGGGAAAAGCTGGGTTGAGAGGGT | 2691 |

### 5.8 Example 8: Bioinformatic Analysis to Identify microRNAs

In order to identify the microRNAs detected with the probes shown, e.g., in Tables 1, 2, 6 to 9, 18 to 21, 23, 27, 28, 30, and 32 to 34, small RNA sequencing (smRNASeq) datasets were analysed using the probe sequences to identify expressed microRNAs detected by those sequences. The analysis identified 97 sequences with precise ends. Those 97 candidate microRNA sequences are show in Table 38.

**Table 38: microRNA candidate sequences corresponding to probes**

| **Name** | **Candidate sequences 5'->3'** | **SEQ ID NO** |
|---|---|---|
| 10233-R | TCTACTGCCTGCTGCTC | 2576 |
| 10333-L | GGAGGGGGTGGGCAGGG | 2577 |
| 10455-L | AGAGGGATGTTTGGCGC | 2578 |
| 10520-L | CAGAGAAGGCTGGAGGAGG | 2579 |
| 10844-R | AACACATGTTTGAAAGT | 2580 |
| 11358-R | CATGTCAGCCTAGTTTCCC | 2581 |
| 11605-L | AAAGAAGGGAAGAGAAGA | 2582 |
| 11688-R | GTCGGCGTCTCCATCCTG | 2583 |
| 12223-L | AGAGGGAGAAGAAACAA | 2584 |
| 12699-R | CCACCACCGCCACCACCC | 2585 |
| 12707-L | AGTTGTTGGATTGCAGA | 2586 |
| 12723-R | CAGGAAGGGGCTGGGGG | 2587 |
| 12730-R | CCCGGAGAGCGGAGCACAACACA | 2588 |
| 12730-R | CCGGAGAGCGGAGCACAAC | 2589 |
| 12911-L | GAGGAAAAGGAAGGAGG | 2590 |
| 12912-L | CTGTGGGTGGAAGGTGCCAGAA | 2591 |
| 12974-R | AAGTTAAATAACTCTGAACCA | 2592 |
| 13071-L | ATGTGCCGAGATGTGAGCAGTC | 2593 |
| 13071-L | ATGTGCCGAGATGTGAGCAGTCAC | 2594 |
| 13108-L | CCAAGGAAGGCAGCAGGC | 2595 |
| 13115-L | TTGGGGTGGTCGGCCCTGGAGG | 2596 |
| 13122-L | GATGGAATTTCCTAAAGG | 2597 |
| 13124-L | GGAGGGGAGGAGACATG | 2598 |
| 13124-L | GGAGGGGAGGAGACATG | 2599 |
| 13163-R | GGGAGGGAGAGAAGGAG | 2600 |
| 13163-R | GGGAGGGAGAGAAGGAGG | 2601 |
| 13163-R | GGGAGGGAGAGAAGGAGGGG | 2602 |
| 13185-L | AGAGGGAAGAAAAAAAA | 2603 |
| 13225-L | AGTGGGCAGGAAGAACCAGGCT | 2604 |
| 13235-R | AGAGCTGAGACTAGAAAGCCCA | 2605 |
| 13237-L | TTGGGGTGGTCGGCCCTGGAGG | 2606 |
| 13245-L | GGGAGTAGAAGGGAAAGACTAT | 2607 |
| 13247-L | GAGGTCGGGAGGGGAAGGCGGCT | 2608 |
| 13252-L | TCAAGGAGCTCACAGTC | 2609 |
| 13254-R | GCATGAGTGGTTCAGTGGT | 2610 |
| 13272-R | TGACTTCTACCTCTTCCAAAG | 2611 |
| 13274-L | TGAGGGAGGGTGGGAGC | 2612 |
| 13278-R | GTGGAGTCCTGGGGAATGGAGA | 2613 |
| 13287-L | AGGGTGGGGTGGCTCCTCTGCAG | 2614 |
| 13309-L | TCCCTGTCCTCCACAAGCT | 2615 |
| 13316-R | CAGCAGCAAGGTGGTGG | 2616 |
| 13331-L | TGGTGCCGAGTGCTCAGAGTG | 2617 |
| 13334-L | AGTGGGAGGGGAGGGGAGTCCTGCCA | 2618 |
| 13343-L | TGAGGGGCCGGGAGCCA | 2619 |
| 13352-R | TAGCCCCATTTCACAGAT | 2620 |
| 13357-L | GCTGCAGGGAGGTGTGGGGA | 2621 |
| 13358-L | AGTGTGGAGGGGGTGGTGA | 2622 |
| 13366-R | TGGGAATAGGAGAGGGCACTG | 2623 |
| 13373-R | TGCCCCACCTGCTGACCACCCTC | 2624 |
| 13375-R | AACCTTGGCCCCTCTCCCCAG | 2625 |
| 13395-R | GTGGAGTCCTGGGGAATGGAGA | 2626 |
| 13398-R | CAAAGTGAGTCTAGTCTGCA | 2627 |
| 13403-L | TTGGGGGGACACGGGTGG | 2628 |
| 13436-L | AGCTGTGGTCACCGGAGCTCAGAGGC | 2629 |
| 13467-L | GAGGGTCTGACTGTCACTTGGA | 2630 |
| 13468-L | GAGGGTCTGATGGCCACTTGGA | 2631 |
| 13472-L | CAGGAGGACGTCACACACAGTG | 2632 |
| 13473-L | TAGGTGGTAGAAGGGCAAACA | 2633 |
| 13499-R | CCCAGTGTTCAGACTTCCTG | 2634 |
| 13500-L | TGGAGAGAGAGGCCTGGAAGA | 2635 |
| 13500-L | TGGAGAGAGAGGCCTGGAAGAT | 2636 |
| 13508-L | TGTGAGGAAGCTAAGAGCAG | 2637 |
| 13522-L | AGTGCAGTGGTGTGATC | 2638 |
| 13523-R | GCAGGGGAAGAAGCCTT | 2639 |
| 13530-L | GGGGGACCCAGGGGAAGGAGG | 2640 |
| 13545-L | ACAGCCTCCCTTGCGCCACAG | 2641 |
| 25-R | TTAGAAAAAGAGGGGGTGAGG | 2642 |
| 3249-L | GCGGCGGCGGCGGCGGC | 2643 |
| 3371-L | TGGGGTGTGGAGGGGAGG | 2644 |
| 3744-R | AGGGGAGCAGGGAGGAA | 2645 |
| 3744-R | GCAGGGAGGAAGGAGAA | 2646 |
| 3923-R | TGGAGAGGGATGAAGGAGAT | 2647 |
| 4440-L | TCTGCCCAGTGCTCTGAATGTCA | 2648 |
| 4440-L | CCAGTGCTCTGAATGTCAAA | 2649 |
| 5080-R | TGGAGAGGGATGAAGGAGA | 2650 |
| 5192-L | GAGGAAGGAAGGGGAAA | 2651 |
| 5192-L | AGGAAGGAAGGGGAAAAA | 2652 |
| 5232-L | CCTTGATGTCCTGCAAATGA | 2653 |
| 5392-R | ATGAGATACTGTCGGAGA | 2654 |
| 5723-R | CTGCTGGAGAAAGGGGAGGAGG | 2655 |
| 5842-R | TTCATTTCTCTTTCATAA | 2656 |
| 5971-R | ACTTCTGGAGCCCAGGAACA | 2657 |
| 6037-R | TAGTTGGAGGAAAATTGGAG | 2658 |
| 6216-L | TGCCCAGTGCTCTGAATGTC | 2659 |
| 6216-R | ACGGCGGGAGTAACTATG | 2660 |
| 6235-R | AAATGGATTTTTGGAGCAG | 2661 |
| 6496-R | AGGGGAAGTGGTGGGTG | 2662 |
| 6496-R | GTGGGTGGGGGAGGGGG | 2663 |
| 6681-R | GGAGGGGCAGAGAGAGAG | 2664 |
| 6864-R | AGGTGGGCACTCTAAGG | 2665 |
| 6906-L | TGGTTGGGGAGAAGACA | 2666 |
| 6930-R | TGCAAGATCAGAGGGGAGA | 2667 |
| 7726-R | TCCTCTTCTCCTCTTCT | 2668 |
| 8433-L | CGGTGGAGGGAAAGGGGAAA | 2669 |
| 8452-L | AACACCCCAGCCATGTA | 2670 |
| 8832-R | CAAAGTGGGGGAAAAACAGGTG | 2671 |
| 9349-R | TCCGAGACCTGGAGCAG | 2672 |

| | |
|---|---|
| [00327] | All publications, patents, patent applications and other documents cited in this application are hereby incorporated by reference in their entireties for all purposes to the same extent as if each individual publication, patent, patent application or other document were individually indicated to be incorporated by reference for all purposes. |
| [00328] | While various specific embodiments have been illustrated and described, it will be appreciated that changes can be made without departing from the spirit and scope of the invention(s). |

### EMBODIMENTS OF THE INVENTION

1. A method for detecting the presence of lung cancer in a subject, the method comprising detecting a level of at least one target RNA in a sample from the subject, wherein the at least one target RNA:
   (i) is capable of specifically hybridizing to a nucleic acid having a sequence selected from SEQ ID NOs: 1 to 397, 1063 to 1210, 2064 to 2183, 2673 to 2680, and 2689; or
   (ii) comprises a sequence that is complementary to at least 15 contiguous nucleotides of a sequence selected from SEQ ID NOs: 1 to 397, 1063 to 1210, 2064 to 2183, 2673 to 2680, and 2689; or
   (iii) comprises at least 15 contiguous nucleotides of a sequence
      selected from SEQ ID NOs: 794 to 1043, 2576 to 2672, and 2692;
      wherein a level of at least one target RNA in the sample that is greater than a normal level of the at least one target RNA indicates the presence of lung cancer in the subject.
2. The method of embodiment 1, wherein the method further comprises comparing the level of the at least one target RNA in the sample to a normal level of the at least one target RNA.
3. A method for facilitating the detection of lung cancer in a subject, comprising:
   (a) detecting a level of at least one target RNA in a sample from the subject, wherein the at least one target RNA:
      (i) is capable of specifically hybridizing to a nucleic acid having a sequence selected from SEQ ID NOs: 1 to 397, 1063 to 1210, 2064 to 2183, 2673 to 2680, and 2689; or
      (ii) comprises a sequence that is complementary to at least 15 contiguous nucleotides of a sequence selected from SEQ ID NOs: 1 to 397, 1063 to 1210, 2064 to 2183, 2673 to 2680, and 2689; or
      (iii) comprises at least 15 contiguous nucleotides of a sequence selected from SEQ ID NOs: 794 to 1043, 2576 to 2672, and 2692; and
   (b) communicating the results of the detection to a medical practitioner for the purpose of determining whether the subject has lung cancer.
4. The method of any one of the preceding embodiments, wherein detecting a level of at least one target RNA in a sample comprises:
   (a) hybridizing nucleic acids of the sample with at least one polynucleotide that is complementary to a target RNA in the sample or to a complement thereof; and
   (b) detecting at least one complex comprising a polynucleotide hybridized to at least one nucleic acid selected from the target RNA, a DNA amplicon of the target RNA, and a complement of the target RNA.
5. A method for detecting the presence of lung cancer in a subject, comprising:
   (a) obtaining a sample from the subject,
   (b) providing the sample to a laboratory for detection of the level of at least one target RNA in the sample, wherein the at least one target RNA:
      (i) is capable of specifically hybridizing to a nucleic acid having a sequence selected from SEQ ID NOs: 1 to 397, 1063 to 1210, 2064 to 2183, 2673 to 2680, and 2689; or
      (ii) comprises a sequence that is complementary to at least 15 contiguous nucleotides of a sequence selected from SEQ ID NOs: 1 to 397, 1063 to 1210, 2064 to 2183, 2673 to 2680, and 2689; or
      (iii) comprises at least 15 contiguous nucleotides of a sequence selected from SEQ ID NOs: 794 to 1043, 2576 to 2672, and 2692; and
   (c) receiving from the laboratory a communication indicating the level of at least one target RNA in the sample;
      wherein a level of at least one target RNA that is greater than a normal level of the at least one target RNA indicates the presence of lung cancer.
6. The method of any one of embodiments 1 to 4, wherein the method further comprises isolating nucleic acids from the sample.
7. The method of embodiment 6, wherein the nucleic acids comprise RNA that has been separated from DNA.
8. The method of any of the preceding embodiments, wherein at least one target RNA in its mature form comprises fewer than 30 nucleotides.
9. The method of any of the preceding embodiments, wherein at least one target RNA is a microRNA.
10. The method of any of the preceding embodiments, wherein levels of at least two target RNAs are detected, wherein at least two of the target RNAs:
   (i) are capable of specifically hybridizing to a nucleic acid having a sequence selected from SEQ ID NOs: 1 to 397, 1063 to 1210, 2064 to 2183, 2673 to 2680, and 2689; or
   (ii) comprise a sequence that is complementary to at least 15 contiguous nucleotides of a sequence selected from SEQ ID NOs: 1 to 397, 1063 to 1210, 2064 to 2183, 2673 to 2680, and 2689; or
   (iii) comprise at least 15 contiguous nucleotides of a sequence
      selected from SEQ ID NOs: 794 to 1043, 2576 to 2672, and 2692; and
      wherein the at least two target RNAs are different.
11. The method of embodiment 10, wherein detection of a level of at least one target RNA that is greater than a normal level of the at least one target RNA indicates the presence of lung cancer.
12. The method of embodiment 10, wherein detection of levels of at least two target RNAs that are greater than normal levels of the at least two target RNAs indicates the presence of lung cancer.
13. The method of embodiment 10, wherein levels of at least three target RNAs are detected, wherein at least three of the target RNAs:
   (i) are capable of specifically hybridizing to a nucleic acid having a sequence selected from SEQ ID NOs: 1 to 397, 1063 to 1210, 2064 to 2183, 2673 to 2680, and 2689; or
   (ii) comprise a sequence that is complementary to at least 15 contiguous nucleotides of a sequence selected from SEQ ID NOs: 1 to 397, 1063 to 1210, 2064 to 2183, 2673 to 2680, and 2689; or
   (iii) comprise at least 15 contiguous nucleotides of a sequence
      selected from SEQ ID NOs: 794 to 1043, 2576 to 2672, and 2692; and
      wherein the at least three target RNAs are different.
14. The method of embodiment 13, wherein detection of a level of at least one target RNA that is greater than a normal level of the at least one target RNA indicates the presence of lung cancer.
15. The method of embodiment 13, wherein detection of levels of at least two target RNAs that are greater than normal levels of the at least two target RNAs indicates the presence of lung cancer.
16. The method of embodiment 13, wherein detection of levels of at least three target RNAs that are greater than normal levels of the at least three target RNAs indicates the presence of lung cancer.
17. The method of embodiment 10, wherein levels of at least five target RNAs are detected.
18. The method of any one of embodiments 10 to 17, wherein a level is detected of at least one target RNA that:
   (i) does not specifically hybridize to a nucleic acid having a sequence selected from SEQ ID NOs: 1 to 397, 1063 to 1210, 2064 to 2183, 2673 to 2680, and 2689; or
   (ii) does not comprise a sequence that is complementary to at least 15 contiguous nucleotides of a sequence selected from SEQ ID NOs: 1 to 397, 1063 to 1210, 2064 to 2183, 2673 to 2680, and 2689; and
   (iii) does not comprise at least 15 contiguous nucleotides of a sequence selected from SEQ ID NOs: 794 to 1043, 2576 to 2672, and 2692.
19. The method of any one of the preceding embodiments, wherein at least one target RNA:
   (a) is capable of specifically hybridizing to a polynucleotide sequence in Table 6; or
   (b) comprises a sequence that is complementary to at least 15 contiguous nucleotides of a polynucleotide sequence in Table 6.
20. The method of embodiment 19, wherein detection of a level of the at least one target RNA that is greater than a normal level of the at least one target RNA indicates the presence of non-small cell lung cancer.
21. The method of any one of embodiments 1 to 18, wherein at least one target RNA:
   (a) is capable of specifically hybridizing to a polynucleotide sequence in Table 7; or
   (b) comprises a sequence that is complementary to at least 15 contiguous nucleotides of a polynucleotide sequence in Table 7.
22. The method of embodiment 21, wherein detection of a level of the at least one target RNA that is greater than a normal level of the at least one target RNA indicates the presence of squamous cell carcinoma.
23. The method of any one of embodiments 1 to 18, wherein at least one target RNA:
   (a) is capable of specifically hybridizing to a polynucleotide sequence in Table 8; or
   (b) comprises a sequence that is complementary to at least 15 contiguous nucleotides of a polynucleotide sequence in Table 8.
24. The method of embodiment 23, wherein detection of a level of the at least one target RNA that is greater than a normal level of the at least one target RNA indicates the presence of adenocarcinoma.
25. The method of any one of embodiments 1 to 18, wherein at least one target RNA:
   (a) is capable of specifically hybridizing to a polynucleotide sequence in Table 9; or
   (b) comprises a sequence that is complementary to at least 15 contiguous nucleotides of a polynucleotide sequence in Table 9.
26. The method of embodiment 25, wherein detection of a level of the at least one target RNA that is greater than a normal level of the at least one target RNA indicates the presence of aggressive lung cancer.
27. The method of any one of embodiments 1 to 18, wherein at least one target RNA:
   (a) is capable of specifically hybridizing to a polynucleotide sequence in Table 32 or Table 33; or
   (b) comprises a sequence that is complementary to at least 15 contiguous nucleotides of a polynucleotide sequence in Table 32 or Table 33.
28. A method for detecting the presence of lung cancer in a subject, the method comprising detecting a level of at least one target RNA in a sample from the subject, wherein the at least one target RNA:
   (i) is capable of specifically hybridizing to a nucleic acid having a sequence selected from SEQ ID NOs: 1 to 397, 1363 to 1707, and 2312 to 2452; or
   (ii) comprises a sequence that is complementary to at least 15 contiguous nucleotides of a sequence selected from SEQ ID NOs: 1 to 397, 1363 to 1707, and 2312 to 2452; wherein a level of at least one target RNA in the sample that is reduced relative to a normal level of the at least one target RNA indicates the presence of lung cancer in the subj ect.
29. The method of embodiment 28, wherein the method further comprises comparing the level of the at least one target RNA in the sample to a normal level of the at least one target RNA.
30. The method of embodiment 28 or 29, wherein the method further comprises detecting a level of at least one second target RNA in a sample from the subject, wherein the at least one second target RNA:
   (i) is capable of specifically hybridizing to a nucleic acid having a sequence selected from SEQ ID NOs: 1 to 397, 1063 to 1210, 2064 to 2183, 2673 to 2680, and 2689; or
   (ii) comprises a sequence that is complementary to at least 15 contiguous nucleotides of a sequence selected from SEQ ID NOs: 1 to 397, 1063 to 1210, 2064 to 2183, 2673 to 2680, and 2689; or
   (iii) comprises at least 15 contiguous nucleotides of a sequence
      selected from SEQ ID NOs: 794 to 1043, 2576 to 2672, and 2692;
      wherein a level of at least one second target RNA in the sample that is greater than a normal level of the at least one second target RNA indicates the presence of lung cancer in the subj ect.
31. A method for facilitating the detection of lung cancer in a subject, comprising:
   (a) detecting a level of at least one target RNA in a sample from the subject, wherein the at least one target RNA:
      (i) is capable of specifically hybridizing to a nucleic acid having a sequence selected from SEQ ID NOs: 1 to 397, 1363 to 1707, and 2312 to 2452; or
      (ii) comprises a sequence that is complementary to at least 15 contiguous nucleotides of a sequence selected from 1 to 397, 1363 to 1707, and 2312 to 2452; and
   (b) communicating the results of the detection to a medical practitioner for the purpose of determining whether the subject has lung cancer.
32. The method of embodiment 31, wherein the method further comprises
   (a) detecting a level of at least one second target RNA in a sample from the subject, wherein the at least one second target RNA:
      (i) is capable of specifically hybridizing to a nucleic acid having a sequence selected from SEQ ID NOs: 1 to 397, 1063 to 1210, 2064 to 2183, 2673 to 2680, and 2689; or
      (ii) comprises a sequence that is complementary to at least 15 contiguous nucleotides of a sequence selected from SEQ ID NOs: 1 to 397, 1063 to 1210, 2064 to 2183, 2673 to 2680, and 2689; or
      (iii) comprises at least 15 contiguous nucleotides of a sequence selected from SEQ ID NOs: 794 to 1043, 2576 to 2672, and 2692; and
   (b) communicating the results of the detection to a medical practitioner for the purpose of determining whether the subject has lung cancer.
33. The method of any one of embodiments 28 to 32, wherein detecting a level of at least one target RNA in a sample comprises:
   (a) hybridizing nucleic acids of the sample with at least one polynucleotide that is complementary to a target RNA in the sample or to a complement thereof; and
   (b) detecting at least one complex comprising a polynucleotide hybridized to at least one nucleic acid selected from the target RNA, a DNA amplicon of the target RNA, and a complement of the target RNA.
34. A method for detecting the presence of lung cancer in a subject, comprising:
   (a) obtaining a sample from the subject;
   (b) providing the sample to a laboratory for detection of the level of at least one target RNA in the sample, wherein the at least one target RNA:
      (i) is capable of specifically hybridizing to a nucleic acid having a sequence selected from SEQ ID NOs: 1 to 397, 1363 to 1707, and 2312 to 2452; or
      (ii) comprises a sequence that is complementary to at least 15 contiguous nucleotides of a sequence selected from SEQ ID NOs: 1 to 397, 1363 to 1707, and 2312 to 2452; and
   (c) receiving from the laboratory a communication indicating the level of at least one target RNA in the sample;
      wherein a level of at least one target RNA that is reduced relative to a normal level of the at least one target RNA indicates the presence of lung cancer.
35. The method of embodiment 34, wherein the sample is provided to a laboratory for detection of the level of at least one second target RNA in the sample, wherein the at least one second target RNA:
   (i) is capable of specifically hybridizing to a nucleic acid having a sequence selected from SEQ ID NOs: 1 to 397, 1063 to 1210, 2064 to 2183, 2673 to 2680, and 2689; or
   (ii) comprises a sequence that is complementary to at least 15 contiguous nucleotides of a sequence selected from SEQ ID NOs: 1 to 397, 1063 to 1210, 2064 to 2183, 2673 to 2680, and 2689; or
   (iii) comprises at least 15 contiguous nucleotides of a sequence selected from SEQ ID NOs: 794 to 1043, 2576 to 2672, and 2692.
36. The method of any one of embodiments 28 to 33, wherein the method further comprises isolating nucleic acids from the sample.
37. The method of embodiment 36, wherein the nucleic acids comprise RNA that has been separated from DNA.
38. The method of any one of embodiments 28 to 37, wherein at least one target RNA in its mature form comprises fewer than 30 nucleotides.
39. The method of any one of embodiments 28 to 38, wherein at least one target RNA is a microRNA.
40. A synthetic polynucleotide comprising a first region, wherein the first region comprises a sequence of at least 8 contiguous nucleotides that is identical or complementary to a sequence of at least 8 contiguous nucleotides of one of SEQ ID NOs: 1 to 165, 254 to 360, 1063 to 1081, 1090 to 1190, 2064 to 2091, 2107 to 2168, 1363 to 1659, 2312 to 2440, and 2576 to 2680.
41. The synthetic polynucleotide of embodiment 40, wherein the first region comprises a sequence of at least 9 contiguous nucleotides that is identical or complementary to a sequence of at least 9 contiguous nucleotides of one of SEQ ID NOs: 1 to 165, 254 to 360, 1063 to 1081, 1090 to 1190, 2064 to 2091, 2107 to 2168, 1363 to 1659, 2312 to 2440, and 2576 to 2680.
42. The synthetic polynucleotide of embodiment 40, wherein the first region comprises a sequence of at least 10 contiguous nucleotides that is identical or complementary to a sequence of at least 10 contiguous nucleotides of one of SEQ ID NOs: 1 to 165, 254 to 360, 1063 to 1081, 1090 to 1190, 2064 to 2091, 2107 to 2168, 1363 to 1659, 2312 to 2440, and 2576 to 2680.
43. The synthetic polynucleotide of embodiment 40, wherein the first region comprises a sequence of at least 12 contiguous nucleotides that is identical or complementary to a sequence of at least 12 contiguous nucleotides of one of SEQ ID NOs: 1 to 165, 254 to 360, 1063 to 1081, 1090 to 1190, 2064 to 2091, 2107 to 2168, 1363 to 1659, 2312 to 2440, and 2576 to 2680.
44. The synthetic polynucleotide of any one of embodiments 40 to 43,
   wherein the polynucleotide comprises a detectable label.
45. The synthetic polynucleotide of embodiment 44, wherein the detectable label is a FRET label.
46. The synthetic polynucleotide of any one of embodiments 40 to 45,
   wherein the first region is identical or complementary to a region of a target RNA.
47. The synthetic polynucleotide of embodiment 46, wherein the polynucleotide further comprises a second region that is not identical or complementary to a region of the target RNA.
48. A composition comprising a plurality of synthetic polynucleotides,
   wherein at least one polynucleotide comprises a first region comprising a sequence of at least 8 contiguous nucleotides that is identical or complementary to a sequence of at least 8 contiguous nucleotides of one or more of SEQ ID NOs: 1 to 165, 254 to 360, 1063 to 1081, 1090 to 1190, 2064 to 2091, 2107 to 2168, 1363 to 1659, 2312 to 2440, and 2576 to 2680.
49. The composition of embodiment 48, wherein at least two polynucleotides of the plurality of synthetic polynucleotides comprise a first region comprising a sequence of at least 9 contiguous nucleotides that is identical or complementary to a sequence of at least 9 contiguous nucleotides of one or more of SEQ ID NOs: 1 to 165, 254 to 360, 1063 to 1081, 1090 to 1190, 2064 to 2091, 2107 to 2168, 1363 to 1659, 2312 to 2440, and 2576 to 2680, and wherein the first regions of the at least two polynucleotides are different.
50. The composition of embodiment 48, wherein at least three polynucleotides of the plurality of synthetic polynucleotides comprise a first region comprising a sequence of at least 10 contiguous nucleotides that is identical or complementary to a sequence of at least 10 contiguous nucleotides of one or more of SEQ ID NOs: 1 to 165, 254 to 360, 1063 to 1081, 1090 to 1190, 2064 to 2091, 2107 to 2168, 1363 to 1659, 2312 to 2440, and 2576 to 2680, and wherein the first regions of the at least three polynucleotides are different.
51. The composition of embodiment 48, wherein at least five polynucleotides of the plurality of synthetic polynucleotides comprise a first region comprising a sequence of at least 12 contiguous nucleotides that is identical or complementary to a sequence of at least 12 contiguous nucleotides of one or more of SEQ ID NOs: 1 to 165, 254 to 360, 1063 to 1081, 1090 to 1190, 2064 to 2091, 2107 to 2168, 1363 to 1659, 2312 to 2440, and 2576 to 2680, and wherein the first regions of the at least five polynucleotides are different.
52. A kit comprising a synthetic polynucleotide of any one of embodiments 40 to 47.
53. A kit comprising a composition of any one of embodiments 48 to 51.
54. The kit of embodiment 52 or embodiment 53, wherein the kit further comprises at least one polymerase.
55. The kit of any one of embodiments 52 to 54, wherein the kit further comprises dNTPs.

## Claims

1. A method for detecting the presence of lung cancer in a subject, the method comprising detecting the level of a target RNA in a sample from the subject, wherein the target RNA:
(i) is capable of specifically hybridizing to a nucleic acid having a sequence of SEQ ID NO: 1089; or
(ii) comprises a sequence that is complementary to at least 15 contiguous nucleotides of a sequence of SEQ ID NO:1089;
wherein a level of the target RNA in the sample that is greater than a normal level of the target RNA indicates the presence of lung cancer in the subject.

2. A method for assessing the effectiveness of a treatment for lung cancer in a patient, comprising:
(a) detecting the level of a target RNA in a sample from the patient, wherein the target RNA:
(i) is capable of specifically hybridizing to a nucleic acid having a sequence of SEQ ID NO: 1089; or
(ii) comprises a sequence that is complementary to at least 15 contiguous nucleotides of a sequence of SEQ ID NO: 1089.

3. The method of claim 1 or claim 2, wherein detecting the level of the target RNA in the sample comprises:
(a) hybridizing nucleic acids of the sample with at least one polynucleotide that is complementary to the target RNA in the sample or to a complement thereof; and
(b) detecting at least one complex comprising a polynucleotide hybridized to at least one nucleic acid selected from the target RNA, a DNA amplicon of the target RNA, and a complement of the target RNA.

4. The method of any one of claims 1 to 3, wherein the method further comprises isolating nucleic acids from the sample and optionally the nucleic acids comprise RNA that has been separated from DNA.

5. The method of any one of the preceding claims, wherein the level of at least one additional target RNA is detected, wherein the additional target RNA:
(i) is capable of specifically hybridizing to a nucleic acid having a sequence selected from SEQ ID NOs: 1 to 397, 1063 to 1088, 1090 to 1210, 2064 to 2183, 2673 to 2680, and 2689; or
(ii) comprises a sequence that is complementary to at least 15 contiguous nucleotides of a sequence selected from SEQ ID NOs: 1 to 397, 1063 to 1088, 1090 to 1210, 2064 to 2183, 2673 to 2680, and 2689; or
(iii) comprise at least 15 contiguous nucleotides of a sequence selected from SEQ ID NOs: 794 to 916, 918 to 1043, 2576 to 2672, and 2692.

6. The method of claim 5, wherein detection of a level of at least one target RNA that is greater than a normal level of the at least one target RNA indicates the presence of lung cancer.

7. The method of claim 5, wherein detection of levels of at least two target RNAs that are greater than normal levels of the at least two target RNAs indicates the presence of lung cancer.

8. The method of claim 5, wherein the levels of at least two additional target RNAs are detected, wherein the at least two additional target RNAs:
(i) are capable of specifically hybridizing to a nucleic acid having a sequence selected from SEQ ID NOs: 1 to 397, 1063 to 1088, 1090 to 1210, 2064 to 2183, 2673 to 2680, and 2689; or
(ii) comprise a sequence that is complementary to at least 15 contiguous nucleotides of a sequence selected from SEQ ID NOs: 1 to 397, 1063 to 1088, 1090 to 1210, 2064 to 2183, 2673 to 2680, and 2689; or
(iii) comprise at least 15 contiguous nucleotides of a sequence selected from SEQ ID NOs: 794 to 916, 918 to 1043, 2576 to 2672, and 2692; and
wherein the at least two additional target RNAs are different.

9. The method of claim 8, wherein detection of a level of at least one target RNA that is greater than a normal level of the at least one target RNA indicates the presence of lung cancer.

10. The method of claim 8, wherein detection of levels of at least two target RNAs that are greater than normal levels of the at least two target RNAs indicates the presence of lung cancer.

11. The method of claim 8, wherein detection of levels of at least three target RNAs that are greater than normal levels of the at least three target RNAs indicates the presence of lung cancer.

12. The method of claim 5, wherein the levels of at least five target RNAs are detected.

13. The method of any one of the preceding claims, wherein the sample is lung tissue.

14. The method of claim 13, wherein the lung tissue is a sample of lung cells obtained by bronchoscopy.

15. The method of any one of claims 1-12, wherein the sample is blood or serum.
